(19) Europäisches Patentamt
European Patent Office
Office européen des brevets

(11) EP 2 240 601 B1

(12) **EUROPEAN PATENT SPECIFICATION**

(45) Date of publication and mention of the grant of the patent:
**12.10.2016 Bulletin 2016/41**

(21) Application number: **08867219.1**

(22) Date of filing: **01.12.2008**

(51) Int Cl.:
***C12Q 1/68*** *(2006.01)*

(86) International application number:
**PCT/IL2008/001561**

(87) International publication number:
**WO 2009/083950 (09.07.2009 Gazette 2009/28)**

(54) **BIOMARKERS FOR THE PREDICTION OF RENAL INJURY**

BIOMARKER FÜR DIE VORHERSAGE VON NIERENLÄSION

BIOMARQUEURS POUR PRÉVOIR L'APPARITION D'UNE LÉSION RÉNALE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **27.12.2007 US 16837 P**

(43) Date of publication of application:
**20.10.2010 Bulletin 2010/42**

(73) Proprietor: **Compugen Ltd.**
**69512 Tel Aviv (IL)**

(72) Inventors:
- **KINAR, Yaron**
  **63264 Tel Aviv (IL)**
- **BEIMAN, Merav**
  **74208 Ness Ziona (IL)**
- **MONTIA, Eve**
  **76305 Rehovot (IL)**
- **WALLACH, Shira**
  **45265 Hod Hasharon (IL)**
- **PERGAM, Tania**
  **75218 Rishon Le Zion (IL)**
- **NOVIK, Amit**
  **40600 Tel Mond (IL)**
- **SAMEAH-GREENWALD, Shirley**
  **44419 Kfar Saba (IL)**
- **COJOCARU, Gad S.**
  **47248 Ramat-Hasharon (IL)**
- **COHEN-DAYAG, Anat**
  **76248 Rehovot (IL)**
- **FURMAN, Yael**
  **47207 Ramat Hasharon (IL)**

(74) Representative: **Becker Kurig Straus**
**Patentanwälte**
**Bavariastrasse 7**
**80336 München (DE)**

(56) References cited:
**WO-A-2006/033701**
**US-A1- 2002 192 745**
**US-A1- 2005 272 080**

- **JUSTO P ET AL: "Cytokine cooperation in renal tubular cell injury: the role of TWEAK." KIDNEY INTERNATIONAL NOV 2006, vol. 70, no. 10, November 2006 (2006-11), pages 1750-1758, XP002524905 ISSN: 0085-2538**
- **YUEN PETER S T ET AL: "Ischemic and nephrotoxic acute renal failure are distinguished by their broad transcriptomic responses." PHYSIOLOGICAL GENOMICS 16 MAY 2006, vol. 25, no. 3, 16 May 2006 (2006-05-16), pages 375-386, XP002524906 ISSN: 1531-2267**
- **AMIN R P ET AL: "Identification of putative gene-based markers of renal toxicity" ENVIRONMENTAL HEALTH PERSPECTIVES 200403 US, vol. 112, no. 4, March 2004 (2004-03), pages 465-479, XP009115515 ISSN: 0091-6765**
- **KHARASCH EVAN D ET AL: "Gene expression profiling of nephrotoxicity from the sevoflurane degradation product fluoromethyl-2,2-difluoro-1-(trifluoromethyl)vinyl ether ("compound A") in rats." TOXICOLOGICAL SCIENCES : AN OFFICIAL JOURNAL OF THE SOCIETY OF TOXICOLOGY APR 2006, vol. 90, no. 2, April 2006 (2006-04), pages 419-431, XP002524907 ISSN: 1096-6080**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 240 601 B1

- YOSHIDA T ET AL: "Monitoring changes in gene expression in renal ischemia-reperfusion in the rat" KIDNEY INTERNATIONAL 2002 US, vol. 61, no. 5, 2002, pages 1646-1654, XP002524908 ISSN: 0085-2538 cited in the application
- HUANG Q ET AL: "ASSESSMENT OF CISPLATIN-INDUCED NEPHROTOXICITY BY MICROARRAY TECHNOLOGY" TOXICOLOGICAL SCIENCES, ACADEMIC PRESS, SAN DIEGO, FL, US, vol. 63, no. 2, 1 October 2001 (2001-10-01), pages 196-207, XP001096461 ISSN: 1096-6080
- DATABASE EMBL [Online] 31 October 2003 (2003-10-31), "Rattus norvegicus tumor necrosis factor receptor superfamily, member 12a, mRNA (cDNA clone MGC:72653 IMAGE:6887545), complete cds." XP002524909 retrieved from EBI accession no. EMBL:BC060537 Database accession no. BC060537
- DATABASE EMBL [Online] 30 April 2004 (2004-04-30), "UI-R-DQ1-clh-g-10-0-UI.s1 UI-R-DQ1 Rattus norvegicus cDNA clone UI-R-DQ1-clh-g-10-0-UI 3’, mRNA sequence." XP002524910 retrieved from EBI accession no. EMBL:CN542345 Database accession no. CN542345

SEQUENCE LISTING

<110> Compugen Ltd.

<120> BIOMARKERS FOR THE PREDICTION OF RENAL INJURY

<130> COMP/043 PCT

<160> 332

<210> 1
<211> 1233
<212> DNA
<213> Artificial

<400> 1

agccccgagt cttggcttgc gggtggtggg agcagacagt tcttgcctcg ggaccggcaa 60
tcatggcttc ggcttggccg cggtctctgc cgcagatcct cgtgttggga ttcggcttgg 120
tgttgatgcg cgccgcggcc ggggagcaag caccaggcac ctccccatgc tctagcggca 180
gctcctggag cgcggacctc gacaagtgca tggactgcgc ttcttgtcca gcgcgaccac 240
acagcgactt ctgcctggga tgtgagtggg ggtggggacg gggctggtgg caagcggatc 300
ctagatctgg gtaggtggtt gttggggcag aaaggaggtc gtagacttag gatataggaa 360
accaggaaaa actgactgag gaagggactt ttataagggt gtgtgttggg agggggtaa 420
gcttgagacc tccttgagga cagactagac tggcgtccca agggcaagat ctgatttgaa 480
ccctgatcac taggcgccgc agcacctcct gcccacttca ggctactgtg gcccattctg 540
gggggcgctc ttagtctggt cctggttttg gcgctggttt ctagtttcct ggtctggaga 600
agatgccgcc ggagagaaaa gtttactacc cccatagagg agactggtgg agagggctgc 660
ccaggtgtgg cactgatcca gtgaggagca cccgcgctgg ggcccactcg tcgtccattc 720
attcattctg gagtcagcct ggccttccag acagaagccg agccagactc tttcaaccac 780
aagggggtgg ggcgaggtgg tgattcacct ccaaggactg ggcttagagt tcagggagcc 840
ttccaaggtg tccaattgcc ctatctctgg gtctggggca gacagagagc ctcaatctgg 900
gtcacaaagc gacccatact aaggaactgc agcatttgca caagggaatc tcttgttccc 960
cacaagtcct ggcagtctgg ctgacttgag gggcagacac aacactgggt cccacccact 1020
cggaggggct ataatgccac tccctgagtt ttagccctag gagtgggtca gattgacaga 1080
cctgttctta acactaaggg ggctggccct ctgggagggc tggccccagt acacacggaa 1140
acaacccatc tcccaagggg gtgatattta ttgtggggat aatgtttgga ggggagggag 1200
acttattaat aaaagaatct ttaactttaa atg 1233

<210> 2
<211> 230
<212> DNA
<213> Artificial

<220>
<223> Synthetic oligonucleotide

<400> 2

gtgagtgggg gtggggacgg ggctggtggc aagcggatcc tagatctggg taggtggttg 60
ttggggcaga aaggaggtcg tagacttagg atataggaaa ccaggaaaaa ctgactgagg 120
aagggacttt tataagggtg tgcgttggga gggggtaag cttgagaccc tttgagggca 180
ggctggattg gcgtcccaag ggcaagatct gatttgaacc cgatcactag 230

<210> 3
<211> 970
<212> DNA
<213> Artificial

<400> 3

ggaggagggc agagccagag gagggacagc ctgatgcaga cagccctgac tcaacctgcc 60
agccccctta cctggccagc cttgaggaga tggaacagcc caggctacaa ggcctgcccc 120
cactcctcaa cttcccttga taatgaacca gaaactgaaa ctaaaacagg caggctcccc 180
gttagagatg agtcacttcc caaagtgact gaagtagccg agaagtggaa acagaggggc 240
agaagagaac ccagggcact gagacagggc tttctgaggg tcgccaagga gcatggcagg 300
catcccagag gtggtggcca tggactgtga gatggtgggg cttgggcctc aaagggtgag 360
tggcctcgcc cgctgcagca ttgtgaacat ccatggcgca gtcctgtatg acaagtacat 420
ccgacccgag ggagagatca cggactacag aacccaagtc agcggggtca cgcctcagca 480
catggtgagg gccacgccat ttggtgaagc caggctagag atcctgcagc ttctgaaagg 540
caagctggtg gtgggccatg acctgaagca cgacttcaat gccctgaagg aggatatgag 600
caagtacacc atctatgaca cgtccacaga caggctgctg tggcatgagg ccaagctgca 660
gtactacagc cgagtgtccc tgaggctgct gtgtaagcgc ctgctacaca agaacatcca 720
ggtgaggagc ttcccggccc ctgtgaccct tctctctctt ctccctcctc tctctctccc 780
tccctcctcc cactcaggta aggagtaacc tgcccttcct cttcctcctc tcctcctcca 840
agcaaggagt cctcctgctt tctcccttag tcctccctct ccttccctga ttctaaaagt 900
gaggatcttc ctctccctcc tccccgtgct cccactcctc ccaagggcag gtcagtccca 960
cctggcctaa 970

<210> 4
<211> 255
<212> DNA
<213> Artificial

<220>
<223> synthetic oligonucleotide

<400> 4

ggaggagggc agagccagag gagggacagc ctgatgcaga cagccctgac tcaacctgcc 60

agccccctta cctgtcagcc ttgaggagat ggaacagccc agcctactag gcctgccccc 120
actccccaac ttcccttgat aatgaaccag aaactgaaac taaaacggct ccgcttgatg 180
agtcacttcc caaagtgact aaagtagctg agaagtagaa acagaggcag aagagaaccc 240
agggcactgg gacag 255

<210> 5
<211> 249
<212> DNA
<213> Artificial

<220>
<223> Synthetic oligonucleotide

<400> 5

```
gtgaggagct tcccggcccc tgtgaccctt ctctctcttc tccctcctct ctctctccct     60
ccctcctccc actcaggtaa ggagtaacct gcccttcctc ttcctcctct cctcctccaa    120
gcaaggagtc ctcctgcttt ctcccttagt cctccctctc cttccctgat tctaaaagtg    180
aggatcttcc tctccctcct ccccgtgctc ccactcctcc caagggcagg tcagtcccac    240
ctggcctaa                                                            249
```

<210> 6
<211> 2447
<212> DNA
<213> Artificial

<400> 6

```
agcgaccggc agggtgcccg cccgacttcc cagctgcccc gtgcggcccg ggcatgccca     60
gtgtgggcgc agccccggct ctccgagcgc ccgggcggag cgggcagccg ggcgcggagc    120
aggcgaaccg agcctcggtg ggcgagcgcc ctggctggag ccgcggcggt agggtggcgg    180
ggccctccat gatgaatggt ttgggggcac ttccctctcg ctgtatttga tagtctgggc    240
agtggagaga tggctgacag tgtcaaaacc tttctgcagg accttggcag gggaatcaaa    300
gactccatct ggggcatctg taccatctca aagctagatg ctcggatcca gcagaagaga    360
gaggaacagc gtcgaagaag ggcaagtagc ctcttggccc agaggagacc ccagagtgta    420
gagcggaagc aagagagtga accacgtatt gttagtagaa ttttccagtg ttgtgcttgg    480
aatggtggag tattctggtt cagtctcctc ttgttttatc gagtgtttat tcctgtactt    540
cagtcagtaa cagcccggat tattggagat ccatcacttc atggagatgt ttggtcatgg    600
ctggaattct tcctcacatc aattttcagt gctctttggg tgctccccct gtttgtgctt    660
agcaaagttg tgaatgccat ttggttccaa gatatagctg acttggcatt tgaagtatca    720
gggaggaaac ctcatccatt ccccagtgtc agcaaaataa ttgctgacat gctcttcaac    780
cttttgctac aggcactttt ccttattcag ggatgtttg tgagtctctt ccccatccat    840
cttgtgggtc agctggttag tctgctgcat atgtctcttc tctattcact gtactgcttt    900
gagtaccgtt ggttcaacaa aggaattgaa atgcaccagc gattgtcgaa catagaaagg    960
```

```
aattggcctt actactttgg gtttggcttg cccttggctt tcctcacagc aatgcaatcc   1020
tcctacatta tcaggtaatt tgggtagaaa aagaattcgt aagcatcttt tgttcagcta   1080
ctgtaccaac attttacatc atttgaccat caggaagatt tgttgctgtt aatagtgaca   1140
tttggtatca ttaaaatgaa aatataaaaa gaaatctagg ctgcctcctg gaggaagata   1200
cttaggagtt cagaagtgaa gagatgaggc ttataatact ttttcctaaa gtcatgcctt   1260
gtataattga ccttttttttc ccttaaacag tggctgcctc ttttctatcc tgtttccttt   1320
attcatcatc agcgccaatg aagcaaagac tcctggaaaa gcatatcttt tccagttgcg   1380
cctattctcc ttggtggtct ttttaagcaa cagacttttc cacaagaccg tctacctgca   1440
gtcagccctg agcagctcgt cctctgcaga gaaattccct tcgccacatc cttctcctgc   1500
caaactgaaa gctgctgcag gccactgagc cctgctgtca aaggggtggg tgggactggg   1560
tggaggatgt ggcagctctt ttctctgttt tcctcccccct gccgtggaag gcagaaccca   1620
ctgccaaggg ccctctgcat agtcccttgt ctttgaattg gaatcttcct gactccagta   1680
tatggatttt taccaccacc ctaggtctgt aaggaccaga ttttccagct gttttttttag   1740
cacttgccag ctcctgtgcc tggactgatt tgagtacttc cccctctcct tgtgtcattt   1800
accctcccac ctcctcctgc cttccagcac ccctgggtga atgggctttg taattttaac   1860
tgttgtattt tgtgaatttg ttgttactgt ttttctgtga agcacatacc ttgtatgtgg   1920
gaggtaaagg agcacgccag ctgctccatg tcactccctc tatagccatc actgtcttgt   1980
tttttgtaa ctcaggttag gttttggtct ttactgctct gctgcagaaa aggaaaggaa   2040
tgggggaaag gagcctaaga gatgggacag atagacctca gccaaactgg ttgggttttg   2100
aggagtcatg ttctttgttc cccttgaagg ggaaacaggt tttttcactg gtacatttaa   2160
agttccccag ctatgggaag gtaccagttc tggacaagtg ccactgcatc atagcaactc   2220
tgaagatgaa acttactgtt ggccactgcc aggtcagact cgtgttttaa ggaatactgg   2280
gtgcttcata taggaactga aggggtaaac ttactaaacc attcaacctg tgattggtga   2340
tgtttttctg tcattttaag agtcgacaca tggggtggga gggcagatgt aaaaaaactt   2400
gtacaatttt aaaatcacaa ttaaacgtga gctggtttcc cataaaa                2447
```

<210> 7
<211> 256
<212> DNA
<213> Artificial

<220>
<223> Synthetic oligonucleotide

<400> 7

gtaatttggg tagaaaaaga attcgtaagc atcttttgtt cagctactgt accaacattt 60

tacatcattt gaccatcagg aagatttgtt gctgttaata gtgacatttg gtatcattaa 120

aatgaaaata taaaaagaaa tctaggctgc ctcctggagg aagatactta ggagttcaga 180

agtgaagaga tgaggcttat aatacttttt cctaaagtca tgccttgtat aattgacctt 240

tttttccctt aaacag 256

<210> 8
<211> 137
<212> DNA
<213> Artificial

<220>
<223> Synthetic oligonucleotide

<400> 8

agcgaccggc agggtgcccg cccgacttcc cagctgcccc gtgcggcccg ggcatgccca 60

gtgtgggcgc agccccggct ctccgagcgc cggggcggag cgggcagccg ggcgcggagc 120

aggcgaaccg aacctcg 137

<210> 9
<211> 2493
<212> DNA
<213> Artificial

<400> 9

```
ataaaatcaa tcccactttc ttgttaaaag gagaaacgat ctgaattttg aaagactaga     60
agcccaactt aaggcctgcc actgcaggat catattttct aaggcaaagc cttctgtgct    120
ggcgctatct atccttgcgt tggagatcca agcactgaag tatgtagagt taacagaagg    180
agtagaatgt attcagaaac attccaagat aagtggccga gatttgacct tctggcaaga    240
gcttgtttcc aagtgtttaa ctgaatattc atcaaacaag tgttccaaac ctaacggtca    300
gaagttgaaa tggattgtgt ctggacgcac tgcacggcaa ctgaagcaca gttattacag    360
aataactcac ctcccaacga ttcctgaaac catttgttag ttgataaatc tggttgttat    420
tctctgtata cagaaaattt tccagtatga tcattttcgt gctacaactg aagaattgaa    480
atactatctt caatataaag aatatgggat gaaaacataa aggaaaagtg aattgttgac    540
tggtctagat agagaatact ggaaggcatt cactgtgtac agtgcgtagc agtctgaaga    600
gaaatgagta tcaaacctct agacacatgc tcatactgct gtcaaaggac tagcgtagaa    660
aagagagtcc tccaaaccgg aagtttaaat gtagttacta aaatagcact tctttaactt    720
acatatcccc ccactgtggc ttatttaaag ttacagaagt ccaagcagaa cgacaaaaga    780
tgtgacccat atatgaacac attttaatct gttcattgat taggagagtg aatatgaact    840
tgcatgatgc ccatgttagg tttctggaaa ctgccggggt atcttaattc tctagtattc    900
tccctctgtg gcagttgggc taatacaaag taactatacg catgagaata taaaatcagt    960
ctctgataca tacacatttt taccatcaaa atttcttaat catagcaaag acttaccttt   1020
ttatgattag gaattttttt tttaatgtat ggcagcacat gcctttaatc ccaacactag   1080
ggaggcagag gcaggtggat ctctttgagt tcgaagccag gctggtcttt acagtgagtt   1140
ccaggacagc tggagagcta cagaatggag agacgctgtc tcaaaaacac tcaaaacaaa   1200
```

caaacaaacc ataccagttt gtaggcagac ttctgttggg ttgggtttgt actgtttgcc 1260
tatgtagcag gattacagca gcagcaacaa aaacggtccc tcaagtcttt ctctgccact 1320
ctgacctgag tttcctacgg tacaggattt actctgagaa acctcagcac cttgccacag 1380
tagccgttgg cagaatggcc tcacggttag ggaaactcct gattctaagc ttgggagagc 1440
tacgcttaga tttgaattca cccaggaagc attcaaatca aggctaaaga cataaatgtg 1500
aaataaaact gtgaaccttc attctaaagt tcatctgact tcccagattt gatcaatata 1560
ttcttaggtg gtattaaaaa tggtaaactg cttaatttaa atctcaaaat ttaaattatg 1620
aggtttacat aaaaaccaac atttcatgaa tgcactttta aggtattaaa aggggtactt 1680
aagcggtaaa tggtttcttg gcacccataa ccaagtaata gttaatttac aggtgggatt 1740
tttttattgc tatgagaatt acatttaaaa ttgtgggtgt tttatataaa gcagatatca 1800
caagttttga aaattgttac ctttactgaa atttgttacc tttaatattt cttctagagg 1860
ataggtattt ataaaagaaa aatttgtcag aactgctgct tcaatctaat cccatttgag 1920
agaatttgtc tttactgtct taataactgg atgaattatc actctgaaaa tgtatttatt 1980
gcactaaagt tagtttaggc ttgataaaac actccaggac atttttacta cagactgttt 2040
ctattaaaac tgctgcttct aatggagaat tttattttaa aagaaaccga gtttatctgc 2100
aaaacctaag tcttatggga cgtaaggaga cacctccata attataagag caccatggta 2160
gtggggagtc tccatgctgg ccttgaatgt atgatccaca ctgttaaaac ataggcagca 2220
gctcagggcc ctggacagcc tgagtcaacc tagagtagct ggaaccattt tgacatgtaa 2280
tggataagaa aattatccat tgagaagcta aacaaaacaa aacaaaacca aagaacgggt 2340
gtattttatc cttaacctct gtaaaccacg tacactgaga acacttcagt tcttcctaaa 2400
ggttgatagg cttcagtctg aaaacaatat tgagtggata accaactacc atcatgcttt 2460
gggtacacct ttcaataaaa ttactgaaat gca 2493

<210> 10
<211> 30
<212> DNA
<213> Artificial

<220>
<223> Synthetic oligonucleotide

<400> 10
ataaaatcaa tcccactttc ttgttaaaag 30

<210> 11
<211> 109
<212> DNA
<213> Artificial

<220>
<223> Synthetic oligonucleotide

<400> 11

```
ataaaatcaa tcccactttc ttgttaaaag gagaaacgat ctgaattttg aaagactaga     60
agcccaactt aaggcctgcc actgcaggat catattttct aaggcaaag                 109
```

<210> 12
<211> 1107
<212> DNA
<213> Artificial

<400> 12

```
agaggccatc catcaaaaga atcacaccaa tcagcgacgt agatgcgtcc cagctagaga     60
gccaggtctc cagcttgttc ccccactgcc cggcgcagtc cttcgctgcg cgctgacccc    120
gaggaaggca gtacttggtc agggcgctga gggaccctcc accgggacgc cggcccctcc    180
ccgggcctct gctcacttga ccccctccc tagtccgtcc ccttagtagg cctgtcggat     240
cggggacgtg gggcgagctg agagcaggcc cggggtgggt ggtcaccgtg gtgaagacgt    300
ggctgtcaag atgatagaag tactgacaac tgactctcag aaactgctac accagctgaa    360
caccctgttg gaacaggagt ctagatgtca gccaaaggtc tgcggcttga aactaattga    420
gtcggcccat gataatggcc tcagaatgac tgcaagatta cgggactttg aagtaaaaga    480
tctacttagt ctaactcagt tctttggctt tgacacggag acattttccc tagctgtgaa    540
tttactggac agattcttgt ctaaaatgaa ggtacaggcg aagcatcttg ggtgtgttgg    600
actgagctgc ttttatttgg ctgtgaaagc gactgaagag gaaaggaatg tcccactggc    660
gactgatttg atccgaataa gtcagtatag gttcacggtt tcagacctga tgagaatgga    720
gaagattgtg ttggagaaag tgtgttggaa agtcaaagct actactgcct ttcaatttct    780
gcagctctat tattcactcg ttcacgacac cttgccattt gaaaggagaa acgatctgaa    840
ttttgaaaga ctagaagccc aacttaaggc ctgccactgc aggatcatat tttctaaggc    900
aaagccttct gtgctggcgc tatctatcct tgcgttggag atccaagcac tgaagtatgt    960
agagttaaca gaaggagtag aatgtattca gaaacattcc aaggtatgcc aaggtgatag   1020
cattgatcct attagcaagc tacaagagaa atgtatccac tgtactcctg aagtaaaata   1080
aaatgctgtg tattgatctg aagctta                                       1107
```

<210> 13
<211> 104
<212> DNA
<213> Artificial

<220>
<223> Synthetic oligonucleotide

<400> 13

gtatgccaag gtgatagcat tgatcctatt agcaagctac aagagaaatg tatccactgt 60

actcctgaag taaaataaaa tgctgtgtat tgatctgaag ctta 104

<210> 14
<211> 203
<212> DNA
<213> Artificial

<220>
<223> Synthetic oligonucleotide

<400> 14

gtatgccaag gtgatagcat tgatcctatt agcaagctac aagagaaatg tatccactgt 60

actcctgaag taaaataaaa tgctgtgtat tgatctgaag cttaccttct gtgctggcgc 120

tatctatcct tgcgttggag atccaagcac tgaagtatgt agagttaaca gaaggagtag 180

aatgtattca gaaacattcc aag 203

<210> 15
<211> 1245
<212> DNA
<213> Artificial

<400> 15

atcccgctgc ggggacagga gacggaaaag caggctggaa gggtagacac aattctcgcg 60
agaagtagag cgaattccct cggagagggg cggttgcgca gaggtacacc acgtgtgcgg 120
tccctccggc gccgagtcag ggtctctcgt cgagtctggg tctaggttag atttggaatc 180
gtggagatgc ggaaggacac ccctcctccg ctcgtgcccc cggcggcccg cgagtggaac 240
ctgcccccca atgcgcccgc atgcatggaa cgtcaattgg aggctgcacg gtaccggtct 300
gatggttccc ttctgctcgg ggtctccagc ctgagtggtc gctgctgggt aggttctctg 360
tggtttttca aggatcctag tgcggccccc aacgaaggtt tctgctctgc tggcgtccag 420
accgaggctg gagtagctga cctcacttgg gtgggggaca aaggtatcct agtggcttct 480
gattcaggtg ctgttgaatt gtgggagcta gatgagaacg agacacttat agtcagcaag 540
ttctgcaagt atgagcatga tgacattgtg tctactgtca ctgtcctgag ctctggcaca 600
caagctgtca gtggtagcaa agactgctgc atcaaaattt gggacctggc tcagcaggta 660
tcactgaatt cataccgagc tcacgctgga caggttacct gtgttgctgc ctctccccac 720
aaagactctg tgtttctttc atgtagtgag gacagtagaa ttttgctctg ggatacccgc 780
tgtcccaagc cggcatcaca gatggcctgc aatgcctctg gctacctccc taccgctttg 840
gcttggcatc ctcagcagag tgaagtcttt gtttttggta aggcagcatg acgtatcgac 900
tctgggaatg gggcggatac aaagtgctac cttcttggag tacatgctcc tgtcagcaca 960
cttgggcact gcatggatta tgtcggtttt acctagcttt ggagaccatc atttttttcct 1020
aataggtttg cttcattgtt tcagctcctt tattctgggc atggttagaa atcagtgagc 1080
tgcatagcaa gtgcttgctg ttagggaact atgttctgct gtggtgtgac tacacatggc 1140
aagagtttgt agttaattaa ttcacagcca catagtgact gactgattct atatcacaaa 1200
ggaaactgtt gtctacatct tgagaaacta ggattcattc aaatc 1245

<210> 16
<211> 224
<212> DNA
<213> Artificial

<220>
<223> Synthetic oligonucleotide

<400> 16

cttggagtac atgctcctgt cagcacactt gggcactgca tggattatgt cggttttacc 60
tagctttgga gaccatcatt ttttcctaat aggtttgctt cattgtttca gctcctttat 120
tctgggcatg gttagaaatc agtgagctgc atagcaagtg cttgctgtta gggaactatg 180
ttctgctgtg gtgtgactac acatggcaag agtttgtagt taat 224

<210> 17
<211> 43
<212> DNA
<213> Artificial

<220>
<223> Synthetic oligonucleotide

<400> 17
gtaaggcagc atgacgtatc gactctggga atggggcgga tac 43

<210> 18
<211> 17
<212> DNA
<213> Artificial

<220>
<223> Synthetic oligonucleotide

<400> 18
gtaataactt tatccag 17

<210> 19
<211> 88
<212> DNA
<213> Artificial

<220>
<223> Synthetic oligonucleotide

<400> 19

taattcacag ccacatagtg actgactgat tctatatcac aaaggaaact gttgtctaca 60

tcttgagaaa ctaggattca ttcaaatc 88

<210> 20
<211> 2535
<212> DNA
<213> Artificial

<400> 20

atcccgctgc ggggacagga gacggaaaag caggctggaa gggtagacac aattctcgcg 60

agaagtagag cgaattccct cggagagggg cggttgcgca gaggtacacc acgtgtgcgg 120
tccctccggc gccgagtcag ggtctctcgt cgagtctggg tctaggttag atttggaatc 180
gtggagatgc ggaaggacac ccctcctccg ctcgtgcccc cggcggcccg cgagtggaac 240
ctgcccccca atgcgcccgc atgcatggaa cgtcaattgg aggctgcacg gtaccggtct 300
gatggttccc ttctgctcgg ggtctccagc ctgagtggtc gctgctgggt aggttctctg 360
tggtttttca aggatcctag tgcggccccc aacgaaggtt tctgctctgc tggcgtccag 420
accgaggctg gagtagctga cctcacttgg gtgggggaca aaggtatcct agtggcttct 480
gattcaggtg ctgttgaatt gtgggagcta gatgagaacg agacacttat agtcagcaag 540
ttctgcaagt atgagcatga tgacattgtg tctactgtca ctgtcctgag ctctggcaca 600
caagctgtca gtggtagcaa agactgctgc atcaaaattt gggacctggc tcagcaggta 660
tcactgaatt cataccgagc tcacgctgga caggttacct gtgttgctgc ctctccccac 720
aaagactctg tgtttctttc atgtagtgag gacagtagaa ttttgctctg ggatacccgc 780
tgtcccaagc cggcatcaca gatggcctgc aatgcctctg gctacctccc taccgctttg 840
gcttggcatc ctcagcagag tgaagtcttt gtttttggtg acgagaatgg atctgtctcc 900
cttgtggaca ccaagaatgc aagctgtacc ctcagctcag ctgtgcactc ccagggtgtc 960
actagactgg tattctcccc acacagtgtc cccctcctga cttctctcag tgaagactgt 1020
tcacttgctg tgctggattc aagcctttct gaggtgttta gaagtcgagc ccacagagac 1080
tttgtgagag atgctacgtg gtctccactc aatcactccc ttcttaccac agttggctgg 1140
gaccatcagg tcatccacca tgttgtgccc ttagagcctc tcccaaaccc tggacctgac 1200
agtgttgtgg agtagaatgg atttcagaaa aaccaaaaca agccctccgt ctgtaagcga 1260
ctactcgatt gcccctgcct tccatgtgtg agagcacagg agccttgtag agcatgtttc 1320
ctccctagcc ccgtgcagta acaggcagat tctcagcctg agggaggctg catcccatag 1380
tgactcagag gaagaaactt cctctgtaaa tggatgtatg tgagtacacg tgtgagtgcg 1440
gtatatagtt tggagtggag aaaattattc ttcagctttc ccaaagcaat gctctttacc 1500
cctgacaaag tgaccagagg attttaatgc ttcccatgtc tgggaattgg cgatgttgtt 1560
agggatatgg aatgtgggta tccctagatt tttgggaata cttcatacta ctcagaggtg 1620
cgtaacttat ttttatatag agtttaattc actattatgg gaattacttc catatacaaa 1680
agtctagccc actgtacctt gagaagacaa aacagttttg tacaagtccc tgttatactg 1740
agtcaaatcc attttctggg tgaataaatt tggcccttgc agtgtagctc ttgttcaccc 1800
ttttccttcc ttggtgttgt gtgtgagaga tgagtgtttt cctgtcctag atttcacaga 1860
taagttgtag ggatgatgga ccacttaaat taaaagtgtt tcagtgaagt gggaaaattg 1920
acatggattg ttacagagaa atctgtcatt tcatttttcc cacctaaaaa ttgattctgg 1980
gtaccttcct ctggagagct gaagtctgaa tgtatttttg gactcctaag atttgagatc 2040
cagcatcagg gaaactgtcg gaaatactaa aaggtgaaat aaagactttt atcctttcat 2100

ctttcctccc cagagctgtt acttattacc tgcttcgctt aggtgaatca ccagtgtcag 2160
acttagctgc ccactgtctt tgtaatctac tgtattccac tccattagag actgtggatc 2220
tgtaactgat cttattggag ctttccaact tgggtttagg aaaagcacat aaaatgggcg 2280
ccctgaagga atgtgatcgt cccttctagt cttgccagtc atgagcctct gttgtaatta 2340
ccctcgagca aaacgcttga gagtttgcag tcaaggtcac ctggggttgg gactgtggaa 2400
gcaaggactt gcattgttac taaattcagg gaaggcataa gataggtagg atcagtttct 2460
gcttcttaga gataaagctt gtgctttgtg tcacttaggt aaggacaggt agtgcattta 2520
catggcctgc tttgg 2535

<210> 21
<211> 171
<212> DNA
<213> Artificial

<220>
<223> Synthetic oligonucleotide

<400> 21

gcactccgtt agcaattatg ggtcagtaac tgatcttatt agagctttac aactttggtt 60
taggaaaagc acataaaatg ggcgccctga aggaccatga gcttccattc tactcttgcc 120
agtcaagagc ctctcgtgat cacccttgag taaagcactt gaatttgcag c 171

<210> 22
<211> 13
<212> DNA
<213> Artificial

<220>
<223> Synthetic oligonucleotide

<400> 22
ctgtgtcagt cag 13

<210> 23
<211> 134
<212> DNA
<213> Artificial

<220>
<223> Synthetic oligonucleotide

<400> 23

gacttggaat tttactaagt tccagggaag gcgtaagagg gaggtagaat cagtctctgc 60
ttctcaagag ataaagcttg tattttgtgt tccttaggga aggacaaatt atccgtttac 120
acggcctgct ttgg 134

<210> 24
<211> 1214
<212> DNA
<213> Artificial

<400> 24

```
tttcccgggg cctgggcgga gagggaggaa aacttcctgg ctgggactcc ggcggtttct      60
gcctgccaac cctcggatcc cgcctaccag tgttggctct tcccgacccc tcccccggcg     120
cccccagtgt ccgccatggc caaagcctac gaccacctct tcaagttgct gctcatcggg     180
gactcggggg tgggcaagac ttgtctgatc attcgctttg cagaggacaa cttcaacagc     240
acttacatct ctaccatcgg aattgatttc aagatccgaa ccgtggacat agaggggaag     300
aggatcaaac tgcaagtgtg ggacacggct ggccaagaac gattcaagac aataactacc     360
gcctattacc gtggagccat gggcattatc ctcgtatatg acatcacaga tgagaaatcc     420
ttcgagaata ttcagaactg gatgaaaagc atcaaagaga atgcctctgc gggagtggag     480
cgcctcctgc tgggaaacaa gtgtgacatg gaggccaagc ggcaggtgca gagagagcag     540
gcggagaagg tgagtcaggc cgggctgctg tggccgaatg ccttgcttct gccctttatc     600
cagctctctc cttcctacct catcccctac agttggctcg agagcacaga atccgatttt     660
ttgagacgag tgccaaatcc agtgtgaatg tggatgaggc tttcagttcc ctggcccgtg     720
acatcttgct caagacagga ggccggagat cgggaaacag cagtaagccc tcaagcactg     780
gcctgaaaac atctgacaag aagaacagca acaagtgctt gttaggctga ggagcatttc     840
ttgcctccta ttcaccctga acctggaggc tagacctgag ggaggtggac tgagggatac     900
tgatggaaaa ctgtggtgag cacctcaggt ggagcctgga ggggaataag gatgacaggc     960
gaggacgaga aagaagaaag gggcagggaa aggaggggga ggaaccaagg atgtgaaagg    1020
tgaacagaag ggatttgaga agagaaagga agaagaagtg tctcaggtct tggacagtcc    1080
aacattaaag tcaacatgct gatctctcca ttcctggttc agggttaggg tcctgagagg    1140
ctggctcggc actactccga gggtccctca ctctacaggt ctttgttagt attaaaggcc    1200
actgtttggc acga                                                      1214
```

<210> 25
<211> 83
<212> DNA
<213> Artificial

<220>
<223> Synthetic oligonucleotide

<400> 25

```
gtgagtcagg ccgggctgct gtggccgaat gccttgcttc tgccctttat ccagctctct      60
ccttcctacc tcatcccta cag                                               83
```

<210> 26

<211> 149
<212> DNA
<213> Artificial

<220>
<223> Synthetic oligonucleotide

<400> 26

```
gtgagtcagg ccgggctgct gtggccgaat gccttgcttc tgccctttat ccagctctct      60
ccttcctacc tcatccccta cagttggctc gagagcacag aatccgattt tttgagacga     120
gtgccaaatc cagtgtgaat gtggatgag                                       149
```

<210> 27
<211> 1411
<212> DNA
<213> Artificial

<400> 27

```
gtgacgcgcc tgtgttgttt cgccattttg ggaaggtcct acaggccgaa tgcaatccgg      60
cgcccgttga ctgccatttg tagaaggtcc gtaaaggctc ctcgcgtcgc gggcgcgtag     120
ccatggaatt gatttcaaga tccgaaccgt ggacatagag gggaagagga tcaaactgca     180
agtgtgggac acggctggcc aagaacgatt caagacaata actaccgcct attaccgtgg     240
agccatgggc attatcctcg tatatgacat cacagatgag aaatccttcg agaatattca     300
gaactggatg aaaagcatca aagagaatgc ctctgcggga gtggagcgcc tcctgctggg     360
aaacaagtgt gacatggagg ccaagcggca ggtgcagaga gagcaggcgg agaagttggc     420
tcgagagcac agaatccgat tttttgagac gagtgccaaa tccagtgtga atgtggatga     480
ggctttcagt tccctggccc gtgacatctt gctcaagaca ggaggccgga gatcgggaaa     540
cagcagtaag ccctcaagca ctggcctgaa aacatctgac aagaagaaca gcaacaagtg     600
cttgttaggc tgaggagcat ttcttgcctc ctattcaccc tgaacctgga ggctagacct     660
gagggaggtg gactgaggga tactgatgga aaactgtggt gagcacctca ggtggagcct     720
ggaggggaat aaggatgaca ggcgaggacg agaaagaaga aaggggcagg gaaaggaggg     780
ggaggaacca aggatgtgaa aggtgaacag aagggatttg agaagagaaa ggaagaagaa     840
gtgtctcagg tcttggacag tccaacatta aagtcaacat gctgatctct ccattcctgg     900
ttcagggtta gggtcctgag aggctggctc ggcactactc cgagggtccc tcactctaca     960
ggtctttgtt agtattaaag gccactgttt ggcacgaatg tcccatttgc attactttca    1020
ttattgtcag aattgctctt cactcaaatc ctatttttgt cacgccaaga tattggttca    1080
cctgaatgtg gctgggttcc ccttccttgc cccaactctt tcactggtga tgaaaacagc    1140
atggggcagc ctgaaggacg gacatcctgt ttccactgtg ggttcccaag gactacaaga    1200
gtggacggaa ccttgccttg agcacacagt aacccaagga caaaggattt gaaccaggct    1260
tcagtaaaca gcagcactta gtatggttta tccaaggaga tgtgggacat ctttgattct    1320
gatgtagtca gcttaggtgt tgggtactgt tagctgcttt tgttagagta ttctcagtgt    1380
tgcacaaaga aatacatgaa caaggtgaga g                                   1411
```

<210> 28
<211> 875
<212> DNA
<213> Artificial

<220>
<223> Synthetic oligonucleotide

<400> 28

ggaaacagca gcaagccctc aagcactgac ctgaaagtat ctgacaagaa gaacagcaac 60
aagtgctcct tgggctgagg aacatttctt gcctcctatt caccctgaac ctggaggcta 120
gacctgaggg aggtggactg aggtagactg atggaaaaca gaggggagga gctgtggtgg 180
tgcctggagg ggtggatgac aggggaggaa ggaaagatga aatgggcagg gaaaggaggg 240
cgaggaacca aggacgtgaa aagtgaagag aaggggtttg agaagagaaa aagaagaagg 300
tctcaggtct cggacgtcca acattaatgt cagtatgctg atctctccat tcctggttca 360
gggttcgggt cccgagaggc tggctcggcc ctactctgag ggtctctcac tccacagatc 420
tttgttagta ttaaaggcca ctgtttggca cgaatgttcc atttgcatta ctttcattat 480
tgtcagaatt gctctttact caaatccaat ttctgtcatg ccaggatatt agttctcctg 540
aatctggctg ggtccccctt ccttacccca actctttaac tggtgatgaa aacagcaagg 600
agaaagggca gcctgaagaa tggacatcct ttttccacta tgggttcctg aggacaagag 660
tggccggaac catgccttta gcaatagtaa gccaggatga aggatttgaa ccaagcttca 720
ggaaacagca gcacttaata tggtttaccc aaggagatgg gaagcatctt tgattctgct 780
gtagtcaaat ggtgtttgat gggcactgtt agctgctttt gttagaaaag aacattctca 840
gtgttccacc aagaaacaca tgaacaaagt aagag 875

<210> 29
<211> 125
<212> DNA
<213> Artificial

<220>
<223> Synthetic oligonucleotide

<400> 29

gtgacgcgcc tgtgttgttt cgccattttg ggaaggtcct acaggccgaa tgcaatccgg 60
cgcccgttga ctgccatttt tagaaggtct gtaaaggctc ctcgtgtcgc ggttgcgtag 120
ccatg 125

<210> 30
<211> 447
<212> DNA
<213> Artificial

<400> 30

tgggggtggg gggatctacc ctctctagag ccctgtccct ctgaccagga ggtgttgtgc 60
cctgtggctg tggcttttcc ccacgatgag ccacatgtcc cttagactct ggggaatgat 120

gtccttcccc ttggcatctg gcctgacatc tgttctctct ccacagattt ctatcactcc 180
aagcgcagat tggtcttctg caagagaaaa ccctgaagtg cccacgggag ccccgccctc 240
ttctgctgtg ggtcaggagg cctcttcccc atcttcggcc ttagccttca ctctgtgtgt 300
cttaattatt atttgtgttt taatttaaac gtctcctgta tatacgctgc ctgcccccctc 360
ccagtctcca aacttaaagt tatttaaaaa acaaaacaaa acaatgaaat tagtaggacg 420
gtaggctcct tagtgctgtt ttttttt 447

<210> 31
<211> 159
<212> DNA
<213> Artificial

<220>
<223> Synthetic oligonucleotide

<400> 31

tgggggtggg ggaatctagc ctctctagag ccctagccct ctgacgagga ggaggtgtag 60
tgccctgtag cttttcccca ggactcgcca catgtccctt tgtctgggga atgatgccct 120
caccctctgc atctggcctg acatctgttc tctctgcag 159

<210> 32
<211> 897
<212> DNA
<213> Artificial

<400> 32

tcagtacata aaatgatata ggacattaaa acatttatgt atatcgtaca ttaaattatt 60
ttccccaagc atataagcat gtaatatata tctaatgatt taggacatac atttaaactc 120
aactataaat tcataacaac atgtctattc tcaaatacat taagataatg cttgttagac 180
atatctgtgt tattagacat gcaccattaa gtcataaacc tttctcttcc atatgactat 240
ccctgtcccc aattggtctc tatttctacc atcctccgtg aaatcaacaa cccgcccact 300
cgtccccctc ttctcgctcc gggcccattc gtcctggggg tgactatact gaaactttac 360
caggcatctg gttcttactt cagggccatc aattggttca tcgtccatac gttcccctta 420
aataagacat ctcgatggta acgggtctaa tcagcccatg atcaacataa ctgtggtgat 480
atacatttgg tatttttaa ttttcggatg ccttcctcaa catagccgtc aaggcatgaa 540
ggtcagcaca aagtcctgtg gaacctttta gttaagggtc atttatcctc atagacaaag 600
ctcgaaagac tattttattc atgtttgtaa gacataaata tttataaata ctgaaaaatc 660
tgtcaacaaa ccccccacc ccctacacct gaaacttcaa tgccaaaccc caaaaacatt 720
aaagcaagaa ttaaataaaa caaaaagcta cttaattctt aaaaggcttc tccattctag 780
tagaccacaa aattttaact taaatcttag cattggtaaa atttcccgac acaaaatctt 840
tccttctaac taaaccctct ttacttgcct accctcagaa aattccacat acaccaa 897

<210> 33
<211> 897
<212> DNA
<213> Artificial

<220>
<223> Synthetic oligonucleotide

<400> 33

```
tcagtacata aaatgatata ggacattaaa acatttatgt atatcgtaca ttaaattatt      60
ttccccaagc atataagcat gtaatatata tctaatgatt taggacatac atttaaactc     120
aactataaat tcataacaac atgtctattc tcaaatacat taagataatg cttgttagac     180
atatctgtgt tattagacat gcaccattaa gtcataaacc tttctcttcc atatgactat     240
ccctgtcccc aattggtctc tatttctacc atcctccgtg aaatcaacaa cccgcccact     300
cgtccccctc ttctcgctcc gggcccattc gtcctggggg tgactatact gaaactttac     360
caggcatctg gttcttactt cagggccatc aattggttca tcgtccatac gttcccctta     420
aataagacat ctcgatggta acgggtctaa tcagcccatg atcaacataa ctgtggtgat     480
atacatttgg tatttttaa ttttcggatg ccttcctcaa catagccgtc aaggcatgaa      540
ggtcagcaca aagtcctgtg gaacctttta gttaagggtc atttatcctc atagacaaag     600
ctcgaaagac tattttattc atgtttgtaa gacataaata tttataaata ctgaaaaatc     660
tgtcaacaaa cccccccacc ccctacacct gaaacttcaa tgccaaaccc caaaaacatt     720
aaagcaagaa ttaaataaaa caaaaagcta cttaattctt aaaaggcttc tccattctag     780
tagaccacaa aattttaact taaatcttag cattggtaaa atttcccgac acaaaatctt     840
tccttctaac taaaccctct ttacttgcct accctcagaa aattccacat acaccaa        897
```

<210> 34
<211> 481
<212> DNA
<213> Artificial

<400> 34

```
tcatctatct tatataagat aatgtctgat ctaaatatga gaccctgtga ggttggccat      60
aagcacactg ttagctcctt ccgtcagacc tttttggggc tggctggtga aacagaatga     120
atcctgttgg gtctgcttct cacagcgtat ggggctgtgt gcttttactc atggtggcag     180
cagcagttgt tgtcacttct ttggccaggc acagtgggtg atccttacag agcagcacag     240
attcccattg attccagtct atgtatttac ttgtggttgg tacagaattt aatgcagata     300
gagtctttaa atatgtgaag atatgcagca ctgattttaa acttaaactg gcatgatact     360
ttccatatgt ctagacacac tggtaaattt ttgttacata ttgttttcaa attttccgtt     420
agtcttgttt ctatgagata cttttattgt gacttaaaat tcttagaaat taaagttctg     480
a                                                                     481
```

<210> 35
<211> 481
<212> DNA
<213> Artificial

<220>
<223> Synthetic oligonucleotide

<400> 35

tcatctatct tatataagat aatgtctgat ctaaatatga gaccctgtga ggttggccat 60
aagcacactg ttagctcctt ccgtcagacc tttttggggc tggctggtga aacagaatga 120
atcctgttgg gtctgcttct cacagcgtat ggggctgtgt gcttttactc atggtggcag 180
cagcagttgt tgtcacttct ttggccaggc acagtgggtg atccttacag agcagcacag 240
attcccattg attccagtct atgtatttac ttgtggttgg tacagaattt aatgcagata 300
gagtctttaa atatgtgaag atatgcagca ctgattttaa acttaaactg gcatgatact 360
ttccatatgt ctagacacac tggtaaattt ttgttacata ttgttttcaa attttccgtt 420
agtcttgttt ctatgagata cttttattgt gacttaaaat tcttagaaat taaagttctg 480
a 481

<210> 36
<211> 1233
<212> DNA
<213> Artificial

<400> 36

aagttcagtg aaatcttgaa taccaaacat ctgacatacc tcacaaattc aggaaagcaa 60
ggatggacct tcacaagtga tgcaagttta atcggataag ttccaaacga gttttaggtc 120
tttagtattg taaataatca ttctgttttt ctacttacct gtttttatca agatacattt 180
ccatgtggag tgacttcaaa atgcttgatt tcatagagcc actaggtggc atctttgtaa 240
aagtggaagc acatgaaaag aaatagccta ggagtaaatg caagatagca aggtagatga 300
gtcgttttga acaagaccta agcaagtgca cagatttagg atgaaaagtt aaattaaaag 360
ctttaacact ttttattttg tgattaatac tatttttcta ttgctatttt cattcagggg 420
acattgacat ttatgtagcc aaacatgttt atgtatttgt gagagagaaa gtgtgtgtgt 480
gcgtgcgcgc gtgttcgtgc acttgctttt gctatgttag taacctatgc agatagacca 540
cctaagtaca ttctgtgtaa ggctttttaa aattgagcat agaaagcaga gttcatagta 600
cattatcaat aagttattaa gatatcacgc tagacttaac agtggaaaga acatgaattt 660
ttctgctgcc tctattccta accctggtgt tttcattctg tccctaacag gaggctaatg 720
aatgataaag ctttgaaggt gaaggggtgg gaaagtgatg ttagtgaaga gatgagagtg 780
gctttgtagc aacagggtat aaaaaagaca acgacgatta taatgaggga gatggatttt 840
acaacacgtt tttaatttca ctaaacttta catgccaaag gatgttaaat atagcacgta 900
ataagtgttt aaaatctggg ctcttgattc agaccgagtt ttaatactgg atctcattca 960
atctttgtgt tttttctttt tcttttcttt tttttccaat agtacggcag tgctaatagc 1020

agtcctttag tagagttact gcattgacta ttggaaatac agataagttg ctgtgcttag 1080

tgataaaact tactaaacat ttgctgtttt ttgtgagata accacatgaa atttgttcag 1140

ggaacatatt tgatctgttc tccaaaatgt cttaagacta aagaatttga tgaaagaaaa 1200

ataaaagctt tgcttctcct gacttagaaa gca 1233

<210> 37
<211> 1437
<212> DNA
<213> Artificial

<220>
<223> Synthetic oligonucleotide

<400> 37

aagttcagtg aaatcttgaa taccaaacat ctgacatacc tcacaaattc aggaaagcaa 60
ggatggacct tcacaagtga tgcaagttta atcggataag ttccaaacga gttttaggtc 120
tttagtattg taaataatca ttctgttttt ctacttacct gtttttatca agatacattt 180
ccatgtggag tgacttcaaa atgcttgatt tcatagagcc actaggtggc atctttgtaa 240
aagtggaagc acatgaaaag aaatagccta ggagtaaatg caagatagca aggtagatga 300
gtcgttttga acaagaccta agcaagtgca cagatttagg atgaaaagtt aaattaaaag 360
ctttaacact ttttattttg tgattaatac tatttttcta ttgctatttt cattcagggg 420
acattgacat ttatgtagcc aaacatgttt atgtatttgt gagagagaaa gtgtgtgtgt 480
gcgtgcgcgc gtgttcgtgc acttgctttt gctatgttag taacctatgc agatagacca 540
cctaagtaca ttctgtgtaa ggctttttaa aattgagcat agaaagcaga gttcatagta 600
cattatcaat aagttattaa gatatcacgc tagacttaac agtggaaaga acatgaattt 660
ttctgctgcc tctattccta accctggtgt tttcattctg tccctaacag gaggctaatg 720
aatgataaag ctttgaaggt gaaggggtgg gaaagtgatg ttagtgaaga gatgagagtg 780
gctttgtagc aacagggtat aaaaaagaca acgacgatta taatgaggga gatggatttt 840
acaacacgtt tttaatttca ctaaacttta catgccaaag gatgttaaat atagcacgta 900
ataagtgttt aaaatctggg ctcttgattc agaccgagtt ttaatactgg atctcattca 960
atctttgtgt tttttctttt tcttttcttt tttttccaat agtacggcag tgctaatagc 1020
agtcctttag tagagttact gcattgacta ttggaaatac agataagttg ctgtgcttag 1080
tgataaaact tactaaacat ttgctgtttt ttgtgagata accacatgaa atttgttcag 1140
ggaacatatt tgatctgttc tccaaaatgt cttaagacta aagaatttga tgaaagaaaa 1200
ataaaagctt tgcttctcct gacttagaaa gcagggtatt tgagagttct tcctgttctt 1260
tctgggtgcc aggaagctaa agcacaaaag caagtagata tacaacagtt gtaaacttgt 1320
gttcttttat aatccctgtc tctcggtgtt catatttgca tttttaaaga tgataacctg 1380
tgagcttaga ctgcttacag gactgccttt ccaaaagtgt gagagagctc ccagaga 1437

<210> 38
<211> 627
<212> DNA
<213> Artificial

<400> 38

gttataataa gtatcctggg tatctaatcc attagtgaca tttcataggc aaccgcatgg 60
acccacatgg gagtgtgaat agaacaggat cccagaggaa cagttccagt aacagagccc 120
acatctgttg ctagctcctt agtttagtat ggacggatgg taaagaccaa agtctatagc 180
acggaaaaga ttaataaatg agtaataaat gggtgttaat aaatgataat ttttgtggta 240
gttatgaagg atagttttag ctaatttaaa gagagtgtta gcttgtgttg gggagaggcc 300
acagtagagg ctgcacttgc cactgctgtg tagcggcact ctcctgactc acttaaaaat 360
tctctggagg tttaagtgag gacccagttc catctctaga tatccaggct ccctagaaca 420
tttccagttg attccaacat gcatttggtg ttaaagacca ctgcagtgtc atttgcacat 480
agacccaaga catggagtta ggttttgttt tttggggttt ttttttgcaa ttctaatgta 540
taatatcaat caatattctt ttgtgtgata ccacatttgg actattgtcc cttcagctga 600
taactgacat taaagaaata cagttga 627

<210> 39
<211> 627
<212> DNA
<213> Artificial

<220>
<223> Synthetic oligonucleotide

<400> 39

gttataataa gtatcctggg tatctaatcc attagtgaca tttcataggc aaccgcatgg 60
acccacatgg gagtgtgaat agaacaggat cccagaggaa cagttccagt aacagagccc 120
acatctgttg ctagctcctt agtttagtat ggacggatgg taaagaccaa agtctatagc 180
acggaaaaga ttaataaatg agtaataaat gggtgttaat aaatgataat ttttgtggta 240
gttatgaagg atagttttag ctaatttaaa gagagtgtta gcttgtgttg gggagaggcc 300
acagtagagg ctgcacttgc cactgctgtg tagcggcact ctcctgactc acttaaaaat 360
tctctggagg tttaagtgag gacccagttc catctctaga tatccaggct ccctagaaca 420
tttccagttg attccaacat gcatttggtg ttaaagacca ctgcagtgtc atttgcacat 480
agacccaaga catggagtta ggttttgttt tttggggttt ttttttgcaa ttctaatgta 540
taatatcaat caatattctt ttgtgtgata ccacatttgg actattgtcc cttcagctga 600
taactgacat taaagaaata cagttga 627

<210> 40
<211> 1654
<212> DNA
<213> Artificial

<400> 40

gcctacattg ctgcggccac cagacggacc aggggccacc agtgccagcc ctgtggcctg 60
tctactggac tctggtcacc aggctcagct tgttgagttc ctcattgtgc actatgagca 120
gatcttcggg atggatgagc tccctctgac ctctgagccc ctgactcaag accctgcctt 180
ggctcctgca ctcctcgaat ccagtcccca gcacccagcc ttactcgttg cccaagactc 240
acagcccctg accatagcct cagattccag cccagacccc aaacaacaca gtgccttgga 300
gaagtgtccc aaggtcgcgc ctcctgagct tacggctctg cagagtaacc gaaaggagga 360
ggaggaggaa gacaccagag atggggcggg ggatgggtcc agccactccc ccgaggactt 420
gctcctggta gcacaatccc ggggccactt cagccgccag ccagtgaagt attcccgggg 480
aggtgtgcga ccagttactc atcagctctc cagcctggct ctggtggcct ccaagctgtg 540
tgaggagact cctatcactg tctcagcagt gcaccgaggt agtttgaggg cacgaggcct 600
gggccctgct gctgcctccc ctgaaggcgg caccctgcgc cggaaccctc tgcccaagca 660
ctttgagatc acccaggaga cagcccggct actctccaag ctggacagtg aggctgtgtc 720
cagaacctcc tgttgtgctg acccagagcc ggaggagtct gaggagcacc tctgaccacc 780
caccatcctg agggacctag gactgaattg atggtcctat atgtctccct acctgaccca 840
acctggtgac caaaccaatt ccatggggtg tggggagaga tggggttacc gagagttcag 900
gtaatgaaga ttacatgcct atgggagtcc tgaaacactt ggttgaagtg tccctccctg 960
ccagaggaga tttaaacaag caaggcaaca caggcaattg ggtcaagac tcaatggtca 1020
attcaggtca gtagatcctt caggtctacc tcagtctctg acctcgagac aggcgtctga 1080
ctgtttcagt cataatggct tctggtcctg ctaccacttg cctccttggc agactccaat 1140
tcagccctgt cacctttatt tactccttcc ttttgtttgt ttgtttgttt gtttgttttg 1200
agccaaggtc tttctgtagc tctggctggt ctggagctcc ctttgcaaac cacaaatcct 1260
ctgtctccca agtgctggcc aaatctgtgt tcttacaacc tccaaataaa accctcttcc 1320
tgcagccggc aggacggccg agcacaatgg tgcttgctgt acagcctcat agaccttcct 1380
tggatctccc agacacactt agtggaagga gagaatccat tcttgcaaat tgtctgacct 1440
atgcacatgc acacaaaaaa ataaattttt aattaaaaaa atctgctggg tagtggtgcc 1500
acacaccttt atttaaacct aacactcagg aggtagaggc aggaggatct cttgagtttg 1560
attccagaca ggtctacaga ttaagttcca ggacagccag ggctacacag agacacccta 1620
tcttgggaag caaaataaag taagataaat cttg 1654

<210> 41
<211> 1259
<212> DNA
<213> Artificial

<220>
<223> Synthetic oligonucleotide

<400> 41

```
ggtccagcca ctcccccgag gacttgctcc tggtagcaca atcccggggc cacttcagcc      60
gccagccagt gaagtattcc cggggaggtg tgcgaccagt tactcatcag ctctccagcc     120
tggctctggt ggcctccaag ctgtgtgagg agactcctat cactgtctca gcagtgcacc     180
gaggtagttt gagggcacga ggcctgggcc ctgctgctgc ctcccctgaa ggcggcaccc     240
tgcgccggaa ccctctgccc aagcactttg agatcaccca ggagacagcc cggctactct     300
ccaagctgga cagtgaggct gtgtccagaa cctcctgttg tgctgaccca gagccggagg     360
agtctgagga gcacctctga ccacccacca tcctgaggga cctaggactg aattgatggt     420
cctatatgtc tccctacctg acccaacctg gtgaccaaac caattccatg ggtgtgggg      480
agagatgggg ttaccgagag ttcaggtaat gaagattaca tgcctatggg agtcctgaaa     540
cacttggttg aagtgtccct ccctgccaga ggagatttaa acaagcaagg caacacaggc     600
aattggggtc aagactcaat ggtcaattca ggtcagtaga tccttcaggt ctacctcagt     660
ctctgacctc gagacaggcg tctgactgtt tcagtcataa tggcttctgg tcctgctacc     720
acttgcctcc ttggcagact ccaattcagc cctgtcacct ttatttactc cttccttttg     780
tttgtttgtt tgtttgtttg ttttgagcca aggtctttct gtagctctgg ctggtctgga     840
gctccctttg caaaccacaa atcctctgtc tcccaagtgc tggccaaatc tgtgttctta     900
caacctccaa ataaaaccct cttcctgcag ccggcaggac ggccgagcac aatggtgctt     960
gctgtacagc ctcatagacc ttccttggat ctcccagaca cacttagtgg aaggagagaa    1020
tccattcttg caaattgtct gacctatgca catgcacaca aaaaaataaa tttttaatta    1080
aaaaaatctg ctgggtagtg gtgccacaca cctttattta aacctaacac tcaggaggta    1140
gaggcaggag gatctcttga gtttgattcc agacaggtct acagattaag ttccaggaca    1200
gccagggcta cacagagaca ccctatcttg ggaagcaaaa taaagtaaga taaatcttg     1259
```

<210> 42
<211> 2221
<212> DNA
<213> Artificial

<400> 42

```
gcgaccggca gggtgcccgc ccgacttccc agctgccccg tgcggcccgg gcatgcccag      60
tgtgggcgca gccccggctc tccgagcgcc ggggcggagc gggcagccgg gcgcggagca     120
ggcgaaccga acctcggtgg gcgagcgccg gggctggagc cgcgggggtc ggtggcggga     180
gctggggacc gaacccaggg ccttgcgctt cttaggcctt ccatgatgag tgatttgggg     240
gcacttccct ctctctgtaa ttgatagtct gggcagtgaa gagatggctg acagtgtcaa     300
aacctttctg caggaccttg gcaggggaat caaagactcc atctggggca tctgtaccat     360
ctcaaagcta gatgctcgga tccagcagaa gagagaggag cagcgtcgaa ggagggcaag     420
tagcctcttg gcccagagga gagcccagag tgtagagcgg aagcaagaga gtgagccacg     480
```

tattgttagt agaattttcc agtgctgtgc ttggaatggt ggagtattct ggttcagtct 540
cctcttgttt taccgagtgt ttattcctgt gcttcaatca gtaacagccc gggttattgg 600
tgatccgtca ctacatggag atgtttggtc atggctggaa ttcttcctca catcaatttt 660
cagtgctctt tgggtgctgc ccctgtttgt gcttagcaaa gttgtgaatg ccatttggtt 720
ccaagatata gctgacttgg catttgaggt atcagggagg aaacctcatc cattccccag 780
tgtcagcaaa ataattgctg acatgctctt caaccttttg ctacaggcac ttttccttat 840
tcaggggatg tttgtgagtc tcttccccat ccatcttgtg ggtcagctgg ttagtttgct 900
gcatatgtct cttctctact cactctactg ctttgagtac cgttggttca acaaaggaat 960
tgaaatgcac cagcgattgt caaacataga aaggaactgg ccttactact ttgggtttgg 1020
cttgcccttg gctttcctca cagcaatgca atcctcctac attatcagtg gctgcctctt 1080
ttctatcctg tttcctttat tcatcatcag cgccaatgaa gcaaagactc ctggaaaagc 1140
atatcttttc cagttgcgcc tcttctcctt ggtggtcttt ttaagcaaca gacttttcca 1200
caagacggtc tacctacagt cggccctgag cagctcatcc tccgcagaga aattcccttc 1260
gccacatcct tctcctgcca aactgaaagc tgctgcaggc cactgagtcc tgctgtcaaa 1320
ggggacgggt gggactgggt ggaggatgtg gcagctcttt tcttggtttt cctcccctgc 1380
tgtggaaggc aggacccact gccaaggggc ctctgcatat tcctttgtct ttgaattgga 1440
atcttcctga ctccggtatg tggattttta ccaccaccct aggtctggaa ggaccagttt 1500
tccagctgtt ttttagcttt tgccagctcc tgtgcctgga ctgatttgag tactcccccc 1560
tcccttgtgt catttacctt cccacctcct cctgcctccc agcacccctg ggtgaatggg 1620
ctttgtaatt ttaactgttg tattttgtga atttgttgtt actgtttcct gtgaagcaca 1680
taccttgtat gtgggaggta aaggagcacg ccagctgctc catgtcactc cctctatagc 1740
catcactgtc ttgttttttt gtaactcagg ttaggttttg gtctttactg ctctgctgca 1800
gaaaaggaaa ggaatggggg aaaggagcct aagagatggg acagatagac ctcagccaaa 1860
ctggttgggt tttgaggagt catgttcttt gttccccttg aaggggaaac aggtttttc 1920
actggtacat ttaaagttcc ccagctatgg gaaggtacca gttctggaca agtgccactg 1980
catcatagca actctgaaga tgaaacttac tgttggccac tgccaggtca gactcgtgtt 2040
ttaaggaata ctgggtgctt catataggaa ctgaaggggt aaacttacta aaccattcaa 2100
cctgtgattg gtgatgtttt tctgtcattt taagagtcga cacatggggt gggagggcag 2160
atgtaaaaaa acttgtacaa ttttaaaatc acaattaaac gtgagctggt ttcccataaa 2220
a 2221

<210> 43
<211> 1078
<212> DNA
<213> Artificial

<220>
<223> Synthetic oligonucleotide

<400> 43

```
tcttttccag ttgcgcctct tctccttggt ggtcttttta agcaacagac ttttccacaa      60
gacggtctac ctacagtcgg ccctgagcag ctcatcctcc gcagagaaat tcccttcgcc     120
acatccttct cctgccaaac tgaaagctgc tgcaggccac tgagtcctgc tgtcaaaggg     180
gacgggtggg actgggtgga ggatgtggca gctcttttct tggttttcct cccctgctgt     240
ggaaggcagg acccactgcc aaggggcctc tgcatattcc tttgtctttg aattggaatc     300
ttcctgactc cggtatgtgg atttttacca ccaccctagg tctggaagga ccagttttcc     360
agctgttttt tagcttttgc cagctcctgt gcctggactg atttgagtac tccccccctcc     420
cttgtgtcat ttaccttccc acctcctcct gcctcccagc acccctgggt gaatgggctt     480
tgtaatttta actgttgtat tttgtgaatt tgttgttact gtttcctgtg aagcacatac     540
cttgtatgtg ggaggtaaag gagcacgcca gctgctccat gtcactccct ctatagccat     600
cactgtcttg tttttttgta actcaggtta ggttttggtc tttactgctc tgctgcagaa     660
aaggaaagga atgggggaaa ggagcctaag agatgggaca gatagacctc agccaaactg     720
gttgggtttt gaggagtcat gttctttgtt ccccttgaag gggaaacagg tttttttcact     780
ggtacattta aagttcccca gctatgggaa ggtaccagtt ctggacaagt gccactgcat     840
catagcaact ctgaagatga aacttactgt tggccactgc caggtcagac tcgtgtttta     900
aggaatactg ggtgcttcat ataggaactg aaggggtaaa cttactaaac cattcaacct     960
gtgattggtg atgtttttct gtcattttaa gagtcgacac atggggtggg agggcagatg    1020
taaaaaaact tgtacaattt taaaatcaca attaaacgtg agctggtttc ccataaaa      1078
```

<210> 44
<211> 966
<212> DNA
<213> Artificial

<400> 44

agaccgttct tgcctctgaa ccggcaatca tggctccggg ttggccgcgg cctctgccgc 60
agctcctcgt gttgggattc gggttggtgt tgatacgcgc cacggccggg gagcaagcac 120
caggcaacgc cccatgctca agcggcagct cctggagcgc ggacctcgac aagtgcatgg 180
actgcgcttc ttgtccagcg cgaccacaca gcgacttctg cctgggatgc gcagcagcac 240
ctcctgccca cttcaggatg ctatggccca ttctgggagg cgctcttagt ctggccctgg 300
ttttggcgct ggtttctggt ttcctggtct ggagacgatg ccgccggaga gaaaagttta 360
ctaccccat agaggagact ggtggagaag gctgcccagg tgtggcactg atccagtgag 420
gagcacccgc gctggtgccc attcatcgtc cattcattca ttctggagcc agcctggctt 480
tccagagaca agccgcgcca gactcttcca accacaaggg ggtggggcga ggtggtgatt 540
cacctccaag gactgggctt agggttcagg ggagccttcc agggtgtcta attgccctgt 600
ctctggttct ggggcagaca gagagcctca agctaggtca caaagcgact catactaagg 660

atctgcagca tttgcacaag ggaatctctt gctccccaca agtcctggag gcctggccga 720
cttgaggggc agacacaaca ctgggttcca cccactcgga tggactgaaa tgccactacc 780
ctgagtttta gccctgggag tgggttagat tgagggacct gttcataaca ctaaggggc 840
tggccctctg ggagggctgg tcccagtaca cacaaacaca acccatcccc caaggggtg 900
atatttattt tggggataaa gtttggaggg gagggagact tattaataaa agaatcttta 960
acttta 966

<210> 45
<211> 603
<212> DNA
<213> Artificial

<220>
<223> Synthetic oligonucleotide

<400> 45

cccccataga ggagactggt ggagaaggct gcccaggtgt ggcactgatc cagtgaggag 60
cacccgcgct ggtgcccatt catcgtccat tcattcattc tggagccagc ctggctttcc 120
agagacaagc cgcgccagac tcttccaacc acaagggggt ggggcgaggt ggtgattcac 180
ctccaaggac tgggcttagg gttcagggga gccttccagg gtgtctaatt gccctgtctc 240
tggttctggg gcagacagag agcctcaagc taggtcacaa agcgactcat actaaggatc 300
tgcagcattt gcacaaggga atctcttgct ccccacaagt cctggaggcc tggccgactt 360
gaggggcaga cacaacactg ggttccaccc actcggatgg actgaaatgc cactaccctg 420
agttttagcc ctgggagtgg gttagattga gggacctgtt cataacacta agggggctgg 480
ccctctggga gggctggtcc cagtacacac aaacacaacc catcccccaa gggggtgata 540
tttattttgg ggataaagtt tggaggggag ggagacttat taataaaaga atctttaact 600
tta 603

<210> 46
<211> 443
<212> DNA
<213> Artificial

<400> 46

aagcactgtt agtttgctgg tctgtctcct ctgtgctctt gtctgtgtct gtgtcctcga 60
tcctgactca agcaatcccg ctctgttttg ctgactcctg agcagacata gcttgtgggc 120
caggaccttg ccagccttga gtggcagtgg cttcagaagt gaaatgatct ggctgtctgc 180
attcgaatga ctccccagtc ccagcctagc ctctcttttg cactgctggt tcagcccact 240
gggcctccgt cctttcctct ggaagggact tggccttggg tgacaaattc ctctttgatg 300
aatgtaccct gtgggaatgt ttcatactga cagattattt ttatttattc aatgtcgtat 360
ttaagatatt tatttttat actgaaggag caccctttt ttaagagaaa taaatgaaat 420

aataaagaac ccattcttgt cga 443

<210> 47
<211> 443
<212> DNA
<213> Artificial

<220>
<223> Synthetic oligonucleotide

<400> 47

aagcactgtt agtttgctgg tctgtctcct ctgtgctctt gtctgtgtct gtgtcctcga 60
tcctgactca agcaatcccg ctctgttttg ctgactcctg agcagacata gcttgtgggc 120
caggaccttg ccagccttga gtggcagtgg cttcagaagt gaaatgatct ggctgtctgc 180
attcgaatga ctccccagtc ccagcctagc ctctcttttg cactgctggt tcagcccact 240
gggcctccgt cctttcctct ggaagggact tggccttggg tgacaaattc ctctttgatg 300
aatgtaccct gtgggaatgt ttcatactga cagattattt ttatttattc aatgtcgtat 360
ttaagatatt tattttttat actgaaggag cacccttttt ttaagagaaa taaatgaaat 420
aataaagaac ccattcttgt cga 443

<210> 48
<211> 808
<212> DNA
<213> Artificial

<400> 48

cagaagagaa cccagggcac tgggacagga cttcctgagg gtctgaggag catggcagac 60
agcccagagg tggtagccat ggactgtgag atggtggggc tcgggcctca aagggtgagt 120
ggcctcgccc gctgcagcat tgtgaacgtc cacagcgcag tcctctatga caagtacatc 180
cagcctgagg gagagatcac ggactacaga acccaagtca gcgggatcac gcctcagcac 240
atggcgaggg ccacgccatt tgctgaagcc aggctagaga tcctgcagct tctgaaaggc 300
aagctggtgg tgggccatga cctgaagcat gacttcagtg ccctgaagga ggacatgaga 360
aaatacacca tctacgacac gtccacagac atgctgctgt ggcacgaggc caagctgcac 420
tgctacagcc gtgtgtccct gaggctgctg tgcaagcgcc tgctgcacaa gagcatccag 480
aacaactggc ggggccactg ctctgtagaa gatgccaggg ccacaatgga gctctacaaa 540
atctctcagc gactcagagc ccagcgaggg ctgccctgcc tgggaacatc agcctgaact 600
tcatcctcat ccaggatcag aagcagctac tccttgaagg accatagttt tccatgaatg 660
agacctgttt ctagctacaa caacaacagc aacaaccaca aaatgcaaaa gcaagaacac 720
tccgccatta cagcaattct cttgctttgg agcaaagaaa aaaaaaacca aactttagta 780
ataaatgact ttgattttgt ctaatcaa 808

<210> 49
<211> 328
<212> DNA
<213> Artificial

<220>
<223> Synthetic oligonucleotide

<400> 49

aacaactggc ggggccactg ctctgtagaa gatgccaggg ccacaatgga gctctacaaa 60
atctctcagc gactcagagc ccagcgaggg ctgccctgcc tgggaacatc agcctgaact 120
tcatcctcat ccaggatcag aagcagctac tccttgaagg accatagttt tccatgaatg 180
agacctgttt ctagctacaa caacaacagc aacaaccaca aaatgcaaaa gcaagaacac 240
tccgccatta cagcaattct cttgctttgg agcaaagaaa aaaaaaacca aactttagta 300
ataaatgact ttgattttgt ctaatcaa 328

<210> 50
<211> 1491
<212> DNA
<213> Artificial

<400> 50

gcaaagtcac actcaggaca caagcgacat ggccctgcgc gcgctccatg cccagcctac 60
aggcggtcct cagctgaggt tcctgctgtt cctgctgctc ttgctgcttc tgctgtcatg 120
gccatctcag gggacgccc tggcattgcc tgagcagcga cgctccctct ctgagtccca 180
actcaacccg gacgagctac ggggtcgctt ccaggacctg ctgagccgac tgcatgccaa 240
ccagagccgg gaggactcga actcagaacc aacccctgac ccagctgtcc ggatactcag 300
tccagaggtg cgattggggt cccacggccg gctgctgctc cgcgtcaacc gggcgtcgct 360
gactcagggt ctccccgaag cctaccgcgt gcaccgagcg ctgctcctgc tgacgccttc 420
gtccaggccc tgggacatca ccaggcccct gcaacgtgcg atcagcctcc agggacccca 480
cgctcgcgca ctgcgcttgc gcctggcgcc gcctcccgac ctagccgtgc tgccctctgg 540
cggcgcgcgc ctggaactgc acttacgatc ggccgccggc agggggcgcc gaagcgcgca 600
tttgcaccca agagactcgt gcccgctggg tcccgggcgc tgctgtcacc tggagactgt 660
gcaggcaact ctcgaggacc taggttggag cgactgggta ctgtccccgc gtcagctgca 720
actgagcatg tgcgtgggcg agtgccccca cctctaccgg tcggccaaca cgcatgcgca 780
gatcaaagca cgcctgcatg gcctgcagcc cgacagagtg ccggccccgt gctgtgtccc 840
ctccagctac accccggtgg ttcttatgca caggacagac agcggcgtgt cactgcagac 900
ttatgatgat ctggtagccc agggctgcca ctgcgcttga gcactgggcc ctgctcctta 960
cctacactcc ccttccatga tgctatttat atttttattt aataatatta ttaatttatt 1020
ggggttgggc tgggtgggtg gattgtgtat ttatttaaaa ctctgctaat aaaggtgagc 1080
ttggtttcta tgcggcgtct cagtgatctc ggcatgattc ccatcaccct agcctcagtt 1140
tcccttgtca cctgagaagt ggagcagagg acgggcctcc attaggctgg cctggggtga 1200

ttttattgac tgacagctga tgtgagtgac cagccagggc ctggtggtcc tgccatgttt 1260
gagccgcaag gaggagcagg acagtcagca gttcattgct gcagcctctg cttcagcgcc 1320
cttctaggtt ccctctggac attcctccca tccccatttc aagccaggcc taacatccca 1380
gaatcagggg gcctctgcaa tccagatagt ccgacaacgg ctggctggga atgaaaagtg 1440
taagaagcca gtggctgctc agtcaacaag actgggtgtc tcggctgctc t 1491

<210> 51
<211> 1183
<212> DNA
<213> Artificial

<220>
<223> Synthetic oligonucleotide

<400> 51

tgcgattggg gtcccacggc cggctgctgc tccgcgtcaa ccgggcgtcg ctgactcagg 60
gtctccccga agcctaccgc gtgcaccgag cgctgctcct gctgacgcct tcgtccaggc 120
cctgggacat caccaggccc ctgcaacgtg cgatcagcct ccagggaccc cacgctcgcg 180
cactgcgctt gcgcctggcg ccgcctcccg acctagccgt gctgccctct ggcggcgcgc 240
gcctggaact gcacttacga tcggccgccg gcagggggcg ccgaagcgcg catttgcacc 300
caagagactc gtgcccgctg ggtcccgggc gctgctgtca cctggagact gtgcaggcaa 360
ctctcgagga cctaggttgg agcgactggg tactgtcccc gcgtcagctg caactgagca 420
tgtgcgtggg cgagtgcccc cacctctacc ggtcggccaa cacgcatgcg cagatcaaag 480
cacgcctgca tggcctgcag cccgacagag tgccggcccc gtgctgtgtc ccctccagct 540
acaccccggt ggttcttatg cacaggacag acagcggcgt gtcactgcag acttatgatg 600
atctggtagc ccagggctgc cactgcgctt gagcactggg ccctgctcct tacctacact 660
ccccttccat gatgctattt atatttttat ttaataatat tattaattta ttggggttgg 720
gctgggtggg tggattgtgt atttatttaa aactctgcta ataaaggtga gcttggtttc 780
tatgcggcgt ctcagtgatc tcggcatgat tcccatcacc ctagcctcag tttcccttgt 840
cacctgagaa gtggagcaga ggacgggcct ccattaggct ggcctggggt gattttattg 900
actgacagct gatgtgagtg accagccagg gcctggtggt cctgccatgt ttgagccgca 960
aggaggagca ggacagtcag cagttcattg ctgcagcctc tgcttcagcg cccttctagg 1020
ttccctctgg acattcctcc catccccatt tcaagccagg cctaacatcc cagaatcagg 1080
gggcctctgc aatccagata gtccgacaac ggctggctgg gaatgaaaag tgtaagaagc 1140
cagtggctgc tcagtcaaca agactgggtg tctcggctgc tct 1183

<210> 52
<211> 3218
<212> DNA

<213> Artificial

<400> 52

caatcctggt gcccagccta tctccccgcg gccgggcgca gtccttcacc gcacgctgaa 60
ccggaggaag gctgcgccta gtcggggcgc tgagggaccc tccaccggga cgccggcccc 120
tccccgggcc tctgctcact tgcccccctg cgagcccgtc cccctagtcg gcctctcgga 180
tcggggacgt ggggcgagct gagagcaggc ccggggtggg tggtcactgt ggagaagacg 240
tggctgtcaa gatgatagaa gtactgacaa ctgactctca gaaactgcta caccagctga 300
acaccctgtt ggaacaggag tccagatgtc agccaaaggt ctgtggcctg aaactgattg 360
agtctgcaca tgataatggc ctcaggatga ctgcaagact ccgggacttt gaagtcaaag 420
atctactgag tctaactcag ttctttggct tcgacacaga aacattttcc cttgctgtga 480
atttactgga cagattcttg tctaaaatga aggtacaggc gaagcatctc ggctgtgtcg 540
gactgagctg cttttatttg gctgtgaaat cgattgaaga ggaaaggaac gtcccgctgg 600
caactgattt gatccggata agtcagtata ggttcacagt ttcagacctg atgagaatgg 660
aaaagattgt gttggagaaa gtgtgctgga aagtcaaagc tactactgcc ttccaatttc 720
tgcagctcta ttactccctc attcgggaga ccttgccatt tgaaaggaga aacgatctga 780
attttgaaag actagaagcc caactgaagg cgtgccactg caggatcata ttttctaagg 840
caaagccttc tgtgctggcg ctggcaatca tcgctttgga gatccaagca ctgaagtatg 900
tggagttaac agaaggagta gaatgtattc agaaacattc caagataagt ggccgagatc 960
tgaccttctg gcaagagctt gtttccaagt gtttaactga atattcatca aacaagtgtt 1020
ccaagccgaa cggtcagaag ttaaaatgga tcgtgtctgg gcgcactgca cgacaactga 1080
agcacagtta ttacaggata acccacctcc caacaattcc cgaaaccatg ggttagttgg 1140
caaatctggt tgttatcctc tgtgtacaga acatttccca gtgagatcgt tttgtgcta 1200
taacttaagg attgaaatac taccttcaat ataaagaata caggatgaaa acagtaaagg 1260
aaacgtgagt ttgttggtct agacagagaa tactgggagg cattcactgt gtaccgcagt 1320
ctgaagagaa atgagtatca aacctctaga cacatgctca tactgctgtc aaaggactag 1380
cgtagaaaag agagtcctcc aaaccggaag tttaaatgta gttactaaaa tagcacttct 1440
ttaacttaca tatcccccca ctgtggctta tttaaagtta cagaagtcca agcagaacga 1500
caaaagatgt gacccatata tgaacacatt ttaatctgtt cattgattag gagagtgaat 1560
atgaacttgc atgatgccca tgttaggttt ctggaaactg ccggggtatc ttaattctct 1620
agtattctcc ctctgtggca gttgggctaa tacaaagtaa ctatacgcat gagaatataa 1680
aatcagtctc tgatacatac acatttttac catcaaaatt tcttaatcat agcaaagact 1740
taccttttta tgattaggaa tttttttttt aatgtatggc agcacatgcc tttaatccca 1800
acactaggga ggcagaggca ggtggatctc tttgagttcg aagccaggct ggtctttaca 1860
gtgagttcca ggacagctgg agagctacag aatggagaga cgctgtctca aaaacactca 1920
aaacaaacaa acaaaccata ccagtttgta ggcagacttc tgttgggttg ggtttgtact 1980
gtttgcctat gcagtgggat tacagcagca gcaacaaaaa ctgtccctga agtctttctc 2040

tgccactgtg acctgagttt cctatggtac gcgatttact ctaagaaacc tcagcccctc 2100
accacgttag ctgttggcaa atggcctcac agttgcggaa agtcccaatt ctaggcttgg 2160
gaaagcaatg cttagatttg aattggccca tgaagcattc aaatcaaggc taaagacata 2220
aatgtgaaat aaaactgtga accttcattt taacattgat ctcacttccc agatttaatc 2280
aatatatact taggtggtat taaaaatggt aaactgccta atttaaatct caaaatttaa 2340
actatgaggt ttacatcaaa gccaacattt cacaaatgta cttttaaggt attaaaagag 2400
gtatttaagc agtaaatggt ttcttggcac ccataaccaa gtaatagtta agttagaggt 2460
gggacttttt tattgctatg agaattacat ttaaactttt gggtgtttta taaaaagcag 2520
atttcacaag ttttgaaaat tgtgaccttt actgaaattt gttaccttta atatttcttc 2580
tagaggatag gtatttataa aagaaaaatt cgtcagaatt gctgcctcaa tctagtccca 2640
tttgagaaaa tttgtttcta ctgtctcaat aactggatga aatatcactc tgaaaacttg 2700
cctattgcac taaagctagt ttaggcttga taaaacactc caggaggttt ttaccacaga 2760
ctgtttctat taaaactgct gcttctcatg tacaattttg ttttaaaagg aaccgagtac 2820
atctgcaaaa cctaagtctt aagggacgtc aggaggtacc ttcagaatta taggatcacc 2880
atggtagtgg ggattctcca tgctggcctt gaatgtttga tcttcactgc tgaaatgtgg 2940
gtagctcctc agcgccctgt agagcctgag tctacctaga atagctgtaa ccattttgac 3000
aagtaatgga taagaaaatt atccattgag aagctaaaaa caaaacaaaa caaaaccaaa 3060
gaacgggtgt attttattct taacctttgt aaaccatcac tgagaacact tcagttcttc 3120
ctaacagctg ttatgcttcg atttgaaaaa aatactgagt ggataaccaa ctaccatcat 3180
gctttgggta cacctttcaa taaaattact gaaatgca 3218

<210> 53
<211> 1558
<212> DNA
<213> Artificial

<220>
<223> Synthetic oligonucleotide

<400> 53

cagtaaagga aacgtgagtt tgttggtcta gacagagaat actgggaggc attcactgtg 60
taccgcagtc tgaagagaaa tgagtatcaa acctctagac acatgctcat actgctgtca 120
aaggactagc gtagaaaaga gagtcctcca aaccggaagt ttaaatgtag ttactaaaat 180
agcacttctt taacttacat atccccccac tgtggcttat ttaaagttac agaagtccaa 240
gcagaacgac aaaagatgtg acccatatat gaacacattt taatctgttc attgattagg 300
agagtgaata tgaacttgca tgatgcccat gttaggtttc tggaaactgc cggggtatct 360
taattctcta gtattctccc tctgtggcag ttgggctaat acaaagtaac tatacgcatg 420
agaatataaa atcagtctct gatacataca catttttacc atcaaaattt cttaatcata 480
gcaaagactt acctttttat gattaggaat ttttttttta atgtatggca gcacatgcct 540
ttaatcccaa cactagggag gcagaggcag gtggatctct ttgagttcga agccaggctg 600
gtctttacag tgagttccag gacagctgga gagctacaga atggagagac gctgtctcaa 660
aaacactcaa aacaaacaaa caaaccatac cagtttgtag gcagacttct gttgggttgg 720
gtttgtactg tttgcctatg cagtgggatt acagcagcag caacaaaaac tgtccctgaa 780
gtctttctct gccactgtga cctgagtttc ctatggtacg cgatttactc taagaaacct 840
cagcccctca ccacgttagc tgttggcaaa tggcctcaca gttgcggaaa gtcccaattc 900
taggcttggg aaagcaatgc ttagatttga attggcccat gaagcattca aatcaaggct 960
aaagacataa atgtgaaata aaactgtgaa ccttcatttt aacattgatc tcacttccca 1020
gatttaatca atatatactt aggtggtatt aaaaatggta aactgcctaa tttaaatctc 1080
aaaatttaaa ctatgaggtt tacatcaaag ccaacatttc acaaatgtac ttttaaggta 1140
ttaaaagagg tatttaagca gtaaatggtt tcttggcacc cataaccaag taatagttaa 1200
gttagaggtg ggactttttt attgctatga gaattacatt taaacttttg ggtgttttat 1260
aaaaagcaga tttcacaagt tttgaaaatt gtgaccttta ctgaaatttg ttacctttaa 1320
tatttcttct agaggatagg tatttataaa agaaaaattc gtcagaattg ctgcctcaat 1380
ctagtcccat ttgagaaaat ttgtttctac tgtctcaata actggatgaa atatcactct 1440
gaaaacttgc ctattgcact aaagctagtt taggcttgat aaaacactcc aggaggtttt 1500
taccacagac tgtttctatt aaaactgctg cttctcatgt acaattttgt tttaaaag 1558

<210> 54
<211> 2030
<212> DNA
<213> Artificial

<400> 54

```
ggggcggatg cgcagaggtg caccacgtgt ggtgttcctc cagcgcagtc aggatctcgt     60
ctagtttggg tctaggttag atttggaatc gtggagatgc ggaaggaaag cccacctccg    120
ctcgtgcccc cggcggcccg cgagtggaac ctgcccccca atgcgcccgc ctgcatggaa    180
cggcagttgg aagctgcacg gtaccgctct gatggttccc ttctgctcgg ggcttccagt    240
ctgagtggtc gctgctgggc aggatctctg tggtttttca aggatcctag tgcggccccc    300
aacgaaggtt tctgctctgc tggcgtccag acggaggctg gagtagctga cctcacttgg    360
gttggggaca aaggtatctt agtggcttct gattcaggtg ctgttgaatt gtgggagcta    420
gatgagaacg agacacttat tgtcagcaag ttctgcaagt atgagcatga tgacattgtg    480
tctactgtca ctgtcctgag ctccggcaca caggctgtca gtggtagcaa agacttctgc    540
atcaaaattt gggacctggc tcagcagatg tcgctgaatt cataccgagc tcatgcggga    600
caggtcacct gtgttgctgc ctctccccac agagaaactg tgtttctttc atgcagtgag    660
gacagtagaa ttttgctctg ggatacccgc tgtcccaagc cggcatcaca gatgggctgc    720
```

aatgcctctg gctacctccc tacctccctg gcttggcatc ctcagcagag tgaaatcttt 780
gtctttggtg atgagaatgg atctgtctcc cttgtggaca ccaagaatgc aagctgcacc 840
ctcagctcag ctgtgcactc gcagtgtgtc actagactgg tgttctctcc acacagtgtt 900
cccttcctgg cctctctcag tgaagactgt tcacttgctg tgctggattc aagcttttct 960
gaggtgttta gaagtcgagc ccacagagac tttgtgagag atgctacatg gtctccactc 1020
aatcactccc ttcttaccac agttggctgg gaccatcagg tcatccacca tgttgtgccg 1080
ttagagcctc tcccagcccc tggacctgac agtgttgtgg agtagaatgg attgcagaag 1140
aaaagagcct tctgtctgca agtgtcgact cgcttgcccc tgccttccat ttgtgagaga 1200
gagagcacag gagccttgta gagcatgatt actccctagt cccgtgcagt aactagtcag 1260
attctcagcc tgagggaggc tgcatcccat agtgacttgg aggaaaaagc ttcctttata 1320
aatggatgta tgtgtgagta cacgtgtgag tgcagtgtat agtttggagt ggagaaaatt 1380
atccttcagc tttcccaaag caatgctctt tacccccgac aaaatgacca gaggatttta 1440
tgcttcccat gtctgggggc tgactatgtt gttagggata tggagtgtgg gtatccctgg 1500
atgcttggga atacttcaca ttactcagag gtgcgtaact tatttttata tagagtttaa 1560
ttcactatat gggaattact tcccatatac aaaagtctag ccagactacc ttgagaagac 1620
agaacagttt tgtacgaatg cctgttaaat ggaatcaaat ccactttctg ggtgaatcaa 1680
tttggccctt ggagttcgcg ctcgctcacc ctgtgttcct tcttttgtgt tgtgtatgag 1740
agatgagtgc tttcctgtcc tagatttcac agttttaggg atgatggagt acttaaatta 1800
aaagtgtttc agtgaagcga gaagattgac atggattgtt acagagaaat ctgtcattgt 1860
catttttccc acctaaaaat tccctgagga ctgatctggg tactttgctc tggagagctg 1920
aagtctgagc gctgtatatt tggactccta agattgaaga tccggcatca gagaaactgt 1980
caaaaatact ggaaggtgaa ataaagactt atcctttcat ctttcctcca 2030

<210> 55
<211> 1146
<212> DNA
<213> Artificial

<220>
<223> Synthetic oligonucleotide

<400> 55

gtttagaagt cgagcccaca gagactttgt gagagatgct acatggtctc cactcaatca 60
ctcccttctt accacagttg gctgggacca tcaggtcatc caccatgttg tgccgttaga 120
gcctctccca gcccctggac ctgacagtgt tgtggagtag aatggattgc agaagaaaag 180
agccttctgt ctgcaagtgt cgactcgctt gcccctgcct tccatttgtg agagagagag 240
cacaggagcc ttgtagagca tgattactcc ctagtcccgt gcagtaacta gtcagattct 300
cagcctgagg gaggctgcat cccatagtga cttggaggaa aaagcttcct ttataaatgg 360
atgtatgtgt gagtacacgt gtgagtgcag tgtatagttt ggagtggaga aaattatcct 420
tcagctttcc caaagcaatg ctctttaccc ccgacaaaat gaccagagga ttttatgctt 480
cccatgtctg ggggctgact atgttgttag ggatatggag tgtgggtatc cctggatgct 540
tgggaatact tcacattact cagaggtgcg taacttattt ttatatagag tttaattcac 600
tatatgggaa ttacttccca tatacaaaag tctagccaga ctaccttgag aagacagaac 660
agttttgtac gaatgcctgt taaatggaat caaatccact ttctgggtga atcaatttgg 720
cccttggagt tcgcgctcgc tcaccctgtg ttccttcttt tgtgttgtgt atgagagatg 780
agtgctttcc tgtcctagat ttcacagttt tagggatgat ggagtactta aattaaaagt 840
gtttcagtga agcgagaaga ttgacatgga ttgttacaga gaaatctgtc attgtcattt 900
ttcccaccta aaaattccct gaggactgat ctgggtactt tgctctggag agctgaagtc 960
tgagcgctgt atatttggac tcctaagatt gaagatccgg catcagagaa actgtcaaaa 1020
atactggaag gtgaaataaa gacttatcct ttcatctttc ctccaaagct tatttattac 1080
ctgctttgct taggtgagtc acctgagtat caatcagtct taactgccca ctgtctttgt 1140
agtcta 1146

<210> 56
<211> 1971
<212> DNA
<213> Artificial

<400> 56

```
ggtaaccagg agttaagcgg gctccctgcc agcgcgaggg ctttaaaagg ggtgatgcaa      60
cgcgctccca gtcacagtct cactcagcga gacgcgcggt gcacggtgct tccccggcgg     120
agccgaccga ccaacccgcg ctccggcaga gtccttggcg ctcgcctgcc ggcgggacag     180
acagcccgcc tctagccgct ctctggaccc tggccgcccc gagcgaagac tggagcaaaa     240
tgatgcttca acatccaggc caggtctctg cctcagaagt cagcgcgacc gccatcgtcc     300
cctgcctctc acctcctggg tcactggtgt ttgaggattt tgctaacctg acaccttttg     360
tcaaggaaga gctgagattc gccatccaga acaagcacct ttgccatcgg atgtcctctg     420
cgctggagtc agtcaccatc aacaacagac ctctggagat gtcagtcacc aagtctgagg     480
tggcccctga agaagatgag agaaaaagga ggcggcggga aagaaacaaa attgctgctg     540
ccaagtgtcg aaacaagaaa aaagagaaga cagagtgcct gcagaaggag tcagagaaac     600
tggagagtgt gaatgccgaa ctgaaggccc agatcgagga gctgaagaat gagaagcagc     660
atctgattta catgctcaac ctgcaccggc ccacgtgtat cgtccgggct cagaacgggc     720
ggacgccgga agacgagagg aacctttta tccaacagat aaaagaagga acattgcaga      780
gctaagcaga ggtggcatgg gggcaattgg ggagtcctta ctgaatcctc cttttccacc     840
ccaaaccctg aagccattgg aaaactggct tcctgtgcac ttctagaatc tcagcagcca     900
cgagctgttg ggtcaggagg gcctgcggtg actactgcgt tgtcccactc tgtccccgag     960
tgaaccgtgg agcaggcagg agcatccttt gtctcaccgg ctccaggatt taggccttac    1020

catcccggcc attctcagat gacctagctg gccccaggct ggggtcccat gcaaagcagg    1080
atcgcactaa tgggatgcag gcagaagtgt ctaccttgac aggtggggtg ggaccacgtc    1140
ctccactgcg gctgacaaca tccctcctag ggaagatgga gtgagaacat tcatcattga    1200
agttgtccaa tggccagggt atgctttcta gaaactatgc tgttctgtcc tagactgact    1260
gtgcataggg cattcatttc tgagcctggt gttgtgctat ttagatgttt gtcttgcaca    1320
acattggcgt gatttttttc cgggagtttc atcagacctg atttccgaga gtttgggggt    1380
ctgccactgt ggacaatatc ccccaaaagt gtttgggtgg ccatgtaaac tggctgatga    1440
ccagctgtgc tactctgtgc tgaccgagga ctgatgcctc cttcccctgt acccactgct    1500
gaggaagaac ccgggcacag cagctgtcct tggctacaaa ctgttacaat gtcacagaac    1560
gaaggcacaa agtcccgctt tcaaagggcg taggactcca cactcagtga cagggcagga    1620
agagccaagg attctccgtt ttcccttcct tcccaccaaa aaccacagcc cgtggagact    1680
ggtatttgaa gccaggagtg gggcaaggaa ggtgtctgca ctgtgggatg ttaactgcgc    1740
ttttgtcttg aagctatttt gagatgcggt ccagagtatt tcagctggga ggtccctccc    1800
actggccacc agggctctgg ctactgttaa aattctgatg tttctgtgaa atcctcagtg    1860
ttcaatccag actcagtagt atattacagt tttctgtaag agagaacgtt acttatttat    1920
cccagtattc ctagcctgtc aacgtaataa aatatcagaa tgagacctgg t             1971
```

<210> 57
<211> 1384
<212> DNA
<213> Artificial

<220>
<223> Synthetic oligonucleotide

<400> 57

gagtcagaga aactggagag tgtgaatgcc gaactgaagg cccagatcga ggagctgaag 60
aatgagaagc agcatctgat ttacatgctc aacctgcacc ggcccacgtg tatcgtccgg 120
gctcagaacg ggcggacgcc ggaagacgag aggaaccttt ttatccaaca gataaaagaa 180
ggaacattgc agagctaagc agaggtggca tgggggcaat tggggagtcc ttactgaatc 240
ctccttttcc accccaaacc ctgaagccat tggaaaactg gcttcctgtg cacttctaga 300
atctcagcag ccacgagctg ttgggtcagg agggcctgcg gtgactactg cgttgtccca 360
ctctgtcccc gagtgaaccg tggagcaggc aggagcatcc tttgtctcac cggctccagg 420
atttaggcct taccatcccg gccattctca gatgacctag ctggccccag gctggggtcc 480
catgcaaagc aggatcgcac taatgggatg caggcagaag tgtctacctt gacaggtggg 540
gtgggaccac gtcctccact gcggctgaca acatccctcc tagggaagat ggagtgagaa 600
cattcatcat tgaagttgtc caatggccag ggtatgcttt ctagaaacta tgctgttctg 660
tcctagactg actgtgcata gggcattcat ttctgagcct ggtgttgtgc tatttagatg 720
tttgtcttgc acaacattgg cgtgattttt ttccgggagt ttcatcagac ctgatttccg 780
agagtttggg ggtctgccac tgtggacaat atcccccaaa agtgtttggg tggccatgta 840
aactggctga tgaccagctg tgctactctg tgctgaccga ggactgatgc ctccttcccc 900
tgtacccact gctgaggaag aacccgggca cagcagctgt ccttggctac aaactgttac 960
aatgtcacag aacgaaggca caaagtcccg ctttcaaagg gcgtaggact ccacactcag 1020
tgacagggca ggaagagcca aggattctcc gttttccctt ccttcccacc aaaaaccaca 1080
gcccgtggag actggtattt gaagccagga gtggggcaag gaaggtgtct gcactgtggg 1140
atgttaactg cgcttttgtc ttgaagctat tttgagatgc ggtccagagt atttcagctg 1200
ggaggtccct cccactggcc accagggctc tggctactgt taaaattctg atgtttctgt 1260
gaaatcctca gtgttcaatc cagactcagt agtatattac agttttctgt aagagagaac 1320
gttacttatt tatcccagta ttcctagcct gtcaacgtaa taaaatatca gaatgagacc 1380
tggt 1384

<210> 58
<211> 1120
<212> DNA
<213> Artificial

<400> 58

ggcctgggcg gagagggagg aaaacttcct ggctgggact ctggcggttt ctgcctgcca 60
accctccgat ccgcctacca gcgttggctc ttcccgaccc ctcccccggc gcccccagtg 120
tccgccatgg ccaaagccta cgaccacctc ttcaagttgc tgctgatcgg ggactcgggg 180
gtgggcaaga cttgtctcat cattcgcttt gcagaggaca acttcaacag cacttacatc 240
tccaccatcg gaattgattt caagatccga actgtggaaa tagaggggaa gagaatcaaa 300
ctgcaagtct gggacacagc tggccaagaa cgattcaaga caataaccac cgcctattac 360
cgtggagcca tgggcattat cctagtatat gacatcacag atgagaaatc cttcgagaat 420
attcagaact ggatgaaaag catcaaagag aacgcgtctg ctggagtgga gcgccttctg 480
ctggggaaca aatgtgacat ggaagccaag cggaaggtgc agagagagca ggctgagagg 540
ttggcccgag agcacagaat ccgatttttt gagacaagtg ccaaatccag tgtgaatgtg 600
gatgaggctt tcagttccct ggcccgtgac atcttgctca agacaggagg ccggagatcg 660
ggaaacagca gcaagccctc aagcactgac ctgaaagtat ctgacaagaa gaacagcaac 720
aagtgctcct tgggctgagg aacatttctt gcctcctatt caccctgaac ctggaggcta 780
gacctgaggg aggtggactg aggtagactg atggaaaaca gaggggagga gctgtggtgg 840
tgcctggagg ggtggatgac aggggaggaa ggaaagatga aatgggcagg gaaaggaggg 900
cgaggaacca aggacgtgaa aagtgaagag aaggggtttg agaagagaaa aagaagaagg 960
tctcaggtct cggaccgtcc aacattaatg tcagtatgct gatctctcca ttcctggttc 1020
agggttcggg tcccgagagg ctggctcggc cctactctga gggtctctca ctccacagat 1080

ctttgttagt attaaaggcc actgtttggc acgaatgttc 1120

<210> 59
<211> 120
<212> DNA
<213> Artificial

<220>
<223> Synthetic oligonucleotide

<400> 59

ttggcccgag agcacagaat ccgatttttt gagacaagtg ccaaatccag tgtgaatgtg 60
gatgaggctt tcagttccct ggcccgtgac atcttgctca agacaggagg ccggagatcg 120

<210> 60
<211> 1877
<212> DNA
<213> Artificial

<400> 60

gatgggatgc atctatcttg tgatatgtac cagccacagg caccatgtcc gatcctggtg 60
atgtccgacc tgttccacac aggagcaaag tatgccgtcg tctgttcggt cccgtggaca 120
gtgagcagtt gagccgcgat tgcgatgcgc tcatggcgag ctgtctccag gaggcccgag 180
aacggtggaa ctttgacttc gccactgaga cgccactgga gggcaactac gtctgggagc 240
gtgttcggag cccagggctg cccaagatct acctgagccc tgggtcccgc cgccgtgatg 300
acctgggagg ggacaagagg cccagtacct cctcggccct gctgcagggg ccagggccag 360
ctccggagga ccacgtggcc ttgtcgctgt cttgcactct ggtgtctcac gcccctgaga 420
ggcctgaaga ctccccgggc gggaccggga catctcaggg ccgaaaacgg aggcagacca 480
gcctaacaga tttctatcac tccaagcgcc gattggtctt ctgcaagaga aagccctgaa 540
gtgcccacgg gaggctcgcc ttcttctgct gtgggtcagg aggcctcttc cccatcttct 600
gccttagcct tcattcagtg tgtcttaatt attatttgtg ttttaattta aacttctcct 660
gtatatactc tgcctgcccc ctcccagcct ccaaacttag ttatttaaaa aacaaaacaa 720
aacaatgaaa ttagtaggac ggtaggctcc ttagtgctgt tttttttttt ttttatgtag 780
accattattt aagtccttct caacccaagc tgtgtttctg atcctggcgg gatggtcctg 840
gcgggatggt cctggcggga tggtcgcact ggcctcatgc catctgcatc tcgcatctcg 900
cccaatcccc gcccctcccc cctagctcct cccccgcccc cactccctgc ctggttcctt 960
gccacttctt acctgggggc gatcctcaga cctgaatagc actttggaca actgagtagg 1020
acttcggggt ctccttgtca tctctaaggc ccgctaggat gacagtgaag cagtcacagc 1080
ctagaacaaa gatgcccggt taggacctaa gcgtaccgtc cagagccttg acatttactc 1140
agacctgtga agatcctttg ccactcctgg ggaccccgcc tcccctgtgg gtctctgcca 1200
gctgcccctc tatttttgag ggttaatctg gtgatctgct gctctccctt cctcagaccc 1260
ttcccctccc caggttggca ggaggcatcc tttccctcgc cacggctcag tggaccagaa 1320

gggaacgggt acacagggta cactaagtgg ggtttcctgg tcctacctca ggcagctcca 1380
gtggcaactg cccttcttat gggtctaggg taggtccttg gtgacgagac gggcttccca 1440
gagcatccct gtgtgtgtgg tgtgtggtgg tggtgggggg tggacttatc tgggatgggg 1500
accccagccg ctgaagtcct cagtgacttg tcccatttct tagtagttgt acaaggagtc 1560
aggccaagat ggtgcctcgg gggctgaggg agctcacagg aactgagcag tgactggtcc 1620
tttcccagta ttgaatactg agcccctgtg ggtgtcgaag cacttagtgg gtctggcccc 1680
aaccccaaac accctgttt ctgtaacacc ctgagctgga ctgtttatct ttagccggga 1740
gaacatgtat tttggtcccc tccctgtctc cgctcggatt gtaaacctcc cacgtgtggg 1800
gatcacaccc tgcactgtcc cgaatcttta caccctatcc caaagctggt gctcaataaa 1860
tacttctaga tgatttg 1877

<210> 61

<211> 1109
<212> DNA
<213> Artificial

<220>
<223> Synthetic oligonucleotide

<400> 61

```
tttttatgt agaccattat ttaagtcctt ctcaacccaa gctgtgtttc tgatcctggc      60
gggatggtcc tggcgggatg gtcctggcgg gatggtcgca ctggcctcat gccatctgca     120
tctcgcatct cgcccaatcc ccgcccctcc cccctagctc ctcccccgcc cccactccct     180
gcctggttcc ttgccacttc ttacctgggg gcgatcctca gacctgaata gcactttgga     240
caactgagta ggacttcggg gtctccttgt catctctaag gcccgctagg atgacagtga     300
agcagtcaca gcctagaaca aagatgcccg gttaggacct aagcgtaccg tccagagcct     360
tgacatttac tcagacctgt gaagatcctt tgccactcct ggggaccccg cctcccctgt     420
gggtctctgc cagctgcccc tctatttttg agggttaatc tggtgatctg ctgctctccc     480
ttcctcagac ccttcccctc cccaggttgg caggaggcat cctttccctc gccacggctc     540
agtggaccag aagggaacgg gtacacaggg tacactaagt ggggtttcct ggtcctacct     600
caggcagctc cagtggcaac tgcccttctt atgggtctag ggtaggtcct tggtgacgag     660
acgggcttcc cagagcatcc ctgtgtgtgt ggtgtgtggt ggtggtgggg ggtggactta     720
tctgggatgg ggaccccagc cgctgaagtc ctcagtgact tgtcccattt cttagtagtt     780
gtacaaggag tcaggccaag atggtgcctc gggggctgag ggagctcaca ggaactgagc     840
agtgactggt cctttcccag tattgaatac tgagcccctg tgggtgtcga agcacttagt     900
gggtctggcc ccaaccccaa acacccctgt ttctgtaaca ccctgagctg gactgtttat     960
ctttagccgg gagaacatgt attttggtcc cctccctgtc tccgctcgga ttgtaaacct    1020
cccacgtgtg gggatcacac cctgcactgt cccgaatctt tacaccctat cccaaagctg    1080
gtgctcaata aatacttcta gatgatttg                                     1109
```

<210> 62
<211> 57
<212> PRT
<213> Artificial

<400> 62

Ala Pro Ser Leu Gly Leu Arg Val Val Gly Ala Asp Ser Ser Cys Leu
1 5 10 15

Gly Thr Gly Asn His Gly Phe Gly Leu Ala Ala Val Ser Ala Ala Asp
20 25 30

Pro Arg Val Gly Ile Arg Leu Gly Val Asp Ala Arg Arg Gly Arg Gly
35 40 45

Ala Ser Thr Arg His Leu Pro Met Leu
50 55

<210> 63
<211> 171
<212> PRT
<213> Artificial

<400> 63

Met Ala Gly Ile Pro Glu Val Val Ala Met Asp Cys Glu Met Val Gly
1 5 10 15

Leu Gly Pro Gln Arg Val Ser Gly Leu Ala Arg Cys Ser Ile Val Asn
20 25 30

Ile His Gly Ala Val Leu Tyr Asp Lys Tyr Ile Arg Pro Glu Gly Glu
35 40 45

Ile Thr Asp Tyr Arg Thr Gln Val Ser Gly Val Thr Pro Gln His Met
50 55 60

Val Arg Ala Thr Pro Phe Gly Glu Ala Arg Leu Glu Ile Leu Gln Leu
65 70 75 80

Leu Lys Gly Lys Leu Val Val Gly His Asp Leu Lys His Asp Phe Asn
85 90 95

Ala Leu Lys Glu Asp Met Ser Lys Tyr Thr Ile Tyr Asp Thr Ser Thr
100 105 110

Asp Arg Leu Leu Trp His Glu Ala Lys Leu Gln Tyr Tyr Ser Arg Val
115 120 125

Ser Leu Arg Leu Leu Cys Lys Arg Leu Leu His Lys Asn Ile Gln Val
130 135 140

Arg Ser Phe Pro Ala Pro Val Thr Leu Leu Ser Leu Leu Pro Pro Leu
145 150 155 160

Ser Leu Pro Pro Ser Ser His Ser Gly Lys Glu
165 170

<210> 64
<211> 280
<212> PRT
<213> Artificial

<400> 64

Met Val Trp Gly His Phe Pro Leu Ala Val Phe Asp Ser Leu Gly Ser
1               5                   10                  15

Gly Glu Met Ala Asp Ser Val Lys Thr Phe Leu Gln Asp Leu Gly Arg
            20                  25                  30

Gly Ile Lys Asp Ser Ile Trp Gly Ile Cys Thr Ile Ser Lys Leu Asp
        35                  40                  45

Ala Arg Ile Gln Gln Lys Arg Glu Glu Gln Arg Arg Arg Arg Ala Ser
    50                  55                  60

Ser Leu Leu Ala Gln Arg Arg Pro Gln Ser Val Glu Arg Lys Gln Glu
65                  70                  75                  80

Ser Glu Pro Arg Ile Val Ser Arg Ile Phe Gln Cys Cys Ala Trp Asn
                85                  90                  95

Gly Gly Val Phe Trp Phe Ser Leu Leu Leu Phe Tyr Arg Val Phe Ile
            100                 105                 110

Pro Val Leu Gln Ser Val Thr Ala Arg Ile Ile Gly Asp Pro Ser Leu
        115                 120                 125

His Gly Asp Val Trp Ser Trp Leu Glu Phe Phe Leu Thr Ser Ile Phe
    130                 135                 140

Ser Ala Leu Trp Val Leu Pro Leu Phe Val Leu Ser Lys Val Val Asn
145                 150                 155                 160

Ala Ile Trp Phe Gln Asp Ile Ala Asp Leu Ala Phe Glu Val Ser Gly
                165                 170                 175

Arg Lys Pro His Pro Phe Pro Ser Val Ser Lys Ile Ile Ala Asp Met
            180                 185                 190

Leu Phe Asn Leu Leu Leu Gln Ala Leu Phe Leu Ile Gln Gly Met Phe
        195                 200                 205

Val Ser Leu Phe Pro Ile His Leu Val Gly Gln Leu Val Ser Leu Leu
    210                 215                 220

His Met Ser Leu Leu Tyr Ser Leu Tyr Cys Phe Glu Tyr Arg Trp Phe
225                 230                 235                 240

Asn Lys Gly Ile Glu Met His Gln Arg Leu Ser Asn Ile Glu Arg Asn
                245                 250                 255

Trp Pro Tyr Tyr Phe Gly Phe Gly Leu Pro Leu Ala Phe Leu Thr Ala
            260                 265                 270

Met Gln Ser Ser Tyr Ile Ile Arg
        275                 280

<210> 65
<211> 154
<212> PRT
<213> Artificial

<400> 65

Met Ile Arg Asn Phe Phe Phe Asn Val Trp Gln His Met Pro Leu Ile
1 5 10 15

Pro Thr Leu Gly Arg Gln Arg Gln Val Asp Leu Phe Glu Phe Glu Ala
20 25 30

Arg Leu Val Phe Thr Val Ser Ser Arg Thr Ala Gly Glu Leu Gln Asn
35 40 45

Gly Glu Thr Leu Ser Gln Lys His Ser Lys Gln Thr Asn Lys Pro Tyr
50 55 60

Gln Phe Val Gly Arg Leu Leu Leu Gly Trp Val Cys Thr Val Cys Leu
65 70 75 80

Cys Ser Arg Ile Thr Ala Ala Ala Thr Lys Thr Val Pro Gln Val Phe
85 90 95

Leu Cys His Ser Asp Leu Ser Phe Leu Arg Tyr Arg Ile Tyr Ser Glu
100 105 110

Lys Pro Gln His Leu Ala Thr Val Ala Val Gly Arg Met Ala Ser Arg
115 120 125

Leu Gly Lys Leu Leu Ile Leu Ser Leu Gly Glu Leu Arg Leu Asp Leu
130 135 140

Asn Ser Pro Arg Lys His Ser Asn Gln Gly
145 150

<210> 66
<211> 37
<212> PRT
<213> Artificial

<400> 66

Arg Pro Ser Ile Lys Arg Ile Thr Pro Ile Ser Asp Val Asp Ala Ser
1 5 10 15

Gln Leu Glu Ser Gln Val Ser Ser Leu Phe Pro His Cys Pro Ala Gln
20 25 30

Ser Phe Ala Ala Arg
35

<210> 67
<211> 234
<212> PRT
<213> Artificial

<400> 67

Met Arg Lys Asp Thr Pro Pro Pro Leu Val Pro Pro Ala Ala Arg Glu
1               5                   10                  15

Trp Asn Leu Pro Pro Asn Ala Pro Ala Cys Met Glu Arg Gln Leu Glu
            20                  25                  30

Ala Ala Arg Tyr Arg Ser Asp Gly Ser Leu Leu Leu Gly Val Ser Ser
        35                  40                  45

Leu Ser Gly Arg Cys Trp Val Gly Ser Leu Trp Phe Phe Lys Asp Pro
    50                  55                  60

Ser Ala Ala Pro Asn Glu Gly Phe Cys Ser Ala Gly Val Gln Thr Glu
65                  70                  75                  80

Ala Gly Val Ala Asp Leu Thr Trp Val Gly Asp Lys Gly Ile Leu Val
                85                  90                  95

Ala Ser Asp Ser Gly Ala Val Glu Leu Trp Glu Leu Asp Glu Asn Glu
            100                 105                 110

Thr Leu Ile Val Ser Lys Phe Cys Lys Tyr Glu His Asp Asp Ile Val
        115                 120                 125

Ser Thr Val Thr Val Leu Ser Ser Gly Thr Gln Ala Val Ser Gly Ser
    130                 135                 140

Lys Asp Cys Cys Ile Lys Ile Trp Asp Leu Ala Gln Gln Val Ser Leu
145                 150                 155                 160

Asn Ser Tyr Arg Ala His Ala Gly Gln Val Thr Cys Val Ala Ala Ser
                165                 170                 175

Pro His Lys Asp Ser Val Phe Leu Ser Cys Ser Glu Asp Ser Arg Ile
            180                 185                 190

Leu Leu Trp Asp Thr Arg Cys Pro Lys Pro Ala Ser Gln Met Ala Cys
        195                 200                 205

Asn Ala Ser Gly Tyr Leu Pro Thr Ala Leu Ala Trp His Pro Gln Gln
    210                 215                 220

Ser Glu Val Phe Val Phe Gly Lys Ala Ala
225                 230

<210> 68
<211> 342
<212> PRT
<213> Artificial

<400> 68

Met Arg Lys Asp Thr Pro Pro Pro Leu Val Pro Pro Ala Ala Arg Glu
1 5 10 15

Trp Asn Leu Pro Pro Asn Ala Pro Ala Cys Met Glu Arg Gln Leu Glu
20 25 30

Ala Ala Arg Tyr Arg Ser Asp Gly Ser Leu Leu Leu Gly Val Ser Ser
35 40 45

Leu Ser Gly Arg Cys Trp Val Gly Ser Leu Trp Phe Phe Lys Asp Pro
50 55 60

Ser Ala Ala Pro Asn Glu Gly Phe Cys Ser Ala Gly Val Gln Thr Glu
65 70 75 80

Ala Gly Val Ala Asp Leu Thr Trp Val Gly Asp Lys Gly Ile Leu Val
85 90 95

Ala Ser Asp Ser Gly Ala Val Glu Leu Trp Glu Leu Asp Glu Asn Glu
100 105 110

Thr Leu Ile Val Ser Lys Phe Cys Lys Tyr Glu His Asp Asp Ile Val
115 120 125

Ser Thr Val Thr Val Leu Ser Ser Gly Thr Gln Ala Val Ser Gly Ser
130 135 140

Lys Asp Cys Cys Ile Lys Ile Trp Asp Leu Ala Gln Gln Val Ser Leu
145 150 155 160

Asn Ser Tyr Arg Ala His Ala Gly Gln Val Thr Cys Val Ala Ala Ser
165 170 175

Pro His Lys Asp Ser Val Phe Leu Ser Cys Ser Glu Asp Ser Arg Ile
180 185 190

Leu Leu Trp Asp Thr Arg Cys Pro Lys Pro Ala Ser Gln Met Ala Cys
195 200 205

Asn Ala Ser Gly Tyr Leu Pro Thr Ala Leu Ala Trp His Pro Gln Gln
210 215 220

Ser Glu Val Phe Val Phe Gly Asp Glu Asn Gly Ser Val Ser Leu Val
225 230 235 240

Asp Thr Lys Asn Ala Ser Cys Thr Leu Ser Ser Ala Val His Ser Gln
245 250 255

Gly Val Thr Arg Leu Val Phe Ser Pro His Ser Val Pro Leu Leu Thr
260 265 270

Ser Leu Ser Glu Asp Cys Ser Leu Ala Val Leu Asp Ser Ser Leu Ser
275 280 285

Glu Val Phe Arg Ser Arg Ala His Arg Asp Phe Val Arg Asp Ala Thr
290 295 300

Trp Ser Pro Leu Asn His Ser Leu Leu Thr Thr Val Gly Trp Asp His
305 310 315 320

Gln Val Ile His His Val Val Pro Leu Glu Pro Leu Pro Asn Pro Gly
325 330 335

Pro Asp Ser Val Val Glu
340

<210> 69
<211> 183
<212> PRT
<213> Artificial

<400> 69

Met Ala Lys Ala Tyr Asp His Leu Phe Lys Leu Leu Leu Ile Gly Asp
1 5 10 15

Ser Gly Val Gly Lys Thr Cys Leu Ile Ile Arg Phe Ala Glu Asp Asn
20 25 30

Phe Asn Ser Thr Tyr Ile Ser Thr Ile Gly Ile Asp Phe Lys Ile Arg
35 40 45

Thr Val Asp Ile Glu Gly Lys Arg Ile Lys Leu Gln Val Trp Asp Thr
50 55 60

Ala Gly Gln Glu Arg Phe Lys Thr Ile Thr Thr Ala Tyr Tyr Arg Gly
65 70 75 80

Ala Met Gly Ile Ile Leu Val Tyr Asp Ile Thr Asp Glu Lys Ser Phe
85 90 95

Glu Asn Ile Gln Asn Trp Met Lys Ser Ile Lys Glu Asn Ala Ser Ala
100 105 110

Gly Val Glu Arg Leu Leu Leu Gly Asn Lys Cys Asp Met Glu Ala Lys
115 120 125

Arg Gln Val Gln Arg Glu Gln Ala Glu Lys Val Ser Gln Ala Gly Leu
130 135 140

Leu Trp Pro Asn Ala Leu Leu Leu Pro Phe Ile Gln Leu Ser Pro Ser
145 150 155 160

Tyr Leu Ile Pro Tyr Ser Trp Leu Glu Ser Thr Glu Ser Asp Phe Leu
165 170 175

Arg Arg Val Pro Asn Pro Val
180

<210> 70
<211> 122
<212> PRT
<213> Artificial

<400> 70

Met Gly Ile Ile Leu Val Tyr Asp Ile Thr Asp Glu Lys Ser Phe Glu
1               5                   10                  15

Asn Ile Gln Asn Trp Met Lys Ser Ile Lys Glu Asn Ala Ser Ala Gly
            20                  25                  30

Val Glu Arg Leu Leu Leu Gly Asn Lys Cys Asp Met Glu Ala Lys Arg
        35                  40                  45

Gln Val Gln Arg Glu Gln Ala Glu Lys Leu Ala Arg Glu His Arg Ile
    50                  55                  60

Arg Phe Phe Glu Thr Ser Ala Lys Ser Ser Val Asn Val Asp Glu Ala
65                  70                  75                  80

Phe Ser Ser Leu Ala Arg Asp Ile Leu Leu Lys Thr Gly Gly Arg Arg
                85                  90                  95

Ser Gly Asn Ser Ser Lys Pro Ser Ser Thr Gly Leu Lys Thr Ser Asp
            100                 105                 110

Lys Lys Asn Ser Asn Lys Cys Leu Leu Gly
        115                 120

<210> 71
<211> 257
<212> PRT
<213> Artificial

<400> 71

```
Pro Thr Leu Leu Arg Pro Pro Asp Gly Pro Gly Ala Thr Ser Ala Ser
1               5                   10                  15

Pro Val Ala Cys Leu Leu Asp Ser Gly His Gln Ala Gln Leu Val Glu
            20                  25                  30

Phe Leu Ile Val His Tyr Glu Gln Ile Phe Gly Met Asp Glu Leu Pro
        35                  40                  45

Leu Thr Ser Glu Pro Leu Thr Gln Asp Pro Ala Leu Ala Pro Ala Leu
    50                  55                  60

Leu Glu Ser Ser Pro Gln His Pro Ala Leu Leu Val Ala Gln Asp Ser
65                  70                  75                  80

Gln Pro Leu Thr Ile Ala Ser Asp Ser Ser Pro Asp Pro Lys Gln His
                85                  90                  95

Ser Ala Leu Glu Lys Cys Pro Lys Val Ala Pro Pro Glu Leu Thr Ala
            100                 105                 110

Leu Gln Ser Asn Arg Lys Glu Glu Glu Glu Glu Asp Thr Arg Asp Gly
        115                 120                 125

Ala Gly Asp Gly Ser Ser His Ser Pro Glu Asp Leu Leu Leu Val Ala
    130                 135                 140

Gln Ser Arg Gly His Phe Ser Arg Gln Pro Val Lys Tyr Ser Arg Gly
145                 150                 155                 160

Gly Val Arg Pro Val Thr His Gln Leu Ser Ser Leu Ala Leu Val Ala
                165                 170                 175

Ser Lys Leu Cys Glu Glu Thr Pro Ile Thr Val Ser Ala Val His Arg
            180                 185                 190

Gly Ser Leu Arg Ala Arg Gly Leu Gly Pro Ala Ala Ala Ser Pro Glu
        195                 200                 205

Gly Gly Thr Leu Arg Arg Asn Pro Leu Pro Lys His Phe Glu Ile Thr
    210                 215                 220

Gln Glu Thr Ala Arg Leu Leu Ser Lys Leu Asp Ser Glu Ala Val Ser
225                 230                 235                 240

Arg Thr Ser Cys Cys Ala Asp Pro Glu Pro Glu Glu Ser Glu Glu His
                245                 250                 255

Leu
```

<210> 72

<211> 340
<212> PRT
<213> Artificial

<400> 72

```
Met Ala Asp Ser Val Lys Thr Phe Leu Gln Asp Leu Gly Arg Gly Ile
1               5                   10                  15

Lys Asp Ser Ile Trp Gly Ile Cys Thr Ile Ser Lys Leu Asp Ala Arg
            20                  25                  30

Ile Gln Gln Lys Arg Glu Glu Gln Arg Arg Arg Arg Ala Ser Ser Leu
        35                  40                  45

Leu Ala Gln Arg Arg Ala Gln Ser Val Glu Arg Lys Gln Glu Ser Glu
    50                  55                  60

Pro Arg Ile Val Ser Arg Ile Phe Gln Cys Cys Ala Trp Asn Gly Gly
65                  70                  75                  80

Val Phe Trp Phe Ser Leu Leu Leu Phe Tyr Arg Val Phe Ile Pro Val
                85                  90                  95

Leu Gln Ser Val Thr Ala Arg Val Ile Gly Asp Pro Ser Leu His Gly
            100                 105                 110

Asp Val Trp Ser Trp Leu Glu Phe Phe Leu Thr Ser Ile Phe Ser Ala
        115                 120                 125

Leu Trp Val Leu Pro Leu Phe Val Leu Ser Lys Val Val Asn Ala Ile
    130                 135                 140

Trp Phe Gln Asp Ile Ala Asp Leu Ala Phe Glu Val Ser Gly Arg Lys
145                 150                 155                 160

Pro His Pro Phe Pro Ser Val Ser Lys Ile Ile Ala Asp Met Leu Phe
                165                 170                 175
```

Asn Leu Leu Leu Gln Ala Leu Phe Leu Ile Gln Gly Met Phe Val Ser
180 185 190

Leu Phe Pro Ile His Leu Val Gly Gln Leu Val Ser Leu Leu His Met
195 200 205

Ser Leu Leu Tyr Ser Leu Tyr Cys Phe Glu Tyr Arg Trp Phe Asn Lys
210 215 220

Gly Ile Glu Met His Gln Arg Leu Ser Asn Ile Glu Arg Asn Trp Pro
225 230 235 240

Tyr Tyr Phe Gly Phe Gly Leu Pro Leu Ala Phe Leu Thr Ala Met Gln
245 250 255

Ser Ser Tyr Ile Ile Ser Gly Cys Leu Phe Ser Ile Leu Phe Pro Leu
260 265 270

Phe Ile Ile Ser Ala Asn Glu Ala Lys Thr Pro Gly Lys Ala Tyr Leu
275 280 285

Phe Gln Leu Arg Leu Phe Ser Leu Val Val Phe Leu Ser Asn Arg Leu
290 295 300

Phe His Lys Thr Val Tyr Leu Gln Ser Ala Leu Ser Ser Ser Ser Ser
305 310 315 320

Ala Glu Lys Phe Pro Ser Pro His Pro Ser Pro Ala Lys Leu Lys Ala
325 330 335

Ala Ala Gly His
340

<210> 73
<211> 138
<212> PRT
<213> Artificial

<400> 73

Thr Val Leu Ala Ser Glu Pro Ala Ile Met Ala Pro Gly Trp Pro Arg
1 5 10 15

Pro Leu Pro Gln Leu Leu Val Leu Gly Phe Gly Leu Val Leu Ile Arg
20 25 30

Ala Thr Ala Gly Glu Gln Ala Pro Gly Asn Ala Pro Cys Ser Ser Gly
35 40 45

Ser Ser Trp Ser Ala Asp Leu Asp Lys Cys Met Asp Cys Ala Ser Cys
50 55 60

Pro Ala Arg Pro His Ser Asp Phe Cys Leu Gly Cys Ala Ala Ala Pro
65 70 75 80

Pro Ala His Phe Arg Met Leu Trp Pro Ile Leu Gly Gly Ala Leu Ser
85 90 95

Leu Ala Leu Val Leu Ala Leu Val Ser Gly Phe Leu Val Trp Arg Arg
100 105 110

Cys Arg Arg Arg Glu Lys Phe Thr Thr Pro Ile Glu Glu Thr Gly Gly
115 120 125

Glu Gly Cys Pro Gly Val Ala Leu Ile Gln
130 135

<210> 74
<211> 198
<212> PRT
<213> Artificial

<400> 74

Gln Lys Arg Thr Gln Gly Thr Gly Thr Gly Leu Pro Glu Gly Leu Arg
1 5 10 15

Ser Met Ala Asp Ser Pro Glu Val Val Ala Met Asp Cys Glu Met Val
20 25 30

Gly Leu Gly Pro Gln Arg Val Ser Gly Leu Ala Arg Cys Ser Ile Val
35 40 45

Asn Val His Ser Ala Val Leu Tyr Asp Lys Tyr Ile Gln Pro Glu Gly
50 55 60

Glu Ile Thr Asp Tyr Arg Thr Gln Val Ser Gly Ile Thr Pro Gln His
65 70 75 80

Met Ala Arg Ala Thr Pro Phe Ala Glu Ala Arg Leu Glu Ile Leu Gln
85 90 95

Leu Leu Lys Gly Lys Leu Val Val Gly His Asp Leu Lys His Asp Phe
100 105 110

Ser Ala Leu Lys Glu Asp Met Arg Lys Tyr Thr Ile Tyr Asp Thr Ser
115 120 125

Thr Asp Met Leu Leu Trp His Glu Ala Lys Leu His Cys Tyr Ser Arg
130 135 140

Val Ser Leu Arg Leu Leu Cys Lys Arg Leu Leu His Lys Ser Ile Gln
145 150 155 160

Asn Asn Trp Arg Gly His Cys Ser Val Glu Asp Ala Arg Ala Thr Met
165 170 175

Glu Leu Tyr Lys Ile Ser Gln Arg Leu Arg Ala Gln Arg Gly Leu Pro
180 185 190

Cys Leu Gly Thr Ser Ala
195

<210> 75
<211> 312
<212> PRT
<213> Artificial

<400> 75

Gln Ser His Thr Gln Asp Thr Ser Asp Met Ala Leu Arg Ala Leu His
1               5                   10                  15

Ala Gln Pro Thr Gly Gly Pro Gln Leu Arg Phe Leu Leu Phe Leu Leu
            20                  25                  30

Leu Leu Leu Leu Leu Leu Ser Trp Pro Ser Gln Gly Asp Ala Leu Ala
        35                  40                  45

Leu Pro Glu Gln Arg Arg Ser Leu Ser Glu Ser Gln Leu Asn Pro Asp
    50                  55                  60

Glu Leu Arg Gly Arg Phe Gln Asp Leu Leu Ser Arg Leu His Ala Asn
65                  70                  75                  80

Gln Ser Arg Glu Asp Ser Asn Ser Glu Pro Thr Pro Asp Pro Ala Val
                85                  90                  95

Arg Ile Leu Ser Pro Glu Val Arg Leu Gly Ser His Gly Arg Leu Leu
            100                 105                 110

Leu Arg Val Asn Arg Ala Ser Leu Thr Gln Gly Leu Pro Glu Ala Tyr
        115                 120                 125

Arg Val His Arg Ala Leu Leu Leu Leu Thr Pro Ser Ser Arg Pro Trp
    130                 135                 140

Asp Ile Thr Arg Pro Leu Gln Arg Ala Ile Ser Leu Gln Gly Pro His
145                 150                 155                 160

Ala Arg Ala Leu Arg Leu Arg Leu Ala Pro Pro Pro Asp Leu Ala Val
                165                 170                 175

Leu Pro Ser Gly Gly Ala Arg Leu Glu Leu His Leu Arg Ser Ala Ala
            180                 185                 190

Gly Arg Gly Arg Arg Ser Ala His Leu His Pro Arg Asp Ser Cys Pro
195 200 205

Leu Gly Pro Gly Arg Cys Cys His Leu Glu Thr Val Gln Ala Thr Leu
210 215 220

Glu Asp Leu Gly Trp Ser Asp Trp Val Leu Ser Pro Arg Gln Leu Gln
225 230 235 240

Leu Ser Met Cys Val Gly Glu Cys Pro His Leu Tyr Arg Ser Ala Asn
245 250 255

Thr His Ala Gln Ile Lys Ala Arg Leu His Gly Leu Gln Pro Asp Arg
260 265 270

Val Pro Ala Pro Cys Cys Val Pro Ser Ser Tyr Thr Pro Val Val Leu
275 280 285

Met His Arg Thr Asp Ser Gly Val Ser Leu Gln Thr Tyr Asp Asp Leu
290 295 300

Val Ala Gln Gly Cys His Cys Ala
305 310

<210> 76
<211> 294
<212> PRT
<213> Artificial

<400> 76

```
Met Ile Glu Val Leu Thr Thr Asp Ser Gln Lys Leu Leu His Gln Leu
1               5                   10                  15

Asn Thr Leu Leu Glu Gln Glu Ser Arg Cys Gln Pro Lys Val Cys Gly
            20                  25                  30

Leu Lys Leu Ile Glu Ser Ala His Asp Asn Gly Leu Arg Met Thr Ala
        35                  40                  45

Arg Leu Arg Asp Phe Glu Val Lys Asp Leu Leu Ser Leu Thr Gln Phe
    50                  55                  60

Phe Gly Phe Asp Thr Glu Thr Phe Ser Leu Ala Val Asn Leu Leu Asp
65                  70                  75                  80

Arg Phe Leu Ser Lys Met Lys Val Gln Ala Lys His Leu Gly Cys Val
                85                  90                  95

Gly Leu Ser Cys Phe Tyr Leu Ala Val Lys Ser Ile Glu Glu Glu Arg
            100                 105                 110
```

Asn Val Pro Leu Ala Thr Asp Leu Ile Arg Ile Ser Gln Tyr Arg Phe
115 120 125

Thr Val Ser Asp Leu Met Arg Met Glu Lys Ile Val Leu Glu Lys Val
130 135 140

Cys Trp Lys Val Lys Ala Thr Thr Ala Phe Gln Phe Leu Gln Leu Tyr
145 150 155 160

Tyr Ser Leu Ile Arg Glu Thr Leu Pro Phe Glu Arg Arg Asn Asp Leu
165 170 175

Asn Phe Glu Arg Leu Glu Ala Gln Leu Lys Ala Cys His Cys Arg Ile
180 185 190

Ile Phe Ser Lys Ala Lys Pro Ser Val Leu Ala Leu Ala Ile Ile Ala
195 200 205

Leu Glu Ile Gln Ala Leu Lys Tyr Val Glu Leu Thr Glu Gly Val Glu
210 215 220

Cys Ile Gln Lys His Ser Lys Ile Ser Gly Arg Asp Leu Thr Phe Trp
225 230 235 240

Gln Glu Leu Val Ser Lys Cys Leu Thr Glu Tyr Ser Ser Asn Lys Cys
245 250 255

Ser Lys Pro Asn Gly Gln Lys Leu Lys Trp Ile Val Ser Gly Arg Thr
260 265 270

Ala Arg Gln Leu Lys His Ser Tyr Tyr Arg Ile Thr His Leu Pro Thr
275 280 285

Ile Pro Glu Thr Met Gly
290

<210> 77
<211> 342
<212> PRT
<213> Artificial

<400> 77

Met Arg Lys Glu Ser Pro Pro Pro Leu Val Pro Pro Ala Ala Arg Glu
1 5 10 15

Trp Asn Leu Pro Pro Asn Ala Pro Ala Cys Met Glu Arg Gln Leu Glu
20 25 30

Ala Ala Arg Tyr Arg Ser Asp Gly Ser Leu Leu Leu Gly Ala Ser Ser
35 40 45

```
Leu Ser Gly Arg Cys Trp Ala Gly Ser Leu Trp Phe Phe Lys Asp Pro
    50                  55                  60

Ser Ala Ala Pro Asn Glu Gly Phe Cys Ser Ala Gly Val Gln Thr Glu
65                  70                  75                  80

Ala Gly Val Ala Asp Leu Thr Trp Val Gly Asp Lys Gly Ile Leu Val
                85                  90                  95

Ala Ser Asp Ser Gly Ala Val Glu Leu Trp Glu Leu Asp Glu Asn Glu
            100                 105                 110

Thr Leu Ile Val Ser Lys Phe Cys Lys Tyr Glu His Asp Asp Ile Val
        115                 120                 125

Ser Thr Val Thr Val Leu Ser Ser Gly Thr Gln Ala Val Ser Gly Ser
    130                 135                 140

Lys Asp Phe Cys Ile Lys Ile Trp Asp Leu Ala Gln Gln Met Ser Leu
145                 150                 155                 160

Asn Ser Tyr Arg Ala His Ala Gly Gln Val Thr Cys Val Ala Ala Ser
                165                 170                 175

Pro His Arg Glu Thr Val Phe Leu Ser Cys Ser Glu Asp Ser Arg Ile
            180                 185                 190

Leu Leu Trp Asp Thr Arg Cys Pro Lys Pro Ala Ser Gln Met Gly Cys
        195                 200                 205

Asn Ala Ser Gly Tyr Leu Pro Thr Ser Leu Ala Trp His Pro Gln Gln
    210                 215                 220

Ser Glu Ile Phe Val Phe Gly Asp Glu Asn Gly Ser Val Ser Leu Val
225                 230                 235                 240

Asp Thr Lys Asn Ala Ser Cys Thr Leu Ser Ser Ala Val His Ser Gln
                245                 250                 255

Cys Val Thr Arg Leu Val Phe Ser Pro His Ser Val Pro Phe Leu Ala
            260                 265                 270

Ser Leu Ser Glu Asp Cys Ser Leu Ala Val Leu Asp Ser Ser Phe Ser
        275                 280                 285

Glu Val Phe Arg Ser Arg Ala His Arg Asp Phe Val Arg Asp Ala Thr
    290                 295                 300

Trp Ser Pro Leu Asn His Ser Leu Leu Thr Thr Val Gly Trp Asp His
305                 310                 315                 320
```

Gln Val Ile His His Val Val Pro Leu Glu Pro Leu Pro Ala Pro Gly
325 330 335

Pro Asp Ser Val Val Glu
340

<210> 78
<211> 124
<212> PRT
<213> Artificial

<400> 78

Met Ser Ser Ala Leu Glu Ser Val Thr Ile Asn Asn Arg Pro Leu Glu
1 5 10 15

Met Ser Val Thr Lys Ser Glu Val Ala Pro Glu Glu Asp Glu Arg Lys
20 25 30

Arg Arg Arg Arg Glu Arg Asn Lys Ile Ala Ala Ala Lys Cys Arg Asn
35 40 45

Lys Lys Lys Glu Lys Thr Glu Cys Leu Gln Lys Glu Ser Glu Lys Leu
50 55 60

Glu Ser Val Asn Ala Glu Leu Lys Ala Gln Ile Glu Glu Leu Lys Asn
65 70 75 80

Glu Lys Gln His Leu Ile Tyr Met Leu Asn Leu His Arg Pro Thr Cys
85 90 95

Ile Val Arg Ala Gln Asn Gly Arg Thr Pro Glu Asp Glu Arg Asn Leu
100 105 110

Phe Ile Gln Gln Ile Lys Glu Gly Thr Leu Gln Ser
115 120

<210> 79
<211> 203
<212> PRT
<213> Artificial

<400> 79

Met Ala Lys Ala Tyr Asp His Leu Phe Lys Leu Leu Leu Ile Gly Asp
1 5 10 15

Ser Gly Val Gly Lys Thr Cys Leu Ile Ile Arg Phe Ala Glu Asp Asn
20 25 30

Phe Asn Ser Thr Tyr Ile Ser Thr Ile Gly Ile Asp Phe Lys Ile Arg
35 40 45

Thr Val Glu Ile Glu Gly Lys Arg Ile Lys Leu Gln Val Trp Asp Thr
50 55 60

Ala Gly Gln Glu Arg Phe Lys Thr Ile Thr Thr Ala Tyr Tyr Arg Gly
65 70 75 80

Ala Met Gly Ile Ile Leu Val Tyr Asp Ile Thr Asp Glu Lys Ser Phe
85 90 95

Glu Asn Ile Gln Asn Trp Met Lys Ser Ile Lys Glu Asn Ala Ser Ala
100 105 110

Gly Val Glu Arg Leu Leu Leu Gly Asn Lys Cys Asp Met Glu Ala Lys
115 120 125

Arg Lys Val Gln Arg Glu Gln Ala Glu Arg Leu Ala Arg Glu His Arg
130 135 140

Ile Arg Phe Phe Glu Thr Ser Ala Lys Ser Ser Val Asn Val Asp Glu
145 150 155 160

Ala Phe Ser Ser Leu Ala Arg Asp Ile Leu Leu Lys Thr Gly Gly Arg
165 170 175

Arg Ser Gly Asn Ser Ser Lys Pro Ser Ser Thr Asp Leu Lys Val Ser
180 185 190

Asp Lys Lys Asn Ser Asn Lys Cys Ser Leu Gly
195 200

<210> 80
<211> 164
<212> PRT
<213> Artificial

<400> 80

Met Ser Asp Pro Gly Asp Val Arg Pro Val Pro His Arg Ser Lys Val
1 5 10 15

Cys Arg Arg Leu Phe Gly Pro Val Asp Ser Glu Gln Leu Ser Arg Asp
20 25 30

Cys Asp Ala Leu Met Ala Ser Cys Leu Gln Glu Ala Arg Glu Arg Trp
35 40 45

Asn Phe Asp Phe Ala Thr Glu Thr Pro Leu Glu Gly Asn Tyr Val Trp
50 55 60

Glu Arg Val Arg Ser Pro Gly Leu Pro Lys Ile Tyr Leu Ser Pro Gly
65 70 75 80

```
Ser Arg Arg Arg Asp Asp Leu Gly Gly Asp Lys Arg Pro Ser Thr Ser
                85                  90                  95

Ser Ala Leu Leu Gln Gly Pro Gly Pro Ala Pro Glu Asp His Val Ala
            100                 105                 110

Leu Ser Leu Ser Cys Thr Leu Val Ser His Ala Pro Glu Arg Pro Glu
        115                 120                 125

Asp Ser Pro Gly Gly Thr Gly Thr Ser Gln Gly Arg Lys Arg Arg Gln
    130                 135                 140

Thr Ser Leu Thr Asp Phe Tyr His Ser Lys Arg Arg Leu Val Phe Cys
145                 150                 155                 160

Lys Arg Lys Pro
```

<210> 81
<211> 2077
<212> DNA
<213> Artificial

<400> 81

```
tccgctccgt tcggccggtt ctcccgggaa gctattaata gcattacgtc agcctgggac      60
tggcaacacg gagtaaacga ccgcgccgcc agcctgaggg ctataaaagg ggtgatgcaa     120
cgctctccaa gccacagtcg cacgcagcca ggcgcgcact gcacagctct cttctctcgc     180
cgccgcccga gcgcaccctt cagcccgcgc gccggccgtg agtcctcggt gctcgcccgc     240
cggccagaca aacagcccgc ccgaccccgt cccgaccctg gccgccccga gcggagcctg     300
gagcaaaatg atgcttcaac acccaggcca ggtctctgcc tcggaagtga gtgcttctgc     360
catcgtcccc tgcctgtccc ctcctgggtc actggtgttt gaggattttg ctaacctgac     420
gccctttgtc aaggaagagc tgaggtttgc catccagaac aagcacctct gccaccggat     480
gtcctctgcg ctggaatcag tcactgtcag cgacagaccc ctcggggtgt ccatcacaaa     540
agccgaggta gcccctgaag aagatgaaag gaaaaagagg cgacgagaaa gaaataagat     600
tgcagctgca aagtgccgaa acaagaagaa ggagaagacg gagtgcctgc agaaagagtc     660
ggagaagctg gaaagtgtga atgctgaact gaaggctcag attgaggagc tcaagaacga     720
gaagcagcat ttgatataca tgctcaacct tcatcggccc acgtgtattg tccgggctca     780
gaatgggagg actccagaag atgagagaaa cctctttatc caacagataa aagaaggaac     840
attgcagagc taagcagtcg tggtatgggg gcgactgggg agtcctcatt gaatcctcat     900
tttataccca aaaccctgaa gccattggag agctgtcttc ctgtgtacct ctagaatccc     960
agcagcagag aaccatcaag gcgggagggc ctgcagtgat tcagcaggcc cttcccattc    1020
```

```
tgccccagag tgggtcttgg accagggcaa gtgcatcttt gcctcaactc caggatttag   1080
gccttaacac actggccatt cttatgttcc agatggcccc cagctggtgt cctgcccgcc   1140
tttcatctgg attctacaaa aaaccaggat gcccaccgtt aggattcagg cagcagtgtc   1200
tgtacctcgg gtgggaggga tggggccatc tccttcaccg tggctaccat tgtcactcgt   1260
aggggatgtg gagtgagaac agcatttagt gaagttgtgc aacggccagg gttgtgcttt   1320
ctagcaaata tgctgttatg tccagaaatt gtgtgtgcaa gaaaactagg caatgtactc   1380
ttccgatgtt tgtgtcacac aacactgatg tgacttttat atgctttttc tcagatctgg   1440
tttctaagag ttttgggggg cggggctgtc accacgtgca gtatctcaag atattcaggt   1500
ggccagaaga gcttgtcagc aagaggagga cagaattctc ccagcgttaa cacaaaatcc   1560
atgggcagca tgatggcagg tcctctgttg caaactcagt tccaaagtca caggaagaaa   1620
gcagaaagtt caacttccaa agggttagga ctctccactc aatgtcttag gtcaggagtt   1680
gtgtctaggc tggaagagcc aaagaatatt ccattttcct ttccttgtgg ttgaaaacca   1740
cagtcagtgg agagatgttt ggaaaccaca gtcagtggag cctgggtggt acccaggctt   1800
tagcattatt ggatgtcaat agcattgttt ttgtcatgta gctgttttaa gaaatctggc   1860
ccagggtgtt tgcagctgtg agaagtcact cacactggcc acaaggacgc tggctactgt   1920
ctattaaaat tctgatgttt ctgtgaaatt ctcagagtgt ttaattgtac tcaatggtat   1980
cattacaatt ttctgtaaga gaaaatatta cttatttatc ctagtattcc taacctgtca   2040
gaataataaa tattggaacc aagacatggt aaacatg                            2077
```

<210> 82
<211> 1925
<212> DNA
<213> Artificial

<400> 82

```
gggcggaggt ggggttagct tcagttgacc aaccatgcct tgaggataaa ttggatggga      60
tcagatggga agatgtgaca agaagagaaa tcctcctcta tataggatgc tctgctgttt     120
cctaaggatt ttcagcacct tgccccaaaa tcaaaatgat gcttcaacac ccaggccagg     180
tctctgcctc ggaagtgagt gcttctgcca tcgtcccctg cctgtcccct cctgggtcac     240
tggtgtttga ggattttgct aacctgacgc cctttgtcaa ggaagagctg aggtttgcca     300
tccagaacaa gcacctctgc caccggatgt cctctgcgct ggaatcagtc actgtcagcg     360
acagacccct cggggtgtcc atcacaaaag ccgaggtagc ccctgaagaa gatgaaagga     420
aaaagaggcg acgagaaaga aataagattg cagctgcaaa gtgccgaaac aagaagaagg     480
agaagacgga gtgcctgcag aaagagtcgg agaagctgga aagtgtgaat gctgaactga     540
aggctcagat tgaggagctc aagaacgaga agcagcattt gatatacatg ctcaaccttc     600
atcggcccac gtgtattgtc cgggctcaga atgggaggac tccagaagat gagagaaacc     660
tctttatcca acagataaaa gaaggaacat tgcagagcta agcagtcgtg gtatgggggc     720
gactggggag tcctcattga atcctcattt tatacccaaa accctgaagc cattggagag     780
ctgtcttcct gtgtacctct agaatcccag cagcagagaa ccatcaaggc gggagggcct     840
gcagtgattc agcaggccct tcccattctg ccccagagtg ggtcttggac cagggcaagt     900
gcatctttgc ctcaactcca ggatttaggc cttaacacac tggccattct tatgttccag     960
atggccccca gctggtgtcc tgcccgcctt tcatctggat tctacaaaaa accaggatgc    1020
ccaccgttag gattcaggca gcagtgtctg tacctcgggt gggagggatg gggccatctc    1080
cttcaccgtg gctaccattg tcactcgtag ggatgtggga gtgagaacag catttagtga    1140
agttgtgcaa cggccagggt tgtgctttct agcaaatatg ctgttatgtc cagaaattgt    1200
gtgtgcaaga aaactaggca atgtactctt ccgatgtttg tgtcacacaa cactgatgtg    1260
acttttatat gctttttctc agatctggtt tctaagagtt ttgggggcg gggctgtcac     1320
cacgtgcagt atctcaagat attcaggtgg ccagaagagc ttgtcagcaa gaggaggaca    1380
gaattctccc agcgttaaca caaaatccat gggcagcatg atggcaggtc ctctgttgca    1440
aactcagttc caaagtcaca ggaagaaagc agaaagttca acttccaaag ggttaggact    1500
ctccactcaa tgtcttaggt caggagttgt gtctaggctg gaagagccaa agaatattcc    1560
attttccttt ccttgtggtt gaaaaccaca gtcagtggag agatgtttgg aaaccacagt    1620
cagtggagcc tgggtggtac ccaggcttta gcattattgg atgtcaatag cattgttttt    1680
gtcatgtagc tgttttaaga aatctggccc agggtgtttg cagctgtgag aagtcactca    1740
cactggccac aaggacgctg gctactgtct attaaaattc tgatgtttct gtgaaattct    1800
cagagtgttt aattgtactc aatggtatca ttacaatttt ctgtaagaga aaatattact    1860
tatttatcct agtattccta acctgtcaga ataataaata ttggaaccaa gacatggtaa    1920
acatg                                                                1925
```

<210> 83
<211> 2454
<212> DNA
<213> Artificial

<400> 83

```
atctgtgaga acgggatcgg cgaggaggcg acggggagga ggcgacgcgg aggggtgagg      60
ggtcctctcc gccgccaggc ggagttggga gccggacgat cccgcacgca gcgcgaggcg     120
ggaccccgga ctgactacat ttcccagtct ccgtcgctcc gcgcggccca ccccttcggc     180
tctgggcccc gcctcgtggt gccggctggt tcttcgcgct cgcccgactt cccagcggcc     240
ccgtgcggcc cgggcatgcc cagtgcgggc gcagcggccc cggccctgga agcgccccgg     300
cggagctggc ccgcggtggg ctaggggcag ggccggagcc gcggcggcgg agctgtggat     360
ccttcatgat gagagatttg gggacacttc tctctcctgt gtgtagttga tagtttggtg     420
gtgaagagat ggctgacagt gtcaaaacct ttctccagga ccttgccaga ggaatcaaag     480
actccatctg ggtatttgt accatctcaa agctagatgc tcgaatccag caaaagagag     540
```

aggagcagcg tcgaagaagg gcaagtagtg tcttggcaca gagaagagcc cagagtatag 600
agcggaagca agagagtgag ccacgtattg ttagtagaat tttccagtgt tgtgcttgga 660
atggtggagt gttctggttc agtctcctct tgttttatcg agtatttatt cctgtgcttc 720
agtcggtaac agcccgaatt atcggtgacc catcactaca tggagatgtt tggtcgtggc 780
tggaattctt cctcacgtca attttcagtg ctctttgggt gctccccttg tttgtgctta 840
gcaaagtggt gaatgccatt tggtttcagg atatagctga cctggcattt gaggtatcag 900
ggaggaagcc tcacccattc cctagtgtca gcaaaataat tgctgacatg ctcttcaacc 960
ttttgctgca ggctcttttc ctcattcagg gaatgtttgt gagtctcttt cccatccatc 1020
ttgtcggtca gctggttagt ctcctgcata tgtcccttct ctactcactg tactgctttg 1080
aatatcgttg gttcaataaa ggaattgaaa tgcaccagcg gttgtctaac atagaaagga 1140
attggcctta ctactttggg tttggtttgc ccttggcttt tctcacagca atgcagtcct 1200
catatattat cagtggctgc cttttctcta tcctctttcc tttattcatt atcagcgcca 1260
atgaagcaaa gaccctggca aagcatatct cttccagttg cgcctcttct ccttggtggt 1320
cttcttaagc aacagactct tccacaagac agtctacctg cagtcggccc tgagcagctc 1380
tacttctgca gagaagttcc cttcaccgca tccgtcgcct gccaaactga aggctactgc 1440
aggtcactga gttgcctgcc atccaaaggg gatgggcggg attggaagaa gctgtggcag 1500
ctcttttccc tgttcacctc ccgcctgcca gggaaggcag gacccgctct gccaagggcc 1560
ctctgcgtat tcccttctct ctgaggaatt gaaatttttg tctctggtgc acgtaaggca 1620
gaatgttccc tgacaccagt gtgtggattt ttaacatcac cgtgagtctg aaaggaccac 1680
aggtttttct gcagctattt tctagcattt gccagtccct gtgcctggac tgattggaac 1740
actttgtttt tctccctgtg ccatttaccc ttccaccttt ccatcctgcc ttctaccacc 1800
cttggatgaa tggattttgt aattctagct gttgtatttt gtgaatttgt taattttgtt 1860
gtttttctgt gaaacacata cattggatat gggaggtaaa ggagtgtccc agttgctcct 1920
ggtcactccc tttatagcca ttactgtctt gtttcttgta actcaggtta ggttttggtc 1980
tctcttgctc cactgcaaaa aaaaaaaaaa aaaaaaaaaa aaaaaaaaag cctgaagaga 2040
tgagatagga ggaaagacct cacagccaga tctgctgggt tttgaggagt gattttcttt 2100
cttccccttg aaggggaaaa agctattttc attggtacat ttaaagtccc ccaactatgg 2160
ggaggtacca attctggaca agtgccacta caacaacact aaacctgaac ttttcaactc 2220
cgttggtggt gggaggcagc gggcagaaat ttactgttgg ccactgccag gtctatttca 2280
tatttcaaag gaatattggg tgctgcatat aggaactgaa ggggtcaatg tattaaacct 2340
gtgattggtt gttttcctgt cattttgaga gactaaatgt ggggggcaga tgtcaaaata 2400
cctgtacaat tttaaaatgt cacaattaaa catgagctgg tttcccacaa agtg 2454

<210> 84
<211> 3460

<212> DNA
<213> Artificial

<400> 84

atctgtgaga acgggatcgg cgaggaggcg acggggagga ggcgacgcgg aggggtgagg 60
ggtcctctcc gccgccaggc ggagttggga gccggacgat cccgcacgca gcgcgaggcg 120
ggaccccgga ctgactacat ttcccagtct ccgtcgctcc gcgcggccca cccccttcggc 180
tctgggcccc gcctcgtggt gccggctggt tcttcgcgct cgcccgactt cccagcggcc 240
ccgtgcggcc cgggcatgcc cagtgcgggc gcagcggccc cggccctgga agcgccccgg 300
cggagctggc ccgcggtggg ctaggggcag ggccggagcc gcggcggcgg agctgtggat 360
ccttcatgat gagagatttg gggacacttc tctctcctgt gtgtagttga tagtttggtg 420
gtgaagagat ggctgacagt gtcaaaacct ttctccagga ccttgccaga ggaatcaaag 480
actccatctg gggtatttgt accatctcaa agctagatgc tcgaatccag caaaagagag 540
aggagcagcg tcgaagaagg gcaagtagtg tcttggcaca gagaagagcc cagagtatag 600
agcggaagca agagagtgag ccacgtattg ttagtagaat tttccagtgt tgtgcttgga 660
atggtggagt gttctggttc agtctcctct tgttttatcg agtatttatt cctgtgcttc 720
agtcggtaac agcccgaatt atcggtgacc catcactaca tggagatgtt tggtcgtggc 780
tggaattctt cctcacgtca attttcagtg ctctttgggt gctccccttg tttgtgctta 840
gcaaagtggt gaatgccatt tggtttcagg atatagctga cctggcattt gaggtatcag 900
ggaggaagcc tcacccattc cctagtgtca gcaaaataat tgctgacatg ctcttcaacc 960
ttttgctgca ggctcttttc ctcattcagg gaatgtttgt gagtctcttt cccatccatc 1020
ttgtcggtca gctggttagt ctcctgcata tgtcccttct ctactcactg tactgctttg 1080
aatatcgttg gttcaataaa ggtaagtcca tctaaagaaa tccagaaaat ggaggcccag 1140
tttggaaatg ctactgctaa gcagacttct taaactactc atagtcaggc ttttggcaaa 1200
caatcctttg agggaagcag cctcattggc aagagggagc ttaccttcgg cttagtgggt 1260
ttaactctca actttgaggc tgggggatgt ggtagtatgg cattatcttt ttggtgctta 1320
acatggtata tctctgactg ggttgtaaga tggcataaat gagcatgagt tgctctaaat 1380
tagagaagtc aaattgatgt cagcatctgg atccttttgg gggtgtttta gaaagacctt 1440
cagatattta ccagggtgtg cgtggttgaa agaggttaac tgatacttac aggatggcat 1500
ccttttgatt tgctatcttg cttagttcca gactaatcct gacaaaggat gctggtgctg 1560
aaattcttaa ttcacttagc ctgtcagctt tgaaattacg attatagaat tctaagaaac 1620
tttgcatgct ttatatcaga tttgtacact tctaatttat atgctgtagc tctgttctaa 1680
atctgtacct aggctgggtg tggtggctca cacctgtaat cccaacactt tgggaggcca 1740
aggtgggtgg atcgcctgag gtcaggagtt caagaccagc ttggccaata tggtgaaacc 1800
ctgtctctac taaaaataca aaaattagct gggcgaggtg gcaggcgcct gtaatcccag 1860
ctacttggga ggctgaggtg ggagaatcgc ttgaaaccag gaggtggagg ttgcagtgag 1920

ctgagattgc accaccgcac tcccgcctgg gcatcaaagc aagactccat ctcaaataat 1980
aataataata ataataataa taataataaa atctgtacct aaaactcatg gtttgtctac 2040
catagttact tagtcacttg actaaaagga aatataatat ctaattgtat tggctttctt 2100
gttttaggaa ttgaaatgca ccagcggttg tctaacatag aaaggaattg gccttactac 2160
tttgggtttg gtttgccctt ggcttttctc acagcaatgc agtcctcata tattatcagt 2220
ggctgccttt tctctatcct ctttccttta ttcattatca gcgccaatga agcaaagacc 2280
ctggcaaagc atatctcttc cagttgcgcc tcttctcctt ggtggtcttc ttaagcaaca 2340
gactcttcca caagacagtc tacctgcagt cggccctgag cagctctact tctgcagaga 2400
agttcccttc accgcatccg tcgcctgcca aactgaaggc tactgcaggt cactgagttg 2460
cctgccatcc aaaggggatg ggcgggattg gaagaagctg tggcagctct tttccctgtt 2520
cacctcccgc ctgccaggga aggcaggacc cgctctgcca agggccctct gcgtattccc 2580
ttctctctga ggaattgaaa tttttgtctc tggtgcacgt aaggcagaat gttccctgac 2640
accagtgtgt ggatttttaa catcaccgtg agtctgaaag gaccacaggt ttttctgcag 2700
ctattttcta gcatttgcca gtccctgtgc ctggactgat tggaacactt tgtttttctc 2760
cctgtgccat ttacccttcc acctttccat cctgccttct accacccttg gatgaatgga 2820
ttttgtaatt ctagctgttg tattttgtga atttgttaat tttgttgttt ttctgtgaaa 2880
cacatacatt ggatatggga ggtaaaggag tgtcccagtt gctcctggtc actcccttta 2940
tagccattac tgtcttgttt cttgtaactc aggttaggtt ttggtctctc ttgctccact 3000
gcaaaaaaaa aaaaaaaaaa aaaaaaaaaa aaaaagcctg aagagatgag ataggaggaa 3060
agacctcaca gccagatctg ctgggttttg aggagtgatt ttctttcttc cccttgaagg 3120
ggaaaaagct attttcattg gtacatttaa agtcccccaa ctatggggag gtaccaattc 3180
tggacaagtg ccactacaac aacactaaac ctgaactttt caactccgtt ggtggtggga 3240
ggcagcgggc agaaatttac tgttggccac tgccaggtct atttcatatt tcaaaggaat 3300
attgggtgct gcatatagga actgaagggg tcaatgtatt aaacctgtga ttggttgttt 3360
tcctgtcatt ttgagagact aaatgtgggg ggcagatgtc aaaatacctg tacaatttta 3420
aaatgtcaca attaaacatg agctggtttc ccacaaagtg 3460

<210> 85
<211> 2560
<212> DNA
<213> Artificial

<400> 85

atctgtgaga acgggatcgg cgaggaggcg acggggagga ggcgacgcgg aggggtgagg 60
ggtcctctcc gccgccaggc ggagttggga gccggacgat cccgcacgca gcgcgaggcg 120
ggaccccgga ctgactacat ttcccagtct ccgtcgctcc gcgcggccca ccccttcggc 180
tctgggcccc gcctcgtggt gccggctggt tcttcgcgct cgcccgactt cccagcggcc 240

ccgtgcggcc cgggcatgcc cagtgcgggc gcagcggccc cggccctgga agcgccccgg 300
cggagctggc ccgcggtggg ctaggggcag ggccggagcc gcggcggcgg agctgtggtt 360
gtgcctaggg agaccagttt tctgccgaat tgcctaggag ctgtggggag atgggcctga 420
atggacccgt ggactcagag gcagcccgtg gagccccgtg ggaaatcctt catgatgaga 480
gatttgggga cacttctctc tcctgtgtgt agttgatagt ttggtggtga agagatggct 540
gacagtgtca aaacctttct ccaggacctt gccagaggaa tcaaagactc catctggggt 600
atttgtacca tctcaaagct agatgctcga atccagcaaa agagagagga gcagcgtcga 660
agaagggcaa gtagtgtctt ggcacagaga agagcccaga gtatagagcg gaagcaagag 720
agtgagccac gtattgttag tagaattttc cagtgttgtg cttggaatgg tggagtgttc 780
tggttcagtc tcctcttgtt ttatcgagta tttattcctg tgcttcagtc ggtaacagcc 840
cgaattatcg gtgacccatc actacatgga gatgtttggt cgtggctgga attcttcctc 900
acgtcaattt tcagtgctct ttgggtgctc cccttgtttg tgcttagcaa agtggtgaat 960
gccatttggt ttcaggatat agctgacctg gcatttgagg tatcagggag gaagcctcac 1020
ccattcccta gtgtcagcaa aataattgct gacatgctct tcaacctttt gctgcaggct 1080
cttttcctca ttcagggaat gtttgtgagt ctctttccca tccatcttgt cggtcagctg 1140
gttagtctcc tgcatatgtc ccttctctac tcactgtact gctttgaata tcgttggttc 1200
aataaaggaa ttgaaatgca ccagcggttg tctaacatag aaaggaattg gccttactac 1260
tttgggtttg gtttgccctt ggcttttctc acagcaatgc agtcctcata tattatcagt 1320
ggctgccttt tctctatcct ctttccttta ttcattatca gcgccaatga agcaaagacc 1380
ctggcaaagc atatctcttc cagttgcgcc tcttctcctt ggtggtcttc ttaagcaaca 1440
gactcttcca caagacagtc tacctgcagt cggccctgag cagctctact tctgcagaga 1500
agttcccttc accgcatccg tcgcctgcca aactgaaggc tactgcaggt cactgagttg 1560
cctgccatcc aaaggggatg ggcgggattg gaagaagctg tggcagctct tttccctgtt 1620
cacctcccgc ctgccaggga aggcaggacc cgctctgcca agggccctct gcgtattccc 1680
ttctctctga ggaattgaaa tttttgtctc tggtgcacgt aaggcagaat gttccctgac 1740
accagtgtgt ggatttttaa catcaccgtg agtctgaaag gaccacaggt ttttctgcag 1800
ctattttcta gcatttgcca gtccctgtgc ctggactgat tggaacactt tgtttttctc 1860
cctgtgccat ttacccttcc acctttccat cctgccttct accacccttg gatgaatgga 1920
ttttgtaatt ctagctgttg tattttgtga atttgttaat tttgttgttt ttctgtgaaa 1980
cacatacatt ggatatggga ggtaaaggag tgtcccagtt gctcctggtc actcccttta 2040
tagccattac tgtcttgttt cttgtaactc aggttaggtt ttggtctctc ttgctccact 2100
gcaaaaaaaa aaaaaaaaaa aaaaaaaaaa aaaaagcctg aagagatgag ataggaggaa 2160
agacctcaca gccagatctg ctgggttttg aggagtgatt ttctttcttc cccttgaagg 2220
ggaaaaagct attttcattg gtacatttaa agtcccccaa ctatggggag gtaccaattc 2280

tggacaagtg ccactacaac aacactaaac ctgaactttt caactccgtt ggtggtggga 2340
ggcagcgggc agaaatttac tgttggccac tgccaggtct atttcatatt tcaaaggaat 2400
attgggtgct gcatatagga actgaagggg tcaatgtatt aaacctgtga ttggttgttt 2460
tcctgtcatt ttgagagact aaatgtgggg ggcagatgtc aaaatacctg tacaatttta 2520
aaatgtcaca attaaacatg agctggtttc ccacaaagtg 2560

<210> 86
<211> 2179
<212> DNA
<213> Artificial

<400> 86

```
ggctaccggc gaggacccct tatgggaagg ggccggaggg agcgggacgg ggccgggcac      60
atccccgggc gaggctgccc cagatccttc atgatgagag atttggggac acttctctct     120
cctgtgtgta gttgatagtt tggtggtgaa gagatggctg acagtgtcaa aacctttctc     180
caggaccttg ccagaggaat caaagactcc atctggggta tttgtaccat ctcaaagcta     240
gatgctcgaa tccagcaaaa gagagaggag cagcgtcgaa gaagggcaag tagtgtcttg     300
gcacagagaa gagcccagag tatagagcgg aagcaagaga gtgagccacg tattgttagt     360
agaattttcc agtgttgtgc ttggaatggt ggagtgttct ggttcagtct cctcttgttt     420
tatcgagtat ttattcctgt gcttcagtcg gtaacagccc gaattatcgg tgacccatca     480
ctacatggag atgtttggtc gtggctggaa ttcttcctca cgtcaatttt cagtgctctt     540
tgggtgctcc ccttgtttgt gcttagcaaa gtggtgaatg ccatttggtt tcaggatata     600
gctgacctgg catttgaggt atcagggagg aagcctcacc cattccctag tgtcagcaaa     660
ataattgctg acatgctctt caacctttg ctgcaggctc ttttcctcat tcagggaatg      720
tttgtgagtc tctttcccat ccatcttgtc ggtcagctgg ttagtctcct gcatatgtcc     780
cttctctact cactgtactg ctttgaatat cgttggttca ataaaggaat tgaaatgcac     840
cagcggttgt ctaacataga aaggaattgg ccttactact ttgggtttgg tttgcccttg     900
gcttttctca cagcaatgca gtcctcatat attatcagtg gctgcctttt ctctatcctc     960
tttcctttat tcattatcag cgccaatgaa gcaaagaccc tggcaaagca tatctcttcc    1020
agttgcgcct cttctccttg gtggtcttct taagcaacag actcttccac aagacagtct    1080
acctgcagtc ggccctgagc agctctactt ctgcagagaa gttcccttca ccgcatccgt    1140
cgcctgccaa actgaaggct actgcaggtc actgagttgc ctgccatcca aaggggatgg    1200
gcgggattgg aagaagctgt ggcagctctt ttccctgttc acctcccgcc tgccagggaa    1260
ggcaggaccc gctctgccaa gggccctctg cgtattccct tctctctgag gaattgaaat    1320
ttttgtctct ggtgcacgta aggcagaatg ttccctgaca ccagtgtgtg gatttttaac    1380
atcaccgtga gtctgaaagg accacaggtt tttctgcagc tattttctag catttgccag    1440
tccctgtgcc tggactgatt ggaacacttt gtttttctcc ctgtgccatt taccctttcca   1500
```

cctttccatc ctgccttcta ccacccttgg atgaatggat tttgtaattc tagctgttgt 1560
attttgtgaa tttgttaatt ttgttgtttt tctgtgaaac acatacattg gatatgggag 1620
gtaaaggagt gtcccagttg ctcctggtca ctccctttat agccattact gtcttgtttc 1680
ttgtaactca ggttaggttt tggtctctct tgctccactg caaaaaaaaa aaaaaaaaaa 1740
aaaaaaaaaa aaaagcctga agagatgaga taggaggaaa gacctcacag ccagatctgc 1800
tgggttttga ggagtgattt tctttcttcc ccttgaaggg gaaaaagcta ttttcattgg 1860
tacatttaaa gtcccccaac tatggggagg taccaattct ggacaagtgc cactacaaca 1920
acactaaacc tgaacttttc aactccgttg gtggtgggag gcagcgggca gaaatttact 1980
gttggccact gccaggtcta tttcatattt caaaggaata ttgggtgctg catataggaa 2040
ctgaaggggt caatgtatta aacctgtgat tggttgtttt cctgtcattt tgagagacta 2100
aatgtggggg gcagatgtca aaatacctgt acaattttaa aatgtcacaa ttaaacatga 2160
gctggtttcc cacaaagtg 2179

<210> 87
<211> 2583
<212> DNA
<213> Artificial

<400> 87

```
atctgtgaga acgggatcgg cgaggaggcg acggggagga ggcgacgcgg aggggtgagg     60
ggtcctctcc gccgccaggc ggagttggga gccggacgat cccgcacgca gcgcgaggcg    120
ggaccccgga ctgactacat ttcccagtct ccgtcgctcc gcgcggccca ccccttcggc    180
tctgggcccc gcctcgtggt gccggctggt tcttcgcgct cgcccgactt cccagcggcc    240
ccgtgcggcc cgggcatgcc cagtgcgggc gcagcggccc cggccctgga agcgccccgg    300
cggagctggc ccgcggtggg ctaggggcag ggccggagcc gcggcggcgg agctgtggat    360
ccttcatgat gagagatttg gggacacttc tctctcctgt gtgtagttga tagtttggtg    420
gtgaagagat ggctgacagt gtcaaaacct ttctccagga ccttgccaga ggaatcaaag    480
actccatctg gggtatttgt accatctcaa agctagatgc tcgaatccag caaaagagag    540
aggagcagcg tcgaagaagg gcaagtagtg tcttggcaca gagaagagcc cagagtatag    600
agcggaagca agagagacgg attcttgccc tgtcaccaag ctgtcacctg agattgccgt    660
ggtgcaatct cagcttactg cagcctctgc ctcccaggtc caagcaattc ttctgcctca    720
gcgaaccggc tggctggaag tacagtgagc cacgtattgt tagtagaatt ttccagtgtt    780
gtgcttggaa tggtggagtg ttctggttca gtctcctctt gttttatcga gtatttattc    840
ctgtgcttca gtcggtaaca gcccgaatta tcggtgaccc atcactacat ggagatgttt    900
ggtcgtggct ggaattcttc ctcacgtcaa ttttcagtgc tctttgggtg ctccccttgt    960
ttgtgcttag caaagtggtg aatgccattt ggtttcagga tatagctgac ctggcatttg   1020
aggtatcagg gaggaagcct cacccattcc ctagtgtcag caaaataatt gctgacatgc   1080
```

tcttcaacct tttgctgcag gctcttttcc tcattcaggg aatgtttgtg agtctctttc 1140
ccatccatct tgtcggtcag ctggttagtc tcctgcatat gtcccttctc tactcactgt 1200
actgctttga atatcgttgg ttcaataaag gaattgaaat gcaccagcgg ttgtctaaca 1260
tagaaaggaa ttggccttac tactttgggt ttggtttgcc cttggctttt ctcacagcaa 1320
tgcagtcctc atatattatc agtggctgcc ttttctctat cctctttcct ttattcatta 1380
tcagcgccaa tgaagcaaag accctggcaa agcatatctc ttccagttgc gcctcttctc 1440
cttggtggtc ttcttaagca acagactctt ccacaagaca gtctacctgc agtcggccct 1500
gagcagctct acttctgcag agaagttccc ttcaccgcat ccgtcgcctg ccaaactgaa 1560
ggctactgca ggtcactgag ttgcctgcca tccaaagggg atgggcggga ttggaagaag 1620
ctgtggcagc tcttttccct gttcacctcc cgcctgccag ggaaggcagg acccgctctg 1680
ccaagggccc tctgcgtatt cccttctctc tgaggaattg aaatttttgt ctctggtgca 1740
cgtaaggcag aatgttccct gacaccagtg tgtggatttt taacatcacc gtgagtctga 1800
aaggaccaca ggtttttctg cagctatttt ctagcatttg ccagtccctg tgcctggact 1860
gattggaaca ctttgttttt ctccctgtgc catttaccct tccacctttc catcctgcct 1920
tctaccaccc ttggatgaat ggattttgta attctagctg ttgtattttg tgaatttgtt 1980
aattttgttg tttttctgtg aaacacatac attggatatg ggaggtaaag gagtgtccca 2040
gttgctcctg gtcactccct ttatagccat tactgtcttg tttcttgtaa ctcaggttag 2100
gttttggtct ctcttgctcc actgcaaaaa aaaaaaaaaa aaaaaaaaaa aaaaaaaagc 2160
ctgaagagat gagataggag gaaagacctc acagccagat ctgctgggtt ttgaggagtg 2220
attttctttc ttccccttga aggggaaaaa gctattttca ttggtacatt taaagtcccc 2280
caactatggg gaggtaccaa ttctggacaa gtgccactac aacaacacta aacctgaact 2340
tttcaactcc gttggtggtg ggaggcagcg ggcagaaatt tactgttggc cactgccagg 2400
tctatttcat atttcaaagg aatattgggt gctgcatata ggaactgaag gggtcaatgt 2460
attaaacctg tgattggttg ttttcctgtc attttgagag actaaatgtg gggggcagat 2520
gtcaaaatac ctgtacaatt ttaaaatgtc acaattaaac atgagctggt ttcccacaaa 2580
gtg 2583

<210> 88
<211> 2906
<212> DNA
<213> Artificial

<400> 88

```
atctgtgaga acgggatcgg cgaggaggcg acggggagga ggcgacgcgg aggggtgagg     60
ggtcctctcc gccgccaggc ggagttggga gccggacgat cccgcacgca gcgcgaggcg    120
ggaccccgga ctgactacat ttcccagtct ccgtcgctcc gcgcggccca ccccttcggc    180
tctgggcccc gcctcgtggt gccggctggt tcttcgcgct cgcccgactt cccagcggcc    240
```

ccgtgcggcc cgggcatgcc cagtgcgggc gcagcggccc cggccctgga agcgccccgg 300
cggagctggc ccgcggtggg ctaggggcag ggccggagcc gcggcggcgg agctgtggat 360
ccttcatgat gagagatttg gggacacttc tctctcctgt gtgtagttga tagtttggtg 420
gtgaagagat ggctgacagt gtcaaaacct ttctccagga ccttgccaga ggaatcaaag 480
actccatctg gggtatttgt accatctcaa agctagatgc tcgaatccag caaaagagag 540
aggagcagcg tcgaagaagg gcaagtagtg tcttggcaca gagaagagcc cagagtatag 600
agcggaagca agagagacgg attcttgccc tgtcaccaag ctgtcacctg agattgccgt 660
ggtgcaatct cagcttactg cagcctctgc ctcccaggtc caagcaattc ttctgcctca 720
gcgaaccggc tggctggaag tacaggtgcg caccaccacg cctggctaat tttttatatt 780
ctttagtgga gacagggttt tgccatgttg gccaggctgg tctcgaactc ctgacctcag 840
ttgatcgcct accacggcct cccaaagtgt taggattaca gccgtgagcc actgcgcttg 900
accatttttt tagtcttttc tcattgactg acattagaac attgggagaa catgaggcac 960
tgaaaaataa tacagtaccc acaaaaggta agatataaaa tgaactactg aatgacaatt 1020
aggaaataac ttatgcttta taaactaaat acatgtgatc ttttccagtg agccacgtat 1080
tgttagtaga attttccagt gttgtgcttg gaatggtgga gtgttctggt tcagtctcct 1140
cttgttttat cgagtattta ttcctgtgct tcagtcggta acagcccgaa ttatcggtga 1200
cccatcacta catggagatg tttggtcgtg gctggaattc ttcctcacgt caattttcag 1260
tgctctttgg gtgctcccct tgtttgtgct tagcaaagtg gtgaatgcca tttggtttca 1320
ggatatagct gacctggcat ttgaggtatc agggaggaag cctcacccat tccctagtgt 1380
cagcaaaata attgctgaca tgctcttcaa ccttttgctg caggctcttt tcctcattca 1440
gggaatgttt gtgagtctct ttcccatcca tcttgtcggt cagctggtta gtctcctgca 1500
tatgtccctt ctctactcac tgtactgctt tgaatatcgt tggttcaata aaggaattga 1560
aatgcaccag cggttgtcta acatagaaag gaattggcct tactactttg ggtttggttt 1620
gcccttggct tttctcacag caatgcagtc ctcatatatt atcagtggct gccttttctc 1680
tatcctcttt cctttattca ttatcagcgc caatgaagca aagaccctgg caaagcatat 1740
ctcttccagt tgcgcctctt ctccttggtg gtcttcttaa gcaacagact cttccacaag 1800
acagtctacc tgcagtcggc cctgagcagc tctacttctg cagagaagtt cccttcaccg 1860
catccgtcgc ctgccaaact gaaggctact gcaggtcact gagttgcctg ccatccaaag 1920
gggatgggcg ggattggaag aagctgtggc agctcttttc cctgttcacc tcccgcctgc 1980
cagggaaggc aggacccgct ctgccaaggg ccctctgcgt attcccttct ctctgaggaa 2040
ttgaaatttt tgtctctggt gcacgtaagg cagaatgttc cctgacacca gtgtgtggat 2100
ttttaacatc accgtgagtc tgaaaggacc acaggttttt ctgcagctat tttctagcat 2160
ttgccagtcc ctgtgcctgg actgattgga acactttgtt tttctccctg tgccatttac 2220
ccttccacct ttccatcctg ccttctacca cccttggatg aatggatttt gtaattctag 2280

ctgttgtatt ttgtgaattt gttaattttg ttgtttttct gtgaaacaca tacattggat 2340
atgggaggta aaggagtgtc ccagttgctc ctggtcactc cctttatagc cattactgtc 2400
ttgtttcttg taactcaggt taggttttgg tctctcttgc tccactgcaa aaaaaaaaaa 2460
aaaaaaaaaa aaaaaaaaaa agcctgaaga gatgagatag gaggaaagac ctcacagcca 2520
gatctgctgg gttttgagga gtgattttct ttcttcccct tgaaggggaa aaagctattt 2580
tcattggtac atttaaagtc ccccaactat ggggaggtac caattctgga caagtgccac 2640
tacaacaaca ctaaacctga acttttcaac tccgttggtg gtgggaggca gcgggcagaa 2700
atttactgtt ggccactgcc aggtctattt catatttcaa aggaatattg ggtgctgcat 2760
ataggaactg aaggggtcaa tgtattaaac ctgtgattgg ttgttttcct gtcattttga 2820
gagactaaat gtgggggca gatgtcaaaa tacctgtaca attttaaaat gtcacaatta 2880
aacatgagct ggtttcccac aaagtg 2906

<210> 89
<211> 3016
<212> DNA
<213> Artificial

<400> 89

```
atctgtgaga acgggatcgg cgaggaggcg acggggagga ggcgacgcgg aggggtgagg      60
ggtcctctcc gccgccaggc ggagttggga gccggacgat cccgcacgca gcgcgaggcg     120
ggaccccgga ctgactacat ttcccagtct ccgtcgctcc gcgcggccca ccccttcggc     180
tctgggcccc gcctcgtggt gccggctggt tcttcgcgct cgcccgactt cccagcggcc     240
ccgtgcggcc cgggcatgcc cagtgcgggc gcagcggccc cggccctgga agcgccccgg     300
cggagctggc ccgcggtggg ctaggggcag ggccggagcc gcggcggcgg agctgtggat     360
ccttcatgat gagagatttg gggacacttc tctctcctgt gtgtagttga tagtttggtg     420
gtgaagagat ggctgacagt gtcaaaacct ttctccagga ccttgccaga ggaatcaaag     480
actccatctg ggtatttgt accatctcaa agctagatgc tcgaatccag caaaagagag      540
aggagcagcg tcgaagaagg gcaagtagtg tcttggcaca gagaagagcc cagagtatag     600
agcggaagca agagagtgag ccacgtattg ttagtagaat tttccagtgt tgtgcttgga     660
atggtggagt gttctggttc agtctcctct tgttttatcg agtatttatt cctgtgcttc     720
agtcggtaac agcccgaatt atcggtaagt gtataccctg ctccttgtct gttggggaca     780
gagtgggaga tgatgtttca gtctccctca gtttactgaa catttcctgt gtaccaggag     840
ctttcactgt ttattttctt ttttgttgtt ttattctttt taaaaattta tatttcgtta     900
ttctcctaga ggccctttaa gaatgggtac tgttgttcta tccattttat gaatgaggaa     960
atgcaagtta ccaaggttaa accattagta agaggcaaag ctgagattta aacccagatg    1020
gtatcctgca gatagcgtac tggtaatcac tgtggcatct cgttaccatc atgtaagcag    1080
gtttgtgtca agcagcacat atctatggtt tctttgaatt ggaattgtgg ccagaatgtg    1140
```

gtgcgtctat tatgagttag actctttgag ataggtatag tgatgtgctg gcaagaacta 1200
gaaagatcca ggaggagaca gagaatagtc acgagatggg ggttccgcat ttggtcttct 1260
ggatttccat ttctccattt ctagtaactc gtttcctctt tcttaggtga cccatcacta 1320
catggagatg tttggtcgtg gctggaattc ttcctcacgt caattttcag tgctctttgg 1380
gtgctcccct tgtttgtgct tagcaaagtg gtgaatgcca tttggtttca ggatatagct 1440
gacctggcat ttgaggtatc agggaggaag cctcacccat tccctagtgt cagcaaaata 1500
attgctgaca tgctcttcaa ccttttgctg caggctcttt tcctcattca gggaatgttt 1560
gtgagtctct ttcccatcca tcttgtcggt cagctggtta gtctcctgca tatgtccctt 1620
ctctactcac tgtactgctt tgaatatcgt tggttcaata aaggaattga aatgcaccag 1680
cggttgtcta acatagaaag gaattggcct tactactttg ggtttggttt gcccttggct 1740
tttctcacag caatgcagtc ctcatatatt atcagtggct gccttttctc tatcctcttt 1800
cctttattca ttatcagcgc caatgaagca aagaccctgg caaagcatat ctcttccagt 1860
tgcgcctctt ctccttggtg gtcttcttaa gcaacagact cttccacaag acagtctacc 1920
tgcagtcggc cctgagcagc tctacttctg cagagaagtt cccttcaccg catccgtcgc 1980
ctgccaaact gaaggctact gcaggtcact gagttgcctg ccatccaaag gggatgggcg 2040
ggattggaag aagctgtggc agctcttttc cctgttcacc tcccgcctgc cagggaaggc 2100
aggacccgct ctgccaaggg ccctctgcgt attcccttct ctctgaggaa ttgaaatttt 2160
tgtctctggt gcacgtaagg cagaatgttc cctgacacca gtgtgtggat ttttaacatc 2220
accgtgagtc tgaaaggacc acaggttttt ctgcagctat tttctagcat ttgccagtcc 2280
ctgtgcctgg actgattgga acactttgtt tttctccctg tgccatttac ccttccacct 2340
ttccatcctg ccttctacca cccttggatg aatggatttt gtaattctag ctgttgtatt 2400
ttgtgaattt gttaattttg ttgtttttct gtgaaacaca tacattggat atgggaggta 2460
aaggagtgtc ccagttgctc ctggtcactc cctttatagc cattactgtc ttgtttcttg 2520
taactcaggt taggttttgg tctctcttgc tccactgcaa aaaaaaaaaa aaaaaaaaaa 2580
aaaaaaaaaa agcctgaaga gatgagatag gaggaaagac ctcacagcca gatctgctgg 2640
gttttgagga gtgattttct ttcttcccct tgaaggggaa aaagctattt tcattggtac 2700
atttaaagtc ccccaactat ggggaggtac caattctgga caagtgccac tacaacaaca 2760
ctaaacctga acttttcaac tccgttggtg gtgggaggca gcgggcagaa atttactgtt 2820
ggccactgcc aggtctattt catatttcaa aggaatattg ggtgctgcat ataggaactg 2880
aaggggtcaa tgtattaaac ctgtgattgg ttgttttcct gtcattttga gagactaaat 2940
gtggggggca gatgtcaaaa tacctgtaca attttaaaat gtcacaatta aacatgagct 3000
ggtttcccac aaagtg 3016

<210> 90
<211> 2665
<212> DNA

<213> Artificial

<400> 90

```
aattctcgcg agaagtagag ttgcggttcc ctcgggagag gggcggaggc tcagaggtgc      60
accacgtggg gtttgctgcc ggagcggagt ctccggccgg cgtccagttt gagtctaggt     120
tggagttgga accgtggaga tgcggaagga aaccccaccc cccctagtgc ccccggcggc     180
ccgggagtgg aatcttcccc caaatgcgcc cgcctgcatg gaacggcagt tggaggctgc     240
gcggtaccgg tccgatgggg cgcttctcct cggggcctcc agcctgagtg ggcgctgctg     300
ggccggctcc ctctggcttt ttaaggaccc ctgtgccgcc cccaacgaag gcttctgctc     360
cgccggagtc caaacggagg ctggagtggc tgacctcact tgggttgggg agagaggtat     420
tctagtggcc tccgattcag gtgctgttga attgtgggaa ctagatgaga atgagacact     480
tattgtcagc aagttctgca agtatgagca tgatgacatt gtgtctacag tcagtgtctt     540
gagctctggc acacaagctg tcagtggtag caaagacatc tgcatcaagg tttgggacct     600
tgctcagcag gtggtactga gttcataccg agctcatgct gctcaggtca cttgtgttgc     660
tgcctctcct cacaaggact ctgtgtttct ttcatgcagc gaggacaata gaattttact     720
ctgggatacc cgctgtccca agccagcatc acagattggc tgcagtgcgc ctggctacct     780
tcctacctcg ctggcttggc atcctcagca aagtgaagtc tttgtctttg gtgatgagaa     840
tgggacagtc tcccttgtgg acaccaagag tacaagctgt gtcctgagct cagctgtaca     900
ctcccagtgt gtcactgggc tggtgttctc cccacacagt gttcccttcc tggcctctct     960
cagtgaagac tgctcacttg ctgtgctgga ctcaagcctt tctgagttgt aagtgtgaag    1020
tggaatcctt tgggaatcag ggaaggggag cagtgggtgg gcgagtcatt ttcctcttca    1080
gaaaactgtg ttgtgctttt ggagaacaga ttctccatct gtatccgaag ggacttggta    1140
atggaagcag gctggctgtt gaccctgtcc attcctgggg cacattcaca accccaagga    1200
caagccgtgt ccttggtaga ggggggtggt aggtgaaatt tttaagtcta gccctccagc    1260
gctctcccac aagggggcgg cagtctaccc tctcacataa agaactgcgt atgaaatgca    1320
gcctgctaca gggtaaaagg cagcacctgg aagacctgaa ttgtacttcc ctccttgccg    1380
tgatttgctg cctgttactg cccctcatca ctgtcttgtt ctatccagca tttcttcatc    1440
tatgaaaata gaagactgga ttggatgact gatgacatcc actgtggata gctacactgc    1500
tatcctggcg atgaggccat acagttggtg aaatgtttag aagccaagcc cacagagact    1560
ttgtgagaga tgcgacttgg tccccgctca atcactccct gcttaccaca gtgggctggg    1620
accatcaggt cgtccaccac gttgtgccca cagaacctct cccagcccct ggacctgcaa    1680
gtgttactga gtagattgga tttaagacaa aaagcaagtc ccccatgagt gtccacttct    1740
ttgccctgcc ctctcagctt gtgagacaac acaggagcct tctatagtat gttgatatgc    1800
tagatctgtg ccgttaatag gcatcgtctc tcagcctgag ggaggctgga ttctgggttc    1860
ctgtagtcac agggaggaaa agctttctta aaaatggaca tgtatgtgcg tgtgagtgtg    1920
```

tgtgtagatt tatagttttt ggtagtggca ggaataaaaa aaatccatcc tacatcttcc 1980
ctaagcactg cctctctctc accccccaaa acaagttgac gaaagggttt tatgtagctg 2040
tctatgagga attggccgtg tctgggtggg ttatgggatg tgggcatccc tgggttcttg 2100
gaagcagctc ttatgctact catagagatg ggattgactt tattttttta tagtgcttaa 2160
ttcaccatta tgagaaatgc ttccagtcac aaaaatgcag cccagctcac tctgaggaag 2220
aagcaggact tggtacggtt ttacacaact ccttaccatt aaactgaatc agaaatccat 2280
tttctggctg aataaaaagt ttggcttgcc tgtgtaatgc ccactccctt ccccctggct 2340
ccctagtgat gggacatata tgagagagaa gtgtttttct atcatagaca ccatagggga 2400
aagtttgggg atgaaggaga gcttaaaggt gtttcaatta agttagaaaa ctgacacagg 2460
ctgttgagaa ttctttgcca cttttcccac cccaaaacag catggggcct gacatcttct 2520
gccctggtcc cctttctctt gatgtggaaa gtctgaatgc agtatttata gacttctaag 2580
gttttaaaat ccagtatcaa gaagaaaatc agaaatactg gttggtgaaa taaagagttt 2640
aggcattgtt ggcctgtctt ttttg 2665

<210> 91
<211> 2507
<212> DNA
<213> Artificial

<400> 91

```
aattctcgcg agaagtagag ttgcggttcc ctcgggagag gggcggaggc tcagaggtgc      60
accacgtggg gtttgctgcc ggagcggagt ctccggccgg cgtccagttt gagtctaggt     120
tggagttgga accgtggaga tgcggaagga aaccccaccc cccctagtgc ccccggcggc     180
ccgggagtgg aatcttcccc caaatgcgcc cgcctgcatg gaacggcagt tggaggctgc     240
gcggtaccgg tccgatgggg cgcttctcct cggggcctcc agcctgagtg ggcgctgctg     300
ggccggctcc ctctggcttt ttaaggaccc ctgtgccgcc cccaacgaag gcttctgctc     360
cgccggagtc caaacggagg ctggagtggc tgacctcact tgggttgggg agagaggtat     420
tctagtggcc tccgattcag gtgctgttga attgtgggaa ctagatgaga atgagacact     480
tattgtcagc aagttctgca agtatgagca tgatgacatt gtgtctacag tcagtgtctt     540
gagctctggc acacaagctg tcagtggtag caaagacatc tgcatcaagg tttgggacct     600
tgctcagcag gtggtactga gttcataccg agctcatgct gctcaggtca cttgtgttgc     660
tgcctctcct cacaaggact ctgtgtttct ttcatgcagc gaggacaata gaattttact     720
ctgggatacc cgctgtccca agccagcatc acagattggc tgcagtgcgc ctggctacct     780
tcctacctcg ctggcttggc atcctcagca aagtgaagtc tttgtctttg gtgatgagaa     840
tgggacagtc tcccttgtgg acaccaagag tacaagctgt gtcctgagct cagctgtaca     900
ctcccagtgt gtcactgggc tggtgttctc cccacacagt gttcccttcc tggcctctct     960
cagtgaagac tgctcacttg ctgtgctgga ctcaagcctt tctgagttgt ttagaagcca    1020
```

agcccacaga gactttgtga gagatgcgac ttggtccccg ctcaatcact ccctgcttac 1080
cacagtgggc tgggaccatc aggtcgtcca ccacgttgtg cccacagaac ctctcccagc 1140
ccctggacct gcaagtgtta ctgagtagat tggatttaag acaaaaagca agtcccccat 1200
gagtgtccac ttctttgccc tgccctctca gcttgtgaga caacacagga gccttctata 1260
gtatgttgat atgctagatc tgtgccgtta ataggcatcg tctctcagcc tgagggaggc 1320
tggattctgg gttcctgtag tcacagggag gaaaagcttt cttaaaaatg gacatgtatg 1380
tgcgtgtgag tgtgtgtgta gatttatagt ttttggtagt ggcaggaata aaaaaaatcc 1440
atcctacatc ttccctaagc actgcctctc tctcaccccc caaaacaagt tgacgaaagg 1500
gttttatgta gctgtctatg aggaattggc cgtgtctggg tgggttatgg gatgtgggca 1560
tccctgggtt cttggaagca gctcttatgc tactcataga gatgggattg actttatttt 1620
tttatagtgc ttaattcacc attatgagaa atgcttccag tcacaaaaat gcagcccagc 1680
tcactctgag gaagaagcag gacttggtac ggttttacac aactccttac cattaaactg 1740
aatcagaaat ccattttctg gctgaataaa aagtttggct tgcctgtgta atgcccactc 1800
ccttccccct ggctccctag tgatgggaca tatatgagag agaagtgttt ttctatcata 1860
gacaccatag gggaaagttt ggggatgaag gagagcttaa aggtgtttca attaagttag 1920
aaaactgaca caggctgttg agaattcttt gccacttttc ccaccccaaa acagcatggg 1980
gcctgacatc ttctgccctg gtcccctttc tcttgatgtg gaaagtctga atgcagtatt 2040
tatagacttc taaggtttta aaatccagta tcaagaagaa aatcagaaat actggttggt 2100
gaaataaaga gtttaggcat tgttggcctg tctttttttga agcatgtgtg ttatgtgtag 2160
ttagatatat ttcacttatg tgagtcatca tggtgttggt cttgtagccc attatttttc 2220
ctgtgcttcc ccagcttccc aaagtagcta gttagaactt aaggtaaata tttattcttg 2280
ggttggtgga gtggatattg ccagttagga gtcatggatc aattactgat tatattgaaa 2340
gtaaatataa tcaattatgt acttttgagc tttgcaggtt caatttaggt aaaaatcaca 2400
ttatgaaact gggaaagtct gaaggaatat gggcaaaata tttctcagta aagcttccat 2460
gcttcaccct tgacatgatt acccttgagt aaaacatggg aatttgt 2507

<210> 92
<211> 2198
<212> DNA
<213> Artificial

<400> 92

aattctcgcg agaagtagag ttgcggttcc ctcgggagag gggcggaggc tcagaggtgc 60
accacgtggg gtttgctgcc ggagcggagt ctccggccgg cgtccagttt gagtctaggt 120
tggagttgga accgtggaga tgcggaagga aaccccaccc cccctagtgc ccccggcggc 180
ccgggagtgg aatcttcccc caaatgcgcc cgcctgcatg gaacggcagt tggaggctgc 240
gcggtaccgg tccgatgggg cgcttctcct cggggcctcc agcctgagtg ggcgctgctg 300

ggccggctcc ctctggcttt ttaaggaccc ctgtgccgcc cccaacgaag gcttctgctc 360
cgccggagtc caaacggagg ctggagtggc tgacctcact tgggttgggg agagaggtat 420
tctagtggcc tccgattcag gtgctgttga attgtgggaa ctagatgaga atgagacact 480
tattgtcagc aagttctgca agtatgagca tgatgacatt gtgtctacag tcagtgtctt 540
gagctctggc acacaagctg tcagtggtag caaagacatc tgcatcaagg tttgggacct 600
tgctcagcag gtggtactga gttcataccg agctcatgct gctcaggtca cttgtgttgc 660
tgcctctcct cacaaggact ctgtgtttct ttcatgcagc gaggacaata gaattttact 720
ctgggatacc cgctgtccca agccagcatc acagattgtg aggctctgct aaccccattc 780
tgtgcaatga ataccaagag gaagggccag ttcctgggct gcagtgcgcc tggctacctt 840
cctacctcgc tggcttggca tcctcagcaa agtgaagtct ttgtctttgg tgatgagaat 900
gggacagtct cccttgtgga caccaagagt acaagctgtg tcctgagctc agctgtacac 960
tcccagtgtg tcactgggct ggtgttctcc ccacacagtg ttcccttcct ggcctctctc 1020
agtgaagact gctcacttgc tgtgctggac tcaagccttt ctgagttgtt tagaagccaa 1080
gcccacagag actttgtgag agatgcgact tggtccccgc tcaatcactc cctgcttacc 1140
acagtgggct gggaccatca ggtcgtccac cacgttgtgc ccacagaacc tctcccagcc 1200
cctggacctg caagtgttac tgagtagatt ggatttaaga caaaaagcaa gtcccccatg 1260
agtgtccact tctttgccct gccctctcag cttgtgagac aacacaggag ccttctatag 1320
tatgttgata tgctagatct gtgccgttaa taggcatcgt ctctcagcct gagggaggct 1380
ggattctggg ttcctgtagt cacagggagg aaaagctttc ttaaaaatgg acatgtatgt 1440
gcgtgtgagt gtgtgtgtag atttatagtt tttggtagtg gcaggaataa aaaaaatcca 1500
tcctacatct tccctaagca ctgcctctct ctcacccccc aaaacaagtt gacgaaaggg 1560
ttttatgtag ctgtctatga ggaattggcc gtgtctgggt gggttatggg atgtgggcat 1620
ccctgggttc ttggaagcag ctcttatgct actcatagag atgggattga ctttattttt 1680
ttatagtgct taattcacca ttatgagaaa tgcttccagt cacaaaaatg cagcccagct 1740
cactctgagg aagaagcagg acttggtacg gttttacaca actccttacc attaaactga 1800
atcagaaatc cattttctgg ctgaataaaa agtttggctt gcctgtgtaa tgcccactcc 1860
cttccccctg gctccctagt gatgggacat atatgagaga gaagtgtttt tctatcatag 1920
acaccatagg ggaaagtttg gggatgaagg agagcttaaa ggtgtttcaa ttaagttaga 1980
aaactgacac aggctgttga gaattctttg ccacttttcc caccccaaaa cagcatgggg 2040
cctgacatct tctgccctgg tcccctttct cttgatgtgg aaagtctgaa tgcagtattt 2100
atagacttct aaggttttaa aatccagtat caagaagaaa atcagaaata ctggttggtg 2160
aaataaagag tttaggcatt gttggcctgt cttttttg 2198

<210> 93
<211> 3984
<212> DNA

<213> Artificial

<400> 93

```
aattctcgcg agaagtagag ttgcggttcc ctcgggagag gggcggaggc tcagaggtgc     60
accacgtggg gtttgctgcc ggagcggagt ctccggccgg cgtccagttt gagtctaggt    120
tggagttgga accgtggaga tgcggaagga aaccccaccc cccctagtgc ccccggcggc    180
ccgggagtgg aatcttcccc caaatgcgcc cgcctgcatg gaacggcagt tggaggctgc    240
gcggtaccgg tccgatgggg cgcttctcct cggggcctcc agcctgagtg ggcgctgctg    300
ggccggctcc ctctggcttt ttaaggaccc ctgtgccgcc cccaacgaag gcttctgctc    360
cgccggagtc caaacggagg ctggagtggc tgacctcact tgggttgggg agagaggtat    420
tctagtggcc tccgattcag gtgctgttga attgtgggaa ctagatgaga atgagacact    480
tattgtcagc aagttctgca agtatgagca tgatgacatt gtgtctacag tcagtgtctt    540
gagctctggc acacaagctg tcagtggtag caaagacatc tgcatcaagg tttgggacct    600
tgctcagcag gtggtactga gttcataccg agctcatgct gctcaggtca cttgtgttgc    660
tgcctctcct cacaaggact ctgtgtttct ttcatgcagc gaggacaata gaattttact    720
ctgggatacc cgctgtccca agccagcatc acagattggc tgcagtgcgc ctggctacct    780
tcctacctcg ctggcttggc atcctcagca aagtgaagtc tttgtctttg gtgatgagaa    840
tgggacagtc tcccttgtgg acaccaagag tacaagctgt gtcctgagct cagctgtaca    900
ctcccagtgt gtcactgggc tggtgttctc cccacacagt gttcccttcc tggcctctct    960
cagtgaagac tgctcacttg ctgtgctgga ctcaagcctt tctgagttgt ttagaagcca   1020
agcccacaga gactttgtga gagatgcgac ttggtccccg ctcaatcact ccctgcttac   1080
cacagtgggc tgggaccatc aggtcgtcca ccacgttgtg cccacagaac ctctcccagc   1140
ccctggacct gcaagtgtta ctgagtagat tggatttaag acaaaaagca agtcccccat   1200
gagtgtccac ttctttgccc tgccctctca gcttgtgaga caacacagga gccttctata   1260
gtatgttgat atgctagatc tgtgccgtta ataggcatcg tctctcagcc tgagggaggc   1320
tggattctgg gttcctgtag tcacagggag gaaaagcttt cttaaaaatg gacatgtatg   1380
tgcgtgtgag tgtgtgtgta gatttatagt ttttggtagt ggcaggaata aaaaaaatcc   1440
atcctacatc ttccctaagc actgcctctc tctcaccccc caaaacaagt tgacgaaagg   1500
gttttatgta gctgtctatg aggaattggc cgtgtctggg tgggttatgg gatgtgggca   1560
tccctgggtt cttggaagca gctcttatgc tactcataga gatgggattg actttatttt   1620
tttatagtgc ttaattcacc attatgagaa atgcttccag tcacaaaaat gcagcccagc   1680
tcactctgag gaagaagcag gacttgctgt tctctggtcc accaagggct atgaggctca   1740
ccccttggat ggtgctcaga accacaagga atactgaaga agccttgcct atgatttcag   1800
gtggcatcag acattgggct aattttcaag gtgtctctcc ttcccaacag gacagtcgga   1860
gtctggttct ttaataaata gctatatgga tcttacttac tggttcacaa tcatattaca   1920
```

```
gcagcaaaaa cacccaagct ctaactgctc tgggcctcca gccatgtttc ctccagatag   1980
catgttagaa aggacaggag attttaaaaa tcagattgga cttcagaggg ctccaaattt   2040
tcttgccaca tctagacctg aaagtaaaga gctctgatta ttaagaggca gattctcgtt   2100
gcatttggac agcgtgagta atggtcacac tgaacacatt agaatcagtt gtgaaattga   2160
ttttgtttca aaaaaggata agcaagctgt agtctcaatt ttgagttaat tgaaatttct   2220
caagaacttc atcagcagct cacatgatac agttttggaa gagtaaacaa ggtaagcaga   2280
caaccctggg accccaaaca cattttcttt tttagttctc catcaaaaat aggcatttct   2340
ggagttttgg tctcactgag tgatcacagg actgctgcaa tccagggaac tgagggagct   2400
gtgagcatct gagaagaaag gttagctctt gttttaagtt gtagagacaa cttaaaacaa   2460
gttgtgacac aattagtgat cccataatct tttatcatct ggtacaacat ttaatatagt   2520
agcattctac ttactgtgga aactccttgt ttcacaaatg atgttctgca gcttcaatat   2580
gtaatggatg gaccatcaaa gttcaaaata taataaggaa tttaattccc aaacaacatc   2640
ctttaacatt ctggtgaaag cccagccagt gtcactggtt gaaagtgcct tcagtggata   2700
tagctatatt tcacctgtcc ctgtgatcat gaggtaccag attccggtca agcatttcac   2760
tttgggaggc accatgttgt aaacattagg aatttcaggt gcaaactaat tttccattct   2820
ggtagtaact cttacctgga gtgtcagcta agtcatgaaa attgtttaga ttagaaaatg   2880
cactttttat atatagccta aattagcttc atgttctgta gtttctgcag cttgtctcag   2940
aaatcctctg agtagaactg atgtatgaga ccctttaaga ccattatcca actgcagttt   3000
gcttggcaga gttagcatta atgcaccctt tgatctaggc acctggaggc aggtaagaca   3060
ggctctgcgt gcaccaccgt ccctggacct gttgtggcgg actccttgaa gcaagatcaa   3120
cttgggcttt gtttgagttt ttaaagtctt ttttttttaa tgtaaaattt gtcattttaa   3180
ctttttaagt atacagttca gtggcattaa gtttatttgc atttttgtgc agctttcacc   3240
acttccatct ccaaaacttt ttcatcatcc caaactgaaa ctgtacttat taaacagtaa   3300
ctccccttcc tcaagaccct ggcaatcacc attcatcttc atttctataa atttgactat   3360
tctagacata cgtgtaagtg ggagcataca atatttggtt tttttagagg ggggttctgg   3420
cttaatttta taaggtgtta acttgttcct caaaatgtgt tctataaact ggcagcattt   3480
gcatatcttg gaacttgctt gaaatacaga atgtccagat ctactgagtc agaatttaca   3540
ttttcaaaag cttcctacgt gactcatgca tattaaagtt tgggaagcac tgacttagat   3600
taccttttga gaattccaga tgggtcagaa accagacaga aatactcagt agtgagaagc   3660
tatggtgtat cagaagctgt taggcatttc atggtttggt agtgagcaag acagatagtt   3720
ttcctgtatt cagcgactta gtctagagag agacaggatg gaattaagtg tttaggtgct   3780
agccaaaagt aaagattcgt agaaaacaag ggttcatatc ccagtcatca aagtgataaa   3840
ttttccctgc ttaacattta gattaaaaag taataattag gccgggtgtg agccctgcgt   3900
ctggcccatg actatcattt ctttcttatg gggtttattg tggtcactgc ctagcttgta   3960
```

tctgattgat cagggaactg attc 3984

<210> 94
<211> 2389
<212> DNA
<213> Artificial

<400> 94

```
aattctcgcg agaagtagag ttgcggttcc ctcgggagag gggcggaggc tcagaggtgc      60
accacgtggg gtttgctgcc ggagcggagt ctccggccgg cgtccagttt gagtctaggt     120
tggagttgga accgtggaga tgcggaagga aaccccaccc cccctagtgc ccccggcggc     180
ccgggagtgg aatcttcccc caaatgcgcc cgcctgcatg gaacggcagt tggaggctgc     240
gcggtaccgg tccggtgagc cgagatcccg ggcctgggag ctcccttacc ccggcccagg     300
gcgcccctcc ccggagactg accctcccag cccatccccg ccctgcatgt tatcctgacc     360
ctgggtcgcg tcaccgagct gtccccagcc ctgccccgcg ttcctgagct cagatccaga     420
cccggccgga acgctgttct gtgcggtctc catggaggcg aacccttcta cccttcacgc     480
gctgacagct tgtacccccc gtagatgggg cgcttctcct cggggcctcc agcctgagtg     540
ggcgctgctg ggccggctcc ctctggcttt ttaaggaccc ctgtgccgcc cccaacgaag     600
gcttctgctc cgccggagtc caaacggagg ctggagtggc tgacctcact tgggttgggg     660
agagaggtat tctagtggcc tccgattcag gtgctgttga attgtgggaa ctagatgaga     720
atgagacact tattgtcagc aagttctgca agtatgagca tgatgacatt gtgtctacag     780
tcagtgtctt gagctctggc acacaagctg tcagtggtag caaagacatc tgcatcaagg     840
tttgggacct tgctcagcag gtggtactga gttcataccg agctcatgct gctcaggtca     900
cttgtgttgc tgcctctcct cacaaggact ctgtgtttct ttcatgcagc gaggacaata     960
gaattttact ctgggatacc cgctgtccca agccagcatc acagattggc tgcagtgcgc    1020
ctggctacct tcctacctcg ctggcttggc atcctcagca aagtgaagtc tttgtctttg    1080
gtgatgagaa tgggacagtc tcccttgtgg acaccaagag tacaagctgt gtcctgagct    1140
cagctgtaca ctcccagtgt gtcactgggc tggtgttctc cccacacagt gttcccttcc    1200
tggcctctct cagtgaagac tgctcacttg ctgtgctgga ctcaagcctt tctgagttgt    1260
ttagaagcca agcccacaga gactttgtga gagatgcgac ttggtccccg ctcaatcact    1320
ccctgcttac cacagtgggc tgggaccatc aggtcgtcca ccacgttgtg cccacagaac    1380
ctctcccagc ccctggacct gcaagtgtta ctgagtagat tggatttaag acaaaaagca    1440
agtcccccat gagtgtccac ttctttgccc tgccctctca gcttgtgaga caacacagga    1500
gccttctata gtatgttgat atgctagatc tgtgccgtta ataggcatcg tctctcagcc    1560
tgagggaggc tggattctgg gttcctgtag tcacagggag gaaaagcttt cttaaaaatg    1620
gacatgtatg tgcgtgtgag tgtgtgtgta gatttatagt ttttggtagt ggcaggaata    1680
aaaaaaatcc atcctacatc ttccctaagc actgcctctc tctcaccccc caaaacaagt    1740
```

tgacgaaagg gttttatgta gctgtctatg aggaattggc cgtgtctggg tgggttatgg 1800
gatgtgggca tccctgggtt cttggaagca gctcttatgc tactcataga gatgggattg 1860
actttatttt tttatagtgc ttaattcacc attatgagaa atgcttccag tcacaaaaat 1920
gcagcccagc tcactctgag gaagaagcag gacttggtac ggttttacac aactccttac 1980
cattaaactg aatcagaaat ccattttctg gctgaataaa aagtttggct tgcctgtgta 2040
atgcccactc ccttcccccct ggctccctag tgatgggaca tatatgagag agaagtgttt 2100
ttctatcata gacaccatag gggaaagttt ggggatgaag gagagcttaa aggtgtttca 2160
attaagttag aaaactgaca caggctgttg agaattcttt gccacttttc ccaccccaaa 2220
acagcatggg gcctgacatc ttctgccctg gtcccctttc tcttgatgtg gaaagtctga 2280
atgcagtatt tatagacttc taaggtttta aaatccagta tcaagaagaa aatcagaaat 2340
actggttggt gaaataaaga gtttaggcat tgttggcctg tctttttg 2389

<210> 95
<211> 2429
<212> DNA
<213> Artificial

<400> 95

```
aattctcgcg agaagtagag ttgcggttcc ctcgggagag gggcggaggc tcagaggtgc      60
accacgtggg gtttgctgcc ggagcggagt ctccggccgg cgtccagttt gagtctaggt     120
tggagttgga accgtggaga tgcggaagga aaccccaccc cccctagtgc ccccggcggc     180
ccgggagtgg aatcttcccc caaatgcgcc cgcctgcatg gaacggcagt tggaggctgc     240
gcggtaccgg tccgatgggg cgcttctcct cggggcctcc agcctgagtg ggcgctgctg     300
ggccggctcc ctctggcttt ttaaggaccc ctgtgccgcc cccaacgaag gcttctgctc     360
cgccggagtc caaacggagg ctggagtggc tgacctcact tgggttgggg agagaggtat     420
tctagtggcc tccgattcag gtgctgttga attgtgggaa ctagatgaga atgagacact     480
tattgtcagc aagttctgca agtatgagca tgatgacatt gtgtctacag tcagtgtctt     540
gagctctggc acacaagctg tcagtggtag caaagacatc tggtgggtcc ctgttctcat     600
tgctcctgtc tctaagcctc ctgtgggtgc ctttcctatc ctgttaacct cattaggtaa     660
cattttgggc ttattctaag aatccagtga cttattttag catgtctata aggtttcctt     720
agggattttg cagtccatcg taagtagacc atagcagcag tccaagaggc aaagacttgg     780
ggaatgttga ttacaggaag ttcttgtttg aacacgtttt tatgttgata gtgtaggtca     840
agattaaatg tctgtttgct tttgcttctt agcatcaagg tttgggacct tgctcagcag     900
gtggtactga gttcataccg agctcatgct gctcaggtca cttgtgttgc tgcctctcct     960
cacaaggact ctgtgtttct ttcatgcagc gaggacaata gaattttact ctgggatacc    1020
cgctgtccca agccagcatc acagattggc tgcagtgcgc ctggctacct tcctacctcg    1080
ctggcttggc atcctcagca aagtgaagtc tttgtctttg gtgatgagaa tgggacagtc    1140
```

tcccttgtgg acaccaagag tacaagctgt gtcctgagct cagctgtaca ctcccagtgt 1200
gtcactgggc tggtgttctc cccacacagt gttcccttcc tggcctctct cagtgaagac 1260
tgctcacttg ctgtgctgga ctcaagcctt tctgagttgt ttagaagcca agcccacaga 1320
gactttgtga gagatgcgac ttggtccccg ctcaatcact ccctgcttac cacagtgggc 1380
tgggaccatc aggtcgtcca ccacgttgtg cccacagaac ctctcccagc ccctggacct 1440
gcaagtgtta ctgagtagat tggatttaag acaaaaagca agtcccccat gagtgtccac 1500
ttctttgccc tgccctctca gcttgtgaga caacacagga gccttctata gtatgttgat 1560
atgctagatc tgtgccgtta ataggcatcg tctctcagcc tgagggaggc tggattctgg 1620
gttcctgtag tcacagggag gaaaagcttt cttaaaaatg gacatgtatg tgcgtgtgag 1680
tgtgtgtgta gatttatagt ttttggtagt ggcaggaata aaaaaaatcc atcctacatc 1740
ttccctaagc actgcctctc tctcaccccc caaaacaagt tgacgaaagg gttttatgta 1800
gctgtctatg aggaattggc cgtgtctggg tgggttatgg gatgtgggca tccctgggtt 1860
cttggaagca gctcttatgc tactcataga gatgggattg actttatttt tttatagtgc 1920
ttaattcacc attatgagaa atgcttccag tcacaaaaat gcagcccagc tcactctgag 1980
gaagaagcag gacttggtac ggttttacac aactccttac cattaaactg aatcagaaat 2040
ccattttctg gctgaataaa aagtttggct tgcctgtgta atgcccactc ccttcccccct 2100
ggctccctag tgatgggaca tatatgagag agaagtgttt ttctatcata gacaccatag 2160
gggaaagttt ggggatgaag gagagcttaa aggtgtttca attaagttag aaaactgaca 2220
caggctgttg agaattcttt gccacttttc ccaccccaaa acagcatggg gcctgacatc 2280
ttctgccctg gtccctttc tcttgatgtg gaaagtctga atgcagtatt tatagacttc 2340
taaggtttta aaatccagta tcaagaagaa aatcagaaat actggttggt gaaataaaga 2400
gtttaggcat tgttggcctg tctttttg 2429

<210> 96
<211> 1563
<212> DNA
<213> Artificial

<400> 96

aattctcgcg agaagtagag ttgcggttcc ctcgggagag gggcggaggc tcagaggtgc 60
accacgtggg gtttgctgcc ggagcggagt ctccggccgg cgtccagttt gagtctaggt 120
tggagttgga accgtggaga tgcggaagga aaccccaccc cccctagtgc ccccggcggc 180
ccgggagtgg aatcttcccc caaatgcgcc cgcctgcatg gaacggcagt tggaggctgc 240
gcggtaccgg tccgatgggg cgcttctcct cggggcctcc agcctgagtg ggcgctgctg 300
ggccggctcc ctctggcttt ttaaggaccc ctgtgccgcc cccaacgaag gcttctgctc 360
cgccggagtc caaacggagg ctggagtggc tgacctcact tgggttgggg agagaggtat 420
tctagtggcc tccgattcag gtgctgttga attgtgggaa ctagatgaga atgagacact 480

tattgtcagc aagttctgca agtatgagca tgatgacatt gtgtctacag tcagtgtctt 540
gagctctggc acacaagctg tcagtggtag caaagacatc tgcatcaagg tttgggacct 600
tgctcagcag gtggtactga gttcataccg agctcatgct gctcaggtca cttgtgttgc 660
tgcctctcct cacaaggact ctgtgtttct ttcatgcagc gaggacaata gaattttact 720
ctgggatacc cgctgtccca agccagcatc acagattggc tgcagtgcgc ctggctacct 780
tcctacctcg ctggcttggc atcctcagca aagtgaagtc tttgtctttg gtgatgagaa 840
tgggacagtc tcccttgtgg acaccaagag tacaagctgt gtcctgagct cagctgtaca 900
ctcccagtgt gtcactgggc tggtgttctc ccacacagt gttcccttcc tggcctctct 960
cagtgaagac tgctcacttg ctgtgctgga ctcaagcctt tctgagttca tttcttcatc 1020
tatgaaaata gaagactgga ttggatgact gatgacatcc actgtggata gctacactgc 1080
tatcctggcg atgaggccat acagttggtg aaatgtttag aagccaagcc cacagagact 1140
ttgtgagaga tgcgacttgg tccccgctca atcactccct gcttaccaca gtgggctggg 1200
accatcaggt cgtccaccac gttgtgccca cagaacctct cccagcccct ggacctgcaa 1260
gtgttactga gtagattgga tttaagacaa aaagcaagtc ccccatgagt gtccacttct 1320
ttgccctgcc ctctcagctt gtgagacaac acaggagcct tctatagtat gttgatatgc 1380
tagatctgtg ccgttaatag gcatcgtctc tcagcctgag ggaggctgga ttctgggttc 1440
ctgtagtcac agggaggaaa agctttctta aaaatggaca tgtatgtgcg tgtgagtgtg 1500
tgtgtagatt tatagttttt ggtagtggca ggaataaaaa aaatccatcc tacatcttcc 1560
cta 1563

<210> 97
<211> 2289
<212> DNA
<213> Artificial

<400> 97

aattctcgcg agaagtagag ttgcggttcc ctcgggagag gggcggaggc tcagaggtgc 60
accacgtggg gtttgctgcc ggagcggagt ctccggccgg cgtccagttt gagtctaggt 120
tggagttgga accgtggaga tgcggaagga aaccccaccc cccctagtgc ccccggcggc 180
ccgggagtgg aatcttcccc caaatgcgcc cgcctgcatg gaacggcagt tggaggctgc 240
gcggtaccgg tccgatgggg cgcttctcct cggggcctcc agcctgagtg ggcgctgctg 300
ggccggctcc ctctggcttt ttaaggaccc ctgtgccgcc cccaacgaag gcttctgctc 360
cgccggagtc caaacggagg ctggagtggc tgacctcact tgggttgggg agagaggtat 420
tctagtggcc tccgattcag gtgagtcact atcccattat ccaccccccac ccccgggtgg 480
aacggtagaa tggagacttg gggtaccgta gggttgagct caatatctaa cgtcctatac 540
tcagtgcttg gaggagcctg agagtgttgg ccccgaatgc aaactcttgt gtgctgttga 600
attgtgggaa ctagatgaga atgagacact tattgtcagc aagttctgca agtatgagca 660

tgatgacatt gtgtctacag tcagtgtctt gagctctggc acacaagctg tcagtggtag 720
caaagacatc tgcatcaagg tttgggacct tgctcagcag gtggtactga gttcataccg 780
agctcatgct gctcaggtca cttgtgttgc tgcctctcct cacaaggact ctgtgtttct 840
ttcatgcagc gaggacaata gaattttact ctgggatacc cgctgtccca agccagcatc 900
acagattggc tgcagtgcgc ctggctacct tcctacctcg ctggcttggc atcctcagca 960
aagtgaagtc tttgtctttg gtgatgagaa tgggacagtc tcccttgtgg acaccaagag 1020
tacaagctgt gtcctgagct cagctgtaca ctcccagtgt gtcactgggc tggtgttctc 1080
cccacacagt gttcccttcc tggcctctct cagtgaagac tgctcacttg ctgtgctgga 1140
ctcaagcctt tctgagttgt ttagaagcca agcccacaga gactttgtga gagatgcgac 1200
ttggtccccg ctcaatcact ccctgcttac cacagtgggc tgggaccatc aggtcgtcca 1260
ccacgttgtg cccacagaac ctctcccagc ccctggacct gcaagtgtta ctgagtagat 1320
tggatttaag acaaaaagca agtcccccat gagtgtccac ttctttgccc tgccctctca 1380
gcttgtgaga caacacagga gccttctata gtatgttgat atgctagatc tgtgccgtta 1440
ataggcatcg tctctcagcc tgagggaggc tggattctgg gttcctgtag tcacagggag 1500
gaaaagcttt cttaaaaatg gacatgtatg tgcgtgtgag tgtgtgtgta gatttatagt 1560
ttttggtagt ggcaggaata aaaaaaatcc atcctacatc ttccctaagc actgcctctc 1620
tctcaccccc caaaacaagt tgacgaaagg gttttatgta gctgtctatg aggaattggc 1680
cgtgtctggg tgggttatgg gatgtgggca tccctgggtt cttggaagca gctcttatgc 1740
tactcataga gatgggattg actttatttt tttatagtgc ttaattcacc attatgagaa 1800
atgcttccag tcacaaaaat gcagcccagc tcactctgag gaagaagcag gacttggtac 1860
ggttttacac aactccttac cattaaactg aatcagaaat ccattttctg gctgaataaa 1920
aagtttggct tgcctgtgta atgcccactc ccttccccct ggctccctag tgatgggaca 1980
tatatgagag agaagtgttt ttctatcata gacaccatag gggaaagttt ggggatgaag 2040
gagagcttaa aggtgtttca attaagttag aaaactgaca caggctgttg agaattcttt 2100
gccacttttc ccaccccaaa acagcatggg gcctgacatc ttctgccctg gtcccctttc 2160
tcttgatgtg gaaagtctga atgcagtatt tatagacttc taaggtttta aaatccagta 2220
tcaagaagaa aatcagaaat actggttggt gaaataaaga gtttaggcat tgttggcctg 2280
tctttttg 2289

<210> 98
<211> 1118
<212> DNA
<213> Artificial

<400> 98

aattctcgcg agaagtagag ttgcggttcc ctcgggagag gggcggaggc tcagaggtgc 60
accacgtggg gtttgctgcc ggagcggagt ctccggccgg cgtccagttt gagtctaggt 120
tggagttgga accgtggaga tgcggaagga aaccccaccc cccctagtgc ccccggcggc 180
ccgggagtgg aatcttcccc caaatgcgcc cgcctgcatg gaacggcagt tggaggctgc 240
gcggtaccgg tccgatgggg cgcttctcct cggggcctcc agcctgagtg ggcgctgctg 300
ggccggctcc ctctggcttt ttaaggaccc ctgtgccgcc cccaacgaag gcttctgctc 360
cgccggagtc caaacggagg ctggagtggc tgacctcact tgggttgggg agagaggtat 420
tctagtggcc tccgattcag gtgctgttga attgtgggaa ctagatgaga atgagacact 480
tattgtcagc aagttctgca agtatgagca tgatgacatt gtgtctacag tcagtgtctt 540
gagctctggc acacaagctg tcagtggtag caaagacatc tgcatcaagg tttgggacct 600
tgctcagcag gtggtactga gttcataccg agctcatgct gctcaggtca cttgtgttgc 660
tgcctctcct cacaaggact ctgtgtttct ttcatgcagc gaggacaata gaattttact 720
ctgggatacc cgctgtccca agccagcatc acagattggc tgcagtgcgc ctggctacct 780
tcctacctcg ctggcttggc atcctcagca aagtgaagtc tttgtctttg gtgatgagaa 840
tgggacagtc tcccttgtgg acaccaagag tacaagctgt gtcctgagct cagctgtaca 900
ctcccagtgt gtcactgggc tggtgttctc cccacacagt gttcccttcc tggcctctct 960
cagtgaagac tgctcacttg ctgtgctgga ctcaagcctt tctgagttgg ctggttcttg 1020
tgctgaaaca ccacgatggt gctgcccata aactcgtgtg ttatttcacc aactgggcac 1080
acagtcggcc aggccctgcc tcgatcttgc cccatgac 1118

<210> 99
<211> 2105
<212> DNA
<213> Artificial

<400> 99

```
aattctcgcg agaagtagag ttgcggttcc ctcgggagag gggcggaggc tcagaggtgc      60
accacgtggg gtttgctgcc ggagcggagt ctccggccgg cgtccagttt gagtctaggt     120
tggagttgga accgtggaga tgcggaagga aaccccaccc cccctagtgc ccccggcggc     180
ccgggagtgg aatcttcccc caaatgcgcc cgcctgcatg gaacggcgct tctcctcggg     240
gcctccagcc tgagtgggcg ctgctgggcc ggctccctct ggctttttaa ggacccctgt     300
gccgccccca acgaaggctt ctgctccgcc ggagtccaaa cggaggctgg agtggctgac     360
ctcacttggg ttggggagag aggtattcta gtggcctccg attcaggtgc tgttgaattg     420
tgggaactag atgagaatga gacacttatt gtcagcaagt tctgcaagta tgagcatgat     480
gacattgtgt ctacagtcag tgtcttgagc tctggcacac aagctgtcag tggtagcaaa     540
gacatctgca tcaaggtttg ggaccttgct cagcaggtgg tactgagttc ataccgagct     600
catgctgctc aggtcacttg tgttgctgcc tctcctcaca aggactctgt gtttctttca     660
tgcagcgagg acaatagaat tttactctgg gatacccgct gtcccaagcc agcatcacag     720
attggctgca gtgcgcctgg ctaccttcct acctcgctgg cttggcatcc tcagcaaagt     780
```

gaagtctttg tctttggtga tgagaatggg acagtctccc ttgtggacac caagagtaca 840
agctgtgtcc tgagctcagc tgtacactcc cagtgtgtca ctgggctggt gttctcccca 900
cacagtgttc ccttcctggc ctctctcagt gaagactgct cacttgctgt gctggactca 960
agcctttctg agttgtttag aagccaagcc cacagagact ttgtgagaga tgcgacttgg 1020
tccccgctca atcactccct gcttaccaca gtgggctggg accatcaggt cgtccaccac 1080
gttgtgccca cagaacctct cccagcccct ggacctgcaa gtgttactga gtagattgga 1140
tttaagacaa aaagcaagtc ccccatgagt gtccacttct ttgccctgcc ctctcagctt 1200
gtgagacaac acaggagcct tctatagtat gttgatatgc tagatctgtg ccgttaatag 1260
gcatcgtctc tcagcctgag ggaggctgga ttctgggttc ctgtagtcac agggaggaaa 1320
agctttctta aaaatggaca tgtatgtgcg tgtgagtgtg tgtgtagatt tatagttttt 1380
ggtagtggca ggaataaaaa aaatccatcc tacatcttcc ctaagcactg cctctctctc 1440
accccccaaa acaagttgac gaaagggttt tatgtagctg tctatgagga attggccgtg 1500
tctgggtggg ttatgggatg tgggcatccc tgggttcttg gaagcagctc ttatgctact 1560
catagagatg ggattgactt tattttttta tagtgcttaa ttcaccatta tgagaaatgc 1620
ttccagtcac aaaaatgcag cccagctcac tctgaggaag aagcaggact tggtacggtt 1680
ttacacaact ccttaccatt aaactgaatc agaaatccat tttctggctg aataaaaagt 1740
ttggcttgcc tgtgtaatgc ccactccctt ccccctggct ccctagtgat gggacatata 1800
tgagagagaa gtgtttttct atcatagaca ccatagggga aagtttgggg atgaaggaga 1860
gcttaaaggt gtttcaatta agttagaaaa ctgacacagg ctgttgagaa ttctttgcca 1920
cttttcccac cccaaaacag catggggcct gacatcttct gccctggtcc cctttctctt 1980
gatgtggaaa gtctgaatgc agtatttata gacttctaag gttttaaaat ccagtatcaa 2040
gaagaaaatc agaaatactg gttggtgaaa taaagagttt aggcattgtt ggcctgtctt 2100
ttttg 2105

<210> 100
<211> 1720
<212> DNA
<213> Artificial

<400> 100

agaaagcaac tagcacagcc atgcccaggc caggacccgt gacacagcga tgctctcaga 60
tgctgctgat gttacagatg ttgttggggg caccctgagg gggcaccctg actctgcagg 120
tgagaacctt ctggggttcc taggaccctc ggacaagcac tctgatccgg atgactccag 180
gtcccaggag ttgcagaaac gccaccagga ccttcagagc accagttaga caaggagagc 240
caggaggagc tgggccccaa cctcatccaa aagcacagac tcccaggctg gaatggtgtc 300
ctcatatcga ggaagaggat actgaggccc agaaatgtgc cctagcttta ctaggagcgc 360
ccccacctaa agatcctccc cctaaataca cccccagacc ccgcccagct gtggtcattg 420

Page 79

gagtgtttac tctgcaggca gggggaggag ggcgggactg agcaggcgga gacggacaaa 480
gtccggggac tataaaggcc ggtccggcag catctggtca gtcccagctc agagccgcaa 540
cctgcacagc catgcccggg caagaactca ggacggtgaa tggctctcag atgctcctgg 600
tgttgctggt gctctcgtgg ctgccgcatg gggcgccct gtctctggcc gaggcgagcc 660
gcgcaagttt cccgggaccc tcagagttgc actccgaaga ctccagattc cgagagttgc 720
ggaaacgcta cgaggacctg ctaaccaggc tgcgggccaa ccagagctgg gaagattcga 780
acaccgacct cgtcccggcc cctgcagtcc ggatactcac gccagaagtg cggctgggat 840
ccggcggcca cctgcacctg cgtatctctc gggccgccct tcccgagggg ctccccgagg 900
cctcccgcct tcaccgggct ctgttccggc tgtccccgac ggcgtcaagg tcgtgggacg 960
tgacacgacc gctgcggcgt cagctcagcc ttgcaagacc ccaggcgccc gcgctgcacc 1020
tgcgactgtc gccgccgccg tcgcagtcgg accaactgct ggcagaatct tcgtccgcac 1080
ggccccagct ggagttgcac ttgcggccgc aagccgccag gggcgccgc agagcgcgtg 1140
cgcgcaacgg ggaccactgt ccgctcgggc ccgggcgttg ctgccgtctg cacacggtcc 1200
gcgcgtcgct ggaagacctg ggctgggccg attgggtgct gtcgccacgg gaggtgcaag 1260
tgaccatgtg catcggcgcg tgcccgagcc agttccgggc ggcaaacatg cacgcgcaga 1320
tcaagacgag cctgcaccgc ctgaagcccg acacggtgcc agcgccctgc tgcgtgcccg 1380
ccagctacaa tcccatggtg ctcattcaaa agaccgacac cggggtgtcg ctccagacct 1440
atgatgactt gttagccaaa gactgccact gcatatgagc agtcctggtc cttccactgt 1500
gcacctgcgc gggggacgcg acctcagttg tcctgccctg tggaatgggc tcaaggttcc 1560
tgagacaccc gattcctgcc caaacagctg tatttatata agtctgttat ttattattaa 1620
tttattgggg tgaccttctt ggggactcgg gggctggtct gatggaactg tgtatttatt 1680
taaaactctg gtgataaaaa taaagctgtc tgaactgttc 1720

<210> 101
<211> 1396
<212> DNA
<213> Artificial

<400> 101

```
agaaagcaac tagcacagcc atgcccaggc caggacccgt gacacagcga tgctctcaga      60
tgctgctgat gttacagatg ttgttggggg cacccctgac tctgcaggtg agaaccttct     120
ggggttccta ggaccctcgg acaagcactc tgatccggat gactccaggt cccaggagtt     180
gcagaaacgc caccaggacc ttcagagcac cagttagaca aggagagcca ggaggagctg     240
ggccccaacc tcatccaaaa gcacagatgc tcctggtgtt gctggtgctc tcgtggctgc     300
cgcatggggg cgccctgtct ctggccgagg cgagccgcgc aagtttcccg ggaccctcag     360
agttgcactc cgaagactcc agattccgag agttgcggaa acgctacgag gacctgctaa     420
ccaggctgcg ggccaaccag agctgggaag attcgaacac cgacctcgtc ccggcccctg     480

cagtccggat actcacgcca gaagtgcggc tgggatccgg cggccacctg cacctgcgta     540
tctctcgggc cgcccttccc gaggggctcc ccgaggcctc ccgccttcac cgggctctgt     600
tccggctgtc cccgacggcg tcaaggtcgt gggacgtgac acgaccgctg cggcgtcagc     660
tcagccttgc aagaccccag gcgcccgcgc tgcacctgcg actgtcgccg ccgccgtcgc     720
agtcggacca actgctggca gaatcttcgt ccgcacggcc ccagctggag ttgcacttgc     780
ggccgcaagc cgccaggggg cgccgcagag cgcgtgcgcg caacggggac cactgtccgc     840
tcgggcccgg gcgttgctgc cgtctgcaca cggtccgcgc gtcgctggaa gacctgggct     900
gggccgattg ggtgctgtcg ccacgggagg tgcaagtgac catgtgcatc ggcgcgtgcc     960
cgagccagtt ccgggcggca aacatgcacg cgcagatcaa gacgagcctg caccgcctga    1020
agcccgacac ggtgccagcg ccctgctgcg tgcccgccag ctacaatccc atggtgctca    1080
ttcaaaagac cgacaccggg gtgtcgctcc agacctatga tgacttgtta gccaaagact    1140
gccactgcat atgagcagtc ctggtccttc cactgtgcac ctgcgcgggg gacgcgacct    1200
cagttgtcct gccctgtgga atgggctcaa ggttcctgag acacccgatt cctgcccaaa    1260
cagctgtatt tatataagtc tgttatttat tattaattta ttggggtgac cttcttgggg    1320
actcgggggc tggtctgatg gaactgtgta tttatttaaa actctggtga taaaaataaa    1380
gctgtctgaa ctgttc                                                    1396
```

<210> 102
<211> 3148
<212> DNA
<213> Artificial

<400> 102

```
agaaagcaac tagcacagcc atgcccaggc caggacccgt gacacagcga tgctctcaga     60
tgctgctgat gttacagatg ttgttggggg caccctgagg gggcaccctg actctgcagg    120
tgagaacctt ctggggttcc taggaccctc ggacaagcac tctgatccgg atgactccag    180
gtcccaggag ttgcagaaac gccaccagga ccttcagagc accagttaga caaggagagc    240
caggaggagc tgggccccaa cctcatccaa aagcacagac tcccaggctg gaatggtgtc    300
ctcatatcga ggaagaggat actgaggccc agaaatgtgc cctagcttta ctaggagcgc    360
ccccacctaa agatcctccc cctaaataca cccccagacc ccgcccagct gtggtcattg    420
gagtgtttac tctgcaggca gggggaggag ggcgggactg agcaggcgga gacggacaaa    480
gtccggggac tataaaggcc ggtccggcag catctggtca gtcccagctc agagccgcaa    540
cctgcacagc catgcccggg caagaactca ggacggtgaa tggctctcag atgctcctgg    600
tgttgctggt gctctcgtgg ctgccgcatg gggcgccct gtctctggcc gaggcgagcc     660
gcgcaagttt cccgggaccc tcagagttgc actccgaaga ctccagattc cgagagttgc    720
ggaaacgcta cgaggacctg ctaaccaggc tgcgggccaa ccagagctgg gaagattcga    780
acaccgacct cgtcccggcc cctgcagtcc ggatactcac gccagaaggt aagtgaaatc    840
```

ttagagatcc cctcccaccc cccaagcagc ccccatatct aatcagggat tcctcatctt 900
gaaaagccca gacctacctt tgagcctcag ttgccccatc tgtgccctgg gtaggaatat 960
cctggatccc cttgggtctg atggggtagc cgatgcctga tttgcaccca caacgtggga 1020
ggttataacc tgtccccaag ttgcaaatgg ggaaactgag gtacagggat gccagggacc 1080
ctcctgaatg tgcacagcac ctgggaaacc cggggacggg gtcgggcgtg cggtgtgggc 1140
atggtccctg acctgggctg gggccaggat ccaagagaaa caatgggggt gtggtgcatg 1200
gatagaagtt tgtgatgggc agagccagct ggagctggtt ctgaatctga tgaaatcgct 1260
tgtcaaggca ctgctaaagt cacatgcaac gagcgacttg ttaggggagg aggagagtat 1320
tttccccatc tctgtgccct ccatggaggc tccaggggtg cagggcactg gatgaggtct 1380
gggctgtggt gggacccagg agggggcagg cagaggggca cactcttagc ccgccctaaa 1440
cccatctgtg ccgtgtgact ccggcaaacc tctcaccctt gctaagccta gcaactaggt 1500
taaggcaccc tcaaacccca gtctccccag tctccctaac acttgtacca gcctgagggg 1560
ctgtgattcc tagaggctca gtagtctgga atgtgatggg tttggcactc ttcatttctc 1620
gggattacac ctgtaatccc agcactttag gaggccaagg tgggagcatc gctttaggcc 1680
agaacattgc agcctgggca gtatagcgag aatccatctc taccaaaaat gtttaaaaaa 1740
aattagccag acatgatggt gtgtgcctgt agtctcagtt attcaggagg ctgaggcggg 1800
aggagcactt gaatccagga gattgaggct gcactgagcc atgatgcagt ggcacgatct 1860
tggctcactg caacctccac cgccccgatt caagccattc ttctgcctca gcctcccgag 1920
tagctgggat cacaggcgcc agccaccatg cccagctaat ttttggtagt tttagtagaa 1980
gcgggcgttt caccatattg gccacatatc ggccaggctg gtcttgaact cctgacctca 2040
agtgatccac ctgcatcggc ctcccaaagt gctgggatta caggcgtgag ccaccgcgcc 2100
cgcacggaga aatgctcttg gtaggggaaa taacttgtgc aaaggcgccg ccgccgtggt 2160
gagatccagc ttggcgtgtt aggggaaatg ggcaccctcg ttcctggaaa acggtaggcc 2220
tgtgggtgac cagcttccct atctgtcttc ccacagtgcg gctgggatcc ggcggccacc 2280
tgcacctgcg tatctctcgg gccgcccttc ccgaggggct ccccgaggcc tcccgccttc 2340
accgggctct gttccggctg tccccgacgg cgtcaaggtc gtgggacgtg acacgaccgc 2400
tgcggcgtca gctcagcctt gcaagacccc aggcgcccgc gctgcacctg cgactgtcgc 2460
cgccgccgtc gcagtcggac caactgctgg cagaatcttc gtccgcacgg ccccagctgg 2520
agttgcactt gcggccgcaa gccgccaggg ggcgccgcag agcgcgtgcg cgcaacgggg 2580
accactgtcc gctcgggccc gggcgttgct gccgtctgca cacggtccgc gcgtcgctgg 2640
aagacctggg ctgggccgat tgggtgctgt cgccacggga ggtgcaagtg accatgtgca 2700
tcggcgcgtg cccgagccag ttccgggcgg caaacatgca cgcgcagatc aagacgagcc 2760
tgcaccgcct gaagcccgac acggtgccag cgccctgctg cgtgcccgcc agctacaatc 2820
ccatggtgct cattcaaaag accgacaccg ggtgtcgct ccagacctat gatgacttgt 2880

tagccaaaga ctgccactgc atatgagcag tcctggtcct tccactgtgc acctgcgcgg 2940

gggacgcgac ctcagttgtc ctgccctgtg gaatgggctc aaggttcctg agacacccga 3000

ttcctgccca aacagctgta tttatataag tctgttattt attattaatt tattggggtg 3060

accttcttgg ggactcgggg gctggtctga tggaactgtg tatttattta aaactctggt 3120

gataaaaata aagctgtctg aactgttc 3148

<210> 103
<211> 2268
<212> DNA
<213> Artificial

<400> 103

```
agaaagcaac tagcacagcc atgcccaggc caggacccgt gacacagcga tgctctcaga      60
tgctgctgat gttacagatg ttgttggggg caccctgagg gggcaccctg actctgcagg     120
tgagaacctt ctggggttcc taggaccctc ggacaagcac tctgatccgg atgactccag     180
gtcccaggag ttgcagaaac gccaccagga ccttcagagc accagttaga caaggagagc     240
caggaggagc tgggccccaa cctcatccaa aagcacagac tcccaggctg gaatggtgtc     300
ctcatatcga ggaagaggat actgaggccc agaaatgtgc cctagcttta ctaggagcgc     360
ccccacctaa agatcctccc cctaaataca cccccagacc ccgcccagct gtggtcattg     420
gagtgtttac tctgcaggca gggggaggag ggcgggactg agcaggcgga gacggacaaa     480
gtccggggac tataaaggcc ggtccggcag catctggtca gtcccagctc agagccgcaa     540
cctgcacagc catgcccggg caagaactca ggacggtgaa tggctctcag atgctcctgg     600
tgttgctggt gctctcgtgg ctgccgcatg gggcgccct gtctctggcc gaggcgagcc     660
gcgcaagttt cccgggaccc tcagagttgc actccgaaga ctccagattc cgagagttgc     720
ggaaacgcta cgaggacctg ctaaccaggc tgcgggccaa ccagagctgg gaagattcga     780
acaccgacct cgtcccggcc cctgcagtcc ggatactcac gccagaaggt aagtgaaatc     840
ttagagatcc cctcccaccc cccaagcagc ccccatatct aatcagggat tcctcatctt     900
gaaaagccca gacctacctt tgagcctcag ttgccccatc tgtgccctgg gtaggaatat     960
cctggatccc cttgggtctg atggggtagc cgatgcctga tttgcaccca caacgtggga    1020
ggttataacc tgtccccaag ttgcaaatgg ggaaactgag gtacagggat gccagggacc    1080
ctcctgaatg tgcacagcac ctgggaaacc cggggacggg gtcgggcgtg cggtgtgggc    1140
atggtccctg acctgggctg gggccaggat ccaagagaaa caatgggggt gtggtgcatg    1200
gatagaagtt tgtgatgggc agagccagct ggagctggtt ctgaatctga tgaaatcgct    1260
tgtcaaggca ctgctaaagt cacatgcaac gagcgacttg ttaggggagg aggagagtat    1320
tttccccatc tctgtgccct ccatggaggc tccaggggtg cagggcactg gatgagtgcg    1380
gctgggatcc ggcggccacc tgcacctgcg tatctctcgg gccgcccttc ccgaggggct    1440
ccccgaggcc tcccgccttc accgggctct gttccggctg tccccgacgg cgtcaaggtc    1500
```

gtgggacgtg acacgaccgc tgcggcgtca gctcagcctt gcaagacccc aggcgcccgc 1560
gctgcacctg cgactgtcgc cgccgccgtc gcagtcggac caactgctgg cagaatcttc 1620
gtccgcacgg ccccagctgg agttgcactt gcggccgcaa gccgccaggg ggcgccgcag 1680
agcgcgtgcg cgcaacgggg accactgtcc gctcgggccc gggcgttgct gccgtctgca 1740
cacggtccgc gcgtcgctgg aagacctggg ctgggccgat tgggtgctgt cgccacggga 1800
ggtgcaagtg accatgtgca tcggcgcgtg cccgagccag ttccgggcgg caaacatgca 1860
cgcgcagatc aagacgagcc tgcaccgcct gaagcccgac acggtgccag cgccctgctg 1920
cgtgcccgcc agctacaatc ccatggtgct cattcaaaag accgacaccg ggtgtcgct 1980
ccagacctat gatgacttgt tagccaaaga ctgccactgc atatgagcag tcctggtcct 2040
tccactgtgc acctgcgcgg gggacgcgac ctcagttgtc ctgccctgtg gaatgggctc 2100
aaggttcctg agacacccga ttcctgccca aacagctgta tttatataag tctgttattt 2160
attattaatt tattggggtg accttcttgg ggactcgggg gctggtctga tggaactgtg 2220
tatttattta aaactctggt gataaaaata aagctgtctg aactgttc 2268

<210> 104
<211> 1925
<212> DNA
<213> Artificial

<400> 104

```
agaaagcaac tagcacagcc atgcccaggc caggacccgt gacacagcga tgctctcaga     60
tgctgctgat gttacagatg ttgttggggg caccctgagg gggcaccctg actctgcagg    120
tgagaacctt ctggggttcc taggaccctc ggacaagcac tctgatccgg atgactccag    180
gtcccaggag ttgcagaaac gccaccagga ccttcagagc accagttaga caaggagagc    240
caggaggagc tgggccccaa cctcatccaa aagcacagac tcccaggctg gaatggtgtc    300
ctcatatcga ggaagaggat actgaggccc agaaatgtgc cctagcttta ctaggagcgc    360
ccccacctaa agatcctccc cctaaataca cccccagacc ccgcccagct gtggtcattg    420
gagtgtttac tctgcaggca gggggaggag ggcgggactg agcaggcgga gacggacaaa    480
gtccggggac tataaaggcc ggtccggcag catctggtca gtcccagctc agagccgcaa    540
cctgcacagc catgcccggg caagaactca ggacggtgaa tggctctcag atgctcctgg    600
tgttgctggt gctctcgtgg ctgccgcatg gggcgccct gtctctggcc gaggcgagcc    660
gcgcaagttt cccgggaccc tcagagttgc actccgaaga ctccagattc cgagagttgc    720
ggaaacgcta cgaggacctg ctaaccaggc tgcgggccaa ccagagctgg gaagattcga    780
acaccgacct cgtcccggcc cctgcagtcc ggatactcac gccagaaggt aagtgaaatc    840
ttagagatcc cctcccaccc cccaagcagc ccccatatct aatcagggat tcctcatctt    900
gaaaagccca gacctacctt tgagcctcag ttgccccatc tgtgccctgg gtaggaatat    960
cctggatccc cttgggtctg atggggtagc cgatgcctga tttgcaccca caacgtggga   1020

ggttataacc tgtccccaag ttgcaaatgg ggaaactgag gtacagggat gccagggacc   1080
ctcctgaatg tgcacagcac ctgggaaacc cggggacggg gtcgggcgtg cggtgtgggc   1140
atggtccctg acctgggctg gggccaggat ccaagagaaa caatgggggt gtggtgcatg   1200
gatagaagtt tgtgatgggc agagccagct ggagctggtt ctgaatctga tgaaatcgct   1260
tgtcaaggca ctgctaaagt cacatgcaac gagcgacttg ttaggggagg aggagagtat   1320
tttccccatc tctgtgccct ccatggaggc tccaggggtg cagggcactg gatgaggtct   1380
gggctgtggt gggacccagg agggggcagg cagaggggca cactcttagc ccgccctaaa   1440
cccatctgtg ccgtgtgact ccggcaaacc tctcaccctt gctaagccta gcaactaggt   1500
taaggcaccc tcaaacccca gtctccccag tctccctaac acttgtacca gcctgagggg   1560
ctgtgattcc tagaggctca gtagtctgga atgtgatggg tttggcactc ttcatttctc   1620
gggattacac ctgtaatccc agcactttag gaggccaagg tgggagcatc gctttaggcc   1680
agaacattgc agcctgggca gtatagcgag aatccatctc taccaaaaat gtttaaaaaa   1740
aattagccag acatgatggt gtgtgcctgt agtctcagtt attcaggagg ctgaggcggg   1800
aggagcactt gaatccagga gattgaggct gcactgagcc atgagtgccc cactgcactc   1860
cagcctgggt aacagcaaga ccataataaa taaataaata agtgaagcaa gtaagttttt   1920
gactg                                                               1925
```

<210> 105
<211> 1034
<212> DNA
<213> Artificial

<400> 105

```
aaggcggggg cgggggcggg gcggcggccg tgggtccctg ccggccggcg gcgggcgcag      60
acagcggcgg gcgcaggacg tgcactatgg ctcggggctc gctgcgccgg ttgctgcggc     120
tcctcgtgct ggggctctgg ctggcgttgc tgcgctccgt ggccggggag caagcgccag     180
gcaccgcccc ctgctcccgc ggcagctcct ggagcgcgga cctggacaag tgcatggact     240
gcgcgtcttg cagggcgcga ccgcacagcg acttctgcct gggctgcgct gcagcacctc     300
ctgcccccttt ccggctgctt tggcccatcc ttgggggcgc tctgagcctg accttcgtgc     360
tggggctgct ttctggcttt ttggtctgga gacgatgccg caggagagag aagttcacca     420
cccccataga ggagaccggc ggagagggct gcccagctgt ggcgctgatc cagtgacaat     480
gtgcccccctg ccagccgggg ctcgcccact catcattcat tcatccattc tagagccagt     540
ctctgcctcc cagacgcggc gggagccaag ctcctccaac cacaaggggg gtgggggggcg     600
gtgaatcacc tctgaggcct gggcccaggg ttcaggggaa ccttccaagg tgtctggttg     660
ccctgcctct ggctccagaa cagaaaggga gcctcacgct ggctcacaca aaacagctga     720
cactgactaa ggaactgcag catttgcaca ggggaggggg gtgccctcct tcctagaggc     780
cctgggggcc aggctgactt ggggggcaga cttgacacta ggccccactc actcagatgt     840
cctgaaattc caccacgggg gtcaccctgg ggggttaggg acctattttt aacactaggg     900
ggctggccca ctaggagggc tggccctaag atacagaccc ccccaactcc ccaaagcggg     960
gaggagatat ttattttggg gagagtttgg aggggaggga gaatttatta ataaaagaat    1020
ctttaacttt aaat                                                      1034
```

<210> 106
<211> 1757
<212> DNA
<213> Artificial

<400> 106

aaggcggggg cgggggcggg gcggcggccg tgggtccctg ccggccggcg gcgggcgcag 60
acagcggcgg gcgcaggacg tgcactatgg ctcggggctc gctgcgccgg ttgctgcggc 120
tcctcgtgct ggggctctgg ctggcgttgc tgcgctccgt ggccggggag caagcgccag 180
gtacgcggga ctccggggtc ggggaacccc gggccggggc tcgggagccg gactgggtgt 240
ctggagaaca gcgccgaact gggggaacag gcggatgtgg ggatctgggg tcaggtggcc 300
ttcaaacagc tcgctcccgg ctgtgggaag ttccatcact ctccaagctt cattcacaat 360
ccgggcctca gtttctccca ccgtccaatt ggggttgggg ggggtcttca gttccacaag 420
tccgaagtac ttcgaatgga gagagaaagt gagtgagggc gggatgttta gtctaggaag 480
gggcacgccc ccagcctggt ccgtacaggg tctaactagc cccagtgatc gacgagttgg 540
ggaggccgtc gagaggcact agggtctgtg ccctctatac catgtggggt gccccccttcc 600
atccggtgac ttcagtgccc agctatgcgg ggggagggac tggggtcggg cccggtgccc 660
aggagtgagt cacccgccgg cggcaggggg gttgtgggga cctgacccccc cccacccccca 720
gcgtcccgcg ctacgccctc cctcccggag ctgccgtgtc ccggtcgggc cgtgcggtca 780
cctgtgagga atgcgcgggg agggcgccgg tgactcacgt tattcgagcc ggccgaccct 840
gacctcagac cccagaatag ccgagtcccg cccccagcct ctgacccgag gccccctccc 900
caggcaccgc cccctgctcc cgcggcagct cctggagcgc ggacctggac aagtgcatgg 960
actgcgcgtc ttgcagggcg cgaccgcaca gcgacttctg cctgggctgc gctgcagcac 1020
ctcctgcccc cttccggctg ctttggccca tccttggggg cgctctgagc ctgaccttcg 1080
tgctggggct gctttctggc tttttggtct ggagacgatg ccgcaggaga gagaagttca 1140
ccaccccccat agaggagacc ggcggagagg gctgcccagc tgtggcgctg atccagtgac 1200
aatgtgcccc ctgccagccg gggctcgccc actcatcatt cattcatcca ttctagagcc 1260
agtctctgcc tcccagacgc ggcgggagcc aagctcctcc aaccacaagg ggggtggggg 1320
gcggtgaatc acctctgagg cctgggccca gggttcaggg gaaccttcca aggtgtctgg 1380
ttgccctgcc tctggctcca gaacagaaag ggagcctcac gctggctcac acaaaacagc 1440
tgacactgac taaggaactg cagcatttgc acaggggagg ggggtgccct ccttcctaga 1500
ggccctgggg gccaggctga cttgggggc agacttgaca ctaggcccca ctcactcaga 1560

tgtcctgaaa ttccaccacg gggtcaccc tggggggtta gggacctatt tttaacacta 1620
gggggctggc ccactaggag ggctggccct aagatacaga cccccccaac tccccaaagc 1680
ggggaggaga tatttatttt ggggagagtt tggaggggag ggagaattta ttaataaaag 1740
aatctttaac tttaaat 1757

<210> 107
<211> 1060
<212> DNA
<213> Artificial

<400> 107

```
aaggcggggg cgggggcggg gcggcggccg tgggtccctg ccggccggcg gcgggcgcag      60
acagcggcgg gcgcaggacg tgcactatgg ctcggggctc gctgcgccgg ttgctgcggc     120
tcctcgtgct ggggctctgg ctggcgttgc tgcgctccgt ggccggggag caagcgccag     180
cctctgaccc gaggccccct ccccaggcac cgccccctgc tcccgcggca gctcctggag     240
cgcggacctg gacaagtgca tggactgcgc gtcttgcagg gcgcgaccgc acagcgactt     300
ctgcctgggc tgcgctgcag cacctcctgc ccccttccgg ctgctttggc ccatccttgg     360
gggcgctctg agcctgacct tcgtgctggg gctgctttct ggctttttgg tctggagacg     420
atgccgcagg agagagaagt tcaccacccc catagaggag accggcggag agggctgccc     480
agctgtggcg ctgatccagt gacaatgtgc cccctgccag ccggggctcg cccactcatc     540
attcattcat ccattctaga gccagtctct gcctcccaga cgcggcggga gccaagctcc     600
tccaaccaca agggggggtgg ggggcggtga atcacctctg aggcctgggc ccagggttca     660
ggggaacctt ccaaggtgtc tggttgccct gcctctggct ccagaacaga aagggagcct     720
cacgctggct cacacaaaac agctgacact gactaaggaa ctgcagcatt tgcacagggg     780
agggggggtgc cctccttcct agaggccctg gggggccaggc tgacttgggg ggcagacttg     840
acactaggcc ccactcactc agatgtcctg aaattccacc acgggggtca ccctgggggg     900
ttagggacct atttttaaca ctagggggct ggcccactag gagggctggc cctaagatac     960
agaccccccc aactccccaa agcggggagg agatatttat tttggggaga gtttggaggg    1020
gagggagaat ttattaataa aagaatcttt aactttaaat                          1060
```

<210> 108
<211> 1269
<212> DNA
<213> Artificial

<400> 108

```
aaggcggggg cgggggcggg gcggcggccg tgggtccctg ccggccggcg gcgggcgcag      60
acagcggcgg gcgcaggacg tgcactatgg ctcggggctc gctgcgccgg ttgctgcggc     120
tcctcgtgct ggggctctgg ctggcgttgc tgcgctccgt ggccggggag caagcgccag     180
gcaccgcccc ctgctcccgc ggcagctcct ggagcgcgga cctggacaag tgcatggact     240
```

gcgcgtcttg cagggcgcga ccgcacagcg acttctgcct gggctgtgag tgggggcag 300
ggccagtggc cagcggaccc cagactggga gggagggtgg ctggtgcgca gagcggggcc 360
gagagctttg catctgggaa atcattcggg aggaggtggg agctgggagg gggctccggt 420
cagggaggag gccacgtttg ggagaaggca gaaggctcag tcttaggggg cggggctggc 480
ctggagaggg gcgaggtctg actccgagtc ccgcccccag gcgctgcagc acctcctgcc 540
cccttccggc tgctttggcc catccttggg ggcgctctga gcctgacctt cgtgctgggg 600
ctgctttctg gctttttggt ctggagacga tgccgcagga gagagaagtt caccaccccc 660
atagaggaga ccggcggaga gggctgccca gctgtggcgc tgatccagtg acaatgtgcc 720
ccctgccagc cggggctcgc ccactcatca ttcattcatc cattctagag ccagtctctg 780
cctcccagac gcggcgggag ccaagctcct ccaaccacaa ggggggtggg gggcggtgaa 840
tcacctctga ggcctgggcc cagggttcag gggaaccttc caaggtgtct ggttgccctg 900
cctctggctc cagaacagaa agggagcctc acgctggctc acacaaaaca gctgacactg 960
actaaggaac tgcagcattt gcacagggga ggggggtgcc ctccttccta gaggccctgg 1020
gggccaggct gacttggggg gcagacttga cactaggccc cactcactca gatgtcctga 1080
aattccacca cgggggtcac cctggggggt tagggaccta tttttaacac taggggggctg 1140
gcccactagg agggctggcc ctaagataca gacccccccca actccccaaa gcggggagga 1200
gatatttatt ttggggagag tttggagggg agggagaatt tattaataaa agaatcttta 1260
actttaaat 1269

<210> 109
<211> 2774
<212> DNA
<213> Artificial

<400> 109

```
aatccggctg gcagccgatt gacctgaccc aggcgagggt tgctgcaccg agtatttagt      60
ttggccccct actgaatgaa gacactttgt taaattctcc aatgagagac gcttagaggt     120
aggggcgtgg aagggggccg gggaatttga ggccagataa cgggaagtgt gagccgattg     180
gccggacttc tcactccacg gcccagccgt ttacggatcg cggaggccat ccatcaaaag     240
aattacacca atcagcggcg aaaatgcccc cttctcgcga gaaagccccg ccctccaata     300
tattcctcgt tagggcaggc gcggcccctt cggctccgag ctgaccctga tcagggccga     360
gttgtctcgg cggcgctgcc gaggcctcca cccaggacag tccccctccc cgggcctctc     420
tcctcttgcc tacgagtccc ctctcctcgt aggcctctcg gatctgatat cgtggggtga     480
ggtgagcagg cccggggagg gtggttaccg ctgaggagct gcagtctctg tcaagatgat     540
agaggtactg acaacaactg actctcagaa actgctacac cagctgaatg ccctgttgga     600
acaggagtct agatgtcagc caaaggtctg tggtttgaga ctaattgagt ctgcacacga     660
taatggcctc agaatgactg caagactaag ggactttgaa gtaaaagatc ttcttagtct     720
```

aactcagttc tttggctttg acacagagac attttctcta gctgtgaatt tactggacag 780
attcctgtct aaaatgaagg tacagcccaa gcaccttggg tgtgttggac tgagctgctt 840
ttatttggct gtaaaatcaa tagaagagga aaggaatgtc ccattggcaa ctgacttgat 900
ccgaataagt caatataggt ttacggtttc agacttgatg agaatggaaa agattgtatt 960
ggagaaggtg tgttggaaag tcaaagctac tactgccttt caatttctgc aactgtatta 1020
ttcactcctt caagagaact tgccacttga aaggagaaat agcattaatt ttgaaagact 1080
agaagctcaa ctgaaggcat gtcattgcag gatcatattt tctaaagcaa agccttctgt 1140
gttggcattg tctatcattg cattagagat ccaagcacag aagtgtgtag agttaacaga 1200
aggaatagaa tgtcttcaga aacattccaa gataaatggc agagatctga ccttctggca 1260
agagcttgta tccaaatgtt taactgaata ttcatcaaat aagtgttcca aaccaaatgt 1320
tcagaagttg aaatggattg tttctgggcg tactgcacgg caattgaagc atagctacta 1380
cagaataact caccttccaa caattcctga aatggtccct taactggatt attacagcac 1440
caaaaaactt ctctgaagcc tttctccaca accttgttct atggattcca taatgttaca 1500
atggatttaa gctatgaagc ctcaaaacat cacgagataa gcatgatggt ctcagacttg 1560
ggaaaactgc ctaatattat gctgtagtgg aattatgttt agatttgaat tcatctgtga 1620
agcattcaaa tcaaagctaa aagcctaaat gtgaaatgct aatgacaagc ctgagaaggt 1680
aaactgtgaa tcttcatttc tatcattgat ctaactttag atattggatc aatatattta 1740
ggtggtattg aaaatgctat tggaggagtc acactaatac tatcaactat cagtcttccc 1800
acagcttcaa tcactgtcat tattctaatc ctactcctac ttaaatttta agttatgagg 1860
tttatgtcaa aagcaacatt tcacaaatgt acttttaagg cataataagg gttaacattc 1920
taggcagtat aaacacaccc cataatgcaa gtaataggta atctagagat gtggacttta 1980
ttgctatatg ggaattacat ttaaatttga gggcatttta tataaagaaa aatacagacc 2040
tataaagttt ggcatattca ttaagttatc ttttaatatt tttttctaga aaacaggtga 2100
catttgtatc tacgataaaa atttttatac agaacctact gcctcaaact gaatcccatc 2160
aagaaaacta gtttctattg tattagtaac tcaaaataaa ttatcacttc gaaaacttgc 2220
tttcccacac taaggtaagt tcagactaga ttgaacactc cagaattttt tactacagac 2280
tgtttttaag ttagaagtga tggcaatttt ataaatagag aatatacttc cactgatgcc 2340
cttactgtgc caaaacaaaa atcttaagaa aagcaagtag acaccttcat aactatgaat 2400
gaagctgctg aagtagtgtt taggatcctc catggcagtt agtgaatgta agaagtacag 2460
tgttaaagtg ttgtaaacag ttactcagtg caatgtatag cctgagtcta tccatgatgg 2520
ctatatccaa tttgacatca cgttatggat cagtacacaa tgaaaaacca aagaaccaca 2580
gtatatctta ttcttaactt ttgtaaacca tgttttatgg gtaacttttt agttttccca 2640
aaaggctgat aaatttcaat attttgaata catcattgtt aattttgagt tggcagaggt 2700
aaactaacca actaccatta tgttttagta ctaaggata tacctttcaa taaagttaat 2760

gaaattcaat cctt 2774

<210> 110
<211> 881
<212> DNA
<213> Artificial

<400> 110

aatccggctg gcagccgatt gacctgaccc aggcgagggt tgctgcaccg agtatttagt 60
ttggccccct actgaatgaa gacactttgt taaattctcc aatgagagac gcttagaggt 120
aggggcgtgg aagggggccg gggaatttga ggccagataa cgggaagtgt gagccgattg 180
gccggacttc tcactccacg gcccagccgt ttacggatcg cggaggccat ccatcaaaag 240
aattacacca atcagcggcg aaaatgcccc cttctcgcga gaaagccccg ccctccaata 300
tattcctcgt tagggcaggc gcggcccctt cggctccgag ctgaccctga tcagggccga 360
gttgtctcgg cggcgctgcc gaggcctcca cccaggacag tccccctccc cgggcctctc 420
tcctcttgcc tacgagtccc ctctcctcgt aggcctctcg gatctgatat cgtggggtga 480
ggtgagcagg cccggggagg gtggttaccg ctgaggagct gcagtctctg tcaagatgat 540
agaggtactg acaacaactg actctcagaa actgctacac cagctgaatg ccctgttgga 600
acaggagtct agatgtcagc caaaggtctg tggtttgaga ctaattgagt ctgcacacga 660
taatggcctc agaatgactg caagactaag ggactttgaa gtaaaagatc ttcttagtct 720
aactcagttc tttggctttg acacagagac attttctcta gctgtgaatt tactggacag 780
attcctgtct aaaatgaagg tatgtttgaa gctacatttt tgtaattttg ctcagtgtgt 840
tttggagatg gaattgttac cttttggctg gtattcataa a 881

<210> 111
<211> 1951
<212> DNA
<213> Artificial

<400> 111

```
ggcatagtac tattcatttt aattttttgg cccaaatcat ctaaaactcc catatggtgc     60
ctttagaagt tagtatctct gatggaaggg gaattccttt caatagttca tttgctatgt    120
taagtgtgca taagctttac tttaaaaatt gacttcaatg tttttctaaa aacatctttt    180
gactagatgt agtattacct aataaaagta ataccatttt ctttttaaag gagaaatagc    240
attaattttg aaagactaga agctcaactg aaggcatgtc attgcaggat catattttct    300
aaagcaaagc cttctgtgtt ggcattgtct atcattgcat tagagatcca agcacagaag    360
tgtgtagagt taacagaagg aatagaatgt cttcagaaac attccaagat aaatggcaga    420
gatctgacct tctggcaaga gcttgtatcc aaatgtttaa ctgaatattc atcaaataag    480
tgttccaaac caaatgttca gaagttgaaa tggattgttt ctgggcgtac tgcacggcaa    540
ttgaagcata gctactacag aataactcac cttccaacaa ttcctgaaat ggtcccttaa    600

ctggattatt acagcaccaa aaaacttctc tgaagccttt ctccacaacc ttgttctatg    660
gattccataa tgttacaatg gatttaagct atgaagcctc aaaacatcac gagataagca    720
tgatggtctc agacttggga aaactgccta atattatgct gtagtggaat tatgtttaga    780
tttgaattca tctgtgaagc attcaaatca aagctaaaag cctaaatgtg aaatgctaat    840
gacaagcctg agaaggtaaa ctgtgaatct tcatttctat cattgatcta actttagata    900
ttggatcaat atatttaggt ggtattgaaa atgctattgg aggagtcaca ctaatactat    960
caactatcag tcttcccaca gcttcaatca ctgtcattat tctaatccta ctcctactta   1020
aattttaagt tatgaggttt atgtcaaaag caacatttca caaatgtact tttaaggcat   1080
aataagggtt aacattctag gcagtataaa cacaccccat aatgcaagta ataggtaatc   1140
tagagatgtg gactttattg ctatatggga attacattta aatttgaggg cattttatat   1200
aaagaaaaat acagacctat aaagtttggc atattcatta agttatcttt taatattttt   1260
ttctagaaaa caggtgacat ttgtatctac gataaaaatt tttatacaga acctactgcc   1320
tcaaactgaa tcccatcaag aaaactagtt tctattgtat tagtaactca aaataaatta   1380
tcacttcgaa aacttgcttt cccacactaa ggtaagttca gactagattg aacactccag   1440
aattttttac tacagactgt ttttaagtta gaagtgatgg caattttata aatagagaat   1500
atacttccac tgatgccctt actgtgccaa aacaaaaatc ttaagaaaag caagtagaca   1560
ccttcataac tatgaatgaa gctgctgaag tagtgtttag gatcctccat ggcagttagt   1620
gaatgtaaga agtacagtgt taaagtgttg taaacagtta ctcagtgcaa tgtatagcct   1680
gagtctatcc atgatggcta tatccaattt gacatcacgt tatggatcag tacacaatga   1740
aaaaccaaag aaccacagta tatcttattc ttaacttttg taaaccatgt tttatgggta   1800
actttttagt tttcccaaaa ggctgataaa tttcaatatt ttgaatacat cattgttaat   1860
tttgagttgg cagaggtaaa ctaaccaact accattatgt tttagtacta agggatatac   1920
ctttcaataa agttaatgaa attcaatcct t                                  1951
```

<210> 112
<211> 1023
<212> DNA
<213> Artificial

<400> 112

```
atccactcca cgaacgtcaa atgcgtcatc tgaggagggg gagggccggg gcaaaggagg      60
ggcaccctga catggagcct gccagctccg tcagccctga ctcggcccgg agctgagctc     120
cccacctgcc ggtagcccag gagatggagc agcccagccc acgtgcccgg ccttccgccc     180
ctgacttcac ttgataacaa actagaaact gaaacagggt cgggatgccg atgccggctt     240
ggagttagag atgagtcacc gctgagagca gctgcagtag ctgagcagtg gcagcagaga     300
ggcagacgtg agctgagggc gcagaggcag gcagcatctc tgagggtccc caaggaacat     360
ggctgggagc cgtgaggtgg tggccatgga ctgcgagatg gtggggctgg ggccccaccg     420
ggagagtggc ctggctcgtt gcagcctcgt gaacgtccac ggtgctgtgc tgtacgacaa     480
gttcatccgg cctgagggag agatcaccga ttacagaacc cgggtcagcg gggtcacccc     540
tcagcacatg gtgggggcca caccatttgc cgtggccagg ctagagatcc tgcagctcct     600
gaaaggcaag ctggtggtgg gtcatgacct gaagcacgac ttccaggcac tgaaagagga     660
catgagcggc tacacaatct acgacacgtc cactgacagg ctgttgtggc gtgaggccaa     720
gctggaccac tgcaggcgtg tctccctgcg ggtgctgagt gagcgcctcc tgcacaagag     780
catccagaac agcctgcttg gacacagctc ggtggaagat gcgagggcaa cgatggagct     840
ctatcaaatc tcccagagaa tccgagcccg ccgagggctg ccccgcctgg ctgtgtcaga     900
ctgaagcccc atccagcccg ttccgcaggg actagaggct ttcggctttt tgggacagca     960
actaccttgc ttttggaaaa tacattttta atagtaaagt ggctctatat tttctctacg    1020
cca                                                                  1023
```

<210> 113
<211> 1856
<212> DNA
<213> Artificial

<400> 113

atccactcca cgaacgtcaa atgcgtcatc tgaggagggg gagggccggg gcaaaggagg 60
ggcaccctga catggagcct gccagctccg tcagccctga ctcggcccgg agctgagctc 120
cccacctgcc ggtagcccag gagatggagc agcccagccc acgtgcccgg ccttccgccc 180
ctgacttcac ttgataacaa actagaaact gaaacagggt cgggatgccg atgccggctt 240
ggagttagag atgagtcacc gctgagagca gctgcagtag ctgagcagtg gcagcagaga 300
ggcagacgtg agctgagggc gcagaggcag gcagcatctc tgagggtccc caaggaacat 360
ggctgggagc cgtgaggtgg tggccatgga ctgcgagatg gtggggctgg ggccccaccg 420
ggagagtggc ctggctcgtt gcagcctcgt gaacgtccac ggtgctgtgc tgtacgacaa 480
gttcatccgg cctgagggag agatcaccga ttacagaacc cgggtcagcg gggtcacccc 540
tcagcacatg gtgggggcca caccatttgc cgtggccagg ctagagatcc tgcagctcct 600
gaaaggcaag ctggtggtgg gtcatgacct gaagcacgac ttccaggcac tgaaagagga 660
catgagcggc tacacaatct acgacacgtc cactgacagg ctgttgtggc gtgaggccaa 720
gctggaccac tgcaggcgtg tctccctgcg ggtgctgagt gagcgcctcc tgcacaagag 780
catccagaac agcctgcttg gacacagctc ggtggaagat gcgagggcaa cgatggagct 840
ctatcaaatc tcccagagaa tccgagcccg ccgagggctg ccccgcctgg ctgtgtcaga 900
ctgaagcccc atccagcccg ttccgcaggg actagaggct ttcggctttt tgggacagca 960
actaccttgc ttttggaaaa tacattttta atagtaaagt ggctctatat tttctctacg 1020
ccatcactgg gtcctcttct tattcttctc tccaagctgg gttaacagta gacaggaccc 1080
atttctgtgt gatgttagga gggaatgaag tcttatgctg gggaggtggg caagtatcaa 1140

tttccttaat atcttgaatc ctgtgggtcc aaaatgtggc ttggaaatct aagtagcatg 1200
tggcttaatt actaatccca ccctttgctg ttgcatccca gccctattcc tggtgcattt 1260
atgcccagag aggtggcatt atttcctggg gtggcattca gctcctcttg agttggtgcc 1320
acagcatttg tgggctttga agcaaaggta caggaaatgt caagggtgcc accccggcaa 1380
ccttgagcaa gtcacccctc ctatttgtaa aatgaggaag gaaaggtaac aaactgtgga 1440
gtcagagaga agtaggttgg aatcctcttt gtcatttagt agctgtttga cctaaggtgg 1500
tttactgaac ttctcagttt ctccatctgt aaaatgagaa ttctagcaac tcgtagggta 1560
tttgtgagat gttgcatgca aagcccccag caccatgcct gtcctagctt aagcacccac 1620
caggtgtcga taagtaattg ttcttccctg gactgcctgc acatctaggg caccccagga 1680
agagtcaccg cactctgttt cggggctcgg ctctctgagg ggagggcatc ctgtatgggg 1740
aggagggtct ggaccagagc tgtccctgat cgctagaatc aacggcagaa ttcttgttat 1800
tttaataata attaataatt tagtgcttcc actaaataaa aatcattgga atggtg 1856

<210> 114
<211> 2964

<212> DNA
<213> Artificial

<400> 114

```
gcattgatgg taggtcctgc tgtgcctggt ccctggctgg tagagtgagg atgagggcag     60
ggaagggaaa acacccaaca aaggtttgct tttgttgttg aaatcaagcc agttaccctg    120
tgggaagtga agcttgctcc caccatagaa ctctgggcaa tgatgtaaag cacacacctc    180
acctcagata tgtctcacag caggggccgg ggaactggga tatttgtaca ccagttccca    240
tcagccacca gctgctgctg ctcctgcgca ctgattccgc agcacttctg gctggtgggc    300
aaagcaggca ggaaaaggca tacggatatg gggggttggg agttcaccct gtgggaaggg    360
cagagggaga ggcgccagac actaagagcc agggctgtac acagggaccc ttcaggtcaa    420
cagctcttta ttaaacacct gctgcctgct ctaggccaga tcctagagac gcagggtgag    480
caagacccrt ctctgtccct gagctcttgg ttggttggga ggtggccctt aaccagatca    540
tcagcacaca tggtaaggag tgtgtgacgg agggaagggc agtgccctgg gagcaggatg    600
aaggatgggg ctggcagggc aggagaccag atctgaacag agcctggagg gtcttagtca    660
aggagggcag aagaaggcat acagagtggg gccaggtgag tcaaagcctg aggctcgggg    720
aggggctggg gagcacggag cccgccccta gcctgctggc aagagctgta ggaatcatgt    780
ccggagtagg gaacagggct gagtctgggc ctgttagcct caaagcacat aaggcacata    840
agggccaggc tgggcttttc ctggagccct tgccgctctg tggctctcag tctggggcct    900
taagcctgac ccagggcctt ctcccaggct ctgtccctgg gctcattaag cctggttgtt    960
ccagaagctc agtaccccac caggcagctc ctgcctgcac tgatgcccct agaaaccagc   1020
cctcccgccc gcccccgctc acatggacag tggtgctact gatcactgtg accagaactg   1080
```

```
acccacagga cacraggagc taacatggta gagctgtcac agttgggcag tggtgttact   1140
gcccctcgag ggagtgaaat agcaggcctg tggctccctg ccaagctgcg cgcatggtag   1200
accgggctgc acaggaaagg aaagaccctg ccatttatga agccctgtgg agggtgctgg   1260
gcacagttct ctcattccat cctttaaggc aggtataatc aggcccattt cacagaagag   1320
gaaacagagg cccagaaagg aaaagtgact tgcccacggt cacagagctc ataagcaggg   1380
gagctgggaa ttgcagcctg gggtttgtct gattccaaat ccggggctct ttacctcccc   1440
acttggtaat aaagggtgct ggtgctgcat tctgcttctg ttcttggctt ccttctccat   1500
agagcagagt ggtagaacac ataacctccc tgtgcctcag tttcctcatc tgtcaaggat   1560
ggtgacaggg gcaggtatgc agaggcacag crcagacgac gccaccatgc tacctgcttg   1620
ctgattaatg atagggtgtc caagccaaaa aataaaacta aacattaaaa aataaaacaa   1680
tacagcgtaa taacggcaac aagaaaaagc ctttttgagc tgggccgtct aaccaggtgt   1740
ccccaacacc ctgtctggag tgcctggaaa aggagcccct gtgagaacag gaagtcctct   1800
ctcgacagag ggacaccaag aaaacgcagc caggtccaac agcccgctgg ctcccatgct   1860
gggtaccggc caggacacca gggctgagcg gggcatgcga agggagatga aaggtgactg   1920
cagtgaggat ttgccctgtg tactcgcctc tgcaagatcc acatctcagc tgcacaactg   1980
gctggcttgt gtgtgagcct cagttttatc atcagtaaaa tgggggaata atcatatcca   2040
gctcagaggt ttaacttcag cctagtgcac ggaagacatg gtaaatggtc atcgcttctc   2100
ccttgaatgg accgagggcc cccgactccc atagcactac cccataaagt ctaggctccc   2160
gacttcggca ttcaaggcct tcagtgataa atccaggccc accatccgtc cccacaccac   2220
ctttcacaca ccccctgccc ccaccctcgc aggcctctta ttttagctgc acgtgctctc   2280
cctgcctccg aaatgcttgc ggcctttcct ttccatttat tgacgccctt gtgttctctg   2340
tgagcagccc ttccctgtgt ccctagctgg agttcctcac tccctcctca gtgtcctgaa   2400
gcttcttgtt agagctgctg cagccactgc gtgtgtctga cagcaagtct gcagcctctg   2460
cctcccttgc gggactgcag gtgacttgaa ggcacagcct ggttcccagg aggctggctt   2520
tcgtgccact ggccccaggg ctgaaggcag gccagctcca gccacctgct gagtctccta   2580
agtccagagt gtcagagacc tcaagagact ctcacccact gacccacctg gggactctgg   2640
aaatcgggat gtgaccaggg aagaccctgt cacggggcct ctgaattcag cctcatccca   2700
agtccccact ctatacttgt gtctgcagaa cagcctgctt ggacacagct cggtggaaga   2760
tgcgagggca acgatggagc tctatcaaat ctcccagaga atccgagccc gccgagggct   2820
gccccgcctg gctgtgtcag actgaagccc catccagccc gttccgcagg gactagaggc   2880
tttcggcttt ttgggacagc aactaccttg cttttggaaa atacattttt aatagtaaag   2940
tggctctata ttttctctac gcca                                          2964
```

<210> 115
<211> 1205

<212> DNA
<213> Artificial

<400> 115

```
ctctttcccg gagcctgggc ggagagggag gaaaacttct tcctggcctg ggctccgtgc     60
cgctctgttt gccaaccgtc cagtcccgcc taccagtgcc gggcgctccc cacccctccc    120
ccggctcccc cggtgtccgc catggccaaa gcctacgacc acctcttcaa gttgctgctg    180
atcggggact cgggggtggg caagacttgt ctgatcattc gctttgcaga ggacaacttc    240
aacaacactt acatctccac catcggaatt gatttcaaga tccgcactgt ggatatagag    300
gggaagaaga tcaaactaca agtctgggac acggctggcc aagagcggtt caagacaata    360
actactgcct actaccgtgg agccatgggc attatcctag tatacgacat cacggatgag    420
aaatctttcg agaatattca gaactggatg aaaagcatca aggagaatgc ctcggctggg    480
gtggagcgcc tcttgctggg gaacaaatgt gacatggagg ccaagaggaa ggtgcagaag    540
gagcaggccg ataagttggc tcgagagcat ggaatccgat tttcgaaac tagtgctaaa    600
tccagtatga atgtggatga ggcttttagt tccctggccc gggacatctt gctcaagtca    660
ggaggccgga gatcaggaaa cggcaacaag cctcccagta ctgacctgaa aacttgtgac    720
aagaagaaca ccaacaagtg ctccctgggc tgaggacccct ttcttgcctc cccaccccgg    780
aagctgaacc tgagggagac aacggcagag ggagtgagca ggggagaaat agcagagggg    840
cttggagggt cacataggta gatggtaaag agaatgagga gaaaaaggag aaaagggaaa    900
agcagaaagg aaaaaaagga agagagagga agggagaagg gagaggaatg aattgaggaa    960
gtgaaagaag gcaaggaggt aggaagagag ggaggaggaa aggaaggaga gatgcctcag   1020
gcttcagacc ttacctgggt tttcagggca aacataaatg taaatacact gatttattct   1080
gttactagat caggttttag ggtcctgcaa aaggctagct cggcactaca ctagggaatt   1140
tgctcctgtt ctgtcacttg tcatggtctt tcttggtatt aaaggccacc atttgcacaa   1200
atgtt                                                                1205
```

<210> 116
<211> 1323
<212> DNA
<213> Artificial

<400> 116

ctctttcccg gagcctgggc ggagagggag gaaaacttct tcctggcctg ggctccgtgc 60
cgctctgttt gccaaccgtc cagtcccgcc taccagtgcc gggcgctccc cacccctccc 120
ccggctcccc cggtgtccgc catggccaaa gcctacgacc acctcttcaa gttgctgctg 180
atcggggact cgggggtggg caagacttgt ctgatcattc gctttgcaga ggacaacttc 240
aacaacactt acatctccac catcggaatt gatttcaaga tccgcactgt ggatatagag 300
gggaagaaga tcaaactaca agtctgggac acggctggcc aagagcggtt caagacaata 360
actactgcct actaccgtgg agccatgggc attatcctag tatacgacat cacggatgag 420

aaatctttcg agaatattca gaactggatg aaaagcatca aggagaatgc ctcggctggg 480
gtggagcgcc tcttgctggg gaacaaatgt gacatggagg ccaagaggaa ggtgcagaag 540
gagcaggccg ataaggtgag ggccaggctg agcaatgttc tagaactggg ctgggagggc 600
caagataggt tgcattgcag aggacttggc tgctgtcctt aattcaattc tcttcccact 660
tcacccctta tagttggctc gagagcatgg aatccgattt ttcgaaacta gtgctaaatc 720
cagtatgaat gtggatgagg cttttagttc cctggcccgg gacatcttgc tcaagtcagg 780
aggccggaga tcaggaaacg gcaacaagcc tcccagtact gacctgaaaa cttgtgacaa 840
gaagaacacc aacaagtgct ccctgggctg aggacccttt cttgcctccc caccccggaa 900
gctgaacctg agggagacaa cggcagaggg agtgagcagg ggagaaatag cagaggggct 960
tggagggtca cataggtaga tggtaaagag aatgaggaga aaaaggagaa aagggaaaag 1020
cagaaaggaa aaaaaggaag agagaggaag ggagaaggga gaggaatgaa ttgaggaagt 1080
gaaagaaggc aaggaggtag gaagagaggg aggaggaaag gaaggagaga tgcctcaggc 1140
ttcagacctt acctgggttt tcagggcaaa cataaatgta aatacactga tttattctgt 1200
tactagatca ggttttaggg tcctgcaaaa ggctagctcg gcactacact agggaatttg 1260
ctcctgttct gtcacttgtc atggtctttc ttggtattaa aggccaccat ttgcacaaat 1320
gtt 1323

<210> 117
<211> 1301
<212> DNA
<213> Artificial

<400> 117

ctctttcccg gagcctgggc ggagagggag gaaaacttct tcctggcctg ggctccgtgc 60
cgctctgttt gccaaccgtc cagtcccgcc taccagtgcc gggcgctccc cacccctccc 120
ccggctcccc cggtgtccgc catggccaaa gcctacgacc acctcttcaa gttgctgctg 180
atcggggact cgggggtggg caagacttgt ctgatcattc gctttgcaga ggacaacttc 240
aacaacactt acatctccac catcggtgag cccgctggcc atgtcccaga ccccgactct 300
cctgtgacct ccttctccgg ctcccggatt actagtgacc ccacttttca gtccccttag 360
agaattgatt tcaagatccg cactgtggat atagagggga agaagatcaa actacaagtc 420
tgggacacgg ctggccaaga gcggttcaag acaataacta ctgcctacta ccgtggagcc 480
atgggcatta tcctagtata cgacatcacg gatgagaaat ctttcgagaa tattcagaac 540
tggatgaaaa gcatcaagga gaatgcctcg gctggggtgg agcgcctctt gctggggaac 600
aaatgtgaca tggaggccaa gaggaaggtg cagaaggagc aggccgataa gttggctcga 660
gagcatggaa tccgattttt cgaaactagt gctaaatcca gtatgaatgt ggatgaggct 720
tttagttccc tggcccggga catcttgctc aagtcaggag gccggagatc aggaaacggc 780
aacaagcctc ccagtactga cctgaaaact tgtgacaaga agaacaccaa caagtgctcc 840

ctgggctgag gaccctttct tgcctcccca ccccggaagc tgaacctgag ggagacaacg 900
gcagagggag tgagcagggg agaaatagca gaggggcttg gagggtcaca taggtagatg 960
gtaaagagaa tgaggagaaa aaggagaaaa gggaaaagca gaaaggaaaa aaaggaagag 1020
agaggaaggg agaagggaga ggaatgaatt gaggaagtga aagaaggcaa ggaggtagga 1080
agagagggag gaggaaagga aggagagatg cctcaggctt cagaccttac ctgggttttc 1140
agggcaaaca taaatgtaaa tacactgatt tattctgtta ctagatcagg ttttagggtc 1200
ctgcaaaagg ctagctcggc actacactag ggaatttgct cctgttctgt cacttgtcat 1260
ggtctttctt ggtattaaag gccaccattt gcacaaatgt t 1301

<210> 118
<211> 1197
<212> DNA
<213> Artificial

<400> 118

```
ctctttcccg gagcctgggc ggagagggag gaaaacttct tcctggcctg ggctccgtgc      60
cgctctgttt gccaaccgtc cagtcccgcc taccagtgcc gggcgctccc caccccctccc    120
ccggctcccc cggtgtccgc catggccaaa gcctacgacc acctcttcaa gttgctgctg     180
atcggggact cgggggtggg caagacttgt ctgatcattc gctttgcaga ggacaacttc     240
aacaacactt acatctccac catcggaatt gatttcaaga tccgcactgt ggatatagag     300
gggaagaaga tcaaactaca agtctgggac acggctggcc aagagcggtt caagacaata     360
actactgcct actaccgtgg agccatgggc attatcctag tatacgacat cacggatgag     420
aaatctttcg agaatattca gaactggatg aaaagcatca aggagaatgc ctcggctggg     480
gtggagcgcc tcttgctggg gaacaaatgt gacatggagg ccaagaggaa ggtgcagaag     540
gagcaggccg ataagttggc tcgagagcat ggaatccgat tttcgaaac tagtgctaaa      600
tccagtatga atgtggatga gttccctggc ccgggacatc ttgctcaagt caggaggccg     660
gagatcagga aacggcaaca agcctcccag tactgacctg aaaacttgtg acaagaagaa     720
caccaacaag tgctccctgg gctgaggacc ctttcttgcc tccccacccc ggaagctgaa     780
cctgagggag acaacggcag agggagtgag caggggagaa atagcagagg ggcttggagg     840
gtcacatagg tagatggtaa agagaatgag gagaaaaagg agaaaaggga aaagcagaaa     900
ggaaaaaaag gaagagagag gaagggagaa gggagaggaa tgaattgagg aagtgaaaga     960
aggcaaggag gtaggaagag agggaggagg aaaggaagga gagatgcctc aggcttcaga    1020
ccttacctgg gttttcaggg caaacataaa tgtaaataca ctgatttatt ctgttactag    1080
atcaggtttt agggtcctgc aaaaggctag ctcggcacta cactagggaa tttgctcctg    1140
ttctgtcact tgtcatggtc tttcttggta ttaaaggcca ccatttgcac aaatgtt       1197
```

<210> 119
<211> 1849
<212> DNA
<213> Artificial

<400> 119

ctcctcattt tcttggtacc atcgccttag tctccccaaa tccacaagaa ctcttctcta 60
tttttctctg ttcccctctt cccctacccc caccacctag aactctctcg atcctatctc 120
catgtttcct gtatccctgt ccgccacatc tttttctccc gatccgatgc tagcctcttt 180
ctgtcttgct gagcttcaaa ctctcccact gtgtccccac ctcaccatct acttcccagg 240
ttccctgccc cagtctccct gcacctcagc tgaacaaggg tttgggaagg gtggaacaag 300
gtggctgggt gtgctggagg gtggcccaag gactgtgatc atgaatatgc agccagcttg 360
gtgaggaacg tggaaatgag gggacttttg gggtctctga gtcagcctgg tgactcatcc 420
tcctcccagg ggcccccagc aggcttgggg gaggtgccca agtttctact aagaagaaca 480
ttgtctcccc tcctcccttc tcttctttct tcatctcctc tggcttggga aatagacagg 540
aagagtctca cgacaattct ggttacaaag gaaaaggaaa aatgcccctc tttcctaatt 600
gtctcaaaag taatcctgtt tagcctcaga ctttcctagt gccaagtttg gcccactagt 660
taattcacat agactggctt cagctacttt tttccttact ctctactagg agggtaggtg 720
ggataccaag agtagagggc tctaagaaaa cagagaagag gaatgtgaaa gtaatatctg 780
gggggagaca gagactgaat tcagagagtt gtcagctggt ataactgaat ggacccaaga 840
ggcagcctaa ggattttaag cctccatgca gaattttcgt caccttttcc ctcccaaaat 900
ccataaaatg aattgatttc aagatccgca ctgtggatat agaggggaag aagatcaaac 960
tacaagtctg ggacacggct ggccaagagc ggttcaagac aataactact gcctactacc 1020
gtggagccat gggcattatc ctagtatacg acatcacgga tgagaaatct ttcgagaata 1080
ttcagaactg gatgaaaagc atcaaggaga atgcctcggc tggggtggag cgcctcttgc 1140
tggggaacaa atgtgacatg gaggccaaga ggaaggtgca gaaggagcag gccgataagt 1200
tggctcgaga gcatggaatc cgatttttcg aaactagtgc taaatccagt atgaatgtgg 1260
atgaggcttt tagttccctg gcccgggaca tcttgctcaa gtcaggaggc cggagatcag 1320
gaaacggcaa caagcctccc agtactgacc tgaaaacttg tgacaagaag aacaccaaca 1380
agtgctccct gggctgagga ccctttcttg cctccccacc ccggaagctg aacctgaggg 1440
agacaacggc agagggagtg agcaggggag aaatagcaga ggggcttgga gggtcacata 1500
ggtagatggt aaagagaatg aggagaaaaa ggagaaaagg gaaaagcaga aaggaaaaaa 1560
aggaagagag aggaagggag aagggagagg aatgaattga ggaagtgaaa gaaggcaagg 1620
aggtaggaag agagggagga ggaaaggaag gagagatgcc tcaggcttca gaccttacct 1680
gggttttcag ggcaaacata aatgtaaata cactgattta ttctgttact agatcaggtt 1740
ttagggtcct gcaaaaggct agctcggcac tacactaggg aatttgctcc tgttctgtca 1800
cttgtcatgg tctttcttgg tattaaaggc caccatttgc acaaatgtt 1849

<210> 120
<211> 2184
<212> DNA
<213> Artificial

<400> 120

gaggcgggcc cgggcggggc ggttgtatat cagggccgcg ctgagctgcg ccagctgagg 60
tgtgagcagc tgccgaagtc agttccttgt ggagccggag ctgggcgcgg attcgccgag 120
gcaccgaggc actcagagga ggcgccatgt cagaaccggc tggggatgtc cgtcagaacc 180
catgcggcag caaggcctgc cgccgcctct tcggcccagt ggacagcgag cagctgagcc 240
gcgactgtga tgcgctaatg gcgggctgca tccaggaggc ccgtgagcga tggaacttcg 300
actttgtcac cgagacacca ctggagggtg acttcgcctg ggagcgtgtg cggggccttg 360
gcctgcccaa gctctacctt cccacggggc cccggcgagg ccgggatgag ttgggaggag 420
gcaggcggcc tggcacctca cctgctctgc tgcaggggac agcagaggaa gaccatgtgg 480
acctgtcact gtcttgtacc cttgtgcctc gctcagggga gcaggctgaa gggtccccag 540
gtggacctgg agactctcag ggtcgaaaac ggcggcagac cagcatgaca gatttctacc 600
actccaaacg ccggctgatc ttctccaaga ggaagcccta atccgcccac aggaagcctg 660
cagtcctgga agcgcgaggg cctcaaaggc ccgctctaca tcttctgcct tagtctcagt 720
ttgtgtgtct taattattat ttgtgtttta atttaaacac ctcctcatgt acataccctg 780
gccgccccct gccccccagc ctctggcatt agaattattt aaacaaaaac taggcggttg 840
aatgagaggt tcctaagagt gctgggcatt tttattttat gaaatactat ttaaagcctc 900
ctcatcccgt gttctccttt tcctctctcc cggaggttgg gtgggccggc ttcatgccag 960
ctacttcctc ctccccactt gtccgctggg tggtaccctc tggaggggtg tggctccttc 1020
ccatcgctgt cacaggcggt tatgaaattc acccccttc ctggacactc agacctgaat 1080
tctttttcat ttgagaagta aacagatggc actttgaagg ggcctcaccg agtgggggca 1140
tcatcaaaaa ctttggagtc ccctcacctc ctctaaggtt gggcagggtg accctgaagt 1200
gagcacagcc tagggctgag ctggggacct ggtaccctcc tggctcttga taccccctc 1260
tgtcttgtga aggcaggggg aaggtggggt cctggagcag accaccccgc ctgccctcat 1320
ggcccctctg acctgcactg gggagcccgt ctcagtgttg agccttttcc ctctttggct 1380
cccctgtacc ttttgaggag ccccagctac ccttcttctc cagctgggct ctgcaattcc 1440
cctctgctgc tgtccctccc ccttgtcctt tcccttcagt accctctcag ctccaggtgg 1500
ctctgaggtg cctgtcccac ccccaccccc agctcaatgg actggaaggg gaagggacac 1560
acaagaagaa gggcacccta gttctacctc aggcagctca agcagcgacc gccccctcct 1620
ctagctgtgg gggtgagggt cccatgtggt ggcacaggcc cccttgagtg gggttatctc 1680
tgtgttaggg gtatatgatg ggggagtaga tctttctagg agggagacac tggcccctca 1740
aatcgtccag cgaccttcct catccacccc atccctcccc agttcattgc actttgatta 1800
gcagcggaac aaggagtcag acattttaag atggtggcag tagaggctat ggacagggca 1860

tgccacgtgg gctcatatgg ggctgggagt agttgtcttt cctggcacta acgttgagcc 1920
cctggaggca ctgaagtgct tagtgtactt ggagtattgg ggtctgaccc caaacacctt 1980
ccagctcctg taacatactg gcctggactg ttttctctcg gctccccatg tgtcctggtt 2040
cccgtttctc cacctagact gtaaacctct cgagggcagg gaccacaccc tgtactgttc 2100
tgtgtctttc acagctcctc ccacaatgct gaatatacag caggtgctca ataaatgatt 2160
cttagtgact ttacttgtaa tatt 2184

<210> 121
<211> 2088
<212> DNA
<213> Artificial

<400> 121

gaggcgggcc cgggcggggc ggttgtatat cagggccgcg ctgagctgcg ccagctgagg 60
tgtgagcagc tgccgaagtc agttccttgt ggagccggag ctgggcgcgg attcgccgag 120
gcaccgaggc actcagagga ggcgccatgt cagaaccggc tggggatgtc cgtcagaacc 180
catgcggcag caaggcctgc cgccgcctct tcggcccagt ggacagcgag cagctgagcc 240
gcgactgtga tgcgctaatg gcgggctgca tccaggaggc ccgtgagcga tggaacttcg 300
actttgtcac cgagacacca ctggagggtg acttcgcctg ggagcgtgtg cggggccttg 360
gcctgcccaa gctctacctt cccacggggc cccggcgagg ccgggatgag ttgggaggag 420
gcaggcggcc tggcacctca cctgctctgc tgcaggggac agcagaggaa gaccatgtgg 480
acctgtcact gtcttgtacc cttgtgcctc gctcagggga gcaggctgaa gggtccccag 540
gtggacctgg agactctcag ggtcgaaaac ggcggcagac cagcatgaca gatttctacc 600
actccaaacg ccggctgatc ttctccaaga ggaagcccta atccgcccac aggaagcctg 660
cagtcctgga agcgcgaggg cctcaaaggc ccgctctaca tcttctgcct tagtctcagt 720
ttgtgtgtct taattattat ttgtgtttta atttaaacac ctcctcatgt acataccctg 780
gccgcccccct gccccccagc ctctggcatt agaattattt aaacaaaaac taggcggttg 840
aatgagaggt tcctaagagt gctgggcatt tttattttat gaaatactat ttaaagcctc 900
ctcatcccgt gttctccttt tcctctctcc cggaggttgg gtgggccggc ttcatgccag 960
ctacttcctc ctccccactt gtccgctggg tggtaccctc tggaggggtg tggctccttc 1020
ccatcgctgt cacaggcggt tatgaaattc acccccttc ctggacactc agacctgaat 1080
tctttttcat ttgagaagta aacagatggc actttgaagg ggcctcaccg agtgggggca 1140
tcatcaaaaa ctttggagtc ccctcacctc ctctaaggtt gggcagggtg accctgaagt 1200
gagcacagcc tagggctgag ctggggacct ggtaccctcc tggctcttga tacccccctc 1260
tgtcttgtga aggcaggggg aaggtggggt cctggagcag accaccccgc ctgccctcat 1320
ggcccctctg acctgcactg gggagcccgt ctcagtgttg agccttttcc ctctttggct 1380
cccctgtacc ttttgaggag ccccagctac ccttcttctc cagctgggct ctgcaattcc 1440

cctctgctgc tgtccctccc ccttgtcctt tcccttcagt accctctcag ctccaggcag 1500
ctcaagcagc gaccgccccc tcctctagct gtgggggtga gggtcccatg tggtggcaca 1560
ggcccccttg agtggggtta tctctgtgtt aggggtatat gatgggggag tagatctttc 1620
taggagggag acactggccc ctcaaatcgt ccagcgacct tcctcatcca ccccatccct 1680
ccccagttca ttgcactttg attagcagcg gaacaaggag tcagacattt taagatggtg 1740
gcagtagagg ctatggacag ggcatgccac gtgggctcat atggggctgg gagtagttgt 1800
ctttcctggc actaacgttg agcccctgga ggcactgaag tgcttagtgt acttggagta 1860
ttggggtctg accccaaaca ccttccagct cctgtaacat actggcctgg actgttttct 1920
ctcggctccc catgtgtcct ggttcccgtt tctccaccta gactgtaaac ctctcgaggg 1980
cagggaccac accctgtact gttctgtgtc tttcacagct cctcccacaa tgctgaatat 2040
acagcaggtg ctcaataaat gattcttagt gactttactt gtaatatt 2088

<210> 122
<211> 2325
<212> DNA
<213> Artificial

<400> 122

```
gaggcgggcc cgggcggggc ggttgtatat cagggccgcg ctgagctgcg ccagctgagg      60
tgtgagcagc tgccgaagtc agttccttgt ggagccggag ctgggcgcgg attcgccgag     120
gcaccgaggc actcagagga ggtgagagag cggcggcaga caacagggga ccccgggccg     180
gcggcccaga gccgagccaa gcgtgcccgc gtgtgtccct gcgtgtccgc gaggatgcgt     240
gttcgcgggt gtgtgctgcg ttcacaggtg tttctgcggc aggcgccatg tcagaaccgg     300
ctggggatgt ccgtcagaac ccatgcggca gcaaggcctg ccgccgcctc ttcggcccag     360
tggacagcga gcagctgagc cgcgactgtg atgcgctaat ggcgggctgc atccaggagg     420
cccgtgagcg atggaacttc gactttgtca ccgagacacc actggagggt gacttcgcct     480
gggagcgtgt gcggggcctt ggcctgccca agctctacct tcccacgggg ccccggcgag     540
gccgggatga gttgggagga ggcaggcggc ctggcacctc acctgctctg ctgcagggga     600
cagcagagga agaccatgtg gacctgtcac tgtcttgtac ccttgtgcct cgctcagggg     660
agcaggctga agggtcccca ggtggacctg gagactctca gggtcgaaaa cggcggcaga     720
ccagcatgac agatttctac cactccaaac gccggctgat cttctccaag aggaagccct     780
aatccgccca caggaagcct gcagtcctgg aagcgcgagg gcctcaaagg cccgctctac     840
atcttctgcc ttagtctcag tttgtgtgtc ttaattatta tttgtgtttt aatttaaaca     900
cctcctcatg tacataccct ggccgccccc tgcccccag cctctggcat tagaattatt      960
taaacaaaaa ctaggcggtt gaatgagagg ttcctaagag tgctgggcat ttttatttta    1020
tgaaatacta tttaaagcct cctcatcccg tgttctcctt ttcctctctc ccggaggttg    1080
ggtgggccgg cttcatgcca gctacttcct cctccccact tgtccgctgg gtggtaccct    1140
```

ctggaggggt gtggctcctt cccatcgctg tcacaggcgg ttatgaaatt caccccttt 1200
cctggacact cagacctgaa ttctttttca tttgagaagt aaacagatgg cactttgaag 1260
gggcctcacc gagtgggggc atcatcaaaa actttggagt cccctcacct cctctaaggt 1320
tgggcagggt gaccctgaag tgagcacagc ctagggctga gctggggacc tggtaccctc 1380
ctggctcttg atacccccct ctgtcttgtg aaggcagggg gaaggtgggg tcctggagca 1440
gaccaccccg cctgccctca tggcccctct gacctgcact ggggagcccg tctcagtgtt 1500
gagccttttc cctctttggc tcccctgtac cttttgagga gccccagcta cccttcttct 1560
ccagctgggc tctgcaattc ccctctgctg ctgtccctcc cccttgtcct ttcccttcag 1620
taccctctca gctccaggtg gctctgaggt gcctgtccca cccccacccc cagctcaatg 1680
gactggaagg ggaagggaca cacaagaaga agggcaccct agttctacct caggcagctc 1740
aagcagcgac cgccccctcc tctagctgtg ggggtgaggg tcccatgtgg tggcacaggc 1800
ccccttgagt ggggttatct ctgtgttagg ggtatatgat gggggagtag atctttctag 1860
gagggagaca ctggcccctc aaatcgtcca gcgaccttcc tcatccaccc catccctccc 1920
cagttcattg cactttgatt agcagcggaa caaggagtca gacattttaa gatggtggca 1980
gtagaggcta tggacagggc atgccacgtg ggctcatatg gggctgggag tagttgtctt 2040
tcctggcact aacgttgagc ccctggaggc actgaagtgc ttagtgtact tggagtattg 2100
gggtctgacc ccaaacacct tccagctcct gtaacatact ggcctggact gttttctctc 2160
ggctccccat gtgtcctggt tcccgtttct ccacctagac tgtaaacctc tcgagggcag 2220
ggaccacacc ctgtactgtt ctgtgtcttt cacagctcct cccacaatgc tgaatataca 2280
gcaggtgctc aataaatgat tcttagtgac tttacttgta atatt 2325

<210> 123
<211> 2634
<212> DNA
<213> Artificial

<400> 123

gaggcgggcc cgggcggggc ggttgtatat cagggccgcg ctgagctgcg ccagctgagg 60
tgtgagcagc tgccgaagtc agttccttgt ggagccggag ctgggcgcgg attcgccgag 120
gcaccgaggc actcagagga ggtgagagag cggcggcaga caacagggga ccccgggccg 180
gcggcccaga gccgagccaa gcgtgcccgc gtgtgtccct gcgtgtccgc gaggatgcgt 240
gttcgcgggt gtgtgctgcg ttcacaggtg tttctgcggc aggtgaatga cgggcgtggg 300
tcggtgcgcg ctcggcttgc gcacacggtg tctctaagtg cgcgggtgac gagagtcggg 360
atgtgccgga gaccccgggg cggagagcgg gattacaagt acaggaatcc ctggtcacgc 420
tccccgcccc tggaaaccca gctggggcga gggagggcgt ggacgggacc gttctgggag 480
ctcgcctttg gctgcggttg gctccaggcc ccaggcgcag tttgctcgcg gcgtggggat 540
gaagtccgtg tccctggagg ggcccaggaa gggcgaggaa agcggagtgg agcgccatgt 600

cagaaccggc tggggatgtc cgtcagaacc catgcggcag caaggcctgc cgccgcctct 660
tcggcccagt ggacagcgag cagctgagcc gcgactgtga tgcgctaatg gcgggctgca 720
tccaggaggc ccgtgagcga tggaacttcg actttgtcac cgagacacca ctggagggtg 780
acttcgcctg ggagcgtgtg cggggccttg gcctgcccaa gctctacctt cccacggggc 840
cccggcgagg ccgggatgag ttgggaggag gcaggcggcc tggcacctca cctgctctgc 900
tgcaggggac agcagaggaa gaccatgtgg acctgtcact gtcttgtacc cttgtgcctc 960
gctcagggga gcaggctgaa gggtccccag gtggacctgg agactctcag ggtcgaaaac 1020
ggcggcagac cagcatgaca gatttctacc actccaaacg ccggctgatc ttctccaaga 1080
ggaagcccta atccgcccac aggaagcctg cagtcctgga agcgcgaggg cctcaaaggc 1140
ccgctctaca tcttctgcct tagtctcagt ttgtgtgtct taattattat ttgtgtttta 1200
atttaaacac ctcctcatgt acataccctg gccgcccccct gccccccagc ctctggcatt 1260
agaattattt aaacaaaaac taggcggttg aatgagaggt tcctaagagt gctgggcatt 1320
tttattttat gaaatactat ttaaagcctc ctcatcccgt gttctccttt tcctctctcc 1380
cggaggttgg gtgggccggc ttcatgccag ctacttcctc ctccccactt gtccgctggg 1440
tggtaccctc tggaggggtg tggctccttc ccatcgctgt cacaggcggt tatgaaattc 1500
accccctttc ctggacactc agacctgaat tctttttcat ttgagaagta aacagatggc 1560
actttgaagg ggcctcaccg agtgggggca tcatcaaaaa ctttggagtc ccctcacctc 1620
ctctaaggtt gggcagggtg accctgaagt gagcacagcc tagggctgag ctggggacct 1680
ggtaccctcc tggctcttga taccccctc tgtcttgtga aggcaggggg aaggtggggt 1740
cctggagcag accaccccgc ctgccctcat ggcccctctg acctgcactg gggagcccgt 1800
ctcagtgttg agccttttcc ctctttggct cccctgtacc ttttgaggag ccccagctac 1860
ccttcttctc cagctgggct ctgcaattcc cctctgctgc tgtccctccc ccttgtcctt 1920
tcccttcagt accctctcag ctccaggtgg ctctgaggtg cctgtcccac ccccaccccc 1980
agctcaatgg actggaaggg gaagggacac acaagaagaa gggcacccta gttctacctc 2040
aggcagctca agcagcgacc gccccctcct ctagctgtgg gggtgagggt cccatgtggt 2100
ggcacaggcc cccttgagtg gggttatctc tgtgttaggg gtatatgatg gggagtaga 2160
tctttctagg agggagacac tggcccctca aatcgtccag cgaccttcct catccacccc 2220
atccctcccc agttcattgc actttgatta gcagcggaac aaggagtcag acattttaag 2280
atggtggcag tagaggctat ggacagggca tgccacgtgg gctcatatgg ggctgggagt 2340
agttgtcttt cctggcacta acgttgagcc cctggaggca ctgaagtgct tagtgtactt 2400
ggagtattgg ggtctgaccc caaacacctt ccagctcctg taacatactg gcctggactg 2460
ttttctctcg gctccccatg tgtcctggtt cccgtttctc cacctagact gtaaacctct 2520
cgagggcagg gaccacaccc tgtactgttc tgtgtctttc acagctcctc ccacaatgct 2580
gaatatacag caggtgctca ataaatgatt cttagtgact ttacttgtaa tatt 2634

<210> 124
<211> 2392
<212> DNA
<213> Artificial

<400> 124

aacatgttga gctctggcat agaagaggct ggtggctatt ttgtccttgg gctgcctgtt 60
ttcaggtgag gaaggggatg gtaggagaca ggagacctct aaagacccca gaaataaagg 120
atgacaagca gagagccccg ggcaggaggc aaaagtcctg tgttccaact atagtcattt 180
ctttgctgca tgatctgagt taggtcacca gacttctctg agccccagtt tccccagcag 240
tgtatacggg ctatgtgggg agtattcagg agacagacaa ctcactcgtc aaatcctccc 300
cttcctggcc aacaaagctg ctgcaaccac agggatttct tctgttcagg cgccatgtca 360
gaaccggctg gggatgtccg tcagaaccca tgcggcagca aggcctgccg ccgcctcttc 420
ggcccagtgg acagcgagca gctgagccgc gactgtgatg cgctaatggc gggctgcatc 480
caggaggccc gtgagcgatg gaacttcgac tttgtcaccg agacaccact ggagggtgac 540
ttcgcctggg agcgtgtgcg gggccttggc ctgcccaagc tctaccttcc cacggggccc 600
cggcgaggcc gggatgagtt gggaggaggc aggcggcctg gcacctcacc tgctctgctg 660
caggggacag cagaggaaga ccatgtggac ctgtcactgt cttgtaccct tgtgcctcgc 720
tcaggggagc aggctgaagg gtccccaggt ggacctggag actctcaggg tcgaaaacgg 780
cggcagacca gcatgacaga tttctaccac tccaaacgcc ggctgatctt ctccaagagg 840
aagccctaat ccgcccacag gaagcctgca gtcctggaag cgcgagggcc tcaaaggccc 900
gctctacatc ttctgcctta gtctcagttt gtgtgtctta attattattt gtgttttaat 960
ttaaacacct cctcatgtac ataccctggc cgcccccctgc cccccagcct ctggcattag 1020
aattatttaa acaaaaacta ggcggttgaa tgagaggttc ctaagagtgc tgggcatttt 1080
tattttatga aatactattt aaagcctcct catcccgtgt tctccttttc ctctctcccg 1140
gaggttgggt gggccggctt catgccagct acttcctcct ccccacttgt ccgctgggtg 1200
gtaccctctg gaggggtgtg gctccttccc atcgctgtca caggcggtta tgaaattcac 1260
ccccctttcct ggacactcag acctgaattc tttttcattt gagaagtaaa cagatggcac 1320
tttgaagggg cctcaccgag tgggggcatc atcaaaaact ttggagtccc ctcacctcct 1380
ctaaggttgg gcagggtgac cctgaagtga gcacagccta gggctgagct ggggacctgg 1440
taccctcctg gctcttgata cccccctctg tcttgtgaag gcagggggaa ggtggggtcc 1500
tggagcagac caccccgcct gccctcatgg cccctctgac ctgcactggg gagcccgtct 1560
cagtgttgag ccttttccct ctttggctcc cctgtacctt ttgaggagcc ccagctaccc 1620
ttcttctcca gctgggctct gcaattcccc tctgctgctg tccctccccc ttgtcctttc 1680
ccttcagtac cctctcagct ccaggtggct ctgaggtgcc tgtcccaccc ccaccccccag 1740
ctcaatggac tggaagggga agggacacac aagaagaagg gcaccctagt tctacctcag 1800

gcagctcaag cagcgaccgc cccctcctct agctgtgggg gtgagggtcc catgtggtgg 1860
cacaggcccc cttgagtggg gttatctctg tgttaggggt atatgatggg ggagtagatc 1920
tttctaggag ggagacactg gcccctcaaa tcgtccagcg accttcctca tccaccccat 1980
ccctccccag ttcattgcac tttgattagc agcggaacaa ggagtcagac attttaagat 2040
ggtggcagta gaggctatgg acagggcatg ccacgtgggc tcatatgggg ctgggagtag 2100
ttgtctttcc tggcactaac gttgagcccc tggaggcact gaagtgctta gtgtacttgg 2160
agtattgggg tctgacccca aacaccttcc agctcctgta acatactggc ctggactgtt 2220
ttctctcggc tccccatgtg tcctggttcc cgtttctcca cctagactgt aaacctctcg 2280
agggcaggga ccacaccctg tactgttctg tgtctttcac agctcctccc acaatgctga 2340
atatacagca ggtgctcaat aaatgattct tagtgacttt acttgtaata tt 2392

<210> 125
<211> 2300
<212> DNA
<213> Artificial

<400> 125

```
aacatgttga gctctggcat agaagaggct ggtggctatt ttgtccttgg gctgcctgtt      60
ttcaggtgag gaaggggatg gtaggagaca ggagacctct aaagacccca ggtcaccaga     120
cttctctgag ccccagtttc cccagcagtg tatacgggct atgtggggag tattcaggag     180
acagacaact cactcgtcaa atcctcccct tcctggccaa caaagctgct gcaaccacag     240
ggatttcttc tgttcaggcg ccatgtcaga accggctggg gatgtccgtc agaacccatg     300
cggcagcaag gcctgccgcc gcctcttcgg cccagtggac agcgagcagc tgagccgcga     360
ctgtgatgcg ctaatggcgg gctgcatcca ggaggcccgt gagcgatgga acttcgactt     420
tgtcaccgag acaccactgg agggtgactt cgcctgggag cgtgtgcggg gccttggcct     480
gcccaagctc taccttccca cggggccccg gcgaggccgg gatgagttgg gaggaggcag     540
gcggcctggc acctcacctg ctctgctgca ggggacagca gaggaagacc atgtggacct     600
gtcactgtct tgtacccttg tgcctcgctc aggggagcag gctgaagggt ccccaggtgg     660
acctggagac tctcagggtc gaaaacggcg gcagaccagc atgacagatt tctaccactc     720
caaacgccgg ctgatcttct ccaagaggaa gccctaatcc gcccacagga agcctgcagt     780
cctggaagcg cgagggcctc aaaggcccgc tctacatctt ctgccttagt ctcagtttgt     840
gtgtcttaat tattatttgt gttttaattt aaacacctcc tcatgtacat accctggccg     900
ccccctgccc cccagcctct ggcattagaa ttatttaaac aaaaactagg cggttgaatg     960
agaggttcct aagagtgctg ggcattttta ttttatgaaa tactatttaa agcctcctca    1020
tcccgtgttc tccttttcct ctctcccgga ggttgggtgg gccggcttca tgccagctac    1080
ttcctcctcc ccacttgtcc gctgggtggt accctctgga ggggtgtggc tccttcccat    1140
cgctgtcaca ggcggttatg aaattcaccc cctttcctgg acactcagac ctgaattctt    1200
```

tttcatttga gaagtaaaca gatggcactt tgaaggggcc tcaccgagtg gggcatcat 1260
caaaaacttt ggagtcccct cacctcctct aaggttgggc agggtgaccc tgaagtgagc 1320
acagcctagg gctgagctgg ggacctggta ccctcctggc tcttgatacc cccctctgtc 1380
ttgtgaaggc agggggaagg tggggtcctg gagcagacca ccccgcctgc cctcatggcc 1440
cctctgacct gcactgggga gcccgtctca gtgttgagcc ttttccctct ttggctcccc 1500
tgtacctttt gaggagcccc agctaccctt cttctccagc tgggctctgc aattcccctc 1560
tgctgctgtc cctccccctt gtcctttccc ttcagtaccc tctcagctcc aggtggctct 1620
gaggtgcctg tcccaccccc acccccagct caatggactg gaaggggaag ggacacacaa 1680
gaagaagggc accctagttc tacctcaggc agctcaagca gcgaccgccc cctcctctag 1740
ctgtgggggt gagggtccca tgtggtggca caggcccccct tgagtggggt tatctctgtg 1800
ttaggggtat atgatggggg agtagatctt tctaggaggg agacactggc ccctcaaatc 1860
gtccagcgac cttcctcatc caccccatcc ctccccagtt cattgcactt tgattagcag 1920
cggaacaagg agtcagacat tttaagatgg tggcagtaga ggctatggac agggcatgcc 1980
acgtgggctc atatggggct gggagtagtt gtctttcctg gcactaacgt tgagcccctg 2040
gaggcactga agtgcttagt gtacttggag tattggggtc tgaccccaaa caccttccag 2100
ctcctgtaac atactggcct ggactgtttt ctctcggctc cccatgtgtc ctggttcccg 2160
tttctccacc tagactgtaa acctctcgag ggcagggacc acaccctgta ctgttctgtg 2220
tctttcacag ctcctcccac aatgctgaat atacagcagg tgctcaataa atgattctta 2280
gtgactttac ttgtaatatt 2300

<210> 126
<211> 2186
<212> DNA
<213> Artificial

<400> 126

```
aacatgttga gctctggcat agaagaggct ggtggctatt ttgtccttgg gctgcctgtt      60
ttcaggtgag gaaggggatg gtaggagaca ggagacctct aaagacccca gctgctgcaa     120
ccacagggat ttcttctgtt caggcgccat gtcagaaccg gctggggatg tccgtcagaa     180
cccatgcggc agcaaggcct gccgccgcct cttcggccca gtggacagcg agcagctgag     240
ccgcgactgt gatgcgctaa tggcgggctg catccaggag gcccgtgagc gatggaactt     300
cgactttgtc accgagacac cactggaggg tgacttcgcc tgggagcgtg tgcggggcct     360
tggcctgccc aagctctacc ttcccacggg gccccggcga ggccgggatg agttgggagg     420
aggcaggcgg cctggcacct cacctgctct gctgcagggg acagcagagg aagaccatgt     480
ggacctgtca ctgtcttgta cccttgtgcc tcgctcaggg gagcaggctg aagggtcccc     540
aggtggacct ggagactctc agggtcgaaa acggcggcag accagcatga cagatttcta     600
ccactccaaa cgccggctga tcttctccaa gaggaagccc taatccgccc acaggaagcc     660
```

tgcagtcctg gaagcgcgag ggcctcaaag gcccgctcta catcttctgc cttagtctca 720
gtttgtgtgt cttaattatt atttgtgttt taatttaaac acctcctcat gtacataccc 780
tggccgcccc ctgcccccca gcctctggca ttagaattat ttaaacaaaa actaggcggt 840
tgaatgagag gttcctaaga gtgctgggca tttttatttt atgaaatact atttaaagcc 900
tcctcatccc gtgttctcct tttcctctct cccggaggtt gggtgggccg gcttcatgcc 960
agctacttcc tcctccccac ttgtccgctg ggtggtaccc tctggagggg tgtggctcct 1020
tcccatcgct gtcacaggcg gttatgaaat tcacccccct tcctggacac tcagacctga 1080
attctttttc atttgagaag taaacagatg gcactttgaa ggggcctcac cgagtggggg 1140
catcatcaaa aactttggag tcccctcacc tcctctaagg ttgggcaggg tgaccctgaa 1200
gtgagcacag cctagggctg agctggggac ctggtaccct cctggctctt gatacccccc 1260
tctgtcttgt gaaggcaggg ggaaggtggg gtcctggagc agaccacccc gcctgccctc 1320
atggcccctc tgacctgcac tggggagccc gtctcagtgt tgagcctttt ccctctttgg 1380
ctcccctgta ccttttgagg agccccagct acccttcttc tccagctggg ctctgcaatt 1440
cccctctgct gctgtccctc ccccttgtcc tttcccttca gtaccctctc agctccaggt 1500
ggctctgagg tgcctgtccc acccccaccc ccagctcaat ggactggaag gggaagggac 1560
acacaagaag aagggcaccc tagttctacc tcaggcagct caagcagcga ccgcccccctc 1620
ctctagctgt gggggtgagg gtcccatgtg gtggcacagg ccccccttgag tggggttatc 1680
tctgtgttag ggtatatgga tgggggagta gatctttcta ggagggagac actggcccct 1740
caaatcgtcc agcgaccttc ctcatccacc ccatccctcc ccagttcatt gcactttgat 1800
tagcagcgga acaaggagtc agacatttta agatggtggc agtagaggct atggacaggg 1860
catgccacgt gggctcatat ggggctggga gtagttgtct ttcctggcac taacgttgag 1920
cccctggagg cactgaagtg cttagtgtac ttggagtatt ggggtctgac cccaaacacc 1980
ttccagctcc tgtaacatac tggcctggac tgttttctct cggctcccca tgtgtcctgg 2040
ttcccgtttc tccacctaga ctgtaaacct ctcgagggca gggaccacac cctgtactgt 2100
tctgtgtctt tcacagctcc tcccacaatg ctgaatatac agcaggtgct caataaatga 2160
ttcttagtga ctttacttgt aatatt 2186

<210> 127
<211> 2304
<212> DNA
<213> Artificial

<400> 127

aacatgttga gctctggcat agaagaggct ggtggctatt ttgtccttgg gctgcctgtt 60
ttcaggtgag gaaggggatg gtaggagaca ggagacctct aaagacccca gttaggtcac 120
cagacttctc tgagccccag tttccccagc agtgtatacg ggctatgtgg ggagtattca 180
ggagacagac aactcactcg tcaaatcctc cccttcctgg ccaacaaagc tgctgcaacc 240

acagggattt cttctgttca ggcgccatgt cagaaccggc tggggatgtc cgtcagaacc 300
catgcggcag caaggcctgc cgccgcctct tcggcccagt ggacagcgag cagctgagcc 360
gcgactgtga tgcgctaatg gcgggctgca tccaggaggc ccgtgagcga tggaacttcg 420
actttgtcac cgagacacca ctggagggtg acttcgcctg ggagcgtgtg cggggccttg 480
gcctgcccaa gctctacctt cccacggggc cccggcgagg ccgggatgag ttgggaggag 540
gcaggcggcc tggcacctca cctgctctgc tgcaggggac agcagaggaa gaccatgtgg 600
acctgtcact gtcttgtacc cttgtgcctc gctcagggga gcaggctgaa gggtccccag 660
gtggacctgg agactctcag ggtcgaaaac ggcggcagac cagcatgaca gatttctacc 720
actccaaacg ccggctgatc ttctccaaga ggaagcccta atccgcccac aggaagcctg 780
cagtcctgga agcgcgaggg cctcaaaggc ccgctctaca tcttctgcct tagtctcagt 840
ttgtgtgtct taattattat ttgtgtttta atttaaacac ctcctcatgt acataccctg 900
gccgccccct gccccccagc ctctggcatt agaattattt aaacaaaaac taggcggttg 960
aatgagaggt tcctaagagt gctgggcatt tttattttat gaaatactat ttaaagcctc 1020
ctcatcccgt gttctccttt tcctctctcc cggaggttgg gtgggccggc ttcatgccag 1080
ctacttcctc ctccccactt gtccgctggg tggtaccctc tggaggggtg tggctccttc 1140
ccatcgctgt cacaggcggt tatgaaattc acccccttc ctggacactc agacctgaat 1200
tctttttcat ttgagaagta aacagatggc actttgaagg ggcctcaccg agtgggggca 1260
tcatcaaaaa ctttggagtc ccctcacctc ctctaaggtt gggcagggtg accctgaagt 1320
gagcacagcc tagggctgag ctggggacct ggtaccctcc tggctcttga tacccccctc 1380
tgtcttgtga aggcaggggg aaggtggggt cctggagcag accaccccgc ctgccctcat 1440
ggcccctctg acctgcactg gggagcccgt ctcagtgttg agccttttcc ctctttggct 1500
cccctgtacc ttttgaggag ccccagctac ccttcttctc cagctgggct ctgcaattcc 1560
cctctgctgc tgtccctccc ccttgtcctt tcccttcagt accctctcag ctccaggtgg 1620
ctctgaggtg cctgtcccac ccccaccccc agctcaatgg actggaaggg gaagggacac 1680
acaagaagaa gggcacccta gttctacctc aggcagctca agcagcgacc gccccctcct 1740
ctagctgtgg gggtgagggt cccatgtggt ggcacaggcc cccttgagtg gggttatctc 1800
tgtgttaggg gtatatgatg ggggagtaga tctttctagg agggagacac tggcccctca 1860
aatcgtccag cgaccttcct catccacccc atccctcccc agttcattgc actttgatta 1920
gcagcggaac aaggagtcag acattttaag atggtggcag tagaggctat ggacagggca 1980
tgccacgtgg gctcatatgg ggctgggagt agttgtcttt cctggcacta acgttgagcc 2040
cctggaggca ctgaagtgct tagtgtactt ggagtattgg ggtctgaccc caaacacctt 2100
ccagctcctg taacatactg gcctggactg ttttctctcg gctccccatg tgtcctggtt 2160
cccgtttctc cacctagact gtaaacctct cgagggcagg gaccacaccc tgtactgttc 2220
tgtgtctttc acagctcctc ccacaatgct gaatatacag caggtgctca ataaatgatt 2280

cttagtgact ttacttgtaa tatt 2304

<210> 128
<211> 2140
<212> DNA
<213> Artificial

<400> 128

```
aacatgttga gctctggcat agaagaggct ggtggctatt ttgtccttgg gctgcctgtt      60
ttcagctgct gcaaccacag ggatttcttc tgttcaggcg ccatgtcaga accggctggg     120
gatgtccgtc agaacccatg cggcagcaag gcctgccgcc gcctcttcgg cccagtggac     180
agcgagcagc tgagccgcga ctgtgatgcg ctaatggcgg gctgcatcca ggaggcccgt     240
gagcgatgga acttcgactt tgtcaccgag acaccactgg agggtgactt cgcctgggag     300
cgtgtgcggg gccttggcct gcccaagctc taccttccca cggggccccg gcgaggccgg     360
gatgagttgg gaggaggcag gcggcctggc acctcacctg ctctgctgca ggggacagca     420
gaggaagacc atgtggacct gtcactgtct tgtacccttg tgcctcgctc aggggagcag     480
gctgaagggt ccccaggtgg acctggagac tctcagggtc gaaaacggcg gcagaccagc     540
atgacagatt tctaccactc caaacgccgg ctgatcttct ccaagaggaa gccctaatcc     600
gcccacagga agcctgcagt cctggaagcg cgagggcctc aaaggcccgc tctacatctt     660
ctgccttagt ctcagtttgt gtgtcttaat tattatttgt gttttaattt aaacacctcc     720
tcatgtacat accctggccg cccccctgccc cccagcctct ggcattagaa ttatttaaac     780
aaaaactagg cggttgaatg agaggttcct aagagtgctg ggcatttttta ttttatgaaa     840
tactatttaa agcctcctca tcccgtgttc tccttttcct ctctcccgga ggttgggtgg     900
gccggcttca tgccagctac ttcctcctcc ccacttgtcc gctgggtggt accctctgga     960
ggggtgtggc tccttcccat cgctgtcaca ggcggttatg aaattcaccc cctttcctgg    1020
acactcagac ctgaattctt tttcatttga gaagtaaaca gatggcactt tgaaggggcc    1080
tcaccgagtg ggggcatcat caaaaacttt ggagtcccct cacctcctct aaggttgggc    1140
agggtgaccc tgaagtgagc acagcctagg gctgagctgg ggacctggta ccctcctggc    1200
tcttgatacc cccctctgtc ttgtgaaggc agggggaagg tggggtcctg gagcagacca    1260
ccccgcctgc cctcatggcc cctctgacct gcactgggga gcccgtctca gtgttgagcc    1320
ttttccctct ttggctcccc tgtacctttt gaggagcccc agctaccctt cttctccagc    1380
tgggctctgc aattcccctc tgctgctgtc cctccccctt gtcctttccc ttcagtaccc    1440
tctcagctcc aggtggctct gaggtgcctg tcccaccccc acccccagct caatggactg    1500
gaaggggaag ggacacacaa gaagaagggc accctagttc tacctcaggc agctcaagca    1560
gcgaccgccc cctcctctag ctgtgggggt gagggtccca tgtggtggca caggccccct    1620
tgagtggggt tatctctgtg ttaggggtat atgatggggg agtagatctt tctaggaggg    1680
agacactggc ccctcaaatc gtccagcgac cttcctcatc caccccatcc ctccccagtt    1740
```

cattgcactt tgattagcag cggaacaagg agtcagacat tttaagatgg tggcagtaga 1800
ggctatggac agggcatgcc acgtgggctc atatggggct gggagtagtt gtctttcctg 1860
gcactaacgt tgagcccctg gaggcactga agtgcttagt gtacttggag tattggggtc 1920
tgaccccaaa caccttccag ctcctgtaac atactggcct ggactgtttt ctctcggctc 1980
cccatgtgtc ctggttcccg tttctccacc tagactgtaa acctctcgag ggcagggacc 2040
acaccctgta ctgttctgtg tctttcacag ctcctcccac aatgctgaat atacagcagg 2100
tgctcaataa atgattctta gtgactttac ttgtaatatt 2140

<210> 129
<211> 2108
<212> DNA
<213> Artificial

<400> 129

aacatgttga gctctggcat agaagaggct ggtggctatt ttgtccttgg gctgcctgtt 60
ttcaggcgcc atgtcagaac cggctgggga tgtccgtcag aacccatgcg gcagcaaggc 120
ctgccgccgc ctcttcggcc cagtggacag cgagcagctg agccgcgact gtgatgcgct 180
aatggcgggc tgcatccagg aggcccgtga gcgatggaac ttcgactttg tcaccgagac 240
accactggag ggtgacttcg cctgggagcg tgtgcggggc cttggcctgc ccaagctcta 300
ccttcccacg gggccccggc gaggccggga tgagttggga ggaggcaggc ggcctggcac 360
ctcacctgct ctgctgcagg ggacagcaga ggaagaccat gtggacctgt cactgtcttg 420
taccettgtg cctcgctcag gggagcaggc tgaagggtcc ccaggtggac ctggagactc 480
tcagggtcga aaacggcggc agaccagcat gacagatttc taccactcca aacgccggct 540
gatcttctcc aagaggaagc cctaatccgc ccacaggaag cctgcagtcc tggaagcgcg 600
agggcctcaa aggcccgctc tacatcttct gccttagtct cagtttgtgt gtcttaatta 660
ttatttgtgt tttaatttaa acacctcctc atgtacatac cctggccgcc ccctgccccc 720
cagcctctgg cattagaatt atttaaacaa aaactaggcg gttgaatgag aggttcctaa 780
gagtgctggg catttttatt ttatgaaata ctatttaaag cctcctcatc ccgtgttctc 840
cttttcctct ctcccggagg ttgggtgggc cggcttcatg ccagctactt cctcctcccc 900
acttgtccgc tgggtggtac cctctggagg ggtgtggctc cttcccatcg ctgtcacagg 960
cggttatgaa attcaccccc tttcctggac actcagacct gaattctttt tcatttgaga 1020
agtaaacaga tggcactttg aaggggcctc accgagtggg ggcatcatca aaaactttgg 1080
agtcccctca cctcctctaa ggttgggcag ggtgaccctg aagtgagcac agcctagggc 1140
tgagctgggg acctggtacc ctcctggctc ttgatacccc cctctgtctt gtgaaggcag 1200
ggggaaggtg ggtcctgga gcagaccacc ccgcctgccc tcatggcccc tctgacctgc 1260
actggggagc ccgtctcagt gttgagcctt ttccctcttt ggctcccctg tacctttga 1320
ggagccccag ctacccttct tctccagctg ggctctgcaa ttcccctctg ctgctgtccc 1380

tccccttgt cctttccctt cagtaccctc tcagctccag gtggctctga ggtgcctgtc 1440

ccaccccac ccccagctca atggactgga aggggaaggg acacacaaga agaagggcac 1500

cctagttcta cctcaggcag ctcaagcagc gaccgccccc tcctctagct gtgggggtga 1560

gggtcccatg tggtggcaca ggccccttg agtggggtta tctctgtgtt aggggtatat 1620

gatgggggag tagatcttc taggagggag acactggccc ctcaaatcgt ccagcgacct 1680

tcctcatcca ccccatccct ccccagttca ttgcactttg attagcagcg gaacaaggag 1740

tcagacattt taagatggtg gcagtagagg ctatggacag ggcatgccac gtgggctcat 1800

atggggctgg gagtagttgt ctttcctggc actaacgttg agcccctgga ggcactgaag 1860

tgcttagtgt acttggagta ttggggtctg accccaaaca ccttccagct cctgtaacat 1920

actggcctgg actgtttct ctcggctccc catgtgtcct ggttcccgtt tctccaccta 1980

gactgtaaac ctctcgaggg cagggaccac accctgtact gttctgtgtc tttcacagct 2040

cctcccacaa tgctgaatat acagcaggtg ctcaataaat gattcttagt gactttactt 2100

gtaatatt 2108

<210> 130
<211> 2275
<212> DNA
<213> Artificial

<400> 130

gaggcgggcc cgggcggggc ggttgtatat cagggccgcg ctgagctgcg ccagctgagg 60
tgtgagcagc tgccgaagtc agttccttgt ggagccggag ctgggcgcgg attcgccgag 120
gcaccgaggc actcagagga gaaactgcaa gttgggactt gtccctagga aaatccagtt 180
gctgccaagg tcgtgcagtc actcagccct ggagtcaagc cagagcaggc aggcgccatg 240
tcagaaccgg ctggggatgt ccgtcagaac ccatgcggca gcaaggcctg ccgccgcctc 300
ttcggcccag tggacagcga gcagctgagc cgcgactgtg atgcgctaat ggcgggctgc 360
atccaggagg cccgtgagcg atggaacttc gactttgtca ccgagacacc actggagggt 420
gacttcgcct gggagcgtgt gcggggcctt ggcctgccca agctctacct tcccacgggg 480
ccccggcgag gccgggatga gttgggagga ggcaggcggc ctggcacctc acctgctctg 540
ctgcagggga cagcagagga agaccatgtg gacctgtcac tgtcttgtac ccttgtgcct 600
cgctcagggg agcaggctga agggtcccca ggtggacctg gagactctca gggtcgaaaa 660
cggcggcaga ccagcatgac agatttctac cactccaaac gccggctgat cttctccaag 720
aggaagccct aatccgccca caggaagcct gcagtcctgg aagcgcgagg gcctcaaagg 780
cccgctctac atcttctgcc ttagtctcag tttgtgtgtc ttaattatta tttgtgtttt 840
aatttaaaca cctcctcatg tacataccct ggccgccccc tgccccccag cctctggcat 900
tagaattatt taaacaaaaa ctaggcggtt gaatgagagg ttcctaagag tgctgggcat 960
ttttatttta tgaaatacta tttaaagcct cctcatcccg tgttctcctt ttcctctctc 1020

ccggaggttg ggtgggccgg cttcatgcca gctacttcct cctccccact tgtccgctgg 1080
gtggtaccct ctggaggggt gtggctcctt cccatcgctg tcacaggcgg ttatgaaatt 1140
cacccccttt cctggacact cagacctgaa ttctttttca tttgagaagt aaacagatgg 1200
cactttgaag gggcctcacc gagtgggggc atcatcaaaa actttggagt cccctcacct 1260
cctctaaggt tgggcagggt gaccctgaag tgagcacagc ctagggctga gctggggacc 1320
tggtaccctc ctggctcttg ataccccccct ctgtcttgtg aaggcagggg gaaggtgggg 1380
tcctggagca gaccaccccg cctgccctca tggcccctct gacctgcact ggggagcccg 1440
tctcagtgtt gagccttttc cctctttggc tcccctgtac cttttgagga gccccagcta 1500
cccttcttct ccagctgggc tctgcaattc ccctctgctg ctgtccctcc cccttgtcct 1560
ttcccttcag taccctctca gctccaggtg gctctgaggt gcctgtccca cccccacccc 1620
cagctcaatg gactggaagg ggaagggaca cacaagaaga agggcaccct agttctacct 1680
caggcagctc aagcagcgac cgccccctcc tctagctgtg ggggtgaggg tcccatgtgg 1740
tggcacaggc cccccttgagt ggggttatct ctgtgttagg ggtatatgat gggggagtag 1800
atctttctag gagggagaca ctggcccctc aaatcgtcca gcgaccttcc tcatccaccc 1860
catccctccc cagttcattg cactttgatt agcagcggaa caaggagtca gacattttaa 1920
gatggtggca gtagaggcta tggacagggc atgccacgtg ggctcatatg gggctgggag 1980
tagttgtctt tcctggcact aacgttgagc ccctggaggc actgaagtgc ttagtgtact 2040
tggagtattg gggtctgacc ccaaacacct tccagctcct gtaacatact ggcctggact 2100
gttttctctc ggctccccat gtgtcctggt tcccgtttct ccacctagac tgtaaacctc 2160
tcgagggcag ggaccacacc ctgtactgtt ctgtgtcttt cacagctcct cccacaatgc 2220
tgaatataca gcaggtgctc aataaatgat tcttagtgac tttacttgta atatt 2275

<210> 131
<211> 2248
<212> DNA
<213> Artificial

<400> 131

gaggcgggcc cgggcggggc ggttgtatat cagggccgcg ctgagctgcg ccagctgagg 60
tgtgagcagc tgccgaagtc agttccttgt ggagccggag ctgggcgcgg attcgccgag 120
gcaccgaggc actcagagga ggaaaatcca gttgctgcca aggtcgtgca gtcactcagc 180
cctggagtca agccagagca ggcaggcgcc atgtcagaac cggctgggga tgtccgtcag 240
aacccatgcg gcagcaaggc ctgccgccgc ctcttcggcc cagtggacag cgagcagctg 300
agccgcgact gtgatgcgct aatggcgggc tgcatccagg aggcccgtga gcgatggaac 360
ttcgactttg tcaccgagac accactggag ggtgacttcg cctgggagcg tgtgcggggc 420
cttggcctgc ccaagctcta ccttcccacg gggccccggc gaggccggga tgagttggga 480
ggaggcaggc ggcctggcac ctcacctgct ctgctgcagg ggacagcaga ggaagaccat 540

gtggacctgt cactgtcttg tacccttgtg cctcgctcag gggagcaggc tgaagggtcc 600
ccaggtggac ctggagactc tcagggtcga aaacggcggc agaccagcat gacagatttc 660
taccactcca aacgccggct gatcttctcc aagaggaagc cctaatccgc ccacaggaag 720
cctgcagtcc tggaagcgcg agggcctcaa aggcccgctc tacatcttct gccttagtct 780
cagtttgtgt gtcttaatta ttatttgtgt tttaatttaa acacctcctc atgtacatac 840
cctggccgcc ccctgccccc cagcctctgg cattagaatt atttaaacaa aaactaggcg 900
gttgaatgag aggttcctaa gagtgctggg catttttatt ttatgaaata ctatttaaag 960
cctcctcatc ccgtgttctc cttttcctct ctcccggagg ttgggtgggc cggcttcatg 1020
ccagctactt cctcctcccc acttgtccgc tgggtggtac cctctggagg ggtgtggctc 1080
cttcccatcg ctgtcacagg cggttatgaa attcaccccc tttcctggac actcagacct 1140
gaattctttt tcatttgaga agtaaacaga tggcactttg aaggggcctc accgagtggg 1200
ggcatcatca aaaactttgg agtcccctca cctcctctaa ggttgggcag ggtgaccctg 1260
aagtgagcac agcctagggc tgagctgggg acctggtacc ctcctggctc ttgatacccc 1320
cctctgtctt gtgaaggcag gggaaggtg gggtcctgga gcagaccacc ccgcctgccc 1380
tcatggcccc tctgacctgc actggggagc ccgtctcagt gttgagcctt ttccctcttt 1440
ggctcccctg taccttttga ggagccccag ctacccttct tctccagctg ggctctgcaa 1500
ttcccctctg ctgctgtccc tcccccttgt cctttccctt cagtaccctc tcagctccag 1560
gtggctctga ggtgcctgtc ccacccccac ccccagctca atggactgga aggggaaggg 1620
acacacaaga agaagggcac cctagttcta cctcaggcag ctcaagcagc gaccgccccc 1680
tcctctagct gtgggggtga gggtcccatg tggtggcaca ggcccccttg agtggggtta 1740
tctctgtgtt aggggtatat gatgggggag tagatctttc taggagggag acactggccc 1800
ctcaaatcgt ccagcgacct tcctcatcca ccccatccct ccccagttca ttgcactttg 1860
attagcagcg gaacaaggag tcagacattt taagatggtg gcagtagagg ctatggacag 1920
ggcatgccac gtgggctcat atggggctgg gagtagttgt ctttcctggc actaacgttg 1980
agcccctgga ggcactgaag tgcttagtgt acttggagta ttggggtctg accccaaaca 2040
ccttccagct cctgtaacat actggcctgg actgttttct ctcggctccc catgtgtcct 2100
ggttcccgtt tctccaccta gactgtaaac ctctcgaggg cagggaccac accctgtact 2160
gttctgtgtc tttcacagct cctcccacaa tgctgaatat acagcaggtg ctcaataaat 2220
gattcttagt gactttactt gtaatatt 2248

<210> 132
<211> 2154
<212> DNA
<213> Artificial

<400> 132

aacatgttga gctctggcat agaagaggct ggtggctatt ttgtccttgg gctgcctgtt 60

ttcaggtgag gaaggggatg gtaggagaca ggagacctct aaagacccca ggcgccatgt 120
cagaaccggc tggggatgtc cgtcagaacc catgcggcag caaggcctgc cgccgcctct 180
tcggcccagt ggacagcgag cagctgagcc gcgactgtga tgcgctaatg gcgggctgca 240
tccaggaggc ccgtgagcga tggaacttcg actttgtcac cgagacacca ctggagggtg 300
acttcgcctg ggagcgtgtg cggggccttg gcctgcccaa gctctacctt cccacggggc 360
cccggcgagg ccgggatgag ttgggaggag gcaggcggcc tggcacctca cctgctctgc 420
tgcaggggac agcagaggaa gaccatgtgg acctgtcact gtcttgtacc cttgtgcctc 480
gctcagggga gcaggctgaa gggtccccag gtggacctgg agactctcag ggtcgaaaac 540
ggcggcagac cagcatgaca gatttctacc actccaaacg ccggctgatc ttctccaaga 600
ggaagcccta atccgcccac aggaagcctg cagtcctgga agcgcgaggg cctcaaaggc 660
ccgctctaca tcttctgcct tagtctcagt ttgtgtgtct taattattat ttgtgtttta 720
atttaaacac ctcctcatgt acataccctg gccgcccccct gccccccagc ctctggcatt 780
agaattattt aaacaaaaac taggcggttg aatgagaggt tcctaagagt gctgggcatt 840
tttattttat gaaatactat ttaaagcctc ctcatcccgt gttctccttt tcctctctcc 900
cggaggttgg gtgggccggc ttcatgccag ctacttcctc ctccccactt gtccgctggg 960
tggtaccctc tggaggggtg tggctccttc ccatcgctgt cacaggcggt tatgaaattc 1020
acccccttc ctggacactc agacctgaat tctttttcat ttgagaagta aacagatggc 1080
actttgaagg ggcctcaccg agtgggggca tcatcaaaaa ctttggagtc ccctcacctc 1140
ctctaaggtt gggcagggtg accctgaagt gagcacagcc tagggctgag ctggggacct 1200
ggtaccctcc tggctcttga taccccctc tgtcttgtga aggcaggggg aaggtggggt 1260
cctggagcag accaccccgc ctgccctcat ggcccctctg acctgcactg gggagcccgt 1320
ctcagtgttg agccttttcc ctctttggct cccctgtacc ttttgaggag ccccagctac 1380
ccttcttctc cagctgggct ctgcaattcc cctctgctgc tgtccctccc ccttgtcctt 1440
tcccttcagt accctctcag ctccaggtgg ctctgaggtg cctgtcccac ccccaccccc 1500
agctcaatgg actggaaggg gaagggacac acaagaagaa gggcacccta gttctacctc 1560
aggcagctca agcagcgacc gccccctcct ctagctgtgg gggtgagggt cccatgtggt 1620
ggcacaggcc cccttgagtg gggttatctc tgtgttaggg gtatatgatg gggggagtaga 1680
tctttctagg agggagacac tggcccctca aatcgtccag cgaccttcct catccacccc 1740
atccctcccc agttcattgc actttgatta gcagcggaac aaggagtcag acattttaag 1800
atggtggcag tagaggctat ggacagggca tgccacgtgg gctcatatgg ggctgggagt 1860
agttgtcttt cctggcacta acgttgagcc cctggaggca ctgaagtgct tagtgtactt 1920
ggagtattgg ggtctgaccc caaacacctt ccagctcctg taacatactg gcctggactg 1980
ttttctctcg gctccccatg tgtcctggtt cccgtttctc cacctagact gtaaacctct 2040
cgagggcagg gaccacaccc tgtactgttc tgtgtctttc acagctcctc ccacaatgct 2100

gaatatacag caggtgctca ataaatgatt cttagtgact ttacttgtaa tatt 2154

<210> 133
<211> 2413
<212> DNA
<213> Artificial

<400> 133

gaggcgggcc cgggcggggc ggttgtatat cagggccgcg ctgagctgcg ccagctgagg 60
tgtgagcagc tgccgaagtc agttccttgt ggagccggag ctgggcgcgg attcgccgag 120
gcaccgaggc actcagagga ggcgccatgt cagaaccggc tggggatgtc cgtcagaacc 180
catgcggcag caaggcctgc cgccgcctct tcggcccagt ggacagcgag cagctgagcc 240
gcgactgtga tgcgctaatg gcgggctgca tccaggaggc ccgtgagcga tggaacttcg 300
actttgtcac cgagacacca ctggagggtg acttcgcctg ggagcgtgtg cggggccttg 360
gcctgcccaa gctctacctt cccacggggc cccggcgagg ccgggatgag ttgggaggag 420
gcaggcggcc tggcacctca cctgctctgc tgcaggggac agcagaggaa gaccatgtgg 480
acctgtcact gtcttgtacc cttgtgcctc gctcagggga gcaggctgaa gggtccccag 540
gtggacctgg agactctcag ggtcgaaaac ggcggcagac cagcatgaca ggtgcggaca 600
tgtgcacgga aggactttgt aagggaccag gattctcaga atccatggtc caagggctga 660
cctgtctggt cctggtccag catgctccag gtagaaggaa acaggcccag agaggggaag 720
caacctccct gaggtcacac agcaagtagg cagcaaagac caactagcta acatttattg 780
ggaatgttca ttatgccagg ccctttgcca agcttctaag atttctacca ctccaaacgc 840
cggctgatct tctccaagag gaagccctaa tccgcccaca ggaagcctgc agtcctggaa 900
gcgcgagggc ctcaaaggcc cgctctacat cttctgcctt agtctcagtt tgtgtgtctt 960
aattattatt tgtgttttaa tttaaacacc tcctcatgta cataccctgg ccgcccccctg 1020
ccccccagcc tctggcatta gaattattta aacaaaaact aggcggttga atgagaggtt 1080
cctaagagtg ctgggcattt ttattttatg aaatactatt taaagcctcc tcatcccgtg 1140
ttctcctttt cctctctccc ggaggttggg tgggccggct tcatgccagc tacttcctcc 1200
tccccacttg tccgctgggt ggtaccctct ggaggggtgt ggctccttcc catcgctgtc 1260
acaggcggtt atgaaattca cccccttcc tggacactca gacctgaatt ctttttcatt 1320
tgagaagtaa acagatggca ctttgaaggg gcctcaccga gtgggggcat catcaaaaac 1380
tttggagtcc cctcacctcc tctaaggttg ggcagggtga ccctgaagtg agcacagcct 1440
agggctgagc tggggacctg gtaccctcct ggctcttgat acccccctct gtcttgtgaa 1500
ggcaggggga aggtggggtc ctggagcaga ccaccccgcc tgccctcatg gcccctctga 1560
cctgcactgg ggagcccgtc tcagtgttga gccttttccc tctttggctc ccctgtacct 1620
tttgaggagc cccagctacc cttcttctcc agctgggctc tgcaattccc ctctgctgct 1680
gtccctcccc cttgtccttt cccttcagta ccctctcagc tccaggtggc tctgaggtgc 1740

ctgtcccacc cccacccccca gctcaatgga ctggaagggg aagggacaca caagaagaag 1800
ggcaccctag ttctacctca ggcagctcaa gcagcgaccg ccccctcctc tagctgtggg 1860
ggtgagggtc ccatgtggtg gcacaggccc ccttgagtgg ggttatctct gtgttagggg 1920
tatatgatgg gggagtagat ctttctagga gggagacact ggcccctcaa atcgtccagc 1980
gaccttcctc atccacccca tccctcccca gttcattgca ctttgattag cagcggaaca 2040
aggagtcaga cattttaaga tggtggcagt agaggctatg gacagggcat gccacgtggg 2100
ctcatatggg gctgggagta gttgtctttc ctggcactaa cgttgagccc ctggaggcac 2160
tgaagtgctt agtgtacttg gagtattggg gtctgacccc aaacaccttc cagctcctgt 2220
aacatactgg cctggactgt tttctctcgg ctccccatgt gtcctggttc ccgtttctcc 2280
acctagactg taaacctctc gagggcaggg accacaccct gtactgttct gtgtctttca 2340
cagctcctcc cacaatgctg aatatacagc aggtgctcaa taaatgattc ttagtgactt 2400
tacttgtaat att 2413

<210> 134
<211> 3542
<212> DNA
<213> Artificial

<400> 134

```
cgtggcctcc gctgggcagg gcgacgcgga accgaggcgg cggcggcggc tgcggcggca      60
ggaaatcggg gctcggcccc gccggcgcgc gaccccatcc ccatcccggt ccctgcagag     120
cgatccccgg gcccagatat ggacgcagca gagccgggac tccccccagg tcctgagggc     180
aggaagaggt acagtgacat cttccggagc ctggacaacc tcgaaatctc actggggaac     240
gtgacccttg agatgctggc tggagaccct ctactctcag aagacccaga acctgacaag     300
acccctacag ccactgttac caacgaagcc agctgttgga gcggcccctc cccagagggt     360
cctgtacccc tcacagggga ggaactggac ttgcggctca ttcggacaaa ggggggtgtg     420
gacgcagccc tggaatatgc caagacctgg agccgctatg ccaaggaact gcttgcctgg     480
actgaaaaga gagccagcta tgagctggag tttgctaaga gcaccatgaa gatcgctgaa     540
gctggcaagg tgtccattca acagcagagc cacatgcctc tgcagtacat ctacaccctg     600
tttctggagc acgatctcag cctgggaacc ctggccatgg agacagtggc ccagcagaaa     660
agagactact accagcccct cgccgccaaa cggactgaga ttgagaagtg gcggaaggag     720
ttcaaggagc agtggatgaa ggagcagaag cggatgaatg aggcggtgca ggcactgcgg     780
cgcgcccagc tgcagtatgt gcaacgcagc gaggacctgc gggcacgctc ccaggggtcc     840
cctgaggact cggcccccca ggcctcgccg ggacctagca agcagcagga gcggcggcgg     900
cgctcgcgag aggaggccca ggccaaggcg caggaggccg aggcgctgta ccaggcctgt     960
gtccgcgagg ccaacgcgcg gcagcaggac ctggagatcg ccaagcagcg aatcgtgtcg    1020
cacgtgcgca agctggtgtt tcagggggat gaagtgctga ggcgggtgac gctgagtctc    1080
```

ttcgggctgc ggggggcgca ggcagagcgt ggcccccgcg ccttcgccgc cctggccgag 1140
tgctgtgcgc cctttgagcc gggccagcgc taccaggagt ttgtacgggc gctgcggccc 1200
gaggccccgc cgcccccgcc gcccgccttc tccttccagg agttccttcc ctccttgaac 1260
agctcccctc tggacatcag aaagaagctc tctgggcctc ttcctccaag gctggatgag 1320
aattcagctg agccaggccc ttgggaggat ccgggcacag gctggcgctg gcaagggact 1380
ccaggcccca ctccgggcag cgatgtggac agcgtgggtg gcggcagcga gtctcggtcc 1440
ctggactcac ccacttccag cccaggcgct ggcacgaggc agctggtgaa ggcttcgtcc 1500
acaggcactg agtcctcaga tgactttgag gagcgagacc ctgacctggg agacgggctg 1560
gagaatgggc tgggcagccc cttcgggaag tggacactgt ccagcgcggc tcagacccac 1620
cagctgcggc gactgcgggg cccagccaag tgccgcgagt gcgaagcctt catggtcagc 1680
gggacggagt gtgaggagtg ctttctgacc tgccacaagc gctgcctgga gactctcctg 1740
atcctctgtg gacacaggcg gctcccagcc cggacacccc tttttggggt tgacttcctg 1800
cagctaccca gggacttccc ggaggaggta ccctttgtgg tcacgaagtg cacggctgag 1860
atagaacacc gtgccctgga tgtgcagggc atttaccggg tcagcgggtc ccgggtccgt 1920
gtggagcggc tgtgccaggc tttcgagaat ggccgagcgt tggtggagct gtcggggaac 1980
tcgcctcatg acgtctcgag tgtcctcaag cgatttcttc aggagctcac cgagcccgtg 2040
atccccttcc acctctacga cgccttcatc tctctggcta agaccttgca tgcagaccct 2100
ggggacgacc ctgggacccc cagccccagc cctgaggtta tccgctcgct gaagaccctc 2160
ttggtacagc tgcctgactc taactacaac accctgcggc acctggtggc ccatctgttc 2220
agggtggctg cacgatttat ggaaaacaag atgtctgcca acaacctggg cattgtgttt 2280
gggccgacac tgctgcggcc gccggacggc ccgcgggcag ccagcgccat ccctgtcacc 2340
tgcctgctgg actctgggca tcaggcccag cttgtggagt tcctcatcgt gcactacgag 2400
cagatctttg ggatggatga gctcccccag gccactgagc ccccgcccca agactccagc 2460
ccagcccctg ggcccctcac aaccagctcc caaccgccac ccccgcacct tgacccagac 2520
tcccagcccc cagtcctagc ctcagacccc ggcccagacc cccagcacca cagtaccctg 2580
gagcagcatc ccacggccac acctaccgag attccaactc cacagagtga ccagagagag 2640
gacgtggctg aagacaccaa agatggggga ggggaagtgt ccagccaagg cccagaggac 2700
tcactcctgg ggacacagtc tcgtggccac ttcagccgcc agccagtgaa gtatccccgg 2760
ggcggtgtga ggcctgtaac ccaccagctg tccagtctgg ccctggtggc ttccaagctg 2820
tgcgaggaga cccccatcac atcagtgccc agagggagtt tgcgggggcg ggggcccagc 2880
cctgcagctg cctcccctga gggcagcccc ctgcgccgca ccccgctgcc caagcatttt 2940
gagattaccc aggagacagc ccggctactc tcgaaattgg acagcgaggc tgtgcccagg 3000
gccacctgct gcccggacgt ccagcctgag gaagccgagg accatctctg accaccctgg 3060
caccttaaat aaggaagagg cccagattgt gaacacggac ccatatatcc ctacctccca 3120

ccacctagtg gccaaacacc ccgccaggag ttcaatgctg ggagaggtcc agagggttcc 3180
tataaggaaa aactatttaa tacatgacct aggggaggcc taaaaccctt ttggggataa 3240
tgtcccagag tcccccact agacacaggt cactgccaag catcagggcc actcgggctc 3300
agaggtcact cagggtcaat actcagggtc agtgcaggtt atgagatcct tggggtccac 3360
cctagtctct gacacctggg acaggggtgc ttttgctact ttggttgtgg tcactccccc 3420
acacctgcct gcctccttca cggactcgaa gtgaccttcc tggaggaggt gggcagctca 3480
gactccacat gctggtggtg cctgaggtct gatggcctct aataaactgt gtcctatatg 3540
cc 3542

<210> 135
<211> 3616
<212> DNA
<213> Artificial

<400> 135

```
cgtggcctcc gctgggcagg gcgacgcgga accgaggcgg cggcggcggc tgcggcggca      60
ggaaatcggg gctcggcccc gccggcgcgc gaccccatcc ccatcccggt ccctgcagag     120
cgatccccgg gcccagatat ggacgcagca gagccgggac tccccccagg tcctgagggc     180
aggaagaggt acagtgacat cttccggagc ctggacaacc tcgaaatctc actggggaac     240
gtgacccttg agatgctggc tggagaccct ctactctcag aagacccaga acctgacaag     300
acccctacag ccactgttac caacgaagcc agctgttgga gcggcccctc cccagagggt     360
cctgtacccc tcacagggga ggaactggac ttgcggctca ttcggacaaa gggggggtgtg     420
gacgcagccc tggaatatgc caagacctgg agccgctatg ccaaggaact gcttgcctgg     480
actgaaaaga gagccagcta tgagctggag tttgctaaga gcaccatgaa gatcgctgaa     540
gctggcaagg tgtccattca acagcagagc cacatgcctc tgcagtacat ctacaccctg     600
tttctggagc acgatctcag cctgggaacc ctggccatgg agacagtggc ccagcagaaa     660
agagactact accagcccct cgccgccaaa cggactgaga ttgagaagtg gcggaaggag     720
ttcaaggagc agtggatgaa ggagcagaag cggatgaatg aggcggtgca ggcactgcgg     780
cgcgcccagc tgcagtatgt gcaacgcagc gaggacctgc gggcacgctc ccaggggtcc     840
cctgaggact cggcccccca ggcctcgccg ggacctagca agcagcagga gcggcggcgg     900
cgctcgcgag aggaggccca ggccaaggcg caggaggccg aggcgctgta ccaggcctgt     960
gtccgcgagg ccaacgcgcg gcagcaggac ctggagatcg ccaagcagcg aatcgtgtcg    1020
cacgtgcgca agctggtgtt tcagggggat gaagtgctga ggcgggtgac gctgagtctc    1080
ttcgggctgc ggggggcgca ggcagagcgt ggcccccgcg ccttcgccgc cctggccgag    1140
tgctgtgcgc cctttgagcc gggccagcgc taccaggagt ttgtacgggc gctgcggccc    1200
gaggccccgc cgcccccgcc gcccgccttc tccttccagg agttccttcc ctccttgaac    1260
agctcccctc tggacatcag aaagaagctc tctgggcctc ttcctccaag gctggatgag    1320
```

aattcagctg agccaggccc ttgggaggat ccgggcacag gctggcgctg gcaagggact 1380
ccaggcccca ctccgggcag cgatgtggac agcgtgggtg gcggcagcga gtctcggtcc 1440
ctggactcac ccacttccag cccaggcgct ggcacgaggc agctggtgaa ggcttcgtcc 1500
acaggcactg agtcctcaga tgactttgag gagcgagacc ctggtgaggc tgaagcaggg 1560
taggtcagga tgggacaggg cagggcacca ggcactcctg accctgtctc cctgcagacc 1620
tgggagacgg gctggagaat gggctgggca gccccttcgg gaagtggaca ctgtccagcg 1680
cggctcagac ccaccagctg cggcgactgc ggggcccagc caagtgccgc gagtgcgaag 1740
ccttcatggt cagcgggacg gagtgtgagg agtgctttct gacctgccac aagcgctgcc 1800
tggagactct cctgatcctc tgtggacaca ggcggctccc agcccggaca ccccttttttg 1860
gggttgactt cctgcagcta cccagggact tcccggagga ggtacccttt gtggtcacga 1920
agtgcacggc tgagatagaa caccgtgccc tggatgtgca gggcatttac cgggtcagcg 1980
ggtcccgggt ccgtgtggag cggctgtgcc aggctttcga gaatggccga gcgttggtgg 2040
agctgtcggg gaactcgcct catgacgtct cgagtgtcct caagcgattt cttcaggagc 2100
tcaccgagcc cgtgatcccc ttccacctct acgacgcctt catctctctg gctaagacct 2160
tgcatgcaga ccctggggac gaccctggga cccccagccc cagccctgag gttatccgct 2220
cgctgaagac cctcttggta cagctgcctg actctaacta caacaccctg cggcacctgg 2280
tggcccatct gttcagggtg gctgcacgat ttatggaaaa caagatgtct gccaacaacc 2340
tgggcattgt gtttgggccg acactgctgc ggccgccgga cggcccgcgg gcagccagcg 2400
ccatccctgt cacctgcctg ctggactctg ggcatcaggc ccagcttgtg gagttcctca 2460
tcgtgcacta cgagcagatc tttgggatgg atgagctccc ccaggccact gagcccccgc 2520
cccaagactc cagcccagcc cctgggcccc tcacaaccag ctcccaaccg ccacccccgc 2580
accttgaccc agactcccag cccccagtcc tagcctcaga ccccggccca gacccccagc 2640
accacagtac cctggagcag catcccacgg ccacacctac cgagattcca actccacaga 2700
gtgaccagag agaggacgtg gctgaagaca ccaaagatgg gggaggggaa gtgtccagcc 2760
aaggcccaga ggactcactc ctggggacac agtctcgtgg ccacttcagc cgccagccag 2820
tgaagtatcc ccggggcggt gtgaggcctg taacccacca gctgtccagt ctggccctgg 2880
tggcttccaa gctgtgcgag gagaccccca tcacatcagt gcccagaggg agtttgcggg 2940
ggcgggggcc cagccctgca gctgcctccc ctgagggcag ccccctgcgc cgcacccccgc 3000
tgcccaagca ttttgagatt acccaggaga cagcccggct actctcgaaa ttggacagcg 3060
aggctgtgcc cagggccacc tgctgcccgg acgtccagcc tgaggaagcc gaggaccatc 3120
tctgaccacc ctggcacctt aaataaggaa gaggcccaga ttgtgaacac ggacccatat 3180
atccctacct cccaccacct agtggccaaa caccccgcca ggagttcaat gctgggagag 3240
gtccagaggg ttcctataag gaaaaactat ttaatacatg acctagggga ggcctaaaac 3300
ccttttgggg ataatgtccc agagtccccc cactagacac aggtcactgc caagcatcag 3360

ggccactcgg gctcagaggt cactcagggt caatactcag ggtcagtgca ggttatgaga 3420

tccttggggt ccaccctagt ctctgacacc tgggacaggg gtgcttttgc tactttggtt 3480

gtggtcactc ccccacacct gcctgcctcc ttcacggact cgaagtgacc ttcctggagg 3540

aggtgggcag ctcagactcc acatgctggt ggtgcctgag gtctgatggc ctctaataaa 3600

ctgtgtccta tatgcc 3616

<210> 136
<211> 3540
<212> DNA
<213> Artificial

<400> 136

cgtggcctcc gctgggcagg gcgacgcgga accgaggcgg cggcggcggc tgcggcggca 60
ggaaatcggg gctcggcccc gccggcgcgc gaccccatcc ccatcccggt ccctgcagag 120
cgatccccgg gcccagatat ggacgcagca gagccgggac tccccccagg tcctgagggc 180
aggaagaggt acagtgacat cttccggagc ctggacaacc tcgaaatctc actggggaac 240
gacccttgag atgctggctg gagaccctct actctcagaa gacccagaac ctgacaagac 300
ccctacagcc actgttacca acgaagccag ctgttggagc ggcccctccc cagagggtcc 360
tgtacccctc acaggggagg aactggactt gcggctcatt cggacaaagg ggggtgtgga 420
cgcagccctg gaatatgcca agacctggag ccgctatgcc aaggaactgc ttgcctggac 480
tgaaaagaga gccagctatg agctggagtt tgctaagagc accatgaaga tcgctgaagc 540
tggcaaggtg tccattcaac agcagagcca catgcctctg cagtacatct acaccctgtt 600
tctggagcac gatctcagcc tgggaaccct ggccatggag acagtggccc agcagaaaag 660
agactactac cagcccctcg ccgccaaacg gactgagatt gagaagtggc ggaaggagtt 720
caaggagcag tggatgaagg agcagaagcg gatgaatgag gcggtgcagg cactgcggcg 780
cgcccagctg cagtatgtgc aacgcagcga ggacctgcgg gcacgctccc aggggtcccc 840
tgaggactcg gccccccagg cctcgccggg acctagcaag cagcaggagc ggcggcggcg 900
ctcgcgagag gaggcccagg ccaaggcgca ggaggccgag gcgctgtacc aggcctgtgt 960
ccgcgaggcc aacgcgcggc agcaggacct ggagatcgcc aagcagcgaa tcgtgtcgca 1020
cgtgcgcaag ctggtgtttc agggggatga agtgctgagg cgggtgacgc tgagtctctt 1080
cgggctgcgg ggggcgcagg cagagcgtgg cccccgcgcc ttcgccgccc tggccgagtg 1140
ctgtgcgccc tttgagccgg gccagcgcta ccaggagttt gtacgggcgc tgcggcccga 1200
ggccccgccg cccccgccgc ccgccttctc cttccaggag ttccttccct ccttgaacag 1260
ctcccctctg gacatcagaa agaagctctc tgggcctctt cctccaaggc tggatgagaa 1320
ttcagctgag ccaggccctt gggaggatcc gggcacaggc tggcgctggc aagggactcc 1380
aggccccact ccgggcagcg atgtggacag cgtgggtggc ggcagcgagt ctcggtccct 1440
ggactcaccc acttccagcc caggcgctgg cacgaggcag ctggtgaagg cttcgtccac 1500

aggcactgag tcctcagatg actttgagga gcgagaccct gacctgggag acgggctgga 1560
gaatgggctg ggcagcccct tcgggaagtg gacactgtcc agcgcggctc agacccacca 1620
gctgcggcga ctgcggggcc cagccaagtg ccgcgagtgc gaagccttca tggtcagcgg 1680
gacggagtgt gaggagtgct ttctgacctg ccacaagcgc tgcctggaga ctctcctgat 1740
cctctgtgga cacaggcggc tcccagcccg gacacccctt tttggggttg acttcctgca 1800
gctacccagg gacttcccgg aggaggtacc ctttgtggtc acgaagtgca cggctgagat 1860
agaacaccgt gccctggatg tgcagggcat ttaccgggtc agcgggtccc gggtccgtgt 1920
ggagcggctg tgccaggctt tcgagaatgg ccgagcgttg gtggagctgt cggggaactc 1980
gcctcatgac gtctcgagtg tcctcaagcg atttcttcag gagctcaccg agcccgtgat 2040
ccccttccac ctctacgacg ccttcatctc tctggctaag accttgcatg cagaccctgg 2100
ggacgaccct gggacccca gccccagccc tgaggttatc cgctcgctga agaccctctt 2160
ggtacagctg cctgactcta actacaacac cctgcggcac ctggtggccc atctgttcag 2220
ggtggctgca cgatttatgg aaaacaagat gtctgccaac aacctgggca ttgtgtttgg 2280
gccgacactg ctgcggccgc cggacggccc gcgggcagcc agcgccatcc ctgtcacctg 2340
cctgctggac tctgggcatc aggcccagct tgtggagttc ctcatcgtgc actacgagca 2400
gatctttggg atggatgagc tcccccaggc cactgagccc ccgccccaag actccagccc 2460
agcccctggg cccctcacaa ccagctccca accgccaccc ccgcaccttg acccagactc 2520
ccagccccca gtcctagcct cagaccccgg cccagacccc cagcaccaca gtaccctgga 2580
gcagcatccc acggccacac ctaccgagat tccaactcca cagagtgacc agagagagga 2640
cgtggctgaa gacaccaaag atgggggagg ggaagtgtcc agccaaggcc cagaggactc 2700
actcctgggg acacagtctc gtggccactt cagccgccag ccagtgaagt atccccgggg 2760
cggtgtgagg cctgtaaccc accagctgtc cagtctggcc ctggtggctt ccaagctgtg 2820
cgaggagacc cccatcacat cagtgcccag agggagtttg cgggggcggg ggcccagccc 2880
tgcagctgcc tcccctgagg gcagccccct gcgccgcacc ccgctgccca agcattttga 2940
gattacccag gagacagccc ggctactctc gaaattggac agcgaggctg tgcccagggc 3000
cacctgctgc ccggacgtcc agcctgagga agccgaggac catctctgac caccctggca 3060
ccttaaataa ggaagaggcc cagattgtga acacggaccc atatatccct acctcccacc 3120
acctagtggc caaacacccc gccaggagtt caatgctggg agaggtccag agggttccta 3180
taaggaaaaa ctatttaata catgacctag gggaggccta aaaccctttt ggggataatg 3240
tcccagagtc cccccactag acacaggtca ctgccaagca tcagggccac tcgggctcag 3300
aggtcactca gggtcaatac tcagggtcag tgcaggttat gagatccttg ggtccaccc 3360
tagtctctga cacctgggac aggggtgctt ttgctacttt ggttgtggtc actcccccac 3420
acctgcctgc ctccttcacg gactcgaagt gaccttcctg gaggaggtgg gcagctcaga 3480
ctccacatgc tggtggtgcc tgaggtctga tggcctctaa taaactgtgt cctatatgcc 3540

<210> 137
<211> 3345
<212> DNA
<213> Artificial

<400> 137

```
cgtggcctcc gctgggcagg gcgacgcgga accgaggcgg cggcggcggc tgcggcggca    60
ggaaatcggg gctcggcccc gccggcgcgc gaccccatcc ccatcccggt ccctgcagag   120
cgatccccgg gcccagatat ggacgcagca gagccgggac tccccccagg tcctgagggc   180
aggaagaggt acagtgacat cttccggagc ctggacaacc tcgaaatctc actggggaac   240
gtgacccttg agatgctggc tggagaccct ctactctcag aagacccaga acctgacaag   300
acccctacag ccactgttac caacgaagcc agctgttgga gcggcccctc cccagagggt   360
cctgtacccc tcacagggga ggaactggac ttgcggctca ttcggacaaa gggggggtgtg   420
gacgcagccc tggaatatgc caagacctgg agccgctatg ccaaggaact gcttgcctgg   480
actgaaaaga gagccagcta tgagctggag tttgctaaga gcaccatgaa gatcgctgaa   540
gctggcaagg tgtccattca acagcagagc cacatgcctc tgcagtacat ctacaccctg   600
tttctggagc acgatctcag cctgggaacc ctggccatgg agacagtggc ccagcagaaa   660
agagactact accagcccct cgccgccaaa cggactgaga ttgagaagtg gcggaaggag   720
ttcaaggagc agtggatgaa ggagcagaag cggatgaatg aggcggtgca ggcactgcgg   780
cgcgcccagc tgcagtatgt gcaacgcagc gaggacctgc gggcacgctc ccaggggtcc   840
cctgaggact cggcccccca ggcctcgccg ggacctagca agcagcagga gcggcggcgg   900
cgctcgcgag aggaggccca ggccaaggcg caggaggccg aggcgctgta ccaggcctgt   960
gtccgcgagg ccaacgcgcg gcagcaggac ctggagatcg ccaagcagcg aatcgtgtcg  1020
cacgtgcgca agctggtgtt tcagggggat gaagtgctga ggcgggtgac gctgagtctc  1080
ttcgggctgc ggggggcgca ggcagagcgt ggcccccgcg ccttcgccgc cctggccgag  1140
tgctgtgcgc cctttgagcc gggccagcgc taccaggagt ttgtacgggc gctgcggccc  1200
gaggccccgc cgcccccgcc gcccgccttc tccttccagg agttccttcc ctccttgaac  1260
agctcccctc tggacatcag aaagaagctc tctgggcctc ttcctccaag gctggatgag  1320
aattcagctg agccaggccc ttgggaggat ccgggcacag gctggcgctg gcaagggact  1380
ccaggcccca ctccgggcag cgatgtggac agcgtgggtg gcggcagcga gtctcggtcc  1440
ctggactcac ccacttccag cccaggcgct ggcacgaggc agctggtgaa ggcttcgtcc  1500
acaggcactg agtcctcaga tgactttgag gagcgagacc ctgacctggg agacgggctg  1560
gagaatgggc tgggcagccc cttcgggaag tggacactgt ccagcgcggc tcagacccac  1620
cagctgcggc gactgcgggg cccagccaag tgccgcgagt gcgaagcctt catggtcagc  1680
gggacggagt gtgaggagtg ctttctgacc tgccacaagc gctgcctgga gactctcctg  1740
atcctctgtg gacacaggcg gctcccagcc cggacacccc tttttggggt tgacttcctg  1800
```

cagctaccca gggacttccc ggaggaggta ccctttgtgg tcacgaagtg cacggctgag 1860
atagaacacc gtgccctgga tgtgcagggc atttaccggg tcagcgggtc ccgggtccgt 1920
gtggagcggc tgtgccaggc tttcgagaat ggccgagcgt tggtggagct gtcggggaac 1980
tcgcctcatg acgtctcgag tgtcctcaag cgatttcttc aggagggtgg ctgcacgatt 2040
tatggaaaac aagatgtctg ccaacaacct gggcattgtg tttgggccga cactgctgcg 2100
gccgccggac ggcccgcggg cagccagcgc catccctgtc acctgcctgc tggactctgg 2160
gcatcaggcc cagcttgtgg agttcctcat cgtgcactac gagcagatct ttgggatgga 2220
tgagctcccc caggccactg agcccccgcc ccaagactcc agcccagccc ctgggcccct 2280
cacaaccagc tcccaaccgc cacccccgca ccttgaccca gactcccagc cccagtcct 2340
agcctcagac cccggcccag acccccagca ccacagtacc ctggagcagc atcccacggc 2400
cacacctacc gagattccaa ctccacagag tgaccagaga gaggacgtgg ctgaagacac 2460
caaagatggg ggaggggaag tgtccagcca aggcccagag gactcactcc tggggacaca 2520
gtctcgtggc cacttcagcc gccagccagt gaagtatccc cggggcggtg tgaggcctgt 2580
aacccaccag ctgtccagtc tggccctggt ggcttccaag ctgtgcgagg agacccccat 2640
cacatcagtg cccagaggga gtttgcgggg gcgggggccc agccctgcag ctgcctcccc 2700
tgagggcagc cccctgcgcc gcaccccgct gcccaagcat tttgagatta cccaggagac 2760
agcccggcta ctctcgaaat tggacagcga ggctgtgccc agggccacct gctgcccgga 2820
cgtccagcct gaggaagccg aggaccatct ctgaccaccc tggcacctta aataaggaag 2880
aggcccagat tgtgaacacg gacccatata tccctacctc ccaccaccta gtggccaaac 2940
accccgccag gagttcaatg ctgggagagg tccagagggt tcctataagg aaaaactatt 3000
taatacatga cctaggggag gcctaaaacc cttttgggga taatgtccca gagtcccccc 3060
actagacaca ggtcactgcc aagcatcagg gccactcggg ctcagaggtc actcagggtc 3120
aatactcagg gtcagtgcag gttatgagat ccttggggtc caccctagtc tctgacacct 3180
gggacagggg tgcttttgct actttggttg tggtcactcc cccacacctg cctgcctcct 3240
tcacggactc gaagtgacct tcctggagga ggtgggcagc tcagactcca catgctggtg 3300
gtgcctgagg tctgatggcc tctaataaac tgtgtcctat atgcc 3345

<210> 138
<211> 124
<212> PRT
<213> Artificial

<400> 138

Met Ser Ser Ala Leu Glu Ser Val Thr Val Ser Asp Arg Pro Leu Gly
1 5 10 15

Val Ser Ile Thr Lys Ala Glu Val Ala Pro Glu Glu Asp Glu Arg Lys
20 25 30

Lys Arg Arg Arg Glu Arg Asn Lys Ile Ala Ala Ala Lys Cys Arg Asn
35 40 45

Lys Lys Lys Glu Lys Thr Glu Cys Leu Gln Lys Glu Ser Glu Lys Leu
50 55 60

Glu Ser Val Asn Ala Glu Leu Lys Ala Gln Ile Glu Glu Leu Lys Asn
65 70 75 80

Glu Lys Gln His Leu Ile Tyr Met Leu Asn Leu His Arg Pro Thr Cys
85 90 95

Ile Val Arg Ala Gln Asn Gly Arg Thr Pro Glu Asp Glu Arg Asn Leu
100 105 110

Phe Ile Gln Gln Ile Lys Glu Gly Thr Leu Gln Ser
115 120

<210> 139
<211> 181
<212> PRT
<213> Artificial

<400> 139

Met Met Leu Gln His Pro Gly Gln Val Ser Ala Ser Glu Val Ser Ala
1 5 10 15

Ser Ala Ile Val Pro Cys Leu Ser Pro Pro Gly Ser Leu Val Phe Glu
20 25 30

Asp Phe Ala Asn Leu Thr Pro Phe Val Lys Glu Glu Leu Arg Phe Ala
35 40 45

Ile Gln Asn Lys His Leu Cys His Arg Met Ser Ser Ala Leu Glu Ser
50 55 60

Val Thr Val Ser Asp Arg Pro Leu Gly Val Ser Ile Thr Lys Ala Glu
65 70 75 80

Val Ala Pro Glu Glu Asp Glu Arg Lys Lys Arg Arg Arg Glu Arg Asn
85 90 95

Lys Ile Ala Ala Ala Lys Cys Arg Asn Lys Lys Lys Glu Lys Thr Glu
100 105 110

Cys Leu Gln Lys Glu Ser Glu Lys Leu Glu Ser Val Asn Ala Glu Leu
115 120 125

Lys Ala Gln Ile Glu Glu Leu Lys Asn Glu Lys Gln His Leu Ile Tyr
130 135 140

Met Leu Asn Leu His Arg Pro Thr Cys Ile Val Arg Ala Gln Asn Gly
145 150 155 160

Arg Thr Pro Glu Asp Glu Arg Asn Leu Phe Ile Gln Gln Ile Lys Glu
165 170 175

Gly Thr Leu Gln Ser
180

<210> 140
<211> 340
<212> PRT
<213> Artificial

<400> 140

```
Met Ala Asp Ser Val Lys Thr Phe Leu Gln Asp Leu Ala Arg Gly Ile
1               5                   10                  15

Lys Asp Ser Ile Trp Gly Ile Cys Thr Ile Ser Lys Leu Asp Ala Arg
            20                  25                  30

Ile Gln Gln Lys Arg Glu Glu Gln Arg Arg Arg Arg Ala Ser Ser Val
        35                  40                  45

Leu Ala Gln Arg Arg Ala Gln Ser Ile Glu Arg Lys Gln Glu Ser Glu
    50                  55                  60

Pro Arg Ile Val Ser Arg Ile Phe Gln Cys Cys Ala Trp Asn Gly Gly
65                  70                  75                  80

Val Phe Trp Phe Ser Leu Leu Leu Phe Tyr Arg Val Phe Ile Pro Val
                85                  90                  95

Leu Gln Ser Val Thr Ala Arg Ile Ile Gly Asp Pro Ser Leu His Gly
            100                 105                 110

Asp Val Trp Ser Trp Leu Glu Phe Phe Leu Thr Ser Ile Phe Ser Ala
        115                 120                 125

Leu Trp Val Leu Pro Leu Phe Val Leu Ser Lys Val Val Asn Ala Ile
    130                 135                 140

Trp Phe Gln Asp Ile Ala Asp Leu Ala Phe Glu Val Ser Gly Arg Lys
145                 150                 155                 160

Pro His Pro Phe Pro Ser Val Ser Lys Ile Ile Ala Asp Met Leu Phe
                165                 170                 175

Asn Leu Leu Leu Gln Ala Leu Phe Leu Ile Gln Gly Met Phe Val Ser
            180                 185                 190
```

Leu Phe Pro Ile His Leu Val Gly Gln Leu Val Ser Leu Leu His Met
195 200 205

Ser Leu Leu Tyr Ser Leu Tyr Cys Phe Glu Tyr Arg Trp Phe Asn Lys
210 215 220

Gly Ile Glu Met His Gln Arg Leu Ser Asn Ile Glu Arg Asn Trp Pro
225 230 235 240

Tyr Tyr Phe Gly Phe Gly Leu Pro Leu Ala Phe Leu Thr Ala Met Gln
245 250 255

Ser Ser Tyr Ile Ile Ser Gly Cys Leu Phe Ser Ile Leu Phe Pro Leu
260 265 270

Phe Ile Ile Ser Ala Asn Glu Ala Lys Thr Pro Gly Lys Ala Tyr Leu
275 280 285

Phe Gln Leu Arg Leu Phe Ser Leu Val Val Phe Leu Ser Asn Arg Leu
290 295 300

Phe His Lys Thr Val Tyr Leu Gln Ser Ala Leu Ser Ser Ser Thr Ser
305 310 315 320

Ala Glu Lys Phe Pro Ser Pro His Pro Ser Pro Ala Lys Leu Lys Ala
325 330 335

Thr Ala Gly His
340

<210> 141
<211> 228
<212> PRT
<213> Artificial

<400> 141

Met Ala Asp Ser Val Lys Thr Phe Leu Gln Asp Leu Ala Arg Gly Ile
1 5 10 15

Lys Asp Ser Ile Trp Gly Ile Cys Thr Ile Ser Lys Leu Asp Ala Arg
20 25 30

Ile Gln Gln Lys Arg Glu Glu Gln Arg Arg Arg Arg Ala Ser Ser Val
35 40 45

Leu Ala Gln Arg Arg Ala Gln Ser Ile Glu Arg Lys Gln Glu Ser Glu
50 55 60

Pro Arg Ile Val Ser Arg Ile Phe Gln Cys Cys Ala Trp Asn Gly Gly
65 70 75 80

Val Phe Trp Phe Ser Leu Leu Leu Phe Tyr Arg Val Phe Ile Pro Val
85 90 95

Leu Gln Ser Val Thr Ala Arg Ile Ile Gly Asp Pro Ser Leu His Gly
100 105 110

Asp Val Trp Ser Trp Leu Glu Phe Phe Leu Thr Ser Ile Phe Ser Ala
115 120 125

Leu Trp Val Leu Pro Leu Phe Val Leu Ser Lys Val Val Asn Ala Ile
130 135 140

Trp Phe Gln Asp Ile Ala Asp Leu Ala Phe Glu Val Ser Gly Arg Lys
145 150 155 160

Pro His Pro Phe Pro Ser Val Ser Lys Ile Ile Ala Asp Met Leu Phe
165 170 175

Asn Leu Leu Leu Gln Ala Leu Phe Leu Ile Gln Gly Met Phe Val Ser
180 185 190

Leu Phe Pro Ile His Leu Val Gly Gln Leu Val Ser Leu Leu His Met
195 200 205

Ser Leu Leu Tyr Ser Leu Tyr Cys Phe Glu Tyr Arg Trp Phe Asn Lys
210 215 220

Gly Lys Ser Ile
225

<210> 142
<211> 342
<212> PRT
<213> Artificial

<400> 142

Met Ala Asp Ser Val Lys Thr Phe Leu Gln Asp Leu Ala Arg Gly Ile
1 5 10 15

Lys Asp Ser Ile Trp Gly Ile Cys Thr Ile Ser Lys Leu Asp Ala Arg
20 25 30

Ile Gln Gln Lys Arg Glu Glu Gln Arg Arg Arg Arg Ala Ser Ser Val
35 40 45

Leu Ala Gln Arg Arg Ala Gln Ser Ile Glu Arg Lys Gln Glu Arg Arg
50 55 60

Ile Leu Ala Leu Ser Pro Ser Cys His Leu Arg Leu Pro Trp Cys Asn
65 70 75 80

Leu Ser Leu Leu Gln Pro Leu Pro Pro Arg Ser Lys Gln Phe Phe Cys
85 90 95

Leu Ser Glu Pro Ala Gly Trp Lys Tyr Ser Glu Pro Arg Ile Val Ser
100 105 110

Arg Ile Phe Gln Cys Cys Ala Trp Asn Gly Gly Val Phe Trp Phe Ser
115 120 125

Leu Leu Leu Phe Tyr Arg Val Phe Ile Pro Val Leu Gln Ser Val Thr
130 135 140

Ala Arg Ile Ile Gly Asp Pro Ser Leu His Gly Asp Val Trp Ser Trp
145 150 155 160

Leu Glu Phe Phe Leu Thr Ser Ile Phe Ser Ala Leu Trp Val Leu Pro
165 170 175

Leu Phe Val Leu Ser Lys Val Val Asn Ala Ile Trp Phe Gln Asp Ile
180 185 190

Ala Asp Leu Ala Phe Glu Val Ser Gly Arg Lys Pro His Pro Phe Pro
195 200 205

Ser Val Ser Lys Ile Ile Ala Asp Met Leu Phe Asn Leu Leu Leu Gln
210 215 220

Ala Leu Phe Leu Ile Gln Gly Met Phe Val Ser Leu Phe Pro Ile His
225 230 235 240

Leu Val Gly Gln Leu Val Ser Leu Leu His Met Ser Leu Leu Tyr Ser
245 250 255

Leu Tyr Cys Phe Glu Tyr Arg Trp Phe Asn Lys Gly Ile Glu Met His
260 265 270

Gln Arg Leu Ser Asn Ile Glu Arg Asn Trp Pro Tyr Tyr Phe Gly Phe
275 280 285

Gly Leu Pro Leu Ala Phe Leu Thr Ala Met Gln Ser Ser Tyr Ile Ile
290 295 300

Ser Gly Cys Leu Phe Ser Ile Leu Phe Pro Leu Phe Ile Ile Ser Ala
305 310 315 320

Asn Glu Ala Lys Thr Leu Ala Lys His Ile Ser Ser Ser Cys Ala Ser
325 330 335

Ser Pro Trp Trp Ser Ser
340

<210> 143
<211> 152
<212> PRT
<213> Artificial

<400> 143

```
Met Ala Asp Ser Val Lys Thr Phe Leu Gln Asp Leu Ala Arg Gly Ile
1               5                   10                  15

Lys Asp Ser Ile Trp Gly Ile Cys Thr Ile Ser Lys Leu Asp Ala Arg
            20                  25                  30

Ile Gln Gln Lys Arg Glu Glu Gln Arg Arg Arg Arg Ala Ser Ser Val
        35                  40                  45

Leu Ala Gln Arg Arg Ala Gln Ser Ile Glu Arg Lys Gln Glu Arg Arg
    50                  55                  60

Ile Leu Ala Leu Ser Pro Ser Cys His Leu Arg Leu Pro Trp Cys Asn
65                  70                  75                  80

Leu Ser Leu Leu Gln Pro Leu Pro Pro Arg Ser Lys Gln Phe Phe Cys
                85                  90                  95

Leu Ser Glu Pro Ala Gly Trp Lys Tyr Arg Cys Ala Pro Pro Arg Leu
            100                 105                 110

Ala Asn Phe Leu Tyr Ser Leu Val Glu Thr Gly Phe Cys His Val Gly
        115                 120                 125

Gln Ala Gly Leu Glu Leu Leu Thr Ser Val Asp Arg Leu Pro Arg Pro
    130                 135                 140

Pro Lys Val Leu Gly Leu Gln Pro
145                 150
```

<210> 144
<211> 163
<212> PRT
<213> Artificial

<400> 144

```
Met Ala Asp Ser Val Lys Thr Phe Leu Gln Asp Leu Ala Arg Gly Ile
1               5                   10                  15

Lys Asp Ser Ile Trp Gly Ile Cys Thr Ile Ser Lys Leu Asp Ala Arg
            20                  25                  30

Ile Gln Gln Lys Arg Glu Glu Gln Arg Arg Arg Arg Ala Ser Ser Val
        35                  40                  45
```

```
Leu Ala Gln Arg Arg Ala Gln Ser Ile Glu Arg Lys Gln Glu Ser Glu
    50                  55                  60

Pro Arg Ile Val Ser Arg Ile Phe Gln Cys Cys Ala Trp Asn Gly Gly
65                  70                  75                  80

Val Phe Trp Phe Ser Leu Leu Leu Phe Tyr Arg Val Phe Ile Pro Val
                85                  90                  95

Leu Gln Ser Val Thr Ala Arg Ile Ile Gly Lys Cys Ile Pro Cys Ser
            100                 105                 110

Leu Ser Val Gly Asp Arg Val Gly Asp Asp Val Ser Val Ser Leu Ser
        115                 120                 125

Leu Leu Asn Ile Ser Cys Val Pro Gly Ala Phe Thr Val Tyr Phe Leu
    130                 135                 140

Phe Cys Cys Phe Ile Leu Phe Lys Asn Leu Tyr Phe Val Ile Leu Leu
145                 150                 155                 160

Glu Ala Leu
```

<210> 145
<211> 342
<212> PRT
<213> Artificial

<400> 145

```
Met Arg Lys Glu Thr Pro Pro Pro Leu Val Pro Pro Ala Ala Arg Glu
1               5                   10                  15

Trp Asn Leu Pro Pro Asn Ala Pro Ala Cys Met Glu Arg Gln Leu Glu
            20                  25                  30

Ala Ala Arg Tyr Arg Ser Asp Gly Ala Leu Leu Leu Gly Ala Ser Ser
        35                  40                  45

Leu Ser Gly Arg Cys Trp Ala Gly Ser Leu Trp Leu Phe Lys Asp Pro
    50                  55                  60

Cys Ala Ala Pro Asn Glu Gly Phe Cys Ser Ala Gly Val Gln Thr Glu
65                  70                  75                  80

Ala Gly Val Ala Asp Leu Thr Trp Val Gly Glu Arg Gly Ile Leu Val
                85                  90                  95

Ala Ser Asp Ser Gly Ala Val Glu Leu Trp Glu Leu Asp Glu Asn Glu
            100                 105                 110
```

```
Thr Leu Ile Val Ser Lys Phe Cys Lys Tyr Glu His Asp Asp Ile Val
        115                 120                 125

Ser Thr Val Ser Val Leu Ser Ser Gly Thr Gln Ala Val Ser Gly Ser
    130                 135                 140

Lys Asp Ile Cys Ile Lys Val Trp Asp Leu Ala Gln Gln Val Val Leu
145                 150                 155                 160

Ser Ser Tyr Arg Ala His Ala Ala Gln Val Thr Cys Val Ala Ala Ser
                165                 170                 175

Pro His Lys Asp Ser Val Phe Leu Ser Cys Ser Glu Asp Asn Arg Ile
            180                 185                 190

Leu Leu Trp Asp Thr Arg Cys Pro Lys Pro Ala Ser Gln Ile Gly Cys
        195                 200                 205

Ser Ala Pro Gly Tyr Leu Pro Thr Ser Leu Ala Trp His Pro Gln Gln
    210                 215                 220

Ser Glu Val Phe Val Phe Gly Asp Glu Asn Gly Thr Val Ser Leu Val
225                 230                 235                 240

Asp Thr Lys Ser Thr Ser Cys Val Leu Ser Ser Ala Val His Ser Gln
                245                 250                 255

Cys Val Thr Gly Leu Val Phe Ser Pro His Ser Val Pro Phe Leu Ala
            260                 265                 270

Ser Leu Ser Glu Asp Cys Ser Leu Ala Val Leu Asp Ser Ser Leu Ser
        275                 280                 285

Glu Leu Phe Arg Ser Gln Ala His Arg Asp Phe Val Arg Asp Ala Thr
    290                 295                 300

Trp Ser Pro Leu Asn His Ser Leu Leu Thr Thr Val Gly Trp Asp His
305                 310                 315                 320

Gln Val Val His His Val Val Pro Thr Glu Pro Leu Pro Ala Pro Gly
                325                 330                 335

Pro Ala Ser Val Thr Glu
            340
```

<210> 146
<211> 290
<212> PRT
<213> Artificial

<400> 146

Met Arg Lys Glu Thr Pro Pro Pro Leu Val Pro Pro Ala Ala Arg Glu
1               5                   10                  15

Trp Asn Leu Pro Pro Asn Ala Pro Ala Cys Met Glu Arg Gln Leu Glu
            20                  25                  30

Ala Ala Arg Tyr Arg Ser Asp Gly Ala Leu Leu Leu Gly Ala Ser Ser
        35                  40                  45

Leu Ser Gly Arg Cys Trp Ala Gly Ser Leu Trp Leu Phe Lys Asp Pro
    50                  55                  60

Cys Ala Ala Pro Asn Glu Gly Phe Cys Ser Ala Gly Val Gln Thr Glu
65                  70                  75                  80

Ala Gly Val Ala Asp Leu Thr Trp Val Gly Glu Arg Gly Ile Leu Val
                85                  90                  95

Ala Ser Asp Ser Gly Ala Val Glu Leu Trp Glu Leu Asp Glu Asn Glu
            100                 105                 110

Thr Leu Ile Val Ser Lys Phe Cys Lys Tyr Glu His Asp Asp Ile Val
        115                 120                 125

Ser Thr Val Ser Val Leu Ser Ser Gly Thr Gln Ala Val Ser Gly Ser
    130                 135                 140

Lys Asp Ile Cys Ile Lys Val Trp Asp Leu Ala Gln Gln Val Val Leu
145                 150                 155                 160

Ser Ser Tyr Arg Ala His Ala Ala Gln Val Thr Cys Val Ala Ala Ser
                165                 170                 175

Pro His Lys Asp Ser Val Phe Leu Ser Cys Ser Glu Asp Asn Arg Ile
            180                 185                 190

Leu Leu Trp Asp Thr Arg Cys Pro Lys Pro Ala Ser Gln Ile Gly Cys
        195                 200                 205

Ser Ala Pro Gly Tyr Leu Pro Thr Ser Leu Ala Trp His Pro Gln Gln
    210                 215                 220

Ser Glu Val Phe Val Phe Gly Asp Glu Asn Gly Thr Val Ser Leu Val
225                 230                 235                 240

Asp Thr Lys Ser Thr Ser Cys Val Leu Ser Ser Ala Val His Ser Gln
                245                 250                 255

Cys Val Thr Gly Leu Val Phe Ser Pro His Ser Val Pro Phe Leu Ala
            260                 265                 270

Ser Leu Ser Glu Asp Cys Ser Leu Ala Val Leu Asp Ser Ser Leu Ser
275 280 285

Glu Leu
290

<210> 147
<211> 302
<212> PRT
<213> Artificial

<400> 147

Met Arg Lys Glu Thr Pro Pro Pro Leu Val Pro Pro Ala Ala Arg Glu
1 5 10 15

Trp Asn Leu Pro Pro Asn Ala Pro Ala Cys Met Glu Arg Gln Leu Glu
20 25 30

Ala Ala Arg Tyr Arg Ser Asp Gly Ala Leu Leu Leu Gly Ala Ser Ser
35 40 45

Leu Ser Gly Arg Cys Trp Ala Gly Ser Leu Trp Leu Phe Lys Asp Pro
50 55 60

Cys Ala Ala Pro Asn Glu Gly Phe Cys Ser Ala Gly Val Gln Thr Glu
65 70 75 80

Ala Gly Val Ala Asp Leu Thr Trp Val Gly Glu Arg Gly Ile Leu Val
85 90 95

Ala Ser Asp Ser Gly Ala Val Glu Leu Trp Glu Leu Asp Glu Asn Glu
100 105 110

Thr Leu Ile Val Ser Lys Phe Cys Lys Tyr Glu His Asp Asp Ile Val
115 120 125

Ser Thr Val Ser Val Leu Ser Ser Gly Thr Gln Ala Val Ser Gly Ser
130 135 140

Lys Asp Ile Cys Ile Lys Val Trp Asp Leu Ala Gln Gln Val Val Leu
145 150 155 160

Ser Ser Tyr Arg Ala His Ala Ala Gln Val Thr Cys Val Ala Ala Ser
165 170 175

Pro His Lys Asp Ser Val Phe Leu Ser Cys Ser Glu Asp Asn Arg Ile
180 185 190

Leu Leu Trp Asp Thr Arg Cys Pro Lys Pro Ala Ser Gln Ile Gly Cys
195 200 205

Ser Ala Pro Gly Tyr Leu Pro Thr Ser Leu Ala Trp His Pro Gln Gln
210 215 220

Ser Glu Val Phe Val Phe Gly Asp Glu Asn Gly Thr Val Ser Leu Val
225 230 235 240

Asp Thr Lys Ser Thr Ser Cys Val Leu Ser Ser Ala Val His Ser Gln
245 250 255

Cys Val Thr Gly Leu Val Phe Ser Pro His Ser Val Pro Phe Leu Ala
260 265 270

Ser Leu Ser Glu Asp Cys Ser Leu Ala Val Leu Asp Ser Ser Leu Ser
275 280 285

Glu Phe Ile Ser Ser Ser Met Lys Ile Glu Asp Trp Ile Gly
290 295 300

<210> 148
<211> 210
<212> PRT
<213> Artificial

<400> 148

Met Arg Lys Glu Thr Pro Pro Pro Leu Val Pro Pro Ala Ala Arg Glu
1 5 10 15

Trp Asn Leu Pro Pro Asn Ala Pro Ala Cys Met Glu Arg Gln Leu Glu
20 25 30

Ala Ala Arg Tyr Arg Ser Asp Gly Ala Leu Leu Leu Gly Ala Ser Ser
35 40 45

Leu Ser Gly Arg Cys Trp Ala Gly Ser Leu Trp Leu Phe Lys Asp Pro
50 55 60

Cys Ala Ala Pro Asn Glu Gly Phe Cys Ser Ala Gly Val Gln Thr Glu
65 70 75 80

Ala Gly Val Ala Asp Leu Thr Trp Val Gly Glu Arg Gly Ile Leu Val
85 90 95

Ala Ser Asp Ser Gly Ala Val Glu Leu Trp Glu Leu Asp Glu Asn Glu
100 105 110

Thr Leu Ile Val Ser Lys Phe Cys Lys Tyr Glu His Asp Asp Ile Val
115 120 125

Ser Thr Val Ser Val Leu Ser Ser Gly Thr Gln Ala Val Ser Gly Ser
130 135 140

Lys Asp Ile Cys Ile Lys Val Trp Asp Leu Ala Gln Gln Val Val Leu
145 150 155 160

Ser Ser Tyr Arg Ala His Ala Ala Gln Val Thr Cys Val Ala Ala Ser
165 170 175

Pro His Lys Asp Ser Val Phe Leu Ser Cys Ser Glu Asp Asn Arg Ile
180 185 190

Leu Leu Trp Asp Thr Arg Cys Pro Lys Pro Ala Ser Gln Ile Val Arg
195 200 205

Leu Cys
210

<210> 149
<211> 179
<212> PRT
<213> Artificial

<400> 149

Met Arg Lys Glu Thr Pro Pro Pro Leu Val Pro Pro Ala Ala Arg Glu
1 5 10 15

Trp Asn Leu Pro Pro Asn Ala Pro Ala Cys Met Glu Arg Gln Leu Glu
20 25 30

Ala Ala Arg Tyr Arg Ser Asp Gly Ala Leu Leu Leu Gly Ala Ser Ser
35 40 45

Leu Ser Gly Arg Cys Trp Ala Gly Ser Leu Trp Leu Phe Lys Asp Pro
50 55 60

Cys Ala Ala Pro Asn Glu Gly Phe Cys Ser Ala Gly Val Gln Thr Glu
65 70 75 80

Ala Gly Val Ala Asp Leu Thr Trp Val Gly Glu Arg Gly Ile Leu Val
85 90 95

Ala Ser Asp Ser Gly Ala Val Glu Leu Trp Glu Leu Asp Glu Asn Glu
100 105 110

Thr Leu Ile Val Ser Lys Phe Cys Lys Tyr Glu His Asp Asp Ile Val
115 120 125

Ser Thr Val Ser Val Leu Ser Ser Gly Thr Gln Ala Val Ser Gly Ser
130 135 140

Lys Asp Ile Trp Trp Val Pro Val Leu Ile Ala Pro Val Ser Lys Pro
145 150 155 160

Pro Val Gly Ala Phe Pro Ile Leu Leu Thr Ser Leu Gly Asn Ile Leu
165 170 175

Gly Leu Phe

<210> 150
<211> 123
<212> PRT
<213> Artificial

<400> 150

Met Arg Lys Glu Thr Pro Pro Pro Leu Val Pro Pro Ala Ala Arg Glu
1               5                   10                  15

Trp Asn Leu Pro Pro Asn Ala Pro Ala Cys Met Glu Arg Gln Leu Glu
            20                  25                  30

Ala Ala Arg Tyr Arg Ser Asp Gly Ala Leu Leu Leu Gly Ala Ser Ser
        35                  40                  45

Leu Ser Gly Arg Cys Trp Ala Gly Ser Leu Trp Leu Phe Lys Asp Pro
    50                  55                  60

Cys Ala Ala Pro Asn Glu Gly Phe Cys Ser Ala Gly Val Gln Thr Glu
65                  70                  75                  80

Ala Gly Val Ala Asp Leu Thr Trp Val Gly Glu Arg Gly Ile Leu Val
                85                  90                  95

Ala Ser Asp Ser Gly Glu Ser Leu Ser His Tyr Pro Pro Pro Pro Pro
            100                 105                 110

Gly Gly Thr Val Glu Trp Arg Leu Gly Val Pro
        115                 120

<210> 151
<211> 303
<212> PRT
<213> Artificial

<400> 151

Met Arg Lys Glu Thr Pro Pro Pro Leu Val Pro Pro Ala Ala Arg Glu
1               5                   10                  15

Trp Asn Leu Pro Pro Asn Ala Pro Ala Cys Met Glu Arg Gln Leu Glu
            20                  25                  30

Ala Ala Arg Tyr Arg Ser Asp Gly Ala Leu Leu Leu Gly Ala Ser Ser
        35                  40                  45

Leu Ser Gly Arg Cys Trp Ala Gly Ser Leu Trp Leu Phe Lys Asp Pro
50 55 60

Cys Ala Ala Pro Asn Glu Gly Phe Cys Ser Ala Gly Val Gln Thr Glu
65 70 75 80

Ala Gly Val Ala Asp Leu Thr Trp Val Gly Glu Arg Gly Ile Leu Val
85 90 95

Ala Ser Asp Ser Gly Ala Val Glu Leu Trp Glu Leu Asp Glu Asn Glu
100 105 110

Thr Leu Ile Val Ser Lys Phe Cys Lys Tyr Glu His Asp Asp Ile Val
115 120 125

Ser Thr Val Ser Val Leu Ser Ser Gly Thr Gln Ala Val Ser Gly Ser
130 135 140

Lys Asp Ile Cys Ile Lys Val Trp Asp Leu Ala Gln Gln Val Val Leu
145 150 155 160

Ser Ser Tyr Arg Ala His Ala Ala Gln Val Thr Cys Val Ala Ala Ser
165 170 175

Pro His Lys Asp Ser Val Phe Leu Ser Cys Ser Glu Asp Asn Arg Ile
180 185 190

Leu Leu Trp Asp Thr Arg Cys Pro Lys Pro Ala Ser Gln Ile Gly Cys
195 200 205

Ser Ala Pro Gly Tyr Leu Pro Thr Ser Leu Ala Trp His Pro Gln Gln
210 215 220

Ser Glu Val Phe Val Phe Gly Asp Glu Asn Gly Thr Val Ser Leu Val
225 230 235 240

Asp Thr Lys Ser Thr Ser Cys Val Leu Ser Ser Ala Val His Ser Gln
245 250 255

Cys Val Thr Gly Leu Val Phe Ser Pro His Ser Val Pro Phe Leu Ala
260 265 270

Ser Leu Ser Glu Asp Cys Ser Leu Ala Val Leu Asp Ser Ser Leu Ser
275 280 285

Glu Leu Ala Gly Ser Cys Ala Glu Thr Pro Arg Trp Cys Cys Pro
290 295 300

<210> 152
<211> 72

<212> PRT
<213> Artificial

<400> 152

```
Met Arg Lys Glu Thr Pro Pro Pro Leu Val Pro Pro Ala Ala Arg Glu
1               5                   10                  15

Trp Asn Leu Pro Pro Asn Ala Pro Ala Cys Met Glu Arg Gln Leu Glu
            20                  25                  30

Ala Ala Arg Tyr Arg Ser Gly Glu Pro Arg Ser Arg Ala Trp Glu Leu
        35                  40                  45

Pro Tyr Pro Gly Pro Gly Arg Pro Ser Pro Glu Thr Asp Pro Pro Ser
    50                  55                  60

Pro Ser Pro Pro Cys Met Leu Ser
65                  70
```

<210> 153
<211> 37
<212> PRT
<213> Artificial

<400> 153

```
Met Arg Lys Glu Thr Pro Pro Pro Leu Val Pro Pro Ala Ala Arg Glu
1               5                   10                  15

Trp Asn Leu Pro Pro Asn Ala Pro Ala Cys Met Glu Arg Arg Phe Ser
            20                  25                  30

Ser Gly Pro Pro Ala
        35
```

<210> 154
<211> 308
<212> PRT
<213> Artificial

<400> 154

```
Met Pro Gly Gln Glu Leu Arg Thr Leu Asn Gly Ser Gln Met Leu Leu
1               5                   10                  15

Val Leu Leu Val Leu Ser Trp Leu Pro His Gly Gly Ala Leu Ser Leu
            20                  25                  30

Ala Glu Ala Ser Arg Ala Ser Phe Pro Gly Pro Ser Glu Leu His Thr
        35                  40                  45

Glu Asp Ser Arg Phe Arg Glu Leu Arg Lys Arg Tyr Glu Asp Leu Leu
    50                  55                  60
```

```
Thr Arg Leu Arg Ala Asn Gln Ser Trp Glu Asp Ser Asn Thr Asp Leu
65                  70                  75                  80

Val Pro Ala Pro Ala Val Arg Ile Leu Thr Pro Glu Val Arg Leu Gly
                85                  90                  95

Ser Gly Gly His Leu His Leu Arg Ile Ser Arg Ala Ala Leu Pro Glu
            100                 105                 110

Gly Leu Pro Glu Ala Ser Arg Leu His Arg Ala Leu Phe Arg Leu Ser
        115                 120                 125

Pro Thr Ala Ser Arg Ser Trp Asp Val Thr Arg Pro Leu Arg Arg Gln
    130                 135                 140

Leu Ser Leu Ala Arg Pro Gln Ala Pro Ala Leu His Leu Arg Leu Ser
145                 150                 155                 160

Pro Pro Pro Ser Gln Ser Asp Gln Leu Leu Ala Glu Ser Ser Ser Ala
                165                 170                 175

Arg Pro Gln Leu Glu Leu His Leu Arg Pro Gln Ala Ala Arg Gly Arg
            180                 185                 190

Arg Arg Ala Arg Ala Arg Asn Gly Asp His Cys Pro Leu Gly Pro Gly
        195                 200                 205

Arg Cys Cys Arg Leu His Thr Val Arg Ala Ser Leu Glu Asp Leu Gly
    210                 215                 220

Trp Ala Asp Trp Val Leu Ser Pro Arg Glu Val Gln Val Thr Met Cys
225                 230                 235                 240

Ile Gly Ala Cys Pro Ser Gln Phe Arg Ala Ala Asn Met His Ala Gln
                245                 250                 255

Ile Lys Thr Ser Leu His Arg Leu Lys Pro Asp Thr Val Pro Ala Pro
            260                 265                 270

Cys Cys Val Pro Ala Ser Tyr Asn Pro Met Val Leu Ile Gln Lys Thr
        275                 280                 285

Asp Thr Gly Val Ser Leu Gln Thr Tyr Asp Asp Leu Leu Ala Lys Asp
    290                 295                 300

Cys His Cys Ile
305
```

<210> 155
<211> 331
<212> PRT

<213> Artificial

<400> 155

Met Thr Pro Gly Pro Arg Ser Cys Arg Asn Ala Thr Arg Thr Phe Arg
1 5 10 15

Ala Pro Val Arg Gln Gly Glu Pro Gly Gly Ala Gly Pro Gln Pro His
20 25 30

Pro Lys Ala Gln Met Leu Leu Val Leu Leu Val Leu Ser Trp Leu Pro
35 40 45

His Gly Gly Ala Leu Ser Leu Ala Glu Ala Ser Arg Ala Ser Phe Pro
50 55 60

Gly Pro Ser Glu Leu His Ser Glu Asp Ser Arg Phe Arg Glu Leu Arg
65 70 75 80

Lys Arg Tyr Glu Asp Leu Leu Thr Arg Leu Arg Ala Asn Gln Ser Trp
85 90 95

Glu Asp Ser Asn Thr Asp Leu Val Pro Ala Pro Ala Val Arg Ile Leu
100 105 110

Thr Pro Glu Val Arg Leu Gly Ser Gly Gly His Leu His Leu Arg Ile
115 120 125

Ser Arg Ala Ala Leu Pro Glu Gly Leu Pro Glu Ala Ser Arg Leu His
130 135 140

Arg Ala Leu Phe Arg Leu Ser Pro Thr Ala Ser Arg Ser Trp Asp Val
145 150 155 160

Thr Arg Pro Leu Arg Arg Gln Leu Ser Leu Ala Arg Pro Gln Ala Pro
165 170 175

Ala Leu His Leu Arg Leu Ser Pro Pro Pro Ser Gln Ser Asp Gln Leu
180 185 190

Leu Ala Glu Ser Ser Ser Ala Arg Pro Gln Leu Glu Leu His Leu Arg
195 200 205

Pro Gln Ala Ala Arg Gly Arg Arg Arg Ala Arg Ala Arg Asn Gly Asp
210 215 220

His Cys Pro Leu Gly Pro Gly Arg Cys Cys Arg Leu His Thr Val Arg
225 230 235 240

Ala Ser Leu Glu Asp Leu Gly Trp Ala Asp Trp Val Leu Ser Pro Arg
245 250 255

Glu Val Gln Val Thr Met Cys Ile Gly Ala Cys Pro Ser Gln Phe Arg
260 265 270

Ala Ala Asn Met His Ala Gln Ile Lys Thr Ser Leu His Arg Leu Lys
275 280 285

Pro Asp Thr Val Pro Ala Pro Cys Cys Val Pro Ala Ser Tyr Asn Pro
290 295 300

Met Val Leu Ile Gln Lys Thr Asp Thr Gly Val Ser Leu Gln Thr Tyr
305 310 315 320

Asp Asp Leu Leu Ala Lys Asp Cys His Cys Ile
325 330

<210> 156
<211> 94
<212> PRT
<213> Artificial

<400> 156

Met Pro Gly Gln Glu Leu Arg Thr Val Asn Gly Ser Gln Met Leu Leu
1 5 10 15

Val Leu Leu Val Leu Ser Trp Leu Pro His Gly Gly Ala Leu Ser Leu
20 25 30

Ala Glu Ala Ser Arg Ala Ser Phe Pro Gly Pro Ser Glu Leu His Ser
35 40 45

Glu Asp Ser Arg Phe Arg Glu Leu Arg Lys Arg Tyr Glu Asp Leu Leu
50 55 60

Thr Arg Leu Arg Ala Asn Gln Ser Trp Glu Asp Ser Asn Thr Asp Leu
65 70 75 80

Val Pro Ala Pro Ala Val Arg Ile Leu Thr Pro Glu Gly Lys
85 90

<210> 157
<211> 129
<212> PRT
<213> Artificial

<400> 157

Met Ala Arg Gly Ser Leu Arg Arg Leu Leu Arg Leu Leu Val Leu Gly
1 5 10 15

Leu Trp Leu Ala Leu Leu Arg Ser Val Ala Gly Glu Gln Ala Pro Gly
20 25 30

Thr Ala Pro Cys Ser Arg Gly Ser Ser Trp Ser Ala Asp Leu Asp Lys
35 40 45

Cys Met Asp Cys Ala Ser Cys Arg Ala Arg Pro His Ser Asp Phe Cys
50 55 60

Leu Gly Cys Ala Ala Ala Pro Pro Ala Pro Phe Arg Leu Leu Trp Pro
65 70 75 80

Ile Leu Gly Gly Ala Leu Ser Leu Thr Phe Val Leu Gly Leu Leu Ser
85 90 95

Gly Phe Leu Val Trp Arg Arg Cys Arg Arg Arg Glu Lys Phe Thr Thr
100 105 110

Pro Ile Glu Glu Thr Gly Gly Glu Gly Cys Pro Ala Val Ala Leu Ile
115 120 125

Gln

<210> 158
<211> 121
<212> PRT
<213> Artificial

<400> 158

Met Ala Arg Gly Ser Leu Arg Arg Leu Leu Arg Leu Leu Val Leu Gly
1 5 10 15

Leu Trp Leu Ala Leu Leu Arg Ser Val Ala Gly Glu Gln Ala Pro Gly
20 25 30

Thr Arg Asp Ser Gly Val Gly Glu Pro Arg Ala Gly Ala Arg Glu Pro
35 40 45

Asp Trp Val Ser Gly Glu Gln Arg Arg Thr Gly Gly Thr Gly Gly Cys
50 55 60

Gly Asp Leu Gly Ser Gly Gly Leu Gln Thr Ala Arg Ser Arg Leu Trp
65 70 75 80

Glu Val Pro Ser Leu Ser Lys Leu His Ser Gln Ser Gly Pro Gln Phe
85 90 95

Leu Pro Pro Ser Asn Trp Gly Trp Gly Gly Ser Ser Val Pro Gln Val
100 105 110

Arg Ser Thr Ser Asn Gly Glu Arg Lys
115 120

<210> 159
<211> 94
<212> PRT
<213> Artificial

<400> 159

```
Met Ala Arg Gly Ser Leu Arg Arg Leu Leu Arg Leu Leu Val Leu Gly
1               5                   10                  15

Leu Trp Leu Ala Leu Leu Arg Ser Val Ala Gly Glu Gln Ala Pro Ala
            20                  25                  30

Ser Asp Pro Arg Pro Pro Pro Gln Ala Pro Pro Pro Ala Pro Ala Ala
        35                  40                  45

Ala Pro Gly Ala Arg Thr Trp Thr Ser Ala Trp Thr Ala Arg Leu Ala
    50                  55                  60

Gly Arg Asp Arg Thr Ala Thr Ser Ala Trp Ala Ala Leu Gln His Leu
65                  70                  75                  80

Leu Pro Pro Ser Gly Cys Phe Gly Pro Ser Leu Gly Ala Leu
                85                  90
```

<210> 160
<211> 250
<212> PRT
<213> Artificial

<400> 160

Met Ala Arg Gly Ser Leu Arg Arg Leu Leu Arg Leu Leu Val Leu Gly
1               5                   10                  15

Leu Trp Leu Ala Leu Leu Arg Ser Val Ala Gly Glu Gln Ala Pro Gly
            20                  25                  30

Thr Ala Pro Cys Ser Arg Gly Ser Ser Trp Ser Ala Asp Leu Asp Lys
        35                  40                  45

Cys Met Asp Cys Ala Ser Cys Arg Ala Arg Pro His Ser Asp Phe Cys
    50                  55                  60

Leu Gly Cys Glu Trp Gly Ala Gly Pro Val Ala Ser Gly Pro Gln Thr
65                  70                  75                  80

Gly Arg Glu Gly Gly Trp Cys Ala Glu Arg Gly Arg Glu Leu Cys Ile
                85                  90                  95

Trp Glu Ile Ile Arg Glu Glu Val Gly Ala Gly Arg Gly Leu Arg Ser
            100                 105                 110

Gly Arg Arg Pro Arg Leu Gly Glu Gly Arg Arg Leu Ser Leu Arg Gly
        115                 120                 125

Arg Gly Trp Pro Gly Glu Gly Arg Gly Leu Thr Pro Ser Pro Ala Pro
    130                 135                 140

Arg Arg Cys Ser Thr Ser Cys Pro Leu Pro Ala Ala Leu Ala His Pro
145                 150                 155                 160

Trp Gly Arg Ser Glu Pro Asp Leu Arg Ala Gly Ala Ala Phe Trp Leu
                165                 170                 175

Phe Gly Leu Glu Thr Met Pro Gln Glu Arg Glu Val His His Pro His
            180                 185                 190

Arg Gly Asp Arg Arg Arg Gly Leu Pro Ser Cys Gly Ala Asp Pro Val
        195                 200                 205

Thr Met Cys Pro Leu Pro Ala Gly Ala Arg Pro Leu Ile Ile His Ser
    210                 215                 220

Ser Ile Leu Glu Pro Val Ser Ala Ser Gln Thr Arg Arg Glu Pro Ser
225                 230                 235                 240

Ser Ser Asn His Lys Gly Gly Gly Gly Arg
                245                 250

<210> 161
<211> 295
<212> PRT
<213> Artificial

<400> 161

Met Ile Glu Val Leu Thr Thr Thr Asp Ser Gln Lys Leu Leu His Gln
1               5                   10                  15

Leu Asn Ala Leu Leu Glu Gln Glu Ser Arg Cys Gln Pro Lys Val Cys
            20                  25                  30

Gly Leu Arg Leu Ile Glu Ser Ala His Asp Asn Gly Leu Arg Met Thr
        35                  40                  45

Ala Arg Leu Arg Asp Phe Glu Val Lys Asp Leu Leu Ser Leu Thr Gln
    50                  55                  60

Phe Phe Gly Phe Asp Thr Glu Thr Phe Ser Leu Ala Val Asn Leu Leu
65                  70                  75                  80

Asp Arg Phe Leu Ser Lys Met Lys Val Gln Pro Lys His Leu Gly Cys
                85                  90                  95

```
Val Gly Leu Ser Cys Phe Tyr Leu Ala Val Lys Ser Ile Glu Glu Glu
            100                 105                 110

Arg Asn Val Pro Leu Ala Thr Asp Leu Ile Arg Ile Ser Gln Tyr Arg
        115                 120                 125

Phe Thr Val Ser Asp Leu Met Arg Met Glu Lys Ile Val Leu Glu Lys
    130                 135                 140

Val Cys Trp Lys Val Lys Ala Thr Thr Ala Phe Gln Phe Leu Gln Leu
145                 150                 155                 160

Tyr Tyr Ser Leu Leu Gln Glu Asn Leu Pro Leu Glu Arg Arg Asn Ser
                165                 170                 175

Ile Asn Phe Glu Arg Leu Glu Ala Gln Leu Lys Ala Cys His Cys Arg
            180                 185                 190

Ile Ile Phe Ser Lys Ala Lys Pro Ser Val Leu Ala Leu Ser Ile Ile
        195                 200                 205

Ala Leu Glu Ile Gln Ala Gln Lys Cys Val Glu Leu Thr Glu Gly Ile
    210                 215                 220

Glu Cys Leu Gln Lys His Ser Lys Ile Asn Gly Arg Asp Leu Thr Phe
225                 230                 235                 240

Trp Gln Glu Leu Val Ser Lys Cys Leu Thr Glu Tyr Ser Ser Asn Lys
                245                 250                 255

Cys Ser Lys Pro Asn Val Gln Lys Leu Lys Trp Ile Val Ser Gly Arg
            260                 265                 270

Thr Ala Arg Gln Leu Lys His Ser Tyr Tyr Arg Ile Thr His Leu Pro
        275                 280                 285

Thr Ile Pro Glu Met Val Pro
    290                 295
```

<210> 162
<211> 114
<212> PRT
<213> Artificial

<400> 162

Met Ile Glu Val Leu Thr Thr Thr Asp Ser Gln Lys Leu Leu His Gln
1 5 10 15

Leu Asn Ala Leu Leu Glu Gln Glu Ser Arg Cys Gln Pro Lys Val Cys
20 25 30

Gly Leu Arg Leu Ile Glu Ser Ala His Asp Asn Gly Leu Arg Met Thr
35 40 45

Ala Arg Leu Arg Asp Phe Glu Val Lys Asp Leu Leu Ser Leu Thr Gln
50 55 60

Phe Phe Gly Phe Asp Thr Glu Thr Phe Ser Leu Ala Val Asn Leu Leu
65 70 75 80

Asp Arg Phe Leu Ser Lys Met Lys Val Cys Leu Lys Leu His Phe Cys
85 90 95

Asn Phe Ala Gln Cys Val Leu Glu Met Glu Leu Leu Pro Phe Gly Trp
100 105 110

Tyr Ser

<210> 163
<211> 181
<212> PRT
<213> Artificial

<400> 163

Met Ala Gly Ser Arg Glu Val Val Ala Met Asp Cys Glu Met Val Gly
1               5                   10                  15

Leu Gly Pro His Arg Glu Ser Gly Leu Ala Arg Cys Ser Leu Val Asn
            20                  25                  30

Val His Gly Ala Val Leu Tyr Asp Lys Phe Ile Arg Pro Glu Gly Glu
        35                  40                  45

Ile Thr Asp Tyr Arg Thr Arg Val Ser Gly Val Thr Pro Gln His Met
    50                  55                  60

Val Gly Ala Thr Pro Phe Ala Val Ala Arg Leu Glu Ile Leu Gln Leu
65                  70                  75                  80

Leu Lys Gly Lys Leu Val Val Gly His Asp Leu Lys His Asp Phe Gln
                85                  90                  95

Ala Leu Lys Glu Asp Met Ser Gly Tyr Thr Ile Tyr Asp Thr Ser Thr
            100                 105                 110

Asp Arg Leu Leu Trp Arg Glu Ala Lys Leu Asp His Cys Arg Arg Val
        115                 120                 125

Ser Leu Arg Val Leu Ser Glu Arg Leu Leu His Lys Ser Ile Gln Asn
    130                 135                 140

Ser Leu Leu Gly His Ser Ser Val Glu Asp Ala Arg Ala Thr Met Glu
145                 150                 155                 160

Leu Tyr Gln Ile Ser Gln Arg Ile Arg Ala Arg Arg Gly Leu Pro Arg
                165                 170                 175

Leu Ala Val Ser Asp
            180

<210> 164
<211> 125
<212> PRT
<213> Artificial

<400> 164

Met Ser His Ser Arg Gly Arg Gly Thr Gly Ile Phe Val His Gln Phe
1               5                   10                  15

Pro Ser Ala Thr Ser Cys Cys Cys Ser Cys Ala Leu Ile Pro Gln His
            20                  25                  30

Phe Trp Leu Val Gly Lys Ala Gly Arg Lys Arg His Thr Asp Met Gly
        35                  40                  45

Gly Trp Glu Phe Thr Leu Trp Glu Gly Gln Arg Glu Arg Arg Gln Thr
    50                  55                  60

Leu Arg Ala Arg Ala Val His Arg Asp Pro Ser Gly Gln Gln Leu Phe
65                  70                  75                  80

Ile Lys His Leu Leu Pro Ala Leu Gly Gln Ile Leu Glu Thr Gln Gly
                85                  90                  95

Glu Gln Asp Pro Ser Leu Ser Leu Ser Ser Trp Leu Val Gly Arg Trp
            100                 105                 110

Pro Leu Thr Arg Ser Ser Ala His Met Val Arg Ser Val
        115                 120                 125

<210> 165
<211> 203
<212> PRT
<213> Artificial

<400> 165

Met Ala Lys Ala Tyr Asp His Leu Phe Lys Leu Leu Leu Ile Gly Asp
1               5                   10                  15

Ser Gly Val Gly Lys Thr Cys Leu Ile Ile Arg Phe Ala Glu Asp Asn
            20                  25                  30

Phe Asn Asn Thr Tyr Ile Ser Thr Ile Gly Ile Asp Phe Lys Ile Arg
35 40 45

Thr Val Asp Ile Glu Gly Lys Lys Ile Lys Leu Gln Val Trp Asp Thr
50 55 60

Ala Gly Gln Glu Arg Phe Lys Thr Ile Thr Thr Ala Tyr Tyr Arg Gly
65 70 75 80

Ala Met Gly Ile Ile Leu Val Tyr Asp Ile Thr Asp Glu Lys Ser Phe
85 90 95

Glu Asn Ile Gln Asn Trp Met Lys Ser Ile Lys Glu Asn Ala Ser Ala
100 105 110

Gly Val Glu Arg Leu Leu Leu Gly Asn Lys Cys Asp Met Glu Ala Lys
115 120 125

Arg Lys Val Gln Lys Glu Gln Ala Asp Lys Leu Ala Arg Glu His Gly
130 135 140

Ile Arg Phe Phe Glu Thr Ser Ala Lys Ser Ser Met Asn Val Asp Glu
145 150 155 160

Ala Phe Ser Ser Leu Ala Arg Asp Ile Leu Leu Lys Ser Gly Gly Arg
165 170 175

Arg Ser Gly Asn Gly Asn Lys Pro Pro Ser Thr Asp Leu Lys Thr Cys
180 185 190

Asp Lys Lys Asn Thr Asn Lys Cys Ser Leu Gly
195 200

<210> 166
<211> 122
<212> PRT
<213> Artificial

<400> 166

Met Gly Ile Ile Leu Val Tyr Asp Ile Thr Asp Glu Lys Ser Phe Glu
1 5 10 15

Asn Ile Gln Asn Trp Met Lys Ser Ile Lys Glu Asn Ala Ser Ala Gly
20 25 30

Val Glu Arg Leu Leu Leu Gly Asn Lys Cys Asp Met Glu Ala Lys Arg
35 40 45

Lys Val Gln Lys Glu Gln Ala Asp Lys Leu Ala Arg Glu His Gly Ile
50 55 60

Arg Phe Phe Glu Thr Ser Ala Lys Ser Ser Met Asn Val Asp Glu Ala
65 70 75 80

Phe Ser Ser Leu Ala Arg Asp Ile Leu Leu Lys Ser Gly Gly Arg Arg
85 90 95

Ser Gly Asn Gly Asn Lys Pro Pro Ser Thr Asp Leu Lys Thr Cys Asp
100 105 110

Lys Lys Asn Thr Asn Lys Cys Ser Leu Gly
115 120

<210> 167
<211> 166
<212> PRT
<213> Artificial

<400> 167

Met Ala Lys Ala Tyr Asp His Leu Phe Lys Leu Leu Leu Ile Gly Asp
1 5 10 15

Ser Gly Val Gly Lys Thr Cys Leu Ile Ile Arg Phe Ala Glu Asp Asn
20 25 30

Phe Asn Asn Thr Tyr Ile Ser Thr Ile Gly Ile Asp Phe Lys Ile Arg
35 40 45

Thr Val Asp Ile Glu Gly Lys Lys Ile Lys Leu Gln Val Trp Asp Thr
50 55 60

Ala Gly Gln Glu Arg Phe Lys Thr Ile Thr Thr Ala Tyr Tyr Arg Gly
65 70 75 80

Ala Met Gly Ile Ile Leu Val Tyr Asp Ile Thr Asp Glu Lys Ser Phe
85 90 95

Glu Asn Ile Gln Asn Trp Met Lys Ser Ile Lys Glu Asn Ala Ser Ala
100 105 110

Gly Val Glu Arg Leu Leu Leu Gly Asn Lys Cys Asp Met Glu Ala Lys
115 120 125

Arg Lys Val Gln Lys Glu Gln Ala Asp Lys Val Arg Ala Arg Leu Ser
130 135 140

Asn Val Leu Glu Leu Gly Trp Glu Gly Gln Asp Arg Leu His Cys Arg
145 150 155 160

Gly Leu Gly Cys Cys Pro
165

<210> 168
<211> 72
<212> PRT
<213> Artificial

<400> 168

```
Met Ala Lys Ala Tyr Asp His Leu Phe Lys Leu Leu Leu Ile Gly Asp
1               5                   10                  15

Ser Gly Val Gly Lys Thr Cys Leu Ile Ile Arg Phe Ala Glu Asp Asn
            20                  25                  30

Phe Asn Asn Thr Tyr Ile Ser Thr Ile Gly Glu Pro Ala Gly His Val
        35                  40                  45

Pro Asp Pro Asp Ser Pro Val Thr Ser Phe Ser Gly Ser Arg Ile Thr
    50                  55                  60

Ser Asp Pro Thr Phe Gln Ser Pro
65                  70
```

<210> 169
<211> 184
<212> PRT
<213> Artificial

<400> 169

```
Met Ala Lys Ala Tyr Asp His Leu Phe Lys Leu Leu Leu Ile Gly Asp
1               5                   10                  15

Ser Gly Val Gly Lys Thr Cys Leu Ile Ile Arg Phe Ala Glu Asp Asn
            20                  25                  30

Phe Asn Asn Thr Tyr Ile Ser Thr Ile Gly Ile Asp Phe Lys Ile Arg
        35                  40                  45

Thr Val Asp Ile Glu Gly Lys Lys Ile Lys Leu Gln Val Trp Asp Thr
    50                  55                  60

Ala Gly Gln Glu Arg Phe Lys Thr Ile Thr Thr Ala Tyr Tyr Arg Gly
65                  70                  75                  80

Ala Met Gly Ile Ile Leu Val Tyr Asp Ile Thr Asp Glu Lys Ser Phe
                85                  90                  95

Glu Asn Ile Gln Asn Trp Met Lys Ser Ile Lys Glu Asn Ala Ser Ala
            100                 105                 110

Gly Val Glu Arg Leu Leu Leu Gly Asn Lys Cys Asp Met Glu Ala Lys
        115                 120                 125
```

Arg Lys Val Gln Lys Glu Gln Ala Asp Lys Leu Ala Arg Glu His Gly
130 135 140

Ile Arg Phe Phe Glu Thr Ser Ala Lys Ser Ser Met Asn Val Asp Glu
145 150 155 160

Phe Pro Gly Pro Gly His Leu Ala Gln Val Arg Arg Pro Glu Ile Arg
165 170 175

Lys Arg Gln Gln Ala Ser Gln Tyr
180

<210> 170
<211> 164
<212> PRT
<213> Artificial

<400> 170

Met Ser Glu Pro Ala Gly Asp Val Arg Gln Asn Pro Cys Gly Ser Lys
1 5 10 15

Ala Cys Arg Arg Leu Phe Gly Pro Val Asp Ser Glu Gln Leu Ser Arg
20 25 30

Asp Cys Asp Ala Leu Met Ala Gly Cys Ile Gln Glu Ala Arg Glu Arg
35 40 45

Trp Asn Phe Asp Phe Val Thr Glu Thr Pro Leu Glu Gly Asp Phe Ala
50 55 60

Trp Glu Arg Val Arg Gly Leu Gly Leu Pro Lys Leu Tyr Leu Pro Thr
65 70 75 80

Gly Pro Arg Arg Gly Arg Asp Glu Leu Gly Gly Gly Arg Arg Pro Gly
85 90 95

Thr Ser Pro Ala Leu Leu Gln Gly Thr Ala Glu Glu Asp His Val Asp
100 105 110

Leu Ser Leu Ser Cys Thr Leu Val Pro Arg Ser Gly Glu Gln Ala Glu
115 120 125

Gly Ser Pro Gly Gly Pro Gly Asp Ser Gln Gly Arg Lys Arg Arg Gln
130 135 140

Thr Ser Met Thr Asp Phe Tyr His Ser Lys Arg Arg Leu Ile Phe Ser
145 150 155 160

Lys Arg Lys Pro

<210> 171
<211> 163
<212> PRT
<213> Artificial

<400> 171

```
Ser Glu Pro Ala Gly Asp Val Arg Gln Asn Pro Cys Gly Ser Lys Ala
1               5                   10                  15

Cys Arg Arg Leu Phe Gly Pro Val Asp Ser Glu Gln Leu Ser Arg Asp
            20                  25                  30

Cys Asp Ala Leu Met Ala Gly Cys Ile Gln Glu Ala Arg Glu Arg Trp
        35                  40                  45

Asn Phe Asp Phe Val Thr Glu Thr Pro Leu Glu Gly Asp Phe Ala Trp
    50                  55                  60

Glu Arg Val Arg Gly Leu Gly Leu Pro Lys Leu Tyr Leu Pro Thr Gly
65                  70                  75                  80

Pro Arg Arg Gly Arg Asp Glu Leu Gly Gly Gly Arg Arg Pro Gly Thr
                85                  90                  95

Ser Pro Ala Leu Leu Gln Gly Thr Ala Glu Glu Asp His Val Asp Leu
            100                 105                 110

Ser Leu Ser Cys Thr Leu Val Pro Arg Ser Gly Glu Gln Ala Glu Gly
        115                 120                 125

Ser Pro Gly Gly Pro Gly Asp Ser Gln Gly Arg Lys Arg Arg Gln Thr
    130                 135                 140

Ser Met Thr Asp Phe Tyr His Ser Lys Arg Arg Leu Ile Phe Ser Lys
145                 150                 155                 160

Arg Lys Pro
```

<210> 172
<211> 200
<212> PRT
<213> Artificial

<400> 172

```
Met Ser Glu Pro Ala Gly Asp Val Arg Gln Asn Pro Cys Gly Ser Lys
1               5                   10                  15

Ala Cys Arg Arg Leu Phe Gly Pro Val Asp Ser Glu Gln Leu Ser Arg
            20                  25                  30
```

Asp Cys Asp Ala Leu Met Ala Gly Cys Ile Gln Glu Ala Arg Glu Arg
35 40 45

Trp Asn Phe Asp Phe Val Thr Glu Thr Pro Leu Glu Gly Asp Phe Ala
50 55 60

Trp Glu Arg Val Arg Gly Leu Gly Leu Pro Lys Leu Tyr Leu Pro Thr
65 70 75 80

Gly Pro Arg Arg Gly Arg Asp Glu Leu Gly Gly Gly Arg Arg Pro Gly
85 90 95

Thr Ser Pro Ala Leu Leu Gln Gly Thr Ala Glu Glu Asp His Val Asp
100 105 110

Leu Ser Leu Ser Cys Thr Leu Val Pro Arg Ser Gly Glu Gln Ala Glu
115 120 125

Gly Ser Pro Gly Gly Pro Gly Asp Ser Gln Gly Arg Lys Arg Arg Gln
130 135 140

Thr Ser Met Thr Gly Ala Asp Met Cys Thr Glu Gly Leu Cys Lys Gly
145 150 155 160

Pro Gly Phe Ser Glu Ser Met Val Gln Gly Leu Thr Cys Leu Val Leu
165 170 175

Val Gln His Ala Pro Gly Arg Arg Lys Gln Ala Gln Arg Gly Glu Ala
180 185 190

Thr Ser Leu Arg Ser His Ser Lys
195 200

<210> 173
<211> 970
<212> PRT
<213> Artificial

<400> 173

```
Met Asp Ala Ala Glu Pro Gly Leu Pro Pro Gly Pro Glu Gly Arg Lys
1               5                   10                  15

Arg Tyr Ser Asp Ile Phe Arg Ser Leu Asp Asn Leu Glu Ile Ser Leu
            20                  25                  30

Gly Asn Val Thr Leu Glu Met Leu Ala Gly Asp Pro Leu Leu Ser Glu
        35                  40                  45

Asp Pro Glu Pro Asp Lys Thr Pro Thr Ala Thr Val Thr Asn Glu Ala
    50                  55                  60
```

```
Ser Cys Trp Ser Gly Pro Ser Pro Glu Gly Pro Val Pro Leu Thr Gly
65                  70                  75                  80

Glu Glu Leu Asp Leu Arg Leu Ile Arg Thr Lys Gly Gly Val Asp Ala
                85                  90                  95

Ala Leu Glu Tyr Ala Lys Thr Trp Ser Arg Tyr Ala Lys Glu Leu Leu
            100                 105                 110

Ala Trp Thr Glu Lys Arg Ala Ser Tyr Glu Leu Glu Phe Ala Lys Ser
        115                 120                 125

Thr Met Lys Ile Ala Glu Ala Gly Lys Val Ser Ile Gln Gln Gln Ser
    130                 135                 140

His Met Pro Leu Gln Tyr Ile Tyr Thr Leu Phe Leu Glu His Asp Leu
145                 150                 155                 160

Ser Leu Gly Thr Leu Ala Met Glu Thr Val Ala Gln Gln Lys Arg Asp
                165                 170                 175

Tyr Tyr Gln Pro Leu Ala Ala Lys Arg Thr Glu Ile Glu Lys Trp Arg
            180                 185                 190

Lys Glu Phe Lys Glu Gln Trp Met Lys Glu Gln Lys Arg Met Asn Glu
        195                 200                 205

Ala Val Gln Ala Leu Arg Arg Ala Gln Leu Gln Tyr Val Gln Arg Ser
    210                 215                 220

Glu Asp Leu Arg Ala Arg Ser Gln Gly Ser Pro Glu Asp Ser Ala Pro
225                 230                 235                 240

Gln Ala Ser Pro Gly Pro Ser Lys Gln Gln Glu Arg Arg Arg Arg Ser
                245                 250                 255

Arg Glu Glu Ala Gln Ala Lys Ala Gln Glu Ala Glu Ala Leu Tyr Gln
            260                 265                 270

Ala Cys Val Arg Glu Ala Asn Ala Arg Gln Gln Asp Leu Glu Ile Ala
        275                 280                 285

Lys Gln Arg Ile Val Ser His Val Arg Lys Leu Val Phe Gln Gly Asp
    290                 295                 300

Glu Val Leu Arg Arg Val Thr Leu Ser Leu Phe Gly Leu Arg Gly Ala
305                 310                 315                 320

Gln Ala Glu Arg Gly Pro Arg Ala Phe Ala Ala Leu Ala Glu Cys Cys
                325                 330                 335
```

```
Ala Pro Phe Glu Pro Gly Gln Arg Tyr Gln Glu Phe Val Arg Ala Leu
            340                 345                 350

Arg Pro Glu Ala Pro Pro Pro Pro Pro Pro Ala Phe Ser Phe Gln Glu
        355                 360                 365

Phe Leu Pro Ser Leu Asn Ser Ser Pro Leu Asp Ile Arg Lys Lys Leu
    370                 375                 380

Ser Gly Pro Leu Pro Pro Arg Leu Asp Glu Asn Ser Ala Glu Pro Gly
385                 390                 395                 400

Pro Trp Glu Asp Pro Gly Thr Gly Trp Arg Trp Gln Gly Thr Pro Gly
                405                 410                 415

Pro Thr Pro Gly Ser Asp Val Asp Ser Val Gly Gly Gly Ser Glu Ser
            420                 425                 430

Arg Ser Leu Asp Ser Pro Thr Ser Ser Pro Gly Ala Gly Thr Arg Gln
        435                 440                 445

Leu Val Lys Ala Ser Ser Thr Gly Thr Glu Ser Ser Asp Asp Phe Glu
    450                 455                 460

Glu Arg Asp Pro Asp Leu Gly Asp Gly Leu Glu Asn Gly Leu Gly Ser
465                 470                 475                 480

Pro Phe Gly Lys Trp Thr Leu Ser Ser Ala Ala Gln Thr His Gln Leu
                485                 490                 495

Arg Arg Leu Arg Gly Pro Ala Lys Cys Arg Glu Cys Glu Ala Phe Met
            500                 505                 510

Val Ser Gly Thr Glu Cys Glu Glu Cys Phe Leu Thr Cys His Lys Arg
        515                 520                 525

Cys Leu Glu Thr Leu Leu Ile Leu Cys Gly His Arg Arg Leu Pro Ala
    530                 535                 540

Arg Thr Pro Leu Phe Gly Val Asp Phe Leu Gln Leu Pro Arg Asp Phe
545                 550                 555                 560

Pro Glu Glu Val Pro Phe Val Val Thr Lys Cys Thr Ala Glu Ile Glu
                565                 570                 575

His Arg Ala Leu Asp Val Gln Gly Ile Tyr Arg Val Ser Gly Ser Arg
            580                 585                 590

Val Arg Val Glu Arg Leu Cys Gln Ala Phe Glu Asn Gly Arg Ala Leu
        595                 600                 605
```

Val Glu Leu Ser Gly Asn Ser Pro His Asp Val Ser Ser Val Leu Lys
610 615 620

Arg Phe Leu Gln Glu Leu Thr Glu Pro Val Ile Pro Phe His Leu Tyr
625 630 635 640

Asp Ala Phe Ile Ser Leu Ala Lys Thr Leu His Ala Asp Pro Gly Asp
645 650 655

Asp Pro Gly Thr Pro Ser Pro Ser Pro Asp Val Ile Arg Ser Leu Lys
660 665 670

Thr Leu Leu Val Gln Leu Pro Asp Ser Asn Tyr Asn Thr Leu Arg His
675 680 685

Leu Val Ala His Leu Phe Arg Val Ala Ala Arg Phe Met Glu Asn Lys
690 695 700

Met Ser Ala Asn Asn Leu Gly Ile Val Phe Gly Pro Thr Leu Leu Arg
705 710 715 720

Pro Pro Asp Gly Pro Arg Ala Ala Ser Ala Ile Pro Val Thr Cys Leu
725 730 735

Leu Asp Ser Gly His Gln Ala Gln Leu Val Glu Phe Leu Ile Val His
740 745 750

Tyr Glu Gln Ile Phe Gly Met Asp Glu Leu Pro Gln Ala Thr Glu Pro
755 760 765

Pro Pro Gln Asp Phe Ser Pro Ala Pro Gly Pro Leu Thr Thr Ser Ser
770 775 780

Gln Pro Pro Pro Pro His Leu Asp Pro Asp Ser Gln Pro Pro Val Leu
785 790 795 800

Ala Ser Asp Pro Gly Pro Asp Pro Gln His His Ser Thr Leu Glu Gln
805 810 815

His Pro Thr Ala Thr Pro Thr Glu Ile Pro Thr Pro Gln Ser Asp Gln
820 825 830

Arg Glu Asp Val Ala Glu Asp Thr Lys Asp Gly Gly Gly Glu Val Ser
835 840 845

Ser Gln Gly Pro Glu Asp Ser Leu Leu Gly Thr Gln Ser Arg Gly His
850 855 860

Phe Ser Arg Gln Pro Cys Lys Tyr Pro Arg Gly Gly Val Arg Pro Val
865 870 875 880

Thr His Gln Leu Ser Ser Leu Ala Leu Val Ala Ser Lys Leu Cys Glu
885 890 895

Glu Thr Pro Ile Thr Ser Val Pro Arg Gly Ser Leu Arg Gly Arg Gly
900 905 910

Pro Ser Pro Ala Ala Ala Ser Pro Glu Gly Ser Pro Leu Arg Arg Thr
915 920 925

Pro Leu Pro Lys His Phe Glu Ile Thr Gln Glu Thr Ala Arg Leu Leu
930 935 940

Ser Lys Leu Asp Ser Glu Ala Val Pro Arg Ala Thr Cys Cys Pro Asp
945 950 955 960

Val Gln Pro Glu Glu Ala Glu Asp His Leu
965 970

<210> 174
<211> 474
<212> PRT
<213> Artificial

<400> 174

Met Asp Ala Ala Glu Pro Gly Leu Pro Pro Gly Pro Glu Gly Arg Lys
1 5 10 15

Arg Tyr Ser Asp Ile Phe Arg Ser Leu Asp Asn Leu Glu Ile Ser Leu
20 25 30

Gly Asn Val Thr Leu Glu Met Leu Ala Gly Asp Pro Leu Leu Ser Glu
35 40 45

Asp Pro Glu Pro Asp Lys Thr Pro Thr Ala Thr Val Thr Asn Glu Ala
50 55 60

Ser Cys Trp Ser Gly Pro Ser Pro Glu Gly Pro Val Pro Leu Thr Gly
65 70 75 80

Glu Glu Leu Asp Leu Arg Leu Ile Arg Thr Lys Gly Gly Val Asp Ala
85 90 95

Ala Leu Glu Tyr Ala Lys Thr Trp Ser Arg Tyr Ala Lys Glu Leu Leu
100 105 110

Ala Trp Thr Glu Lys Arg Ala Ser Tyr Glu Leu Glu Phe Ala Lys Ser
115 120 125

Thr Met Lys Ile Ala Glu Ala Gly Lys Val Ser Ile Gln Gln Gln Ser
130 135 140

His Met Pro Leu Gln Tyr Ile Tyr Thr Leu Phe Leu Glu His Asp Leu
145 150 155 160

Ser Leu Gly Thr Leu Ala Met Glu Thr Val Ala Gln Gln Lys Arg Asp
165 170 175

Tyr Tyr Gln Pro Leu Ala Ala Lys Arg Thr Glu Ile Glu Lys Trp Arg
180 185 190

Lys Glu Phe Lys Glu Gln Trp Met Lys Glu Gln Lys Arg Met Asn Glu
195 200 205

Ala Val Gln Ala Leu Arg Arg Ala Gln Leu Gln Tyr Val Gln Arg Ser
210 215 220

Glu Asp Leu Arg Ala Arg Ser Gln Gly Ser Pro Glu Asp Ser Ala Pro
225 230 235 240

Gln Ala Ser Pro Gly Pro Ser Lys Gln Gln Glu Arg Arg Arg Arg Ser
245 250 255

Arg Glu Glu Ala Gln Ala Lys Ala Gln Glu Ala Glu Ala Leu Tyr Gln
260 265 270

Ala Cys Val Arg Glu Ala Asn Ala Arg Gln Gln Asp Leu Glu Ile Ala
275 280 285

Lys Gln Arg Ile Val Ser His Val Arg Lys Leu Val Phe Gln Gly Asp
290 295 300

Glu Val Leu Arg Arg Val Thr Leu Ser Leu Phe Gly Leu Arg Gly Ala
305 310 315 320

Gln Ala Glu Arg Gly Pro Arg Ala Phe Ala Ala Leu Ala Glu Cys Cys
325 330 335

Ala Pro Phe Glu Pro Gly Gln Arg Tyr Gln Glu Phe Val Arg Ala Leu
340 345 350

Arg Pro Glu Ala Pro Pro Pro Pro Pro Pro Ala Phe Ser Phe Gln Glu
355 360 365

Phe Leu Pro Ser Leu Asn Ser Ser Pro Leu Asp Ile Arg Lys Lys Leu
370 375 380

Ser Gly Pro Leu Pro Pro Arg Leu Asp Glu Asn Ser Ala Glu Pro Gly
385 390 395 400

Pro Trp Glu Asp Pro Gly Thr Gly Trp Arg Trp Gln Gly Thr Pro Gly
405 410 415

Pro Thr Pro Gly Ser Asp Val Asp Ser Val Gly Gly Gly Ser Glu Ser
420 425 430

Arg Ser Leu Asp Ser Pro Thr Ser Ser Pro Gly Ala Gly Thr Arg Gln
435 440 445

Leu Val Lys Ala Ser Ser Thr Gly Thr Glu Ser Ser Asp Asp Phe Glu
450 455 460

Glu Arg Asp Pro Gly Glu Ala Glu Ala Gly
465 470

<210> 175
<211> 36
<212> PRT
<213> Artificial

<400> 175

Met Asp Ala Ala Glu Pro Gly Leu Pro Pro Gly Pro Glu Gly Arg Lys
1 5 10 15

Arg Tyr Ser Asp Ile Phe Arg Ser Leu Asp Asn Leu Glu Ile Ser Leu
20 25 30

Gly Asn Asp Pro
35

<210> 176
<211> 694
<212> PRT
<213> Artificial

<400> 176

Met Asp Ala Ala Glu Pro Gly Leu Pro Pro Gly Pro Glu Gly Arg Lys
1               5                   10                  15

Arg Tyr Ser Asp Ile Phe Arg Ser Leu Asp Asn Leu Glu Ile Ser Leu
            20                  25                  30

Gly Asn Val Thr Leu Glu Met Leu Ala Gly Asp Pro Leu Leu Ser Glu
        35                  40                  45

Asp Pro Glu Pro Asp Lys Thr Pro Thr Ala Thr Val Thr Asn Glu Ala
    50                  55                  60

Ser Cys Trp Ser Gly Pro Ser Pro Glu Gly Pro Val Pro Leu Thr Gly
65                  70                  75                  80

Glu Glu Leu Asp Leu Arg Leu Ile Arg Thr Lys Gly Gly Val Asp Ala
                85                  90                  95

Ala Leu Glu Tyr Ala Lys Thr Trp Ser Arg Tyr Ala Lys Glu Leu Leu
100 105 110

Ala Trp Thr Glu Lys Arg Ala Ser Tyr Glu Leu Glu Phe Ala Lys Ser
115 120 125

Thr Met Lys Ile Ala Glu Ala Gly Lys Val Ser Ile Gln Gln Gln Ser
130 135 140

His Met Pro Leu Gln Tyr Ile Tyr Thr Leu Phe Leu Glu His Asp Leu
145 150 155 160

Ser Leu Gly Thr Leu Ala Met Glu Thr Val Ala Gln Gln Lys Arg Asp
165 170 175

Tyr Tyr Gln Pro Leu Ala Ala Lys Arg Thr Glu Ile Glu Lys Trp Arg
180 185 190

Lys Glu Phe Lys Glu Gln Trp Met Lys Glu Gln Lys Arg Met Asn Glu
195 200 205

Ala Val Gln Ala Leu Arg Arg Ala Gln Leu Gln Tyr Val Gln Arg Ser
210 215 220

Glu Asp Leu Arg Ala Arg Ser Gln Gly Ser Pro Glu Asp Ser Ala Pro
225 230 235 240

Gln Ala Ser Pro Gly Pro Ser Lys Gln Gln Glu Arg Arg Arg Arg Ser
245 250 255

Arg Glu Glu Ala Gln Ala Lys Ala Gln Glu Ala Glu Ala Leu Tyr Gln
260 265 270

Ala Cys Val Arg Glu Ala Asn Ala Arg Gln Gln Asp Leu Glu Ile Ala
275 280 285

Lys Gln Arg Ile Val Ser His Val Arg Lys Leu Val Phe Gln Gly Asp
290 295 300

Glu Val Leu Arg Arg Val Thr Leu Ser Leu Phe Gly Leu Arg Gly Ala
305 310 315 320

Gln Ala Glu Arg Gly Pro Arg Ala Phe Ala Ala Leu Ala Glu Cys Cys
325 330 335

Ala Pro Phe Glu Pro Gly Gln Arg Tyr Gln Glu Phe Val Arg Ala Leu
340 345 350

Arg Pro Glu Ala Pro Pro Pro Pro Pro Pro Ala Phe Ser Phe Gln Glu
355 360 365

Phe Leu Pro Ser Leu Asn Ser Ser Pro Leu Asp Ile Arg Lys Lys Leu
370 375 380

Ser Gly Pro Leu Pro Pro Arg Leu Asp Glu Asn Ser Ala Glu Pro Gly
385 390 395 400

Pro Trp Glu Asp Pro Gly Thr Gly Trp Arg Trp Gln Gly Thr Pro Gly
405 410 415

Pro Thr Pro Gly Ser Asp Val Asp Ser Val Gly Gly Gly Ser Glu Ser
420 425 430

Arg Ser Leu Asp Ser Pro Thr Ser Ser Pro Gly Ala Gly Thr Arg Gln
435 440 445

Leu Val Lys Ala Ser Ser Thr Gly Thr Glu Ser Ser Asp Asp Phe Glu
450 455 460

Glu Arg Asp Pro Asp Leu Gly Asp Gly Leu Glu Asn Gly Leu Gly Ser
465 470 475 480

Pro Phe Gly Lys Trp Thr Leu Ser Ser Ala Ala Gln Thr His Gln Leu
485 490 495

Arg Arg Leu Arg Gly Pro Ala Lys Cys Arg Glu Cys Glu Ala Phe Met
500 505 510

Val Ser Gly Thr Glu Cys Glu Glu Cys Phe Leu Thr Cys His Lys Arg
515 520 525

Cys Leu Glu Thr Leu Leu Ile Leu Cys Gly His Arg Arg Leu Pro Ala
530 535 540

Arg Thr Pro Leu Phe Gly Val Asp Phe Leu Gln Leu Pro Arg Asp Phe
545 550 555 560

Pro Glu Glu Val Pro Phe Val Val Thr Lys Cys Thr Ala Glu Ile Glu
565 570 575

His Arg Ala Leu Asp Val Gln Gly Ile Tyr Arg Val Ser Gly Ser Arg
580 585 590

Val Arg Val Glu Arg Leu Cys Gln Ala Phe Glu Asn Gly Arg Ala Leu
595 600 605

Val Glu Leu Ser Gly Asn Ser Pro His Asp Val Ser Ser Val Leu Lys
610 615 620

Arg Phe Leu Gln Glu Gly Gly Cys Thr Ile Tyr Gly Lys Gln Asp Val
625 630 635 640

Cys Gln Gln Pro Gly His Cys Val Trp Ala Asp Thr Ala Ala Ala Ala
645 650 655

Gly Arg Pro Ala Gly Ser Gln Arg His Pro Cys His Leu Pro Ala Gly
660 665 670

Leu Trp Ala Ser Gly Pro Ala Cys Gly Val Pro His Arg Ala Leu Arg
675 680 685

Ala Asp Leu Trp Asp Gly
690

<210> 177
<211> 1977
<212> DNA
<213> Artificial

<400> 177

ctgggattgg taacctggag ttaagcgggc tccctgccaa cgcgagggct ttaaaagggg 60
tgatgcaacg cgctcccagc cacagtctca ctcagcgaga cgccgcgcac ggtgcttccc 120
cagtggagcc aatcggctaa cccgcgctcc ggcagagtcc ttggcgctcg cccgccggcg 180
ggacagacca cccgcctctg gccgctctct ggaccctggc cgccccgagc gaagactgga 240
gcaaaatgat gcttcaacat ccaggccagg tctctgcctc agaagtcagt gcgaccgcca 300
ttgtcccctg cctctcacct cctgggtcac tggtatttga ggattttgct aacctgacac 360
cctttgtcaa ggaagagctg agattcgcca tccagaataa acacctctgc catcggatgt 420
cctctgcgct ggagtcagtt accgtcaaca acagacccct ggagatgtca gtcaccaagt 480
ctgaggcggc ccctgaagaa gatgagagga aaaggaggcg gcgagaaaga aataaaattg 540
ctgctgccaa gtgtcgaaac aagaaaaagg agaagacaga gtgcctgcag aaagagtcag 600
agaaactgga gagtgtgaat gctgagctga aggcccagat tgaggagctg aagaatgaga 660
aacagcattt gatatacatg ctcaacctgc accggcccac ctgcatcgtc cgggctcaga 720
atggacggac accggaagac gagaggaacc tctttatcca acagataaaa gaaggaacat 780
tgcagagcta agcagaggtg gcacggaggc aattggggag ttcttactga atcctccttt 840
tccaccccac accctgaagc cattggaaaa ctggcttcct gtgcacttct agaatcccag 900
cagccaagag ccgttggggc aggagggcct gtggtgacct actgcattga cccactctgc 960
ccccgagtga accgtggagc aggcaggagc atcctttgtc tcaccaattc caggatttag 1020
gccttatcat cccggccagt ctcagatgac ctagctggcc ccaggctggg gtcctatgca 1080
aagcaggatc ccactaatgg gattcaggca gaagtgtcta ccttgatagg tggggtggga 1140
ccacatcctc cactgtggct gacaacgccc ttccaaggga atatggaatg agaacattca 1200
ttattgaggt tgtccaatgg ccagggtatg ctttctagaa aatatgctgt tctgtcccag 1260
aatgactgtg catagggtat ccgtttcaga gcctggtgtt gtgctattta gatgtttgtc 1320

ttgcacaaca ttggcatgat ttttccggga gtttcatcag atctgatttc tgagagtctg 1380
gggatctgcc atggtggaaa gtgcccctca aaagcatttg tgtggccaca tgaactggct 1440
ggcaccaggg gagtgaaact ggctgatgac cagctgagcc actttgtgcc aacagaggat 1500
ggacgacacc tttccctgta cccactgcag aggaagaacc ctgggcacag cagctttgtc 1560
cttggctaca aactgttaca acgtcacaca atgaaggcac aaagtccaac tttcaaaggg 1620
tgtaggactc catactcagt gacagggcag gaagagccaa agataaccac agccacagcc 1680
tgtggagacc agggttggaa gccaggtgca gggccaggca tctgcattgt gggatgttaa 1740
tggcactttt gtcttgtagc tattttgaga tgtggtccag agcatttcag ctgggagatc 1800
tccctctggc caccaggact ctggctactg ttaaaatcct gatgtttctg tggaatcctc 1860
agtgtttaat cccactcaat agtatcatta cagttttctg taagagaaaa tattacttat 1920
ttatcccagt attcctagcc tgtcaacata ataaatatcg gaacaaaacc tggtaaa 1977

<210> 178
<211> 1871
<212> DNA
<213> Artificial

<400> 178

ctgggattgg taacctggag ttaagcgggc tccctgccaa cgcgagggct ttaaaagggg 60
tgatgcaacg cgctcccagc cacagtctca ctcagcgaga cgccgcgcac ggtgcttccc 120
cagtggagcc aatcggctaa cccgcgctcc ggcagagtcc ttggcgctcg cccgccggcg 180
ggacagacca cccgcctctg gccgctctct ggaccctggc cgccccgagc gaagactgga 240
gcaaaatgat gcttcaacat ccaggccaga gctgagattc gccatccaga ataaacacct 300
ctgccatcgg atgtcctctg cgctggagtc agttaccgtc aacaacagac ccctggagat 360
gtcagtcacc aagtctgagg cggcccctga agaagatgag aggaaaagga ggcggcgaga 420
aagaaataaa attgctgctg ccaagtgtcg aaacaagaaa aaggagaaga cagagtgcct 480
gcagaaagag tcagagaaac tggagagtgt gaatgctgag ctgaaggccc agattgagga 540
gctgaagaat gagaaacagc atttgatata catgctcaac ctgcaccggc ccacctgcat 600
cgtccgggct cagaatggac ggacaccgga agacgagagg aacctcttta tccaacagat 660
aaaagaagga acattgcaga gctaagcaga ggtggcacgg aggcaattgg ggagttctta 720
ctgaatcctc cttttccacc ccacaccctg aagccattgg aaaactggct tcctgtgcac 780
ttctagaatc ccagcagcca agagccgttg gggcaggagg gcctgtggtg acctactgca 840
ttgacccact ctgcccccga gtgaaccgtg gagcaggcag gagcatcctt tgtctcacca 900
attccaggat ttaggcctta tcatcccggc cagtctcaga tgacctagct ggccccaggc 960
tggggtccta tgcaaagcag gatcccacta atgggattca ggcagaagtg tctaccttga 1020
taggtggggt gggaccacat cctccactgt ggctgacaac gcccttccaa gggaatatgg 1080
aatgagaaca ttcattattg aggttgtcca atggccaggg tatgctttct agaaaatatg 1140

ctgttctgtc ccagaatgac tgtgcatagg gtatccgttt cagagcctgg tgttgtgcta 1200
tttagatgtt tgtcttgcac aacattggca tgatttttcc gggagtttca tcagatctga 1260
tttctgagag tctggggatc tgccatggtg gaaagtgccc ctcaaaagca tttgtgtggc 1320
cacatgaact ggctggcacc aggggagtga aactggctga tgaccagctg agccactttg 1380
tgccaacaga ggatggacga cacctttccc tgtacccact gcagaggaag aaccctgggc 1440
acagcagctt tgtccttggc tacaaactgt tacaacgtca cacaatgaag gcacaaagtc 1500
caactttcaa agggtgtagg actccatact cagtgacagg gcaggaagag ccaaagataa 1560
ccacagccac agcctgtgga gaccagggtt ggaagccagg tgcagggcca ggcatctgca 1620
ttgtgggatg ttaatggcac ttttgtcttg tagctatttt gagatgtggt ccagagcatt 1680
tcagctggga gatctccctc tggccaccag gactctggct actgttaaaa tcctgatgtt 1740
tctgtggaat cctcagtgtt taatcccact caatagtatc attacagttt tctgtaagag 1800
aaaatattac ttatttatcc cagtattcct agcctgtcaa cataataaat atcggaacaa 1860
aacctggtaa a 1871

<210> 179
<211> 3932
<212> DNA
<213> Artificial

<400> 179

```
ggggcgacac cactgggtcg gcagaggcag gaaatcgggc tggtcccggg cggcgcgcag      60
ccctgtccct ttctcagcat tgccgcgcgt tccccacccc accccctgcc cccgggcatc     120
gccatggact cagcggagac agagctaacc ccagctcctg agggcaggaa gagatacagt     180
gacatcttcc agagcctgga caacctcgag atatcattgg ggaacgtaac ttttgatccc     240
ctggctggag accctgtacg cagagaagac ctggagcctg acaaagctga cacagccaca     300
gtggtgactg aggagaacag tgaagccagt agttggcggg atctctcccc agaaggtcct     360
gcacccctca cagaggaaga actggatttg cgactcattc ggaccaaggg tggtgtagat     420
gctgccctgg agtatgctaa agcatggagc cgctatgcca aggaactgct ggcctggaca     480
gacaagagag ccaactacga gctcgagttt gctaagagta tcatgaagct cgctgaggct     540
ggcaaggtgt ccattctcca gcagagccaa atgccactcc agtacatcta caccttgttt     600
ctggagcatg acctcagcct gggagccctg gccctggaga cagtggccca acagaagaga     660
gactactacc agcctctggc ggccaaaagg atggaaattg agaagtggag gaaggaattc     720
aaggaacagt ggctgaagga gcagaagcgc atgaatgagg cagtgcaggc actgcggcgc     780
tccgagctcc agtacattca acgcagagag gacctgaggg cacgctccca ggggtcccct     840
gaggaccctc cttcccaggc atctcctgga tccaacaagc aacaggagcg cagacggcgc     900
tcccgagagg aggcacaggc caaggcgcat gaggcggagg ctctgtacca ggcctgtgtc     960
cgggaggcca attctcgcca gcaggatctg gagaccacca agcggcggat agtgtcacat    1020
```

gtgcgcaagc tggtgctgca aggagacgaa gtgcttaggc gggtgacgct gggtctgttt 1080
gagctgcgag gggcgcaggc agagcgagga ccccgctcct tctctgctct ggctgagtgc 1140
tgtgtgccct ttgaacctgg ccagcgctac caggagtttg ttcggacact gcagcctggg 1200
gccccaccgc cgccatctcc ggccttctgc ttccaggagt tcacagctgt ggtgcacagt 1260
ttccctcagg acacaaaaaa gaagttttcg ggcctctac ctccaaggct ggaggaggag 1320
ggttcccctg agcctggccc ttgggaggtt gccagcttag gcagccaggg aatcccaggc 1380
agtgatgtgg acagtgtagg tggtggcagt gagtcccggt ctttggattc tcccacttcc 1440
agcccaggtg ctggggcccg ccgacttgta aaggcttcgt ccacaggcac ggagtcctcc 1500
gatgactttg aggagcgaga ccctgatctg gggatggga tagagaatgg agtaggcagc 1560
ccgttcagga agtggacact gtccacagct gctcaaaccc accgactgcg gcggctgcgg 1620
ggcccagcca agtgcagaga atgtgaagcc ttcatggtca gcgggacaga atgtgaagag 1680
tgctttttga cctgtcacaa gcgctgcctg gagaccctcc tcatcctttg tggacaccgg 1740
cggcttccgg cccggatgtc cctctttggg gttgacttcc tacagctccc cagagatttc 1800
cctgaggagg ttccctttgt gattaccaga tgcacagctg agatagagca ccgtgccctg 1860
ggcttgcagg gtatctatcg ggtcagcggg tctcgggtac gtgtggagcg gctgtgccag 1920
gcctttgaga atggccgagc actggtcgag ctgtccggga actctcctca cgatatcacc 1980
agcgttctca agcgatttct tcaggagctc actgatcccg tggtcccctt ccacttgtac 2040
gacgccttca tctctctggc aaagaccctg catgcagacc ctggggacga ccctgggacc 2100
cccaacccca gccctgagat tattcgctcg ctgaagaccc tcttggtgca gctgcctgac 2160
tctaactaca gtaccctgcg acacctggtg gcccatctgt tcagggtggc tgctcggttt 2220
gaagaaaaca agatgtctgc caacaatctg ggaattgtat ttgggcctac actgctgcgg 2280
ccaccagatg gacccagggc caccggtgcc agccctgtgg cctgtctgct ggactccggt 2340
caccaggctc agctggttga gttcctcatt gtgcactatg agcagatctt tggaatggat 2400
gagctccctc tggcctcgga gcctctgacc caagaccctg gcttggctcc tgcatgcctt 2460
gaatccagtc cccagcaccc agcctcactt cttgcccaag acacacagcc cctgaccata 2520
gccttggatt ccagcccaga ccccaaacac cacagtgcct tggagaagtg ccccgaggtc 2580
acacctcctg agcttgcaac tctgcagagg gaccagaggg aggaggaggt ggaagacacc 2640
agagatgggg caggggatgg gtccagccac tgccctgagg acttggccct gggagcacaa 2700
tcccggggcc acttcagccg ccagccagtg aagtattccc ggggaggtgt gaggccagtt 2760
actcatcaac tctccagcct ggctctggtg gcctccaagc tgtgtgagga gactcctgtc 2820
actgtctcag cagtgcaccg aggtagtttg agggtacgag gtttgggccc tgctgctgcc 2880
tgccctgaag gcagccccct gcgccggaac cctctgccca agcactttga gatcacccag 2940
gagacagccc gacttctctc caagctgaac agtgatgctg tgtccaggac cacctgttgt 3000
gctgacccag agcctgagga gtctgaagag cacctctgac cacctaccat cctgagggac 3060

ccagaactga attgatggtc cctgacccaa cctggtgacc aaaccaattc agtggggtgt 3120
ggggagagac ggggttacca gtgagttccg gtaatgaaga ttacctacct gtgggagtcc 3180
caagacactt ggttgaagtg tcccatcatc tccctgccag aggagattta aacagctagg 3240
caacacaggt gcaggtgttt cggggtcaag actcaatggt caatacagat cagtagatcc 3300
ttggggacta cccgagtctc tgacctctga gacgggtgtc ttgactattt cagtcataat 3360
ggctcctggc cctactacac ttgcagcagt aacagactcc caagttagca ggtggctgca 3420
gcccagccct gtctcctttt aatttgtttg tttgtttgtt tgttttaagc taagtctcac 3480
tgtgtagctc tggctggtct ggagctccct ttgtgagcca caaatcctct gcctcccaag 3540
tgctggctaa atctgtgttc ttacaacctc caaataaaac catatcctgc tgccggcagg 3600
agggctgacc gcagtggtgc ttgctgtaaa gcctcacatt tgttggacct cccagagcca 3660
cttagtggaa ggagagagcc catccctaca aattgtctga cctgtgcaca tgcacaaata 3720
aaagaaaatt ttaatttaaa aaaaaaaatc cgctgggcgg tagtgccaca cacctctact 3780
tgatcctagc actcaggaga tagaggcagg aggatctctg agttttatgc cagccaggtg 3840
tacagagaaa gttccaggac agccagggct acacagagaa accctgtttc agaaaaacaa 3900
aataaaataa gataaatctt gtctgttctt gc 3932

<210> 180
<211> 3922
<212> DNA
<213> Artificial

<400> 180

```
ggggcgacac cactgggtcg gcagaggcag gaaatcgggc tggtcccggg cggcgcgcag     60
ccctgtccct ttctcagcat tgccgcgcgt tccccacccc accccctgcc cccgggcatc    120
gccatggact cagcggagac agagctaacc ccagctcctg agggcaggaa gagatacagt    180
gacatcttcc agagcctgga caacctcgag atatcattgg ggaacgtaac ttttgatccc    240
ctggctggag accctgtacg cagagaagac ctggagcctg acaaagctga cacagccaca    300
gtggtgactg aggagaacag tgaagccagt agttggcggg atctctcccc agaaggtcct    360
gcacccctca cagaggaaga actggatttg cgactcattc ggaccaaggg tggtgtagat    420
gctgccctgg agtatgctaa agcatggagc cgctatgcca aggaactgct ggcctggaca    480
gacaagagag ccaactacga gctcgagttt gctaagagta tcatgaagct cgctgaggct    540
ggcaaggtgt ccattctcca gcagagccaa atgccactcc agtacatcta caccttgttt    600
ctggagcatg acctcagcct gggagccctg gccctggaga cagtggccca acagaagaga    660
gactactacc agcctctggc ggccaaaagg atggaaattg agaagtggag gaaggaattc    720
aaggaacagt ggctgaagga gcagaagcgc atgaatgagg cagtgcaggc actgcggcgc    780
tccgagctcc agtacattca acgcagagag gacctgaggg cacgctccca ggggtcccct    840
gaggaccctc cttcccaggc atctcctgga tccaacaagc aacaggagcg cagacggcgc    900
```

tcccgagagg aggcacaggc caaggcgcat gaggcggagg ctctgtacca ggcctgtgtc 960
cgggaggcca attctcgcca gcaggatctg gagaccacca agcggcggat agtgtcacat 1020
gtgcgcaagc tggtgctgca aggagacgaa gtgcttaggc gggtgacgct gggtctgttt 1080
gagctgcgag gggcgcaggc agagcgagga ccccgctcct tctctgctct ggctgagtgc 1140
tgtgtgccct ttgaacctgg ccagcgctac caggagtttg ttcggacact gcagcctggg 1200
gccccaccgc cgccatctcc ggccttctgc ttccaggagt tcacagctgt ggtgcacagg 1260
acacaaaaaa gaagttttcg gggcctctac ctccaaggct ggaggaggag ggttcccctg 1320
agcctggccc ttgggaggtt gccagcttag gcagccaggg aatcccaggc agtgatgtgg 1380
acagtgtagg tggtggcagt gagtcccggt ctttggattc tcccacttcc agcccaggtg 1440
ctggggcccg ccgacttgta aaggcttcgt ccacaggcac ggagtcctcc gatgactttg 1500
aggagcgaga ccctgatctg gggggatgga tagagaatgg agtaggcagc ccgttcagga 1560
agtggacact gtccacagct gctcaaaccc accgactgcg gcggctgcgg ggcccagcca 1620
agtgcagaga atgtgaagcc ttcatggtca gcgggacaga atgtgaagag tgctttttga 1680
cctgtcacaa gcgctgcctg gagaccctcc tcatcctttg tggacaccgg cggcttccgg 1740
cccggatgtc cctctttggg gttgacttcc tacagctccc cagagatttc cctgaggagg 1800
ttccctttgt gattaccaga tgcacagctg agatagagca ccgtgccctg ggcttgcagg 1860
gtatctatcg ggtcagcggg tctcgggtac gtgtggagcg gctgtgccag gcctttgaga 1920
atggccgagc actggtcgag ctgtccggga actctcctca cgatatcacc agcgttctca 1980
agcgatttct tcaggagctc actgatcccg tggtcccctt ccacttgtac gacgccttca 2040
tctctctggc aaagaccctg catgcagacc ctggggacga ccctgggacc cccaacccca 2100
gccctgagat tattcgctcg ctgaagaccc tcttggtgca gctgcctgac tctaactaca 2160
gtaccctgcg acacctggtg gcccatctgt tcagggtggc tgctcggttt gaagaaaaca 2220
agatgtctgc caacaatctg ggaattgtat ttgggcctac actgctgcgg ccaccagatg 2280
gacccagggc caccggtgcc agccctgtgg cctgtctgct ggactccggt caccaggctc 2340
agctggttga gttcctcatt gtgcactatg agcagatctt tggaatggat gagctccctc 2400
tggcctcgga gcctctgacc caagaccctg gcttggctcc tgcatgcctt gaatccagtc 2460
cccagcaccc agcctcactt cttgcccaag acacacagcc cctgaccata gccttggatt 2520
ccagcccaga ccccaaacac cacagtgcct tggagaagtg ccccgaggtc acacctcctg 2580
agcttgcaac tctgcagagg gaccagaggg aggaggaggt ggaagacacc agagatgggg 2640
caggggatgg gtccagccac tgccctgagg acttggccct gggagcacaa tcccggggcc 2700
acttcagccg ccagccagtg aagtattccc ggggaggtgt gaggccagtt actcatcaac 2760
tctccagcct ggctctggtg gcctccaagc tgtgtgagga gactcctgtc actgtctcag 2820
cagtgcaccg aggtagtttg agggtacgag gtttgggccc tgctgctgcc tgccctgaag 2880
gcagcccccct gcgccggaac cctctgccca agcactttga gatcacccag gagacagccc 2940

gacttctctc caagctgaac agtgatgctg tgtccaggac cacctgttgt gctgacccag 3000

agcctgagga gtctgaagag cacctctgac cacctaccat cctgagggac ccagaactga 3060

attgatggtc cctgacccaa cctggtgacc aaaccaattc agtggggtgt ggggagagac 3120

ggggttacca gtgagttccg gtaatgaaga ttacctacct gtgggagtcc caagacactt 3180

ggttgaagtg tcccatcatc tccctgccag aggagattta aacagctagg caacacaggt 3240

gcaggtgttt cggggtcaag actcaatggt caatacagat cagtagatcc ttggggacta 3300

cccgagtctc tgacctctga gacgggtgtc ttgactattt cagtcataat ggctcctggc 3360

cctactacac ttgcagcagt aacagactcc caagttagca ggtggctgca gcccagccct 3420

gtctcctttt aatttgtttg tttgtttgtt tgttttaagc taagtctcac tgtgtagctc 3480

tggctggtct ggagctccct ttgtgagcca caaatcctct gcctcccaag tgctggctaa 3540

atctgtgttc ttacaacctc caaataaaac catatcctgc tgccggcagg agggctgacc 3600

gcagtggtgc ttgctgtaaa gcctcacatt tgttggacct cccagagcca cttagtggaa 3660

ggagagagcc catccctaca aattgtctga cctgtgcaca tgcacaaata aaagaaaatt 3720

ttaatttaaa aaaaaaaatc cgctgggcgg tagtgccaca cacctctact tgatcctagc 3780

actcaggaga tagaggcagg aggatctctg agttttatgc cagccaggtg tacagagaaa 3840

gttccaggac agccagggct acacagagaa accctgtttc agaaaaacaa aataaaataa 3900

gataaatctt gtctgttctt gc 3922

<210> 181
<211> 3865
<212> DNA
<213> Artificial

<400> 181

```
ggggcgacac cactgggtcg gcagaggcag gaaatcgggc tggtcccggg cggcgcgcag     60
ccctgtccct ttctcagcat tgccgcgcgt tccccacccc accccctgcc cccgggcatc    120
gccatggact cagcggagac agagctaacc ccagctcctg agggcaggaa gagatacagt    180
gacatcttcc agagcctgga caacctcgag atatcattgg ggaacgtaac ttttgatccc    240
ctggctggag accctgtacg cagagaagac ctggagcctg acaaagctga cacagccaca    300
gtggtgactg aggagaacag tgaagccagt agttggcggg atctctcccc agaaggtcct    360
gcacccctca cagaggaaga actggatttg cgactcattc ggaccaaggg tggtgtagat    420
gctgccctgg agtatgctaa agcatggagc cgctatgcca aggaactgct ggcctggaca    480
gacaagagag ccaactacga gctcgagttt gctaagagta tcatgaagct cgctgaggct    540
ggcaaggtgt ccattctcca gcagagccaa atgccactcc agtacatcta caccttgttt    600
ctggagcatg acctcagcct gggagccctg gccctggaga cagtggccca acagaagaga    660
gactactacc agcctctggc ggccaaaagg atggaaattg agaagtggag gaaggaattc    720
aaggaacagt ggctgaagga gcagaagcgc atgaatgagg cagtgcaggc actgcggcgc    780
```

tccgagctcc agtacattca acgcagagag gacctgaggg cacgctccca ggggtcccct 840
gaggaccctc cttcccaggc atctcctgga tccaacaagc aacaggagcg cagacggcgc 900
tcccgagagg aggcacaggc caaggcgcat gaggcggagg ctctgtacca ggcctgtgtc 960
cgggaggcca attctcgcca gcaggatctg gagaccacca agcggcggat agtgtcacat 1020
gtgcgcaagc tggtgctgca aggagacgaa gtgcttaggc gggtgacgct gggtctgttt 1080
gagctgcgag ggcgcaggc agagcgagga ccccgctcct tctctgctct ggctgagtgc 1140
tgtgtgccct ttgaacctgg ccagcgctac caggagtttg ttcggacact gcagcctggg 1200
gccccaccgc cgccatctcc ggccttctgc ttccaggagt tcacagctgt ggtgcacagt 1260
ttccctcagg acacaaaaaa gaagttttcg gggcctctac ctccaaggct ggaggaggag 1320
ggttcccctg agcctggccc ttgggaggtt gccagcttag gcagccaggg aatcccaggc 1380
agtgatgtgg acagtgtagg tggtggcagt gagtcccggt ctttggattc tcccacttcc 1440
agcccaggtg ctggggcccg ccgacttgta aaggcttcgt ccacaggcac ggagtcctcc 1500
gatgactttg aggagcgaga ccctgatctg gggatggga tagagaatgg agtaggcagc 1560
ccgttcagga agtggacact gtccacagct gctcaaaccc accgactgcg gcggctgcgg 1620
ggcccagcca agtgcagaga atgtgaagcc ttcatggtca gcgggacaga atgtgaagag 1680
tgctttttga cctgtcacaa gcgctgcctg gagaccctcc tcatcctttg tggacaccgg 1740
cggcttccgg cccggatgtc cctctttggg gttgacttcc tacagctccc cagagatttc 1800
cctgaggagg ttccctttgt gattaccaga tgcacagctg agatagagca ccgtgccctg 1860
ggcttgcagg gtatctatcg ggtcagcggg tctcgggtac gtgtggagcg gctgtgccag 1920
gcctttgaga atggccgagc actggtcgag ctgtccggga actctcctca cgatatcacc 1980
agcgttctca agcgatttct tcaggagctc actgatcccg tggtcccctt ccacttgtac 2040
gacgccttca tctctctggc aaagaccctg catgcagacc ctggggacga ccctgggacc 2100
cccaacccca gccctgagat tattcgctcg ctgaagaccc tcttggtgca gctgcctgac 2160
tctaactaca gtaccctgcg acacctggtg gcccatctgt tcagggtggc tgctcggttt 2220
gaagaaaaca agatgtctgc caacaatctg ggaattgtat ttgggcctac actgctgcgg 2280
ccaccagatg gacccagggc caccggtgcc agccctgtgg cctgtctgct ggactccggt 2340
caccaggctc agctggttga gttcctcatt gtgcactatg agcagatctt tggaatggat 2400
gagctccctc tggcctcgga gcctctgacc caagaccctg gcttggctcc tgcatgcctt 2460
gaatccagtc cccagcaccc agcctcactt cttgcccaag acacacagcc cctgaccata 2520
gccttggatt ccagcccaga ccccaaacac cacagtgcct tggagaagtg ccccgaggtc 2580
acacctcctg agggtccagc cactgccctg aggacttggc cctgggagca caatcccggg 2640
gccacttcag ccgccagcca gtgaagtatt cccggggagg tgtgaggcca gttactcatc 2700
aactctccag cctggctctg gtggcctcca agctgtgtga ggagactcct gtcactgtct 2760
cagcagtgca ccgaggtagt ttgagggtac gaggtttggg ccctgctgct gcctgccctg 2820

aaggcagccc cctgcgccgg aaccctctgc ccaagcactt tgagatcacc caggagacag 2880
cccgacttct ctccaagctg aacagtgatg ctgtgtccag gaccacctgt tgtgctgacc 2940
cagagcctga ggagtctgaa gagcacctct gaccacctac catcctgagg gacccagaac 3000
tgaattgatg gtccctgacc caacctggtg accaaaccaa ttcagtgggg tgtggggaga 3060
gacggggtta ccagtgagtt ccggtaatga agattaccta cctgtgggag tcccaagaca 3120
cttggttgaa gtgtcccatc atctccctgc cagaggagat ttaaacagct aggcaacaca 3180
ggtgcaggtg tttcggggtc aagactcaat ggtcaataca gatcagtaga tccttgggga 3240
ctacccgagt ctctgacctc tgagacgggt gtcttgacta tttcagtcat aatggctcct 3300
ggccctacta cacttgcagc agtaacagac tcccaagtta gcaggtggct gcagcccagc 3360
cctgtctcct tttaatttgt ttgtttgttt gtttgtttta agctaagtct cactgtgtag 3420
ctctggctgg tctggagctc cctttgtgag ccacaaatcc tctgcctccc aagtgctggc 3480
taaatctgtg ttcttacaac ctccaaataa aaccatatcc tgctgccggc aggagggctg 3540
accgcagtgg tgcttgctgt aaagcctcac atttgttgga cctcccagag ccacttagtg 3600
gaaggagaga gcccatccct acaaattgtc tgacctgtgc acatgcacaa ataaaagaaa 3660
attttaattt aaaaaaaaaa atccgctggg cggtagtgcc acacacctct acttgatcct 3720
agcactcagg agatagaggc aggaggatct ctgagtttta tgccagccag gtgtacagag 3780
aaagttccag gacagccagg gctacacaga gaaaccctgt ttcagaaaaa caaaataaaa 3840
taagataaat cttgtctgtt cttgc 3865

<210> 182
<211> 2764
<212> DNA
<213> Artificial

<400> 182

ggggcgacac cactgggtcg gcagaggcag gaaatcgggc tggtcccggg cggcgcgcag 60

ccctgtccct ttctcagcat tgccgcgcgt tccccacccc accccctgcc cccgggcatc 120

gccatggact cagcggagac agagctaacc ccagctcctg agggcaggaa gagatacagt 180

gacatcttcc agagcctgga caacctcgag atatcattgg ggaacgtaac ttttgatccc 240

ctggctggag accctgtacg cagagaagac ctggagcctg acaaagctga cacagccaca 300

gtggtgactg aggagaacag tgaagccagt agttggcggg atctctcccc agaaggtcct 360

gcacccctca cagaggaaga actggatttg cgactcattc ggaccaaggg tggtgtagat 420

gctgccctgg agtatgctaa agcatggagc cgctatgcca aggaactgct ggcctggaca 480

gacaagagag ccaactacga gctcgagttt gctaagagta tcatgaagct cgctgaggct 540

ggcaaggtgt ccattctcca gcagagccaa atgccactcc agtacatcta caccttgttt 600

ctggagcatg acctcagcct gggagccctg gccctggaga cagtggccca acagaagaga 660

gactactacc agcctctggc ggccaaaagg atggaaattg agaagtggag gaaggaattc 720

aaggaacagt ggctgaagga gcagaagcgc atgaatgagg cagtgcaggc actgcggcgc 780
tccgagctcc agtacattca acgcagagag gacctgaggg cacgctccca ggggtcccct 840
gaggaccctc cttcccaggc atctcctgga tccaacaagc aacaggagcg cagacggcgc 900
tcccgagagg aggcacaggc caaggcgcat gaggcggagg ctctgtacca ggcctgtgtc 960
cgggaggcca attctcgcca gcaggatctg gagaccacca agcggcggat agtgtcacat 1020
gtgcgcaagc tggtgctgca aggagacgaa gtgcttaggc gggtgacgct gggtctgttt 1080
gagctgcgag ggcgcaggc agagcgagga ccccgctcct tctctgctct ggctgagtgc 1140
tgtgtgccct ttgaacctgg ccagcgctac caggagtttg ttcggacact gcagcctggg 1200
gccccaccgc cgccatctcc ggccttctgc ttccaggagt tcacagctgt ggtgcacagt 1260
ttccctcagg acacaaaaaa gaagttttcg ggcctctac ctccaaggct ggaggaggag 1320
ggttcccctg agcctggccc ttgggaggtt gccagcttag gcagccaggg aatcccaggc 1380
agtgatgtgg acagtgtagg tggtggcagt gagtcccggt ctttggattc tcccacttcc 1440
agcccaggtg ctggggcccg ccgacttgta aaggcttcgt ccacaggcac ggagtcctcc 1500
gatgactttg aggagcgaga ccctgatctg ggggatggga tagagaatgg agtaggcagc 1560
ccgttcagga agtggacact gtccacagct gctcaaaccc accgactgcg gcggctgcgg 1620
ggcccagcca agtgcagaga atgtgaagcc ttcatggtca gcgggacaga atgtgaagag 1680
tgctttttga cctgtcacaa gcgctgcctg gagaccctcc tcatcctttg tggacaccgg 1740
cggcttccgg cccggatgtc cctctttggg gttgacttcc tacagctccc cagagatttc 1800
cctgaggagg ttccctttgt gattaccaga tgcacagctg agatagagca ccgtgccctg 1860
ggcttgcagg gtatctatcg ggtcagcggg tctcgggtac gtgtggagcg gctgtgccag 1920
gcctttgaga atggccgagc actggtcgag ctgtccggga actctcctca cgatatcacc 1980
agcgttctca agcgatttct tcaggagctc actgatcccg tggtcccctt ccacttgtac 2040
gacgccttca tctctctggc aaagaccctg catgcagacc ctggggacga ccctgggacc 2100
cccaacccca gccctgagat tattcgctcg ctgaagaccc tcttggtgca gctgcctgac 2160
tctaactaca gtaccctgcg acacctggtg gcccatctgt tcagggtggc tgctcggttt 2220
gaagaaaaca agatgtctgc caacaatctg ggaattgtat ttgggcctac actgctgcgg 2280
ccaccagatg gacccagggc caccggtgcc agccctgtgg cctgtctgct ggactccggt 2340
caccaggctc agctggttga gttcctcatt gtgcactatg agcagatctt tggaatggat 2400
gagctccctc tggcctcgga gcctctgacc caagaccctg gcttggctcc tgcatgcctt 2460
gaatccagtc cccagcaccc agcctcactt cttgcccaag acacacagcc cctgaccata 2520
gccttggatt ccagcccaga ccccaaacac cacagtgcct tggagaagtg ccccgaggtc 2580
acacctcctg agttttggtt tttcgagaca gggtttctct gtgtagccct ggctgtcctg 2640
gaactcactc tgtagaccag gctggcctcg aactcaaaaa tctgcctgcc tctgcctccc 2700
gagtgctggg attaaaggcc tgcgccacca ccgcccagct gttctgttgt tttatctgac 2760

tttg 2764

<210> 183
<211> 3735
<212> DNA
<213> Artificial

<400> 183

```
ggggcgacac cactgggtcg gcagaggcag gaaatcgggc tggtcccggg cggcgcgcag      60
ccctgtccct ttctcagcat tgccgcgcgt tccccacccc accccctgcc cccgggcatc     120
gccatggact cagcggagac agagctaacc ccagctcctg agggcaggaa gagatacagt     180
gacatcttcc agagcctgga caacctcgag atatcattgg ggaacgtaac ttttgatccc     240
ctggctggag accctgtacg cagagaagac ctggagcctg acaaagctga cacagccaca     300
gtggtgactg aggagaacag tgaagccagt agttggcggg atctctcccc agaaggtcct     360
gcacccctca cagaggaaga actggatttg cgactcattc ggaccaaggg tggtgtagat     420
gctgccctgg agtatgctaa agcatggagc cgctatgcca aggaactgct ggcctggaca     480
gacaagagag ccaactacga gctcgagttt gctaagagta tcatgaagct cgctgaggct     540
ggcaaggtgt ccattctcca gcagagccaa atgccactcc agtacatcta caccttgttt     600
ctggagcatg acctcagcct gggagccctg gccctggaga cagtggccca acagaagaga     660
gactactacc agcctctggc ggccaaaagg atggaaattg agaagtggag gaaggaattc     720
aaggaacagt ggctgaagga gcagaagcgc atgaatgagg cagtgcaggc actgcggcgc     780
tccgagctcc agtacattca acgcagagag gacctgaggg cacgctccca ggggtcccct     840
gaggaccctc cttcccaggc atctcctgga tccaacaagc aacaggagcg cagacggcgc     900
tcccgagagg aggcacaggc caaggcgcat gaggcggagg ctctgtacca ggcctgtgtc     960
cgggaggcca attctcgcca gcaggatctg gagaccacca agcggcggat agtgtcacat    1020
gtgcgcaagc tggtgctgca aggagacgaa gtgcttaggc gggtgacgct gggtctgttt    1080
gagctgcgag gggcgcaggc agagcgagga ccccgctcct tctctgctct ggctgagtgc    1140
tgtgtgccct ttgaacctgg ccagcgctac caggagtttg ttcggacact gcagcctggg    1200
gccccaccgc cgccatctcc ggccttctgc ttccaggagt tcacagctgt ggtgcacagt    1260
ttccctcagg acacaaaaaa gaagttttcg gggcctctac ctccaaggct ggaggaggag    1320
ggttcccctg agcctggccc ttgggaggtt gccagcttag gcagccaggg aatcccaggc    1380
agtgatgtgg acagtgtagg tggtggcagt gagtcccggt ctttggattc tcccacttcc    1440
agcccaggtg ctggggcccg ccgacttgta aaggcttcgt ccacaggcac ggagtcctcc    1500
gatgactttg aggagcgaga ccctgatctg gggggatggga tagagaatgg agtaggcagc    1560
ccgttcagga agtggacact gtccacagct gctcaaaccc accgactgcg gcggctgcgg    1620
ggcccagcca agtgcagaga atgtgaagcc ttcatggtca gcgggacaga atgtgaagag    1680
tgctttttga cctgtcacaa gcgctgcctg gagaccctcc tcatcctttg tggacaccgg    1740
```

cggcttccgg cccggatgtc cctctttggg gttgacttcc tacagctccc cagagatttc 1800
cctgaggagg ttccctttgt gattaccaga tgcacagctg agatagagca ccgtgccctg 1860
ggcttgcagg gtatctatcg ggtcagcggg tctcgggtac gtgtggagcg gctgtgccag 1920
gcctttgaga atggccgagc actggtcgag ctgtccggga actctcctca cgatatcacc 1980
agcgttctca agcgatttct tcaggagggt ggctgctcgg tttgaagaaa acaagatgtc 2040
tgccaacaat ctgggaattg tatttgggcc tacactgctg cggccaccag atggacccag 2100
ggccaccggt gccagccctg tggcctgtct gctggactcc ggtcaccagg ctcagctggt 2160
tgagttcctc attgtgcact atgagcagat ctttggaatg gatgagctcc ctctggcctc 2220
ggagcctctg acccaagacc ctggcttggc tcctgcatgc cttgaatcca gtccccagca 2280
cccagcctca cttcttgccc aagacacaca gcccctgacc atagccttgg attccagccc 2340
agaccccaaa caccacagtg ccttggagaa gtgccccgag gtcacacctc ctgagcttgc 2400
aactctgcag agggaccaga gggaggagga ggtggaagac accagagatg ggcaggggga 2460
tgggtccagc cactgccctg aggacttggc cctgggagca caatcccggg gccacttcag 2520
ccgccagcca gtgaagtatt cccggggagg tgtgaggcca gttactcatc aactctccag 2580
cctggctctg gtggcctcca agctgtgtga ggagactcct gtcactgtct cagcagtgca 2640
ccgaggtagt ttgagggtac gaggtttggg ccctgctgct gcctgccctg aaggcagccc 2700
cctgcgccgg aaccctctgc ccaagcactt tgagatcacc caggagacag cccgacttct 2760
ctccaagctg aacagtgatg ctgtgtccag gaccacctgt tgtgctgacc cagagcctga 2820
ggagtctgaa gagcacctct gaccacctac catcctgagg gacccagaac tgaattgatg 2880
gtccctgacc caacctggtg accaaaccaa ttcagtgggg tgtggggaga gacggggtta 2940
ccagtgagtt ccggtaatga agattaccta cctgtgggag tcccaagaca cttggttgaa 3000
gtgtcccatc atctccctgc cagaggagat ttaaacagct aggcaacaca ggtgcaggtg 3060
tttcggggtc aagactcaat ggtcaataca gatcagtaga tccttgggga ctacccgagt 3120
ctctgacctc tgagacgggt gtcttgacta tttcagtcat aatggctcct ggccctacta 3180
cacttgcagc agtaacagac tcccaagtta gcaggtggct gcagcccagc cctgtctcct 3240
tttaatttgt ttgtttgttt gtttgtttta agctaagtct cactgtgtag ctctggctgg 3300
tctggagctc cctttgtgag ccacaaatcc tctgcctccc aagtgctggc taaatctgtg 3360
ttcttacaac ctccaaataa aaccatatcc tgctgccggc aggagggctg accgcagtgg 3420
tgcttgctgt aaagcctcac atttgttgga cctcccagag ccacttagtg gaaggagaga 3480
gcccatccct acaaattgtc tgacctgtgc acatgcacaa ataaaagaaa attttaattt 3540
aaaaaaaaaa atccgctggg cggtagtgcc acacacctct acttgatcct agcactcagg 3600
agatagaggc aggaggatct ctgagtttta tgccagccag gtgtacagag aaagttccag 3660
gacagccagg gctacacaga gaaaccctgt ttcagaaaaa caaaataaaa taagataaat 3720
cttgtctgtt cttgc 3735

<210> 184
<211> 3874
<212> DNA
<213> Artificial

<400> 184

```
ggggcgacac cactgggtcg gcagaggcag gaaatcgggc tggtcccggg cggcgcgcag     60
ccctgtccct ttctcagcat tgccgcgcgt tccccacccc accccctgcc cccgggcatc    120
gccatggact cagcggagac agagctaacc ccagctcctg agggcaggaa gagatacagt    180
gacatcttcc agagcctgga caacctcgag atatcattgg ggaacgtaac ttttgatccc    240
ctggctggag accctgtacg cagagaagac ctggagcctg acaaagctga cacagccaca    300
gtggtgactg aggagaggaa gaactggatt tgcgactcat tcggaccaag ggtggtgtag    360
atgctgccct ggagtatgct aaagcatgga gccgctatgc caaggaactg ctggcctgga    420
cagacaagag agccaactac gagctcgagt ttgctaagag tatcatgaag ctcgctgagg    480
ctggcaaggt gtccattctc cagcagagcc aaatgccact ccagtacatc tacaccttgt    540
ttctggagca tgacctcagc ctgggagccc tggccctgga gacagtggcc caacagaaga    600
gagactacta ccagcctctg gcggccaaaa ggatggaaat tgagaagtgg aggaaggaat    660
tcaaggaaca gtggctgaag gagcagaagc gcatgaatga ggcagtgcag gcactgcggc    720
gctccgagct ccagtacatt caacgcagag aggacctgag ggcacgctcc caggggtccc    780
ctgaggaccc tccttcccag gcatctcctg gatccaacaa gcaacaggag cgcagacggc    840
gctcccgaga ggaggcacag gccaaggcgc atgaggcgga ggctctgtac caggcctgtg    900
tccgggaggc caattctcgc cagcaggatc tggagaccac caagcggcgg atagtgtcac    960
atgtgcgcaa gctggtgctg caaggagacg aagtgcttag gcgggtgacg ctgggtctgt   1020
ttgagctgcg aggggcgcag gcagagcgag gaccccgctc cttctctgct ctggctgagt   1080
gctgtgtgcc ctttgaacct ggccagcgct accaggagtt tgttcggaca ctgcagcctg   1140
gggccccacc gccgccatct ccggccttct gcttccagga gttcacagct gtggtgcaca   1200
gtttccctca ggacacaaaa aagaagtttt cggggcctct acctccaagg ctggaggagg   1260
agggttcccc tgagcctggc ccttgggagg ttgccagctt aggcagccag ggaatcccag   1320
gcagtgatgt ggacagtgta ggtggtggca gtgagtcccg gtctttggat tctcccactt   1380
ccagcccagg tgctggggcc cgccgacttg taaaggcttc gtccacaggc acggagtcct   1440
ccgatgactt tgaggagcga gaccctgatc tgggggatgg gatagagaat ggagtaggca   1500
gcccgttcag gaagtggaca ctgtccacag ctgctcaaac ccaccgactg cggcggctgc   1560
ggggcccagc caagtgcaga gaatgtgaag ccttcatggt cagcgggaca gaatgtgaag   1620
agtgcttttt gacctgtcac aagcgctgcc tggagaccct cctcatcctt tgtggacacc   1680
ggcggcttcc ggcccggatg tccctctttg ggggttgactt cctacagctc cccagagatt   1740
tccctgagga ggttcccttt gtgattacca gatgcacagc tgagatagag caccgtgccc   1800
```

tgggcttgca gggtatctat cgggtcagcg ggtctcgggt acgtgtggag cggctgtgcc 1860
aggcctttga gaatggccga gcactggtcg agctgtccgg gaactctcct cacgatatca 1920
ccagcgttct caagcgattt cttcaggagc tcactgatcc cgtggtcccc ttccacttgt 1980
acgacgcctt catctctctg gcaaagaccc tgcatgcaga ccctggggac gaccctggga 2040
cccccaaccc cagccctgag attattcgct cgctgaagac cctcttggtg cagctgcctg 2100
actctaacta cagtaccctg cgacacctgg tggcccatct gttcagggtg gctgctcggt 2160
ttgaagaaaa caagatgtct gccaacaatc tgggaattgt atttgggcct acactgctgc 2220
ggccaccaga tggacccagg gccaccggtg ccagccctgt ggcctgtctg ctggactccg 2280
gtcaccaggc tcagctggtt gagttcctca ttgtgcacta tgagcagatc tttggaatgg 2340
atgagctccc tctggcctcg gagcctctga cccaagaccc tggcttggct cctgcatgcc 2400
ttgaatccag tccccagcac ccagcctcac ttcttgccca agacacacag cccctgacca 2460
tagccttgga ttccagccca gaccccaaac accacagtgc cttggagaag tgccccgagg 2520
tcacacctcc tgagcttgca actctgcaga gggaccagag ggaggaggag gtggaagaca 2580
ccagagatgg ggcaggggat gggtccagcc actgccctga ggacttggcc ctgggagcac 2640
aatcccgggg ccacttcagc cgccagccag tgaagtattc ccggggaggt gtgaggccag 2700
ttactcatca actctccagc ctggctctgg tggcctccaa gctgtgtgag gagactcctg 2760
tcactgtctc agcagtgcac cgaggtagtt tgagggtacg aggtttgggc cctgctgctg 2820
cctgccctga aggcagcccc ctgcgccgga accctctgcc caagcacttt gagatcaccc 2880
aggagacagc ccgacttctc tccaagctga acagtgatgc tgtgtccagg accacctgtt 2940
gtgctgaccc agagcctgag gagtctgaag agcacctctg accacctacc atcctgaggg 3000
acccagaact gaattgatgg tccctgaccc aacctggtga ccaaaccaat tcagtggggt 3060
gtggggagag acggggttac cagtgagttc cggtaatgaa gattacctac ctgtgggagt 3120
cccaagacac ttggttgaag tgtcccatca tctccctgcc agaggagatt taaacagcta 3180
ggcaacacag gtgcaggtgt ttcggggtca agactcaatg gtcaatacag atcagtagat 3240
ccttggggac tacccgagtc tctgacctct gagacgggtg tcttgactat ttcagtcata 3300
atggctcctg gccctactac acttgcagca gtaacagact cccaagttag caggtggctg 3360
cagcccagcc ctgtctcctt ttaatttgtt tgtttgtttg tttgttttaa gctaagtctc 3420
actgtgtagc tctggctggt ctggagctcc ctttgtgagc cacaaatcct ctgcctccca 3480
agtgctggct aaatctgtgt tcttacaacc tccaaataaa accatatcct gctgccggca 3540
ggagggctga ccgcagtggt gcttgctgta aagcctcaca tttgttggac ctcccagagc 3600
cacttagtgg aaggagagag cccatcccta caaattgtct gacctgtgca catgcacaaa 3660
taaaagaaaa ttttaattta aaaaaaaaaa tccgctgggc ggtagtgcca cacacctcta 3720
cttgatccta gcactcagga gatagaggca ggaggatctc tgagttttat gccagccagg 3780
tgtacagaga aagttccagg acagccaggg ctacacagag aaaccctgtt tcagaaaaac 3840

aaaataaaat aagataaatc ttgtctgttc ttgc 3874

<210> 185
<211> 845
<212> DNA
<213> Artificial

<400> 185

agcagttaaa gagcactagc tttgctttca gaggactcag atttggttct tagcatccac 60
atggtggttc acagtcatca gtgactctag tcccagggga ttaaataccc tttcagacct 120
ccatgggcac tgactgcagg cacatggtga aggaaaaaca cttccataca taaaaataat 180
tactacaatt taagctaaaa gggcaccaaa cactttatag atcttgatga gaggctatat 240
caaattttat acattgtgac accggttttt tatagatttt ctagcatata ttttagatct 300
atttatcatc tatctatcta tctatctatc tatctttcta tcatctatct tatataaaat 360
aaagtctgac ctaaatatga gaccctgtat ggttgtctaa aaacatactg ttagctcctt 420
cagtcacacc tgtttggggc tggctggtga atcagaagga accttgttgg gtctgctgct 480
caccacctat ggggctgtgg gcatggtggc agcagcagtt gttgccactt ctgctttggc 540
caagcacagt aggtgatcct gatagtcagc agcacagatt cccattgatt ctagtctatg 600
tatttgctta tggttggtac aatatttaat gtatataggc agaagtatta tagggtcttt 660
aaatatgtga acatatgtag cactgctttt aaacttaaaa cggatatgat atttttcata 720
tgtcaagaca cactggtaaa tttctgttac atattgtttt caagttttct gttagtcttg 780
tttctatgag gtacttttat tgtaacttaa aattcttaga aattaaagtt ctgagaaaaa 840
cctta 845

<210> 186
<211> 1871
<212> DNA
<213> Artificial

<400> 186

gtcttcccca gaggccctaa ctacagccac taccctttca gggcccaggc tgaccgtgga 60
tggggctcct ggcaccttca tcctcctcct ccatcttcta tctgagcctg agacgaactg 120
cccacccccct ccagccacct gccgcctcct gtttggaagg aggcccgcag cagatttcct 180
cttccctcca ttcccaagag gaacccctgg tctggggata ctgaggcagg cacagggcag 240
ggcacgtgtg agcatccagt catcctgaaa ggtaaatctg gttcctgaag cgacaactgt 300
tcgtacaggg ttttgaagga ttggtaggag ttccacagag gacaccgaag aggtgcagtc 360
cctggtttgt ccttatgtcc cagcccattt gggcacagct gtaatgtcta ctcctgggtc 420
actgaggcat aggggtgact tgcctgaact tgccttttct aggttttcct tggcttcacc 480
tttgatgggg gtggagggag gagggaggga aagaggagga aagagagagg gagagagtcc 540
tggtcccaag aggaaggaag aagccacttg aacttcagtc tggactccat cacatgttct 600

gtggggacgg tcagccctca ctcaagtccc agacccggcg gtttccctcc ctcccacccc 660
ctccccctgc ggagccctgg gattcccgcc cgaattagcc tggtcacccg agttggcatc 720
gcacacgggg gcggagcgga cagagtccag tgggtataaa ggcagctgca gagggcttaa 780
ctcaaagaca cactcaggac acaagcgaca tggccccgcc cgcgctccag gcccagcctc 840
caggcggctc tcaactgagg ttcctgctgt tcctgctgct gttgctgctg ctgctgtcat 900
ggccatcgca gggggacgcc ctggcaatgc ctgaacagcg accctccggc cctgagtccc 960
aactcaacgc cgacgagcta cggggtcgct tccaggacct gctgagccgg ctgcatgcca 1020
accagagccg agaggactcg aactcagaac caagtcctga cccagctgtc cggatactca 1080
gtccagaggt gagattgggg tcccacggcc agctgctact ccgcgtcaac cgggcgtcgc 1140
tgagtcaggg tctccccgaa gcctaccgcg tgcaccgagc gctgctcctg ctgacgccga 1200
cggcccgccc ctgggacatc actaggcccc tgaagcgtgc gctcagcctc cagggacccc 1260
gtgctcccgc attacgcctg cgcctgacgc cgcctccgga cctggctatg ctgccctctg 1320
gcggcacaca gctggaactg cgcttacggg tagccgccgg caggggggcgc cgaagcgcgc 1380
atgcgcaccc aagagactcg tgcccactgg gtccggggcg ctgctgtcac ctggagactg 1440
tgcaggcaac tcttgaagac ttgggctgga gcgactgggt gctgtccccg cgccagctgc 1500
agctgagcat gtgcgtgggc gagtgtcccc acctgtatcg ctccgcgaac acgcatgcgc 1560
agatcaaagc acgcctgcat ggcctgcagc ctgacaaggt gcctgccccg tgctgtgtcc 1620
cctccagcta caccccggtg gttcttatgc acaggacaga cagtggtgtg tcactgcaga 1680
cttatgatga cctggtggcc cggggctgcc actgcgcttg agcaccgggc cctgctcctc 1740
acctacactc cccttcaagg atgctattta tatttgtatt tattaatatt attaatttat 1800
tggggtcggg ctgggtggat ggattgtgta tttatttaaa actctgctaa taaaggtgag 1860
cttggtttct a 1871

<210> 187
<211> 1916
<212> DNA
<213> Artificial

<400> 187

```
tgcagcagcc gagaggtgtg agccgccgcg gtgtcagagt ctaggggaat tggagtcagg     60
cgcagatcca cagcgatatc cagacattca gagccacagg caccatgtcc aatcctggtg    120
atgtccgacc tgttccgcac aggagcaaag tgtgccgttg tctcttcggt cccgtggaca    180
gtgagcagtt gcgccgtgat tgcgatgcgc tcatggcggg ctgtctccag gaggcccgag    240
aacggtggaa ctttgacttc gtcacggaga cgccgctgga gggcaacttc gtctgggagc    300
gcgttcggag cctagggctg cccaaggtct acctgagccc tgggtcccgc agccgtgacg    360
acctgggagg ggacaagagg cccagtactt cctctgccct gctgcagggg ccagctccgg    420
aggaccacgt ggccttgtcg ctgtcttgca ctctggtgtc tgagcggcct gaagattccc    480
```

cgggtgggcc cggaacatct cagggccgaa aacggaggca gaccagcctg acagatttct 540
atcactccaa gcgcagattg gtcttctgca agagaaaacc ctgaagtgcc cacgggagcc 600
ccgccctctt ctgctgtggg tcaggaggcc tcttccccat cttcggcctt agccctcact 660
ctgtgtgtct taattattat ttgtgtttta atttaaacgt ctcctgtata tacgctgcct 720
gccctctccc agtctccaaa cttaaagtta tttaaaaaaa gaacaaaaca aaacaaaaaa 780
aaccaaaaca aaacaaacct aaattagtag gacggtaggg cccttagtgt gggggatttc 840
tattatgtag attattatta tttaagcccc tcccaaccca agctctgtgt ttcctatacc 900
ggaggaacag tcctactgat atcaacccat ctgcatccgt ttcacccaac ccccctcccc 960
ccattccctg cctggttcct tgccacttct tacctggggg tgatcctcag acctgaatag 1020
cactttggaa aaatgagtag gactttgggg tctccttgtc acctctaagg ccagctagga 1080
tgacagtgaa gcagtcacag cctagaacag ggatggcagt taggactcaa ccgtaatatc 1140
ccgactcttg acattgctca gacctgtgaa gacaggaatg gtccccactc tggatcccct 1200
ttgccactcc tggggagccc acctctcctg tgggtctctg ccagctgccc ctctattttg 1260
gagggttaat ctggtgatct gctgctcttt tcccccaccc catacttccc cttctgcagg 1320
tcggcaggag gcatatctag gcacttgccc cacagctcag tggactggaa gggaatgtat 1380
atgcagggta cactaagtgg gattccctgg tcttacctta ggcagctcca gtggcaaccc 1440
cctgcattgt gggtctaggg tgggtccttg gtggtgagac aggcctccca gagcattcta 1500
tggtgtgtgg tggtgggggt gggcttatct gggatgggga ccccagttgg ggttctcagt 1560
gacttctccc atttcttagt agcagttgta caaggagcca ggccaagatg gtgtcttggg 1620
ggctaaggga gctcacagga cactgagcaa tggctgatcc tttctcagtg ttgaataccg 1680
tgggtgtcaa agcacttagt gggtctgact ccagccccaa acatccctgt ttctgtaaca 1740
tcctggtctg gactgtctac ccttagcccg caccccaaga acatgtattg tggctccctc 1800
cctgtctcca ctcagattgt aagcgtctca cgagaaggga cagcaccctg cattgtcccg 1860
agtcctcaca cccgacccca aagctggtgc tcaataaata cttctcgatg atttat 1916

<210> 188
<211> 3512
<212> DNA
<213> Artificial

<400> 188

```
agagaccatc catcaaaaga accacaccaa tcagcgacgt agatgcgtcc cagctagaga    60
gccaggtctc cagcttgttc ccccactgcc cggcgcagtc cttcgctgcg cgctgacccc   120
gaggaaggca gtacttggtc agggcgctga gggaccctcc accgggacgc cggcccctcc   180
ccgggcctct gctcacttga cccccactcc ctagtccgtc cccttagtag gcctgtcgga   240
tcggggacgt ggggcgagct gagagcaggc ccggggtggg tggtcaccgt ggtgaagacg   300
tggctgtcaa gatgatagaa gtactgacaa ctgactctca gaaactgcta caccagctga   360
```

acaccctgtt ggaacaggag tctagatgtc agccaaaggt ctgcggcttg aaactaattg 420
agtcggccca tgataatggc ctcagaatga ctgcaagatt acgggacttt gaagtaaaag 480
atctacttag tctaactcag ttctttggct ttgacacgga gacattttcc ctagctgtga 540
atttactgga cagattcttg tctaaaatga aggtacaggc gaagcatctt gggtgtgttg 600
gactgagctg cttttatttg gctgtgaaag cgactgaaga ggaaaggaat gtcccactgg 660
cgactgattt gatccgaata agtcagtata ggttcacggt ttcagacctg atgagaatgg 720
agaagattgt gttggagaaa gtgtgttgga aagtcaaagc tactactgcc tttcaatttc 780
tgcagctcta ttattcactc gttcacgaca ccttgccatt tgagaggaga aacgatctga 840
attttgaaag actagaagcc caacttaagg cctgccactg caggatcata ttttctaagg 900
caaagccttc tgtgctggcg ctatctatcc ttgcgttgga gatccaagca ctgaaatacg 960
tagagttaac agaaggagta gaatgtattc agaaacattc caagataagt ggccgagatt 1020
tgaccttctg gcaagagctt gtttccaagt gtttaactga atattcatca aacaagtgct 1080
ccaaacctaa cggtcagaag ttgaaatgga ttgtgtctgg acgcactgca cggcaactga 1140
agcacagtta ttatagaata actcacctcc caacgattcc tgagaccatt tgttagttga 1200
taaatctggt tgttattctc tgtatacaga aaattttcca gtatgatcat tttctgctac 1260
aactgaagaa ttgaaatact atcttcaata taaagaatat gggatgaaaa cataaaggaa 1320
aagtgaattg ttgactggtc tagatagaga atactggaag gcattcactg tgtacagtgc 1380
gtagcagttt taagagaaaa gacatatcaa acccctagat atacgctaat acttttcatc 1440
aaaagattag cgtagtagca aagagaatac tttaaactcg aattttaaaa gtagttactg 1500
aaatagcact tctttaaatt acgtaccacc ccactgtagc ttatttaaag ttgcataagc 1560
ccatgcagaa caacaagcaa tgtgacccat atatgaacaa attttaatct gcccatcgac 1620
tatgaaaatg aagtacaaac ctgggtgatg gacttacaaa gtaatatagg gcatgcccat 1680
gttaggtttc tggaaactgc cagagtgtct taattctata gctagtattt tacctctata 1740
gcatttggac taatacaaag taattatatg catgaaaata taaaattggt ctctgataca 1800
tacacatttt tgacatctaa atttcttaat catagcaaag atttatcttt ttatgattaa 1860
aaacaatttt ttccttaatg catggcagca catgccttta atcccagcac tagagaggca 1920
gagacaggta gatctctgag ttcaaagcca ggctggtcta cacagtgagt tccaagacag 1980
ctagagacct tgtctcaaaa acactcaaaa caaccaaaca ccaggggtaa atgttgctgg 2040
gaagtcaggg aagatggttt agaagggaag cctgacaacc tgacttcatt cttcaggacc 2100
cacatgatgg aaggagagaa cgaagaactc ccaagttctc tcacatatgc acatacctca 2160
ccaccccccg caggaaatac atgatcatgc gtctgagata tcaccagttc acctttagca 2220
gctcgcagtt tgtaggcaga tttctgttaa gttgggtctg tgttgtttgc ctatgtagca 2280
ggattacagc agcagcaaaa acggtccctc aagtctttct gccactctga cctgagtttc 2340
ctacggtaca ggatttactc tgagaaacct cagcaccttg ccacagtagc cttggcagaa 2400

tggcctcacg gttagggaaa ctcctgattc taagcttggg agagctacgc ttagatttga 2460
attcacccag gaagcattca aatcaaggct aaagacataa atgtgaaata aaactgtgaa 2520
ccttcattct aaagttcatc tgacttccca gatttgatca atatattctt aggtggtatt 2580
aaaaatggta aactgcttaa tttaaatctc aaaatttaaa ttatgaggtt tacataaaaa 2640
ccaacatttc atgaatgcac ttttaaggta ttaaaagggg tacttaagcg gtaaatggtt 2700
tcttggcacc cataaccaag taatagttaa tttacaggtg ggattttttt ttattgctat 2760
gagaattaca tttaaaattg tgggtgtttt atataaagca gatatcacaa gttttgaaaa 2820
tttgttacct ttaatatttc ttctagagaa taggtgtttg tatccataat aaaagaaaaa 2880
tttgtcagaa ctgctgcttc aatctaatcc catttgagag aattgtcttt actgtcttaa 2940
taactggatg aattatcact ctgaaaatgt atttattgca ctaaagttag tttaggcttg 3000
ataaaacact ccagacattt ttactacaga ctgtttctat aaaactgcca ttgcttctaa 3060
tggagaattt tattttaaaa gaaataaaat tgctgagttt atctgcaaaa cctttctcta 3120
agtcttatgg gacgtaagga gacacctcca taattataag agccgttgtg ctcagagtct 3180
ccatgctggc ttgaatgtat gatccacact gttaaaacat aggcagcagc tcagggccct 3240
ggacagcctg agtcaaccta gagtagctgg aaccattttg acatgtaatg gataagaaaa 3300
ttatccattg agaagctgaa caataaacca aagaacgggt gtattttatc cttaacctct 3360
gtaaaccacg ttacactgag aacacttcag ttcttcctaa aggttgatag gcttcagtct 3420
gaaaacaata ttgatttgga gtggacagaa gttaactaac caactaccat tatgttttga 3480
atacaccttt caataaaatg attgaaatgc aa 3512

<210> 189
<211> 3433
<212> DNA
<213> Artificial

<400> 189

agagaccatc catcaaaaga accacaccaa tcagcgacgt agatgcgtcc cagctagaga 60
gccaggtctc cagcttgttc ccccactgcc cggcgcagtc cttcgctgcg cgctgacccc 120
gaggaaggca gtacttggtc agggcgctga gggaccctcc accgggacgc cggcccctcc 180
ccgggcctct gctcacttga cccccactcc ctagtccgtc cccttagtag gcctgtcgga 240
tcggggacgt ggggcgagct gagagcaggc ccggggtggg tggtcaccgt ggtgaagacg 300
tggctgtcaa gatgatagaa gtactgacaa ctgactctca gaaactgcta caccagctga 360
acaccctgtt ggaacaggag tctagatgtc agccaaaggt ctgcggcttg aaactaattg 420
agtcggccca tgataatggc ctcagaatga ctgcaagatt acgggacttt gaagtaaaag 480
atctacttag tctaactcag ttctttggct ttgacacgga gacattttcc ctagctgtga 540
atttactgga cagattcttg tctaaaatga aggtacaggc gaagcatctt gggtgtgttg 600
gactgagctg cttttatttg gctgtgaaag cgactgaaga ggaaaggaat gtcccactgg 660

cgactgattt gatccgaata agtcagtata ggttcacggt ttcagacctg atgagaatgg 720
agaagattgt gttggagaaa gtgtgttgga aagtcaaagc tactactgcc tttcaatttc 780
tgcagctcta ttattcactc gttcacgaca ccttgccatt tgagagcctt ctgtgctggc 840
gctatctatc cttgcgttgg agatccaagc actgaaatac gtagagttaa cagaaggagt 900
agaatgtatt cagaaacatt ccaagataag tggccgagat ttgaccttct ggcaagagct 960
tgtttccaag tgtttaactg aatattcatc aaacaagtgc tccaaaccta acggtcagaa 1020
gttgaaatgg attgtgtctg gacgcactgc acggcaactg aagcacagtt attatagaat 1080
aactcacctc ccaacgattc ctgagaccat ttgttagttg ataaatctgg ttgttattct 1140
ctgtatacag aaaattttcc agtatgatca ttttctgcta caactgaaga attgaaatac 1200
tatcttcaat ataaagaata tgggatgaaa acataaagga aaagtgaatt gttgactggt 1260
ctagatagag aatactggaa ggcattcact gtgtacagtg cgtagcagtt ttaagagaaa 1320
agacatatca aaccccctaga tatacgctaa tacttttcat caaaagatta gcgtagtagc 1380
aaagagaata ctttaaactc gaattttaaa agtagttact gaaatagcac ttctttaaat 1440
tacgtaccac cccactgtag cttatttaaa gttgcataag cccatgcaga acaacaagca 1500
atgtgaccca tatatgaaca aattttaatc tgcccatcga ctatgaaaat gaagtacaaa 1560
cctgggtgat ggacttacaa agtaatatag ggcatgccca tgttaggttt ctggaaactg 1620
ccagagtgtc ttaattctat agctagtatt ttacctctat agcatttgga ctaatacaaa 1680
gtaattatat gcatgaaaat ataaaattgg tctctgatac atacacattt ttgacatcta 1740
aatttcttaa tcatagcaaa gatttatctt tttatgatta aaaacaattt tttccttaat 1800
gcatggcagc acatgccttt aatcccagca ctagagaggc agagacaggt agatctctga 1860
gttcaaagcc aggctggtct acacagtgag ttccaagaca gctagagacc ttgtctcaaa 1920
aacactcaaa acaaccaaac accaggggta aatgttgctg ggaagtcagg gaagatggtt 1980
tagaagggaa gcctgacaac ctgacttcat tcttcaggac ccacatgatg gaaggagaga 2040
acgaagaact cccaagttct ctcacatatg cacatacctc accacccccc gcaggaaata 2100
catgatcatg cgtctgagat atcaccagtt cacctttagc agctcgcagt ttgtaggcag 2160
atttctgtta agttgggtct gtgttgtttg cctatgtagc aggattacag cagcagcaaa 2220
aacggtccct caagtctttc tgccactctg acctgagttt cctacggtac aggatttact 2280
ctgagaaacc tcagcacctt gccacagtag ccttggcaga atggcctcac ggttagggaa 2340
actcctgatt ctaagcttgg gagagctacg cttagatttg aattcaccca ggaagcattc 2400
aaatcaaggc taaagacata aatgtgaaat aaaactgtga accttcattc taaagttcat 2460
ctgacttccc agatttgatc aatatattct taggtggtat taaaaatggt aaactgctta 2520
atttaaatct caaaatttaa attatgaggt ttacataaaa accaacattt catgaatgca 2580
cttttaaggt attaaaaggg gtacttaagc ggtaaatggt ttcttggcac ccataaccaa 2640
gtaatagtta atttacaggt gggatttttt tttattgcta tgagaattac atttaaaatt 2700

gtgggtgttt tatataaagc agatatcaca agttttgaaa atttgttacc tttaatattt 2760
cttctagaga ataggtgttt gtatccataa taaaagaaaa atttgtcaga actgctgctt 2820
caatctaatc ccatttgaga gaattgtctt tactgtctta ataactggat gaattatcac 2880
tctgaaaatg tatttattgc actaaagtta gtttaggctt gataaaacac tccagacatt 2940
tttactacag actgtttcta taaaactgcc attgcttcta atggagaatt ttattttaaa 3000
agaaataaaa ttgctgagtt tatctgcaaa acctttctct aagtcttatg ggacgtaagg 3060
agacacctcc ataattataa gagccgttgt gctcagagtc tccatgctgg cttgaatgta 3120
tgatccacac tgttaaaaca taggcagcag ctcagggccc tggacagcct gagtcaacct 3180
agagtagctg gaaccatttt gacatgtaat ggataagaaa attatccatt gagaagctga 3240
acaataaacc aaagaacggg tgtattttat ccttaacctc tgtaaaccac gttacactga 3300
gaacacttca gttcttccta aaggttgata ggcttcagtc tgaaaacaat attgatttgg 3360
agtggacaga agttaactaa ccaactacca ttatgttttg aatacacctt tcaataaaat 3420
gattgaaatg caa 3433

<210> 190
<211> 4172
<212> DNA
<213> Artificial

<400> 190

agccctgctt ctccttctaa caacaccttt cccaccaccg tgtcaggctc catctgtggt 60
gtgggtgctc tacctctctg gtcctcagct tgggggtggg gtggaagtgg ggggagcctc 120
aggccaccaa cttcagacgg aagtcaggtg gggggcaggg tgaggagcca acgccagagg 180
agcctcagga ggagagggaa gcagaggggc ccaacaacga tggtgtcacc ctgaagactg 240
gacaactgtg cagactgtgg agggctgcga gaccacggct ccagtatata aatcaggcaa 300
attccccatt tgagcatgaa cttctgaaaa cggcctgcat ctaaggtctc ctccaaggcc 360
ctctggagtc cagcccataa tgaaggtctt ggccgcaggg attgtgccct tactgctgct 420
ggttctgcac tggaaacacg gggcagggag ccctcttccc atcacccctg taaatgccac 480
ctgtgccata cgccacccat gccacggcaa cctcatgaac cagatcaaga atcaactggc 540
acagctcaat ggcagcgcca atgctctctt catttcctat tacacagctc aaggggagcc 600
gtttcccaac aacgtggaaa agctatgtgc gcctaacatg acagacttcc catctttcca 660
tggcaacggg acagagaaga ccaagttggt ggagctgtat cggatggtcg catacctgag 720
cgcctccctg accaatatca cccgggacca gaaggtcctg aaccccactg ccgtgagcct 780
ccaggtcaag ctcaatgcta ctatagacgt catgaggggc ctcctcagca atgtgctttg 840
ccgtctgtgc aacaagtacc gtgtgggcca cgtggatgtg ccacctgtcc ccgaccactc 900
tgacaaagaa gccttccaaa ggaaaaagtt gggttgccag cttctgggga catacaagca 960
agtcataagt gtggtggtcc aggccttcta gagaggaggt cttgaatgta ccatggactg 1020

agggacctca ggagcaggat ccggaggtgg ggagggggct caaaatgtgc tggggtttgg 1080
gacattgtta aatgcaaaac ggggctgctg gcagacccca gggatttcca ggtactcact 1140
gcactctggg ctgggccatg atggaatctg gcaaagttga aacttccata ggcagagctt 1200
ctatacagcc cagcaccagc tagaaatggc aatgagggtg ttggtctgag agatttctgt 1260
ctcactcact cactcactca ctctcactca ctcactcact cactcactca gccccttgct 1320
tgctgggtgt atgaacaagc tgcacaagtt gtctacagca gacagcaaag ggctgggaag 1380
tgtcctagac ccctacagag tcaccatcat ctggtccttt gctgtctctc agagaaactt 1440
tggaaggctt ggttgggatg tgagagagct aaggggactg ggatccagaa ggaatccttt 1500
tattttattt tattttattt tattttattt tattttataa gttttgtggg tggaagggta 1560
ccctggggtg gaatgatgga atgtgtcttc tcttgagttg gatgagagag ttcaggctta 1620
gagactgtca gatggaagag tctaggtcac cagtgttcag gctcccacag aagcacagcg 1680
gccagcttcc agttgtcaaa gcctgacgaa ctcggttagc ttctatgcag ttccccccac 1740
agcctggcgt ggttggggtc tgccagctgg acctagaggt gaggtgtgtg caggcaggaa 1800
gaggcaggct gcaaaggcag gttcccagag tcctcccggg gaaggacctc taactgtcta 1860
ggagtcaggg aaggagcaag gcagccagcc attgctgagg cagtagccga ctgcagctct 1920
catctgcttc tcaacccctg agaacaggtg atcttgagca gacagacagg tagcataaag 1980
tagaatgtcg ggtctgaggc cccggaggtc gcaaaggtac ttgaagggga ccagagggct 2040
gtcttgggtc cctggagcat ggagaagcag aacttgaggt cagggtctca gggaagatga 2100
ggcccagagt gctgtgtttg atccagcaca gctgtctatt tattactatg tcctatttat 2160
attaacttat tggtgcttta aatggcaaag ttaattcccc gaaatggtat gaggctcctt 2220
ccatgggagc tggggccgag actctccacc tagtggggcc tggtctggag gcacatgatt 2280
gttacaggtg cagctcatgg gtcaaatcag agagctggct agctcctctg tctcccactg 2340
tgactcactt ttagggtgtc agggtccccc agaaaaagct gggccagttt gtctctctgc 2400
ttctgtctct gtctctccga gtctgtctct gtctgtctct gtgtctctgt ctctttgtct 2460
ctctctgtct ctgtctctct ctctctctcc ccgccccccc cccttctccc tctggtctcc 2520
aagggggtgg aacagtttct tgttgttttg tcccactgag ctctctggca ccccctagat 2580
tcctgctatg cggtgcacca ttcataatga agtgaatggc tctggaacct tgggcaaaac 2640
tgattccttc ctcaaatcgt agctgaggag tgctgaaaca tcctgacccg gcacccagcg 2700
tgctttcgac cagcatggaa gctcctcggg tggcccgaac acccacagag ggtgaataca 2760
ggaggttgga gcagtgcagg ccctgaactg ggcctgaaca gctgcccagt gcgccagaga 2820
aggggagatc aaggcccgag acgcctggga cacagaccag gaagctgtgg tccttgcttc 2880
atcgctgcct tcccactccc gcccatgtct gggctcccag gcagggaatc cgatctgatc 2940
tctcctttgt gctgaggcca ggcaagcaga ggaacgccct cgatctggga gcagggtagg 3000
gaggaaggca gccaagctgg ggcagtggct gactacagag ctagctgcct gcctctcagg 3060

ctctgaacag ggcggtcctt agcagttcag cagtgggatt ctgcttcacg cggttttgca 3120
cctttctctg tcactctcta agcactttac ctggacggca ggtggacagg ccctggagct 3180
ctggcttagg aaaggcctgg aaccatagat gcagcaagga gactatggtg ggggccacgc 3240
gtgtcagcga caaagttact ccaccgtact cctgttgctg cgtcaggctc atctcaggac 3300
tggctgccct tctccaagct gagagtcaat ttgtctaaaa gccaagatga tgccacagcc 3360
tggggcctgt tgggctttgt catcacttca catttgtatg gacttggact ctctgggctc 3420
cgcccacctg gcagctttga aggctcaggg accaatggac tctctccgtg cacgcccccg 3480
tccccccaac gcaaccacct acctgcgtct tactccatca gttgcccagc atcccagaac 3540
cattgagcct ttggggaaaa cagactttag gggcaggtag ttgctcacct gacatctttc 3600
acctggaagc attgacttcc accgagcata gtaggtagtg tgtctggacc agagaaaaag 3660
ggatggggca ttttgcagtt tatccagaga gaagcaaagg ggcctttatt tattatttaa 3720
aacttcaaac ctgaaagcac tgagagttta ctggtctgcc cccctccccc cactcttgtc 3780
tatttctgtg tccttgatcc cgactcaagc aacccagctc tgctttgcct gctctctgga 3840
gcagacatgg tatgtgggcc aggaccccgg agtcttgcat ggtagcggct tcagaaggga 3900
aatgatatgg ctgtctgcat tcggatgact ccccagtccc agcccagcct ctcctttgca 3960
ctgctgctct ccctctttcc tttcctttgg aagggacttg gccttgggtg acaaattcct 4020
ctttgatgaa tgtaccctgt gggaatgttt catactgaca gattattttt atttattcaa 4080
tgtcatattt aaaatattta ttttttatac tgaaggagtg tctttttttt tttaaagaaa 4140
aaatgaaata ataaagaact cattcttgtt ga 4172

<210> 191
<211> 2111
<212> DNA
<213> Artificial

<400> 191

ggtggggctg agggagggaa acaaacaaaa tcccgctgcg gggacaggag acggaaaagc 60
aggctggaag ggtagacaca attctcgcga gaagtagagc gaattccctc ggagaggggc 120
ggttgcgcag aggtacacca cgtgtgcggt ccctccggcg ccgagtcagg gtctctcgtc 180
gagtctgggt ctaggttaga tttggaatcg tggagatgcg gaaggacacc cctcctccgc 240
tcgtgccccc ggcggcccgc gagtggaacc tgccccccaa tgcgcccgca tgcatggaac 300
gtcaattgga ggctgcacgg taccggtctg atggttccct tctgctcggg gtctccagcc 360
tgagtggtcg ctgctgggta ggttctctgt ggtttttcaa ggatcctagt gcggccccca 420
acgaaggttt ctgctctgct ggcgtccaga ccgaggctgg agtagctgac ctcacttggg 480
tgggggacaa aggtatccta gtggcttctg attcaggtgc tgttgaattg tgggagctag 540
atgagaacga gacacttata gtcagcaagt tctgcaagta tgagcatgat gacattgtgt 600
ctactgtcac tgtcctgagc tctggcacac aagctgtcag tggtagcaaa gactgctgca 660

tcaaaatttg ggacctggct cagcaggtat cactgaattc ataccgagct cacgctggac 720
aggttacctg tgttgctgcc tctccccaca aagactctgt gtttctttca tgtagtgagg 780
acagtagaat tttgctctgg gatacccgct gtcccaagcc ggcatcacag atggcctgca 840
atgcctctgg ctacctccct accgctttgg cttggcatcc tcagcagagt gaagtctttg 900
tttttggtga cgagaatgga tctgtctccc ttgtggacac caagaatgca agctgtaccc 960
tcagctcagc tgtgcactcc cagggtgtca ctagactggt attctcccca cacagtgtcc 1020
ccctcctgac ttctctcagt gaagactgtt cacttgctgt gctggattca agcctttctg 1080
aggtgtttag aagtcgagcc cacagagact ttgtgagaga tgctacgtgg tctccactca 1140
atcactccct tcttaccaca gttggctggg accatcaggt catccaccat gttgtgccct 1200
tagagcctct cccaaaccct ggacctgaca gtgttgtgga gtagaatgga tttcagaaaa 1260
accaaaacaa gccctccgtc tgtaagcgac tactcgattg cccctgcctt ccatgtgtga 1320
gagcacagga gccttgtaga gcatgtttcc tccctagccc cgtgcagtaa caggcagatt 1380
ctcagcctga gggaggctgc atcccatagt gactcagagg aagaaacttc ctctgtaaat 1440
ggatgtatgt gagtacacgt gtgagtgcgg tatatagttt ggagtggaga aaattattct 1500
tcagctttcc caaagcaatg ctctttaccc ctgacaaagt gaccagagga ttttaatgct 1560
tcccatgtct gggaattggc gatgttaggg atatggaatg tgggtatccc tagatttttg 1620
ggaatacttc atactactca gaggtgcgta acttattttt atatagagtt taattcacta 1680
ttatgggaat tacttccata tacaaaagtc tagcccactg taccttgaga agacaaaaca 1740
gttttgtaca agtccctgtt atactgagtc aaatccattt tctgggtgaa taaatttggc 1800
cctgcagtgt agctcttgtt cacccttcc ttccttggtg ttgtgtgtga gagatgagtg 1860
ttttcctgtc ctagatttca cagataagtt gtagggatga tggaccactt aaaagtgttt 1920
cagtgaagtg ggaaaattga catggattgt tacagagaaa tctgtcattt ttcccaccta 1980
aaaattgatc tgggtacctt cctctggaga gctgaagtct gaatgtattt ttggactcct 2040
aagatttgag atccagcatc agggaaactg tcggaaatac taaaaggtga aataaagact 2100
tttatccttg a 2111

<210> 192
<211> 2594
<212> DNA
<213> Artificial

<400> 192

ggtggggctg agggagggaa acaaacaaaa tcccgctgcg gggacaggag acggaaaagc 60
aggctggaag ggtagacaca attctcgcga gaagtagagc gaattccctc ggagaggggc 120
ggttgcgcag aggtacacca cgtgtgcggt ccctccggcg ccgagtcagg gtctctcgtc 180
gagtctgggt ctaggttaga tttggaatcg tggagatgcg gaaggacacc cctcctccgc 240
tcgtgccccc ggcggcccgc gagtggaacc tgccccccaa tgcgcccgca tgcatggaac 300

gtcaattgga ggctgcacgg taccggtctg atggttccct tctgctcggg gtctccagcc 360
tgagtggtcg ctgctgggta ggttctctgt ggtttttcaa ggatcctagt gcggccccca 420
acgaaggttt ctgctctgct ggcgtccaga ccgaggctgg agtagctgac ctcacttggg 480
tgggggacaa aggtatccta gtggcttctg attcaggtgc tgttgaattg tgggagctag 540
atgagaacga gacacttata gtcagcaagt tctgcaagta tgagcatgat gacattgtgt 600
ctactgtcac tgtcctgagc tctggcacac aagctgtcag tggtagcaaa gactgctgca 660
tcaaaatttg ggacctggct cagcaggtat cactgaattc ataccgagct cacgctggac 720
aggttacctg tgttgctgcc tctccccaca aagactctgt gtttctttca tgtagtgagg 780
acagtagaat tttgctctgg gatacccgct gtcccaagcc ggcatcacag atggcctgca 840
atgcctctgg ctacctccct accgctttgg cttggcatcc tcagcagagt gaagtctttg 900
tttttggtga cgagaatgga tctgtctccc ttgtggacac caagaatgca agctgtaccc 960
tcagctcagc tgtgcactcc cagggtgtca ctagactggt attctcccca cacaggtgct 1020
gtgtgtcacc aggaacttgg aagggatggg tggggacagt ggtgaaggag taggaatgga 1080
gtgacacagg gaggaggtat ctatttggca aagctgcttc cattggtcct tagctggggc 1140
agtactctaa cttggctttc ccaagcttgt cagtgtagtc tttatttggt ttgtatagtt 1200
tgagaagacc taaaatataa tctcattcct gctcaaagcc tgtgagtctg agtaactgtt 1260
gctaatgtag agaaggagcc attgggctgg tgggctgcta gtgtggtttc tgtgagccct 1320
gttccatttt ctttgccttt ccttgggatt gtaatggtag taagacctaa acctcaagtg 1380
gccatcagga tgccttctcc cctgtctcag tttacagtgg ggtcagggct ggccctgata 1440
gattttggtt gtggaatcta tggactttat cttcttttcc ttttgtgtcc tctcctagtg 1500
tccccctcct gacttctctc agtgaagact gttcacttgc tgtgctggat tcaagccttt 1560
ctgaggtgtt tagaagtcga gcccacagag actttgtgag agatgctacg tggtctccac 1620
tcaatcactc ccttcttacc acagttggct gggaccatca ggtcatccac catgttgtgc 1680
ccttagagcc tctcccaaac cctggacctg acagtgttgt ggagtagaat ggatttcaga 1740
aaaaccaaaa caagccctcc gtctgtaagc gactactcga ttgcccctgc cttccatgtg 1800
tgagagcaca ggagccttgt agagcatgtt tcctccctag ccccgtgcag taacaggcag 1860
attctcagcc tgagggaggc tgcatcccat agtgactcag aggaagaaac ttcctctgta 1920
aatggatgta tgtgagtaca cgtgtgagtg cggtatatag tttggagtgg agaaaattat 1980
tcttcagctt tcccaaagca atgctcttta cccctgacaa agtgaccaga ggattttaat 2040
gcttcccatg tctgggaatt ggcgatgtta gggatatgga atgtgggtat ccctagattt 2100
ttgggaatac ttcatactac tcagaggtgc gtaacttatt tttatataga gtttaattca 2160
ctattatggg aattacttcc atatacaaaa gtctagccca ctgtaccttg agaagacaaa 2220
acagttttgt acaagtccct gttatactga gtcaaatcca ttttctgggt gaataaattt 2280
ggccctgcag tgtagctctt gttcaccctt tccttccttg gtgttgtgtg tgagagatga 2340

gtgttttcct gtcctagatt tcacagataa gttgtaggga tgatggacca cttaaaagtg 2400

tttcagtgaa gtgggaaaat tgacatggat tgttacagag aaatctgtca tttttcccac 2460

ctaaaaattg atctgggtac cttcctctgg agagctgaag tctgaatgta tttttggact 2520

cctaagattt gagatccagc atcagggaaa ctgtcggaaa tactaaaagg tgaaataaag 2580

acttttatcc ttga 2594

<210> 193
<211> 2723
<212> DNA
<213> Artificial

<400> 193

```
ggtggggctg agggagggaa acaaacaaaa tcccgctgcg gggacaggag acggaaaagc      60
aggctggaag ggtagacaca attctcgcga gaagtagagc gaattccctc ggagaggggc     120
ggttgcgcag aggtacacca cgtgtgcggt ccctccggcg ccgagtcagg gtctctcgtc     180
gagtctgggt ctaggttaga tttggaatcg tggagatgcg gaaggacacc cctcctccgc     240
tcgtgccccc ggcggcccgc gagtggaacc tgccccccaa tgcgcccgca tgcatggaac     300
gtcaattgga ggctgcacgg taccggtctg atggttccct tctgctcggg gtctccagcc     360
tgagtggtcg ctgctgggta ggttctctgt ggtttttcaa ggatcctagt gcggccccca     420
acgaaggttt ctgctctgct ggcgtccaga ccgaggctgg agtagctgac ctcacttggg     480
tgggggacaa aggtatccta gtggcttctg attcaggtgc tgttgaattg tgggagctag     540
atgagaacga gacacttata gtcagcaagt tctgcaagta tgagcatgat gacattgtgt     600
ctactgtcac tgtcctgagc tctggcacac aagctgtcag tggtagcaaa gactgctgca     660
tcaaaatttg ggacctggct cagcaggtat cactgaattc ataccgagct cacgctggac     720
aggttacctg tgttgctgcc tctccccaca aagactctgt gtttctttca tgtagtgagg     780
acagtagaat tttgctctgg gatacccgct gtcccaagcc ggcatcacag atggcctgca     840
atgcctctgg ctacctccct accgctttgg cttggcatcc tcagcagagt gaagtctttg     900
tttttggtaa ggcagcatga cgtaccgtag actctgggaa tggggaggat gacaaagtgc     960
taccttcaat ggagtacatg cccctgtcag cacatgcagg cactgcatga attatgccag    1020
tttgaggtag ctatggagag catctttttt tccctagtgg gtttgtttcc tcattgttcc    1080
agcttcttta ttctgggcat ggttagaagt cagtgagctg tggagcaagt gcttgctgtt    1140
atggaattat gttctgctgt ggtgtgactg catatggtaa gagtttgtag ttaattgtcc    1200
cagccacata gtgacttact gattctatac cacaaaggaa actgttgtct acattttgag    1260
agactaggat tcattcaaat ccaggtctgg ctcacatcaa agtatggttt ttgttgttgt    1320
tttttttttt tttcttcctc aagacagggt ctgtatgaag tttgggctgt cttagaacct    1380
gatgagtaga ccaggctgac cttgaactca aagatccttc tgcttcagcc tcccacatac    1440
tagaattaaa ggtgtatact atacttagcc aggagcgtga attcttagcc actgtgacgt    1500
```

gctttatttc ttctgcaggt gacgagaatg gatctgtctc ccttgtggac accaagaatg 1560
caagctgtac cctcagctca gctgtgcact cccagggtgt cactagactg gtattctccc 1620
cacacagtgt ccccctcctg acttctctca gtgaagactg ttcacttgct gtgctggatt 1680
caagcctttc tgaggtgttt agaagtcgag cccacagaga ctttgtgaga gatgctacgt 1740
ggtctccact caatcactcc cttcttacca cagttggctg ggaccatcag gtcatccacc 1800
atgttgtgcc cttagagcct ctcccaaacc ctggacctga cagtgttgtg gagtagaatg 1860
gatttcagaa aaaccaaaac aagccctccg tctgtaagcg actactcgat tgcccctgcc 1920
ttccatgtgt gagagcacag gagccttgta gagcatgttt cctccctagc cccgtgcagt 1980
aacaggcaga ttctcagcct gagggaggct gcatcccata gtgactcaga ggaagaaact 2040
tcctctgtaa atggatgtat gtgagtacac gtgtgagtgc ggtatatagt ttggagtgga 2100
gaaaattatt cttcagcttt cccaaagcaa tgctctttac ccctgacaaa gtgaccagag 2160
gattttaatg cttcccatgt ctgggaattg gcgatgttag ggatatggaa tgtgggtatc 2220
cctagatttt tgggaatact tcatactact cagaggtgcg taacttattt ttatatagag 2280
tttaattcac tattatggga attacttcca tatacaaaag tctagcccac tgtaccttga 2340
gaagacaaaa cagttttgta caagtccctg ttatactgag tcaaatccat tttctgggtg 2400
aataaatttg gccctgcagt gtagctcttg ttcacccttt ccttccttgg tgttgtgtgt 2460
gagagatgag tgttttcctg tcctagattt cacagataag ttgtagggat gatggaccac 2520
ttaaaagtgt ttcagtgaag tgggaaaatt gacatggatt gttacagaga aatctgtcat 2580
ttttcccacc taaaaattga tctgggtacc ttcctctgga gagctgaagt ctgaatgtat 2640
ttttggactc ctaagatttg agatccagca tcagggaaac tgtcggaaat actaaaaggt 2700
gaaataaaga cttttatcct tga 2723

<210> 194
<211> 2094
<212> DNA
<213> Artificial

<400> 194

```
ggtggggctg agggagggaa acaaacaaaa tcccgctgcg gggacaggag acggaaaagc     60
aggctggaag ggtagacaca attctcgcga gaagtagagc gaattccctc ggagaggggc    120
ggttgcgcag aggtacacca cgtgtgcggt ccctccggcg ccgagtcagg gtctctcgtc    180
gagtctgggt ctaggttaga tttggaatcg tggagatgcg gaaggacacc cctcctccgc    240
tcgtgccccc ggcggcccgc gagtggaacc tgccccccaa tgcgcccgca tgcatggaac    300
gtcaattgga ggctgcacgg taccggtctg atggttccct tctgctcggg gtctccagcc    360
tgagtggtcg ctgctgggta ggttctctgt ggtttttcaa ggatcctagt gcggccccca    420
acgaaggttt ctgctctgct ggcgtccaga ccgaggctgg agtagctgac ctcacttggg    480
tgggggacaa aggtatccta gtggcttctg attcaggtgc tgttgaattg tgggagctag    540
```

atgagaacga gacacttata gtcagcaagt tctgcaagta tgagcatgat gacattgtgt 600
ctactgtcac tgtcctgagc tctggcacac aagctgtcag tggtagcaaa gactgctgca 660
tcaaaatttg ggacctggct cagcaggtat cactgaattc ataccgagct cacgctggac 720
aggttacctg tgttgctgcc tctccccaca aagactctgt gtttctttca tgtagtgagg 780
acagtagaat tttgctctgg gatacccgct gtcccaagcc ggcatcacag atggcctgca 840
atgcctctgg ctacctccct accgctttgg cttggcatcc tcagcagagt gaagtctttg 900
tttttggtga cgagaatgga tctgtctccc ttgtggacac caagaatgca agctgtaccc 960
tcagctcagc tgtgcactcc cagggtgtca ctagactgtg tcccccctcct gacttctctc 1020
agtgaagact gttcacttgc tgtgctggat tcaagccttt ctgaggtgtt tagaagtcga 1080
gcccacagag actttgtgag agatgctacg tggtctccac tcaatcactc ccttcttacc 1140
acagttggct gggaccatca ggtcatccac catgttgtgc ccttagagcc tctcccaaac 1200
cctggacctg acagtgttgt ggagtagaat ggatttcaga aaaaccaaaa caagccctcc 1260
gtctgtaagc gactactcga ttgcccctgc cttccatgtg tgagagcaca ggagccttgt 1320
agagcatgtt tcctccctag ccccgtgcag taacaggcag attctcagcc tgagggaggc 1380
tgcatcccat agtgactcag aggaagaaac ttcctctgta aatggatgta tgtgagtaca 1440
cgtgtgagtg cggtatatag tttggagtgg agaaaattat tcttcagctt tcccaaagca 1500
atgctcttta cccctgacaa agtgaccaga ggattttaat gcttcccatg tctgggaatt 1560
ggcgatgtta gggatatgga atgtgggtat ccctagattt ttgggaatac ttcatactac 1620
tcagaggtgc gtaacttatt tttatataga gtttaattca ctattatggg aattacttcc 1680
atatacaaaa gtctagccca ctgtaccttg agaagacaaa acagttttgt acaagtccct 1740
gttatactga gtcaaatcca ttttctgggt gaataaattt ggccctgcag tgtagctctt 1800
gttcaccctt tccttccttg gtgttgtgtg tgagagatga gtgttttcct gtcctagatt 1860
tcacagataa gttgtaggga tgatggacca cttaaaagtg tttcagtgaa gtgggaaaat 1920
tgacatggat tgttacagag aaatctgtca tttttcccac ctaaaaattg atctgggtac 1980
cttcctctgg agagctgaag tctgaatgta tttttggact cctaagattt gagatccagc 2040
atcagggaaa ctgtcggaaa tactaaaagg tgaaataaag acttttatcc ttga 2094

<210> 195
<211> 2596
<212> DNA
<213> Artificial

<400> 195

ggaagtgctc ctgcggctcc ggttgccggc gtgactttga gctcttcccg ctctgctgcc 60
ggcgctgagc acaaccgatc acctcagagc aggagccttg gaagtggctg ttccaggctt 120
gccatgcggc tgctgcgggt cgccgcagcc ctggggcgtg gaccgttccc tcgggtccct 180
gcagtcctgg gctggcaggg gaagcaggct gattggaaga cccgccgatg gtgttcgtca 240

gggccagttc ccaatgaaaa aatacggaac attgggatct cagctcacat tgattctggg 300
aagacgacgc taacagaacg ggtgctatac tacactggca ggattgccac gatgcacgag 360
gtgaaaggta aagatggagt tggcgctgtc atggattcca tggagctgga gaggcagcga 420
gggatcacga tccagtcagc agctacgtat accatgtgga aagacatcaa tatcaacatc 480
atagacacgc caggccacgt ggactttacc atagaagtgg aaagagccct gcgggtcctg 540
gacggtgcag tccttgttct ctgtgcggtt ggcggagtcc agtgccagac catgacagtc 600
agtcgtcaga tgaagcgcta caacgttccc tttttaacct tcattaacaa actggaccga 660
atgggctcta acccatccag ggccctccaa caaatgagat ctaaactaaa tcacaatgct 720
gcatttgtac aaatacccat tggtttagag ggtgatttta agggaattat agacctcatt 780
gaagaacgag ctatttactt tgatggagac tttggtcaga ttgtccgata tgacgagatt 840
ccggctggat tgcgggccgc agccgctgac caccggcagg agctcattga gtgcgttgct 900
aactcagatg agcagcttgg tgagctcttc ctggaagaaa agatcccctc agtttccgac 960
ttaaagcgag caatccggag agctactctg agtcgatcct tcactcctgt atttttggga 1020
agtgctttga aaaacaaagg agttcaacct cttttggacg ctgttttaga atacctgccc 1080
aatccatctg aggtccagaa ttacgctata ctcaatcaga atgactcaaa agagaagacc 1140
aaaatcttaa tgaaccccca aagagatgat tcgcacccat ttgtaggcct ggcttttaaa 1200
ctggaggcgg gccgttttgg acagctgact tatgtccgaa attatcaagg tgaactgaag 1260
aagggaagca ccatctacaa cacgaggacc gggaagaaag tgcgcgtgca gcggctagtg 1320
cgcatgcacg ccgacatgat ggaggatgtt gaagaagtat atgctggaga catctgtgca 1380
ttgtttggca ttgactgtgc aagtggagat acattcacaa acaaagataa cagtgacctt 1440
tctatggaat cgattcatgt tcctgagcct gtcatttcaa tagcaatgag gccttctaac 1500
aagaatgatc tggagaaatt ttccaaaggt attggcaggt ttacacggga agaccccaca 1560
tttaaagtcc actttgaccc tgagagcaag gagaccattg tgtcagggat gggagagctg 1620
cacctggaaa tctatgctca gagaatggag agagaatatg gctgcccttg tatcacagga 1680
aaacccaaag tagcctttag agagaccatt gttgccccag tgccgtttga ttttacacat 1740
aaaaagcagt cgggtggtgc cggccagttt ggaaaagtaa taggagtgct ggagcccttg 1800
cccccagagg actacacgaa gttggagttt tctgatgaga catttgggtc caatgttcca 1860
aagcagtttg tccccgctgt tgaaaagggg tttctggatg cctgcgaaaa aggccctctt 1920
tctggtcaca agctttctgg gctacggttt gttctgcaag atggagcaca ccacatggtt 1980
gattcgaatg aaatctcttt catccgagct ggagaaggtg ctcttaaaca agccttggca 2040
aatggcacac tatgtattat tgagcctatt atgtctgtgg aagttatagc cccccaatgaa 2100
ttccagggaa cagtgttcgc agggattaac cggcggcatg gagtgattac agggcaggac 2160
ggcattgagg actacttcac actgtatgca gatgttcccc taaataatat gtttggttat 2220
tccactgagc ttaggtcttg cacagaggga aaaggagagt acacgatgga gtactgcagg 2280

taccagccgt gttcaccatc cacacaggaa gagctcatca ataagtattt ggaagctaca 2340

ggtcagcttc ccgtaaaaaa agggaaagcc aagaactgac tctcctcact caggatatac 2400

tgactatttg actaccacac ttggggatga tggagtctgc ttgaatgaat tctgacggca 2460

gaaacaaatt taggagcccc tgctggccca cgttcaggag cttctgtatg tacgccaagg 2520

ataactgtgt atttaaactg tattgactat ttttattgtt tagtaaaaga ccttaaataa 2580

aattatatta ttactg 2596

<210> 196
<211> 1971
<212> DNA
<213> Artificial

<400> 196

```
ggaagtgctc ctgcggctcc ggttgccggc gtgactttga gctcttcccg ctctgctgcc     60
ggcgctgagc acaaccgatc acctcagagc aggagccttg gaagtggctg ttccaggctt    120
gccatgcggc tgctgcgggt cgccgcagcc ctggggcgtg gaccgttccc tcgggtccct    180
gcagtcctgg gctggcaggg gaagcaggct gattggaaga cccgccgatg gtgttcgtca    240
gggccagttc ccaatgaaaa aatacggaac attgggatct cagctcacat tgattctggg    300
aagacgacgc taacagaacg ggtgctatac tacactggca ggattgccac gatgcacgag    360
gtgaaaggta aagatggagt tggcgctgtc atggattcca tggagctgga gaggcagcga    420
gggatcacga tccagtcagc agctacgtat accatgtgga aagacatcaa tatcaacatc    480
atagacacgc caggccacgt ggactttacc atagaagtgg aaagagccct gcgggtcctg    540
gacggtgcag tccttgttct ctgtgcggtt ggcggagtcc agtgccagac catgacagtc    600
agtcgtcaga tgaagcgcta caacgttccc tttttaacct tcattaacaa actggaccga    660
atgggctcta acccatccag ggccctccaa caaatgagat ctaaactaaa tcacaatgct    720
gcatttgtac aaatacccat tggtttagag ggtgatttta agggaattat agacctcatt    780
gaagaacgag ctatttactt tgatggagac tttggtcaga ttgtccgata tgacgagatt    840
ccggctggat tgcgggccgc agccgctgac caccggcagg agctcattga gtgcgttgct    900
aactcagatg agcagcttgg tgagctcttc ctggaagaaa agatcccctc agtttccgac    960
ttaaagcgag caatccggag agctactctg agtcgatcct tcactcctgt atttttggga   1020
agtgctttga aaaacaaagg agttcaacct cttttggacg ctgttttaga atacctgccc   1080
aatccatctg aggtccagaa ttacgctata ctcaatcaga atgactcaaa agagaagacc   1140
aaaatcttaa tgaaccccca aagagatgat tcgcacccat ttgtaggcct ggcttttaaa   1200
ctggaggcgg gccgttttgg acagctgact tatgtccgaa attatcaagg tgaactgaag   1260
aagggaagca ccatctacaa cacgaggacc gggaagaaag tgcgcgtgca gcggctagtg   1320
cgcatgcacg ccgacatgat ggaggatgtt gaagaagtat atgctggaga catctgtgca   1380
ttgtttggca ttgactgtgc aagtggagat acattcacaa acaaagataa cagtgacctt   1440
```

tctatggaat cgattcatgt tcctgagcct gtcatttcaa tagcaatgag gccttctaac 1500
aagaatgatc tggagaaatt ttccaaaggt attggcaggt ttacacggga agaccccaca 1560
tttaaagtcc actttgaccc tgagagcaag gagaccattg tgtcagggat gggagagctg 1620
cacctggaaa tctatgctca gagaatggag agagaatatg gctgcccttg tatcacagga 1680
aaacccaaag tagcctttag agagaccatt gttgccccag tgccagtggc atcaaataaa 1740
gaaaagttgt atatgttacc tgcgatgcaa tgtcgtggag tagaatggtg taatctaact 1800
caatgaagtc atcgtttctt tgctacaaca acaacaacaa caacaaaatg cttagacaac 1860
atcatggcaa aaatgacatc ttaatttctt ttttgcatta atgttgattt attcacttca 1920
ggttagcctg gcagaaaact agtttcttaa aataataaca attcttcttt c 1971

<210> 197
<211> 1001
<212> DNA
<213> Artificial

<400> 197

agccctgagt ctggcttgcg ggtggtggga gcagacagtt cttgcctcgg gaccggcaat 60
catggcttcg gcttggccgc ggtctctgcc gcagatcctc gtgttgggat tcggcttggt 120
gttgatgcgc gccgcggccg gggagcaagc accaggcacc tccccatgct ctagcggcag 180
ctcctggagc gcggacctcg acaagtgcat ggactgcgct tcttgtccag cgcgaccaca 240
cagcgacttc tgcctgggat gcgccgcagc acctcctgcc cacttcaggc tactgtggcc 300
cattctgggg ggcgctctta gtctggtcct ggttttggcg ctggtttcta gtttcctggt 360
ctggagaaga tgccgccgga gagaaaagtt tactaccccc atagaggaga ctggtggaga 420
gggctgccca ggtgtggcac tgatccagtg aggagcaccc gcgctggggc ccactcgtcg 480
tccattcatt cattctggag tcagcctggc cttccagaca gaagccgagc cagactcttt 540
caaccacaag ggggtggggc gaggtggtga ttcacctcca aggactgggc ttagagttca 600
gggagccttc caaggtgtcc aattgcccta tctctgggtc tggggcagac agagagcctc 660
aatctgggtc acaaagcgac ccatactaag gaactgcagc atttgcacaa gggaatctct 720
tgttccccac aagtcctggc agtctggctg acttgagggg cagacacaac actgggtccc 780
acccactcgg aggggctata atgccactcc ctgagtttta gccctaggag tgggtcagat 840
tgacagacct gttcttaaca ctaaggggggc tggccctctg ggagggctgg ccccagtaca 900
cacggaaaca accatctccc aagggggggtg atatttattg tggggataat gtttggaggg 960
gagggagact tattaataaa agaatcttta actttaagat a 1001

<210> 198
<211> 1078
<212> DNA

<213> Artificial

<400> 198

```
agccctgagt ctggcttgcg ggtggtggga gcagacagtt cttgcctcgg gaccggcaat      60
catggcttcg gcttggccgc ggtctctgcc gcagatcctc gtgttgggat tcggcttggt     120
gttgatgcgc gccgcggccg gggagcaagc accaggcagc aagttccaag tgggaaaggt     180
ctcaagtttt atccgcagtc cgagcttcag tttcgctacc tgtccaattg aggcacctcc     240
ccatgctcta gcggcagctc ctggagcgcg gacctcgaca agtgcatgga ctgcgcttct     300
tgtccagcgc gaccacacag cgacttctgc ctgggatgcg ccgcagcacc tcctgcccac     360
ttcaggctac tgtggcccat tctgggggc gctcttagtc tggtcctggt tttggcgctg      420
gtttctagtt tcctggtctg gagaagatgc cgccggagag aaaagtttac taccccccata     480
gaggagactg gtggagaggg ctgcccaggt gtggcactga tccagtgagg agcacccgcg     540
ctggggccca ctcgtcgtcc attcattcat tctggagtca gcctggcctt ccagacagaa     600
gccgagccag actctttcaa ccacaagggg gtggggcgag gtggtgattc acctccaagg     660
actgggctta gagttcaggg agccttccaa ggtgtccaat tgccctatct ctgggtctgg     720
ggcagacaga gagcctcaat ctgggtcaca aagcgaccca tactaaggaa ctgcagcatt     780
tgcacaaggg aatctcttgt tccccacaag tcctggcagt ctggctgact tgaggggcag     840
acacaacact gggtcccacc cactcggagg ggctataatg ccactccctg agttttagcc     900
ctaggagtgg gtcagattga cagacctgtt cttaacacta agggggctgg ccctctggga     960
gggctggccc cagtacacac ggaaacaacc atctcccaag gggggtgata tttattgtgg    1020
ggataatgtt tggaggggag ggagacttat taataaaaga atctttaact ttaagata      1078
```

<210> 199
<211> 910
<212> DNA
<213> Artificial

<400> 199

agccctgagt ctggcttgcg ggtggtggga gcagacagtt cttgcctcgg gaccggcaat 60
catggcttcg gcttggccgc ggtctctgcc gcagatcctc gtgttgggat tcggcttggt 120
gttgatgcgc gccgcggccg gggagcaagc accaggcacc tccccatgct ctagcggcag 180
ctcctggagc gcggacctcg acaagtgcat ggactgcgct tcttgtccag cgcgaccaca 240
cagcgacttc tgcctgggat tttcctggtc tggagaagat gccgccggag agaaaagttt 300
actaccccca tagaggagac tggtggagag ggctgcccag gtgtggcact gatccagtga 360
ggagcacccg cgctggggcc cactcgtcgt ccattcattc attctggagt cagcctggcc 420
ttccagacag aagccgagcc agactctttc aaccacaagg gggtggggcg aggtggtgat 480
tcacctccaa ggactgggct tagagttcag ggagccttcc aaggtgtcca attgccctat 540
ctctgggtct ggggcagaca gagagcctca atctgggtca caaagcgacc catactaagg 600
aactgcagca tttgcacaag ggaatctctt gttccccaca agtcctggca gtctggctga 660
cttgaggggc agacacaaca ctgggtccca cccactcgga ggggctataa tgccactccc 720

tgagttttag ccctaggagt gggtcagatt gacagacctg ttcttaacac taagggggct 780
ggccctctgg gagggctggc cccagtacac acggaaacaa ccatctccca aggggggtga 840
tatttattgt ggggataatg tttggagggg agggagactt attaataaaa gaatctttaa 900
ctttaagata 910

<210> 200
<211> 952
<212> DNA
<213> Artificial

<400> 200

```
gacgcacgac acaacctctc gattccggac gtgcctgatg gtggctcttc tcgttacgcc      60
ccaagaccta gcactgcctg tcgcccagca actaaggcgg caggtccgtg ctgtctcgaa     120
ccacagaaac ctaggagaac tacacgaagc acccttatgc taaaaagcgc ccagaactat     180
ccagccacgc cggatcccct cccccaccgc gccgacctgt cccgccttct aacctctaac     240
ctgattcctg tcttccgcgc ctgcgtaaaa cgtctccagt gcgcacgccg gtaggcccgc     300
ccctcgcgct gtaccacgcc ctcaaaggct ccgcctctgc tgcttgaatc ccacccctct     360
atctgcagga ccctgagccg taatgatcct gcaacagccc ctggagcgag gtcccccaag     420
tcgggaccca cgggccacca ctggggtaac tcgcggccta aacgccagtc tttcccccag     480
ggaacctttg cacaagcagt tcctgtctga ggagaacatg gccacccatt tctcccgact     540
cagcctacat aatgaccacc cctactgcag ccccccctgtg acttttcccg aagccctgcc    600
accactcagg agcccttgcc cagagctgct tctctggcgc tatcccggga gcctgattcc     660
tgaagccctc aggctgctga ggctggggga tacccccagt ccctactacc ctgcgtcccc     720
agctggggat atcgtggagc tctgagtatt gatggcctgt gtccctccct atcttctttc     780
tgacaacaaa gaccagcaac tcccagaaag ggatgaagtc cttctgcaaa cctggccacc     840
aggaccttcc ctctgacaag aggacactag ctcggtggaa ccctcagtga aaggcagcct     900
gggaaaggga aaatgttacc ccattagtga ataaagacct gtgagcagtt ga             952
```

<210> 201
<211> 1108
<212> DNA
<213> Artificial

<400> 201

```
gggaggaaaa cttcctggct gggactccgg cggtttctgc ctgccaaccc tcggatcccg      60
cctaccagtg ttggctcttc ccgacccctc ccccggcgcc cccagtgtcc gccatggcca     120
aagcctacga ccacctcttc aagttgctgc tcatcgggga ctcgggggtg ggcaagactt     180
gtctgatcat tcgctttgca gaggacaact tcaacagcac ttacatctct accatcggaa     240
ttgatttcaa gatccgaacc gtggacatag aggggaagag gatcaaactg caagtgtggg     300
acacggctgg ccaagaacga ttcaagacaa taactaccgc ctattaccgt ggagccatgg     360
```

gcattatcct cgtatatgac atcacagatg agaaatcctt cgagaatatt cagaactgga 420
tgaaaagcat caaagagaat gcctctgcgg gagtggagcg cctcctgctg ggaaacaagt 480
gtgacatgga ggccaagcgg caggtgcaga gagagcaggc ggagaagttg gctcgagagc 540
acagaatccg atttttgag acgagtgcca aatccagtgt gaatgtggat gaggctttca 600
gttccctggc ccgtgacatc ttgctcaaga caggaggccg gagatcggga accaacagta 660
agccctcaag cactggcctg aaaacatctg acaagaagaa gaacaagtgc ttgttaggct 720
gagagcattt cttgcctcct attcaccctg aacctggagg ctagacctga gggagtcgga 780
ctgagggatt gcagatggga gaactgtggt ggcacctcaa ggggagatga ggggaataag 840
gagaccggcg aggacgagac ggaagaaagg ggcagggaaa ggagggggag gaaccaagga 900
tgtgaaaggt gaacagaagg gatttgagaa gaggaaagga agaagaaatg aatggctcag 960
gccttggaca gtccaacatt aaagtcaaca tgctgatctc tccattcctg gttcagggtt 1020
agggtcctga gaggctggct cggcactact ccgagggtcc ctcactctac aaggtctttg 1080
ttagtattaa aggccactgt tttgcatg 1108

<210> 202
<211> 1037
<212> DNA
<213> Artificial

<400> 202

```
gaaagggagg agtagggcag ggccagagga gggacagcct gacgcggcca ccagccctga      60
ctcaacctgc cagccccctt acctggccag ccttgaggag atggaacagc ccaggctaca     120
aggcctgccc ccactcctca acttcccttg ataatgaacc agaaactgaa actaaaacag     180
gcaggctccc cgttagagat gagtcacttc ccaaagtgac tgaagtagcc gagaagtgga     240
aacagagggg cagaagagaa cccagggcac tgagacaggg ctttctgagg gtcgccaagg     300
agcatggcag gcatcccaga ggtggtggcc atggactgtg agatggtggg gcttgggcct     360
caaagggtga gtggcctcgc ccgctgcagc attgtgaaca tccatggcgc agtcctgtat     420
gacaagtaca tccgacccga gggagagatc acggactaca gaacccaagt cagcggggtc     480
acgcctcagc acatggtgag ggccacgcca tttggtgaag ccaggctaga gatcctgcag     540
cttctgaaag gcaagctggt ggtgggccat gacctgaagc acgacttcaa tgccctgaag     600
gaggatatga gcaagtacac catctatgac acgtccacag acaggctgct gtggcatgag     660
gccaagctgc agtactacag ccgagtgtcc ctgaggctgc tgtgtaagcg cctgctacac     720
aagaacatcc agaacaactg gcggggccac tgctctgtgg aagatgccag ggccacaatg     780
gagctctaca aaatctctca gcgactcaga gcccagcgag ggctgccttg ccctgggacg     840
tcagactgaa cttcatcctc atccagggtt agaagctgcc actcctcaag ttctccatga     900
atgagacctg tttctaacaa ccactgccac caaccaccgc aatgtgtaaa accaagaaca     960
ctcccccatt acagcaactc tcttgctttg aggcaaaaac aacaacaaca acaacaacaa    1020
caacaacagc aaaacca                                                   1037
```

<210> 203
<211> 1017
<212> DNA
<213> Artificial

<400> 203

```
gaaagggagg agtagggcag ggccagagga gggacagcct gacgcggcca ccagccctga      60
ctcaacctgc cagcccccttt acctggccag ccttgaggag atggaacagc ccaggctaca    120
aggcctgccc ccactcctca acttcccttg ataatgaacc agaaactgaa actaaaacag    180
gcaggctccc cgttagagat gagtcacttc ccaaagtgac tgaagtagcc gagaagtgga    240
aacagagggg cagaagagaa cccagggcac tgagacaggg ctttctgagg gtcgccaagg    300
agcatggcag gcatcccaga ggtggtggcc atggactgtg agatggtggg gcttgggcct    360
caaagggtga gtggcctcgc ccgctgcagc attgtgaaca tccatggcgc agtcctgtat    420
gacaagtaca tccgacccga gggagagatc acggactaca gaacccaagt cagcggggtc    480
acgcctcagc acatggtgag ggccacgcca tttggtgaag ccaggctaga gatcctgcag    540
cttctgaaag gcaagctggt ggtgggccat gacctgaagc acgacttcaa tgccctgaag    600
gaggatatga gcaagtacac catctatgac acgtccacag acaggctgct gtggcatgag    660
gccaagctgc agtactacag ccgagtgtcc ctgaggctgc tgtgtaagcg cctgctacac    720
aagaacatcc aggtgaggag tctcccggcc cccatgaccc ttcttgtctc tctctcttct    780
ccctcctccc tctcgttctc cctcttccca ctcagatgct ctttctcttt ctccttcctc    840
tcctactcct ccaagcaagg agttctcctc ctttcttcct tagtcctccc tctccttcct    900
tgcttctaaa agtgagggtc ttcctctccc tcctccccac actcccactc ctcccaaggg    960
cagatcagtc ccacctgcct aagctggctc caggtaagga acctctgcag cctgctg       1017
```

<210> 204
<211> 1164
<212> DNA
<213> Artificial

<400> 204

```
agaacccagg aggaagaaca gcctggcttg aggccctcta aaggtctgct gctgtttgtc      60
aactctccca gggctttctg agggtcgcca aggagcatgg caggcatccc agaggtggtg    120
gccatggact gtgagatggt ggggcttggg cctcaaaggg tgagtggcct cgcccgctgc    180
agcattgtga acatccatgg cgcagtcctg tatgacaagt acatccgacc cgagggagag    240
atcacggact acagaaccca agtcagcggg gtcacgcctc agcacatggt gagggccacg    300
ccatttggtg aagccaggct agagatcctg cagcttctga aaggcaagct ggtggtgggc    360
catgacctga agcacgactt caatgccctg aaggaggata tgagcaagta caccatctat    420
gacacgtcca cagacaggct gctgtggcat gaggccaagc tgcagtacta cagccgagtg    480
```

tccctgaggc tgctgtgtaa gcgcctgcta cacaagaaca tccaggtcct gcctggcagc 540
ctcttggggg ttggaggatg catcttgcca ggcactgaca tccttcatct tctgctctat 600
gtcggaatgg tcaggattgc tgatctccgc ttgctgacgc cctttctacc tccgagctgc 660
ctcgcttgcc cactgctccc ggagagtctt gcttctgctc gctcgcacgc tgttatttct 720
gccctgtcca gctctagtca cctgctgaca cccctcccaa acccgagtca gggacctcag 780
ggacacgtgg acagactgag cggccagctc caggactggg gaggcagccc cttagctcct 840
gctctccctg tgtctgcaga acaactggcg gggccactgc tctgtggaag atgccagggc 900
cacaatggag ctctacaaaa tctctcagcg actcagagcc cagcgagggc tgccttgccc 960
tgggacgtca gactgaactt catcctcatc cagggttaga agctgccact cctcaagttc 1020
tccatgaatg agacctgttt ctaacaacca ctgccaccaa ccaccgcaat gtgtaaaacc 1080
aagaacactc ccccattaca gcaactctct tgctttgagg caaaaacaac aacaacaaca 1140
acaacaacaa caacagcaaa acca 1164

<210> 205
<211> 2224
<212> DNA
<213> Artificial

<400> 205

```
agcgaccggc aggctgcccg cccgacttcc cagctgcccc gtgcggcccg ggcatgccca      60
gtgtgggcgc agccccggct ctccgagcgc ccgggcggag cgggcagccg ggcgcggagc     120
aggcgaaccg agcctcggtg ggcgagcgcc ctggctggag ccgcggcggt agggtggcgg     180
ggccctccat gatgaatggt ttgggggcac ttccctctcg ctgtatttga tagtctgggc     240
agtggagaga tggctgacag tgtcaaaacc tttctgcagg accttggcag gggaatcaaa     300
gactccatct ggggcatctg taccatctca aagctagatg ctcggatcca gcagaagaga     360
gaggaacagc gtcgaagaag ggcaagtagc ctcttggccc agaggagacc ccagagtgta     420
gagcggaagc aagagagtga accacgtatt gttagtagaa ttttccagtg ttgtgcttgg     480
aatggtggag tattctggtt cagtctcctc ttgttttatc gagtgtttat tcctgtactt     540
cagtcagtaa cagcccggat tattggagat ccatcacttc atggagatgt ttggtcatgg     600
ctggaattct tcctcacatc aattttcagt gctctttggg tgctccccct gtttgtgctt     660
agcaaagttg tgaatgccat ttggttccaa gatatagctg acttggcatt tgaagtatca     720
gggaggaaac ctcatccatt ccccagtgtc agcaaaataa ttgctgacat gctcttcaac     780
cttttgctac aggcactttt ccttattcag ggatgtttg tgagtctctt ccccatccat      840
cttgtgggtc agctggttag tctgctgcat atgtctcttc tctattcact gtactgcttt     900
gagtaccgtt ggttcaacaa aggaattgaa atgcaccagc gattgtcgaa catagaaagg     960
aattggcctt actactttgg gtttggcttg cccttggctt tcctcacagc aatgcaatcc    1020
tcctacatta tcagtggctg cctcttttct atcctgtttc ctttattcat catcagcgcc    1080
```

aatgaagcaa agactcctgg aaaagcatat cttttccagt tgcgcctatt ctccttggtg 1140
gtctttttaa gcaacagact tttccacaag accgtctacc tgcagtcagc cctgagcagc 1200
tcgtcctctg cagagaaatt cccttcgcca catccttctc ctgccaaact gaaagctgct 1260
gcaggccact gagccctgct gtcaaagggg tgggtgggac tgggtggagg atgtggcagc 1320
tcttttctct gttttcctcc ccctgccgtg gaaggcagaa cccactgcca agggccctct 1380
gcatagtccc ttgtctttga attggaatct tcctgactcc agtatatgga tttttaccac 1440
caccctaggt ctgtaaggac cagagttttc cagctgtttt tttagcactt gccagctcct 1500
gtgcctggac tgattgattt gagtactttt ttccccttt ccttgtgtca tttaccctcc 1560
cacttcctcc tgccttccag cacccctgga tgaatgggct ttgtaatttt aactgttgta 1620
ttttgtgaat ttgttgttac tgtttttctg tgaagcacat acattgtatg tgggaggtaa 1680
aggggcattc cagttgctcc agtcactccc tctatagcca tcactgtctt gttttctgta 1740
actcaggtta ggttttggtc tctattgctc tgctgcagaa aaggaaagaa aggagtgggg 1800
gaaatggagc ctgaagagtt ggggcagata gacctcagcc aaactggctg ggttttgagg 1860
agtcatgttc tttcttcccc ttgaagggga aagagttttt tccactggtc catttaaagt 1920
ttcccagcta tggggtggta ccagttctgg acaagtgcca ctgcatcata gtatgctcgg 1980
agaatctgaa ccttactctg aagatgaaat ttactgttgg ccactgccag gtcagactgg 2040
tgttttaagg aatactgggt gcttcatata ggaactgaag ggtaaactt actaaaccat 2100
tcaacctgtg attggtgatg ttttcctgtc attttaagag tcgacacatg ggtgggggg 2160
cagatgtaaa aaaacttgta caattttaaa atatcacaat taaacgtgag ctggtttccc 2220
aaaa 2224

<210> 206
<211> 2144
<212> DNA
<213> Artificial

<400> 206

```
agcgaccggc aggctgcccg cccgacttcc cagctgcccc gtgcggcccg ggcatgccca      60
gtgtgggcgc agccccggct ctccgagcgc ccgggcggag cgggcagccg ggcgcggagc     120
aggcgaaccg agcctcggtg ggcgagcgcc ctggctggag ccgcggcggt agggtggcgg     180
ggccctccat gatgaatggt ttgggggcac ttccctctcg ctgtatttga tagtctgggc     240
agtggagaga tggctgacag tgtcaaaacc tttctgcagg accttggcag gggaatcaaa     300
gactccatct ggggcatctg taccatctca aagctagatg ctcggatcca gcagaagaga     360
gaggaacagc gtcgaagaag ggcaagtagc ctcttggccc agaggagacc ccagagtgta     420
gagcggaagc aagagagtga accacgtatt gttagtagaa ttttccagtg ttgtgcttgg     480
aatggtggag tattctggtt cagtctcctc ttgttttatc gagtgtttat tcctgtactt     540
cagtcagtaa cagcccggat tattggagat ccatcacttc atggagatgt ttggtcatgg     600
```

ctggaattct tcctcacatc aattttcagt gctctttggg tgctccccct gtttgtgctt 660
agcaaagttg tgaatgccat ttggttccaa gatatagctg acttggcatt tgaagtatca 720
gggaggaaac ctcatccatt ccccagtgtc agcaaaataa ttgctgacat gctcttcaac 780
cttttgctac aggcactttt ccttattcag gggatgtttg tgagtctctt ccccatccat 840
cttgtgggtc agctggttag tctgctgcat atgtctcttc tctattcact gtactgcttt 900
gagtaccgtt ggttcaacaa aggaattgaa atgcaccagc gattgtcgaa catagaaagg 960
aattggcctt actactttgg gtttggcttg cccttggctt tcctcacagc aatgcaatcc 1020
tcctacatta tcagtggctg cctcttttct atcctgtttc ctttattcat catcagcgcc 1080
aatgaagcaa agactcctgg aaaagcatat cttttccagt tgcgcctatt ctccttggtg 1140
gtctttttaa gcaacagact tttccacaag accgtctacc tgcagccact gagccctgct 1200
gtcaaagggg tgggtgggac tgggtggagg atgtggcagc tcttttctct gttttcctcc 1260
ccctgccgtg gaaggcagaa cccactgcca agggccctct gcatagtccc ttgtctttga 1320
attggaatct tcctgactcc agtatatgga tttttaccac caccctaggt ctgtaaggac 1380
cagagttttc cagctgtttt tttagcactt gccagctcct gtgcctggac tgattgattt 1440
gagtactttt ttcccccttt ccttgtgtca tttaccctcc cacttcctcc tgccttccag 1500
cacccctgga tgaatgggct ttgtaatttt aactgttgta ttttgtgaat ttgttgttac 1560
tgtttttctg tgaagcacat acattgtatg tgggaggtaa aggggcattc cagttgctcc 1620
agtcactccc tctatagcca tcactgtctt gttttctgta actcaggtta ggttttggtc 1680
tctattgctc tgctgcagaa aaggaaagaa aggagtgggg gaaatggagc ctgaagagtt 1740
ggggcagata gacctcagcc aaactggctg ggttttgagg agtcatgttc tttcttcccc 1800
ttgaagggga aagagttttt tccactggtc catttaaagt ttcccagcta tggggtggta 1860
ccagttctgg acaagtgcca ctgcatcata gtatgctcgg agaatctgaa ccttactctg 1920
aagatgaaat ttactgttgg ccactgccag gtcagactgg tgttttaagg aatactgggt 1980
gcttcatata ggaactgaag ggtaaactt actaaaccat tcaacctgtg attggtgatg 2040
ttttcctgtc attttaagag tcgacacatg ggtggggggg cagatgtaaa aaaacttgta 2100
caattttaaa atatcacaat taaacgtgag ctggtttccc aaaa 2144

<210> 207
<211> 2507
<212> DNA
<213> Artificial

<400> 207

agcgaccggc aggctgcccg cccgacttcc cagctgcccc gtgcggcccg ggcatgccca 60
gtgtgggcgc agccccggct ctccgagcgc ccgggcggag cgggcagccg ggcgcggagc 120
aggcgaaccg agcctcggtg ggcgagcgcc ctggctggag ccgcggcggt agggtggcgg 180
ggccctccat gatgaatggt ttgggggcac ttccctctcg ctgtatttga tagtctgggc 240

agtggagaga tggctgacag tgtcaaaacc tttctgcagg accttggcag gggaatcaaa 300
gactccatct ggggcatctg taccatctca aagctagatg ctcggatcca gcagaagaga 360
gaggaacagc gtcgaagaag ggcaagtagc ctcttggccc agaggagacc ccagagtgta 420
gagcggaagc aagagagtga accacgtatt gttagtagaa ttttccagtg ttgtgcttgg 480
aatggtggag tattctggtt cagtctcctc ttgttttatc gagtgtttat tcctgtactt 540
cagtcagtaa cagcccggat tattggagat ccatcacttc atggagatgt ttggtcatgg 600
ctggaattct tcctcacatc aattttcagt gctctttggg tgctccccct gtttgtgctt 660
agcaaagttg tgaatgccat ttggttccaa gtaggtgcag gacaaaatgg aactgggttc 720
tacatgctgg gttgactagg ttacacattt tagcctatga agttagtggc ctataaaact 780
taatccttaa cgtttttaag ccaagcagtc aggtaatatt tgtggttttg taaaatgaaa 840
ttactgtctt gggttactga gacagacttt tctcacctgg cagttctagc agcaaaccag 900
tggccttaac tctggggatg aacactggga ttatttaatc catattttgc tatattattg 960
ttctttgttt caggatatag ctgacttggc atttgaagta tcagggagga aacctcatcc 1020
attccccagt gtcagcaaaa taattgctga catgctcttc aacctttgc tacaggcact 1080
tttccttatt caggggatgt ttgtgagtct cttccccatc catcttgtgg gtcagctggt 1140
tagtctgctg catatgtctc ttctctattc actgtactgc tttgagtacc gttggttcaa 1200
caaaggaatt gaaatgcacc agcgattgtc gaacatagaa aggaattggc cttactactt 1260
tgggtttggc ttgcccttgg ctttcctcac agcaatgcaa tcctcctaca ttatcagtgg 1320
ctgcctcttt tctatcctgt ttcctttatt catcatcagc gccaatgaag caaagactcc 1380
tggaaaagca tatcttttcc agttgcgcct attctccttg gtggtctttt taagcaacag 1440
acttttccac aagaccgtct acctgcagtc agccctgagc agctcgtcct ctgcagagaa 1500
attcccttcg ccacatcctt ctcctgccaa actgaaagct gctgcaggcc actgagccct 1560
gctgtcaaag gggtgggtgg gactgggtgg aggatgtggc agctcttttc tctgttttcc 1620
tccccctgcc gtggaaggca gaacccactg ccaagggccc tctgcatagt cccttgtctt 1680
tgaattggaa tcttcctgac tccagtatat ggatttttac caccacccta ggtctgtaag 1740
gaccagagtt ttccagctgt ttttttagca cttgccagct cctgtgcctg gactgattga 1800
tttgagtact tttttccccc tttccttgtg tcatttaccc tcccacttcc tcctgccttc 1860
cagcacccct ggatgaatgg gctttgtaat tttaactgtt gtattttgtg aatttgttgt 1920
tactgttttt ctgtgaagca catacattgt atgtgggagg taaaggggca ttccagttgc 1980
tccagtcact ccctctatag ccatcactgt cttgttttct gtaactcagg ttaggttttg 2040
gtctctattg ctctgctgca gaaaaggaaa gaaaggagtg ggggaaatgg agcctgaaga 2100
gttggggcag atagacctca gccaaactgg ctgggttttg aggagtcatg ttctttcttc 2160
cccttgaagg ggaaagagtt ttttccactg gtccatttaa agtttcccag ctatggggtg 2220
gtaccagttc tggacaagtg ccactgcatc atagtatgct cggagaatct gaaccttact 2280

ctgaagatga aatttactgt tggccactgc caggtcagac tggtgtttta aggaatactg 2340

ggtgcttcat ataggaactg aaggggtaaa cttactaaac cattcaacct gtgattggtg 2400

atgttttcct gtcattttaa gagtcgacac atgggtgggg gggcagatgt aaaaaaactt 2460

gtacaatttt aaaatatcac aattaaacgt gagctggttt cccaaaa 2507

<210> 208
<211> 181
<212> PRT
<213> Artificial

<400> 208

Met Met Leu Gln His Pro Gly Gln Val Ser Ala Ser Glu Val Ser Ala
1               5                   10                  15

Thr Ala Ile Val Pro Cys Leu Ser Pro Pro Gly Ser Leu Val Phe Glu
            20                  25                  30

Asp Phe Ala Asn Leu Thr Pro Phe Val Lys Glu Glu Leu Arg Phe Ala
        35                  40                  45

Ile Gln Asn Lys His Leu Cys His Arg Met Ser Ser Ala Leu Glu Ser
    50                  55                  60

Val Thr Val Asn Asn Arg Pro Leu Glu Met Ser Val Thr Lys Ser Glu
65                  70                  75                  80

Ala Ala Pro Glu Glu Asp Glu Arg Lys Arg Arg Arg Arg Glu Arg Asn
                85                  90                  95

Lys Ile Ala Ala Ala Lys Cys Arg Asn Lys Lys Lys Glu Lys Thr Glu
            100                 105                 110

Cys Leu Gln Lys Glu Ser Glu Lys Leu Glu Ser Val Asn Ala Glu Leu
        115                 120                 125

Lys Ala Gln Ile Glu Glu Leu Lys Asn Glu Lys Gln His Leu Ile Tyr
    130                 135                 140

Met Leu Asn Leu His Arg Pro Thr Cys Ile Val Arg Ala Gln Asn Gly
145                 150                 155                 160

Arg Thr Pro Glu Asp Glu Arg Asn Leu Phe Ile Gln Gln Ile Lys Glu
                165                 170                 175

Gly Thr Leu Gln Ser
            180

<210> 209

<211> 971
<212> PRT
<213> Artificial

<400> 209

Met Asp Ser Ala Glu Thr Glu Leu Thr Pro Ala Pro Glu Gly Arg Lys
1 5 10 15

Arg Tyr Ser Asp Ile Phe Gln Ser Leu Asp Asn Leu Glu Ile Ser Leu
20 25 30

Gly Asn Val Thr Phe Asp Pro Leu Ala Gly Asp Pro Val Arg Arg Glu
35 40 45

Asp Leu Glu Pro Asp Lys Ala Asp Thr Ala Thr Val Val Thr Glu Glu
50 55 60

Asn Ser Glu Ala Ser Ser Trp Arg Asp Leu Ser Pro Glu Gly Pro Ala
65 70 75 80

Pro Leu Thr Glu Glu Glu Leu Asp Leu Arg Leu Ile Arg Thr Lys Gly
85 90 95

Gly Val Asp Ala Ala Leu Glu Tyr Ala Lys Ala Trp Ser Arg Tyr Ala
100 105 110

Lys Glu Leu Leu Ala Trp Thr Asp Lys Arg Ala Asn Tyr Glu Leu Glu
115 120 125

Phe Ala Lys Ser Ile Met Lys Leu Ala Glu Ala Gly Lys Val Ser Ile
130 135 140

Leu Gln Gln Ser Gln Met Pro Leu Gln Tyr Ile Tyr Thr Leu Phe Leu
145 150 155 160

Glu His Asp Leu Ser Leu Gly Ala Leu Ala Leu Glu Thr Val Ala Gln
165 170 175

Gln Lys Arg Asp Tyr Tyr Gln Pro Leu Ala Ala Lys Arg Met Glu Ile
180 185 190

Glu Lys Trp Arg Lys Glu Phe Lys Glu Gln Trp Leu Lys Glu Gln Lys
195 200 205

Arg Met Asn Glu Ala Val Gln Ala Leu Arg Arg Ser Glu Leu Gln Tyr
210 215 220

Ile Gln Arg Arg Glu Asp Leu Arg Ala Arg Ser Gln Gly Ser Pro Glu
225 230 235 240

Asp Pro Pro Ser Gln Ala Ser Pro Gly Ser Asn Lys Gln Gln Glu Arg
245 250 255

Arg Arg Arg Ser Arg Glu Glu Ala Gln Ala Lys Ala His Glu Ala Glu
260 265 270

Ala Leu Tyr Gln Ala Cys Val Arg Glu Ala Asn Ser Arg Gln Gln Asp
275 280 285

Leu Glu Thr Thr Lys Arg Arg Ile Val Ser His Val Arg Lys Leu Val
290 295 300

Leu Gln Gly Asp Glu Val Leu Arg Arg Val Thr Leu Gly Leu Phe Glu
305 310 315 320

Leu Arg Gly Ala Gln Ala Glu Arg Gly Pro Arg Ser Phe Ser Ala Leu
325 330 335

Ala Glu Cys Cys Val Pro Phe Glu Pro Gly Gln Arg Tyr Gln Glu Phe
340 345 350

Val Arg Thr Leu Gln Pro Gly Ala Pro Pro Pro Pro Ser Pro Ala Phe
355 360 365

Cys Phe Gln Glu Phe Thr Ala Val Val His Ser Phe Pro Gln Asp Thr
370 375 380

Lys Lys Lys Phe Ser Gly Pro Leu Pro Pro Arg Leu Glu Glu Glu Gly
385 390 395 400

Ser Pro Glu Pro Gly Pro Trp Glu Val Ala Ser Leu Gly Ser Gln Gly
405 410 415

Ile Pro Gly Ser Asp Val Asp Ser Val Gly Gly Gly Ser Glu Ser Arg
420 425 430

Ser Leu Asp Ser Pro Thr Ser Ser Pro Gly Ala Gly Ala Arg Arg Leu
435 440 445

Val Lys Ala Ser Ser Thr Gly Thr Glu Ser Ser Asp Asp Phe Glu Glu
450 455 460

Arg Asp Pro Asp Leu Gly Asp Gly Ile Glu Asn Gly Val Gly Ser Pro
465 470 475 480

Phe Arg Lys Trp Thr Leu Ser Thr Ala Ala Gln Thr His Arg Leu Arg
485 490 495

Arg Leu Arg Gly Pro Ala Lys Cys Arg Glu Cys Glu Ala Phe Met Val
500 505 510

Ser Gly Thr Glu Cys Glu Glu Cys Phe Leu Thr Cys His Lys Arg Cys
515 520 525

Leu Glu Thr Leu Leu Ile Leu Cys Gly His Arg Arg Leu Pro Ala Arg
530 535 540

Met Ser Leu Phe Gly Val Asp Phe Leu Gln Leu Pro Arg Asp Phe Pro
545 550 555 560

Glu Glu Val Pro Phe Val Ile Thr Arg Cys Thr Ala Glu Ile Glu His
565 570 575

Arg Ala Leu Gly Leu Gln Gly Ile Tyr Arg Val Ser Gly Ser Arg Val
580 585 590

Arg Val Glu Arg Leu Cys Gln Ala Phe Glu Asn Gly Arg Ala Leu Val
595 600 605

Glu Leu Ser Gly Asn Ser Pro His Asp Ile Thr Ser Val Leu Lys Arg
610 615 620

Phe Leu Gln Glu Leu Thr Asp Pro Val Val Pro Phe His Leu Tyr Asp
625 630 635 640

Ala Phe Ile Ser Leu Ala Lys Thr Leu His Ala Asp Pro Gly Asp Asp
645 650 655

Pro Gly Thr Pro Asn Pro Ser Pro Glu Ile Ile Arg Ser Leu Lys Thr
660 665 670

Leu Leu Val Gln Leu Pro Asp Ser Asn Tyr Ser Thr Leu Arg His Leu
675 680 685

Val Ala His Leu Phe Arg Val Ala Ala Arg Phe Glu Glu Asn Lys Met
690 695 700

Ser Ala Asn Asn Leu Gly Ile Val Phe Gly Pro Thr Leu Leu Arg Pro
705 710 715 720

Pro Asp Gly Pro Arg Ala Thr Gly Ala Ser Pro Val Ala Cys Leu Leu
725 730 735

Asp Ser Gly His Gln Ala Gln Leu Val Glu Phe Leu Ile Val His Tyr
740 745 750

Glu Gln Ile Phe Gly Met Asp Glu Leu Pro Leu Ala Ser Glu Pro Leu
755 760 765

Thr Gln Asp Pro Gly Leu Ala Pro Ala Cys Leu Glu Ser Ser Pro Gln
770 775 780

His Pro Ala Ser Leu Leu Ala Gln Asp Thr Gln Pro Leu Thr Ile Ala
785 790 795 800

Leu Asp Ser Ser Pro Asp Pro Lys His His Ser Ala Leu Glu Lys Cys
805 810 815

Pro Glu Val Thr Pro Pro Glu Leu Ala Thr Leu Gln Arg Asp Gln Arg
820 825 830

Glu Glu Glu Val Glu Asp Thr Arg Asp Gly Ala Gly Asp Gly Ser Ser
835 840 845

His Cys Pro Glu Asp Leu Ala Leu Gly Ala Gln Ser Arg Gly His Phe
850 855 860

Ser Arg Gln Pro Val Lys Tyr Ser Arg Gly Gly Val Arg Pro Val Thr
865 870 875 880

His Gln Leu Ser Ser Leu Ala Leu Val Ala Ser Lys Leu Cys Glu Glu
885 890 895

Thr Pro Val Thr Val Ser Ala Val His Arg Gly Ser Leu Arg Val Arg
900 905 910

Gly Leu Gly Pro Ala Ala Ala Cys Pro Glu Gly Ser Pro Leu Arg Arg
915 920 925

Asn Pro Leu Pro Lys His Phe Glu Ile Thr Gln Glu Thr Ala Arg Leu
930 935 940

Leu Ser Lys Leu Asn Ser Asp Ala Val Ser Arg Thr Thr Cys Cys Ala
945 950 955 960

Asp Pro Glu Pro Glu Glu Ser Glu Glu His Leu
965 970

<210> 210
<211> 408
<212> PRT
<213> Artificial

<400> 210

Met Asp Ser Ala Glu Thr Glu Leu Thr Pro Ala Pro Glu Gly Arg Lys
1 5 10 15

Arg Tyr Ser Asp Ile Phe Gln Ser Leu Asp Asn Leu Glu Ile Ser Leu
20 25 30

Gly Asn Val Thr Phe Asp Pro Leu Ala Gly Asp Pro Val Arg Arg Glu
35 40 45

Asp Leu Glu Pro Asp Lys Ala Asp Thr Ala Thr Val Val Thr Glu Glu
50 55 60

Asn Ser Glu Ala Ser Ser Trp Arg Asp Leu Ser Pro Glu Gly Pro Ala
65 70 75 80

Pro Leu Thr Glu Glu Glu Leu Asp Leu Arg Leu Ile Arg Thr Lys Gly
85 90 95

Gly Val Asp Ala Ala Leu Glu Tyr Ala Lys Ala Trp Ser Arg Tyr Ala
100 105 110

Lys Glu Leu Leu Ala Trp Thr Asp Lys Arg Ala Asn Tyr Glu Leu Glu
115 120 125

Phe Ala Lys Ser Ile Met Lys Leu Ala Glu Ala Gly Lys Val Ser Ile
130 135 140

Leu Gln Gln Ser Gln Met Pro Leu Gln Tyr Ile Tyr Thr Leu Phe Leu
145 150 155 160

Glu His Asp Leu Ser Leu Gly Ala Leu Ala Leu Glu Thr Val Ala Gln
165 170 175

Gln Lys Arg Asp Tyr Tyr Gln Pro Leu Ala Ala Lys Arg Met Glu Ile
180 185 190

Glu Lys Trp Arg Lys Glu Phe Lys Glu Gln Trp Leu Lys Glu Gln Lys
195 200 205

Arg Met Asn Glu Ala Val Gln Ala Leu Arg Arg Ser Glu Leu Gln Tyr
210 215 220

Ile Gln Arg Arg Glu Asp Leu Arg Ala Arg Ser Gln Gly Ser Pro Glu
225 230 235 240

Asp Pro Pro Ser Gln Ala Ser Pro Gly Ser Asn Lys Gln Gln Glu Arg
245 250 255

Arg Arg Arg Ser Arg Glu Glu Ala Gln Ala Lys Ala His Glu Ala Glu
260 265 270

Ala Leu Tyr Gln Ala Cys Val Arg Glu Ala Asn Ser Arg Gln Gln Asp
275 280 285

Leu Glu Thr Thr Lys Arg Arg Ile Val Ser His Val Arg Lys Leu Val
290 295 300

Leu Gln Gly Asp Glu Val Leu Arg Arg Val Thr Leu Gly Leu Phe Glu
305 310 315 320

Leu Arg Gly Ala Gln Ala Glu Arg Gly Pro Arg Ser Phe Ser Ala Leu
325 330 335

Ala Glu Cys Cys Val Pro Phe Glu Pro Gly Gln Arg Tyr Gln Glu Phe
340 345 350

Val Arg Thr Leu Gln Pro Gly Ala Pro Pro Pro Pro Ser Pro Ala Phe
355 360 365

Cys Phe Gln Glu Phe Thr Ala Val Val His Arg Thr Gln Lys Arg Ser
370 375 380

Phe Arg Gly Leu Tyr Leu Gln Gly Trp Arg Arg Arg Val Pro Leu Ser
385 390 395 400

Leu Ala Leu Gly Arg Leu Pro Ala
405

<210> 211
<211> 846
<212> PRT
<213> Artificial

<400> 211

Met Asp Ser Ala Glu Thr Glu Leu Thr Pro Ala Pro Glu Gly Arg Lys
1 5 10 15

Arg Tyr Ser Asp Ile Phe Gln Ser Leu Asp Asn Leu Glu Ile Ser Leu
20 25 30

Gly Asn Val Thr Phe Asp Pro Leu Ala Gly Asp Pro Val Arg Arg Glu
35 40 45

Asp Leu Glu Pro Asp Lys Ala Asp Thr Ala Thr Val Val Thr Glu Glu
50 55 60

Asn Ser Glu Ala Ser Ser Trp Arg Asp Leu Ser Pro Glu Gly Pro Ala
65 70 75 80

Pro Leu Thr Glu Glu Glu Leu Asp Leu Arg Leu Ile Arg Thr Lys Gly
85 90 95

Gly Val Asp Ala Ala Leu Glu Tyr Ala Lys Ala Trp Ser Arg Tyr Ala
100 105 110

Lys Glu Leu Leu Ala Trp Thr Asp Lys Arg Ala Asn Tyr Glu Leu Glu
115 120 125

Phe Ala Lys Ser Ile Met Lys Leu Ala Glu Ala Gly Lys Val Ser Ile
130 135 140

Leu Gln Gln Ser Gln Met Pro Leu Gln Tyr Ile Tyr Thr Leu Phe Leu
145 150 155 160

```
Glu His Asp Leu Ser Leu Gly Ala Leu Ala Leu Glu Thr Val Ala Gln
                165                 170                 175

Gln Lys Arg Asp Tyr Tyr Gln Pro Leu Ala Ala Lys Arg Met Glu Ile
            180                 185                 190

Glu Lys Trp Arg Lys Glu Phe Lys Glu Gln Trp Leu Lys Glu Gln Lys
        195                 200                 205

Arg Met Asn Glu Ala Val Gln Ala Leu Arg Arg Ser Glu Leu Gln Tyr
    210                 215                 220

Ile Gln Arg Arg Glu Asp Leu Arg Ala Arg Ser Gln Gly Ser Pro Glu
225                 230                 235                 240

Asp Pro Pro Ser Gln Ala Ser Pro Gly Ser Asn Lys Gln Gln Glu Arg
                245                 250                 255

Arg Arg Arg Ser Arg Glu Glu Ala Gln Ala Lys Ala His Glu Ala Glu
            260                 265                 270

Ala Leu Tyr Gln Ala Cys Val Arg Glu Ala Asn Ser Arg Gln Gln Asp
        275                 280                 285

Leu Glu Thr Thr Lys Arg Arg Ile Val Ser His Val Arg Lys Leu Val
    290                 295                 300

Leu Gln Gly Asp Glu Val Leu Arg Arg Val Thr Leu Gly Leu Phe Glu
305                 310                 315                 320

Leu Arg Gly Ala Gln Ala Glu Arg Gly Pro Arg Ser Phe Ser Ala Leu
                325                 330                 335

Ala Glu Cys Cys Val Pro Phe Glu Pro Gly Gln Arg Tyr Gln Glu Phe
            340                 345                 350

Val Arg Thr Leu Gln Pro Gly Ala Pro Pro Pro Pro Ser Pro Ala Phe
        355                 360                 365

Cys Phe Gln Glu Phe Thr Ala Val Val His Ser Phe Pro Gln Asp Thr
    370                 375                 380

Lys Lys Lys Phe Ser Gly Pro Leu Pro Pro Arg Leu Glu Glu Glu Gly
385                 390                 395                 400

Ser Pro Glu Pro Gly Pro Trp Glu Val Ala Ser Leu Gly Ser Gln Gly
                405                 410                 415

Ile Pro Gly Ser Asp Val Asp Ser Val Gly Gly Gly Ser Glu Ser Arg
            420                 425                 430
```

Ser Leu Asp Ser Pro Thr Ser Ser Pro Gly Ala Gly Ala Arg Arg Leu
435 440 445

Val Lys Ala Ser Ser Thr Gly Thr Glu Ser Ser Asp Asp Phe Glu Glu
450 455 460

Arg Asp Pro Asp Leu Gly Asp Gly Ile Glu Asn Gly Val Gly Ser Pro
465 470 475 480

Phe Arg Lys Trp Thr Leu Ser Thr Ala Ala Gln Thr His Arg Leu Arg
485 490 495

Arg Leu Arg Gly Pro Ala Lys Cys Arg Glu Cys Glu Ala Phe Met Val
500 505 510

Ser Gly Thr Glu Cys Glu Glu Cys Phe Leu Thr Cys His Lys Arg Cys
515 520 525

Leu Glu Thr Leu Leu Ile Leu Cys Gly His Arg Arg Leu Pro Ala Arg
530 535 540

Met Ser Leu Phe Gly Val Asp Phe Leu Gln Leu Pro Arg Asp Phe Pro
545 550 555 560

Glu Glu Val Pro Phe Val Ile Thr Arg Cys Thr Ala Glu Ile Glu His
565 570 575

Arg Ala Leu Gly Leu Gln Gly Ile Tyr Arg Val Ser Gly Ser Arg Val
580 585 590

Arg Val Glu Arg Leu Cys Gln Ala Phe Glu Asn Gly Arg Ala Leu Val
595 600 605

Glu Leu Ser Gly Asn Ser Pro His Asp Ile Thr Ser Val Leu Lys Arg
610 615 620

Phe Leu Gln Glu Leu Thr Asp Pro Val Val Pro Phe His Leu Tyr Asp
625 630 635 640

Ala Phe Ile Ser Leu Ala Lys Thr Leu His Ala Asp Pro Gly Asp Asp
645 650 655

Pro Gly Thr Pro Asn Pro Ser Pro Glu Ile Ile Arg Ser Leu Lys Thr
660 665 670

Leu Leu Val Gln Leu Pro Asp Ser Asn Tyr Ser Thr Leu Arg His Leu
675 680 685

Val Ala His Leu Phe Arg Val Ala Ala Arg Phe Glu Glu Asn Lys Met
690 695 700

Ser Ala Asn Asn Leu Gly Ile Val Phe Gly Pro Thr Leu Leu Arg Pro
705 710 715 720

Pro Asp Gly Pro Arg Ala Thr Gly Ala Ser Pro Val Ala Cys Leu Leu
725 730 735

Asp Ser Gly His Gln Ala Gln Leu Val Glu Phe Leu Ile Val His Tyr
740 745 750

Glu Gln Ile Phe Gly Met Asp Glu Leu Pro Leu Ala Ser Glu Pro Leu
755 760 765

Thr Gln Asp Pro Gly Leu Ala Pro Ala Cys Leu Glu Ser Ser Pro Gln
770 775 780

His Pro Ala Ser Leu Leu Ala Gln Asp Thr Gln Pro Leu Thr Ile Ala
785 790 795 800

Leu Asp Ser Ser Pro Asp Pro Lys His His Ser Ala Leu Glu Lys Cys
805 810 815

Pro Glu Val Thr Pro Pro Glu Gly Pro Ala Thr Ala Leu Arg Thr Trp
820 825 830

Pro Trp Glu His Asn Pro Gly Ala Thr Ser Ala Ala Ser Gln
835 840 845

<210> 212
<211> 843
<212> PRT
<213> Artificial

<400> 212

Met Asp Ser Ala Glu Thr Glu Leu Thr Pro Ala Pro Glu Gly Arg Lys
1 5 10 15

Arg Tyr Ser Asp Ile Phe Gln Ser Leu Asp Asn Leu Glu Ile Ser Leu
20 25 30

Gly Asn Val Thr Phe Asp Pro Leu Ala Gly Asp Pro Val Arg Arg Glu
35 40 45

Asp Leu Glu Pro Asp Lys Ala Asp Thr Ala Thr Val Val Thr Glu Glu
50 55 60

Asn Ser Glu Ala Ser Ser Trp Arg Asp Leu Ser Pro Glu Gly Pro Ala
65 70 75 80

Pro Leu Thr Glu Glu Glu Leu Asp Leu Arg Leu Ile Arg Thr Lys Gly
85 90 95

Gly Val Asp Ala Ala Leu Glu Tyr Ala Lys Ala Trp Ser Arg Tyr Ala
100 105 110

Lys Glu Leu Leu Ala Trp Thr Asp Lys Arg Ala Asn Tyr Glu Leu Glu
115 120 125

Phe Ala Lys Ser Ile Met Lys Leu Ala Glu Ala Gly Lys Val Ser Ile
130 135 140

Leu Gln Gln Ser Gln Met Pro Leu Gln Tyr Ile Tyr Thr Leu Phe Leu
145 150 155 160

Glu His Asp Leu Ser Leu Gly Ala Leu Ala Leu Glu Thr Val Ala Gln
165 170 175

Gln Lys Arg Asp Tyr Tyr Gln Pro Leu Ala Ala Lys Arg Met Glu Ile
180 185 190

Glu Lys Trp Arg Lys Glu Phe Lys Glu Gln Trp Leu Lys Glu Gln Lys
195 200 205

Arg Met Asn Glu Ala Val Gln Ala Leu Arg Arg Ser Glu Leu Gln Tyr
210 215 220

Ile Gln Arg Arg Glu Asp Leu Arg Ala Arg Ser Gln Gly Ser Pro Glu
225 230 235 240

Asp Pro Pro Ser Gln Ala Ser Pro Gly Ser Asn Lys Gln Gln Glu Arg
245 250 255

Arg Arg Arg Ser Arg Glu Glu Ala Gln Ala Lys Ala His Glu Ala Glu
260 265 270

Ala Leu Tyr Gln Ala Cys Val Arg Glu Ala Asn Ser Arg Gln Gln Asp
275 280 285

Leu Glu Thr Thr Lys Arg Arg Ile Val Ser His Val Arg Lys Leu Val
290 295 300

Leu Gln Gly Asp Glu Val Leu Arg Arg Val Thr Leu Gly Leu Phe Glu
305 310 315 320

Leu Arg Gly Ala Gln Ala Glu Arg Gly Pro Arg Ser Phe Ser Ala Leu
325 330 335

Ala Glu Cys Cys Val Pro Phe Glu Pro Gly Gln Arg Tyr Gln Glu Phe
340 345 350

Val Arg Thr Leu Gln Pro Gly Ala Pro Pro Pro Pro Ser Pro Ala Phe
355 360 365

Cys Phe Gln Glu Phe Thr Ala Val Val His Ser Phe Pro Gln Asp Thr
370 375 380

Lys Lys Lys Phe Ser Gly Pro Leu Pro Pro Arg Leu Glu Glu Glu Gly
385 390 395 400

Ser Pro Glu Pro Gly Pro Trp Glu Val Ala Ser Leu Gly Ser Gln Gly
405 410 415

Ile Pro Gly Ser Asp Val Asp Ser Val Gly Gly Gly Ser Glu Ser Arg
420 425 430

Ser Leu Asp Ser Pro Thr Ser Ser Pro Gly Ala Gly Ala Arg Arg Leu
435 440 445

Val Lys Ala Ser Ser Thr Gly Thr Glu Ser Ser Asp Asp Phe Glu Glu
450 455 460

Arg Asp Pro Asp Leu Gly Asp Gly Ile Glu Asn Gly Val Gly Ser Pro
465 470 475 480

Phe Arg Lys Trp Thr Leu Ser Thr Ala Ala Gln Thr His Arg Leu Arg
485 490 495

Arg Leu Arg Gly Pro Ala Lys Cys Arg Glu Cys Glu Ala Phe Met Val
500 505 510

Ser Gly Thr Glu Cys Glu Glu Cys Phe Leu Thr Cys His Lys Arg Cys
515 520 525

Leu Glu Thr Leu Leu Ile Leu Cys Gly His Arg Arg Leu Pro Ala Arg
530 535 540

Met Ser Leu Phe Gly Val Asp Phe Leu Gln Leu Pro Arg Asp Phe Pro
545 550 555 560

Glu Glu Val Pro Phe Val Ile Thr Arg Cys Thr Ala Glu Ile Glu His
565 570 575

Arg Ala Leu Gly Leu Gln Gly Ile Tyr Arg Val Ser Gly Ser Arg Val
580 585 590

Arg Val Glu Arg Leu Cys Gln Ala Phe Glu Asn Gly Arg Ala Leu Val
595 600 605

Glu Leu Ser Gly Asn Ser Pro His Asp Ile Thr Ser Val Leu Lys Arg
610 615 620

Phe Leu Gln Glu Leu Thr Asp Pro Val Val Pro Phe His Leu Tyr Asp
625 630 635 640

Ala Phe Ile Ser Leu Ala Lys Thr Leu His Ala Asp Pro Gly Asp Asp
645 650 655

Pro Gly Thr Pro Asn Pro Ser Pro Glu Ile Ile Arg Ser Leu Lys Thr
660 665 670

Leu Leu Val Gln Leu Pro Asp Ser Asn Tyr Ser Thr Leu Arg His Leu
675 680 685

Val Ala His Leu Phe Arg Val Ala Ala Arg Phe Glu Glu Asn Lys Met
690 695 700

Ser Ala Asn Asn Leu Gly Ile Val Phe Gly Pro Thr Leu Leu Arg Pro
705 710 715 720

Pro Asp Gly Pro Arg Ala Thr Gly Ala Ser Pro Val Ala Cys Leu Leu
725 730 735

Asp Ser Gly His Gln Ala Gln Leu Val Glu Phe Leu Ile Val His Tyr
740 745 750

Glu Gln Ile Phe Gly Met Asp Glu Leu Pro Leu Ala Ser Glu Pro Leu
755 760 765

Thr Gln Asp Pro Gly Leu Ala Pro Ala Cys Leu Glu Ser Ser Pro Gln
770 775 780

His Pro Ala Ser Leu Leu Ala Gln Asp Thr Gln Pro Leu Thr Ile Ala
785 790 795 800

Leu Asp Ser Ser Pro Asp Pro Lys His His Ser Ala Leu Glu Lys Cys
805 810 815

Pro Glu Val Thr Pro Pro Glu Phe Trp Phe Phe Glu Thr Gly Phe Leu
820 825 830

Cys Val Ala Leu Ala Val Leu Glu Leu Thr Leu
835 840

<210> 213
<211> 633
<212> PRT
<213> Artificial

<400> 213

Met Asp Ser Ala Glu Thr Glu Leu Thr Pro Ala Pro Glu Gly Arg Lys
1 5 10 15

Arg Tyr Ser Asp Ile Phe Gln Ser Leu Asp Asn Leu Glu Ile Ser Leu
20 25 30

Gly Asn Val Thr Phe Asp Pro Leu Ala Gly Asp Pro Val Arg Arg Glu
35 40 45

Asp Leu Glu Pro Asp Lys Ala Asp Thr Ala Thr Val Val Thr Glu Glu
50 55 60

Asn Ser Glu Ala Ser Ser Trp Arg Asp Leu Ser Pro Glu Gly Pro Ala
65 70 75 80

Pro Leu Thr Glu Glu Glu Leu Asp Leu Arg Leu Ile Arg Thr Lys Gly
85 90 95

Gly Val Asp Ala Ala Leu Glu Tyr Ala Lys Ala Trp Ser Arg Tyr Ala
100 105 110

Lys Glu Leu Leu Ala Trp Thr Asp Lys Arg Ala Asn Tyr Glu Leu Glu
115 120 125

Phe Ala Lys Ser Ile Met Lys Leu Ala Glu Ala Gly Lys Val Ser Ile
130 135 140

Leu Gln Gln Ser Gln Met Pro Leu Gln Tyr Ile Tyr Thr Leu Phe Leu
145 150 155 160

Glu His Asp Leu Ser Leu Gly Ala Leu Ala Leu Glu Thr Val Ala Gln
165 170 175

Gln Lys Arg Asp Tyr Tyr Gln Pro Leu Ala Ala Lys Arg Met Glu Ile
180 185 190

Glu Lys Trp Arg Lys Glu Phe Lys Glu Gln Trp Leu Lys Glu Gln Lys
195 200 205

Arg Met Asn Glu Ala Val Gln Ala Leu Arg Arg Ser Glu Leu Gln Tyr
210 215 220

Ile Gln Arg Arg Glu Asp Leu Arg Ala Arg Ser Gln Gly Ser Pro Glu
225 230 235 240

Asp Pro Pro Ser Gln Ala Ser Pro Gly Ser Asn Lys Gln Gln Glu Arg
245 250 255

Arg Arg Arg Ser Arg Glu Glu Ala Gln Ala Lys Ala His Glu Ala Glu
260 265 270

Ala Leu Tyr Gln Ala Cys Val Arg Glu Ala Asn Ser Arg Gln Gln Asp
275 280 285

Leu Glu Thr Thr Lys Arg Arg Ile Val Ser His Val Arg Lys Leu Val
290 295 300

Leu Gln Gly Asp Glu Val Leu Arg Arg Val Thr Leu Gly Leu Phe Glu
305 310 315 320

Leu Arg Gly Ala Gln Ala Glu Arg Gly Pro Arg Ser Phe Ser Ala Leu
325 330 335

Ala Glu Cys Cys Val Pro Phe Glu Pro Gly Gln Arg Tyr Gln Glu Phe
340 345 350

Val Arg Thr Leu Gln Pro Gly Ala Pro Pro Pro Pro Ser Pro Ala Phe
355 360 365

Cys Phe Gln Glu Phe Thr Ala Val Val His Ser Phe Pro Gln Asp Thr
370 375 380

Lys Lys Lys Phe Ser Gly Pro Leu Pro Pro Arg Leu Glu Glu Glu Gly
385 390 395 400

Ser Pro Glu Pro Gly Pro Trp Glu Val Ala Ser Leu Gly Ser Gln Gly
405 410 415

Ile Pro Gly Ser Asp Val Asp Ser Val Gly Gly Gly Ser Glu Ser Arg
420 425 430

Ser Leu Asp Ser Pro Thr Ser Ser Pro Gly Ala Gly Ala Arg Arg Leu
435 440 445

Val Lys Ala Ser Ser Thr Gly Thr Glu Ser Ser Asp Asp Phe Glu Glu
450 455 460

Arg Asp Pro Asp Leu Gly Asp Gly Ile Glu Asn Gly Val Gly Ser Pro
465 470 475 480

Phe Arg Lys Trp Thr Leu Ser Thr Ala Ala Gln Thr His Arg Leu Arg
485 490 495

Arg Leu Arg Gly Pro Ala Lys Cys Arg Glu Cys Glu Ala Phe Met Val
500 505 510

Ser Gly Thr Glu Cys Glu Glu Cys Phe Leu Thr Cys His Lys Arg Cys
515 520 525

Leu Glu Thr Leu Leu Ile Leu Cys Gly His Arg Arg Leu Pro Ala Arg
530 535 540

Met Ser Leu Phe Gly Val Asp Phe Leu Gln Leu Pro Arg Asp Phe Pro
545 550 555 560

Glu Glu Val Pro Phe Val Ile Thr Arg Cys Thr Ala Glu Ile Glu His
565 570 575

```
Arg Ala Leu Gly Leu Gln Gly Ile Tyr Arg Val Ser Gly Ser Arg Val
            580                 585                 590

Arg Val Glu Arg Leu Cys Gln Ala Phe Glu Asn Gly Arg Ala Leu Val
        595                 600                 605

Glu Leu Ser Gly Asn Ser Pro His Asp Ile Thr Ser Val Leu Lys Arg
    610                 615                 620

Phe Leu Gln Glu Gly Gly Cys Ser Val
625                 630
```

<210> 214
<211> 78
<212> PRT
<213> Artificial

<400> 214

```
Met Asp Ser Ala Glu Thr Glu Leu Thr Pro Ala Pro Glu Gly Arg Lys
1               5                   10                  15

Arg Tyr Ser Asp Ile Phe Gln Ser Leu Asp Asn Leu Glu Ile Ser Leu
            20                  25                  30

Gly Asn Val Thr Phe Asp Pro Leu Ala Gly Asp Pro Val Arg Arg Glu
        35                  40                  45

Asp Leu Glu Pro Asp Lys Ala Asp Thr Ala Thr Val Val Thr Glu Glu
    50                  55                  60

Arg Lys Asn Trp Ile Cys Asp Ser Phe Gly Pro Arg Val Val
65                  70                  75
```

<210> 215
<211> 303
<212> PRT
<213> Artificial

<400> 215

```
Met Ala Pro Pro Ala Leu Gln Ala Gln Pro Pro Gly Gly Ser Gln Leu
1               5                   10                  15

Arg Phe Leu Leu Phe Leu Leu Leu Leu Leu Leu Leu Leu Ser Trp Pro
            20                  25                  30

Ser Gln Gly Asp Ala Leu Ala Met Pro Glu Gln Arg Arg Ser Gly Pro
        35                  40                  45

Glu Ser Gln Leu Asn Ala Asp Glu Leu Arg Gly Arg Phe Gln Asp Leu
    50                  55                  60
```

```
Leu Ser Arg Leu His Ala Asn Gln Ser Arg Glu Asp Ser Asn Ser Glu
65                  70                  75                  80

Pro Ser Pro Asp Pro Ala Val Arg Ile Leu Ser Pro Glu Val Arg Leu
                85                  90                  95

Gly Ser His Gly Gln Leu Leu Leu Arg Val Asn Arg Ala Ser Leu Ser
            100                 105                 110

Gln Gly Leu Pro Glu Ala Tyr Arg Val His Arg Ala Leu Leu Leu Leu
        115                 120                 125

Thr Pro Thr Thr Arg Pro Trp Asp Ile Thr Arg Pro Leu Lys Arg Ala
    130                 135                 140

Leu Ser Leu Gln Gly Pro Arg Ala Pro Ala Leu Arg Leu Arg Leu Thr
145                 150                 155                 160

Pro Pro Pro Asp Leu Ala Met Leu Pro Ser Gly Gly Ala Gln Leu Glu
                165                 170                 175

Leu Arg Leu Arg Val Ala Ala Gly Arg Gly Arg Arg Ser Ala His Ala
            180                 185                 190

His Pro Arg Asp Ser Cys Pro Leu Gly Pro Gly Arg Cys Cys His Leu
        195                 200                 205

Glu Thr Val Gln Ala Thr Leu Glu Asp Leu Gly Trp Ser Asp Trp Val
    210                 215                 220

Leu Ser Pro Arg Gln Leu Gln Leu Ser Met Cys Val Gly Glu Cys Pro
225                 230                 235                 240

His Leu Tyr Arg Ser Ala Asn Thr His Ala Gln Ile Lys Ala Arg Leu
                245                 250                 255

His Gly Leu Gln Pro Asp Lys Val Pro Ala Pro Cys Cys Val Pro Ser
            260                 265                 270

Ser Tyr Thr Pro Val Val Leu Met His Arg Thr Asp Ser Gly Val Ser
        275                 280                 285

Leu Gln Thr Tyr Asp Asp Leu Val Ala Arg Gly Cys His Cys Ala
    290                 295                 300
```

<210> 216
<211> 159
<212> PRT
<213> Artificial

<400> 216

Met Ser Asn Pro Gly Asp Val Arg Pro Val Pro His Arg Ser Lys Val
1               5                   10                  15

Cys Arg Cys Leu Phe Gly Pro Val Asp Ser Glu Gln Leu Arg Arg Asp
            20                  25                  30

Cys Asp Ala Leu Met Ala Gly Cys Leu Gln Glu Ala Arg Glu Arg Trp
        35                  40                  45

Asn Phe Asp Phe Val Thr Glu Thr Pro Leu Glu Gly Asn Phe Val Trp
    50                  55                  60

Glu Arg Val Arg Ser Leu Gly Leu Pro Lys Val Tyr Leu Ser Pro Gly
65                  70                  75                  80

Ser Arg Ser Arg Asp Asp Leu Gly Gly Asp Lys Arg Pro Ser Thr Ser
                85                  90                  95

Ser Ala Leu Leu Gln Gly Pro Ala Pro Glu Asp His Val Ala Leu Ser
            100                 105                 110

Leu Ser Cys Thr Leu Val Ser Glu Arg Pro Glu Asp Ser Pro Gly Gly
        115                 120                 125

Pro Gly Thr Ser Gln Gly Arg Lys Arg Arg Gln Thr Ser Leu Thr Asp
    130                 135                 140

Phe Tyr His Ser Lys Arg Arg Leu Val Phe Cys Lys Arg Lys Pro
145                 150                 155

<210> 217
<211> 294
<212> PRT
<213> Artificial

<400> 217

Met Ile Glu Val Leu Thr Thr Asp Ser Gln Lys Leu Leu His Gln Leu
1               5                   10                  15

Asn Thr Leu Leu Glu Gln Glu Ser Arg Cys Gln Pro Lys Val Cys Gly
            20                  25                  30

Leu Lys Leu Ile Glu Ser Ala His Asp Asn Gly Leu Arg Met Thr Ala
        35                  40                  45

Arg Leu Arg Asp Phe Glu Val Lys Asp Leu Leu Ser Leu Thr Gln Phe
    50                  55                  60

Phe Gly Phe Asp Thr Glu Thr Phe Ser Leu Ala Val Asn Leu Leu Asp
65                  70                  75                  80

Arg Phe Leu Ser Lys Met Lys Val Gln Ala Lys His Leu Gly Cys Val
85 90 95

Gly Leu Ser Cys Phe Tyr Leu Ala Val Lys Ala Thr Glu Glu Glu Arg
100 105 110

Asn Val Pro Leu Ala Thr Asp Leu Ile Arg Ile Ser Gln Tyr Arg Phe
115 120 125

Thr Val Ser Asp Leu Met Arg Met Glu Lys Ile Val Leu Glu Lys Val
130 135 140

Cys Trp Lys Val Lys Ala Thr Thr Ala Phe Gln Phe Leu Gln Leu Tyr
145 150 155 160

Tyr Ser Leu Val His Asp Thr Leu Pro Phe Glu Arg Arg Asn Asp Leu
165 170 175

Asn Phe Glu Arg Leu Glu Ala Gln Leu Lys Ala Cys His Cys Arg Ile
180 185 190

Ile Phe Ser Lys Ala Lys Pro Ser Val Leu Ala Leu Ser Ile Leu Ala
195 200 205

Leu Glu Ile Gln Ala Leu Lys Tyr Val Glu Leu Thr Glu Gly Val Glu
210 215 220

Cys Ile Gln Lys His Ser Lys Ile Ser Gly Arg Asp Leu Thr Phe Trp
225 230 235 240

Gln Glu Leu Val Ser Lys Cys Leu Thr Glu Tyr Ser Ser Asn Lys Cys
245 250 255

Ser Lys Pro Asn Gly Gln Lys Leu Lys Trp Ile Val Ser Gly Arg Thr
260 265 270

Ala Arg Gln Leu Lys His Ser Tyr Tyr Arg Ile Thr His Leu Pro Thr
275 280 285

Ile Pro Glu Thr Ile Cys
290

<210> 218
<211> 187
<212> PRT
<213> Artificial

<400> 218

Met Ile Glu Val Leu Thr Thr Asp Ser Gln Lys Leu Leu His Gln Leu
1 5 10 15

Asn Thr Leu Leu Glu Gln Glu Ser Arg Cys Gln Pro Lys Val Cys Gly
20 25 30

Leu Lys Leu Ile Glu Ser Ala His Asp Asn Gly Leu Arg Met Thr Ala
35 40 45

Arg Leu Arg Asp Phe Glu Val Lys Asp Leu Leu Ser Leu Thr Gln Phe
50 55 60

Phe Gly Phe Asp Thr Glu Thr Phe Ser Leu Ala Val Asn Leu Leu Asp
65 70 75 80

Arg Phe Leu Ser Lys Met Lys Val Gln Ala Lys His Leu Gly Cys Val
85 90 95

Gly Leu Ser Cys Phe Tyr Leu Ala Val Lys Ala Thr Glu Glu Glu Arg
100 105 110

Asn Val Pro Leu Ala Thr Asp Leu Ile Arg Ile Ser Gln Tyr Arg Phe
115 120 125

Thr Val Ser Asp Leu Met Arg Met Glu Lys Ile Val Leu Glu Lys Val
130 135 140

Cys Trp Lys Val Lys Ala Thr Thr Ala Phe Gln Phe Leu Gln Leu Tyr
145 150 155 160

Tyr Ser Leu Val His Asp Thr Leu Pro Phe Glu Ser Leu Leu Cys Trp
165 170 175

Arg Tyr Leu Ser Leu Arg Trp Arg Ser Lys His
180 185

<210> 219
<211> 203
<212> PRT
<213> Artificial

<400> 219

Met Lys Val Leu Ala Ala Gly Ile Val Pro Leu Leu Leu Leu Val Leu
1 5 10 15

His Trp Lys His Gly Ala Gly Ser Pro Leu Pro Ile Thr Pro Val Asn
20 25 30

Ala Thr Cys Ala Ile Arg His Pro Cys His Gly Asn Leu Met Asn Gln
35 40 45

Ile Lys Asn Gln Leu Ala Gln Leu Asn Gly Ser Ala Asn Ala Leu Phe
50 55 60

Ile Ser Tyr Tyr Thr Ala Gln Gly Glu Pro Phe Pro Asn Asn Val Glu
65 70 75 80

Lys Leu Cys Ala Pro Asn Met Thr Asp Phe Pro Ser Phe His Gly Asn
85 90 95

Gly Thr Glu Lys Thr Lys Leu Val Glu Leu Tyr Arg Met Val Ala Tyr
100 105 110

Leu Ser Ala Ser Leu Thr Asn Ile Thr Arg Asp Gln Lys Val Leu Asn
115 120 125

Pro Thr Ala Val Ser Leu Gln Val Lys Leu Asn Ala Thr Ile Asp Val
130 135 140

Met Arg Gly Leu Leu Ser Asn Val Leu Cys Arg Leu Cys Asn Lys Tyr
145 150 155 160

Arg Val Gly His Val Asp Val Pro Pro Val Pro Asp His Ser Asp Lys
165 170 175

Glu Ala Phe Gln Arg Lys Lys Leu Gly Cys Gln Leu Leu Gly Thr Tyr
180 185 190

Lys Gln Val Ile Ser Val Val Val Gln Ala Phe
195 200

<210> 220
<211> 342
<212> PRT
<213> Artificial

<400> 220

Met Arg Lys Asp Thr Pro Pro Pro Leu Val Pro Pro Ala Ala Arg Glu
1 5 10 15

Trp Asn Leu Pro Pro Asn Ala Pro Ala Cys Met Glu Arg Gln Leu Glu
20 25 30

Ala Ala Arg Tyr Arg Ser Asp Gly Ser Leu Leu Leu Gly Val Ser Ser
35 40 45

Leu Ser Gly Arg Cys Trp Val Gly Ser Leu Trp Phe Phe Lys Asp Pro
50 55 60

Ser Ala Ala Pro Asn Glu Gly Phe Cys Ser Ala Gly Val Gln Thr Glu
65 70 75 80

Ala Gly Val Ala Asp Leu Thr Trp Val Gly Asp Lys Gly Ile Leu Val
85 90 95

```
Ala Ser Asp Ser Gly Ala Val Glu Leu Trp Glu Leu Asp Glu Asn Glu
            100                 105                 110

Thr Leu Ile Val Ser Lys Phe Cys Lys Tyr Glu His Asp Asp Ile Val
        115                 120                 125

Ser Thr Val Thr Val Leu Ser Ser Gly Thr Gln Ala Val Ser Gly Ser
    130                 135                 140

Lys Asp Cys Cys Ile Lys Ile Trp Asp Leu Ala Gln Gln Val Ser Leu
145                 150                 155                 160

Asn Ser Tyr Arg Ala His Ala Gly Gln Val Thr Cys Val Ala Ala Ser
                165                 170                 175

Pro His Lys Asp Ser Val Phe Leu Ser Cys Ser Glu Asp Ser Arg Ile
            180                 185                 190

Leu Leu Trp Asp Thr Arg Cys Pro Lys Pro Ala Ser Gln Met Ala Cys
        195                 200                 205

Asn Ala Ser Gly Tyr Leu Pro Thr Ala Leu Ala Trp His Pro Gln Gln
    210                 215                 220

Ser Glu Val Phe Val Phe Gly Asp Glu Asn Gly Ser Val Ser Leu Val
225                 230                 235                 240

Asp Thr Lys Asn Ala Ser Cys Thr Leu Ser Ser Ala Val His Ser Gln
                245                 250                 255

Gly Val Thr Arg Leu Val Phe Ser Pro His Ser Val Pro Leu Leu Thr
            260                 265                 270

Ser Leu Ser Glu Asp Cys Ser Leu Ala Val Leu Asp Ser Ser Leu Ser
        275                 280                 285

Glu Val Phe Arg Ser Arg Ala His Arg Asp Phe Val Arg Asp Ala Thr
    290                 295                 300

Trp Ser Pro Leu Asn His Ser Leu Leu Thr Thr Val Gly Trp Asp His
305                 310                 315                 320

Gln Val Ile His His Val Val Pro Leu Glu Pro Leu Pro Asn Pro Gly
                325                 330                 335

Pro Asp Ser Val Val Glu
            340
```

<210> 221
<211> 285
<212> PRT

<213> Artificial

<400> 221

```
Met Arg Lys Asp Thr Pro Pro Pro Leu Val Pro Pro Ala Ala Arg Glu
1               5                   10                  15

Trp Asn Leu Pro Pro Asn Ala Pro Ala Cys Met Glu Arg Gln Leu Glu
            20                  25                  30

Ala Ala Arg Tyr Arg Ser Asp Gly Ser Leu Leu Leu Gly Val Ser Ser
        35                  40                  45

Leu Ser Gly Arg Cys Trp Val Gly Ser Leu Trp Phe Phe Lys Asp Pro
    50                  55                  60

Ser Ala Ala Pro Asn Glu Gly Phe Cys Ser Ala Gly Val Gln Thr Glu
65                  70                  75                  80

Ala Gly Val Ala Asp Leu Thr Trp Val Gly Asp Lys Gly Ile Leu Val
                85                  90                  95

Ala Ser Asp Ser Gly Ala Val Glu Leu Trp Glu Leu Asp Glu Asn Glu
            100                 105                 110

Thr Leu Ile Val Ser Lys Phe Cys Lys Tyr Glu His Asp Asp Ile Val
        115                 120                 125

Ser Thr Val Thr Val Leu Ser Ser Gly Thr Gln Ala Val Ser Gly Ser
    130                 135                 140

Lys Asp Cys Cys Ile Lys Ile Trp Asp Leu Ala Gln Gln Val Ser Leu
145                 150                 155                 160

Asn Ser Tyr Arg Ala His Ala Gly Gln Val Thr Cys Val Ala Ala Ser
                165                 170                 175

Pro His Lys Asp Ser Val Phe Leu Ser Cys Ser Glu Asp Ser Arg Ile
            180                 185                 190

Leu Leu Trp Asp Thr Arg Cys Pro Lys Pro Ala Ser Gln Met Ala Cys
        195                 200                 205

Asn Ala Ser Gly Tyr Leu Pro Thr Ala Leu Ala Trp His Pro Gln Gln
    210                 215                 220

Ser Glu Val Phe Val Phe Gly Asp Glu Asn Gly Ser Val Ser Leu Val
225                 230                 235                 240

Asp Thr Lys Asn Ala Ser Cys Thr Leu Ser Ser Ala Val His Ser Gln
                245                 250                 255
```

Gly Val Thr Arg Leu Val Phe Ser Pro His Arg Cys Cys Val Ser Pro
260 265 270

Gly Thr Trp Lys Gly Trp Val Gly Thr Val Val Lys Glu
275 280 285

<210> 222
<211> 234
<212> PRT
<213> Artificial

<400> 222

```
Met Arg Lys Asp Thr Pro Pro Pro Leu Val Pro Pro Ala Ala Arg Glu
1               5                   10                  15

Trp Asn Leu Pro Pro Asn Ala Pro Ala Cys Met Glu Arg Gln Leu Glu
            20                  25                  30

Ala Ala Arg Tyr Arg Ser Asp Gly Ser Leu Leu Leu Gly Val Ser Ser
        35                  40                  45

Leu Ser Gly Arg Cys Trp Val Gly Ser Leu Trp Phe Phe Lys Asp Pro
    50                  55                  60

Ser Ala Ala Pro Asn Glu Gly Phe Cys Ser Ala Gly Val Gln Thr Glu
65                  70                  75                  80

Ala Gly Val Ala Asp Leu Thr Trp Val Gly Asp Lys Gly Ile Leu Val
                85                  90                  95

Ala Ser Asp Ser Gly Ala Val Glu Leu Trp Glu Leu Asp Glu Asn Glu
            100                 105                 110

Thr Leu Ile Val Ser Lys Phe Cys Lys Tyr Glu His Asp Asp Ile Val
        115                 120                 125

Ser Thr Val Thr Val Leu Ser Ser Gly Thr Gln Ala Val Ser Gly Ser
    130                 135                 140

Lys Asp Cys Cys Ile Lys Ile Trp Asp Leu Ala Gln Gln Val Ser Leu
145                 150                 155                 160

Asn Ser Tyr Arg Ala His Ala Gly Gln Val Thr Cys Val Ala Ala Ser
                165                 170                 175

Pro His Lys Asp Ser Val Phe Leu Ser Cys Ser Glu Asp Ser Arg Ile
            180                 185                 190

Leu Leu Trp Asp Thr Arg Cys Pro Lys Pro Ala Ser Gln Met Ala Cys
        195                 200                 205

Asn Ala Ser Gly Tyr Leu Pro Thr Ala Leu Ala Trp His Pro Gln Gln
    210                 215                 220

Ser Glu Val Phe Val Phe Gly Lys Ala Ala
225                 230
```

<210> 223
<211> 269
<212> PRT
<213> Artificial

<400> 223

```
Met Arg Lys Asp Thr Pro Pro Pro Leu Val Pro Pro Ala Ala Arg Glu
1               5                   10                  15

Trp Asn Leu Pro Pro Asn Ala Pro Ala Cys Met Glu Arg Gln Leu Glu
            20                  25                  30

Ala Ala Arg Tyr Arg Ser Asp Gly Ser Leu Leu Leu Gly Val Ser Ser
        35                  40                  45

Leu Ser Gly Arg Cys Trp Val Gly Ser Leu Trp Phe Phe Lys Asp Pro
    50                  55                  60

Ser Ala Ala Pro Asn Glu Gly Phe Cys Ser Ala Gly Val Gln Thr Glu
65                  70                  75                  80

Ala Gly Val Ala Asp Leu Thr Trp Val Gly Asp Lys Gly Ile Leu Val
                85                  90                  95

Ala Ser Asp Ser Gly Ala Val Glu Leu Trp Glu Leu Asp Glu Asn Glu
            100                 105                 110

Thr Leu Ile Val Ser Lys Phe Cys Lys Tyr Glu His Asp Asp Ile Val
        115                 120                 125

Ser Thr Val Thr Val Leu Ser Ser Gly Thr Gln Ala Val Ser Gly Ser
    130                 135                 140

Lys Asp Cys Cys Ile Lys Ile Trp Asp Leu Ala Gln Gln Val Ser Leu
145                 150                 155                 160

Asn Ser Tyr Arg Ala His Ala Gly Gln Val Thr Cys Val Ala Ala Ser
                165                 170                 175

Pro His Lys Asp Ser Val Phe Leu Ser Cys Ser Glu Asp Ser Arg Ile
            180                 185                 190

Leu Leu Trp Asp Thr Arg Cys Pro Lys Pro Ala Ser Gln Met Ala Cys
        195                 200                 205

Asn Ala Ser Gly Tyr Leu Pro Thr Ala Leu Ala Trp His Pro Gln Gln
    210                 215                 220

Ser Glu Val Phe Val Phe Gly Asp Glu Asn Gly Ser Val Ser Leu Val
225                 230                 235                 240

Asp Thr Lys Asn Ala Ser Cys Thr Leu Ser Ser Ala Val His Ser Gln
                245                 250                 255

Gly Val Thr Arg Leu Cys Pro Pro Pro Asp Phe Ser Gln
            260                 265
```

<210> 224
<211> 751
<212> PRT
<213> Artificial

<400> 224

Met Arg Leu Leu Arg Val Ala Ala Ala Leu Gly Arg Gly Pro Phe Pro
1 5 10 15

Arg Val Pro Ala Val Leu Gly Trp Gln Gly Lys Gln Ala Asp Trp Lys
20 25 30

Thr Arg Arg Trp Cys Ser Ser Gly Pro Val Pro Asn Glu Lys Ile Arg
35 40 45

Asn Ile Gly Ile Ser Ala His Ile Asp Ser Gly Lys Thr Thr Leu Thr
50 55 60

Glu Arg Val Leu Tyr Tyr Thr Gly Arg Ile Ala Thr Met His Glu Val
65 70 75 80

Lys Gly Lys Asp Gly Val Gly Ala Val Met Asp Ser Met Glu Leu Glu
85 90 95

Arg Gln Arg Gly Ile Thr Ile Gln Ser Ala Ala Thr Tyr Thr Met Trp
100 105 110

Lys Asp Ile Asn Ile Asn Ile Ile Asp Thr Pro Gly His Val Asp Phe
115 120 125

Thr Ile Glu Val Glu Arg Ala Leu Arg Val Leu Asp Gly Ala Val Leu
130 135 140

Val Leu Cys Ala Val Gly Gly Val Gln Cys Gln Thr Met Thr Val Ser
145 150 155 160

Arg Gln Met Lys Arg Tyr Asn Val Pro Phe Leu Thr Phe Ile Asn Lys
165 170 175

Leu Asp Arg Met Gly Ser Asn Pro Ser Arg Ala Leu Gln Gln Met Arg
180 185 190

Ser Lys Leu Asn His Asn Ala Ala Phe Val Gln Ile Pro Ile Gly Leu
195 200 205

Glu Gly Asp Phe Lys Gly Ile Ile Asp Leu Ile Glu Glu Arg Ala Ile
210 215 220

Tyr Phe Asp Gly Asp Phe Gly Gln Ile Val Arg Tyr Asp Glu Ile Pro
225 230 235 240

Ala Gly Leu Arg Ala Ala Ala Ala Asp His Arg Gln Glu Leu Ile Glu
245 250 255

Cys Val Ala Asn Ser Asp Glu Gln Leu Gly Glu Leu Phe Leu Glu Glu
260 265 270

Lys Ile Pro Ser Val Ser Asp Leu Lys Arg Ala Ile Arg Arg Ala Thr
275 280 285

Leu Ser Arg Ser Phe Thr Pro Val Phe Leu Gly Ser Ala Leu Lys Asn
290 295 300

Lys Gly Val Gln Pro Leu Leu Asp Ala Val Leu Glu Tyr Leu Pro Asn
305 310 315 320

Pro Ser Glu Val Gln Asn Tyr Ala Ile Leu Asn Gln Asn Asp Ser Lys
325 330 335

Glu Lys Thr Lys Ile Leu Met Asn Pro Gln Arg Asp Asp Ser His Pro
340 345 350

Phe Val Gly Leu Ala Phe Lys Leu Glu Ala Gly Arg Phe Gly Gln Leu
355 360 365

Thr Tyr Val Arg Asn Tyr Gln Gly Glu Leu Lys Lys Gly Ser Thr Ile
370 375 380

Tyr Asn Thr Arg Thr Gly Lys Lys Val Arg Val Gln Arg Leu Val Arg
385 390 395 400

Met His Ala Asp Met Met Glu Asp Val Glu Glu Val Tyr Ala Gly Asp
405 410 415

Ile Cys Ala Leu Phe Gly Ile Asp Cys Ala Ser Gly Asp Thr Phe Thr
420 425 430

Asn Lys Asp Asn Ser Asp Leu Ser Met Glu Ser Ile His Val Pro Glu
435 440 445

Pro Val Ile Ser Ile Ala Met Arg Pro Ser Asn Lys Asn Asp Leu Glu
450 455 460

Lys Phe Ser Lys Gly Ile Gly Arg Phe Thr Arg Glu Asp Pro Thr Phe
465 470 475 480

Lys Val His Phe Asp Pro Glu Ser Lys Glu Thr Ile Val Ser Gly Met
485 490 495

Gly Glu Leu His Leu Glu Ile Tyr Ala Gln Arg Met Glu Arg Glu Tyr
500 505 510

Gly Cys Pro Cys Ile Thr Gly Lys Pro Lys Val Ala Phe Arg Glu Thr
515 520 525

Ile Val Ala Pro Val Pro Phe Asp Phe Thr His Lys Lys Gln Ser Gly
530 535 540

Gly Ala Gly Gln Phe Gly Lys Val Ile Gly Val Leu Glu Pro Leu Pro
545 550 555 560

Pro Glu Asp Tyr Thr Lys Leu Glu Phe Ser Asp Glu Thr Phe Gly Ser
565 570 575

Asn Val Pro Lys Gln Phe Val Pro Ala Val Glu Lys Gly Phe Leu Asp
580 585 590

Ala Cys Glu Lys Gly Pro Leu Ser Gly His Lys Leu Ser Gly Leu Arg
595 600 605

Phe Val Leu Gln Asp Gly Ala His His Met Val Asp Ser Asn Glu Ile
610 615 620

Ser Phe Ile Arg Ala Gly Glu Gly Ala Leu Lys Gln Ala Leu Ala Asn
625 630 635 640

Gly Thr Leu Cys Ile Ile Glu Pro Ile Met Ser Val Glu Val Ile Ala
645 650 655

Pro Asn Glu Phe Gln Gly Thr Val Phe Ala Gly Ile Asn Arg Arg His
660 665 670

Gly Val Ile Thr Gly Gln Asp Gly Ile Glu Asp Tyr Phe Thr Leu Tyr
675 680 685

Ala Asp Val Pro Leu Asn Asn Met Phe Gly Tyr Ser Thr Glu Leu Arg
690 695 700

Ser Cys Thr Glu Gly Lys Gly Glu Tyr Thr Met Glu Tyr Cys Arg Tyr
705 710 715 720

Gln Pro Cys Ser Pro Ser Thr Gln Glu Glu Leu Ile Asn Lys Tyr Leu
725 730 735

Glu Ala Thr Gly Gln Leu Pro Val Lys Lys Gly Lys Ala Lys Asn
740 745 750

<210> 225
<211> 560
<212> PRT
<213> Artificial

<400> 225

Met Arg Leu Leu Arg Val Ala Ala Ala Leu Gly Arg Gly Pro Phe Pro
1               5                   10                  15

Arg Val Pro Ala Val Leu Gly Trp Gln Gly Lys Gln Ala Asp Trp Lys
            20                  25                  30

Thr Arg Arg Trp Cys Ser Ser Gly Pro Val Pro Asn Glu Lys Ile Arg
        35                  40                  45

Asn Ile Gly Ile Ser Ala His Ile Asp Ser Gly Lys Thr Thr Leu Thr
    50                  55                  60

Glu Arg Val Leu Tyr Tyr Thr Gly Arg Ile Ala Thr Met His Glu Val
65                  70                  75                  80

Lys Gly Lys Asp Gly Val Gly Ala Val Met Asp Ser Met Glu Leu Glu
                85                  90                  95

Arg Gln Arg Gly Ile Thr Ile Gln Ser Ala Ala Thr Tyr Thr Met Trp
            100                 105                 110

Lys Asp Ile Asn Ile Asn Ile Ile Asp Thr Pro Gly His Val Asp Phe
        115                 120                 125

Thr Ile Glu Val Glu Arg Ala Leu Arg Val Leu Asp Gly Ala Val Leu
    130                 135                 140

Val Leu Cys Ala Val Gly Gly Val Gln Cys Gln Thr Met Thr Val Ser
145                 150                 155                 160

Arg Gln Met Lys Arg Tyr Asn Val Pro Phe Leu Thr Phe Ile Asn Lys
                165                 170                 175

Leu Asp Arg Met Gly Ser Asn Pro Ser Arg Ala Leu Gln Gln Met Arg
            180                 185                 190

Ser Lys Leu Asn His Asn Ala Ala Phe Val Gln Ile Pro Ile Gly Leu
        195                 200                 205

Glu Gly Asp Phe Lys Gly Ile Ile Asp Leu Ile Glu Glu Arg Ala Ile
210 215 220

Tyr Phe Asp Gly Asp Phe Gly Gln Ile Val Arg Tyr Asp Glu Ile Pro
225 230 235 240

Ala Gly Leu Arg Ala Ala Ala Ala Asp His Arg Gln Glu Leu Ile Glu
245 250 255

Cys Val Ala Asn Ser Asp Glu Gln Leu Gly Glu Leu Phe Leu Glu Glu
260 265 270

Lys Ile Pro Ser Val Ser Asp Leu Lys Arg Ala Ile Arg Arg Ala Thr
275 280 285

Leu Ser Arg Ser Phe Thr Pro Val Phe Leu Gly Ser Ala Leu Lys Asn
290 295 300

Lys Gly Val Gln Pro Leu Leu Asp Ala Val Leu Glu Tyr Leu Pro Asn
305 310 315 320

Pro Ser Glu Val Gln Asn Tyr Ala Ile Leu Asn Gln Asn Asp Ser Lys
325 330 335

Glu Lys Thr Lys Ile Leu Met Asn Pro Gln Arg Asp Asp Ser His Pro
340 345 350

Phe Val Gly Leu Ala Phe Lys Leu Glu Ala Gly Arg Phe Gly Gln Leu
355 360 365

Thr Tyr Val Arg Asn Tyr Gln Gly Glu Leu Lys Lys Gly Ser Thr Ile
370 375 380

Tyr Asn Thr Arg Thr Gly Lys Lys Val Arg Val Gln Arg Leu Val Arg
385 390 395 400

Met His Ala Asp Met Met Glu Asp Val Glu Glu Val Tyr Ala Gly Asp
405 410 415

Ile Cys Ala Leu Phe Gly Ile Asp Cys Ala Ser Gly Asp Thr Phe Thr
420 425 430

Asn Lys Asp Asn Ser Asp Leu Ser Met Glu Ser Ile His Val Pro Glu
435 440 445

Pro Val Ile Ser Ile Ala Met Arg Pro Ser Asn Lys Asn Asp Leu Glu
450 455 460

Lys Phe Ser Lys Gly Ile Gly Arg Phe Thr Arg Glu Asp Pro Thr Phe
465 470 475 480

Lys Val His Phe Asp Pro Glu Ser Lys Glu Thr Ile Val Ser Gly Met
485 490 495

Gly Glu Leu His Leu Glu Ile Tyr Ala Gln Arg Met Glu Arg Glu Tyr
500 505 510

Gly Cys Pro Cys Ile Thr Gly Lys Pro Lys Val Ala Phe Arg Glu Thr
515 520 525

Ile Val Ala Pro Val Pro Val Ala Ser Asn Lys Glu Lys Leu Tyr Met
530 535 540

Leu Pro Ala Met Gln Cys Arg Gly Val Glu Trp Cys Asn Leu Thr Gln
545 550 555 560

<210> 226
<211> 129
<212> PRT
<213> Artificial

<400> 226

Met Ala Ser Ala Trp Pro Arg Ser Leu Pro Gln Ile Leu Val Leu Gly
1 5 10 15

Phe Gly Leu Val Leu Met Arg Ala Ala Ala Gly Glu Gln Ala Pro Gly
20 25 30

Thr Ser Pro Cys Ser Ser Gly Ser Ser Trp Ser Ala Asp Leu Asp Lys
35 40 45

Cys Met Asp Cys Ala Ser Cys Pro Ala Arg Pro His Ser Asp Phe Cys
50 55 60

Leu Gly Cys Ala Ala Ala Pro Pro Ala His Phe Arg Leu Leu Trp Pro
65 70 75 80

Ile Leu Gly Gly Ala Leu Ser Leu Val Leu Val Leu Ala Leu Val Ser
85 90 95

Ser Phe Leu Val Trp Arg Arg Cys Arg Arg Arg Glu Lys Phe Thr Thr
100 105 110

Pro Ile Glu Glu Thr Gly Gly Glu Gly Cys Pro Gly Val Ala Leu Ile
115 120 125

Gln

<210> 227
<211> 139
<212> PRT

<213> Artificial

<400> 227

```
Met Ala Ser Ala Trp Pro Arg Ser Leu Pro Gln Ile Leu Val Leu Gly
1               5                   10                  15

Phe Gly Leu Val Leu Met Arg Ala Ala Ala Gly Glu Gln Ala Pro Gly
            20                  25                  30

Ser Lys Phe Gln Val Gly Lys Val Ser Ser Phe Ile Arg Ser Pro Ser
        35                  40                  45

Phe Ser Phe Ala Thr Cys Pro Ile Glu Ala Pro Pro His Ala Leu Ala
    50                  55                  60

Ala Ala Pro Gly Ala Arg Thr Ser Thr Ser Ala Trp Thr Ala Leu Leu
65                  70                  75                  80

Val Gln Arg Asp His Thr Ala Thr Ser Ala Trp Asp Ala Pro Gln His
                85                  90                  95

Leu Leu Pro Thr Ser Gly Tyr Cys Gly Pro Phe Trp Gly Ala Leu Leu
            100                 105                 110

Val Trp Ser Trp Phe Trp Arg Trp Phe Leu Val Ser Trp Ser Gly Glu
        115                 120                 125

Asp Ala Ala Gly Glu Lys Ser Leu Leu Pro Pro
    130                 135
```

<210> 228
<211> 83
<212> PRT
<213> Artificial

<400> 228

```
Met Ala Ser Ala Trp Pro Arg Ser Leu Pro Gln Ile Leu Val Leu Gly
1               5                   10                  15

Phe Gly Leu Val Leu Met Arg Ala Ala Ala Gly Glu Gln Ala Pro Gly
            20                  25                  30

Thr Ser Pro Cys Ser Ser Gly Ser Ser Trp Ser Ala Asp Leu Asp Lys
        35                  40                  45

Cys Met Asp Cys Ala Ser Cys Pro Ala Arg Pro His Ser Asp Phe Cys
    50                  55                  60

Leu Gly Phe Ser Trp Ser Gly Glu Asp Ala Ala Gly Glu Lys Ser Leu
65                  70                  75                  80
```

Leu Pro Pro

<210> 229
<211> 120
<212> PRT
<213> Artificial

<400> 229

Met Ile Leu Gln Gln Pro Leu Glu Arg Gly Pro Pro Ser Arg Asp Pro
1 5 10 15

Arg Ala Thr Thr Gly Val Thr Arg Gly Leu Asn Ala Ser Leu Ser Pro
20 25 30

Arg Glu Pro Leu His Lys Gln Phe Leu Ser Glu Glu Asn Met Ala Thr
35 40 45

His Phe Ser Arg Leu Ser Leu His Asn Asp His Pro Tyr Cys Ser Pro
50 55 60

Pro Val Thr Phe Pro Glu Ala Leu Pro Pro Leu Arg Ser Pro Cys Pro
65 70 75 80

Glu Leu Leu Leu Trp Arg Tyr Pro Gly Ser Leu Ile Pro Glu Ala Leu
85 90 95

Arg Leu Leu Arg Leu Gly Asp Thr Pro Ser Pro Tyr Tyr Pro Ala Ser
100 105 110

Pro Ala Gly Asp Ile Val Glu Leu
115 120

<210> 230
<211> 202
<212> PRT
<213> Artificial

<400> 230

```
Met Ala Lys Ala Tyr Asp His Leu Phe Lys Leu Leu Leu Ile Gly Asp
1               5                   10                  15

Ser Gly Val Gly Lys Thr Cys Leu Ile Ile Arg Phe Ala Glu Asp Asn
            20                  25                  30

Phe Asn Ser Thr Tyr Ile Ser Thr Ile Gly Ile Asp Phe Lys Ile Arg
        35                  40                  45

Thr Val Asp Ile Glu Gly Lys Arg Ile Lys Leu Gln Val Trp Asp Thr
    50                  55                  60

Ala Gly Gln Glu Arg Phe Lys Thr Ile Thr Thr Ala Tyr Tyr Arg Gly
65                  70                  75                  80

Ala Met Gly Ile Ile Leu Val Tyr Asp Ile Thr Asp Glu Lys Ser Phe
                85                  90                  95

Glu Asn Ile Gln Asn Trp Met Lys Ser Ile Lys Glu Asn Ala Ser Ala
            100                 105                 110

Gly Val Glu Arg Leu Leu Leu Gly Asn Lys Cys Asp Met Glu Ala Lys
        115                 120                 125

Arg Gln Val Gln Arg Glu Gln Ala Glu Lys Leu Ala Arg Glu His Arg
    130                 135                 140

Ile Arg Phe Phe Glu Thr Ser Ala Lys Ser Ser Val Asn Val Asp Glu
145                 150                 155                 160

Ala Phe Ser Ser Leu Ala Arg Asp Ile Leu Leu Lys Thr Gly Gly Arg
                165                 170                 175

Arg Ser Gly Thr Asn Ser Lys Pro Ser Ser Thr Gly Leu Lys Thr Ser
            180                 185                 190

Asp Lys Lys Lys Asn Lys Cys Leu Leu Gly
        195                 200
```

<210> 231
<211> 300
<212> PRT
<213> Artificial

<400> 231

Met Ala Gly Ile Pro Glu Val Val Ala Met Asp Cys Glu Met Val Gly
1               5                   10                  15

Leu Gly Pro Gln Arg Val Ser Gly Leu Ala Arg Cys Ser Ile Val Asn
            20                  25                  30

Ile His Gly Ala Val Leu Tyr Asp Lys Tyr Ile Arg Pro Glu Gly Glu
        35                  40                  45

Ile Thr Asp Tyr Arg Thr Gln Val Ser Gly Val Thr Pro Gln His Met
    50                  55                  60

Val Arg Ala Thr Pro Phe Gly Glu Ala Arg Leu Glu Ile Leu Gln Leu
65                  70                  75                  80

Leu Lys Gly Lys Leu Val Val Gly His Asp Leu Lys His Asp Phe Asn
                85                  90                  95

Ala Leu Lys Glu Asp Met Ser Lys Tyr Thr Ile Tyr Asp Thr Ser Thr
100 105 110

Asp Arg Leu Leu Trp His Glu Ala Lys Leu Gln Tyr Tyr Ser Arg Val
115 120 125

Ser Leu Arg Leu Leu Cys Lys Arg Leu Leu His Lys Asn Ile Gln Val
130 135 140

Leu Pro Gly Ser Leu Leu Gly Val Gly Gly Cys Ile Leu Pro Gly Thr
145 150 155 160

Asp Ile Leu His Leu Leu Leu Tyr Val Gly Met Val Arg Ile Ala Asp
165 170 175

Leu Arg Leu Leu Thr Pro Phe Leu Pro Pro Ser Cys Leu Ala Cys Pro
180 185 190

Leu Leu Pro Glu Ser Leu Ala Ser Ala Arg Ser His Ala Val Ile Ser
195 200 205

Ala Leu Ser Ser Ser Ser His Leu Leu Thr Pro Leu Pro Asn Pro Ser
210 215 220

Gln Gly Pro Gln Gly His Val Asp Arg Leu Ser Gly Gln Leu Gln Asp
225 230 235 240

Trp Gly Gly Ser Pro Leu Ala Pro Ala Leu Pro Val Ser Ala Glu Gln
245 250 255

Leu Ala Gly Pro Leu Leu Cys Gly Arg Cys Gln Gly His Asn Gly Ala
260 265 270

Leu Gln Asn Leu Ser Ala Thr Gln Ser Pro Ala Arg Ala Ala Leu Pro
275 280 285

Trp Asp Val Arg Leu Asn Phe Ile Leu Ile Gln Gly
290 295 300

<210> 232
<211> 181
<212> PRT
<213> Artificial

<400> 232

Met Ala Gly Ile Pro Glu Val Val Ala Met Asp Cys Glu Met Val Gly
1 5 10 15

Leu Gly Pro Gln Arg Val Ser Gly Leu Ala Arg Cys Ser Ile Val Asn
20 25 30

Ile His Gly Ala Val Leu Tyr Asp Lys Tyr Ile Arg Pro Glu Gly Glu
35 40 45

Ile Thr Asp Tyr Arg Thr Gln Val Ser Gly Val Thr Pro Gln His Met
50 55 60

Val Arg Ala Thr Pro Phe Gly Glu Ala Arg Leu Glu Ile Leu Gln Leu
65 70 75 80

Leu Lys Gly Lys Leu Val Val Gly His Asp Leu Lys His Asp Phe Asn
85 90 95

Ala Leu Lys Glu Asp Met Ser Lys Tyr Thr Ile Tyr Asp Thr Ser Thr
100 105 110

Asp Arg Leu Leu Trp His Glu Ala Lys Leu Gln Tyr Tyr Ser Arg Val
115 120 125

Ser Leu Arg Leu Leu Cys Lys Arg Leu Leu His Lys Asn Ile Gln Asn
130 135 140

Asn Trp Arg Gly His Cys Ser Val Glu Asp Ala Arg Ala Thr Met Glu
145 150 155 160

Leu Tyr Lys Ile Ser Gln Arg Leu Arg Ala Gln Arg Gly Leu Pro Cys
165 170 175

Pro Gly Thr Ser Asp
180

<210> 233
<211> 201
<212> PRT
<213> Artificial

<400> 233

Met Ala Gly Ile Pro Glu Val Val Ala Met Asp Cys Glu Met Val Gly
1 5 10 15

Leu Gly Pro Gln Arg Val Ser Gly Leu Ala Arg Cys Ser Ile Val Asn
20 25 30

Ile His Gly Ala Val Leu Tyr Asp Lys Tyr Ile Arg Pro Glu Gly Glu
35 40 45

Ile Thr Asp Tyr Arg Thr Gln Val Ser Gly Val Thr Pro Gln His Met
50 55 60

Val Arg Ala Thr Pro Phe Gly Glu Ala Arg Leu Glu Ile Leu Gln Leu
65 70 75 80

Leu Lys Gly Lys Leu Val Val Gly His Asp Leu Lys His Asp Phe Asn
85 90 95

Ala Leu Lys Glu Asp Met Ser Lys Tyr Thr Ile Tyr Asp Thr Ser Thr
100 105 110

Asp Arg Leu Leu Trp His Glu Ala Lys Leu Gln Tyr Tyr Ser Arg Val
115 120 125

Ser Leu Arg Leu Leu Cys Lys Arg Leu Leu His Lys Asn Ile Gln Val
130 135 140

Arg Ser Leu Pro Ala Pro Met Thr Leu Leu Val Ser Leu Ser Ser Pro
145 150 155 160

Ser Ser Leu Ser Phe Ser Leu Phe Pro Leu Arg Cys Ser Phe Ser Phe
165 170 175

Ser Phe Leu Ser Tyr Ser Ser Lys Gln Gly Val Leu Leu Leu Ser Ser
180 185 190

Leu Val Leu Pro Leu Leu Pro Cys Phe
195 200

<210> 234
<211> 358
<212> PRT
<213> Artificial

<400> 234

Met Val Trp Gly His Phe Pro Leu Ala Val Phe Asp Ser Leu Gly Ser
1 5 10 15

Gly Glu Met Ala Asp Ser Val Lys Thr Phe Leu Gln Asp Leu Gly Arg
20 25 30

Gly Ile Lys Asp Ser Ile Trp Gly Ile Cys Thr Ile Ser Lys Leu Asp
35 40 45

Ala Arg Ile Gln Gln Lys Arg Glu Glu Gln Arg Arg Arg Arg Ala Ser
50 55 60

Ser Leu Leu Ala Gln Arg Arg Pro Gln Ser Val Glu Arg Lys Gln Glu
65 70 75 80

Ser Glu Pro Arg Ile Val Ser Arg Ile Phe Gln Cys Cys Ala Trp Asn
85 90 95

Gly Gly Val Phe Trp Phe Ser Leu Leu Leu Phe Tyr Arg Val Phe Ile
100 105 110

```
Pro Val Leu Gln Ser Val Thr Ala Arg Ile Ile Gly Asp Pro Ser Leu
        115                 120                 125

His Gly Asp Val Trp Ser Trp Leu Glu Phe Phe Leu Thr Ser Ile Phe
    130                 135                 140

Ser Ala Leu Trp Val Leu Pro Leu Phe Val Leu Ser Lys Val Val Asn
145                 150                 155                 160

Ala Ile Trp Phe Gln Asp Ile Ala Asp Leu Ala Phe Glu Val Ser Gly
                165                 170                 175

Arg Lys Pro His Pro Phe Pro Ser Val Ser Lys Ile Ile Ala Asp Met
            180                 185                 190

Leu Phe Asn Leu Leu Leu Gln Ala Leu Phe Leu Ile Gln Gly Met Phe
        195                 200                 205

Val Ser Leu Phe Pro Ile His Leu Val Gly Gln Leu Val Ser Leu Leu
    210                 215                 220

His Met Ser Leu Leu Tyr Ser Leu Tyr Cys Phe Glu Tyr Arg Trp Phe
225                 230                 235                 240

Asn Lys Gly Ile Glu Met His Gln Arg Leu Ser Asn Ile Glu Arg Asn
                245                 250                 255

Trp Pro Tyr Tyr Phe Gly Phe Gly Leu Pro Leu Ala Phe Leu Thr Ala
            260                 265                 270

Met Gln Ser Ser Tyr Ile Ile Ser Gly Cys Leu Phe Ser Ile Leu Phe
        275                 280                 285

Pro Leu Phe Ile Ile Ser Ala Asn Glu Ala Lys Thr Pro Gly Lys Ala
    290                 295                 300

Tyr Leu Phe Gln Leu Arg Leu Phe Ser Leu Val Val Phe Leu Ser Asn
305                 310                 315                 320

Arg Leu Phe His Lys Thr Val Tyr Leu Gln Ser Ala Leu Ser Ser Ser
                325                 330                 335

Ser Ser Ala Glu Lys Phe Pro Ser Pro His Pro Ser Pro Ala Lys Leu
            340                 345                 350

Lys Ala Ala Ala Gly His
        355
```

<210> 235
<211> 374
<212> PRT

<213> Artificial

<400> 235

Met Val Trp Gly His Phe Pro Leu Ala Val Phe Asp Ser Leu Gly Ser
1               5                   10                  15

Gly Glu Met Ala Asp Ser Val Lys Thr Phe Leu Gln Asp Leu Gly Arg
            20                  25                  30

Gly Ile Lys Asp Ser Ile Trp Gly Ile Cys Thr Ile Ser Lys Leu Asp
        35                  40                  45

Ala Arg Ile Gln Gln Lys Arg Glu Glu Gln Arg Arg Arg Arg Ala Ser
    50                  55                  60

Ser Leu Leu Ala Gln Arg Arg Pro Gln Ser Val Glu Arg Lys Gln Glu
65                  70                  75                  80

Ser Glu Pro Arg Ile Val Ser Arg Ile Phe Gln Cys Cys Ala Trp Asn
                85                  90                  95

Gly Gly Val Phe Trp Phe Ser Leu Leu Leu Phe Tyr Arg Val Phe Ile
            100                 105                 110

Pro Val Leu Gln Ser Val Thr Ala Arg Ile Ile Gly Asp Pro Ser Leu
        115                 120                 125

His Gly Asp Val Trp Ser Trp Leu Glu Phe Phe Leu Thr Ser Ile Phe
    130                 135                 140

Ser Ala Leu Trp Val Leu Pro Leu Phe Val Leu Ser Lys Val Val Asn
145                 150                 155                 160

Ala Ile Trp Phe Gln Asp Ile Ala Asp Leu Ala Phe Glu Val Ser Gly
                165                 170                 175

Arg Lys Pro His Pro Phe Pro Ser Val Ser Lys Ile Ile Ala Asp Met
            180                 185                 190

Leu Phe Asn Leu Leu Leu Gln Ala Leu Phe Leu Ile Gln Gly Met Phe
        195                 200                 205

Val Ser Leu Phe Pro Ile His Leu Val Gly Gln Leu Val Ser Leu Leu
    210                 215                 220

His Met Ser Leu Leu Tyr Ser Leu Tyr Cys Phe Glu Tyr Arg Trp Phe
225                 230                 235                 240

Asn Lys Gly Ile Glu Met His Gln Arg Leu Ser Asn Ile Glu Arg Asn
                245                 250                 255

Trp Pro Tyr Tyr Phe Gly Phe Gly Leu Pro Leu Ala Phe Leu Thr Ala
260 265 270

Met Gln Ser Ser Tyr Ile Ile Ser Gly Cys Leu Phe Ser Ile Leu Phe
275 280 285

Pro Leu Phe Ile Ile Ser Ala Asn Glu Ala Lys Thr Pro Gly Lys Ala
290 295 300

Tyr Leu Phe Gln Leu Arg Leu Phe Ser Leu Val Val Phe Leu Ser Asn
305 310 315 320

Arg Leu Phe His Lys Thr Val Tyr Leu Gln Pro Leu Ser Pro Ala Val
325 330 335

Lys Gly Val Gly Gly Thr Gly Trp Arg Met Trp Gln Leu Phe Ser Leu
340 345 350

Phe Ser Ser Pro Cys Arg Gly Arg Gln Asn Pro Leu Pro Arg Ala Leu
355 360 365

Cys Ile Val Pro Cys Leu
370

<210> 236
<211> 179
<212> PRT
<213> Artificial

<400> 236

Met Val Trp Gly His Phe Pro Leu Ala Val Phe Asp Ser Leu Gly Ser
1 5 10 15

Gly Glu Met Ala Asp Ser Val Lys Thr Phe Leu Gln Asp Leu Gly Arg
20 25 30

Gly Ile Lys Asp Ser Ile Trp Gly Ile Cys Thr Ile Ser Lys Leu Asp
35 40 45

Ala Arg Ile Gln Gln Lys Arg Glu Glu Gln Arg Arg Arg Arg Ala Ser
50 55 60

Ser Leu Leu Ala Gln Arg Arg Pro Gln Ser Val Glu Arg Lys Gln Glu
65 70 75 80

Ser Glu Pro Arg Ile Val Ser Arg Ile Phe Gln Cys Cys Ala Trp Asn
85 90 95

Gly Gly Val Phe Trp Phe Ser Leu Leu Leu Phe Tyr Arg Val Phe Ile
100 105 110

Pro Val Leu Gln Ser Val Thr Ala Arg Ile Ile Gly Asp Pro Ser Leu
115 120 125

His Gly Asp Val Trp Ser Trp Leu Glu Phe Phe Leu Thr Ser Ile Phe
130 135 140

Ser Ala Leu Trp Val Leu Pro Leu Phe Val Leu Ser Lys Val Val Asn
145 150 155 160

Ala Ile Trp Phe Gln Val Gly Ala Gly Gln Asn Gly Thr Gly Phe Tyr
165 170 175

Met Leu Gly

<210> 237
<211> 20
<212> DNA
<213> Artificial

<220>
<223> Synthetic oligonucleotide

<400> 237
gggaaatcgt gcgtgacatt 20

<210> 238
<211> 17
<212> DNA
<213> Artificial

<220>
<223> Synthetic oligonucleotide

<400> 238
gcggcagtgg ccatctc 17

<210> 239
<211> 76
<212> DNA
<213> Artificial

<220>
<223> Synthetic oligonucleotide

<400> 239

gggaaatcgt gcgtgacatt aaagagaagc tgtgctatgt tgccctagac ttcgagcaag 60

agatggccac tgccgc 76

<210> 240
<211> 20
<212> DNA

<213> Artificial

<220>
<223> Synthetic oligonucleotides

<400> 240
taccctattg agcacggcat 20

<210> 241
<211> 20
<212> DNA
<213> Artificial

<220>
<223> Synthetic oligonucleotide

<400> 241
cgcagctcgt tgtagaaggt 20

<210> 242
<211> 80
<212> DNA
<213> Artificial

<220>
<223> Synthetic oligonucleotide

<400> 242

taccccattg aacacggcat catcacgaac tgggatgaca tggagaagat ctggcaccac 60

tccttctaca acgagctgcg 80

<210> 243
<211> 21
<212> DNA
<213> Artificial

<220>
<223> Synthetic oligonucleotide

<400> 243
gcgaaagtgg aaaagccaag t 21

<210> 244
<211> 24
<212> DNA
<213> Artificial

<220>
<223> Synthetic oligonucleotide

<400> 244
gccacatcaa caggactctt gtag 24

<210> 245
<211> 76
<212> DNA

<213> Artificial

<220>
<223> Synthetic oligonucleotide

<400> 245

gcgaaagtgg aaaagccaag tacaaagcct aaaagacagc ggcaagttga atctacaaga 60

gtcctgttga tgtggc 76

<210> 246
<211> 23
<212> DNA
<213> Artificial

<220>
<223> Synthetic oligonucleotide

<400> 246
caagcatacc aagaagcatt tga 23

<210> 247
<211> 18
<212> DNA
<213> Artificial

<220>
<223> Synthetic oligonucleotide

<400> 247
gggccagacc cagtctga 18

<210> 248
<211> 76
<212> DNA
<213> Artificial

<220>
<223> Synthetic oligonucleotide

<400> 248

caagcatacc aagaagcatt tgaaatcagc aaaaaggaga tgcagccgac acaccccatc 60

agactgggtc tggccc 76

<210> 249
<211> 20
<212> DNA
<213> Artificial

<220>
<223> Synthetic oligonucleotide

<400> 249

ccaaggactg ggcttagggt 20

<210> 250
<211> 22
<212> DNA
<213> Artificial

<220>
<223> Synthetic oligonucleotide

<400> 250
agagattccc ttgtgcaaat gc 22

<210> 251
<211> 144
<212> DNA
<213> Artificial

<220>
<223> Synthetic oligonucleotide

<400> 251

ccaaggactg ggcttagggt tcaggggagc cttccagggt gtctaattgc cctgtctctg 60
gttctggggc agacagagag cctcaagcta ggtcacaaag cgactcatac taaggatctg 120
cagcatttgc acaagggaat ctct 144

<210> 252
<211> 22
<212> DNA
<213> Artificial

<220>
<223> Synthetic oligonucleotide

<400> 252
gatctgggta ggtggttgtt gg 22

<210> 253
<211> 22
<212> DNA
<213> Artificial

<220>
<223> Synthetic oligonucleotide

<400> 253
cgcacaccct tataaaagtc cc 22

<210> 254
<211> 102
<212> DNA
<213> Artificial

<220>
<223> Synthetic oligonucleotide

<400> 254

gatctgggta ggtggttgtt ggggcagaaa ggaggtcgta gacttaggat ataggaaacc 60

aggaaaaact gactgaggaa gggactttta taagggtgtg cg 102

<210> 255
<211> 24
<212> DNA
<213> Artificial

<220>
<223> Synthetic oligonucleotide

<400> 255
atctcgatgg taacgggtct aatc 24

<210> 256
<211> 20
<212> DNA
<213> Artificial

<220>
<223> Synthetic oligonucleotide

<400> 256
tttgtgctga ccttcatgcc 20

<210> 257
<211> 124
<212> DNA
<213> Artificial

<220>
<223> Synthetic oligonucleotide

<400> 257

atctcgatgg taacgggtct aatcagccca tgatcaacat aactgtggtg atatacattt 60

ggtatttttt aattttcgga tgccttcctc aacatagccg tcaaggcatg aaggtcagca 120

caaa 124

<210> 258
<211> 21
<212> DNA
<213> Artificial

<220>
<223> Synthetic oligonucleotide

<400> 258
ccagcctagc ctctcttttg c 21

<210> 259
<211> 23
<212> DNA

<213> Artificial

<220>
<223> Synthetic oligonucleotide

<400> 259
aacattccca cagggtacat tca 23

<210> 260
<211> 121
<212> DNA
<213> Artificial

<220>
<223> Synthetic oligonucleotide

<400> 260

ccagcctagc ctctcttttg cactgctggt tcagcccact gggcctccgt cctttcctct 60
ggaagggact tggccttggg tgacaaattc ctctttgatg aatgtaccct gtgggaatgt 120
t 121

<210> 261
<211> 20
<212> DNA
<213> Artificial

<220>
<223> Synthetic oligonucleotide

<400> 261
gggccacaat ggagctctac 20

<210> 262
<211> 26
<212> DNA
<213> Artificial

<220>
<223> Synthetic oligonucleotide

<400> 262
acaggtctca ttcatggaaa actatg 26

<210> 263
<211> 151
<212> DNA
<213> Artificial

<220>
<223> Synthetic oligonucleotide

<400> 263

gggccacaat ggagctctac aaaatctctc agcgactcag agcccagcga gggctgccct 60
gcctgggaac atcagcctga acttcatcct catccaggat cagaagcagc tactccttga 120
aggaccatag ttttccatga atgagacctg t 151

<210> 264
<211> 17
<212> DNA
<213> Artificial

<220>
<223> Synthetic oligonucleotide

<400> 264
ggaggagggc agagcca 17

<210> 265
<211> 25
<212> DNA
<213> Artificial

<220>
<223> Synthetic oligonucleotide

<400> 265
tctggttcat tatcaaggga agttg 25

<210> 266
<211> 151
<212> DNA
<213> Artificial

<220>
<223> Synthetic oligonucleotide

<400> 266

ggaggagggc agagccagag gagggacagc ctgatgcaga cagccctgac tcaacctgcc 60
agccccctta cctgtcagcc ttgaggagat ggaacagccc agcctactag gcctgccccc 120
actccccaac ttcccttgat aatgaaccag a 151

<210> 267
<211> 22
<212> DNA
<213> Artificial

<220>
<223> Synthetic oligonucleotide

<400> 267
atcctgttgg gtctgcttct ca 22

<210> 268
<211> 30
<212> DNA

<213> Artificial

<220>
<223> Synthetic oligonucleotide

<400> 268
cacaagtaaa tacatagact ggaatcaatg 30

<210> 269
<211> 155
<212> DNA
<213> Artificial

<220>
<223> Synthetic oligonucleotide

<400> 269

atcctgttgg gtctgcttct cacagcgtat ggggctgtgt gcttttactc atggtggcag 60
cagcagttgt tgtcacttct ttggccaggc acagtgggtg atccttacag agcagcacag 120
attcccattg attccagtct atgtatttac ttgtg 155

<210> 270
<211> 25
<212> DNA
<213> Artificial

<220>
<223> Synthetic oligonucleotide

<400> 270
tgtttaaaat ctgggctctt gattc 25

<210> 271
<211> 31
<212> DNA
<213> Artificial

<220>
<223> Synthetic oligonucleotide

<400> 271
gtatttccaa tagtcaatgc agtaactcta c 31

<210> 272
<211> 155
<212> DNA
<213> Artificial

<220>
<223> Synthetic oligonucleotide

<400> 272

tgtttaaaat ctgggctctt gattcagacc gagttttaat actggatctc attcaatctt 60
tgtgtttttt ctttttcttt tcttttttt ccaatagtac ggcagtgcta atagcagtcc 120
tttagtagag ttactgcatt gactattgga aatac 155

<210> 273
<211> 21
<212> DNA
<213> Artificial

<220>
<223> Synthetic oligonucleotide

<400> 273
gccacagtag aggctgcact t 21

<210> 274
<211> 24
<212> DNA
<213> Artificial

<220>
<223> Synthetic oligonucleotide

<400> 274
gaatcaactg gaaatgttct aggg 24

<210> 275
<211> 137
<212> DNA
<213> Artificial

<220>
<223> Synthetic oligonucleotide

<400> 275

gccacagtag aggctgcact tgccactgct gtgtagcggc actctcctga ctcacttaaa 60
aattctctgg aggtttaagt gaggacccag ttccatctct agatatccag gctccctaga 120
acatttccag ttgattc 137

<210> 276
<211> 20
<212> DNA
<213> Artificial

<220>
<223> Synthetic oligonucleotide

<400> 276
ctgcaactga gcatgtgcgt 20

<210> 277
<211> 19
<212> DNA

<213> Artificial

<220>
<223> Synthetic oligonucleotide

<400> 277
tgcataagaa ccaccgggg 19

<210> 278
<211> 155
<212> DNA
<213> Artificial

<220>
<223> Synthetic oligonucleotide

<400> 278

ctgcaactga gcatgtgcgt gggcgagtgc ccccacctct accggtcggc caacacgcat 60
gcgcagatca aagcacgcct gcatggcctg cagcccgaca gagtgccggc cccgtgctgt 120
gtcccctcca gctacacccc ggtggttctt atgca 155

<210> 279
<211> 16
<212> DNA
<213> Artificial

<220>
<223> Synthetic oligonucleotide

<400> 279
ggacggccga gcacaa 16

<210> 280
<211> 22
<212> DNA
<213> Artificial

<220>
<223> Synthetic oligonucleotide

<400> 280
gtgtgcatgt gcataggtca ga 22

<210> 281
<211> 123
<212> DNA
<213> Artificial

<220>
<223> Synthetic oligonucleotide

<400> 281

ggacggccga gcacaatggt gcttgctgta cagcctcata gaccttcctt ggatctccca 60
gacacactta gtggaaggag agaatccatt cttgcaaatt gtctgaccta tgcacatgca 120
cac 123

<210> 282
<211> 26
<212> DNA
<213> Artificial

<220>
<223> Synthetic oligonucleotide

<400> 282
tactgtttcc tgtgaagcac atacct 26

<210> 283
<211> 23
<212> DNA
<213> Artificial

<220>
<223> Synthetic oligonucleotide

<400> 283
gcagagcagt aaagaccaaa acc 23

<210> 284
<211> 137
<212> DNA
<213> Artificial

<220>
<223> Synthetic oligonucleotide

<400> 284

tactgtttcc tgtgaagcac ataccttgta tgtgggaggt aaaggagcac gccagctgct 60
ccatgtcact ccctctatag ccatcactgt cttgtttttt tgtaactcag gttaggtttt 120
ggtctttact gctctgc 137

<210> 285
<211> 21
<212> DNA
<213> Artificial

<220>
<223> Synthetic oligonucleotide

<400> 285
aatctaggct gcctcctgga g 21

<210> 286
<211> 28
<212> DNA

<213> Artificial

<220>
<223> Synthetic oligonucleotide

<400> 286
aggtcaatta tacaaggcat gactttag 28

<210> 287
<211> 101
<212> DNA
<213> Artificial

<220>
<223> Synthetic oligonucleotide

<400> 287

aatctaggct gcctcctgga ggaagatact taggagttca gaagtgaaga gatgaggctt 60

ataatacttt ttcctaaagt catgccttgt ataattgacc t 101

<210> 288
<211> 20
<212> DNA
<213> Artificial

<220>
<223> Synthetic oligonucleotide

<400> 288
tcgtcagaat tgctgcctca 20

<210> 289
<211> 24
<212> DNA
<213> Artificial

<220>
<223> Synthetic oligonucleotide

<400> 289
tctgtggtaa aaacctcctg gagt 24

<210> 290
<211> 151
<212> DNA
<213> Artificial

<220>
<223> Synthetic oligonucleotide

<400> 290

tcgtcagaat tgctgcctca atctagtccc atttgagaaa atttgtttct actgtctcaa 60
taactggatg aaatatcact ctgaaaactt gcctattgca ctaaagctag tttaggcttg 120
ataaaacact ccaggaggtt tttaccacag a 151

<210> 291
<211> 30
<212> DNA
<213> Artificial

<220>
<223> Synthetic oligonucleotide

<400> 291
ataaaatcaa tcccactttc ttgttaaaag 30

<210> 292
<211> 26
<212> DNA
<213> Artificial

<220>
<223> Synthetic oligonucleotide

<400> 292
ctttgcctta gaaaatatga tcctgc 26

<210> 293
<211> 109
<212> DNA
<213> Artificial

<220>
<223> Synthetic oligonucleotide

<400> 293

ataaaatcaa tcccactttc ttgttaaaag gagaaacgat ctgaattttg aaagactaga 60
agcccaactt aaggcctgcc actgcaggat catattttct aaggcaaag 109

<210> 294
<211> 28
<212> DNA
<213> Artificial

<220>
<223> Synthetic oligonucleotide

<400> 294
gagatccaag cactgaagta tgtagagt 28

<210> 295
<211> 28
<212> DNA
<213> Artificial

<220>
<223> Synthetic oligonucleotide

<400> 295
tcaggagtac agtggataca tttctctt 28

<210> 296
<211> 134
<212> DNA
<213> Artificial

<220>
<223> Synthetic oligonucleotide

<400> 296

gagatccaag cactgaagta tgtagagtta acagaaggag tagaatgtat tcagaaacat 60

tccaaggtat gccaaggtga tagcattgat cctattagca agctacaaga gaaatgtatc 120

cactgtactc ctga 134

<210> 297
<211> 21
<212> DNA
<213> Artificial

<220>
<223> Synthetic oligonucleotide

<400> 297
gggcactgca tggattatgt c 21

<210> 298
<211> 21
<212> DNA
<213> Artificial

<220>
<223> Synthetic oligonucleotide

<400> 298
ccatgcccag aataaaggag c 21

<210> 299
<211> 101
<212> DNA
<213> Artificial

<220>
<223> Synthetic oligonucleotide

<400> 299

gggcactgca tggattatgt cggttttacc tagctttgga gaccatcatt ttttcctaat 60

aggtttgctt cattgtttca gctcctttat tctgggcatg g 101

<210> 300
<211> 24
<212> DNA
<213> Artificial

<220>
<223> Synthetic oligonucleotide

<400> 300
ctccgttagc aattatgggt cagt 24

<210> 301
<211> 21
<212> DNA
<213> Artificial

<220>
<223> Synthetic oligonucleotide

<400> 301
gaagctcatg gtccttcagg g 21

<210> 302
<211> 102
<212> DNA
<213> Artificial

<220>
<223> Synthetic oligonucleotide

<400> 302

ctccgttagc aattatgggt cagtaactga tcttattaga gctttacaac tttggtttag 60
gaaaagcaca taaaatgggc gccctgaagg accatgagct tc 102

<210> 303
<211> 23
<212> DNA
<213> Artificial

<220>
<223> Synthetic oligonucleotide

<400> 303
ctgtcattgt catttttccc acc 23

<210> 304
<211> 22
<212> DNA
<213> Artificial

<220>
<223> Synthetic oligonucleotide

<400> 304
atgccggatc ttcaatctta gg 22

<210> 305
<211> 118
<212> DNA
<213> Artificial

<220>
<223> Synthetic oligonucleotide

<400> 305

ctgtcattgt catttttccc acctaaaaat tccctgagga ctgatctggg tactttgctc 60

tggagagctg aagtctgagc gctgtatatt tggactccta agattgaaga tccggcat 118

<210> 306
<211> 21
<212> DNA
<213> Artificial

<220>
<223> Synthetic oligonucleotide

<400> 306
aactggctga tgaccagctg t 21

<210> 307
<211> 17
<212> DNA
<213> Artificial

<220>
<223> Synthetic oligonucleotide

<400> 307
tgctgtgccc gggttct 17

<210> 308
<211> 95
<212> DNA
<213> Artificial

<220>
<223> Synthetic oligonucleotide

<400> 308

aactggctga tgaccagctg tgctactctg tgctgaccga ggactgatgc ctccttcccc 60

tgtacccact gctgaggaag aacccgggca cagca 95

<210> 309
<211> 17
<212> DNA
<213> Artificial

<220>
<223> Synthetic oligonucleotide

<400> 309
gtgagtcagg ccgggct 17

<210> 310
<211> 23
<212> DNA
<213> Artificial

<220>
<223> Synthetic oligonucleotide

<400> 310
tcaaaaaatc ggattctgtg ctc 23

<210> 311
<211> 115
<212> DNA
<213> Artificial

<220>
<223> Synthetic oligonucleotide

<400> 311

gtgagtcagg ccgggctgct gtggccgaat gccttgcttc tgccctttat ccagctctct 60

ccttcctacc tcatccccta cagttggctc gagagcacag aatccgattt tttga 115

<210> 312
<211> 21
<212> DNA
<213> Artificial

<220>
<223> Synthetic oligonucleotide

<400> 312
ttctcctgaa tctggctggg t 21

<210> 313
<211> 23
<212> DNA
<213> Artificial

<220>
<223> Synthetic oligonucleotide

<400> 313
tggaaaaagg atgtccattc ttc 23

<210> 314
<211> 105
<212> DNA
<213> Artificial

<220>
<223> Synthetic oligonucleotide

<400> 314

ttctcctgaa tctggctggg tccccttcc ttaccccaac tctttaactg gtgatgaaaa 60

cagcaaggag aaagggcagc ctgaagaatg gacatccttt ttcca 105

<210> 315
<211> 25
<212> DNA
<213> Artificial

<220>
<223> Synthetic oligonucleotide

<400> 315
agaatccgat tttttgagac aagtg 25

<210> 316
<211> 20
<212> DNA
<213> Artificial

<220>
<223> Synthetic oligonucleotide

<400> 316
gatctccggc ctcctgtctt 20

<210> 317
<211> 104
<212> DNA
<213> Artificial

<220>
<223> Synthetic oligonucleotide

<400> 317

agaatccgat tttttgagac aagtgccaaa tccagtgtga atgtggatga ggctttcagt 60

tccctggccc gtgacatctt gctcaagaca ggaggccgga gatc 104

<210> 318
<211> 20
<212> DNA
<213> Artificial

<220>
<223> Synthetic oligonucleotide

<400> 318
caagccctca agcactgacc 20

<210> 319
<211> 21
<212> DNA
<213> Artificial

<220>
<223> Synthetic oligonucleotide

<400> 319
ggtctagcct ccaggttcag g 21

<210> 320
<211> 113
<212> DNA
<213> Artificial

<220>
<223> Synthetic oligonucleotide

<400> 320

caagccctca agcactgacc tgaaagtatc tgacaagaag aacagcaaca agtgctcctt 60

gggctgagga acatttcttg cctcctattc accctgaacc tggaggctag acc 113

<210> 321
<211> 19
<212> DNA
<213> Artificial

<220>
<223> Synthetic oligonucleotide

<400> 321
gggtggggga atctagcct 19

<210> 322
<211> 20
<212> DNA
<213> Artificial

<220>
<223> Synthetic oligonucleotide

<400> 322
ccccagacaa agggacatgt 20

<210> 323
<211> 106
<212> DNA
<213> Artificial

<220>
<223> Synthetic oligonucleotide

<400> 323

gggtggggga atctagcctc tctagagccc tagccctctg acgaggagga ggtgtagtgc 60

cctgtagctt ttccccagga ctcgccacat gtccctttgt ctgggg 106

<210> 324
<211> 22

<212> DNA
<213> Artificial

<220>
<223> Synthetic oligonucleotide

<400> 324
cgctgaagtc ctcagtgact tg 22

<210> 325
<211> 22
<212> DNA
<213> Artificial

<220>
<223> Synthetic oligonucleotide

<400> 325
cactaagtgc ttcgacaccc ac 22

<210> 326
<211> 161
<212> DNA
<213> Artificial

<220>
<223> Synthetic oligonucleotide

<400> 326

cgctgaagtc ctcagtgact tgtcccattt cttagtagtt gtacaaggag tcaggccaag 60
atggtgcctc gggggctgag ggagctcaca ggaactgagc agtgactggt cctttcccag 120
tattgaatac tgagcccctg tgggtgtcga agcacttagt g 161

<210> 327
<211> 19
<212> DNA
<213> Artificial

<220>
<223> Synthetic oligonucleotide

<400> 327
acagcctgat gcagacagc 19

<210> 328
<211> 25
<212> DNA
<213> Artificial

<220>
<223> Synthetic oligonucleotide

<400> 328
tctggttcat tatcaaggga agttg 25

<210> 329
<211> 127
<212> DNA
<213> Artificial

<220>
<223> Synthetic oligonucleotide

<400> 329

acagcctgat gcagacagcc ctgactcaac ctgccagccc ccttacctgg ccagccttga 60
ggagatggaa cagcccaggc tacaaggcct gcccccactc ctcaacttcc cttgataatg 120
aaccaga 127

<210> 330
<211> 25
<212> DNA
<213> Artificial

<220>
<223> Synthetic oligonucleotide

<400> 330
cagaattgct gcctcaatct agtcc 25

<210> 331
<211> 24
<212> DNA
<213> Artificial

<220>
<223> Synthetic oligonucleotide

<400> 331
ggttttgcag atgtactcgg ttcc 24

<210> 332
<211> 219
<212> DNA
<213> Artificial

<220>
<223> Synthetic oligonucleotide

<400> 332

cagaattgct gcctcaatct agtcccattt gagaaaattt gtttctactg tctcaataac 60
tggatgaaat atcactctga aaacttgcct attgcactaa agctagttta ggcttgataa 120
aacactccag gaggttttta ccacagactg tttctattaa aactgctgct tctcatgtac 180
aattttgttt taaaaggaac cgagtacatc tgcaaaacc 219

## Description

### Field of the invention

The present invention relates to means and methods for predicting the onset of renal injury based on measuring the expression of polynucleotide markers, particularly measuring the expression of sets of novel diagnostic polynucelotides.

### Background of the invention

Renal injury is a general term used to describe any damage to the kidney caused by various conditions including primary renal dysfunction, response to external substances or secondary renal pathology.

Renal injury commonly occurs after administration of pharmaceutical or toxic agents of various types. The process is typically initiated by a toxic injury to tubular epithelial cells in various nephron segments or by injury to specific cell types in the glomerulus. The initial injury is often followed by cellular proliferation and repair processes that attempts to restore normal renal function.

Early recognition of renal injury is hampered by the lack of accurate markers and the shortcoming of and over-reliance on serum markers of impaired glomerular filtration rate (i.e., serum creatinine and blood urea nitrogen, see e.g., Schrier et al, J Clin Invest, 114(1):5-14 (2004)). Drugs associated with the development of tubular nephrosis include aminoglycoside antibiotics, antifungals, antineoplastics, immunosuppresants and radiocontrast dyes, among others.

Similarly to the human clinical setting, long-term treatment of rats during preclinical drug development with relatively low doses of, for example, aminoglycoside antibiotics, heavy metal toxicants or antineoplastic drugs, leads to the development of degenerative lesions of the renal tubules. However, histopathological or clinical indications of kidney injury are not readily apparent in the early course of treatment, thus necessitating expensive and lengthy studies.

Changes in the expression of mRNA specifically expressed in the injured kidney cells are some of the earliest events that accompany renal injury. This is further accompanied by changes in the expression of other genes that contribute either to cellular repair or recovery of renal function or in those that mediate fibrosis and further pathology of the kidney (Matejka GL. et al., Exp Nephrol, 1998 6:253-264; Norman JT. et al. Proc Natl Acad Sci USA, 1988 85:6768-6772; Safirstein R. et al. Kidney Int, 1990 37:1515-1521). For example, elevation in the expression of heme oxygenase I (*HO-1*), kidney injury molecule-1 (*KIM-1*), clusterin, thymosin beta-4, osteopontin, and several growth factors have been reported in various models of renal injury (Hammerman et al, 1998 Curr Oppin Nephrol Hypertens 7 :419-424; Yoshida et al, 2002 Kidney International 61: 1646-1654; Amin R.P et al. 2004 Environ Health Perspect. 112(4):465-479; Thomas R.S et. al. 2001 Mol Pharmacol. 60(6):1189-1194).

Yuen et al. (Yuen P S T et al., 2006. Kidney Intern. 70(10):1750-1758) describe the use of microarrays for identifying early biomarkers that distinguish ischemic from nephrotoxic acute renal failure (ARF) or biomarkers that detect both injury types. Using Western blotting it was confirmed that heme oxygenase-1 (HO-1) and activating transcription factor-3 (ATF3) proteins were upregulated. However, their localization changed within the kidney. Yuen et al. concluded that microarray-identified genes must be evaluated not only for protein levels but also for anatomical distribution among different zones, nephron segments, or cell types.

The study of Amin et al. (Amin R P et al., *ibid.*) examined the changes in the expression profile of genes associated with the administration of three different nephrotoxicants - cisplatin, gentamicin, and puromycin to assess the usefulness of microarrays in the understanding of mechanism(s) of nephrotoxicity. Analysis of profile components revealed some novel changes in the expression of genes that appeared to be associated with injury in specific portions of the nephron and reflected the mechanism of action of these various nephrotoxicants. Amin el al. concluded that renal gene expression profiling coupled with analysis of classical end points affords promising opportunities to reveal potential new mechanistic markers of renal toxicity.

Kharasch et al. (Kharasch E D et al. 2006. Toxicological Sciences 90(2):419-31) evaluated the gene expression profile of kidneys in rats to which a nephrotoxic dose of the haloalkene fluoromethyl-2,2-difluoro-1-(trifluoromethyl)-vinyl ether (FDVE) was administered. FDVE caused significant diuresis and necrosis at 24 h, with normal urine output and evidence of tubular regeneration at 72 h. There were 517 informative genes that were differentially expressed >1.5-fold ($p < 0.05$) versus control at 24 h, of which 283 and 234 were upregulated and downregulated, respectively. Major classes of upregulated genes included those involved in apoptosis, oxidative stress, and inflammatory response (mostly at 24 h), and regeneration and repair; downregulated genes were generally associated with transporters and intermediary metabolism. The rapid changes in gene expression after FDVE adminsitration provide potential insights into the mechanism of FDVE toxification, and potential biomarkers for FDVE nephrotoxicity which are more sensitive than conventional measures of renal function.

International Patent Application Publication No. WO 2006/033701 to ICONIX PHARMACEUTICALS, INC. provides gene signatures as well as methods, apparatuses and reagents useful for predicting future renal tubule injury, based on the expression levels of genes in the signatures. In one particular embodiment that invention provides a method for predicting whether a compound will induce renal tubule injury using gene expression data from sub-acute treatments. However, the WO 2006/033701 Application discloses that the necessary set useful for generating signatures of varying size and performance includes 186 genes, which is a very large number of genes of which expression should be measured.

International Patent Application Publication No. WO 02/095000 to GENE LOGIC, INC. provides toxicity markers identified in tissues or cells exposed to a known renal toxin, based on the elucidation of the global changes in gene expression. The genes may be used as toxicity markers in drug screening and toxicity assays. That Application includes a database of genes characterized by toxin-induced differential expression designed for use with microarrays and other solid-phase probes. The WO 02/095000 Application does not provide specific combination(s) of markers that can be used for toxicity prediction, and thus the methods disclosed require measuring expression of a vast number of genes.

The development of methods to predict the future onset of renal injury and gain a greater understanding of its underlying mechanism would facilitate the development of more reliable clinical diagnostics and safer therapeutic drugs. Moreover, improved preclinical markers for renal injury, particularly of well-defined gene signatures including small number of genes would dramatically reduce the time, cost, and amount of compound required for prioritizing and selecting lead candidates for progression through drug development.

**Summary of the invention**

The present invention provides a diagnostic isolated polynucleotide marker and methods and kits useful in predicting the onset of renal injury, particularly an injury resulting from exposure to a toxin or pharmaceutical agent.

The present invention is based in part on the elucidation of the global changes in gene expression in tissues or cells exposed to known toxins, in particular renal toxins, as compared to unexposed, or exposed to control compounds, tissues or cells, as well as on the identification of individual genes that are differentially expressed upon exposure of the cells to a toxin. The present invention is advantageous over hitherto known methods using gene sets for predicting renal injury, providing sets of only few genes necessary for accurate prediction. Moreover, the present invention provides sets based on the expression of unique polynucleotides and proteins associated with renal injury.

Thus, according to one aspect, the present invention provides an isolated polynucleotide marker comprising the nucleic acid sequence as set forth in SEQ. ID NO: 2.

According to certain embodiments of the present invention, the novel polynucleotide described herein is a marker for diagnosing renal injury. The marker of the invention can be employed for prediction of the onset of renal injury.

According to certain embodiments, the isolated polynucleotide consists of the nucleic acid sequence set forth in SEQ ID NO: 2.

The present invention further provides a method for predicting renal injury in a subject receiving a treatment with a compound, comprising (a) measuring in a biological sample the expression level of a set of ploynucleotide otained from at least one test mammal at a selected time period after the compound had been administered to said mammal; and (b) determining whether the sample is in the positive class for onset of renal injury using a classifier comprising the set of polynucleotide markers for which the expression level is measured, wherein the expression of the set of markers is indicative of the onset of renal injury. The method is characteriued in that (i) the set comprises four polynucleotide markers comprising a first polynucleotide having the nucleic acid sequence set forth in SEQ ID NO:2 or the nucleic acid sequence set forth in SEQ ID NO:254; a second polynucleotide having the nucleic acid sequence set forth in SEQ ID NO:4 or the nucleic acid sequence set forth in SEQ ID NO:5 or the nucleic acid sequence set forth in SEQ ID NO:266 or the nucleic acid sequence set forth in SEQ ID NO:329; a third polynucleotide having the nucleic acid sequence set forth in SEQ ID NO:7 or the nucleic acid sequence set forth in SEQ ID NO:8 or the nucleic acid sequence set forth in SEQ ID NO:287; and a fourth polynucleotide having the nucleic acid sequence set forth in SEQ ID NO: 13 or the nucleic acid sequence set forth in SEQ ID NO:14 or the nucleic acid sequence set forth in SEQ ID NO:296; or

(ii) the set comprises four polynucleotide markers comprising a first polynucleotide having the nucleic acid sequence set forth in SEQ ID NO:2 or the nucleic acid sequence set forth in SEQ ID NO:254; a second polynucleotide having the nucleic acid sequence set forth in SEQ ID NO:4 or the nucleic acid sequence set forth in SEQ ID NO:5 or the nucleic acid sequence set forth in SEQ ID NO:266 or the nucleic acid sequence set forth in SEQ ID NO:329; a third polynucleotide having the nucleic acid sequence set forth in SEQ ID NO:49 or the nucleic acid sequence set forth in SEQ ID NO:263; and a fourth polynucleotide having the nucleic acid sequence set forth in SEQ ID NO:53 or the nucleic acid sequence set forth in SEQ ID NO:290 or the nucleic acid sequence set forth in SEQ ID NO:332; or

(iii) the set comprises six polynucleotide markers comprising a first polynucleotide having the nucleic acid sequence set forth in SEQ ID NO:2 or the nucleic acid sequence set forth in SEQ ID NO:254; a second polynucleotide having the nucleic acid sequence set forth in SEQ ID NO:4 or the nucleic acid sequence set forth in SEQ ID NO:5 or the nucleic acid sequence set forth in SEQ ID NO:266 or the nucleic acid sequence set forth in SEQ ID NO:329; a third polynucleotide having the nucleic acid sequence set forth in SEQ ID NO:49 or the nucleic acid sequence set forth in SEQ ID NO:263; a fourth polynucleotide having the nucleic acid sequence set forth in SEQ ID NO:7 or the nucleic acid sequence set forth in SEQ ID NO:8 or the nucleic acid sequence set forth in SEQ ID NO:287; a fifth polynucleotide having the nucleic acid sequence set forth in SEQ ID NO: 13 or the nucleic acid sequence set forth in SEQ ID NO:14 or the nucleic acid sequence set forth in SEQ ID NO:296; and a sixth polynucleotide having the nucleic acid sequence set forth in SEQ ID NO:53 or the nucleic acid sequence set forth in SEQ ID NO:290 or the nucleic acid sequence set forth in SEQ ID NO:332.

According to certain embodiments, the subject is a mammal selected from the group consisting of a human, cat, dog, monkey, mouse, pig, rabbit, and rat. According to other embodiments, the subject is a test subject. According to typical embodiments the test subject is a rat. The test compound may be administered to the subject in a form selected from the group consisting of intravenously (IV), orally (PO, per os), intraperitoneally (IP), intranasal, inhalation, eye drops and ointments. According to yet other embodiments, the biological sample is selected from the group consisting of kidney tissue, body fluid and body secretion.

The compound may be administered once or the administration may be repeated at any desired regime. It is to be understood that the selected period of time after which the sample is obtained refers to the time after the last compound administration.

According to other embodiments, the test compound is a nephrotoxic agent. According to one embodiment, the nephrotoxic agent is selected from the group consisting of aminoglycosides; platinum based chemotherapy agents; heavy metals; DNA interacting drugs; antifungal agents; proximal tubule damaging agents; and vasoconstriction compounds.

According to one embodiment, the renal injury is associated with at least one kidney disease or pathology, selected from the group consisting of nephrotoxicity, renal toxicity, nephritis, kidney necrosis, kidney damage, glomerular and tubular injury, focal segmental glomerulosclerosis, kidney dysfunction, nephritic syndrome, acute renal failure, chronic renal failure, proximal tubal dysfunction, acute kidney transplant rejection and chronic kidney transplant refection.

The method can also be used for predicting at least one toxic effect of the compound; predicting the progression of a toxic effect of the compound; predicting the renal toxicity of the compound; or identifying an agent that modulates the onset or progression of a toxic response.

According to certain embodiments, the selected time period after which the sample is obtained from the test subject is prior to the appearance of histopathological or clinical indications of renal injury. According to one embodiment, the selected time period is any one of about 1 day, about 5 days, about 7 days, about 14 days, about 21 or about 28 days after administering the compound to the at least one test subject. According to typical embodiments, the selected time period is 1 day or less after administration. According to typical embodiments, the selected time period is 5 days after administration. According to other typical embodiments, the selected time period is 7 days after administration.

According to other embodiments, the classifier is a random forest classifier. In alternative embodiments, the classifier may be another linear or non-linear classifier. According to currently typical embodiments the classifier for renal injury is capable of performing with a training log odds ratio of greater than or equal to 3.75.

Any method for detecting the marker expression as is known to a person skilled in the art may be used according to the teachings of the present invention. According to certain embodiments, the expression level of the markers is detected at the polynucleotide level by an amplification or hybridization assay. According to typical embodiments, the amplification assay is selected from the group consisting of quantitative or semi-quantitative PCR, Northern blot, dot or slot blot, nuclease protection and microarray assays. According to other embodiments, the expression level of the markers is detected at the polypeptide level by an immunoassay. According to typical embodiments the immunoassay is selected from the group consisting of an ELISA, an RIA, a slot blot, immunohistochemical assay, FACS, a radioimaging assay or a Western blot.

The present invention also provides a kit for detecting renal injury comprising a set of probes or pairs of primers that hybridize to at least a plurality of markers and reagents for detecting the plurality of polynucleotide markers. The kit is characterized in that (i) said plurality of polynucleotide markers comprises a first polynucleotide having a the nucleic acid sequence set forth in SEQ ID NO:2 or the nucleic acid sequence set forth in SEQ ID NO:254; a second polynucleotide having the nucleic acid sequence set forth in SEQ ID NO:4 or the nucleic acid sequence set forth in SEQ ID NO:5 or the nucleic acid sequence set forth in SEQ ID NO:266 or the nucleic acid sequence set forth in SEQ ID NO:329; a third polynucleotide having the nucleic acid sequence set forth in SEQ ID NO:7 or the nucleic acid sequence set forth in SEQ ID NO:8 or the nucleic acid sequence set forth in SEQ ID NO:287; and a fourth polynucleotide having the nucleic acid sequence set forth in SEQ ID NO:13 or the nucleic acid sequence set forth in SEQ ID NO:14 or the nucleic acid sequence set forth in SEQ ID NO:296; or

(ii) said plurality of polynucleotide markers comprises a first polynucleotide having the nucleic acid sequence set forth in SEQ ID NO:2 or the nucleic acid sequence set forth in SEQ ID NO:254; a second polynucleotide having the nucleic acid sequence set forth in SEQ ID NOs:4 or the nucleic acid sequence set forth in SEQ ID NO:5 or the nucleic acid sequence set forth in SEQ ID NO:266 or the nucleic acid sequence set forth in SEQ ID NO:329; a third polynucleotide having the nucleic acid sequence set forth in SEQ ID NO:49 or the nucleic acid sequence set forth in SEQ ID NO:263; and a fourth polynucleotide having the nucleic acid sequence set forth in SEQ ID NO:53 or the nucleic acid sequence set forth in SEQ ID NO:290 or the nucleic acid sequence set forth in SEQ ID NO:332; or

(iii) said plurality of polynucleotide markers comprises a first polynucleotide having the nucleic acid sequence set forth in SEQ ID NO:2 or the nucleic acid sequence set forth in SEQ ID NO:254; a second polynucleotide having the nucleic acid sequence set forth in SEQ ID NO:4 or the nucleic acid sequence set forth in SEQ ID NO:5 or the nucleic acid sequence set forth in SEQ ID NO:266 or the nucleic acid sequence set forth in SEQ ID NO:329; a third polynucleotide having the nucleic acid sequence set forth in SEQ ID NO:49 or the nucleic acid sequence set forth in SEQ ID NO:263; a fourth polynucleotide having the nucleic acid sequence set forth in SEQ ID NO:7 or the nucleic acid sequence set forth in SEQ ID NO:8 or the nucleic acid sequence set forth in SEQ ID NO:287; a fifth polynucleotide having the nucleic acid sequence set forth in SEQ ID NO: 13 or the nucleic acid sequence set forth in SEQ ID NO:14 or the nucleic acid sequence set forth in SEQ ID NO:296; and a sixth polynucleotide having r the nucleic acid sequence set forth in SEQ ID NO:53 or the nucleic acid sequence set forth in SEQ ID NO:290 or the nucleic acid sequence set forth in SEQ ID NO:332.

In one embodiment, the set comprises a probe or pair of primers having the nucleic acids sequence set forth in SEQ ID NO:252, SEQ ID NO:253 or a combination thereof; and

(i) a probe or pair of primer having the nucleic acid sequence set forth in SEQ ID NOs:264, 265 or a combination thereof, or SEQ ID NOs:327, 328 or a combination thereof; SEQ ID NOs:285, 286 or a combination thereof; and SEQ ID NOs:294, 295 or a combination thereof; or

(ii) a probe or pair of primer having the nucleic acid sequence set forth in SEQ ID NOs:264, 265 or a combination thereof, or SEQ ID NOs:327, 328 or a combination thereof, or SEQ ID NOs:261, 262 or a combination thereof; SEQ ID NOs:288, 289 or a combination thereof; and SEQ ID NOs:330, 331 or a combination thereof; or

(iii) a probe or pair of primer having the nucleic acid sequence set forth in SEQ ID NOs: 264, 265 or a combination thereof, or SEQ ID NOs:327, 328, or a combination thereof; SEQ ID NOs:261, 262 or a combination thereof; SEQ ID NOs: 285, 286 or a combination thereof; SEQ ID NOs:294, 295 or a combination thereof; and SEQ ID NOs: 288, 289 or a combination thereof or SEQ ID NOs:330, 331 or a combination thereof.

According to one embodiment, the kit comprises reagents for detecting the marker expression by employing a NAT-based technology. In one embodiment, the NAT-based assay is selected from the group consisting of a PCR, Real-Time PCR, LCR, Self-Sustained Synthetic Reaction, Q-Beta Replicase, Cycling Probe Reaction, Branched DNA, RFLP analysis, DGGE/TGGE, Single-Strand Conformation Polymorphism, Dideoxy Fingerprinting, Microarrays, Fluorescence, *In Situ* Hybridization or Comparative Genomic Hybridization.

Other objects, features and advantages of the present invention will become clear from the following description and drawings.

**Brief description of the figures**

**Figure 1** presents chemical structure of T1 drug

**Figure 2** presents chemical structure of T2 drug

**Figure 3** presents histological section of the kidney, magnified x200. Figure 3A presents a control animal. The arrows represent normal aspects of cortical tubules (eosinophilic cytoplasm). Figure 3B presents 50mg/kg T2-treated animal. The arrows represent basophilic tubules in the cortex, indicating post-necrotic regeneration.

**Figure 4** presents histological section of the kidney, magnified x400. Figure 4A presents a control animal. The arrows represent normal aspects of cortical tubules (eosinophilic cytoplasm). Figure 4B presents 50mg/kg T2-treated animal. The arrows represent basophilic tubules in the cortex, indicating post-necrotic regeneration.

**Figure 5** presents histological section of the kidney, magnified x600. Figure 5A presents a control animal. The arrows represent normal aspects of cortical tubules (eosinophilic cytoplasm). Figure 5B presents 50mg/kg T2-treated animal. The arrows represent basophilic tubules in the cortex, indicating post-necrotic regeneration.

**Figure 6** presents the ROC curve of the Random-Forest classifier based on the Real Time PCR measurements from the validation stage of the labeled samples. The ROC curve is based on the out-of-the-bag method for estimating the performance of a Random-Forest classifier.

**Detailed description of the invention**

The present invention provides a diagnostic isolated polynucleotide marker, as well as a method and a kit for predicting the onset of renal injury.

The present invention is based in part on gene expression data obtained from sub-acute or short-term treatments with a certain compound. The present invention now discloses necessary and sufficient sets of genes and specific signatures comprising these genes that allow gene expression data to be used to identify the ability of a compound treatment to induce late onset renal injury before actual histological or clinical indication are apparent. Further, the invention provides kits comprising means for detecting the disclosed gene sets and signatures. The means and methods provided by the present invention enable the detection of a compound toxicity using short term studies, avoiding lengthy and costly long term studies.

Definitions

"Marker": in some embodiments, the phrase "marker" in the context of the present invention is used interchangeably with the phrase "gene" and refers to a nucleic acid fragment, a peptide, or a polypeptide, which is differentially expressed in a sample taken from subjects having one of the herein-described diseases or conditions, as compared to a comparable sample taken from subjects who do not have one the above-described diseases or conditions.

As used herein, the term "expression" refers to the amount of a nucleic acid molecule, peptide or polypeptide present in a sample at a certain time point, which amount may be the result of any process taking place in a cell within the sample, including, but not limited to, gene transcription and translation and degradation or stabilization of the gene product.

"Renal injury" refers to any damage to the kidney that can be caused by primary renal dysfunction (i.e Alport's syndrome, response to external substances (nephrotoxicants), infections, altered blood supply, malignancies, etc.) or secondary renal pathology (i.e. complications of diabetes mellitus, multiple myeloma, etc.).

Renal injury includes but is not limited to: nephritis, kidney necrosis, kidney damage, nephrotoxicity, renal toxicity, glomerular and tubular injury, focal segmental glomerulosclerosis, kidney dysfunction, nephritic syndrome, acute renal failure, chronic renal failure, proximal tubal dysfunction, acute kidney transplant rejection, chronic kidney transplant refection.

"Nephrotoxicant": in the context of the present invention is used interchangeably with the phrase "nephrotoxic agent" and/or "renal toxin", and refers to every substance (chemical compound and/or protein, recombinant or endogenous, including toxins or medications) that accumulates or that its clearance is via the renal system and causes renal injury. Examples of substance families that can cause such renal injury include but are not limited to: Aminoglycosides (i.e. Gentamicin Tobramycin, Amikacin, Kanamycin, Neomycin, Netilmicin, Paromomycin, Streptomycin, Tobramycin and Apramycin); Platinum based chemotherapy (i.e. Cisplatic, carboplatin); Heavy metals (i.e. Cadmium Chloride, Chromium, Arsenic, Lead, Mercury, Mangane); DNA interacting drugs (i.e. Doxorubicin, Daunorubicin, Epirubicin, Idarubicin, Mitoxantrone); Antifungal (i.e. Amphotericin B); Proximal tubule damaging agents (i.e. Acyclovir, Foscarnet, Pentamidine, Ifosfamide); and Vasoconstriction compounds (i.e. Radiocontrast agents, Cyclosporine, Tacrolimus).

As used herein, the term "test subject" refers to a subject receiving a compound or a treatment in order to evaluate the effect of the compound or treatment on the subject, including its efficacy, side effects, adverse effects and the like. According to typical embodiments, the test subject is a mammal.

"Multivariate dataset" as used herein, refers to any dataset comprising a plurality of different variables including but not limited to chemogenomic datasets comprising intensity measurements from gene expression experiments, such as those carried out on polynucleotide microarrays, or multiple protein binding affinities measured using a protein chip. Other examples of multivariate data include assemblies of data from a plurality of standard toxicological or pharmacological assays (e.g., blood analytes measured using enzymatic assays, antibody based ELISA or other detection techniques).

"Variable" as used herein, refers to any value that may vary. For example, variables may include relative or absolute amounts of biological molecules, such as mRNA or proteins, or other biological metabolites. Variables may also include dosing amounts of test compounds.

"Classifier" as used herein, refers to a function of a set of variables that is capable of answering a classification question. A "classification question" may be of any type susceptible to yielding a yes or no answer (e.g., "Is the unknown a member of the class or does it belong with everything else outside the class?"). A valid classifier is defined as a classifier capable of achieving a performance for its classification task at or above a selected threshold value. For example, a log odds ratio > 3.75 represents a preferred threshold of the present invention. Higher or lower threshold values may be selected depending of the specific classification task.

"Random Forest" as used herein, refers to a type of non-linear classifier based on the majority decision of a collection of decision trees, each stating the outcome label (e.g. - the answer to the classification question), according to the values of the input variables. (Breiman, Leo "Random Forests". Machine Learning 45 (1), 5-32, 2001). We use the random forest algorithm supplied by the R programming language.

"Signature" as used herein, refers to a combination of variables. As well as, possibly, a classification algorithm that provides a unique value or function capable of answering a classification question. A signature may include as few as one variable. Signatures include but are not limited to Random Forest classifiers.

"Log odds ratio" or "LOR" is used herein to summarize the performance of classifiers or signatures. LOR is defined generally as the natural log of the ratio of predicting a subject to be positive when it is positive, versus the odds of predicting a subject to be positive when it is negative. LOR is estimated herein using a set of training or test cross-validation partitions according to the following equation,

$$LOR = \ln \frac{\left(\sum_{i-1}^{c} TP_i + 0.5\right) * \left(\sum_{i=1}^{c} TN_i + 0.5\right)}{\left(\sum_{i=1}^{c} FP_i + 0.5\right) * \left(\sum_{i=1}^{c} FN_i + 0.5\right)}$$

Where c (typically c =100 as described herein) equals the number of partitions, and $TP_i$,$TN_i$,$FP_i$ and $FN_i$ represent the number of true positive, true negative, false positive, and false negative occurrences in the test set of the $i^{th}$ partition, respectively.

"Accuracy" as used herein, refers to an alternative mean of summarizing the performance of classifiers of signatures. Accuracy is the percentage of correctly labeled samples. It is estimated herein using a set of training or test cross-validation partitions according to the following equation,

$$ACC = 100 * \frac{\sum_{i=1}^{c} (TP_i + TN_i)}{\sum_{i=1}^{c} N_i}$$

Where c (typically c =100 as described herein) equals the number of partitions, $TP_i$,$TN_i$, and $N_i$ represent the number of true positive, true negative, and all samples in the test set of the $i^{th}$ partition, respectively.

"Array" as used herein, refers to a set of different biological molecules (e.g., polynucleotides, peptides, carbohydrates, etc.). An array may be immobilized in or on one or more solid substrates (e.g., glass slides, beads, or gels) or may be a collection of different molecules in solution (e.g., a set of PCR primers). An array may include a plurality of biological polymers of a single class (e.g., polynucleotides) or a mixture of different classes of biopolymers (e.g., an array including both proteins and nucleic acids immobilized on a single substrate).

"Array data" as used herein refers to any set of constants and/or variables that may be observed, measured or otherwise derived from an experiment using an array, including but not limited to: fluorescence (or other signaling moiety) intensity ratios, binding affinities, hybridization stringency, temperature, buffer concentrations.

"Proteomic data" as used herein refers to any set of constants and/or variables that may be observed, measured or otherwise derived from an experiment involving a plurality of mPvNA translation products (e.g., proteins, peptides, etc) and/or small molecular weight metabolites or exhaled gases associated with these translation products.

General Methods of the Invention

The present invention provides a novel diagnostic isolated polynucleotide marker useful for predicting the onset of renal injury. Also disclosed is a list of genes that may be used to create a signature that performs above a certain minimal threshold level for a specific prediction of renal injury. This set of genes also may be used to derive additional signatures with varying numbers of genes and levels of performance for particular applications (e.g., diagnostic assays and devices).

Using Signatures for Predicting Renal Injury

A diagnostic usually consists in performing one or more assays and in assigning a sample to one or more categories based on the results of the assay(s). Desirable attributes of diagnostic assays include high sensitivity and specificity measured in terms of low false negative and false positive rates and overall accuracy. Because diagnostic assays are often used to assign large number of samples to given categories, the issues of cost per assay and throughput (number of assays per unit time or per worker hour) are of paramount importance. Typically the development of a diagnostic assay involves the following steps: (1) define the end point to diagnose, e.g., renal injury; (2) identify one or more markers whose alteration correlates with the end point, e.g., elevation of expression of a gene set; and (3) develop a specific, accurate, high - throughput and cost-effective assay for that marker. In order to increase throughput and decrease costs several diagnostics are often combined in a panel of assays, especially when the detection methodologies are compatible. For example several ELISA-based assays, each using different antibodies to ascertain different end points may be combined in a single panel and commercialized as a single kit. Even in this case, however, each of the ELISA- based assays had to be developed individually often requiring the generation of specific reagents.

Disclosed are signatures comprising as few as 2 genes, preferably as few as 4 genes, preferably as few as 5 genes, that are useful for determining a therapeutic or toxicological end-point for renal injury. These signatures (and the genes from which they are composed) may also be used in the design of improved diagnostic kits that answer the same questions as a large microarray but using a much smaller fraction of data. Generally, the reduction of information in a large chemogenomic dataset to a simple signature enables much simpler devices compatible with low cost high throughput multi-analyte measurement.

Consequently, the signatures provide the ability to produce cheaper, higher throughput, diagnostic measurement methods or strategies. In particular, the diagnostic marker is useful in diagnostic assays and the associated diagnostic kits. As used herein, diagnostic assays include assays that may be used for test subjects, patient prognosis and therapeutic monitoring.

Diagnostic marker sets may include markers representing a subset of genes disclosed in Table 12-18, and tables 26-27, and the genes homologous thereto, disclosed in table 21. The diagnostic marker set can be a plurality of polynucleotides or polypeptides representing specific genes in the list contained in these tables. Such biopolymer marker sets are immediately applicable in any of the diagnostic assay methods (and the associate kits) well known for polynucleotides and polypeptides (e.g., DNA arrays, RT-PCR, immunoassays or other receptor based assays for polypeptides or proteins). Thus, a very simple diagnostic array may be designed that answers 3 or 4 specific classification questions and includes only 10-20 polynucleotides representing the approximately 5-10 genes in each of the signatures. Of course, depending on the level of accuracy required the LOR threshold for selecting a sufficient gene signature may be varied.

The diagnostic marker sets may be provided in kits, wherein the kits may or may not comprise additional reagents or components necessary for the particular diagnostic application in which the marker set is to be employed. Thus, for a polynucleotide array applications, the diagnostic marker sets may be provided in a kit which further comprises one or more of the additional requisite reagents for amplifying and/or labeling a microarray probe or target (e.g., polymerases, labeled nucleotides, and the a variety of array formats (for either polynucleotides and/or polypeptides) are well-known in the art and may be used with the methods and subsets produced by the present invention. Photolithographic or micromirror methods may be used to spatially direct light-induced chemical modifications of spacer units or functional groups resulting in attachment at specific localized regions on the surface of the substrate. Light-directed methods of controlling reactivity and immobilizing chemical compounds on solid substrates are well-known in the art and described in U.S. Patent Nos. 4,562,157, 5,143,854, 5,556,961, 5,968,740, and 6,153,744, and PCT publication WO 99/42813.

Alternatively, a plurality of molecules may be attached to a single substrate by precise deposition of chemical reagents. For example, methods for achieving high spatial resolution in depositing small volumes of a liquid reagent on a solid substrate are disclosed in U.S Patent Nos. 5,474,796 and 5,807,522.

It should also be noted that in many cases a single diagnostic device may not satisfy all needs. However, even for an initial exploratory investigation (e.g., classifying drug- treated rats) DNA arrays with sufficient gene sets of varying size (number of genes), each adapted to a specific follow-up technology, can be created. In addition, in the case of drug-treated rats, different arrays may be defined for each tissue.

Alternatively, a single substrate may be produced with several different small arrays of genes in different areas on the surface of the substrate. Each of these different arrays may represent a sufficient set of genes for the same classification question but with a different optimal gene signature for each different tissue. Thus, a single array could be used for particular diagnostic question regardless of the tissue source of the sample (or even if the sample was from a mixture of tissue sources, e.g., in a forensic sample).

The genes identified in Table 12 may be used as markers or drug targets to evaluate the effects of a candidate drug, chemical compound or other agent on a cell or tissue sample. The genes may also be used as drug targets to screen for agents that modulate their expression and/or activity. In various formats, a candidate drug or agent can be screened for the ability to stimulate the transcription or expression of a given marker or markers or to down-regulate or counteract the transcription or expression of a marker or markers. One can also compare the specificity of a drug's effects by looking at the number of markers which the drug induces and comparing them. More specific drugs will have less transcriptional targets. Similar sets of markers identified for two drugs may indicate a similarity of effects.

Assays to monitor the expression of a marker or markers as defined in Table 12 may utilize any available means of monitoring for changes in the expression level of the nucleic acids of the invention. As used herein, an agent is said to modulate the expression of a nucleic acid of the invention if it is capable of up-or down-regulating expression of the nucleic acid in a cell.

Nucleic Acid Assay Formats

The genes identified as being differentially expressed upon exposure to a known renal toxin (Tables 12-18 and 26-27, and Table 21) may be used in a variety of nucleic acid detection assays to detect or quantify the expression level of a gene or multiple genes in a given sample.

Any assay format to detect gene expression may be used. For example, traditional Northern blotting, dot or slot blot, nuclease protection, primer directed amplification, RT-PCR, semi-or quantitative PCR, branched-chain DNA and differential display methods may be used for detecting gene expression levels. Those methods are useful for some embodiments of the invention. In cases where smaller numbers of genes are detected, amplification based assays may be most efficient.

Methods and assays of the invention, however, may be most efficiently designed with hybridization-based methods for detecting the expression of a large number of genes.

Any hybridization assay format may be used, including solution-based and solid support-based assay formats. Solid supports containing oligonucleotide probes for differentially expressed genes can be filters, polyvinyl chloride dishes, particles, beads, microparticles or silicon or glass based chips, etc. Such chips, wafers and hybridization methods are widely available, for example, those disclosed by Beattie (WO 95/11755).

Any solid surface, to which oligonucleotides can be bound, either directly or indirectly, either covalently or non-covalently, can be used. A preferred solid support is a high density array or DNA chip. These contain a particular oligonucleotide probe in a predetermined location on the array. Each predetermined location may contain more than one molecule of the probe, but each molecule within the predetermined location has an identical sequence. Such predetermined locations are termed features. There may be, for example, from 2, 10, 100, 1000 to 10,000, 100,000 or 400,000 or more of such features on a single solid support. The solid support or the area within which the probes are attached may be on the order of about a square centimeter. Probes corresponding to the genes of Tables 12-18 and 26-27, or Table 21 may be attached to single or multiple solid support structures, e. g., the probes may be attached to a single chip or to multiple chips to comprise a chip set.

Oligonucleotide probe arrays for expression monitoring can be made and used according to any techniques known in the art (see for example, Lockhart et al. (1996), NatBiotechnol 14: 1675-1680; McGall et al. (1996), Proc Nat Acad Sci USA 93: 13555-13460). Such probe arrays may contain at least two or more oligonucleotides that are complementary to or hybridize to two or more of the genes described in Tables 12-18 and 26-27 or Table 21. Such arrays may contain oligonucleotides that are complementary to or hybridize to any subset of the genes in any one or all of Tables 14, 15, 17, 18, 26 and 27. Alternatively, such arrays contain oligonucleotides that are complementary to or hybridize to all or nearly all of the genes in any one of Tables 13, 16, 26 and 27. Preferred arrays contain all or nearly all of the genes listed in Tables 12-18 and 26-27 or Table 21. Also, arrays are constructed that contain oligonucleotides to detect all or nearly all of the genes in any one or all of Tables 12-18, 26-27 and Table 21, in particular Tables 13, 14, 16, 27, 26 and 27 on a single solid support substrate, such as a chip.

NAT Assays

Detection of a nucleic acid of interest in a biological sample may also optionally be effected by NAT-based assays, which involve nucleic acid amplification technology, such as PCR for example (or variations thereof such as real-time PCR for example).

As used herein, a "primer" defines an oligonucleotide which is capable of annealing to (hybridizing with) a target sequence, thereby creating a double stranded region which can serve as an initiation point for DNA synthesis under suitable conditions.

Amplification of a selected, or target, nucleic acid sequence may be carried out by a number of suitable methods. See generally Kwoh et al., 1990, Am. Biotechnol. Lab. 8:14 Numerous amplification techniques have been described and can be readily adapted to suit particular needs of a person of ordinary skill. Non-limiting examples of amplification techniques include polymerase chain reaction (PCR), ligase chain reaction (LCR), strand displacement amplification (SDA), transcription-based amplification, the q3 replicase system and NASBA (Kwoh et al., 1989, Proc. Natl. Acad. Sci. USA 86, 1173-1177; Lizardi et al., 1988, BioTechnology 6:1197-1202; Malek et al., 1994, Methods Mol. Biol., 28:253-260; and Sambrook et al., 1989, supra).

The terminology "amplification pair" (or "primer pair") refers herein to a pair of oligonucleotides (oligos) of the present invention, which are selected to be used together in amplifying a selected nucleic acid sequence by one of a number of types of amplification processes, preferably a polymerase chain reaction. Other types of amplification processes include ligase chain reaction, strand displacement amplification, or nucleic acid sequence-based amplification, as explained in greater detail below. As commonly known in the art, the oligos are designed to bind to a complementary sequence under selected conditions.

Amplification of a nucleic acid sample from a patient is amplified under conditions which favor the amplification of the most abundant differentially expressed nucleic acid. Alternatively, RT-PCR is carried out on an mRNA sample from a patient under conditions which favor the amplification of the most abundant mRNA. Also alternatively, the amplification of the differentially expressed nucleic acids is carried out simultaneously. It will be realized by a person skilled in the art that such methods could be adapted for the detection of differentially expressed proteins instead of differentially expressed nucleic acid sequences.

The nucleic acid (i.e. DNA or RNA) for practicing the present invention may be obtained according to well known methods.

Oligonucleotide primers of the present invention may be of any suitable length, depending on the particular assay format and the particular needs and targeted genomes employed. Optionally, the oligonucleotide primers are at least 12 nucleotides in length, preferably between 15 and 24 molecules, and they may be adapted to be especially suited to a chosen nucleic acid amplification system. As commonly known in the art, the oligonucleotide primers can be designed by taking into consideration the melting point of hybridization thereof with its targeted sequence (Sambrook et al., 1989, Molecular Cloning -A Laboratory Manual, 2nd Edition, CSH Laboratories; Ausubel et al., 1989, in Current Protocols in Molecular Biology, John Wiley & Sons Inc., N.Y.).

It will be appreciated that antisense oligonucleotides may be employed to quantify expression of a splice isoform of interest. Such detection is effected at the pre-mRNA level. Essentially the ability to quantitate transcription from a splice site of interest can be effected based on splice site accessibility. Oligonucleotides may compete with splicing factors for the splice site sequences. Thus, low activity of the antisense oligonucleotide is indicative of splicing activity.

The polymerase chain reaction and other nucleic acid amplification reactions are well known in the art (various non-limiting examples of these reactions are described in greater detail below). The pair of oligonucleotides according to this aspect of the present invention are preferably selected to have compatible melting temperatures (Tm), e.g., melting temperatures which differ by less than that 7°C, preferably less than 5°C, more preferably less than 4°C, most preferably less than 3°C, ideally between 3 °C and 0 °C.

Polymerase Chain Reaction (PCR): The polymerase chain reaction (PCR), as described in U.S. Pat. Nos. 4,683,195 and 4,683,202 to Mullis and Mullis et al., is a method of increasing the concentration of a segment of target sequence in a mixture of genomic DNA without cloning or purification. This technology provides one approach to the problems of low target sequence concentration. PCR can be used to directly increase the concentration of the target to an easily detectable level. This process for amplifying the target sequence involves the introduction of a molar excess of two oligonucleotide primers which are complementary to their respective strands of the double-stranded target sequence to the DNA mixture containing the desired target sequence. The mixture is denatured and then allowed to hybridize. Following hybridization, the primers are extended with polymerase so as to form complementary strands. The steps of denaturation, hybridization (annealing), and polymerase extension (elongation) can be repeated as often as needed, in order to obtain relatively high concentrations of a segment of the desired target sequence.

The length of the segment of the desired target sequence is determined by the relative positions of the primers with respect to each other, and, therefore, this length is a controllable parameter. Because the desired segments of the target sequence become the dominant sequences (in terms of concentration) in the mixture, they are said to be "PCR-amplified."

Ligase Chain Reaction (LCR or LAR): The ligase chain reaction [LCR; sometimes referred to as "Ligase Amplification Reaction" (LAR)] has developed into a well-recognized alternative method of amplifying nucleic acids. In LCR, four oligonucleotides, two adjacent oligonucleotides which uniquely hybridize to one strand of target DNA, and a complementary set of adjacent oligonucleotides, which hybridize to the opposite strand are mixed and DNA ligase is added to the mixture. Provided that there is complete complementarity at the junction, ligase will covalently link each set of hybridized molecules. Importantly, in LCR, two probes are ligated together only when they base-pair with sequences in the target sample, without gaps or mismatches. Repeated cycles of denaturation, and ligation amplify a short segment of DNA. LCR has also been used in combination with PCR to achieve enhanced detection of single-base changes: see for example Segev, PCT Publication No. WO9001069 A1 (1990). However, because the four oligonucleotides used in this assay can pair to form two short ligatable fragments, there is the potential for the generation of target-independent background signal. The use of LCR for mutant screening is limited to the examination of specific nucleic acid positions.

Self-Sustained Synthetic Reaction (3SR/NASBA): The self-sustained sequence replication reaction (3SR) is a transcription-based in vitro amplification system that can exponentially amplify RNA sequences at a uniform temperature. The amplified RNA can then be utilized for mutation detection. In this method, an oligonucleotide primer is used to add a phage RNA polymerase promoter to the 5' end of the sequence of interest. In a cocktail of enzymes and substrates that includes a second primer, reverse transcriptase, RNase H, RNA polymerase and ribo-and deoxyribonucleoside triphosphates, the target sequence undergoes repeated rounds of transcription, cDNA synthesis and second-strand synthesis to amplify the area of interest. The use of 3SR to detect mutations is kinetically limited to screening small segments of DNA (e.g., 200-300 base pairs).

Q-Beta (Qβ) Replicase: In this method, a probe which recognizes the sequence of interest is attached to the replicatable RNA template for Qβ replicase. A previously identified major problem with false positives resulting from the replication of unhybridized probes has been addressed through use of a sequence-specific ligation step. However, available thermostable DNA ligases are not effective on this RNA substrate, so the ligation must be performed by T4 DNA ligase at low temperatures (37 degrees C.). This prevents the use of high temperature as a means of achieving specificity as in the LCR, the ligation event can be used to detect a mutation at the junction site, but not elsewhere.

A successful diagnostic method must be very specific. A straight-forward method of controlling the specificity of nucleic acid hybridization is by controlling the temperature of the reaction. While the 3SR/NASBA, and Qβ systems are all able to generate a large quantity of signal, one or more of the enzymes involved in each cannot be used at high temperature (i.e., > 55°C). Therefore the reaction temperatures cannot be raised to prevent non-specific hybridization of the probes. If probes are shortened in order to make them melt more easily at low temperatures, the likelihood of having more than one perfect match in a complex genome increases. For these reasons, PCR and LCR currently dominate the research field in detection technologies.

The basis of the amplification procedure in the PCR and LCR is the fact that the products of one cycle become usable templates in all subsequent cycles, consequently doubling the population with each cycle. The final yield of any such doubling system can be expressed as: $(1+X)^n = y$, where "X" is the mean efficiency (percent copied in each cycle), "n" is the number of cycles, and "y" is the overall efficiency, or yield of the reaction. If every copy of a target DNA is utilized as a template in every cycle of a polymerase chain reaction, then the mean efficiency is 100%. If 20 cycles of PCR are performed, then the yield will be $2^{20}$, or 1,048,576 copies of the starting material. If the reaction conditions reduce the mean efficiency to 85%, then the yield in those 20 cycles will be only $1.85^{20}$, or 220,513 copies of the starting material. In other words, a PCR running at 85 % efficiency will yield only 21 % as much final product, compared to a reaction running at 100 % efficiency. A reaction that is reduced to 50% mean efficiency will yield less than 1 % of the possible product.

In practice, routine polymerase chain reactions rarely achieve the theoretical maximum yield, and PCRs are usually run for more than 20 cycles to compensate for the lower yield. At 50% mean efficiency, it would take 34 cycles to achieve the million-fold amplification theoretically possible in 20, and at lower efficiencies, the number of cycles required becomes prohibitive. In addition, any background products that amplify with a better mean efficiency than the intended target will become the dominant products.

Also, many variables can influence the mean efficiency of PCR, including target DNA length and secondary structure, primer length and design, primer and dNTP concentrations, and buffer composition, to name but a few. Contamination of the reaction with exogenous DNA (e.g., DNA spilled onto lab surfaces) or cross-contamination is also a major consideration. Reaction conditions must be carefully optimized for each different primer pair and target sequence, and the process can take days, even for an experienced investigator. The laboriousness of this process, including numerous technical considerations and other factors, presents a significant drawback to using PCR in the clinical setting. Indeed, PCR has yet to penetrate the clinical market in a significant way. The same concerns arise with LCR, as LCR must also be optimized to use different oligonucleotide sequences for each target sequence. In addition, both methods require expensive equipment, capable of precise temperature cycling.

Many applications of nucleic acid detection technologies, such as in studies of allelic variation, involve not only detection of a specific sequence in a complex background, but also the discrimination between sequences with few, or single, nucleotide differences. One method of the detection of allele-specific variants by PCR is based upon the fact that it is difficult for Taq polymerase to synthesize a DNA strand when there is a mismatch between the template strand and the 3' end of the primer. An allele-specific variant may be detected by the use of a primer that is perfectly matched with only one of the possible alleles; the mismatch to the other allele acts to prevent the extension of the primer, thereby preventing the amplification of that sequence. This method has a substantial limitation in that the base composition of the mismatch influences the ability to prevent extension across the mismatch, and certain mismatches do not prevent extension or have only a minimal effect.

A similar 3'-mismatch strategy is used with greater effect to prevent ligation in the LCR. Any mismatch effectively blocks the action of the thermostable ligase, but LCR still has the drawback of target-independent background ligation products initiating the amplification. Moreover, the combination of PCR with subsequent LCR to identify the nucleotides at individual positions is also a clearly cumbersome proposition for the clinical laboratory.

The direct detection method may be, for example a cycling probe reaction (CPR) or a branched DNA analysis.

When a sufficient amount of a nucleic acid to be detected is available, there are advantages to detecting that sequence directly, instead of making more copies of that target, (e.g., as in PCR and LCR). Most notably, a method that does not amplify the signal exponentially is more amenable to quantitative analysis. Even if the signal is enhanced by attaching multiple dyes to a single oligonucleotide, the correlation between the final signal intensity and amount of target is direct. Such a system has an additional advantage that the products of the reaction will not themselves promote further reaction, so contamination of lab surfaces by the products is not as much of a concern. Recently devised techniques have sought to eliminate the use of radioactivity and/or improve the sensitivity in automatable formats. Two examples are the "Cycling Probe Reaction" (CPR), and "Branched DNA" (bDNA).

Cycling probe reaction (CPR): The cycling probe reaction (CPR), uses a long chimeric oligonucleotide in which a central portion is made of RNA while the two termini are made of DNA. Hybridization of the probe to a target DNA and exposure to a thermostable RNase H causes the RNA portion to be digested. This destabilizes the remaining DNA portions of the duplex, releasing the remainder of the probe from the target DNA and allowing another probe molecule to repeat the process. The signal, in the form of cleaved probe molecules, accumulates at a linear rate. While the repeating process increases the signal, the RNA portion of the oligonucleotide is vulnerable to RNases that may carried through sample preparation.

Branched DNA: Branched DNA (bDNA), involves oligonucleotides with branched structures that allow each individual oligonucleotide to carry 35 to 40 labels (e.g., alkaline phosphatase enzymes). While this enhances the signal from a hybridization event, signal from non-specific binding is similarly increased.

The detection of at least one sequence change may be accomplished by, for example restriction fragment length polymorphism (RFLP analysis), allele specific oligonucleotide (ASO) analysis, Denaturing/Temperature Gradient Gel Electrophoresis (DGGE/TGGE), Single-Strand Conformation Polymorphism (SSCP) analysis or Dideoxy fingerprinting (ddF).

The demand for tests which allow the detection of specific nucleic acid sequences and sequence changes is growing rapidly in clinical diagnostics. As nucleic acid sequence data for genes from humans and pathogenic organisms accumulates, the demand for fast, cost-effective, and easy-to-use tests for as yet mutations within specific sequences is rapidly increasing.

A handful of methods have been devised to scan nucleic acid segments for mutations. One option is to determine the entire gene sequence of each test sample (e.g., a bacterial isolate). For sequences under approximately 600 nucleotides, this may be accomplished using amplified material (e.g., PCR reaction products). This avoids the time and expense associated with cloning the segment of interest. However, specialized equipment and highly trained personnel are required, and the method is too labor-intense and expensive to be practical and effective in the clinical setting.

In view of the difficulties associated with sequencing, a given segment of nucleic acid may be characterized on several other levels. At the lowest resolution, the size of the molecule can be determined by electrophoresis by comparison to a known standard run on the same gel. A more detailed picture of the molecule may be achieved by cleavage with combinations of restriction enzymes prior to electrophoresis, to allow construction of an ordered map. The presence of specific sequences within the fragment can be detected by hybridization of a labeled probe, or the precise nucleotide sequence can be determined by partial chemical degradation or by primer extension in the presence of chain-terminating nucleotide analogs.

Restriction fragment length polymorphism (RFLP): For detection of single-base differences between like sequences, the requirements of the analysis are often at the highest level of resolution. For cases in which the position of the nucleotide in question is known in advance, several methods have been developed for examining single base changes without direct sequencing. For example, if a mutation of interest happens to fall within a restriction recognition sequence, a change in the pattern of digestion can be used as a diagnostic tool (e.g., restriction fragment length polymorphism [RFLP] analysis).

Single point mutations have been also detected by the creation or destruction of RFLPs. Mutations are detected and localized by the presence and size of the RNA fragments generated by cleavage at the mismatches. Single nucleotide mismatches in DNA heteroduplexes are also recognized and cleaved by some chemicals, providing an alternative strategy to detect single base substitutions, generically named the "Mismatch Chemical Cleavage" (MCC). However, this method requires the use of osmium tetroxide and piperidine, two highly noxious chemicals which are not suited for use in a clinical laboratory.

RFLP analysis suffers from low sensitivity and requires a large amount of sample. When RFLP analysis is used for the detection of point mutations, it is, by its nature, limited to the detection of only those single base changes which fall within a restriction sequence of a known restriction endonuclease. Moreover, the majority of the available enzymes has 4 to 6 base-pair recognition sequences, and cleave too frequently for many large-scale DNA manipulations. Thus, it is applicable only in a small fraction of cases, as most mutations do not fall within such sites.

A handful of rare-cutting restriction enzymes with 8 base-pair specificities have been isolated and these are widely used in genetic mapping, but these enzymes are few in number, are limited to the recognition of G+C-rich sequences, and cleave at sites that tend to be highly clustered. Recently, endonucleases encoded by group I introns have been discovered that might have greater than 12 base-pair specificity, but again, these are few in number.

Allele specific oligonucleotide (ASO): If the change is not in a recognition sequence, then allele-specific oligonucleotides (ASOs), can be designed to hybridize in proximity to the mutated nucleotide, such that a primer extension or ligation event can bused as the indicator of a match or a mismatch. Hybridization with radioactively labeled allelic specific oligonucleotides (ASO) also has been applied to the detection of specific point mutations. The method is based on the differences in the melting temperature of short DNA fragments differing by a single nucleotide. Stringent hybridization and washing conditions can differentiate between mutant and wild-type alleles. The ASO approach applied to PCR products also has been extensively utilized by various researchers to detect and characterize point mutations in ras genes and gsp/gip oncogenes. Because of the presence of various nucleotide changes in multiple positions, the ASO method requires the use of many oligonucleotides to cover all possible oncogenic mutations.

With either of the techniques described above (i.e., RFLP and ASO), the precise location of the suspected mutation must be known in advance of the test. That is to say, they are inapplicable when one needs to detect the presence of a mutation within a gene or sequence of interest.

Denaturing/Temperature Gradient Gel Electrophoresis (DGGE/TGGE): Two other methods rely on detecting changes in electrophoretic mobility in response to minor sequence changes. One of these methods, termed "Denaturing Gradient Gel Electrophoresis" (DGGE) is based on the observation that slightly different sequences will display different patterns of local melting when electrophoretically resolved on a gradient gel. In this manner, variants can be distinguished, as differences in melting properties of homoduplexes versus heteroduplexes differing in a single nucleotide can detect the presence of mutations in the target sequences because of the corresponding changes in their electrophoretic mobilities. The fragments to be analyzed, usually PCR products, are "clamped" at one end by a long stretch of G-C base pairs (30-80) to allow complete denaturation of the sequence of interest without complete dissociation of the strands. The attachment of a GC "clamp" to the DNA fragments increases the fraction of mutations that can be recognized by DGGE. Attaching a GC clamp to one primer is critical to ensure that the amplified sequence has a low dissociation temperature. Modifications of the technique have been developed, using temperature gradients, and the method can be also applied to RNA:RNA duplexes.

Limitations on the utility of DGGE include the requirement that the denaturing conditions must be optimized for each type of DNA to be tested. Furthermore, the method requires specialized equipment to prepare the gels and maintain the needed high temperatures during electrophoresis. The expense associated with the synthesis of the clamping tail on one oligonucleotide for each sequence to be tested is also a major consideration. In addition, long running times are required for DGGE. The long running time of DGGE was shortened in a modification of DGGE called constant denaturant gel electrophoresis (CDGE). CDGE requires that gels be performed under different denaturant conditions in order to reach high efficiency for the detection of mutations.

A technique analogous to DGGE, termed temperature gradient gel electrophoresis (TGGE), uses a thermal gradient rather than a chemical denaturant gradient. TGGE requires the use of specialized equipment which can generate a temperature gradient perpendicularly oriented relative to the electrical field. TGGE can detect mutations in relatively small fragments of DNA therefore scanning of large gene segments requires the use of multiple PCR products prior to running the gel.

Single-Strand Conformation Polymorphism (SSCP): Another common method, called "Single-Strand Conformation Polymorphism" (SSCP) was developed by Hayashi, Sekya and colleagues and is based on the observation that single strands of nucleic acid can take on characteristic conformations in non-denaturing conditions, and these conformations influence electrophoretic mobility. The complementary strands assume sufficiently different structures that one strand may be resolved from the other. Changes in sequences within the fragment will also change the conformation, consequently altering the mobility and allowing this to be used as an assay for sequence variations.

The SSCP process involves denaturing a DNA segment (e.g., a PCR product) that is labeled on both strands, followed by slow electrophoretic separation on a non-denaturing polyacrylamide gel, so that intra-molecular interactions can form and not be disturbed during the run. This technique is extremely sensitive to variations in gel composition and temperature. A serious limitation of this method is the relative difficulty encountered in comparing data generated in different laboratories, under apparently similar conditions.

Dideoxy fingerprinting (ddF): The dideoxy fingerprinting (ddF) is another technique developed to scan genes for the presence of mutations. The ddF technique combines components of Sanger dideoxy sequencing with SSCP. A dideoxy sequencing reaction is performed using one dideoxy terminator and then the reaction products are electrophoresed on nondenaturing polyacrylamide gels to detect alterations in mobility of the termination segments as in SSCP analysis. While ddF is an improvement over SSCP in terms of increased sensitivity, ddF requires the use of expensive dideoxynucleotides and this technique is still limited to the analysis of fragments of the size suitable for SSCP (i.e., fragments of 200-300 bases for optimal detection of mutations).

In addition to the above limitations, all of these methods are limited as to the size of the nucleic acid fragment that can be analyzed. For the direct sequencing approach, sequences of greater than 600 base pairs require cloning, with the consequent delays and expense of either deletion sub-cloning or primer walking, in order to cover the entire fragment. SSCP and DGGE have even more severe size limitations. Because of reduced sensitivity to sequence changes, these methods are not considered suitable for larger fragments. Although SSCP is reportedly able to detect 90 % of single-base substitutions within a 200 base-pair fragment, the detection drops to less than 50 % for 400 base pair fragments. Similarly, the sensitivity of DGGE decreases as the length of the fragment reaches 500 base-pairs. The ddF technique, as a combination of direct sequencing and SSCP, is also limited by the relatively small size of the DNA that can be screened.

The step of searching for any of the nucleic acid sequences described here, in cells derived from a cancer patient can be effected by any suitable technique, including, but not limited to, nucleic acid sequencing, polymerase chain reaction, ligase chain reaction, self-sustained synthetic reaction, Qβ-Replicase, cycling probe reaction, branched DNA, restriction fragment length polymorphism analysis, mismatch chemical cleavage, heteroduplex analysis, allele-specific oligonucleotides, denaturing gradient gel electrophoresis, constant denaturant gel electrophoresis, temperature gradient gel electrophoresis and dideoxy fingerprinting.

Detection may also optionally be performed with a chip or other such device. The nucleic acid sample which includes the candidate region to be analyzed is preferably isolated, amplified and labeled with a reporter group. This reporter group can be a fluorescent group such as phycoerythrin. The labeled nucleic acid is then incubated with the probes immobilized on the chip using a fluidics station.

Once the reaction is completed, the chip is inserted into a scanner and patterns of hybridization are detected. The hybridization data is collected, as a signal emitted from the reporter groups already incorporated into the nucleic acid, which is now bound to the probes attached to the chip. Since the sequence and position of each probe immobilized on the chip is known, the identity of the nucleic acid hybridized to a given probe can be determined.

It will be appreciated that when utilized along with automated equipment, the above described detection methods can be used to screen multiple samples for a disease and/or pathological condition both rapidly and easily.

Immunoassays

Assays to monitor the expression of a marker or markers as defined in Table 12 may utilize any available means of monitoring for changes in the expression level of the disclosed polypeptides and/or proteins. These assays do not form part of the present invention. As used herein, an agent is said to modulate the expression of an amino acid if it is capable of up-or down-regulating expression of the amino acid in a cell.

The genes identified as being differentially expressed upon exposure to a known renal toxin (Tables 12-18, 26-27 and Table 21) may be used in a variety of amino acid detection assays to detect or quantify the level of a polypeptide and/or protein or multiple polypeptides and/or proteins in a given sample.

Any assay format to detect polypeptide and/or protein levels may be used. For example, immunoassay, such as ELISA, an RIA, a slot blot, immunohistochemical assay, FACS, a radio-imaging assay or a Western blot, or other receptor based assays for polypeptides or proteins.

"Immunoassay" is an assay that uses an antibody to specifically bind an antigen. The immunoassay is characterized by the use of specific binding properties of a particular antibody to isolate, target, and/or quantify the antigen.

The phrase "specifically (or selectively) binds" to an antibody or "specifically (or selectively) immunoreactive with," or "specifically interacts or binds" when referring to a protein or peptide (or other epitope), refers, in some embodiments, to a binding reaction that is determinative of the presence of the protein in a heterogeneous population of proteins and other biologics. Thus, under designated immunoassay conditions, the specified antibodies bind to a particular protein at least two times greater than the background (non-specific signal) and do not substantially bind in a significant amount to other proteins present in the sample. Specific binding to an antibody under such conditions may require an antibody that is selected for its specificity for a particular protein. For example, polyclonal antibodies raised to seminal basic protein from specific species such as rat, mouse, or human can be selected to obtain only those polyclonal antibodies that are specifically immunoreactive with seminal basic protein and not with other proteins, except for polymorphic variants and alleles of seminal basic protein. This selection may be achieved by subtracting out antibodies that cross-react with seminal basic protein molecules from other species. A variety of immunoassay formats may be used to select antibodies specifically immunoreactive with a particular protein. For example, solid-phase ELISA immunoassays are routinely used to select antibodies specifically immunoreactive with a protein (see, e.g., Harlow & Lane, Antibodies, A Laboratory Manual (1988), for a description of immunoassay formats and conditions that can be used to determine specific immunoreactivity). Typically a specific or selective reaction will be at least twice background signal or noise and more typically more than 10 to 100 times background.

Exemplary detectable labels, optionally and preferably for use with immunoassays, include but are not limited to magnetic beads, fluorescent dyes, radiolabels, enzymes (e.g., horse radish peroxide, alkaline phosphatase and others commonly used in an ELISA), and calorimetric labels such as colloidal gold or colored glass or plastic beads. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker are incubated simultaneously with the mixture.

Kits

Also disclosed are kits combining, in different combinations, high density oligonucleotide arrays, reagents for use with the arrays, protein encoded by the genes of Table 12 and homologous gene thereto, signal detection and array-processing instruments, gene expression databases and analysis and database management software described above.

The kits may be used, for example, to predict or model the toxic response of a test compound, to monitor the progression of renal disease states, to identify genes that show promise as new drug targets and to screen known and newly designed drugs as discussed above.

The kits may be used in the pharmaceutical industry, where the need for receiving toxicity and other indications relating to a drug as early as possible is strong due to the high costs associated with drug development, but where bioinformatics, in particular gene expression informatics, is still lacking. These kits will reduce the costs, time and risks associated with traditional new drug screening using cell cultures and laboratory animals. The results of large-scale drug screening of pre-grouped patient populations, pharmacogenomics testing, can also be applied to select drugs with greater efficacy and fewer side-effects. The kits may also be used by smaller biotechnology companies and research institutes who do not have the facilities for performing such large-scale testing themselves.

Without further description, it is believed that one of ordinary skill in the art can, using the preceding description and the following illustrative examples, make and utilize the compounds of the present invention and practice the claimed methods. The following working examples therefore, specifically point out the preferred embodiments of the present invention, and are not to be construed as limiting.

The general means and methods of the invention as described above are exemplified below. The following examples are offered as illustrations of specific embodiments and are not intended to limit the invention as defined in the claims.

EXAMPLES

*Example 1: Identification of Toxicity Markers*

*Experimental* ***Design***

The renal toxins Gentamycin (antibiotic), Cisplatin (chemotherapeutic agent), Tobramycin (antibiotic), Cadmium chloride (heavy metal in the following industries: plastic, electroplating, batteries, paints, smelter), Doxorubicin (chemotherapeutic antibiotic) and Valprioc acid (antiepileptic agent that was used as a negative control), were administered to male and female Sprague-Dawley rats at various time points and routes of administration as describes below. The Sprague-Dawley Crl:CD(SD) rats were selected for the current investigation, since this strain is frequently used in preclinical investigations including investigations of microarray expression profiling during preclinical drug development.

The test items (positive and negative controls) were administered to animals in accordance with the usual route of administration in humans (i.e. IP for gentamycin, cisplatin, tobramycin and cadmium; IV for doxorubicin; and oral for valproic acid). Control animals were treated intraperitoneal with saline.

Each substance was administered to the animals once or daily for 5 days or 28 days at the appropriate dose level. The test substances and dose levels were selected according to the predicted pathological effects of each compound: the selected dose of each compound was expected to cause no histological changes after single dose administration, but to induce significant kidney toxicity after 28 days of daily treatment.

Dose formulation:

Gentamycin: injection (80 mg/mL
Cisplatin: injection (10 mg/20mL
Tobramycin (Brulamycin): injection (40 mg/1mL and 80 mg/2mL)

Cadmium chloride: a stock solution was prepared according to the valid SOP of IDRI (VIG/010 "Preparation of injection").

Doxorubicin: injection (20 mg/10mL),
Valproic acid - negative control. A suspension in concentration of 100 mg/mL for oral use.

Randomization and group allocation: prior to treatment, the animals were assigned to the experimental groups based on their body weight. Mean body weight of each group at randomization was not deviate ± 20% of the mean population weight. Males and females were randomized separately. For the randomization, a computer program was used.

The original study groups and doses are shown in Table 1 below. Allocation to the dose group, 3 animals in each group, is shown in Table 2.

Table 1: Original study groups and doses

| Groups | Test items | Dose (mg/kg) | Dose Volume (ml/kg) | Conc. of stock (mg/ml) | Route of administration | No. of animals | |
|---|---|---|---|---|---|---|---|
| | | | | | | Males | Females |
| 1MF | Control/Saline | - | 5 | - | IP | 9 | 9 |
| 2MF | Gentamycin | 40 | 2 | 20 | IP | 9 | 9 |
| 3MF | Cisplatin | 0.5 | 2 | 0.25 | IP | 9 | 9 |
| 4MF | Tobramycin | 40 | 2 | 20 | IP | 9 | 9 |
| 5MF | Cadmium chloride | 2 | 2 | 1 | IP | 9 | 9 |
| 6MF | Doxorubicin | 4 | 2 | 2 | IV | 9 | 9 |
| 7MF | Valproic acid | 500 | 5 | 100 | PO | 9 | 9 |
| IV - intravenous, IP - intraperitoneal, PO - per oral | | | | | | | |

Table 2: Allocation to the dose group, 3 animals in each group

| **Groups** | **Duration of treatment (days)** |
|---|---|
| 1MF | 1, 5, 28 |
| 2MF | 1, 5, 28 |
| 3MF | 1, 5, 28 |
| 4MF | 1, 5, 28 |
| 5MF | 1, 5, 28 |
| 6MF | 1, 5, 28 |
| 7MF | 1, 5, 28 |

RESULTS

The animals were treated with 6 different test items each for 3 duration of treatment (1, 5 and 28 days). The actual study design is shown in Table 3.

Table 3: Study design

| **Groups** | **Test items** | **Dose (mg/kg)** | **Dose Volume (ml/kg)** | **Conc. of stock (mg/ml)** | **Route of adminis tration** | **No. of animals** | |
|---|---|---|---|---|---|---|---|
| | | | | | | Males | Females |
| 1MF | Control/Saline | - | 2 | - | IP | 9 | 9 |
| 2MF | Gentamycin | 40 | 2 | 20 | IP | 9 | 9 |
| 3MF | Cisplatin | 0.5 | 2 | 0.25 | IP | 9 | 9 |
| 4MF | Tobramycin | 40 | 2 | 20 | IP | 9 | 9 |

(continued)

| Groups | Test items | Dose (mg/kg) | Dose Volume (ml/kg) | Conc. of stock (mg/ml) | Route of adminis tration | No. of animals | |
|---|---|---|---|---|---|---|---|
| | | | | | | Males | Females |
| 5MF | Cadmium cl.* | 2<br>0.75* | 2 | 1<br>0.375 | IP | 9 | 9 |
| 6MF | Doxorubicin** | 4<br>1** | 2 | 2<br>0.5 | IV | 9 | 9 |
| 7MF | Valproic acid | 500 | 5 | 100 | PO | 9 | 9 |
| 8MF*** | - | - | - | - | - | 3 | 3 |
| IV - intravenous, IP - intraperitoneal, PO -oral (per os) | | | | | | | |

Modifications to the original study design:

*Animals treated with Cadmium chloride did not tolerate the treatment well, for that reason the dose was modified from 2 mg/kg to 0.75 mg/kg. Since males were in poor condition and excluded from the study, control (naive) animals were introduced to the 5M dose group and treated with the lower dose for 24 days.

**From day 7 onwards, a sudden decay was observed in the condition of males and females treated with 4 mg/kg Doxorubicin (6MF group). This decay affected the 28-days study group of 6MF. These animals died or became moribund; therefore, naive animals were introduced into this group. The dose of the Doxorubicin treated group was lowered .from 4 mg/kg to 1 mg/kg. However, males from Day 19-20 and females from Day 22 received 0.5 mg/kg dose of Doxorubicin, due to their decrease in body weight.

*** 8MF control groups were introduced in the study, including 3 males and 3 females. Animals from these groups did not receive any treatment; however samples (tissue, blood, urine) were taken from them.

During the treatment period the animals were observed daily for clinical symptoms, their body weight was measured weekly and the water consumption was measured daily.

The day before autopsy, urinalysis was performed and blood samples were taken for hematology and clinical chemistry examinations.

On the day of autopsy the animals were anaesthetized with isoflurane, blood was collected for PBMC isolation then subjected to autopsy. Tissues and organs (adrenals, brain, kidneys, liver, spleen and thymus) were weighed and subjected to gross pathological and histopathological examinations.

Sterile kidney and liver samples were taken from the animals, frozen in liquid nitrogen and stored at -80°C. PBMC (Peripheral Blood Mononuclear Cell) and urine samples were stored at 80°C as well. RNA was extracted from Kidney samples for toxicogenomic analysis for prediction of nephrotoxicity.

***Finding summary:***

1. Toxicants effects

**28-DAY**

**Gentamycin**

Signs of nephrotoxicity occurred in all animals of both sexes. During autopsy enlarged and discolored kidneys were observed in males. Kidney weight increased in both sexes. These changes were accompanied by clinical pathological findings: higher creatinine values in males and granular cylinders in the urine of females. The test item affected body weight gain of animals in both sexes. The nephrotoxic effect of the test item was confirmed by histopathology.

**Cisplatin**

The most severe nephrotoxic changes were found in Cisplatin treated groups of both sexes. Enlarged and discolored kidneys, increased kidneys and decreased thymus weight were found in both sexes. The nephrotoxic effect was detected during clinical pathology: Higher values of creatinine and urea nitrogen in both sexes & mild changes in the urine sediment. The test item affected body weight gain and water consumption in both sexes.

**Tobramycin**

Enlarged, discolored kidneys, with increased weights were observed in males. Clinical pathology revealed a nephrotoxic effect and higher values of creatinine and urea nitrogen in the blood as well as granular cylinders in the urine sediment of both sexes. Histopathological examinations confirmed nephrotoxic changes in both sexes.

**Cadmium chloride**

Mild clinical symptoms (hollow psoas, colic) were recorded in both sexes immediately after the administration. The test item affected body weight gain in both sexes. Slightly lower water consumption was recorded in males. Round bordered spleen and liver and thickening of peritoneum were recorded in both sexes, which correspond with higher liver and spleen weight. In addition increased weight of kidneys and decreased thymus were recorded in females. AST (Aspartate Aminotransferase) values were elevated in both sexes. A few transitional epithelial cells were observed in the urine of females. During histopathological examination significant nephrotoxicity was found only in a single female.

**Doxorubicin**

The test item caused severe clinical symptoms and lethality when administered in a 4 mg/kg dose. Two out of 3 males and 1 out of 3 females died. The lowered dose of 1 mg/kg of the test item was not lethal, but affected body weight gain in both sexes and water consumption in males. An increased weight of kidneys was found in males. The liver weight increased, while the weight of the thymus and spleen decreased significantly in both sexes. Elevated values of AST, ALT and Gamma GT were recorded in the male and decreased values of total protein, Albumin and Globulin were found in both sexes. Mild to moderate lymphoid depletion of the thymus was observed in both sexes.

**Valproic acid**

No histopathological signs of nephrotoxicity were found. The only toxic effect was a decreased total protein and Globulin value found in males.

**Control/Saline**

No histopathological signs of nephrotoxicity were found

**5-DAY**

No lethality was recorded in any groups of both sexes.

**Gentamycin**

No nephrotoxicity was observed, except elevated urea nitrogen levels found in females. Body weight gain was decreased in females. No renal damage was found at histopathological examination.

**Cisplatin**

Mild, focal tubular epithelial cell necrosis in the medulla of kidneys was noted in a single male. No remarkable observations were found at autopsy. Decreased body weight gain and water consumption were recorded in both sexes.

**Tobramycin**

No renal damage was found during histopathology. Elevated urea nitrogen levels were found in females. Cadmium chloride: No renal damage was found. The test item caused mild clinical symptoms (hollow psoas, colic) immediately after the administration. Body weight gain decreased in both sexes. Slightly increased Gamma GT values were recorded in females.

**Doxorubicin**

No renal damage was found. Decreased body weight gain and water consumption was recorded in both sexes. Minimal to mild thymus atrophy and minimal lymphoid depletion of the spleen were found in males which was in accordance with gross pathological findings. RBC, HBG, HCT, reticulocyte and WBC counts were lower in both sexes.

**Valproic acid**

No renal damage was found. Slightly lower total protein and Albumin values were found in males.

**Control/Saline**

No histopathological signs of nephrotoxicity were found

**SINGLE DAY**

No renal damage was found.

**Cisplatin**

Decreased body weight gain was recorded in both sexes. Slightly lower lymphocytes and increased neutrophils counts were recorded in males.

**Cadmium chloride**

Mild clinical symptoms (hollow psoas, colic) were observed in both sexes immediately after the administration. Decreased body weight gain was recorded in both sexes. Lower lymphocytes and increased neutrophils counts were found in males.

**Doxorubicin**

Decreased body weight gain was recorded in both sexes. Lower relative weights of spleen were recorded in females. Higher WBC and neutrophil counts, decreasing tendency of total protein, Albumin and Globulin were found in males.

**Control/Saline**

No histopathological signs of nephrotoxicity were found

<u>2. Histopathology</u>

At histopathological examination after **28-day treatment,** nephrotoxicity occurred in the following decreasing order: Cisplatin, Gentamycin, Tobramycin and Doxorubicin in males; Cisplatin, Cadmium chloride, Gentamycin, Doxorubicin and Tobramycin in females. Males were more sensitive to most of the substances.

Valproic acid proved to be non-nephrotoxic.

No renal damage was found after **5-day treatment** in all dosed groups of both sexes, compared to the controls, except a single male treated with Cisplatin, where mild, focal tubular epithelial cell necrosis in the medulla was found.

No test item related renal damage was found after **single treatment** in all dosed groups of both sexes, compared to the controls.

Table 4 presents the summarized findings recorded after the 28-day treatment period in the different dosed groups of both sexes.

Table 4: Findings after the 28-day treatment period

| Compound | Dose (mg/kg) | Findings | | | |
|---|---|---|---|---|---|
| | | Autopsy | Organ weight | Clinical pathology | Histopathology |
| Gentamycin | 40 | ♂♀: ↑↑, pale kidneys | ♂♀: ↑↑ kidneys | ♂: ↑CREA ♀: granular cylinders in urine | Kidneys ♂♀: lymphocytic inflammation, tubular basophilia in the cortex. ♂:tubular dilatation in the medulla |
| Cisplatin | 0.5 | ♂♀: ↑↑, pale kidneys | ♂♀: ↑↑ kidneys | ♂♀: ↑↑BUN, CREA ♂: trains. epith. cells in urine ♀ (1/3): oval lipid cell in urine | Kidneys ♂♀: lymphocytic inflammation, tubular basophilia in the cortex. Tubular dilatation, bizarre forms in the medulla. |
| Tobramycin | 40 | ♂♀: ↑↑, pale kidneys | ♂: ↑↑ kidneys | ♂♀: ↑↑BUN, CREA ♂♀: granular cylinders in urine ♂: trans. epith. cells in urine | Kidneys: ♂♀: lymphocytic inflammation, tubular basophilia in the cortex. ♂: tubular dilatation and basophilia in the medulla. |
| Cadmium chloride | 0.75 | ♂♀: ↑↑ liver, spleen, thickening of peritoneum | ♂♀: ↑↑ liver, spleen | ♂♀: ↑↑ AST ♀: trans. epith. cells in urine | Spleen: ♂: lymphoid depletion |
| Doxorubicin | 1 | ♂♀: ↑↑ thymus | ♂♀: ↑↑ thymus | ♂: ↑↑AST, ALT, γGT ♂♀: ↓↓TP, Alb., Glob. | Kidneys: ♂: tubular dilatation and basophilia in the cortex. Tubular dilatation in the medulla. |
| | | | | | Thymus: ♂♀: lymphoid depletion |

CREA - creatinine, BUN - Blood urea nitrogen, AST - aspartate aminotransferase, ALT - alanine aminotransferase, Alb - Albumin

Table 5 presents the summarized findings recorded after the 5-day treatment period in the different dosed groups.

Table 5: Findings after the 5-day treatment period

| Compound | Dose (mg/kg) | Findings | | | |
|---|---|---|---|---|---|
| | | Autopsy | Organ weight | Clinical pathology | Histopathology |
| Gentamycin | 40 | No effect | No effect | ♀:↑↑BUN | No renal damage |
| Cisplatin | 0.5 | No effect | No effect | ♂ (No.24): ↑↑PLT, ↓↓reticulocytes, ↑BUN and CREA | ♂ (No.24): tubular necrosis in the medulla |
| Tobramycin | 40 | No effect | No effect | ♀:↑↑BUN | No renal damage |
| Cadmium chloride | 2. | No effect | ♂♀: ↓thymus | ♀:↑ γGT | No renal damage |
| Doxorubicin | 4 | ♂♀: ↓↓thymus and spleen | ♂♀: ↓↓thymus and spleen | ♂♀: ↓↓RBC, HGB, HCT, reticulocytes, WBC | No renal damage. ♂: Thymus: atrophy Spleen: lymphoid depletion |

No test item related renal damage was found after single treatment in all dosed groups of both sexes, compared to the controls.

3. Lethality

**28-DAYS TREATMENT**

Death occurred only in Doxorubicin treated groups with the originally planned dose. No lethality was recorded after lowering the dose from 4 mg/kg to 1 mg/kg.

Table 6: Lethality in Doxorubicin treated groups

| | Died | Moribund |
|---|---|---|
| Males | 2/3 (Day 8) | 1/3 (Day 8) |
| Females | 1/3 (Day 9) | 2/3 (Day 9) |

**SINGLE AND 5-DAYS TREATMENT**

No lethality was recorded in any groups of both sexes.

4. Clinical Symptoms

**28-DAYS TREATMENT**

Clinical symptoms were observed only in Cadmium chloride groups and in the 4 mg/kg (originally planned dose) Doxorubicin treated groups of both sexes.

**SINGLE AND 5-DAY TREATMENT**

Clinical symptoms were observed only in Cadmium chloride groups of both sexes. No clinical symptoms were observed in any other groups of both sexes.

5. Body weight and body gain

**28-DAY TREATMENT**

Effect on body weight and body weight gain was seen in groups of both sexes treated with Doxorubicin, Cadmium chloride, Cisplatin and Gentamycin.

**5-DAYS TREATMENT**

Decreased body weight gain was recorded in males treated with Cisplatin, Cadmium chloride, Doxorubicin, Valproic acid and in all dosed groups of females.

6. Water Consumption

Compared with controls the following observations were made concerning water consumption:

**28-DAY TREATMENT**

Males

Sight decrease during the last week in groups dosed with Cisplatin, Cadmium chloride and Valproic acid; Significant decrease from Week 2 in the Doxorubicin treated group.

Females

Decreased during Week 4 in Cisplatin treated group.

**5-DAY TREATMENT**

Decrease in Cisplatin and Doxorubicin treated groups of both sexes.
Decrease in Tobramycin and Cadmium chloride treated females
Increase in Valproic acid treated females

6. Autopsy

**28-DAY TREATMENT**

At necropsy of animals treated with 4 mg/kg Doxorubicin who died (male No.: 52, 54 and female No.: 154) dehydration and anaemic organs were observed.

Scheduled autopsy

Alterations were found in the kidneys in both sexes treated with Cisplatin and in males treated with Gentamycin and Tobramycin. Pale and enlarged kidneys were found in a single female (No.145) treated with Cadmium chloride. Smaller thymuses were found in Doxorubicin treated groups of both sexes.Round bordered liver, enlarged spleen and thickening of the peritoneum were found in both sexes treated with Cadmium chloride.

**5-DAY TREATMENT**

No alterations were found in the kidneys in the dosed groups of both sexes. No remarkable observations were found at autopsy in females. Smaller thymus and spleen were recorded in the Doxorubicin treated group of males.

**SINGLE TREATMENT**

No alterations were found in the kidneys in the dosed groups of both sexes. No remarkable observations were found in females. Hemorrhagic thymus were found with different incidence in the dosed groups in males, including controls.

7. Organ Weights

**28-DAY TREATMENT**

Compared with controls the following observations were made concerning the relative organ weights, taking into consideration the small number of groups (3/sex/group):

> Increased liver weights in groups of both sexes treated with Cadmium chloride and Doxorubicin; Increased weights of kidneys in males treated with Gentamycin, Cisplatin, Tobramycin and Doxorubicin; Higher weights of kidneys in females treated with Gentamycin, Cisplatin and Cadmium chloride; Weights of spleen were higher in Gentamycin treated males and Cadmium chloride treated groups of both sexes. Lower weights were recorded in Doxorubicin treated groups of both sexes; Decreased thymus weights were found in Gentamycin and Tobramycin treated males, in Cisplatin and Doxorubicin treated groups of both sexes and in females treated with Cadmium chloride.

**5-DAY TREATMENT**

No effect was observed in kidneys of the dosed groups of both sexes.
Compared with controls the following observations were made concerning the relative organ weights, taking into consideration the small number of groups (3/sex/group): Lower weights of spleen in Doxorubicin treated groups of both sexes; Lower thymus weights in Doxorubicin, Cadmium chloride and Valproic acid treated groups of both sexes.

**SINGLE TREATMENT**

No effect was observed in kidneys of the dosed groups of both sexes.No significant alterations were recorded in males.
Compared with controls the following tendencies were observed in females concerning the relative organ weights, taking into consideration the small number of groups (3/sex/group): Lower weights of liver in Cisplatin treated group; Lower weights of thymus in Gentamycin, Cisplatin and Valproic acid treated groups; Lower weights of spleen in Cisplatin and Doxorubicin treated groups.

***Expression analysis***

RNA isolation from kidney tissues

RNA was isolated from the kidney tissues using the following procedure:

Step 1: Homogenization of Tissue: Add 200 mg of tissue sample to 7ml of cold TRI Reagent (MRC) on ice. Homogenize tissue using several short pulses (5 sec.) of the homogenizer.

Step 2: Phase separation: Add 0.2ml of BCP (1-BROMO-3-CHLORO-PROPANE, SIGMA, Cat. No. B-9673) per 1 ml TRI Reagent. Cover the sample tightly and mix vigorously for 15 sec. Incubate the sample at RT 2-3min. Spin the sample at 12000g for 20min at 4°C.

Step 3: RNA precipitation: Following centrifugation, the mixture separates into a lower red, phenol-chloroform phase, an interphase, and a colorless upper aqueous phase. Remove the top aqueous layer to a fresh tube. Add 0.5ml Isopropanol per 1ml TRI Reagent used in the original homogenization step. Mix the tube by vortex and incubate the sample at RT for 7-8min. Spin the sample at 12000g for 15min at 4°C.

Step 4: RNA wash: Remove the sup and add a volume of 80% ethanol (made with DEPC-treated water, stored at -20°C) equal to the original volume of TRI Reagent to rinse the pellet. Centrifuge the sample at 12000g for 5min at 4°C. Remove the ethanol and resuspend the RNA pellet in 400ul DEPC water.

Step 5: RNA purification using phenol-chlorophorm extraction and MaXtract low Density tubes: Equilibrate the phenol:chlorophorm:isoamyl alcohol mix (25:24:1) (Ambion, Cat. No. 9732) to room temperature. Pellet the MaXtract tubes (QIAGEN, Cat. No. 129016) prior to use by centrifugation 1 minute at maximum speed. To the RNA dissolved add an equal volume (400ul) of phenol:chlorophorm:isoamyl (25:24:1) alcohol mix and vortex. Transfer the entire Total RNA-phenol/chlorophorm mix to MaXtract tube. Centrifuge at 13000rpm for 2 min. Transfer the aqueous upper phase to a fresh non-stick 1.5 ml tube (Ambion, Cat. No. AM12450). Pay attention not to touch the gel with the pipet tip as it reduces the grade of RNA purity. Repeat steps 5.3-5.5.

Step 6: RNA precipitation II: Precipitate the RNA by adding 0.1 vol 3M sodium acetate (RNAse free, Sigma Cat. No. S-7899) and 0.8 volume isopropanol (RNA vol.+sodium acetate vol.) For 400 $\mu$l RNA: 40 $\mu$l Sodium acetate and 352 $\mu$l isopropanol. Vortex 5 sec. Store at -20°C over night. Fast cool the centrifuge. Spin the samples at full speed (14000 RPM) for 20 min at 4°C pellet would be at the bottom.

Step 7: RNA wash II and resuspending: Pipette off the supernatant. Rinse twice the pellet with 0.5 ml 80% ethanol (made with DEPC-treated water , stored at -20 °C), centrifuge for 5 min at 9500 RPM, 4 °C. Pipette out the ethanol, then short spin the samples in Eppendorf centrifuge at full speed for 10 sec., and pipette out the remainder of ethanol. Air dry the pellet. Simultaneously, preheat the DEPC treated water to 55-70°C. Check the RNA for dryness. When dry, it is almost completely transparent. Add appropriate volume of DEPC treated water to the pellet (20-150 $\mu$l) depending on the size of the pellet - do not mix! Heat the samples 10 min. at 55-70 °C. Mix well by pipetation.

Final RNA product was quantified by spectrophotometric quantification. The quality of the RNA was evaluated by measuring the 260/280 and 260/230 ratios and confirmed by agarose gel. RNA was deemed of a suitable quality for microarray analysis if the 260/280 ratio was between 1.8 and 2.1, 260/230 ratio was higher than 1.5 and the gel showed no visible degradation products lower than the 18S ribosomal band.

Microarray Hybridization

RNA samples extracted from the 1-Day and 5-Days treated animals and were sent to hybridization on Affymetrix array GeneChip® Rat Genome 230 2.0. The hybridization was performed according to the Affymetrix' following protocol:

Step 1: Target Preparation: Using protocols in Affymetrix' manual Section 2, double-stranded cDNA is synthesized from total RNA (or purified poly-A messenger RNA) isolated from tissue or cells. An in vitro transcription (IVT) reaction is then done to produce biotin-labeled cRNA from the cDNA. The cRNA is fragmented before hybridization.

Step 2: Target Hybridization: A hybridization cocktail is prepared, including the fragmented target, probe array controls, BSA, and herring sperm DNA. It is then hybridized to the probe array during a 16-hour incubation. The hybridization process is describes in Affymetrix' manuals.

Step 3: Fluidics Station Setup: Specific experimental information is defined using Affymetrix® Microarray Suite or GeneChip Operating Software (GCOS) on a PC-compatible workstation. The probe array type, sample description, and comments are entered and saved with a unique experiment name. The fluidics station is then prepared for use by priming with the appropriate buffers.

Step 4: Probe Array Washing and Staining: Immediately following hybridization, the probe array undergoes an automated washing and staining protocol on the fluidics station.

Step 5: Probe Array Scan: Once the probe array has been hybridized, washed, and stained, it is scanned. Each workstation running Affymetrix Microarray Suite or GCOS can control one scanner. The software defines the probe cells and computes intensity for each cell. Each complete probe array image is stored in a separate data file identified by the experiment name and is saved with a data image file (.dat) extension.

Step 6: Data Analysis: The .dat image is analyzed for probe intensities; results are reported in tabular and graphical formats.

The expression levels were extracted using Affymetrix' MAS5 algorithm. Further multiplicative normalization on the data - setting the 95th percentile of the expression vector of each sample to an arbitrary constant value (1200) - was then performed.

Classifier on Microarray Data

Finding signatures using machine learning algorithms requires two steps - selecting a limited set of features to be used for the signature, and building a classifier using these features. The feature selection stage is especially significant here for several reasons. Large number of features means a low signal to noise ratio, tampering the classifier performance, large number of features, compared to the relatively small number of samples, means that spurious features might exists. These are features that give good classification on the experiment data *by chance*, causing the classifier to be over-fitted to the learning set and have low prediction ability, and finally - a small number of features is important for practical application of the signature.

The initial features selection process is done by performing Mann-Whitney (Wilcoxon) rank-sum test on the data, considering only probesets whose overall normalized expression mean is over 25, to avoid working at noise-level.

Further features selection is done iteratively by building a Random Forest classifier, using the initial list of features, and estimating the importance of features as given by the algorithm's "Out of the Bag" approach. Less important features (those that have low impact on the performance of the classifier) are removed from the features list and the process is repeated.

The performance of the classifier built as just described, is estimated by calculating the LOR and Accuracy after performing a cross validation process - some of the samples are removed from the data. The whole process of features-selection and classifier building is performed on the data, and the prediction is tested on the left-out samples.

The following example illustrates the results of the process - working on the Day-5 samples of animals treated with toxic compounds, not including animals treated with Cadmium-Chloride (see pathological data, above) ("Toxic"), as compared to all controls samples (Saline and Valproic-acid treated animals at Day-1 and Day-5, as well as naive rats) ("Control"). A list of 20 features (probesets) was initially selected, and then iteratively reduced to 6 features. The random forest classifier was used with the iterative list of features, as well as the gender of the rat used as an extra two-value feature. The random forest classifier was used with 2500 decision trees in each run. The cross validation was performed by leaving-out randomly selected samples - 3 control samples and 3 toxic samples. This was repeated 350 times. The bound for deciding whether a sample is predicted as toxic or control, was chosen as to maximize the Accuracy. The "Confusion matrix" for this optimal bound is given in Table 7.

Table 7: "Confusion matrix"

| | Control | *Toxic* |
|---|---|---|
| Predicted as Control | 965 | *85* |
| *Predicted as Toxic* | *151* | *899* |

The Accuracy of the signatures as evaluated by this process is 89%, and the LOR is 4.2

Selecting Genes for Validation

Following the analysis performed on the Day-5 data, 20 top-scoring probesets were selected for further validation using qRT-PCR. The probesets were mapped to the trasnscriptome produced by the LEADS clustering and assembly system (described in Sorek, R., Ast, G. & Graur, D. Alu-containing exons are alternatively spliced. Genome Res 12, 1060-7 (2002); US patent No: 6,625,545; and U.S. Patent Application No. 10/426,002, published as US20040101876 on May 27 2004. Briefly, the software cleans the expressed sequences from repeats, vectors and immunoglobulins. It then aligns the expressed sequences to the genome taking alternatively splicing into account and clusters overlapping expressed sequences into "clusters" that represent genes or partial genes) and the relevant contig and gene (whenever existing) were identified. As evident in table 8 bellow, the mapping of the probeset 1375422_at was not unique so we selected all relevant contigs.

Novel variants for the relevant genes were also identified. This was done based on the available rat ESTs and mRNAs and, when a human and mouse homologues was identified - the mouse ESTs and mRNAs and human transcripts were added the relevant rat ESTs and mRNAs to construct a combined informative contigs.

Table 8: Probe sets selected for qRT-PCR validation, with their LEADS gene name and internal candidate name

| Probe set | Internal Name | Gene Name |
|---|---|---|
| 1375422_at | BE097535_DB71_T0 | BE097535 |
| 1390507_at | AI045075_DB71_T0 | ISG20 |
| 1367764_at | H31883_DB71_T0 | CCNG1 |
| 1369268_at | RATLRFI_DB71_T0 | ATF3 |
| 1380692_at | BE104394_DB71_T0 | BE104394 |
| 1388674_at | RNU24174_DB71_T0 | CDKN1A |
| 1371785_at | AA686189_DB71_T0 | TNFRSF12A |
| 1393728_at | AA964541_DB71_T0 | AA964541 |
| 1369814_at | RNU90447_DB71_T0 | CCL20 |
| 1397637_at | AI502869_DB71_T0 | AI502869 |
| 1388560_at | H33998_DB71_T0 | WDR77 |
| 1392102_at | AI454051_DB71_T0 | AI454051 |
| 1370153_at | BI293562_DB71_T0 | GDF15 |
| 1388140_at | RATRAB13X_DB71_T0 | RAB13 |
| 1390833_at | AW919147_DB71_T0 | AW919147 |
| Probe set | Internal Name | Gene Name |
| 1384934_at | BQ200887_DB71_T0 | BQ200887 |
| 1375895_at | AI549068_DB71_T0 | AI549068 |
| 1388642_at | H31799_DB71_T0 | EI24 |
| 1383162_at | AA799594_DB71_T0 | AA799594 |
| 1384070_at | H31045_DB71_T0 | H31045 |

RT Preparation and Real-Time qRT-PCR Analysis

Before RT preparation the RNA was treated with DNA-free (Ambion, cat. No. AM1906) DNase treatment and removal - according to manufacturer protocol. DNase procedure was repeated until no DNA is detected in a PCR reaction using the RNA samples.

RT preparation - Purified RNA (4μg) is mixed with 600ng Random Hexamer primers (Invitrogen, Cat. No.

48190-011) and 500 μM dNTP (Takara, Cat. No. 4030) in a total volume of 62.4 μl. The mixture is then incubated for 5 min at 65 °C and then quickly chilled on ice. Thereafter, 20 μl of 5X SuperscriptII first strand buffer (Invitrogen, Cat. No. Y00146), 9.6μl 0.1M DTT (Invitrogen, Cat. No. Y00147) and 160 units RNasin (Promega, Cat. No. N251A) are added, and the mixture is incubated for 10 min at 25 °C, followed by further incubation at 42°C for 2 min. Then, 4 μl (800units) of SuperscriptII (Invitrogen, Cat. No. 18064-022) is added and the reaction (final volume of 100μl) is incubated for 50 min at 42°C and then inactivated at 70°C for 15min. The resulting cDNA is diluted 1:20 in TE buffer (10 mM Tris pH=8, 1 mM EDTA pH=8).

cDNA (5μl), prepared as described above, is used as a template in Real-Time PCR reactions using the SYBR Green I assay (PE Applied Biosystem) with specific primers in 100nM concentration if not indicated otherwise and UNG Enzyme (Eurogentech or equal) Amplification is effected as follows: 50°C for 2 min, 95°C for 10 min, and then 40 cycles of 95°C for 15sec, followed by 60°C for 1 min (if not indicated otherwise). Amplification step is followed by dissociation step. Detection is performed by using the PE Applied Biosystem SDS 7000. The cycle in which the reactions achieved a threshold level (Ct) of fluorescence is registered and is used to calculate the relative transcript quantity in the RT reactions. Non-detected samples are assigned Ct value of 41 and are calculated accordingly. The relative quantity is calculated using the equation Q=efficiency^-Ct. The efficiency of the PCR reaction is calculated from a standard curve, created by using serial dilutions of several reverse transcription (RT) reactions. To minimize inherent differences in the RT reaction, the resulting relative quantities are normalized to normalization factor calculated as follows:

The expression of four housekeeping (HSKP) genes from different pathways, ACTB (Entrez Gene ID: 81822), ACTG2 (Entrez Gene ID: 25365), HPRT (Entrez Gene ID: 24465) and YWHAZ (Entrez Gene ID: 25578), is found by SYBR green detection. The relative quantity (Q) of each housekeeping gene in each sample is calculated as described above. In order to calculate the "relative Q relative to MED" each Q for each HSKP gene is divided by the median quantity of this gene in all panel samples. To finally achieve the normalization factor of each sample the geometric mean of all four "relative Q relative to MED" was calculated. The normalization factor was then used for further calculations.

The sequences of the amplicons derived from the housekeeping genes measured in all the examples were as follows:

ACTB (Entrez Gene ID: 81822),

Forward Primer (SEQ ID NO: 237): GGGAAATCGTGCGTGACATT

Reverse Primer: (SEQ ID NO: 238): GCGGCAGTGGCCATCTC

Amplicon (SEQ ID NO: 239):

GGGAAATCGTGCGTGACATTAAAGAGAAGCTGTGCTATGTTGCCCTAGACTTCGAGC
AAGAGATGGCCACTGCCGC

ACTG2 (Entrez Gene ID: 25365),

Forward Primer (SEQ ID NO: 240): TACCCTATTGAGCACGGCAT

Reverse Primer (SEQ ID NO: 241): CGCAGCTCGTTGTAGAAGGT

Amplicon (SEQ ID NO: 242):

TACCCCATTGAACACGGCATCATCACGAACTGGGATGACATGGAGAAGATCTGGCAC
CACTCCTTCTACAACGAGCTGCG

HPRT (Entrez Gene ID: 24465)

Forward Primer (SEQ ID NO: 243): GCGAAAGTGGAAAAGCCAAGT

Reverse Primer (SEQ ID NO: 244): GCCACATCAACAGGACTCTTGTAG

Amplicon (SEQ ID NO: 245):

GCGAAAGTGGAAAAGCCAAGTACAAAGCCTAAAAGACAGCGGCAAGTTGAATCTAC AAGAGTCCTGTTGATGTGGC

YWHAZ (Entrez Gene ID: 25578)

Forward Primer (SEQ ID NO: 246): CAAGCATACCAAGAAGCATTTGA

Reverse Primer (SEQ ID NO: 247): GGGCCAGACCCAGTCTGA

Amplicon (SEQ ID NO: 248):

CAAGCATACCAAGAAGCATTTGAAATCAGCAAAAAGGAGATGCAGCCGACACACCCC ATCAGACTGGGTCTGGCCC

The markers of the present invention were tested with regard to their expression in a panel of kidney tissues samples from treated and control rats. Unless otherwise noted, all experimental data relates to the novel polynucleotides and proteins of the present invention, named according to the segment being tested (as expression was tested through RT-PCR as described). A description of the tissue samples used in the kidney testing panel is provided in Table 9 below. Tests were then performed as described in the "Materials and Experimental Procedures" section above.

The name comprises of the rat's group, as was described in table 2, the rats ID number, day of treatment and the nephrotoxicant it was exposed to:

Gent - Gentamycin, Cis - Cisplatin, Tob - Tobramycin, CadCl - Cadmium chloride, Dox - Doxorubicin, ValpA - Valproic Acid.

Table 9: Definitions of the tissue samples included in the panel based on their origin and treatment

| # | Toxin used | Day | | Name |
|---|---|---|---|---|
| 1 | Gentamycin | 1 | day 1 toxicants M | **1_2M10_day1_Gent** |
| 2 | Gentamycin | 1 | | **2_2M11_day1_Gent** |
| 3 | Gentamycin | 1 | | **3_2M12_day1_Gent** |
| 4 | Cisplatin | 1 | | **4_3M19_day1_Cis** |
| 5 | Cisplatin | 1 | | **5_3M20_day1_Cis** |
| 6 | Cisplatin | 1 | | **6_3M21_day1_Cis** |
| 7 | Tobramycin | 1 | | **7_4M28_day1_Tob** |
| 8 | Tobramycin | 1 | | **8_4M29_day1_Tob** |
| 9 | Tobramycin | 1 | | **9_4M30_day1_Tob** |
| 10 | Cadmium chloride | 1 | | **10_5M37_day1_CadCl** |
| 11 | Cadmium chloride | 1 | | **11_5M38_day1_CadCl** |
| 12 | Cadmium chloride | 1 | | **12_5M39_day1_CadCl** |
| 13 | Doxorubicin | 1 | | **13_6M46_day1_Dox** |
| 14 | Doxorubicin | 1 | | **14_6M47_day1_Dox** |
| 15 | Doxorubicin | 1 | | **15_6M48_day1_Dox** |
| 16 | Gentamycin | 1 | day 1 toxicants F | **16_2F110_day1_Gent** |
| 17 | Gentamycin | 1 | | **17_2F111_day1_Gent** |
| 18 | Gentamycin | 1 | | **18_2F112_day1_Gent** |
| 19 | Cisplatin | 1 | | **19_3F119_day1_Cis** |
| 20 | Cisplatin | 1 | | **20_3F120_day1_Cis** |
| 21 | Cisplatin | 1 | | **21_3F121_day1_Cis** |
| 22 | Tobramycin | 1 | | **22_4F128_day1_Tob** |
| 23 | Tobramycin | 1 | | **23_4F129_day1_Tob** |
| 24 | Tobramycin | 1 | | **24_4F130_day1_Tob** |
| 25 | Cadmium chloride | 1 | | **25_5F137_day1_CadCl** |
| 26 | Cadmium chloride | 1 | | **26_5F138_day1_CadCl** |

| | | | | |
|---|---|---|---|---|
| **27** | Cadmium chloride | 1 | | **27_5F139_day1_CadCl** |
| **28** | Doxorubicin | 1 | | **28_6F146_day1_Dox** |
| # | Toxin used | Day | | **Name** |
| **29** | Doxorubicin | 1 | | **29_6F147_day1_Dox** |
| **30** | Doxorubicin | 1 | | **30_6F148_day1_Dox** |
| **31** | Gentamycin | 5 | day 5 toxicants M | **31_2M13_day5_Gent** |
| **32** | Gentamycin | 5 | | **32_2M14_day5_Gent** |
| **33** | Gentamycin | 5 | | **33_2M15_day5_Gent** |
| **34** | Cisplatin | 5 | | **34_3M22_day5_Cis** |
| **35** | Cisplatin | 5 | | **35_3M23_day5_Cis** |
| **36** | Cisplatin | 5 | | **36_3M24_day5_Cis** |
| **37** | Tobramycin | 5 | | **37_4M31_day5_Tob** |
| **38** | Tobramycin | 5 | | **38_4M32_day5_Tob** |
| **39** | Tobramycin | 5 | | **39_4M33_day5_Tob** |
| **40** | Cadmium chloride | 5 | | **40_5M40_day5_CadCl** |
| **41** | Cadmium chloride | 5 | | **41_5M41_day5_CadCl** |
| **42** | Cadmium chloride | 5 | | **42_5M42_day5_CadCl** |
| **43** | Doxorubicin | 5 | | **43_6M49_day5_Dox** |
| **44** | Doxorubicin | 5 | | **44_6M50_day5_Dox** |
| **45** | Gentamycin | 5 | day 5 toxicants F | **45_2F113_day5_Gent** |
| **46** | Gentamycin | 5 | | **46_2F114_day5_Gent** |
| **47** | Gentamycin | 5 | | **47_2F115_day5_Gent** |
| **48** | Cisplatin | 5 | | **48_3F122_day5_Cis** |
| **49** | Cisplatin | 5 | | **49_3F123_day5_Cis** |
| **50** | Cisplatin | 5 | | **50_3F124_day5_Cis** |
| **51** | Tobramycin | 5 | | **51_4F131_day5_Tob** |
| **52** | Tobramycin | 5 | | **52_4F132_day5_Tob** |
| **53** | Tobramycin | 5 | | **53_4F133_day5_Tob** |
| **54** | Cadmium chloride | 5 | | **54_5F141_day5_CadCl** |
| **55** | Cadmium chloride | 5 | | **55_5F142_day5_CadCl** |
| **56** | Doxorubicin | 5 | | **56_6F149_day5_Dox** |
| **57** | Doxorubicin | 5 | | **57_6F150_day5_Dox** |
| **58** | Doxorubicin | 5 | | **58_6F151_day5_Dox** |
| **59** | Naïve | 28 | Controls All days | **59_8M81_day28_Naïve** |
| **60** | Naïve | 28 | | **60_8M82_day28_Naïve** |
| **61** | Naïve | 28 | | **61_8M83_day28_Naïve** |
| **62** | Naïve | 28 | | **62_8F181_day28_Naïve** |
| **63** | Naïve | 28 | | **63_8F182_day28_Naïve** |

| | | | | |
|---|---|---|---|---|
| **64** | Naïve | 28 | | **64_8F183_day28_Naïve** |

| # | Toxin used | Day | | **Name** |
|---|---|---|---|---|
| **65** | Control/Saline | 1 | | **65_1M1_day1_Saline** |
| **66** | Control/Saline | 1 | | **66_1M2_day1_Saline** |
| **67** | Control/Saline | 1 | | **67_1M3_day1_Saline** |
| **68** | Control/Saline | 1 | | **68_1F101_day1_Saline** |
| **69** | Control/Saline | 1 | | **69_1F102_day1_Saline** |
| **70** | Control/Saline | 1 | | **70_1F103_day1_Saline** |
| **71** | Control/Saline | 5 | | **71_1M4_day5_Saline** |
| **72** | Control/Saline | 5 | | **72_1M5_day5_Saline** |
| **73** | Control/Saline | 5 | | **73_1M6_day5_Saline** |
| **74** | Control/Saline | 5 | | **74_1F104_day5_Saline** |
| **75** | Control/Saline | 5 | | **75_1F105_day5_Saline** |
| **76** | Gentamycin | 28 | day 28 toxicants M | **76_2M16_day28_Gent** |
| **77** | Gentamycin | 28 | | **77_2M17_day28_Gent** |
| **78** | Gentamycin | 28 | | **78_2M18_day28_Gent** |
| **79** | Cisplatin | 28 | | **79_3M25_day28_Cis** |
| **80** | Cisplatin | 28 | | **80_3M26_day28_Cis** |
| **81** | Cisplatin | 28 | | **81_3M27_day28_Cis** |
| **82** | Tobramycin | 28 | | **82_4M34_day28_Tob** |
| **83** | Tobramycin | 28 | | **83_4M35_day28_Tob** |
| **84** | Tobramycin | 28 | | **84_4M36_day28_Tob** |
| **85** | Cadmium chloride | 28 | | **85_5M43_day28_CadCl** |
| **86** | Cadmium chloride | 28 | | **86_5M44_day28_CadCl** |
| **87** | Cadmium chloride | 28 | | **87_5M45_day28_CadCl** |
| **88** | Doxorubicin | 28 | | **88_6M71_day28_Dox** |
| **89** | Doxorubicin | 28 | | **89_6M72_day28_Dox** |
| **90** | Doxorubicin | 28 | | **90_6M73_day28_Dox** |
| **91** | Gentamycin | 28 | day 28 toxicants F | **91_2F116_day28_Gent** |
| **92** | Gentamycin | 28 | | **92_2F117_day28_Gent** |
| **93** | Gentamycin | 28 | | **93_2F118_day28_Gent** |
| **94** | Cisplatin | 28 | | **94_3F125_day28_Cis** |
| **95** | Cisplatin | 28 | | **95_3F126_day28_Cis** |
| **96** | Cisplatin | 28 | | **96_3F127_day28_Cis** |
| **97** | Tobramycin | 28 | | **97_4F134_day28_Tob** |
| **98** | Tobramycin | 28 | | **98_4F135_day28_Tob** |
| **99** | Tobramycin | 28 | | **99_4F136_day28_Tob** |

| | | | | |
|---|---|---|---|---|
| **100** | Cadmium chloride | 28 | | **100_5F143_day28_CadCl** |
| **101** | Cadmium chloride | 28 | | **101_5F144_day28_CadCl** |

| # | Toxin used | Day | | **Name** |
|---|---|---|---|---|
| **102** | Cadmium chloride | 28 | day 28 toxicants F | **102_5F145_day28_CadCl** |
| **103** | Doxorubicin | 28 | | **103_6F171_day28_Dox** |
| **104** | Doxorubicin | 28 | | **104_6F172_day28_Dox** |
| **105** | Doxorubicin | 28 | | **105_6F173_day28_Dox** |
| **106** | Control/Saline | 28 | Day 28 control | **106_1M7_day28_Saline** |
| **107** | Control/Saline | 28 | | **107_1M8_day28_Saline** |
| **108** | Control/Saline | 28 | | **108_1M9_day28_Saline** |
| **109** | Control/Saline | 28 | | **109_1F107_day28_Saline** |
| **110** | Control/Saline | 28 | | **110_1F108_day28_Saline** |
| **111** | Control/Saline | 28 | | **111_1F109_day28_Saline** |
| **112** | Valproic acid | 1 | Valproic acid - all days | **112_7M55_day1_ValpA** |
| **113** | Valproic acid | 1 | | **113_7M56_day1_ValpA** |
| **114** | Valproic acid | 1 | | **114_7M57_day1_ValpA** |
| **115** | Valproic acid | 1 | | **115_7F155_day1_ValpA** |
| **116** | Valproic acid | 1 | | **116_7F156_day1_ValpA** |
| **117** | Valproic acid | 1 | | **117_7F157_day1_ValpA** |
| **118** | Valproic acid | 5 | | **118_7M58_day5_ValpA** |
| **119** | Valproic acid | 5 | | **119_7M59_day5_ValpA** |
| **120** | Valproic acid | 5 | | **120_7M60_day5_ValpA** |
| **121** | Valproic acid | 5 | | **121_7F158_day5_ValpA** |
| **122** | Valproic acid | 5 | | **122_7F159_day5_ValpA** |
| **123** | Valproic acid | 5 | | **123_7F160_day5_ValpA** |
| **124** | Valproic acid | 28 | | **124_7M61_day28_ValpA** |
| **125** | Valproic acid | 28 | | **125_7M62_day28_ValpA** |
| **126** | Valproic acid | 28 | | **126_7M63_day28_ValpA** |
| **127** | Valproic acid | 28 | | **127_7F161_day28_ValpA** |
| **128** | Valproic acid | 28 | | **128_7F162_day28_ValpA** |
| **129** | Valproic acid | 28 | | **129_7F163_day28_ValpA** |

Classifier on qRT-PCR Data

The following tables summarize the microarray and qRT-PCR data for the markers disclosed herein. The mean normalized expression level as measured by the microarray, as well as the mean normalized qRT-PCR expression levels, and the relevant Mann-Whitney scores p-values are given for each candidate.

Table 10: Mean expression levels and Mann-Whitney rank sum score p-values for microarray data of Day-1, Day-5 and control samples

| Candidate | | Microarray Data | | | | |
|---|---|---|---|---|---|---|
| Probeset | Gene name | Normal mean | Tox Day-1 mean | Tox Day-5 mean | Day-1 p-value | Day-5 p-value |
| 1371785_at | TNFRSF12A | 212 | 312 | 392 | 0.002 | 3.00E-05 |
| 1383162_at | AA799594 | 4055 | 3521 | 3385 | 0.002 | 6.00E-03 |
| 1393728_at | AA964541 | 35 | 46 | 56 | 0.01 | 6.00E-05 |
| 1390507_at | ISG20 | 32 | 47 | 48 | 0.0006 | 6.00E-06 |
| 1392102_at | AI454051 | 85 | 87 | 98 | 0.95 | 6.00E-04 |
| 1397637_at | AI502869 | 65 | 59 | 56 | 0.03 | 3.00E-04 |
| 1390833_at | AW919147 | 32 | 28 | 25 | 0.04 | 2.00E-04 |
| 1370153_at | GDF15 | 38 | 48 | 73 | 0.01 | 1.00E-04 |
| 1384070_at | H31045 | 88 | 99 | 108 | 0.02 | 3.50E-05 |
| 1388642_at | EI24 | 867 | 860 | 917 | 0.38 | 8.00E-04 |
| 1367764_at | CCNG1 | 757 | 864 | 1229 | 0.03 | 4.50E-05 |
| 1388560_at | WDR77 | 532 | 529 | 572 | 0.82 | 4.00E-04 |
| 1369268_at | ATF3 | 30 | 69 | 86 | 0.007 | 4.00E-06 |
| 1388140_at | RAB13 | 171 | 180 | 193 | 0.004 | 8.00E-04 |
| 1388674_at | CDKN1A | 97 | 123 | 192 | 0.003 | 5.00E-06 |

Table 11: Mean expression levels and Mann-Whitney rank sum score p-values for qRT-PCR data of Day-1, Day-5 and control samples

| Candidate | | qRT-PCR Data | | | | |
|---|---|---|---|---|---|---|
| Internal name | Gene name | Normal mean | Tox Day-1 mean | Tox Day-5 mean | Day-1 p-value | Day5 p-value |
| W41270_DB81_seg11 | TNFRSF12A | 1.08 | 1.42 | 2.27 | 4.00E-04 | 7.50E-10 |
| AA686189_DB71_seg6 | TNFRSF12A | 0.99 | 1.27 | 1.44 | 8.00E-04 | 7.50E-06 |
| AA799594_DB71_seg0 | AA799594 | 1.66 | 1.45 | 1.48 | 8.00E-03 | 0.01 |
| AA964541_DB71_seg0 | AA964541 | 0.87 | 1.24 | 1.43 | 2.00E-06 | 3.00E-08 |
| W64472_DB81_seg2 | ISG20 | 1.06 | 1.14 | 2.32 | 0.35 | 2.00E-10 |
| AI045075_DB71_seg6 | ISG20 | 1.14 | 1.58 | 1.46 | 2.50E-04 | 6.50-04 |
| AI454051_DB71_seg0 | AI454051 | 1.53 | 1.46 | 1.48 | 0.4 | 0.7 |
| AI502869_DB71_seg0 | AI502869 | 1.62 | 1.34 | 1.21 | 7.00E-03 | 2.50E-05 |
| AW919147_DB71_seg0 | AW919147 | 1.60 | 1.52 | 1.43 | 0.4 | 0.01 |
| BI293562_DB71_seg2 | GDF15 | 0.97 | 1.11 | 1.64 | 0.03 | 7.50E-07 |
| H31045_DB71_seg5 | H31045 | 1.51 | 0.99 | 1.51 | 0.015 | 0.5 |
| W83813_DB81_seg27 | EI24 | 1.30 | 1.42 | 2.18 | 0.55 | 1.50E-08 |
| H31799_DB71_seg23 | EI24 | 1.48 | 1.56 | 1.43 | 0.25 | 0.01 |
| MUSCYCG1R_DB81_seg15-17 | CCNG1 | 1.20 | 1.17 | 2.18 | 0.25 | 1.00E-07 |

(continued)

| Candidate | | qRT-PCR Data | | | | |
|---|---|---|---|---|---|---|
| Internal name | Gene name | Normal mean | Tox Day-1 mean | Tox Day-5 mean | Day-1 p-value | Day5 p-value |
| MUSCYCG1R_DB81_seg19-20 | CCNG1 | 1.16 | 1.35 | 2.2 | 6.00E-03 | 3.00E-09 |
| H31883_DB71_seg13 | CCNG1 | 1.25 | 1.41 | 1.51 | 5.00E-03 | 0.04 |
| W33294_DB81_seg23 | WDR77 | 1.44 | 1.27 | 1.9 | 0.05 | 7.00E-04 |
| W33294_DB81_seg44 | WDR77 | 1.37 | 1.42 | 2.02 | 0.6 | 8.00E-06 |
| H33998_DB71_seg19 | WDR77 | 1.31 | 1.58 | 2.13 | 0.04 | 2.00E-07 |
| RATLRFI_DB71_seg9 | ATF3 | 0.84 | 1.47 | 1.39 | 0.025 | 5.00E-04 |
| RATRAB13X_DB81_seg15-17 | RAB 13 | 1.32 | 1.37 | 1.89 | 0.05 | 3.00E-07 |
| RATRAB13X_DB81_seg22 | RAB13 | 1.36 | 1.37 | 2.12 | 0.04 | 3.00E-07 |
| RATRAB13X_s DB71_eg11-13 | RAB 13 | 1.40 | 1.59 | 1.42 | 1.00E-03 | 0.6 |
| MMU09507_DB81_seg15 | CDKN1A | 0.88 | 0.94 | 1.74 | 0.08 | 2.00E-06 |
| RNU24174_DB71_seg8 | CDKN1A | 0.76 | 0.91 | 1.21 | 2.00E-05 | 3.00E-05 |

The qRT-PCR data was used to construct the optimal Random-Forest classifier for Day-5 (Table 13) and Day-1 (Table 16). The optimal performance of the Day-5 signatures, as measured by cross validation is - Accuracy of 94% and LOR of 5.6. We further tested the performance of the optimal classifier on the Day-5 animals treated with Cadmium Chloride and found that 5 out of 6 samples were classified correctly as toxic. The optimal performance of the Day-1 signature is - Accuracy of 89% and LOR of 4.2

Table 14 and Table 15 further list genes that contribute to signatures for classification of Day-5 data. Table 17 and Table 18 further list genes that contribute to signatures of classification of Day-1. These genes can be used to replace the genes in Table 13 and Table 16 to yield classifiers with LOR of at least 3.75.

Table 12: All genes

| Candidate ID | Contig | Internal name | Gene name | NV* / GenbankID | Reference |
|---|---|---|---|---|---|
| 1 | AA686189 | W41270_DB81_ seg11 | TNFRSF12A | NV of NM181086 | |
| 2 | AA686189 | AA686189_ DB71_seg6 | TNFRSF12A | NM181086 | Peter et al., Physiol. Genomics 25: 375-386, 2006. |
| 3 | AA799594 | AA799594_ DB71_seg0 | AA799594 | AI008646; DY471185 (ESTS, no RNA) | |
| 4 | AA964541 | AA964541_ DB71_seg0 | AA964541 | AA964541 (one sequence - EST) | WO02095000 |
| 5 | AI045075 | W64472_DB81_seg2 | ISG20 | NV of NM001008510 | |
| 6 | AI045075 | AI045075_DB71_seg6 | ISG20 | NM001008510 | WO02095000 |
| 7 | AI454051 | AI454051_DB71_seg0 | AI454051 | BE118959 (EST, no RNA) | |
| 8 | AI502869 | AI502869_DB71_seg0 | AI502869 | AI502869;DV713733; AI145914;CB796640 (ESTs, no RNA) | |
| 9 | AW919147 | AW919147_DB71_seg0 | AW919147 | AW91914; BF392593 (ESTS, no RNA) | |
| 10 | BI293562 | BI293562_DB71_seg2 | GDF15 | NM01921;BI293562 (BI293562 is an EST that extends the transcript) | Peter et al., Physiol. Genomics 25: 375-386, 2006. |
| 11 | H31045 | H31045_DB71_seg5 | H31045 | CO569017;DY312216; CF115262;BM392249; AI556451 (ESTS, no RNA.) | |
| 12 | H31799 | W83813_DB81_seg27 | EI24 | NV of NM001025660 | |
| 13 | H31799 | H31799_DB71_seg23 | EI24 | NM001025660 | |
| 14 | H31883 | MUSCYCG1R_DB81_ seg15-17 | CCNG1 | NV of NM012923 | |
| 15 | H31883 | MUSCYCG1R_DB81_ seg19-20 | CCNG1 | NV of NM012923 | |
| 16 | H31883 | H31883_DB71_seg13 | CCNG1 | NM012923 | Thompson et al., Environ Health Perspect. 2004 Mar;112(4):488 -94; WO02095000 |
| 17 | H33998 | W33294_DB81_seg23 | WDR77 | NV of NM001008771 | |
| 18 | H33998 | W33294_DB81_seg44 | WDR77 | NV of NM001008771 | |
| 19 | H33998 | H33998_DB71_seg19 | WDR77 | NM001008771 | |

(continued)

| Candidate ID | Contig | Internal name | Gene name | NV* / GenbankID | Reference |
|---|---|---|---|---|---|
| 20 | RATLRFI | RATLRFI_DB71_seg9 | ATF3 | NM012912 | Peter et al., Physiol. Genomics 25: 375-386, 2006. WO2004048598 WO200295000 |
| 21 | RATRAB13X | RATRAB13X_DB81_seg15-17 | RAB 13 | NV of NM031092 | |
| 22 | RATRAB13X | RATRAB13X_DB81_seg22 | RAB 13 | NV of NM031092 | |
| 23 | RATRAB13X | RATRAB13X_DB71_seg11-13 | RAB 13 | NM031092 | WO02095000 |
| 24 | RNU24174 | MMU09507_DB81_seg15 | CDKN1A | NV of NM080782 | |
| 25 | RNU24174 | RNU24174_DB71_ seg8 | CDKN1A | NM080782 | Peter et al., Physiol. Genomics 25: 375-386, 2006. WO2004048598 ; WO0295000 |

*NV identifies novel polynucleotides and proteins, which may or may be not variants of known proteins.

Table 13: Optimal signature for Day-5 data

| Candidate ID | Contig | Internal name | Gene name |
|---|---|---|---|
| 1 | AA686189 | W41270_DB81_seg11 | TNFRSF12A |
| 5 | A045075 | W64472_DB81_seg2 | ISG20 |
| 12 | H31799 | W83813_DB81_seg27 | EI24 |
| 15 | H31883 | MUSCYCG1R_DB81_seg19-20 | CCNG1 |

Table 14: Novel-Variants for Day-5 signatures

| Candidate ID | Contig | Internal name | Gene name |
|---|---|---|---|
| 1 | AA686189 | W41270_DB81_seg11 | TNFRSF12A |
| 3 | AA799594 | AA799594_DB71_seg0 | AA799594 |
| 5 | A045075 | W64472_DB81_seg2 | ISG20 |
| 7 | AI454051 | AI454051_DB71_seg0 | AI454051 |
| 8 | AI502869 | AI502869_DB71_segO | AI502869 |
| 9 | AW919147 | AW919147_DB71_seg0 | AW919147 |
| 12 | H31799 | W83813_DB81_seg27 | EI24 |
| 14 | H31883 | MUSCYCG1R_DB81_seg15-17 | CCNG1 |
| 15 | H31883 | MUSCYCG1R_DB81_seg_19-20 | CCNG1 |
| 18 | H33998 | W33294_DB81_seg44 | WDR77 |
| 21 | RATRAB13X | RATRAB13X_DB81_seg15-17 | RAB 13 |
| 22 | RATRAB13X | RATRAB13X_DB81_seg22 | RAB 13 |
| 24 | RNU24174 | MMU09507_DB81_seg15 | CDKN1A |

Table 15: Wild-Types for Day-5 signatures

| Candidate ID | Contig | Internal name | Gene name |
|---|---|---|---|
| 4 | AA964541 | AA964541_DB71_seg0 | AA964541 |
| 10 | BI293562 | BI293562_DB71_seg2 | GDF15 |
| 19 | H33998 | H33998_DB71_seg19 | WDR77 |
| 20 | RATLRFI | RATLRFI_DB71_seg9 | ATF3 |
| 25 | RNU24174 | RNA24174_DB71_seg8 | CDKN1A |

Table 16: Optimal signature for Day-1 data

| Candidate ID | Contig | Internal name | Gene name |
|---|---|---|---|
| 1 | AA686189 | W41270_DB81_seg11 | TNFRSF12A |
| 4 | AA964541 | AA964541_DB71_seg0 | AA964541 |
| 6 | A045075 | AI045075_DB71_seg6 | ISG20 |
| 15 | H31883 | MUSCYCG1R_DB81_seg19-20 | CCNG1 |
| 20 | RATLRFI | RATLRFI_ DB71_seg9 | ATF3 |

(continued)

| Candidate ID | Contig | Internal name | Gene name |
|---|---|---|---|
| 23 | RATRAB13X | RATRAB13X_DB71_seg11-13 | RAB 13 |
| 25 | RNU24174 | RNA24174_DB71_seg8 | CDKN1A |

Table 17: Novel-Variants for Day-1 signatures

| Candidate ID | Contig | Internal name | Gene name |
|---|---|---|---|
| 1 | AA686189 | W41270_DB81_seg11 | TNFRSF12A |
| 3 | AA799594 | AA799594_DB71_seg0 | AA799594 |
| 8 | AI502869 | AI502869_DB71_seg0 | AI502869 |
| 11 | H31045 | H31045_DB71_seg5 | H31045 |
| 14 | H31883 | MUSCYCG1R_DB81_seg15-17 | CCNG1 |
| 15 | H31883 | MUSCYCG1R_DB81_seg19-20 | CCNG1 |
| 17 | H33998 | W33294_DB81_seg23 | WDR77 |
| 21 | RATRAB13X | RATRAB13X_DB81_seg15-17 | RAB 13 |

Table 18: Wild-Types for Day-1 signatures

| Candidate ID | Contig | Internal name | Gene name |
|---|---|---|---|
| 2 | AA686189 | AA686189_DB71_seg6 | TNFRSF12A |
| 4 | AA964541 | AA964541_DB71_seg0 | AA964541 |
| 6 | A045075 | AI045075_DB71_seg6 | ISG20 |
| 16 | H31883 | H31883_DB71_seg13 | CCNG1 |
| 19 | H33998 | H33998_DB71_seg19 | WDR77 |
| 20 | RATLRFI | RATLRFI_DB71_seg9 | ATF3 |
| 23 | RATRAB13X | RATRAB13X_DB71_seg11-13 | RAB 13 |
| 25 | RNU24174 | RNU24174_DB71_seg8 | CDKN1A |

Table 19 below summarizes the SEQ ID NOs for nodes, detector nodes, other unique nodes, transcripts and proteins for each marker disclosed herein. Nodes are segments within a transcript that might, according to the predictions made by the LEADS platform, represent a single exon or an alternative extension of an exon. Unique nodes are the ones that appear only in a novel variant or polynucleotide and not in the wild-type. The nodes listed for new variants and polynucleotides are unique nodes. When other unique nodes exist for new-variants and polynucleotides of the same genes, they are also given. The detector nodes are the set of nodes within which the primers were designed. When possible it is the same as the unique node (and not given separately), but when the unique node is too short the detector node might include other neighboring nodes as well.

Table 19: Candidate SEQ IDs

| Contig | Internal name | Candidate ID | Node SEQ. ID No. | Detector nodes SEQ. ID No. | Other unique nodes SEQ. ID No. | Transcript SEQ. ID No. | Protein SEQ. ID No. |
|---|---|---|---|---|---|---|---|
| AA686189 | W41270_DB81_seg11 | 1 | 2 | | | 1 | 62 |
| AA686189 | AA686189_DB71_seg6 | 2 | 45 | | | 44 | 73 |
| AA799594 | AA799594_DB71_seg0 | 3 | 33 | | | 32 | |
| AA964541 | AA964541_DB71_seg0 | 4 | 47 | | | 46 | |
| AI045075 | W64472_DB81_seg2 | 5 | 4 | | 5 | 3 | 63 |
| AI045075 | AI045075_DB71_seg6 | 6 | 49 | | | 48 | 74 |
| AI454051 | AI454051_DB71_seg0 | 7 | 35 | | | 34 | |
| AI502869 | AI502869_DB71_seg0 | 8 | 37 | | | 36 | |
| AW919147 | AW919147_DB71_seg0 | 9 | 39 | | | 38 | |
| BI293562 | BI293562_DB71_seg2 | 10 | 51 | | | 50 | 75 |
| H31045 | H31045_DB71_seg5 | 11 | 41 | | | 40 | 71 |
| H31799 | W83813_DB81_seg27 | 12 | 7 | | 8 | 6 | 64 |
| H31799 | H31799_DB71_seg23 | 13 | 43 | | | 42 | 72 |
| H31883 | MUSCYCG1R_DB81_seg15-17 | 14 | 10 | 11 | | 9 | 65 |
| H31883 | MUSCYCG1R_DB81_seg19-20 | 15 | 13 | 14 | | 12 | 66 |
| H31883 | H31883_DB71_seg13 | 16 | 53 | | | 52 | 76 |
| H33998 | W33294_DB81_seg23 | 17 | 16 | | 17 18 19 | 15 | 67 |
| H33998 | W33294_DB81_seg44 | 18 | 21 | | 2223 | 20 | 68 |
| H33998 | H33998_DB71_seg19 | 19 | 55 | | | 54 | 77 |
| RATLRFI | RATLRFI_DB71_seg9 | 20 | 57 | | | 56 | 78 |
| RATRAB13X | RATRAB13X_DB81_seg15-17 | 21 | 25 | 26 | | 24 | 69 |
| RATRAB13X | RATRAB13X_DB81_seg22 | 22 | 28 | | 29 | 27 | 70 |
| RATRAB13X | RATRAB13X_DB71_seg11-13 | 23 | 59 | | | 58 | 79 |
| RNU24174 | MMU09507_DB81_seg15 | 24 | 31 | | | 30 | |
| RNU24174 | RNU24174_DB71_seg8 | 25 | 61 | | | 60 | 80 |

Table 20 below summarizes the SEQ ID NOs for primers used to amplify specific amplicons for each marker disclosed herein.

Table 20: Candidate Primers and Amplicons

| Contig | Internal name | Candidate ID | Forward primer SEQ. ID No. | Reverse primer SEQ. ID No. | Amplicon SEQ. ID No. |
|---|---|---|---|---|---|
| AA686189 | W41270_DB81_seg11 | 1 | 252 | 253 | 254 |
| AA686189 | AA686189_DB71_seg6 | 2 | 249 | 250 | 251 |
| AA799594 | AA799594_DB71_seg0 | 3 | 255 | 256 | 257 |
| AA964541 | AA964541_DB71_seg0 | 4 | 258 | 259 | 260 |
| AI045075 | W64472_DB81_seg2 | 5 | 264 | 265 | 266 |
| AI045075 | W64472_DB81_seg2_F6R1 | 5 | 327 | 328 | 329 |
| AI045075 | AI045075_DB71_seg6 | 6 | 261 | 262 | 263 |
| AI454051 | AI454051_DB71_seg0 | 7 | 267 | 268 | 269 |
| AI502869 | AI502869_DB71_seg0 | 8 | 270 | 271 | 272 |
| AW919147 | AW919147_DB71_seg0 | 9 | 273 | 274 | 275 |
| BI293562 | BI293562_DB71_seg2 | 10 | 276 | 277 | 278 |
| H31045 | H31045_DB71_seg5 | 11 | 279 | 280 | 281 |
| H31799 | W83813_DB81_seg27 | 12 | 285 | 286 | 287 |
| H31799 | H31799_DB71_seg23 | 13 | 282 | 283 | 284 |
| H31883 | MUSCYCG1R_DB81_seg15-17 | 14 | 291 | 292 | 293 |
| H31883 | MUSCYCG1R_DB81_seg19-20 | 15 | 294 | 295 | 296 |
| H31883 | H31883_DB71_seg13 | 16 | 288 | 289 | 290 |
| H31883 | H31883_DB71_seg13_F5R5 | 16 | 330 | 331 | 332 |
| H33998 | W33294_DB81_seg23 | 17 | 297 | 298 | 299 |
| H33998 | W33294_DB81_seg44 | 18 | 300 | 301 | 302 |
| H33998 | H33998_DB71_seg19 | 19 | 303 | 304 | 305 |
| RATLRFI | RATLRFI_DB71_seg9 | 20 | 306 | 307 | 308 |
| RATRAB13X | RATRAB13X_DB81_seg15-17 | 21 | 309 | 310 | 311 |
| RATRAB13X | RATRAB13X_DB81_seg22 | 22 | 312 | 313 | 314 |
| RATRAB13X | RATRAB13X_DB71_seg11-13 | 23 | 315 | 316 | 317 |
| RNU24174 | MMU09507_DB81_seg15 | 24 | 321 | 322 | 323 |
| RNU24174 | RNU24174_DB71 _seg8 | 25 | 324 | 325 | 326 |

Table 21 below provides human and mouse orthologous sequences for each rat marker disclosed herein.

Table 21: Candidates Homologues

| Contig | Internal name | Candidate ID | Human homologue transcripts SEQ. ID No. | Human homologue protein SEQ. ID No. | Mouse homologue transcripts SEQ. ID No. | Mouse homologue proteins SEQ. ID No. |
|---|---|---|---|---|---|---|
| AA686189 | W41270_DB81 _seg11 | 1 | 105 106 107 108 | 157 158 159 160 | 197 198 199 200 | 226 227 228 229 |

(continued)

| Contig | Internal name | Candidate ID | Human homologue transcripts SEQ. ID No. | Human homologue protein SEQ. ID No. | Mouse homologue transcripts SEQ. ID No. | Mouse homologue proteins SEQ. ID No. |
|---|---|---|---|---|---|---|
| AA686189 | AA686189_DB71_seg6 | 2 | 105 106 107 108 | 157 158 159 160 | 197 198 199 200 | 226 227 228 229 |
| AA799594 | AA799594_DB71_seg0 | 3 | | | | |
| AA964541 | AA964541_DB71_seg0 | 4 | | | | 190 |
| AI045075 | W64472_DB81_seg2 | 5 | 112 113 114 | 163 164 | 202 203 204 | 231 232 233 |
| AI045075 | AI045075_DB71_seg6 | 6 | 112 113 114 | 163 164 | 202 203 204 | 231 232 233 |
| AI454051 | AI454051_DB71_seg0 | 7 | | | | 185 |
| AI502869 | AI502869_DB71_seg0 | 8 | | | | |
| AW919147 | AW919147_DB71_seg0 | 9 | | | | 195 196 |
| BI293562 | BI293562_DB71_seg2 | 10 | 100 101 102 103 104 | 154 155 156 | 186 | 215 |
| H31045 | H31045_DB71_seg5 | 11 | 134 135 136 137 | 173 174 175 176 | 179 180 181 182 183 184 | 209 210 211 212 213 214 |
| H31799 | W83813_DB81_seg27 | 12 | 83 84 85 86 87 88 89 | 140 141 142 143 144 | 205 206 207 | 234 235 236 |
| H31799 | H31799_DB71_seg23 | 13 | 83 84 85 86 87 88 89 | 140 141 142 143 144 | 205 206 207 | 234 235 236 |
| H31883 | MUSCYCG1R_DB81_seg15-17 | 14 | 109 110 111 | 161 162 | 188 189 | 217218 |
| H31883 | MUSCYCG1R_DB81_seg19-20 | 15 | 109 110 111 | 161 162 | 188 189 | 217218 |
| H31883 | H31883_DB71_seg13 | 16 | 109 110 111 | 161 162 | 188 189 | 217218 |
| H33998 | W33294_DB81_seg23 | 17 | 90 91 92 394 95 96 97 98 99 | 145 146 147 148 149 150 151 152 153 | 191 192 193 194 | 220 221 222 223 |
| H33998 | W33294_DB81_seg44 | 18 | 90 91 92 394 95 96 97 98 99 | 145 146 147 148 149 150 151 152 153 | 191 192 193 194 | 220 221 222 223 |
| H33998 | H33998_DB71_seg19 | 19 | 90 91 92 394 95 96 97 98 99 | 145 146 147 148 149 150 151 152 153 | 191 192 193 194 | 220 221 222 223 |
| RATLRFI | RATLRFI_DB71_seg9 | 20 | 81 82 | 138 139 | 177 178 | 208 |
| RATRAB13X | RATRAB13X_DB81_seg15-17 | 21 | 115 116 117 118 119 | 165 166 167 168 169 | 201 | 230 |

(continued)

| Contig | Internal name | Candidate ID | Human homologue transcripts SEQ. ID No. | Human homologue protein SEQ. ID No. | Mouse homologue transcripts SEQ. ID No. | Mouse homologue proteins SEQ. ID No. |
|---|---|---|---|---|---|---|
| RATRAB13X | RATRAB13X _DB81_seg22 | 22 | 115 116 117 118 119 | 165 166 167 168 169 | 201 | 230 |
| RATRAB13X | RATRAB13X _DB71_seg11-13 | 23 | 115 116 117 118 119 | 165 166 167 168 169 | 201 | 230 |
| RNU24174 | MMU09507_ DB81_seg15 | 24 | 120 121 122 123 124 125 126 127 128 129 130 131 132 133 | 170 171 172 | 187 | 216 |
| RNU24174 | RNU24174_D B71_seg8 | 25 | 120 121 122 123 124 125 126 127 128 129 130 131 132 133 | 170 171 172 | 187 | 216 |

***EXAMPLE 2: Real Time qPCR analysis of the selected markers***

Example 2.1: Expression of tumor necrosis factor receptor superfamily, member 12a, AA686189, transcripts which are detectable by amplicon as depicted in sequence name AA686189_DB71_seg6 in kidney tissues of treated or untreated rats

Expression of tumor necrosis factor receptor superfamily, member 12a transcripts detectable by or according to seg6 - AA686189_DB71_seg6_F1R1 (SEQ ID NO: 251) amplicon and primers AA686189_DB71_seg6_F1 (SEQ ID NO: 249) and AA686189_DB71_seg6_R1 (SEQ ID NO: 250) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled AA686189_DB71_seg6 in Table 22 contains the normalized expression values of the above-indicated tumor necrosis factor receptor superfamily, member 12a transcript in treated or untreated kidney samples.

As is evident from the column entitled AA686189_DB71_seg6 in Table 22, the level of expression of the tumor necrosis factor receptor superfamily, member 12a transcript detectable by the above amplicon was significantly higher in the samples treated with toxic compounds (samples 1-58 and 76-105, in kidney tissue panel described in Table 9 hereinabove) than in the control samples (naive, saline and valproic acid treated samples; samples numbers- 59-75 and 106-129, in kidney tissue panel described in Table 9 hereinabove). Statistical analysis was applied to verify the significance of these results, P-value for day 1: $8.00E^{-04}$ and P-value for day 5: $7.5E^{-06}$.

Primer pairs are also optionally and preferably used in context with the present invention; for example, for the above experiment, the following primer pair was used as a non-limiting illustrative example only of a suitable primer pair AA686189_DB71_seg6_F1 (SEQ ID NO: 249) forward primer; and AA686189_DB71_seg6_R1 (SEQ ID NO: 250) reverse primer.

Amplicons obtained through the use of any suitable primer pair; for example, for the above experiment, may be used in the context of the present invention; the following amplicon was obtained as a non-limiting illustrative example only of a suitable amplicon: AA686189_DB71_seg6_F1R1 (SEQ ID NO: 251).

Forward primer (AA686189_DB71_seg6_F1 (SEQ ID NO: 249)): CCAAGGACTGGGCTTAGGGT

Reverse primer (AA686189_DB71_seg6_R1 (SEQ ID NO: 250)): AGAGATTCCCTTGTGCAAATGC

Amplicon (AA686189_DB71_seg6_F1R1 (SEQ ID NO: 251))

CCAAGGACTGGGCTTAGGGTTCAGGGGAGCCTTCCAGGGTGTCTAATTGCCCTGTCTC
TGGTTCTGGGGCAGACAGAGAGCCTCAAGCTAGGTCACAAAGCGACTCATACTAAGGATCT
GCAGCATTTGCACAAGGGAATCTCT

Example 2.2: Expression of tumor necrosis factor receptor superfamily, member 12a, AA686189, transcripts which are detectable by amplicon as depicted in sequence name W41270_DB81_seg11 in kidney tissues of treated or untreated rats

Expression of tumor necrosis factor receptor superfamily, member 12a transcripts detectable by or according to seg11 - W41270_DB81_seg11_F2R2 (SEQ ID NO: 254) amplicon and primers W41270_DB81_seg11_F2 (SEQ ID NO: 252) and W41270_DB81_seg11_R2 (SEQ ID NO: 253) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled W41270_DB81_seg11 in Table 22 contains the normalized expression values of the above-indicated tumor necrosis factor receptor superfamily, member 12a transcript in treated or untreated kidney samples.

As is evident from the column entitled W41270_DB81_seg11 in Table 22, the level of expression of the tumor necrosis factor receptor superfamily, member 12a transcript detectable by the above amplicon was significantly higher in the samples treated with toxic compounds (samples 1-58 and 76-105 , in kidney tissue panel described in Table 9 hereinabove) than in the control samples (naive, saline and valproic acid treated samples; samples numbers- 59-75 and 106-129, in kidney tissue panel described in Table 9 hereinabove). Statistical analysis was applied to verify the significance of these results, P-value for day 1: $4.00E^{-04}$, and P-value fro day 5: $7.50E^{-10}$.

Primer pairs may also be used in the context of the present invention; for example, for the above experiment, the following primer pair was used as a non-limiting illustrative example only of a suitable primer pair: W41270_DB81_seg11_F2 (SEQ ID NO: 252) forward primer; and W41270_DB81_seg11_R2 (SEQ ID NO: 253) reverse primer.

Amplicons obtained through the use of any suitable primer pair; for example, for the above experiment, may also be used in the context of the present invention; the following amplicon was obtained as a non-limiting illustrative example only of a suitable amplicon: W41270_DB81_seg11_F2R2 (SEQ ID NO: 254).

Forward primer (W41270_DB81_seg11_F2 (SEQ ID NO: 252)): GATCTGGGTAGGTGGTTGTTGG

Reverse primer (W41270_DB81_seg11_R2 (SEQ ID NO: 253)): CGCACACCCTTATAAAAGTCCC

Amplicon (W41270_DB81_seg11_F2R2 (SEQ ID NO: 254)):

GATCTGGGTAGGTGGTTGTTGGGGCAGAAAGGAGGTCGTAGACTTAGGATATAGGAA
ACCAGGAAAAACTGACTGAGGAAGGGACTTTTATAAGGGTGTGCG

Example 2.3: Expression of AA799594, transcripts which are detectable by amplicon as depicted in sequence name AA799594_DB71_seg0 in kidney tissues of treated or untreated rats

Expression of A799594 detectable by or according to seg0 - AA799594_DB71_seg0_F1R1 (SEQ ID NO: 257) amplicon and primers AA799594_DB71_seg0_F1 (SEQ ID NO: 255) and AA799594_DB71_seg0_R1 (SEQ ID NO: 256) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled AA799594_DB71_seg0 in Table 22 contains the normalized expression values of the above-indicated AA799594 transcript in treated or untreated kidney samples.

As is evident from the column entitled AA799594_DB71_seg0 in Table 22, the level of expression of AA799594 transcript detectable by the above amplicon was significantly higher in the samples treated with toxic compounds (samples 1-58 and 76-105, in kidney tissue panel described in hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 1: $8.00E^{-03}$ and P-value for day 5: 0.01.

Primer pairs may also be used in the context of the present invention; for example, for the above experiment, the following primer pair was used as a non-limiting illustrative example only of a suitable primer pair:

AA799594_DB71_seg0_F1 (SEQ ID NO: 255) forward primer; and AA799594_DB71_seg0_R1 (SEQ ID NO: 256) reverse primer.

Amplicons obtained through the use of any suitable primer pair; for example, for the above experiment, may also be used in the context of the present invention; the following amplicon was obtained as a non-limiting illustrative example only of a suitable amplicon: AA799594_DB71_seg0_F1R1 (SEQ ID NO: 257).

Forward primer (AA799594_DB71_seg0_F1 (SEQ ID NO: 255)): ATCTCGATGGTAACGGGTCTAATC

Reverse primer (AA799594_DB71_seg0_R1 (SEQ ID NO: 256)): TTTGTGCTGACCTTCATGCC

Amplicon (AA799594_DB71_seg0_F1R1 (SEQ ID NO: 257)):

ATCTCGATGGTAACGGGTCTAATCAGCCCATGATCAACATAACTGTGGTGATATACAT
TTGGTATTTTTTAATTTTCGGATGCCTTCCTCAACATAGCCGTCAAGGCATGAAGGTCAGCAC
AAA

Example 2.4: Expression of AA964541, transcripts which are detectable by amplicon as depicted in sequence name AA964541_DB71_seg0 in kidney tissues of treated or untreated rats

Expression of AA964541 transcripts detectable by or according to seg0 - AA964541_DB71_seg0_F2R2 (SEQ ID NO: 260) amplicon and primers AA964541_DB71_seg0_F2 (SEQ ID NO: 258) and AA964541_DB71_seg0_R2 (SEQ ID NO: 259) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled AA964541_DB71_seg0 in Table 22 contains the normalized expression values of the above-indicated AA964541 transcript in treated or untreated kidney samples.

As is evident from the column entitled AA964541_DB71_seg0 in Table 22, the level of expression of A964541 transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 1: $2.00E^{-06}$, and P-Value for day5: $3.00E^{-08}$.

Primer pairs may also be used in the context ofthe present invention; for example, for the above experiment, the following primer pair was used as a non-limiting illustrative example only of a suitable primer pair: AA964541_DB71_seg0_F2 (SEQ ID NO: 258) forward primer; and AA964541_DB71 _seg0_R2 (SEQ ID NO: 259) reverse primer.

Amplicons obtained through the use of any suitable primer pair; for example, for the above experiment, may also be used in the context of the present invention; the following amplicon was obtained as a non-limiting illustrative example only of a suitable amplicon:

AA964541_DB71_seg0_F2R2 (SEQ ID NO: 260).

Forward primer (AA964541_DB71_seg0_F2 (SEQ ID NO: 258)): CCAGCCTAGCCTCTCTTTTGC

Reverse primer (AA964541_DB71_seg0_R2 (SEQ ID NO: 259)): AACATTCCCACAGGGTACATTCA

Amplicon (AA964541_DB71_seg0_F2R2 (SEQ ID NO: 260)):

CCAGCCTAGCCTCTCTTTTGCACTGCTGGTTCAGCCCACTGGGCCTCCGTCCTTTCCTC
TGGAAGGGACTTGGCCTTGGGTGACAAATTCCTCTTTGATGAATGTACCCTGTGGGAATGTT

Example 2.5: Expression of Interferon stimulated exonuclease 20 (ISG20), AI045075, transcripts which are detectable by amplicon as depicted in sequence name AI045075_DB71_seg6 in kidney tissues of treated or untreated rats

Expression of Interferon stimulated exonuclease 20 (ISG20) transcripts detectable by or according to seg6 - AI045075_DB71_seg6_F2R2 (SEQ ID NO: 263) amplicon and primers AI045075_DB71_seg6_F2 (SEQ ID NO: 261) and AI045075_DB71_seg6_R2 (SEQ ID NO: 262) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled AI045075_DB71_seg6 in Table 22 contains the normalized expression values of the above-indicated Interferon stimulated exonuclease 20 (ISG20) transcript in treated or untreated kidney samples.

As is evident from the column entitled AI045075_DB71_seg6 in Table 22, the level of expression of the Interferon stimulated exonuclease 20 (ISG20) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-Value for day 1: $2.50E^{-04}$, and P-Value for day5: $6.50E^{-04}$.

Primer pairs may also be used in the context of the present invention; for example, for the above experiment, the following primer pair was used as a non-limiting illustrative example only of a suitable primer pair: AI045075_DB71_seg6_F2 (SEQ ID NO: 261) forward primer; and AI045075_DB71_seg6_R2 (SEQ ID NO: 262) reverse primer.

Amplicons obtained through the use of any suitable primer pair; for example, for the above experiment, may also be used in the context of the present invention; the following amplicon was obtained as a non-limiting illustrative example only of a suitable amplicon: AI045075_DB71_seg6_F2R2 (SEQ ID NO: 263).

Forward primer (AI045075_DB71_seg6_F2 (SEQ ID NO: 261)): GGGCCACAATGGAGCTCTAC

Reverse primer (AI045075_DB71_seg6_R2 (SEQ ID NO: 262)): ACAGGTCTCATTCATGGAAAACTATG

Amplicon (AI045075_DB71_seg6_F2R2 (SEQ ID NO: 263)):

GGGCCACAATGGAGCTCTACAAAATCTCTCAGCGACTCAGAGCCCAGCGAGGGCTGC
CCTGCCTGGGAACATCAGCCTGAACTTCATCCTCATCCAGGATCAGAAGCAGCTACTCCTTG
AAGGACCATAGTTTTCCATGAATGAGACCTGT

Example 2.6: Expression of Interferon stimulated exonuclease 20 (ISG20), AI045075, transcripts which are detectable by amplicon as depicted in sequence name W64472_DB81_seg2_ in kidney tissues of treated or untreated rats

Expression of Interferon stimulated exonuclease 20 (ISG20), AI045075, transcripts detectable by or according to seg2 - W64472_DB81_seg2_F1R1 (SEQ ID NO: 266) amplicon and primers W64472_DB81_seg2_F1 (SEQ ID NO: 264) and W64472_DB81_seg2_R1 (SEQ ID NO: 265) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled W64472_DB81_seg2 in Table 22 contains the normalized expression values of the above-indicated Interferon stimulated exonuclease 20 (ISG20), A045075, transcript in treated or untreated kidney samples.

As is evident from the column entitled W64472_DB81_seg2 in Table 22, the level of expression of the Interferon stimulated exonuclease 20 (ISG20), AI045075, transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 5: $2.00E^{-10}$.

Primer pairs may also be used in the context of the present invention; for example, for the above experiment, the following primer pair was used as a non-limiting illustrative example only of a suitable primer pair: W64472_DB81_seg2_F1 (SEQ ID NO: 264) forward primer; and W64472_DB81_seg2_R1 (SEQ ID NO: 265) reverse primer.

Amplicons obtained through the use of any suitable primer pair; for example, for the above experiment, may also be used in the context of the present invention; the following amplicon was obtained as a non-limiting illustrative example only of a suitable amplicon:

W64472_DB81_seg2_F1R1 (SEQ ID NO: 266).

Forward primer (W64472_DB81_seg2_F1 (SEQ ID NO: 264)): GGAGGAGGGCAGAGCCA

Reverse primer (W64472_DB81_seg2_R1 (SEQ ID NO: 265)): TCTGGTTCATTATCAAGGGAAGTTG

Amplicon (W64472_DB81_seg2_F1R1 (SEQ ID NO: 266)):

GGAGGAGGGCAGAGCCAGAGGAGGGACAGCCTGATGCAGACAGCCCTGACTCAACC
TGCCAGCCCCCTTACCTGTCAGCCTTGAGGAGATGGAACAGCCCAGCCTACTAGGCCTGCCC
CCACTCCCCAACTTCCCTTGATAATGAACCAGA

Example 2.7: Expression of AI454051 transcripts which are detectable by amplicon as depicted in sequence name AI454051_DB71_seg0 in kidney tissues of treated or untreated rats

Expression of AI454051 transcripts detectable by or according to seg0 - AI454051_DB71_seg0_F1R1 (SEQ ID NO: 269) amplicon and primers AI454051_DB71_seg0_F1 (SEQ ID NO: 267) and AI454051_DB71_seg0_R1 (SEQ ID NO: 268) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled AI454051_DB71_seg0 in Table 22 contains the normalized expression values of the above-indicated AI454051 transcript in treated or untreated kidney samples.

As is evident from the column entitled AI454051_DB71_seg0 in Table 22, the level of expression of the AI454051 transcript detectable by the above amplicon was lower in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove).

Primer pairs may also be used in the context of; for example, for the above experiment, the following primer pair was used as a non-limiting illustrative example only of a suitable primer pair: AI454051_DB71_seg0_F1 (SEQ ID NO: 267) forward primer; and AI454051_DB71_seg0_R1 (SEQ ID NO: 268) reverse primer.

Amplicons obtained through the use of any suitable primer pair; for example, for the above experiment, may also be used in the context of the present invention; the following amplicon was obtained as a non-limiting illustrative example only of a suitable amplicon: AI454051_DB71_seg0_F1R1 (SEQ ID NO: 269).

Forward primer (AI454051_DB71_seg0_F1 (SEQ ID NO: 267)): ATCCTGTTGGGTCTGCTTCTCA

Reverse primer (AI454051_DB71_seg0_R1 (SEQ ID NO: 268)): CACAAGTAAATACATAGACTGGAATCAATG

Amplicon (AI454051_DB71_seg0_F1R1 (SEQ ID NO: 269)):

ATCCTGTTGGGTCTGCTTCTCACAGCGTATGGGGCTGTGTGCTTTTACTCATGGTGGCA
GCAGCAGTTGTTGTCACTTCTTTGGCCAGGCACAGTGGGTGATCCTTACAGAGCAGCACAGA
TTCCCATTGATTCCAGTCTATGTATTTACTTGTG

Example 2.8: Expression of AI502869 transcripts which are detectable by amplicon as depicted in sequence name AI502869_DB71_seg0 in kidney tissues of treated or untreated rats

Expression of AI502869 transcripts detectable by or according to seg0 - AI502869_DB71_seg0_F1R1 (SEQ ID NO: 272) amplicon and primers AI502869_DB71_seg0_F1 (SEQ ID NO: 270) and AI502869_DB71_seg0_R1 (SEQ ID NO: 271) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled AI502869_DB71_seg0 in Table 22 contains the normalized expression values of the above-indicated AI502869 transcript in treated or untreated kidney samples.

As is evident from the column entitled AI502869_DB71_seg0_F1R1 (SEQ ID NO: 272) in Table 22, the level of expression of the AI502869 transcript detectable by the above amplicon was significantly lower in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 1: $7.00E^{-03}$ and P-value for day 5: $2.50E^{-05}$.

Primer pairs may also be used in the context of the present invention; for example, for the above experiment, the following primer pair was used as a non-limiting illustrative example only of a suitable primer pair: AI502869_DB71_seg0_F1 (SEQ ID NO: 270) forward primer; and AI502869_DB71_seg0_R1 (SEQ ID NO: 271) reverse primer.

Amplicons obtained through the use of any suitable primer pair; for example, for the above experiment, may also be used in the context of the present invention; the following amplicon was obtained as a non-limiting illustrative example only of a suitable amplicon:

Forward primer (AI502869_DB71_seg0_F1 (SEQ ID NO: 270)): TGTTTAAAATCTGGGCTCTTGATTC

Reverse primer (AI502869_DB71_seg0_R1 (SEQ ID NO: 271)): GTATTTCCAATAGTCAATGCAGTAACTCTAC

Amplicon (AI502869_DB71_seg0_F1R1 (SEQ ID NO: 272)):

TGTTTAAAATCTGGGCTCTTGATTCAGACCGAGTTTTAATACTGGATCTCATTCAATCT TTGTGTTTTTCTTTTCTTTTCTTTTTTTCCAATAGTACGGCAGTGCTAATAGCAGTCCTTTA GTAGAGTTACTGCATTGACTATTGGAAATAC

Example 2.9: Expression of AW919147 transcripts which are detectable by amplicon as depicted in sequence name AW919147_DB71_seg0 in kidney tissues of treated or untreated rats

Expression of AW919147 transcripts detectable by or according to seg0 - AW919147_DB71_seg0_F2R2 (SEQ ID NO: 275) amplicon and primers AW919147_DB71_seg0_F2 (SEQ ID NO: 273) and AW919147_DB71_seg0_R2 (SEQ ID NO: 274) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled AW919147_DB71_seg0 in Table 22 contains the normalized expression values of the above-indicated AW919147 transcript in treated or untreated kidney samples.

As is evident from the column entitled AW919147_DB71_seg0 in Table 22, the level of expression of the AW919147 transcript detectable by the above amplicon was lower in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 5: 0.01.

Primer pairs may also be used in the context of the present invention; for example, for the above experiment, the following primer pair was used as a non-limiting illustrative example only of a suitable primer pair: AW919147_DB71_seg0_F2 (SEQ ID NO: 273) forward primer; and AW919147_DB71_seg0_R2 (SEQ ID NO: 274) reverse primer.

Amplicons obtained through the use of any suitable primer pair; for example, for the above experiment, may also be used in the context of the present invention; the following amplicon was obtained as a non-limiting illustrative example only of a suitable amplicon AW919147_DB71_seg0_F2R2 (SEQ ID NO: 275):

Forward primer (AW919147_DB71_seg0_F2 (SEQ ID NO: 273)): GCCACAGTAGAGGCTGCACTT

Reverse primer (AW919147_DB71_seg0_R2 (SEQ ID NO: 274)): GAATCAACTGGAAATGTTCTAGGG

Amplicon (AW919147_DB71_seg0_F2R2 (SEQ ID NO: 275)):

GCCACAGTAGAGGCTGCACTTGCCACTGCTGTGTAGCGGCACTCTCCTGACTCACTTA AAAATTCTCTGGAGGTTTAAGTGAGGACCCAGTTCCATCTCTAGATATCCAGGCTCCCTAGA ACATTTCCAGTTGATTC

Example 2.10: Expression of growth differentiation factor 15 (GDF15), BI293562, transcripts which are detectable by amplicon as depicted in sequence name BI293562_DB71_seg2 in kidney tissues of treated or untreated rats

Expression of growth differentiation factor 15 (GDF15) transcripts detectable by or according to seg2 - BI293562_DB71_seg2_F2R2 (SEQ ID NO: 278) amplicon and primers BI293562_DB71_seg2_F2 (SEQ ID NO: 276) and BI293562_DB71_seg2_R2 (SEQ ID NO: 277) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled BI293562_DB71_seg2 in Table 22 contains the normalized expression values of the above-indicated growth differentiation factor 15 (GDF15) transcript in treated or untreated kidney samples.

As is evident from the column entitled BI293562_DB71_seg2 in Table 22, the level of expression of the growth differentiation factor 15 (GDF15) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 1: 0.03 and P-value for day 5: 7.50E$^{-07}$.

Primer pairs may also be used in the context ofthe present invention; for example, for the above experiment, the following primer pair was used as a non-limiting illustrative example only of a suitable primer pair: BI293562_DB71_seg2_F2 (SEQ ID NO: 276) forward primer; and BI293562_DB71_seg2_R2 (SEQ ID NO: 277) reverse primer.

Amplicons obtained through the use of any suitable primer pair; for example, for the above experiment, may also be used in the context of the present invention; the following amplicon was obtained as a non-limiting illustrative example only of a suitable amplicon BI293562_DB71_seg2_F2R2 (SEQ ID NO: 278):

Forward primer (BI293562_DB71_seg2_F2 (SEQ ID NO: 276)): CTGCAACTGAGCATGTGCGT

Reverse primer (BI293562_DB71_seg2_R2 (SEQ ID NO: 277)): TGCATAAGAACCACCGGGG

Amplicon (BI293562_DB71_seg2_F2R2 (SEQ ID NO: 278)):

CTGCAACTGAGCATGTGCGTGGGCGAGTGCCCCCACCTCTACCGGTCGGCCAACACGC
ATGCGCAGATCAAAGCACGCCTGCATGGCCTGCAGCCCGACAGAGTGCCGGCCCCGTGCTG
TGTCCCCTCCAGCTACACCCCGGTGGTTCTTATGCA

<u>Example 2.11:</u> Expression H31045 transcripts which are detectable by amplicon as depicted in sequence name H31045_DB71_seg5 in kidney tissues of treated or untreated rats

Expression of H31045 transcripts detectable by or according to seg5 - H31045_DB71_seg5_F3R3 (SEQ ID NO: 281) amplicon and primers H31045_DB71_seg5_F3 (SEQ ID NO: 279) and H31045_DB71_seg5_R3 (SEQ ID NO: 280) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled H31045_DB71_seg5 in Table 22 contains the normalized expression values of the above-indicated H31045_transcript in treated or untreated kidney samples.

As is evident from the column entitled H31045_DB71_seg5 in Table 22, the level of expression of the H31045 transcript detectable by the above amplicon was significantly lower in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 1: 0.015.

Primer pairs may also be used in the context of the present invention; for example, for the above experiment, the following primer pair was used as a non-limiting illustrative example only of a suitable primer pair: H31045_DB71_seg5_F3 (SEQ ID NO: 279) forward primer; and H31045_DB71_seg5_R3 (SEQ ID NO: 280) reverse primer.

Amplicons obtained through the use of any suitable primer pair; for example, for the above experiment, may also be used in the context of the present invention; the following amplicon was obtained as a non-limiting illustrative example only of a suitable amplicon H31045_DB71_seg5_F3R3 (SEQ ID NO: 281):

Forward primer (H31045_DB71_seg5_F3 (SEQ ID NO: 279)): GGACGGCCGAGCACAA

Reverse primer (H31045_DB71_seg5_R3 (SEQ ID NO: 280)): GTGTGCATGTGCATAGGTCAGA

Amplicon (H31045_DB71_seg5_F3R3 (SEQ ID NO: 281)):

GGACGGCCGAGCACAATGGTGCTTGCTGTACAGCCTCATAGACCTTCCTTGGATCTCC CAGACACACTTAGTGGAAGGAGAGAATCCATTCTTGCAAATTGTCTGACCTATGCACATGCA CAC

Example 2.12: Expression Etoposide induced 2.4 mRNA (EI24) H31799 transcripts which are detectable by amplicon as depicted in sequence name H31799_DB71_seg23 in kidney tissues of treated or untreated rats

Expression of Etoposide induced 2.4 mRNA (EI24) transcripts detectable by or according to seg23 - H31799_DB71_seg23_F1R1 (SEQ ID NO: 284) amplicon and primers H31799_DB71_seg23_F1 (SEQ ID NO: 282) and H31799_DB71_seg23_R1 (SEQ ID NO: 283) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled H31799_DB71_seg23 in Table 22 contains the normalized expression values of the above-indicated Etoposide induced 2.4 mRNA (EI24) transcript in treated or untreated kidney samples.

As is evident from the column entitled H31799_DB71_seg23 in Table 22, the level of expression of the Etoposide induced 2.4 mRNA (EI24) transcript detectable by the above amplicon was higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 5: 0.01.

Primer pairs may also be used in the context of the present invention; for example, for the above experiment, the following primer pair was used as a non-limiting illustrative example only of a suitable primer pair: H31799_DB71_seg23_F1 (SEQ ID NO: 282) forward primer; and H31799_DB71_seg23_R1 (SEQ ID NO: 283) reverse primer.

Amplicons obtained through the use of any suitable primer pair; for example, for the above experiment, may also be used in the context of the present invention; the following amplicon was obtained as a non-limiting illustrative example only of a suitable amplicon H31799_DB71_seg23_F1R1 (SEQ ID NO: 284):

Forward primer (H31799_DB71_seg23_F1 (SEQ ID NO: 282)): TACTGTTTCCTGTGAAGCACATACCT

Reverse primer (H31799_DB71_seg23_R1 (SEQ ID NO: 283)): GCAGAGCAGTAAAGACCAAAACC

Amplicon (H31799_DB71_seg23_F1R1 (SEQ ID NO: 284)):

TACTGTTTCCTGTGAAGCACATACCTTGTATGTGGGAGGTAAAGGAGCACGCCAGCTG CTCCATGTCACTCCCTCTATAGCCATCACTGTCTTGTTTTTTGTAACTCAGGTTAGGTTTTGG TCTTTACTGCTCTGC

Example 2.13: Expression Etoposide induced 2.4 (EI24) mRNA, H31799, transcripts which are detectable by amplicon as depicted in sequence name W83813_DB81_seg27 in kidney tissues of treated or untreated rats

Expression of Etoposide induced 2.4 mRNA (EI24) transcripts detectable by or according to seg27 - W83813_DB81_seg27_F1R1 (SEQ ID NO: 287) amplicon and primers W83813_DB81_seg27_F1 (SEQ ID NO: 285) and W83813_DB81_seg27_R1 (SEQ ID NO: 286) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled W83813_DB81_seg27 in Table 22 contains the normalized expression values of the above-indicated Etoposide induced 2.4 mRNA (EI24) transcript in treated or untreated kidney samples.

As is evident from the column entitled W83813_DB81_seg27 in Table 22, the level of expression of the Etoposide induced 2.4 mRNA (EI24) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 5: 1.50E-08.

Primer pairs may also be used in the context of the present invention; for example, for the above experiment, the following primer pair was used as a non-limiting illustrative example only of a suitable primer pair: W83813_DB81_seg27_F1 (SEQ ID NO: 285) forward primer; and W83813_DB81_seg27_R1 (SEQ ID NO: 286) reverse primer.

Amplicons obtained through the use of any suitable primer pair; for example, for the above experiment, may also be used in the context of the present invention; the following amplicon was obtained as a non-limiting illustrative example only of a suitable amplicon W83813_DB81_seg27_F1R1 (SEQ ID NO: 287):

Forward primer (W83813_DB81_seg27_F1 (SEQ ID NO: 285)): AATCTAGGCTGCCTCCTGGAG

Reverse primer (W83813_DB81_seg27_R1 (SEQ ID NO: 286)): AGGTCAATTATACAAGGCATGACTTTAG

Amplicon (W83813_DB81_seg27_F1R1 (SEQ ID NO: 287)):

AATCTAGGCTGCCTCCTGGAGGAAGATACTTAGGAGTTCAGAAGTGAAGAGATGAGG
CTTATAATACTTTTTCCTAAAGTCATGCCTTGTATAATTGACCT

Example 2.14: Expression of Cyclin-G1 (CCNG1), H31883, transcripts which are detectable by amplicon as depicted in sequence name H31883_DB71_seg13 in kidney tissues of treated or untreated rats

Expression of Cyclin-G1 (CCNG1) transcripts detectable by or according to seg13 - H31883_DB71_seg13_F1R1 (SEQ ID NO: 290) amplicon and primers H31883_DB71_seg13_F1 (SEQ ID NO: 288) and H31883_DB71_seg13_R1 (SEQ ID NO: 289) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled H31883_DB71_seg13 in Table 22 contains the normalized expression values of the above-indicated Cyclin-G1 (CCNG1) transcript in treated or untreated kidney samples.

As is evident from the column entitled H31883_DB71_seg13 in Table 22, the level of expression of the Cyclin-G1 (CCNG1) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 1: 5.00E-03 and P-value for day 5: 0.04.

Primer pairs may also be used in the context of the present invention; for example, for the above experiment, the following primer pair was used as a non-limiting illustrative example only of a suitable primer pair: H31883_DB71_seg13_F1 (SEQ ID NO: 288) forward primer; and H31883_DB71_seg13_R1 (SEQ ID NO: 289) reverse primer.

Amplicons obtained through the use of any suitable primer pair; for example, for the above experiment, may also be used in the context of the present invention; the following amplicon was obtained as a non-limiting illustrative example only of a suitable amplicon: H31883_DB71_seg13_F1R1 (SEQ ID NO: 290).

Forward primer (H31883_DB71_seg13_F1 (SEQ ID NO: 288)): TCGTCAGAATTGCTGCCTCA

Reverse primer (H31883_DB71_seg13_R1 (SEQ ID NO: 289)): TCTGTGGTAAAAACCTCCTGGAGT

Amplicon (H31883_DB71_seg13_FR1 (SEQ ID NO: 290)):

TCGTCAGAATTGCTGCCTCAATCTAGTCCCATTTGAGAAAATTTGTTTCTACTGTCTCA
ATAACTGGATGAAATATCACTCTGAAAACTTGCCTATTGCACTAAAGCTAGTTTAGGCTTGA
TAAAACACTCCAGGAGGTTTTTACCACAGA

Example 2.15: Expression of Cyclin-G1 (CCNG1), H31883, transcripts which are detectable by amplicon as depicted in sequence name MUSCYCG1R_DB81_seg15-17 in kidney tissues of treated or untreated rats

Expression of Cyclin-G1 (CCNG1) detectable by or according to seg15-17 - MUSCYCG1R_DB81_seg15-17_F1R1 (SEQ ID NO: 293) amplicon and primers MUSCYCG1R_DB81_seg15-17_F1 (SEQ ID NO: 291) and MUSCYCG1R_DB81_seg15-17_R1 (SEQ ID NO: 292) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled MUSCYCG1R_DB81_seg15-17 in Table 22 contains the normalized expression values of the above-indicated Cyclin-G1 (CCNG1) transcript in treated or untreated kidney samples.

As is evident from the column entitled MUSCYCG1R_DB81_seg15-17 in Table 22, the level of expression of the Cyclin-G1 (CCNG1) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 5: $1.00E^{-07}$.

Primer pairs may also be used in the context of the present invention; for example, for the above experiment, the following primer pair was used as a non-limiting illustrative example only of a suitable primer pair: MUSCYCG1R_DB81_seg15-17_F1 (SEQ ID NO: 291) forward primer; and MUSCYCG1R_DB81_seg15-17_R1 (SEQ ID NO: 292) reverse primer.

Amplicons obtained through the use of any suitable primer pair; for example, for the above experiment, may also be used in the context of the present invention; the following amplicon was obtained as a non-limiting illustrative example only of a suitable amplicon: MUSCYCG1R_DB81_seg15-17_F1R1 (SEQ ID NO: 293).

Forward primer (MUSCYCG1R_DB81_seg15-17_F1 (SEQ ID NO: 291)): ATAAAATCAATCCCACTTTCTTGTTAAAAG

Reverse primer (MUSCYCG1R_DB81_seg15-17_R1 (SEQ ID NO: 292)): CTTTGCCTTAGAAAATATGATCCTGC

Amplicon (MUSCYCG1R_DB81_seg15-17_F1R1 (SEQ ID NO: 293)):

ATAAAATCAATCCCACTTTCTTGTTAAAAGGAGAAACGATCTGAATTTTGAAAGACTA
GAAGCCCAACTTAAGGCCTGCCACTGCAGGATCATATTTTCTAAGGCAAAG

Example 2.16: Expression of Cyclin-G1 (CCNG1), H31883, transcripts which are detectable by amplicon as depicted in sequence name MUSCYCG1R_DB81_seg19-20 in kidney tissues of treated or untreated rats

Expression of Cyclin-G1 (CCNG1) transcripts detectable by or according to seg 19-20 - MUSCYCG1R_DB81_seg19-20_F1R1 (SEQ ID NO: 296) amplicon and primers MUSCYCG1R_DB81_seg19-20_F1 (SEQ ID NO: 294) and MUSCYCG1R_DB81_seg19-20_R1 (SEQ ID NO: 295) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled MUSCYCG1R_DB81_seg19-20 in Table 22 contains the normalized expression values of the above-indicated Cyclin-G1 (CCNG1) transcript in treated or untreated kidney samples.

As is evident from the column entitled MUSCYCG1R_DB81_seg19-20 in Table 22, the level of expression of the Cyclin-G1 (CCNG1) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 1: $6.00E^{-03}$ and P-value for day 5: $3.00E^{-09}$.

Primer pairs may also be used in the context ofthe present invention; for example, for the above experiment, the following primer pair was used as a non-limiting illustrative example only of a suitable primer pair: MUSCYCG1R_DB81_seg19-20_F1 (SEQ ID NO: 294) forward primer; and MUSCYCG1R_DB81_seg19-20_R1 (SEQ ID NO: 295) reverse primer.

Amplicons obtained through the use of any suitable primer pair; for example, for the above experiment, may also be used in the context of the present invention; the following amplicon was obtained as a non-limiting illustrative example only of a suitable amplicon: MUSCYCG1R_DB81_seg19-20_F1R1 (SEQ ID NO: 296).

Forward primer (MUSCYCG1R_DB81_seg19-20_F1 (SEQ ID NO: 294)): GAGATCCAAGCACTGAAGTATGTAGAGT

Reverse primer (MUSCYCG1R_DB81_seg19-20_R1 (SEQ ID NO: 295)): TCAGGAGTACAGTGGATACATTTCTCTT

Amplicon (MUSCYCG1R_DB81_seg19-20_F1R1 (SEQ ID NO: 296)):

GAGATCCAAGCACTGAAGTATGTAGAGTTAACAGAAGGAGTAGAATGTATTCAGAAA CATTCCAAGGTATGCCAAGGTGATAGCATTGATCCTATTAGCAAGCTACAAGAGAAATGTAT CCACTGTACTCCTGA

Example 2.17: Expression of repeat domain 77 (WDR77), H33998, transcripts which are detectable by amplicon as depicted in sequence name W33294_DB81_seg23 in kidney tissues of treated or untreated rats

Expression of repeat domain 77 (WDR77) transcripts detectable by or according to seg23 - W33294_DB81_seg23_F1R1 (SEQ ID NO: 299) amplicon and primers W33294_DB81_seg23_F1 (SEQ ID NO: 297) and W33294_DB81_seg23_R1 (SEQ ID NO: 298) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled W33294_DB81_seg23 in Table 22 contains the normalized expression values of the above-indicated repeat domain 77 (WDR77) transcript in treated or untreated kidney samples.

As is evident from the column entitled W33294_DB81_seg23 in Table 22, the level of expression of the repeat domain 77 (WDR77) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 5: $7.00E^{-04}$.

Primer pairs may also be used in the context ofthe present invention; for example, for the above experiment, the following primer pair was used as a non-limiting illustrative example only of a suitable primer pair: W33294_DB81_seg23_F1 (SEQ ID NO: 297) forward primer; and W33294_DB81_seg23_R1 (SEQ ID NO: 298) reverse primer.

Amplicons obtained through the use of any suitable primer pair; for example, for the above experiment, may also be used in the context of the present invention; the following amplicon was obtained as a non-limiting illustrative example only of a suitable amplicon: W33294_DB81_seg23_F1R1 (SEQ ID NO: 299).

Forward primer (W33294_DB81_seg23_F1 (SEQ ID NO: 297)): GGGCACTGCATGGATTATGTC

Reverse primer (W33294_DB81_seg23_R1 (SEQ ID NO: 298)): CCATGCCCAGAATAAAGGAGC

Amplicon (W33294_DB81_seg23_F1R1 (SEQ ID NO: 299)):

GGGCACTGCATGGATTATGTCGGTTTTACCTAGCTTTGGAGACCATCATTTTTTCCTAA TAGGTTTGCTTCATTGTTTCAGCTCCTTTATTCTGGGCATGG

Example 2.18: Expression of repeat domain 77 (WDR77), H33998, transcripts which are detectable by amplicon as depicted in sequence name W33294_DB81_seg44 in kidney tissues of treated or untreated rats

Expression of repeat domain 77 (WDR77) transcripts detectable by or according to seg44 - W33294_DB81_seg44_F1R1 (SEQ ID NO: 302) amplicon and primers W33294_DB81_seg44_F1 (SEQ ID NO: 300) and W33294_DB81_seg44_R1 (SEQ ID NO: 301) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled W33294_DB81_seg44 in Table 22 contains the normalized expression values of the above-indicated repeat domain 77 (WDR77) transcript in treated or untreated kidney samples.

As is evident from the column entitled W33294_DB81_seg44 in Table 22, the level of expression of the repeat domain 77 (WDR77) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 5: 8.00E-06.

Primer pairs may also be used in the context ofthe present invention; for example, for the above experiment, the following primer pair was used as a non-limiting illustrative example only of a suitable primer pair: W33294_DB81_seg44_F1 (SEQ ID NO: 300) forward primer; and W33294_DB81_seg44_R1 (SEQ ID NO: 301) reverse primer.

Amplicons obtained through the use of any suitable primer pair; for example, for the above experiment, may also be used in the context of the present invention; the following amplicon was obtained as a non-limiting illustrative example only of a suitable amplicon: W33294_DB81_seg44_F1R1 (SEQ ID NO: 302).

Forward primer (W33294_DB81_seg44_F1 (SEQ ID NO: 300)): CTCCGTTAGCAATTATGGGTCAGT

Reverse primer (W33294_DB81_seg44_R1 (SEQ ID NO: 301)): GAAGCTCATGGTCCTTCAGGG

Amplicon (W33294_DB81_seg44_F1R1 (SEQ ID NO: 302)):

CTCCGTTAGCAATTATGGGTCAGTAACTGATCTTATTAGAGCTTTACAACTTTGGTTTA
GGAAAAGCACATAAAATGGGCGCCCTGAAGGACCATGAGCTTC

Example 2.19: Expression of repeat domain 77 (WDR77), H33998, transcripts which are detectable by amplicon as depicted in sequence name H33998_DB71_seg19 in kidney tissues of treated or untreated rats

Expression of repeat domain 77 (WDR77) transcripts detectable by or according to seg19 - H33998_DB71_seg19_F3R3 (SEQ ID NO: 305) amplicon and primers H33998_DB71_seg19_F3 (SEQ ID NO: 303) and H33998_DB71_seg19_R3 (SEQ ID NO: 304) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled H33998_DB71_seg19 in Table 22 contains the normalized expression values of the above-indicated repeat domain 77 (WDR77) transcript in treated or untreated kidney samples.

As is evident from the column entitled H33998_DB71_seg19 in Table 22, the level of expression of the repeat domain 77 (WDR77) transcript detectable by the above amplicon was significantly higher in the samples treated with toxic compounds (samples 1-58 and 76-105, in kidney tissue panel described in Table 9 hereinabove) than in the control samples (naï0ve, saline and valproic acid treated samples; samples numbers- 59-75 and 106-129, in kidney tissue panel described in Table 9 hereinabove). Statistical analysis was applied to verify the significance of these results, P-value for day 1: 0.04 and P-value fro day 5: 2.00E-07.

Primer pairs may also be used in the context of the present invention; for example, for the above experiment, the following primer pair was used as a non-limiting illustrative example only of a suitable primer pair: H33998_DB71_seg19_F3 (SEQ ID NO: 303) forward primer; and H33998_DB71_seg19_R3 (SEQ ID NO: 304) reverse primer.

Amplicons obtained through the use of any suitable primer pair; for example, for the above experiment, may also be used in the context of the present invention; the following amplicon was obtained as a non-limiting illustrative example only of a suitable amplicon: H33998_DB71_seg19_F3R3 (SEQ ID NO: 305).

Forward primer (H33998_DB71_seg19_F3R3 (SEQ ID NO: 303)): CTGTCATTGTCATTTTTCCCACC

Reverse primer (H33998_DB71_seg19_F3R3 (SEQ ID NO: 304)): ATGCCGGATCTTCAATCTTAGG

Amplicon (H33998_DB71_seg19_F3R3 (SEQ ID NO: 305)):

CTGTCATTGTCATTTTTCCCACCTAAAAATTCCCTGAGGACTGATCTGGGTACTTTGCT
CTGGAGAGCTGAAGTCTGAGCGCTGTATATTTGGACTCCTAAGATTGAAGATCCGGCAT

Example 2.20: Expression of Activating transcription factor 3 (ATF3), RATLRFI, transcripts which are detectable by amplicon as depicted in sequence name RATLRFI_DB71_seg9 in kidney tissues of treated or untreated rats

Expression of Activating transcription factor 3 (ATF3) transcripts detectable by or according to seg9 - RATLRFI_DB71_seg9_F1R1 (SEQ ID NO: 308) amplicon and primers RATLRFI_DB71_seg9_F1 (SEQ ID NO: 306) and RATLRFI_DB71_seg9_R1 (SEQ ID NO: 307) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled RATLRFI_DB71_seg9 in Table 22 contains the normalized expression values of the above-indicated Activating transcription factor 3 (ATF3) transcript in treated or untreated kidney samples.

As is evident from the column entitled RATLRFI_DB71_seg9 in Table 22, the level of expression of the Activating transcription factor 3 (ATF3) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 1: 0.025 and P-value fro day 5: $5.00E^{-04}$.

Primer pairs may also be used in the context ofthe present invention; for example, for the above experiment, the following primer pair was used as a non-limiting illustrative example only of a suitable primer pair: RATLRFI_DB71_seg9_F1 (SEQ ID NO: 306) forward primer; and RATLRFI_DB71_seg9_R1 (SEQ ID NO: 307) reverse primer.

Amplicons obtained through the use of any suitable primer pair; for example, for the above experiment, may also be used in the context of the present invention; the following amplicon was obtained as a non-limiting illustrative example only of a suitable amplicon: RATLRFI_DB71_seg9_F1R1 (SEQ ID NO: 308).

Forward primer (RATLRFI_DB71_seg9_F1 (SEQ ID NO: 306)): AACTGGCTGATGACCAGCTGT

Reverse primer (RATLRFI_DB71_seg9_R1 (SEQ ID NO: 307)): TGCTGTGCCCGGGTTCT

Amplicon (RATLRFI_DB71_seg9_F1R1 (SEQ ID NO: 308)):

AACTGGCTGATGACCAGCTGTGCTACTCTGTGCTGACCGAGGACTGATGCCTCCTTCC
CCTGTACCCACTGCTGAGGAAGAACCCGGGCACAGCA

Example 2.21: Expression of Ras-related protein Rab-13 (RAB13), RATRAB13X, transcripts which are detectable by amplicon as depicted in sequence name RATRAB13X_DB81_seg15-17 in kidney tissues of treated or untreated rats

Expression of Ras-related protein Rab-13 (RAB13) transcripts detectable by or according to seg15-17 - RATRAB13X_DB81_seg15-17_F1R1 (SEQ ID NO: 311) amplicon and primers RATRAB13X_DB81_seg15-17_F1 (SEQ ID NO: 309) and RATRAB13X_DB81_seg15-17_R1 (SEQ ID NO: 310) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled RATRAB13X_DB81_seg15-17 in Table 22 contains the normalized expression values of the above-indicated Ras-related protein Rab-13 (RAB13) transcript in treated or untreated kidney samples.

As is evident from the column entitled RATRAB13X_DB81_seg15-17 in Table 22, the level of expression of the Ras-related protein Rab-13 (RAB13) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 1: 0.05 and P-value for day 5: $3.00E^{-07}$.

Primer pairs may also be used in the context of the present invention; for example, for the above experiment, the following primer pair was used as a non-limiting illustrative example only of a suitable primer pair: RATRAB13X_DB81_seg15-17_F1 (SEQ ID NO: 309) forward primer; and RATRAB13X_DB81_seg15-17_R1 (SEQ ID NO: 310) reverse primer.

Amplicons obtained through the use of any suitable primer pair; for example, for the above experiment, may also be used in the context of the present invention; the following amplicon was obtained as a non-limiting illustrative example only of a suitable amplicon: RATRAB13X_DB81_seg15-17_F1R1 (SEQ ID NO: 311).

Forward primer (RATRAB13X_DB81_seg15-17_F1 (SEQ ID NO: 309)): GTGAGTCAGGCCGGGCT

Reverse primer (RATRAB13X_DB81_seg15-17_R1 (SEQ ID NO: 310)): TCAAAAAATCGGATTCTGTGCTC

Amplicon (RATRAB13X_DB81_seg15-17_F1R1 (SEQ ID NO: 311)):

GTGAGTCAGGCCGGGCTGCTGTGGCCGAATGCCTTGCTTCTGCCCTTTATCCAGCTCTC TCCTTCCTACCTCATCCCCTACAGTTGGCTCGAGAGCACAGAATCCGATTTTTTGA

Example 2.22: Expression of Ras-related protein Rab-13 (RAB13), RATRAB13X, transcripts which are detectable by amplicon as depicted in sequence name RATRAB13X_DB81_seg22 in kidney tissues of treated or untreated rats

Expression of Ras-related protein Rab-13 (RAB13) transcripts detectable by or according to seg22 - RATRAB13X_DB81_seg22_F2R2 (SEQ ID NO: 314) amplicon and primers RATRAB13X_DB81_seg22_F2 (SEQ ID NO: 312) and RATRAB13X_DB81_seg22_R2 (SEQ ID NO: 313) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled RATRAB13X_DB81_seg22 in Table 22 contains the normalized expression values of the above-indicated Ras-related protein Rab-13 (RAB13) transcript in treated or untreated kidney samples.

As is evident from the column entitled RATRAB13X_DB81_seg22 in Table 22, the level of expression of the Ras-related protein Rab-13 (RAB13) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 1: 0.04 and P-value for day 5: $3.00E^{-07}$.

Primer pairs may also be used in the context of the present invention; for example, for the above experiment, the following primer pair was used as a non-limiting illustrative example only of a suitable primer pair: RATRAB13X_DB81_seg22_F2 (SEQ ID NO: 312) forward primer; and RATRAB 13X_DB81_seg22_R2 (SEQ ID NO: 313) reverse primer.

Amplicons obtained through the use of any suitable primer pair; for example, for the above experiment, may also be used in the context of the present invention; the following amplicon was obtained as a non-limiting illustrative example only of a suitable amplicon: RATRAB13X_DB81_seg22_F2R2 (SEQ ID NO: 314).

Forward primer (RATRAB 13X_DB81_seg22_F2 (SEQ ID NO: 312)): TTCTCCTGAATCTGGCTGGGT

Reverse primer (RATRAB 13X_DB81_seg22_R2 (SEQ ID NO: 313)): TGGAAAAAGGATGTCCATTCTTC

Amplicon (RATRAB13X_DB81_seg22_F2R2 (SEQ ID NO: 314)):

TTCTCCTGAATCTGGCTGGGTCCCCCTTCCTTACCCCAACTCTTTAACTGGTGATGAAA ACAGCAAGGAGAAAGGGCAGCCTGAAGAATGGACATCCTTTTTCCA

Example 2.23: Expression of Ras-related protein Rab-13 (RAB13), RATRAB13X, transcripts which are detectable by amplicon as depicted in sequence name RATRAB13X_DB71_seg11-13 in kidney tissues of treated or untreated rats

Expression of Ras-related protein Rab-13 (RAB13) transcripts detectable by or according to seg11-13 - RATRAB13X_DB71_seg11-13_F1R1 (SEQ ID NO: 317) amplicon and primers RATRAB13X_DB71_seg11-13_F1 (SEQ ID NO: 315) and RATRAB13X_DB71_seg11-13_R1 (SEQ ID NO: 316) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled RATRAB13X_DB71_seg11-13 in Table 22 contains the normalized expression values of the above-indicated Ras-related protein Rab-13 (RAB13) transcript in treated or untreated kidney samples.

As is evident from the column entitled RATRAB13X_DB71_seg11-13 in Table 22, the level of expression of the Ras-related protein Rab-13 (RAB13) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 1: 1.00E$^{-03}$.

Primer pairs may also be used in the context ofthe present invention; for example, for the above experiment, the following primer pair was used as a non-limiting illustrative example only of a suitable primer pair: RATRAB13X_DB71_seg11-13_F1 (SEQ ID NO: 315) forward primer; and RATRAB13X_DB71_seg11-13_R1 (SEQ ID NO: 316) reverse primer.

Amplicons obtained through the use of any suitable primer pair; for example, for the above experiment, may also be used in the context of the present invention; the following amplicon was obtained as a non-limiting illustrative example only of a suitable amplicon: RATRAB13X_DB71_seg11-13_F1R1 (SEQ ID NO: 317).

Forward primer (RATRAB13X_DB71_seg11-13_F1 (SEQ ID NO: 315)): AGAATCCGATTTTTTGAGACAAGTG

Reverse primer (RATRAB13X_DB71_seg11-13_R1 (SEQ ID NO: 316)): GATCTCCGGCCTCCTGTCTT

Amplicon (RATRAB13X_DB71_seg11-13_F1R1 (SEQ ID NO: 317)):

AGAATCCGATTTTTTGAGACAAGTGCCAAATCCAGTGTGAATGTGGATGAGGCTTTCA
GTTCCCTGGCCCGTGACATCTTGCTCAAGACAGGAGGCCGGAGATC

Example 2.24: Expression of Cyclin-dependent kinase inhibitor 1A (CDKN1A), RNU24174, transcripts which are detectable by amplicon as depicted in sequence name MMU09507_DB81_seg15 in kidney tissues of treated or untreated rats

Expression of Cyclin-dependent kinase inhibitor 1A (CDKN1A) transcripts detectable by or according to seg15 - MMU09507_DB81_seg15_F1R1 (SEQ ID NO: 323) amplicon and primers MMU09507_DB81_seg15_F1 (SEQ ID NO: 321) and MMU09507_DB81_seg15_R1 (SEQ ID NO: 322) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled MMU09507_DB81_seg15 in Table 22 contains the normalized expression values of the above-indicated Cyclin-dependent kinase inhibitor 1A (CDKN1A) transcript in treated or untreated kidney samples.

As is evident from the column entitled MMU09507_DB81_seg15 in Table 22, the level of expression of the Cyclin-dependent kinase inhibitor 1A (CDKN1A) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 5: 3.00E$^{-03}$.

Primer pairs may also be used in the context ofthe present invention; for example, for the above experiment, the following primer pair was used as a non-limiting illustrative example only of a suitable primer pair: MMU09507_DB81_seg15_F1 (SEQ ID NO: 321) forward primer; and MMU09507_DB81_seg15_R1 (SEQ ID NO: 322) reverse primer.

Amplicons obtained through the use of any suitable primer pair; for example, for the above experiment, may also be used in the context of the present invention; the following amplicon was obtained as a non-limiting illustrative example only of a suitable amplicon: MMU09507_DB81_seg15_F1R1 (SEQ ID NO: 323).

Forward primer (MMU09507_DB81_seg15_F1 (SEQ ID NO: 321)): GGGTGGGGGAATCTAGCCT

Reverse primer (MMU09507_DB81_seg15_R1 (SEQ ID NO: 322)): CCCCAGACAAAGGGACATGT

Amplicon (MMU09507_DB81_seg15_F1R1 (SEQ ID NO: 323)):

GGGTGGGGGAATCTAGCCTCTCTAGAGCCCTAGCCCTCTGACGAGGAGGAGGTGTAG
TGCCCTGTAGCTTTTCCCCAGGACTCGCCACATGTCCCTTTGTCTGGGG

Example 2.25: Expression of Cyclin-dependent kinase inhibitor 1A (CDKN1A), RNU24174, transcripts which are detectable by amplicon as depicted in sequence name RNU24174_DB71_seg8 in kidney tissues of treated or untreated rats

Expression of Cyclin-dependent kinase inhibitor 1A (CDKN1A) transcripts detectable by or according to seg8 - RNU24174_DB71_seg8_F1R1 (SEQ ID NO: 326) amplicon and primers RNU24174_DB71_seg8_F1 (SEQ ID NO: 324) and RNU24174_DB71_seg8_R1 (SEQ ID NO: 325) was measured by real time PCR. The value of the expression was measured by Real-Time PCR and normalized relative to the expression of the house keeping genes, as described in section "RT PREPARATION and Real-TIME RT-PCR ANALYSIS" hereinabove.

The column entitled RNU24174_DB71_seg8 in Table 22 contains the normalized expression values of the above-indicated Cyclin-dependent kinase inhibitor 1A (CDKN1A) transcript in treated or untreated kidney samples.

As is evident from the column entitled RNU24174_DB71_seg8 in Table 22, the level of expression of the Cyclin-dependent kinase inhibitor 1A (CDKN1A) transcript detectable by the above amplicon was significantly higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 1: $2.00E^{-05}$ and P-value for day 5: $3.00E^{-05}$.

Primer pairs may also be used in the context ofthe present invention; for example, for the above experiment, the following primer pair was used as a non-limiting illustrative example only of a suitable primer pair: RNU24174_DB71_seg8_F1 (SEQ ID NO: 324) forward primer; and RNU24174_DB71_seg8_R1 (SEQ ID NO: 325) reverse primer.

Amplicons obtained through the use of any suitable primer pair; for example, for the above experiment, may also be used in the context of the present invention; the following amplicon was obtained as a non-limiting illustrative example only of a suitable amplicon: RNU24174_DB71_seg8_F1R1 (SEQ ID NO: 326).

Forward primer (RNU24174_DB71_seg8_F1 (SEQ ID NO: 324)): CGCTGAAGTCCTCAGTGACTTG

Reverse primer (RNU24174_DB71_seg8_R1 (SEQ ID NO: 325)): CACTAAGTGCTTCGACACCCAC

Amplicon (RNU24174_DB71_seg8_F1R1 (SEQ ID NO: 326)):

CGCTGAAGTCCTCAGTGACTTGTCCCATTTCTTAGTAGTTGTACAAGGAGTCAGGCCA
AGATGGTGCCTCGGGGGCTGAGGGAGCTCACAGGAACTGAGCAGTGACTGGTCCTTTCCCA
GTATTGAATACTGAGCCCCTGTGGGTGTCGAAGCACTTAGTG

Table 22 presents the normalized Real Time PCR results for all 25 amplicons detailed in examples 2.1-2.25 for all samples checked. The qRT-PCR measurements were normalized according to each samples normalization factor as described in "RT Preparation and Real-Time qRT-PCR Analysis" and further multiplied by a constant factor for ease of viewing.

Table 22: normalized Real Time PCR

| Sample | AA686189_ DB71_seg6 | AA799594_ DB71_seg0 | AA964541_ DB71_seg0 | AI045075_ DB71_seg6 |
|---|---|---|---|---|
| 68_1F101_day1_Saline | 0.44 | 1.17 | 0.25 | 0.51 |
| 69_1F102_day1_Saline | 0.38 | 1.16 | 0.28 | 0.44 |
| 70_1F103_day1_Saline | 0.64 | 0.92 | 0.77 | 0.54 |
| 74_1F104_day5_Saline | 0.63 | 1.69 | 0.41 | 0.42 |
| 75_1F105_day5_Saline | 0.54 | 1.56 | 0.42 | 0.59 |
| 109_1F107_day28_Saline | 0.78 | 1.6 | 0.49 | 0.79 |
| 110_1F108_day28_Saline | 0.62 | 1.08 | 0.46 | 0.65 |
| 111_1F109_day28_Saline | 0.68 | 0.9 | 0.45 | 0.9 |
| 65_1M1_day1_Saline | 0.72 | 1.02 | 0.54 | 0.79 |
| 66_1M2_day1_Saline | 0.65 | 0.87 | 0.65 | 0.36 |
| 67_1M3_day1_Saline | 0.55 | 1.17 | 0.4 | 0.84 |
| 71_1M4_day5_Saline | 0.45 | 1.28 | 0.49 | 0.6 |
| 72_1M5_day5_Saline | 0.54 | 0.67 | 0.38 | 0.56 |
| 73_1M6_day5_Saline | 0.41 | 0.86 | 0.41 | 0.45 |
| 106_1M7_day28_Saline | 0.81 | 1.06 | 0.48 | 0.64 |
| 107_1M8_day28_Saline | 0.36 | 1.24 | 0.25 | 0.42 |
| 108_1M9_day28_Saline | 0.73 | 1.36 | 0.3 | 0.55 |
| 16_2F110_day1_Gent | 1.19 | 1.25 | 1.35 | 1.5 |
| 17_2F111_day1_Gent | 0.65 | 0.72 | 0.74 | 0.93 |
| 18_2F112_day1_Gent | 1.07 | 0.93 | 1.29 | 0 |
| 45_2F113_day5_Gent | 0.64 | 0.99 | 0.86 | 0.76 |
| 46_2F114_day5_Gent | 0.72 | 0.89 | 0.58 | 1.04 |
| 47_2F115_day5_Gent | 1.21 | 1.52 | 1.04 | 0.62 |
| 91_2F116_day28_Gent | 2.7 | 0.84 | 3.19 | 2.09 |
| 92_2F117_day28_Gent | 2.09 | 0.8 | 1.77 | 3.14 |
| 93_2F118_day28_Gent | 2.48 | 0.89 | 2.16 | 1.74 |
| 1_2M10_day1_Gent | 0.94 | 0.57 | 0.9 | 1.07 |
| 2_2M11_day1_Gent | 1.04 | 0.95 | 1.19 | 1.03 |
| 3_2M12_day1_Gent | 0.6 | 0.92 | 0.44 | 0.78 |
| 31_2M13_day5_Gent | 0.81 | 0.89 | 0.91 | 1.1 |
| 32_2M14_day5_Gent | 0.83 | 0.56 | 0.76 | 0 |
| 33_2M15_day5_Gent | 0.75 | 1.04 | 0.87 | 1.04 |
| 76_2M16_day28_Gent | 1.98 | 0.54 | 3.33 | 1.25 |
| 77_2M17_day28_Gent | 1.23 | 0.5 | 1.45 | 1.99 |
| 78_2M18_day28_Gent | 2.51 | 0.67 | 3.18 | 1.29 |
| 19_3F119_day1_Cis | 0.7 | 1.17 | 0.88 | 1.01 |

| Sample | AA686189_ DB71_seg6 | AA799594_ DB71_seg0 | AA964541_ DB71_seg0 | AI045075_ DB71_seg6 |
|---|---|---|---|---|
| 20_3F120_day1_Cis | 0.44 | 0.39 | 0.58 | 0.68 |
| 21_3F121_day1_Cis | 0.66 | 0.98 | 0.41 | 0.79 |
| 48_3F122_day5_Cis | 1.08 | 1.16 | 0.99 | 0.86 |
| 49_3F123_day5_Cis | 0.93 | 1.34 | 0.74 | 0.53 |
| 50_3F124_day5_Cis | 0.76 | 1.11 | 0.76 | 0.76 |
| 94_3F125_day28_Cis | 0 | 0.74 | 2.63 | 2.17 |
| 95_3F126_day28_Cis | 3.57 | 0.64 | 5.28 | 3.09 |
| 96_3F127_day28_Cis | 6.06 | 0.52 | 6.85 | 3.37 |
| 4_3M19_day1_Cis | 0.48 | 0.59 | 0.4 | 0.64 |
| 5_3M20_day1_Cis | 0.42 | 0.99 | 0.29 | 0.62 |
| 6_3M21_day1_Cis | 0.68 | 0.87 | 0.56 | 1.09 |
| 34_3M22_day5_Cis | 1.5 | 0.93 | 1.94 | 1.93 |
| 35_3M23_day5_Cis | 0.69 | 0.71 | 0.67 | 0.81 |
| 36_3M24_day5_Cis | 4.48 | 0.75 | 4.8 | 1.33 |
| 79_3M25_day28_Cis | 3.15 | 0.36 | 3.72 | 3.18 |
| 80_3M26_day28_Cis | 4.29 | 0.97 | 4.71 | 2.72 |
| 81_3M27_day28_Cis | 3.86 | 0.62 | 3.97 | 1.57 |
| 22_4F128_day1_Tob | 1.19 | 0.87 | 0.56 | 1.66 |
| 23_4F129_day1_Tob | 0.77 | 0.83 | 0.82 | 0.88 |
| 24_4F130_day1_Tob | 2.14 | 1.07 | 2.02 | 3.16 |
| 51_4F131_day5_Tob | 0.85 | 1.38 | 0.82 | 0.92 |
| 52_4F132_day5_Tob | 1.04 | 0.88 | 0.77 | 1.61 |
| 53_4F133_day5_Tob | 0.78 | 0.88 | 0.65 | 1.56 |
| 97_4F134_day28_Tob | 2.1 | 0.71 | 1.68 | 0.94 |
| 98_4F135_day28_Tob | 1.65 | 0.75 | 1.37 | 0.79 |
| 99_4F136_day28_Tob | 2.23 | 0.84 | 1.59 | 1.19 |
| 7_4M28_day1_Tob | 0.65 | 0.94 | 0.47 | 0.56 |
| 8_4M29_day1_Tob | 0.85 | 1 | 0.75 | 1.03 |
| 9_4M30_day1_Tob | 1.11 | 0.61 | 1.28 | 1.07 |
| 37_4M31_day5_Tob | 0.53 | 0.81 | 0.53 | 0.74 |
| 38_4M32_day5_Tob | 0.6 | 0.74 | 0.59 | 0.63 |
| 39_4M33_day5_Tob | 0.44 | 1.03 | 0.34 | 0.69 |
| 82_4M34_day28_Tob | 2.37 | 0.63 | 2.42 | 1.04 |
| 83_4M35_day28_Tob | 3.35 | 0.66 | 3.33 | 1.76 |
| 84_4M36_day28_Tob | 2.4 | 0.62 | 2.07 | 0.99 |
| 25_5F137_day1_CadCl | 0.38 | 1.25 | 0.41 | 0.6 |
| 26_5F138_day1_CadCl | 0.52 | 1.43 | 0.55 | 0.54 |
| 27_5F139_day1_CadCl | 0.36 | 1.27 | 0.3 | 0.5 |
| 54_5F141_day5_CadCl | 0.47 | 1.12 | 0.29 | 0.98 |
| 55_5F142_day5_CadCl | 0.29 | 0.9 | 0.27 | 0.49 |
| 100_5F143_day28_CadCl | 0.64 | 1.14 | 0.32 | 1.72 |
| 101_5F144_day28_CadCl | 0.58 | 0.88 | 0.42 | 1.15 |
| 102_5F145_day28_CadCl | 1.94 | 0.37 | 1.72 | 0.86 |

| Sample | AA686189_ DB71_seg6 | AA799594_ DB71_seg0 | AA964541_ DB71_seg0 | AI045075_ DB71_seg6 |
|---|---|---|---|---|
| 10_5M37_day1_CadCl | 0.38 | 1.09 | 0.28 | 0.66 |
| 11_5M38_day1_CadCl | 0.59 | 1.15 | 0.6 | 0.87 |
| 12_5M39_day1_CadCl | 0.64 | 1.05 | 0.56 | 0.84 |
| 40_5M40_day5_CadCl | 0.39 | 1.23 | 0.42 | 0.85 |
| 41_5M41_day5_CadCl | 0.5 | 0.98 | 0.41 | 0.81 |
| 42_5M42_day5_CadCl | 0.38 | 1.01 | 0.42 | 0.83 |
| 85_5M43_day28_CadCl | 0.4 | 0.66 | 0.32 | 0.75 |
| 86_5M44_day28_CadCl | 0.51 | 0.77 | 0.55 | 0.63 |
| 87_5M45_day28_CadCl | 0.82 | 0.83 | 0.72 | 0.71 |
| 28_6F146_day1_Dox | 0.78 | 1.1 | 0.62 | 0.61 |
| 29_6F147_day1_Dox | 0.4 | 1.52 | 0.46 | 0.64 |
| 30_6F148_day1_Dox | 0.26 | 1.52 | 0.33 | 3.12 |
| 56_6F149_day5_Dox | 0.51 | 0.91 | 0.5 | 0.76 |
| 57_6F150_day5_Dox | 0.48 | 0.84 | 0.46 | 0.93 |
| 58_6F151_day5_Dox | 0.38 | 1.24 | 0.4 | 0.57 |
| 103_6F171_day28_Dox | 1.5 | 1.24 | 1.04 | 0.66 |
| 104_6F172_day28_Dox | 0.62 | 1.39 | 0.55 | 0.82 |
| 105_6F173_day28_Dox | 0.04 | 1.29 | 1.3 | 1.6 |
| 13_6M46_day1_Dox | 0.59 | 1.26 | 0.54 | 1.12 |
| 14_6M47_day1_Dox | 0.49 | 0.92 | 0.55 | 1.21 |
| 15_6M48_day1_Dox | 0.39 | 0.99 | 0.38 | 0.78 |
| 43_6M49_day5_Dox | 1.2 | 0.84 | 1.14 | 0.93 |
| 44_6M50_day5_Dox | 0.6 | 1.22 | 0.48 | 2.05 |
| 88_6M71_day28_Dox | 6.37 | 0.45 | 5.73 | 1.49 |
| 89_6M72_day28_Dox | 0.92 | 1.06 | 0.96 | 0.9 |
| 90_6M73_day28_Dox | 1.28 | 0.92 | 1.43 | 0.87 |
| 115_7F155_day1_ValpA | 0.45 | 1.42 | 0.4 | 1.22 |
| 116_7F156_day1_ValpA | 0.42 | 1.1 | 0.31 | 0.01 |
| 117_7F157_day1_ValpA | 0.37 | 1.1 | 0.32 | 0.57 |
| 121_7F158_day5_ValpA | 0.36 | 1.33 | 0.38 | 0.79 |
| 122_7F159_day5_ValpA | 0.02 | 1.21 | 0.36 | 0.66 |
| 123_7F160_day5_ValpA | 0.53 | 1.37 | 0.28 | 0.66 |
| 127_7F161_day28_ValpA | 0.45 | 1.17 | 0.28 | 0.68 |
| 128_7F162_day28_ValpA | 0.58 | 1.55 | 0.32 | 1.12 |
| 129_7F163_day28_ValpA | 0.42 | 1.32 | 0.28 | 1.23 |
| 112_7M55_day1_ValpA | 0.4 | 0.92 | 0.39 | 0.88 |
| 113_7M56_day1_ValpA | 0.5 | 1.28 | 0.33 | 0.83 |
| 114_7M57_day1_ValpA | 0.48 | 0.84 | 0.42 | 0.72 |
| 118_7M58_day5_ValpA | 0.37 | 0.99 | 0.15 | 0.92 |
| 119_7M59_day5_ValpA | 0.36 | 0.86 | 0.25 | 0.47 |
| 120_7M60_day5_ValpA | 0.29 | 0.91 | 0.18 | 0.42 |
| 124_7M61_day28_ValpA | 0.21 | 1.05 | 0.15 | 0.62 |
| 125_7M62_day28_ValpA | 0.45 | 0.81 | 0.36 | 0.86 |

| Sample | AA686189_DB71_seg6 | AA799594_DB71_seg0 | AA964541_DB71_seg0 | AI045075_DB71_seg6 |
|---|---|---|---|---|
| 126_7M63_day28_ValpA | 0.4 | 1.23 | 0.25 | 0.59 |
| 62_8F181_day28_Naive | 0.47 | 1.59 | 0.39 | 0.55 |
| 63_8F182_day28_Naive | 0.6 | 1.23 | 0.37 | 0.51 |
| 64_8F183_day28_Naive | 0.42 | 0.84 | 0.38 | 0.52 |
| 59_8M81_day28_Naive | 0.74 | 1.46 | 0.68 | 0.7 |
| 60_8M82_day28_Naive | 0.46 | 1.26 | 0.3 | 0.33 |
| 61_8M83_day28_Naive | 0.45 | 1.18 | 0.34 | 0.53 |

| Sample | AI454051_DB71_seg0 | AW919147_DB71_seg0 | BI293562_DB71_seg2 |
|---|---|---|---|
| 68_1F101_day1_Saline | 0.96 | 0.9 | 0.61 |
| 69_1F102_day1_Saline | 0.99 | 0.96 | 0.67 |
| 70_1F103_day1_Saline | 0.88 | 1.03 | 0.68 |
| 74_1F104_day5_Saline | 1.08 | 0.92 | 0.2 |
| 75_1F105_day5_Saline | 0.93 | 0.9 | 0.41 |
| 109_1F107_day28_Saline | 1.3 | 1.09 | 0.29 |
| 110_1F108_day28_Saline | 1.33 | 1.12 | 0.37 |
| 111_1F109_day28_Saline | 0.7 | 0.84 | 0.16 |
| 65_1M1_day1_Saline | 0.91 | 0.9 | 1.45 |
| 66_1M2_day1_Saline | 0.11 | 0.73 | 0.42 |
| 67_1M3_day1_Saline | 1.13 | 1.05 | 0.7 |
| 71_1M4_day5_Saline | 1.01 | 0.86 | 0.83 |
| 72_1M5_day5_Saline | 0.73 | 1.17 | 0.4 |
| 73_1M6_day5_Saline | 0.49 | 0.92 | 0.33 |
| 106_1M7_day28_Saline | 0.86 | 1.3 | 0.63 |
| 107_1M8_day28_Saline | 0.88 | 1.28 | 0.32 |
| 108_1M9_day28_Saline | 1.16 | 1.7 | 0.87 |
| 16_2F110_day1_Gent | 0.9 | 1.72 | 0.59 |
| 17_2F111_day1_Gent | 0.75 | 0.72 | 0.94 |
| 18_2F112_day1_Gent | 0.57 | 0.76 | 1.04 |
| 45_2F113_day5_Gent | 0.63 | 0.86 | 0.95 |
| 46_2F114_day5_Gent | 1.04 | 0.88 | 0.76 |
| 47_2F115_day5_Gent | 1.19 | 0.95 | 2.8 |
| 91_2F116_day28_Gent | 0.67 | 0.73 | 2.01 |
| 92_2F117_day28_Gent | 1 | 1.12 | 1.19 |
| 93_2F118_day28_Gent | 0.95 | 0.89 | 0.81 |
| 1_2M10_day1_Gent | 0.4 | 0.72 | 0.93 |
| 2_2M11_day1_Gent | 0.54 | 1.1 | 0.55 |
| 3_2M12_day1_Gent | 0.79 | 0.99 | 0.41 |
| 31_2M13_day5_Gent | 0.8 | 0.71 | 2.32 |
| 32_2M14_day5_Gent | 0.63 | 0.79 | 1.61 |
| 33_2M15_day5_Gent | 0.82 | 0.78 | 0.93 |
| 76_2M16_day28_Gent | 1.14 | 0.75 | 0.63 |

| Sample | AI454051_DB71_seg0 | AW919147_DB71_seg0 | BI293562_DB71_seg2 |
|---|---|---|---|
| 77_2M17_day28_Gent | 0.64 | 0.57 | 0.72 |
| 78_2M18_day28_Gent | 0.71 | 0.93 | 1.16 |
| 19_3F119_day1_Cis | 0.94 | 0.8 | 0.74 |
| 20_3F120_day1_Cis | 1.22 | 1.09 | 0.5 |
| 21_3F121_day1_Cis | 1 | 0.87 | 0.38 |
| 48_3F122_day5_Cis | 1.05 | 1.39 | 0.74 |
| 49_3F123_day5_Cis | 1.43 | 1.05 | 0.67 |
| 50_3F124_day5_Cis | 1.39 | 0.83 | 0.7 |
| 94_3F125_day28_Cis | 0.68 | 1.08 | 5.98 |
| 95_3F126_day28_Cis | 0.8 | 0.54 | 12.27 |
| 96_3F127_day28_Cis | 0.87 | 0.72 | 8.01 |
| 4_3M19_day1_Cis | 0.65 | 0.87 | 0.7 |
| 5_3M20_day1_Cis | 0.96 | 1.21 | 0.39 |
| 6_3M21_day1_Cis | 1.45 | 0.88 | 0.71 |
| 34_3M22_day5_Cis | 0.92 | 1.08 | 3.45 |
| 35_3M23_day5_Cis | 1.16 | 0.8 | 0.81 |
| 36_3M24_day5_Cis | 0.86 | 0.88 | 1.76 |
| 79_3M25_day28_Cis | 0.73 | 0.66 | 6.24 |
| 80_3M26_day28_Cis | 0.82 | 0.84 | 2.93 |
| 81_3M27_day28_Cis | 0.78 | 0.54 | 3.33 |
| 22_4F128_day1_Tob | 1.16 | 0.8 | 0.29 |
| 23_4F129_day1_Tob | 1.36 | 1.06 | 0.42 |
| 24_4F130_day1_Tob | 0.8 | 0.98 | 0.73 |
| 51_4F131_day5_Tob | 1.26 | 0.88 | 0.71 |
| 52_4F132_day5_Tob | 1.23 | 0.99 | 1.62 |
| 53_4F133_day5_Tob | 1.32 | 1.05 | 0.84 |
| 97_4F134_day28_Tob | 0.94 | 0.8 | 1.32 |
| 98_4F135_day28_Tob | 1.29 | 0.86 | 1.02 |
| 99_4F136_day28_Tob | 0.72 | 0.72 | 0.35 |
| 7_4M28_day1_Tob | 0.86 | 0.82 | 0.58 |
| 8_4M29_day1_Tob | 1.29 | 1.33 | 1.38 |
| 9_4M30_day1_Tob | 1.17 | 1.22 | 1.04 |
| 37_4M31_day5_Tob | 0.61 | 0.58 | 0.3 |
| 38_4M32_day5_Tob | 0.57 | 0.63 | 0.44 |
| 39_4M33_day5_Tob | 0.63 | 1.14 | 0.59 |
| 82_4M34_day28_Tob | 1 | 0.76 | 0.84 |
| 83_4M35_day28_Tob | 0.94 | 1.23 | 1.78 |
| 84_4M36_day28_Tob | 1 | 0.63 | 1.07 |
| 25_5F137_day1_CadCl | 1.47 | 1 | 0.36 |
| 26_5F138_day1_CadCl | 1.46 | 1.21 | 0.4 |
| 27_5F139_day1_CadCl | 1.08 | 0.76 | 0.61 |
| 54_5F141_day5_CadCl | 1.54 | 1.06 | 0.43 |
| 55_5F142_day5_CadCl | 1.35 | 1.86 | 0.63 |

| Sample | AI454051_DB71_seg0 | AW919147_DB71_seg0 | BI293562_DB71_seg2 |
|---|---|---|---|
| 100_5F143_day28_CadCl | 0.79 | 0.77 | 0.4 |
| 101_5F144_day28_CadCl | 1.05 | 0.91 | 0.81 |
| 102_5F145_day28_CadCl | 0.54 | 0.42 | 2.59 |
| 10_5M37_day1_CadCl | 1.48 | 1 | 0.5 |
| 11_5M38_day1_CadCl | 2.13 | 1.1 | 0.51 |
| 12_5M39_day1_CadCl | 0.77 | 1.02 | 0.39 |
| 40_5M40_day5_CadCl | 0.84 | 1.3 | 0.48 |
| 41_5M41_day5_CadCl | 0.68 | 0.94 | 0.88 |
| 42_5M42_day5_CadCl | 0.92 | 1 | 0.78 |
| 85_5M43_day28_CadCl | 1.29 | 1.31 | 0.72 |
| 86_5M44_day28_CadCl | 1.31 | 1.18 | 1.35 |
| 87_5M45_day28_CadCl | 0.75 | 0.68 | 0.31 |
| 28_6F146_day1_Dox | 1.2 | 1.35 | 0.38 |
| 29_6F147_day1_Dox | 1.05 | 1.2 | 0.39 |
| 30_6F148_day1_Dox | 1.54 | 1.09 | 0.42 |
| 56_6F149_day5_Dox | 1.17 | 0.99 | 0.68 |
| 57_6F150_day5_Dox | 1.35 | 0.89 | 0.51 |
| 58_6F151_day5_Dox | 1.44 | 1.1 | 0.7 |
| 103_6F171_day28_Dox | 0.85 | 0.88 | 0.99 |
| 104_6F172_day28_Dox | 0.92 | 1.05 | 0.58 |
| 105_6F173_day28_Dox | 0.87 | 0.82 | 0 |
| 13_6M46_day1_Dox | 0.8 | 1.1 | 0.53 |
| 14_6M47_day1_Dox | 0.8 | 1.11 | 0.42 |
| 15_6M48_day1_Dox | 1.03 | 0.91 | 0.32 |
| 43_6M49_day5_Dox | 0.3 | 1.01 | 1.39 |
| 44_6M50_day5_Dox | 0.95 | 1.16 | 1.04 |
| 88_6M71_day28_Dox | 0.96 | 0.48 | 1.81 |
| 89_6M72_day28_Dox | 1.11 | 1.42 | 0.55 |
| 90_6M73_day28_Dox | 0.72 | 1.2 | 0.71 |
| 115_7F155_day1_ValpA | 1.56 | 1.43 | 0.45 |
| 116_7F156_day1_ValpA | 1.08 | 0.84 | 0.38 |
| 117_7F157_day1_ValpA | 1.17 | 1.84 | 0.4 |
| 121_7F158_day5_ValpA | 1.49 | 1.69 | 0.36 |
| 122_7F159_day5_ValpA | 1.52 | 1.83 | 0.39 |
| 123_7F160_day5_ValpA | 1.72 | 0.94 | 0.12 |
| 127_7F161_day28_ValpA | 1.16 | 0.92 | 0.18 |
| 128_7F162_day28_ValpA | 1.31 | 1.31 | 0.59 |
| 129_7F163_day28_ValpA | 1.32 | 1.06 | 0.5 |
| 112_7M55_day1_ValpA | 1.08 | 0.83 | 0.46 |
| 113_7M56_day1_ValpA | 0.97 | 0.96 | 0.52 |
| 114_7M57_day1_ValpA | 0.86 | 1.02 | 0.5 |
| 118_7M58_day5_ValpA | 1 | 0.97 | 0.59 |
| 119_7M59_day5_ValpA | 0.99 | 1.27 | 0.45 |

| Sample | AI454051_DB71_seg0 | AW919147_DB71_seg0 | BI293562_DB71_seg2 |
|---|---|---|---|
| 120_7M60_day5_ValpA | 0.81 | 0.79 | 0.62 |
| 124_7M61_day28_ValpA | 0.91 | 1.12 | 0.21 |
| 125_7M62_day28_ValpA | 1.16 | 1.16 | 0.31 |
| 126_7M63_day28_ValpA | 0.95 | 1.11 | 0.35 |
| 62_8F181_day28_Naive | 0.92 | 1.02 | 0.41 |
| 63_8F182_day28_Naive | 1.07 | 1.08 | 0.49 |
| 64_8F183_day28_Naive | 0.81 | 1.23 | 0.38 |
| 59_8M81_day28_Naive | 1.75 | 1.27 | 0.72 |
| 60_8M82_day28_Naive | 0.75 | 0.8 | 0.3 |
| 61_8M83_day28_Naive | 0.66 | 0.94 | 0.39 |

| Sample | H31045_ DB71_seg5 | H31799_ DB71_seg23 | H31883_ DB71_seg13 | AI502869_ DB71_seg0 |
|---|---|---|---|---|
| 68_1F101_day1_Saline | 0.16 | 1.05 | 0.88 | 0.98 |
| 69_1F102_day1_Saline | 1.03 | 0.96 | 0.36 | 0.68 |
| 70_1F103_day1_Saline | 0.24 | 1.06 | 0.75 | 0.73 |
| 74_1F104_day5_Saline | 0.15 | 1.16 | 0.71 | 0.99 |
| 75_1F105_day5_Saline | 0.67 | 0.7 | 0.59 | 0.67 |
| 109_1F107_day28_Saline | 0.81 | 1.21 | 0.83 | 1.25 |
| 110_1F108_day28_Saline | 1 | 1.13 | 0.89 | 2.1 |
| 111_1F109_day28_Saline | 2.96 | 1.06 | 0.83 | 1.32 |
| 65_1M1_day1_Saline | 0.93 | 1.12 | 0.68 | 0.62 |
| 66_1M2_day1_Saline | 0.69 | 0.87 | 0.69 | 0.6 |
| 67_1M3_day1_Saline | 0.19 | 1.06 | 0.78 | 0.62 |
| 71_1M4_day5_Saline | 0.28 | 0.98 | 0.63 | 0.82 |
| 72_1M5_day5_Saline | 0.18 | 1.11 | 0.56 | 0.86 |
| 73_1M6_day5_Saline | 0.25 | 0.88 | 0.68 | 0.52 |
| 106_1M7_day28_Saline | 0.42 | 0.82 | 0.88 | 1.24 |
| 107_1M8_day28_Saline | 2.19 | 1.13 | 0.73 | 1.14 |
| 108_1M9_day28_Saline | 0.36 | 1.06 | 0.84 | 1.31 |
| 16_2F110_day1_Gent | 0.27 | 1.19 | 0.99 | 0.83 |
| 17_2F111_day1_Gent | 0.22 | 0.88 | 0.92 | 0.61 |
| 18_2F112_day1_Gent | 0.73 | 0.93 | 0.82 | 0.53 |
| 45_2F113_day5_Gent | 0.52 | 0.92 | 0.73 | 0.73 |
| 46_2F114_day5_Gent | 1.01 | 0.91 | 0.55 | 0.62 |
| 47_2F115_day5_Gent | 0.4 | 1.36 | 0.91 | 0.96 |
| 91_2F116_day28_Gent | 0.79 | 0.88 | 0.88 | 0.66 |
| 92_2F117_day28_Gent | 0.84 | 1.05 | 0.98 | 0.74 |
| 93_2F118_day28_Gent | 0.67 | 1.17 | 0.96 | 0.73 |
| 1_2M10_day1_Gent | 0.16 | 1 | 0.82 | 1.53 |
| 2_2M11_day1_Gent | 0.19 | 1.04 | 0.61 | 0.99 |
| 3_2M12_day1_Gent | 0.15 | 1.2 | 0.73 | 0.76 |

| Sample | H31045_ DB71_seg5 | H31799_ DB71_seg23 | H31883_ DB71_seg13 | AI502869_ DB71_seg0 |
|---|---|---|---|---|
| 31_2M13_day5_Gent | 1.57 | 1.08 | 1.01 | 0.51 |
| 32_2M14_day5_Gent | 1.47 | 0.76 | 0.7 | 0.43 |
| 33_2M15_day5_Gent | 0.33 | 0.88 | 0.65 | 0.53 |
| 76_2M16_day28_Gent | 1.11 | 1.17 | 1.05 | 1.36 |
| 77_2M17_day28_Gent | 4.65 | 0.37 | 0.64 | 0.92 |
| 78_2M18_day28_Gent | 0.45 | 1.06 | 0.8 | 0.77 |
| 19_3F119_day1_Cis | 0.24 | 1.03 | 2.03 | 0.67 |
| 20_3F120_day1_Cis | 0.28 | 1.09 | 1.06 | 0.78 |
| 21_3F121_day1_Cis | 0.22 | 1.04 | 1.07 | 0.74 |
| 48_3F122_day5_Cis | 1.8 | 1.1 | 1.17 | 0.8 |
| 49_3F123_day5_Cis | 0.34 | 0.74 | 1.44 | 0.93 |
| 50_3F124_day5_Cis | 0.51 | 1.23 | 1.2 | 0.74 |
| 94_3F125_day28_Cis | 0.73 | 1.05 | 2.72 | 1.03 |
| 95_3F126_day28_Cis | 0.73 | 1.15 | 4.07 | 1.03 |
| 96_3F127_day28_Cis | 0.81 | 0.98 | 3.83 | 1.25 |
| 4_3M19_day1_Cis | 0.82 | 1.22 | 0.74 | 0.87 |
| 5_3M20_day1_Cis | 0.21 | 1.27 | 0.71 | 0.87 |
| 6_3M21_day1_Cis | 1.22 | 1.12 | 1.52 | 0.84 |
| 34_3M22_day5_Cis | 3.25 | 0.92 | 1.73 | 0.52 |
| 35_3M23_day5_Cis | 0.23 | 1.05 | 1.3 | 1.13 |
| 36_3M24_day5_Cis | 1.66 | 1.06 | 2.43 | 0.61 |
| 79_3M25_day28_Cis | 0.88 | 0.88 | 2.41 | 0.74 |
| 80_3M26_day28_Cis | 2.92 | 1.21 | 3.37 | 1.35 |
| 81_3M27_day28_Cis | 2.96 | 0.9 | 2.23 | 0.95 |
| 22_4F128_day1_Tob | 0.2 | 0.91 | 0.87 | 0.52 |
| 23_4F129_day1_Tob | 0.18 | 0.87 | 0.68 | 1.32 |
| 24_4F130_day1_Tob | 0.74 | 1.2 | 0.87 | 0.79 |
| 51_4F131_day5_Tob | 1.19 | 1.1 | 0.82 | 0.81 |
| 52_4F132_day5_Tob | 0.42 | 0.92 | 0.69 | 0.74 |
| 53_4F133_day5_Tob | 0.38 | 0.83 | 0.52 | 0.74 |
| 97_4F134_day28_Tob | 0.76 | 1.03 | 0.84 | 1.08 |
| 98_4F135_day28_Tob | 1 | 0.71 | 0.63 | 2.38 |
| 99_4F136_day28_Tob | 0.63 | 1.15 | 0.84 | 1.04 |
| 7_4M28_day1_Tob | 0.2 | 1.09 | 0.83 | 0.87 |
| 8_4M29_day1_Tob | 1.03 | 1.14 | 0.69 | 1.02 |
| 9_4M30_day1_Tob | 0.22 | 1.17 | 0.61 | 1.28 |
| 37_4M31_day5_Tob | 0.9 | 0.71 | 0.48 | 0.42 |
| 38_4M32_day5_Tob | 0.29 | 0.72 | 0.47 | 0.5 |
| 39_4M33_day5_Tob | 1.65 | 0.96 | 0.82 | 0.72 |
| 82_4M34_day28_Tob | 2.08 | 0.94 | 0.6 | 1.22 |
| 83_4M35_day28_Tob | 2.68 | 0.88 | 0.73 | 0.99 |
| 84_4M36_day28_Tob | 2.92 | 1.12 | 0.73 | 1.22 |
| 25_5F137_day1_CadCl | 1.97 | 1 | 0.73 | 0.54 |

| Sample | H31045_ DB71_seg5 | H31799_ DB71_seg23 | H31883_ DB71_seg13 | AI502869_ DB71_seg0 |
|---|---|---|---|---|
| 26_5F138_day1_CadCl | 0.39 | 1.24 | 0.63 | 1.37 |
| 27_5F139_day1_CadCl | 0.39 | 1 | 0.87 | 0.56 |
| 54_5F141_day5_CadCl | 0.56 | 0.83 | 0.87 | 0.94 |
| 55_5F142_day5_CadCl | 0.32 | 0.99 | 0.86 | 0.73 |
| 100_5F143_day28_CadCl | 2.88 | 1.59 | 0.97 | 0.73 |
| 101_5F144_day28_CadCl | 0.83 | 0.94 | 1.02 | 1.22 |
| 102_5F145_day28_CadCl | 0.57 | 0.67 | 0.86 | 0.44 |
| 10_5M37_day1_CadCl | 0.19 | 0.91 | 0.65 | 1.14 |
| 11_5M38_day1_CadCl | 0.19 | 0.9 | 0.71 | 2.59 |
| 12_5M39_day1_CadCl | 0.18 | 1.15 | 0.78 | 0.86 |
| 40_5M40_day5_CadCl | 0.42 | 1.17 | 0.83 | 0.69 |
| 41_5M41_day5_CadCl | 0.4 | 0.98 | 0.78 | 0.68 |
| 42_5M42_day5_CadCl | 0.46 | 0.94 | 0.64 | 0.85 |
| 85_5M43_day28_CadCl | 2.23 | 1.05 | 0.95 | 2.47 |
| 86_5M44_day28_CadCl | 2.41 | 1.02 | 0.8 | 2.93 |
| 87_5M45_day28_CadCl | 2 | 0.76 | 0.86 | 1.18 |
| 28_6F146_day1_Dox | 1.15 | 1.04 | 0.93 | 0.71 |
| 29_6F147_day1_Dox | 0.3 | 0.99 | 0.89 | 0.81 |
| 30_6F148_day1_Dox | 1.95 | 0.87 | 0.67 | 0.67 |
| 56_6F149_day5_Dox | 1.86 | 0.95 | 1.16 | 0.51 |
| 57_6F150_day5_Dox | 1.19 | 1.08 | 1.03 | 1 |
| 58_6F151_day5_Dox | 0.52 | 0.66 | 0.9 | 1.07 |
| 103_6F171_day28_Dox | 2.16 | 1.16 | 1.48 | 1.33 |
| 104_6F172_day28_Dox | 2.58 | 1.25 | 1.77 | 1.48 |
| 105_6F173_day28_Dox | 0.51 | 1.57 | 2.29 | 0.98 |
| 13_6M46_day1_Dox | 0.23 | 1.07 | 1.02 | 0.77 |
| 14_6M47_day1_Dox | 0.32 | 1.26 | 1.07 | 0.68 |
| 15_6M48_day1_Dox | 0.75 | 0.92 | 0.9 | 0.78 |
| 43_6M49_day5_Dox | 0.35 | 0.56 | 1.21 | 0.73 |
| 44_6M50_day5_Dox | 1.41 | 0.9 | 1.38 | 0.71 |
| 88_6M71_day28_Dox | 1.57 | 0.64 | 1.45 | 0.42 |
| 89_6M72_day28_Dox | 2.33 | 1.11 | 1.59 | 1.49 |
| 90_6M73_day28_Dox | 2.12 | 1.06 | 1.93 | 1.28 |
| 115_7F155_day1_ValpA | 0.68 | 1.29 | 1.02 | 0.86 |
| 116_7F156_day1_ValpA | 1.71 | 0.9 | 0.01 | 1.09 |
| 117_7F157_day1_ValpA | 0.53 | 0 | 0.66 | 1.13 |
| 121_7F158_day5_ValpA | 0.56 | 0.94 | 1.54 | 1.81 |
| 122_7F159_day5_ValpA | 1 | 1.19 | 1.1 | 2.53 |
| 123_7F160_day5_ValpA | 0.57 | 1.15 | 1.05 | 1.94 |
| 127_7F161_day28_ValpA | 4.35 | 0.98 | 0.92 | 1.24 |
| 128_7F162_day28_ValpA | 0.58 | 0.67 | 0.9 | 1.19 |
| 129_7F163_day28_ValpA | 1.41 | 0.99 | 0.81 | 1.18 |
| 112_7M55_day1_ValpA | 3.33 | 1.11 | 0.75 | 1.33 |

| Sample | H31045_ DB71_seg5 | H31799_ DB71_seg23 | H31883_ DB71_seg13 | AI502869_ DB71_seg0 |
|---|---|---|---|---|
| 113_7M56_day1_ValpA | 2.68 | 1.23 | 0.9 | 1 |
| 114_7M57_day1_ValpA | 0.73 | 1.16 | 0.95 | 1.43 |
| 118_7M58_day5_ValpA | 2.42 | 0.51 | 1 | 1.37 |
| 119_7M59_day5_ValpA | 0.83 | 1.06 | 0.48 | 1.06 |
| 120_7M60_day5_ValpA | 2.53 | 1.02 | 0.63 | 1.29 |
| 124_7M61_day28_ValpA | 1.58 | 0.98 | 0.71 | 0.97 |
| 125_7M62_day28_ValpA | 0.2 | 1.03 | 1.11 | 1.46 |
| 126_7M63_day28_ValpA | 0.47 | 0.75 | 0.72 | 1.43 |
| 62_8F181_day28_Naive | 1.21 | 1.05 | 0.79 | 1.24 |
| 63_8F182_day28_Naive | 0.25 | 0.94 | 0.73 | 0.78 |
| 64_8F183_day28_Naive | 0.25 | 1 | 0.66 | 0.77 |
| 59_8M81_day28_Naive | 0.54 | 1.26 | 0 | 1 |
| 60_8M82_day28_Naive | 0.26 | 1.05 | 0.58 | 0.79 |
| 61_8M83_day28_Naive | 0.29 | 0.82 | 0.68 | 0.64 |

| Sample | H33998_DB71_seg19 | RATLRFI_DB71_seg9 | RATRAB13X_DB71_seg11-13 |
|---|---|---|---|
| 68_1F101_day1_Saline | 0.73 | 0.37 | 0.97 |
| 69_1F102_day1_Saline | 1.01 | 0.28 | 0.78 |
| 70_1F103_day1_Saline | 0.88 | 0.09 | 0.98 |
| 74_1F104_day5_Saline | 0.93 | 0.36 | 0.97 |
| 75_1F105_day5_Saline | 1.1 | 0.49 | 0.94 |
| 109_1F107_day28_Saline | 0.41 | 0.44 | 1 |
| 110_1F108_day28_Saline | 0.59 | 0.37 | 1.11 |
| 111_1F109_day28_Saline | 0.57 | 0.58 | 0.92 |
| 65_1M1_day1_Saline | 0.51 | 0.01 | 0.83 |
| 66_1M2_day1_Saline | 0.98 | 0.32 | 1.06 |
| 67_1M3_day1_Saline | 0.51 | 0.36 | 0.87 |
| 71_1M4_day5_Saline | 1.26 | 0.21 | 0.76 |
| 72_1M5_day5_Saline | 1.13 | 0.25 | 0.76 |
| 73_1M6_day5_Saline | 0.98 | 0.22 | 0.64 |
| 106_1M7_day28_Saline | 0.6 | 0.32 | 0.82 |
| 107_1M8_day28_Saline | 0.36 | 0.12 | 0.77 |
| 108_1M9_day28_Saline | 0.73 | 0.43 | 0.91 |
| 16_2F110_day1_Gent | 1.31 | 0.98 | 1.16 |
| 17_2F111_day1_Gent | 0.83 | 1.54 | 0.7 |
| 18_2F112_day1_Gent | 0.56 | 1.09 | 0.77 |
| 45_2F113_day5_Gent | 1.9 | 0.48 | 0.9 |
| 46_2F114_day5_Gent | 1.5 | 0.43 | 0.82 |
| 47_2F115_day5_Gent | 2.19 | 4.15 | 1.12 |
| 91_2F116_day28_Gent | 0.4 | 5.63 | 0.83 |
| 92_2F117_day28_Gent | 0.7 | 4.17 | 1.12 |
| 93_2F118_day28_Gent | 0.56 | 2.43 | 0.88 |

| Sample | H33998_DB71_seg19 | RATLRFI_DB71_seg9 | RATRAB13X_DB71_seg11-13 |
|---|---|---|---|
| 1_2M10_day1_Gent | 1.51 | 2.74 | 2.79 |
| 2_2M11_day1_Gent | 1.35 | 1.72 | 1.14 |
| 3_2M12_day1_Gent | 0.99 | 0.27 | 1.03 |
| 31_2M13_day5_Gent | 1.91 | 1.35 | 0.9 |
| 32_2M14_day5_Gent | 0.83 | 1.13 | 0.61 |
| 33_2M15_day5_Gent | 1.16 | 0.59 | 0.8 |
| 76_2M16_day28_Gent | 0.4 | 2.56 | 1.17 |
| 77_2M17_day28_Gent | 0.49 | 1.59 | 0.76 |
| 78_2M18_day28_Gent | 0.42 | 2.51 | 1.22 |
| 19_3F119_day1_Cis | 0.85 | 1.49 | 0.91 |
| 20_3F120_day1_Cis | 1 | 0.3 | 1.12 |
| 21_3F121_day1_Cis | 0.87 | 0.57 | 1.09 |
| 48_3F122_day5_Cis | 1.79 | 0.79 | 1.06 |
| 49_3F123_day5_Cis | 1.84 | 0.44 | 1.01 |
| 50_3F124_day5_Cis | 1.95 | 0.32 | 1.02 |
| 94_3F125_day28_Cis | 0.55 | 2.33 | 1.29 |
| 95_3F126_day28_Cis | 0.69 | 3.05 | 1.62 |
| 96_3F127_day28_Cis | 0.85 | 3.8 | 1.78 |
| 4_3M19_day1_Cis | 0.65 | 0.24 | 0.87 |
| 5_3M20_day1_Cis | 0.75 | 0.2 | 1.15 |
| 6_3M21_day1_Cis | 1.14 | 0.37 | 1.16 |
| 34_3M22_day5_Cis | 1.49 | 0.65 | 1.13 |
| 35_3M23_day5_Cis | 0.46 | 0.34 | 0.74 |
| 36_3M24_day5_Cis | 2.14 | 1.97 | 1.27 |
| 79_3M25_day28_Cis | 0.61 | 3.03 | 1.57 |
| 80_3M26_day28_Cis | 0.62 | 2.4 | 1.24 |
| 81_3M27_day28_Cis | 0.41 | 2.26 | 1.31 |
| 22_4F128_day1_Tob | 0.75 | 1.03 | 1.11 |
| 23_4F129_day1_Tob | 0.76 | 0.68 | 1.06 |
| 24_4F130_day1_Tob | 0.77 | 3.08 | 1.22 |
| 51_4F131_day5_Tob | 1.65 | 0.75 | 1.21 |
| 52_4F132_day5_Tob | 1.88 | 3.08 | 0.98 |
| 53_4F133_day5_Tob | 1.61 | 0.79 | 0.87 |
| 97_4F134_day28_Tob | 0.99 | 1.94 | 1.25 |
| 98_4F135_day28_Tob | 0.44 | 2.39 | 0.9 |
| 99_4F136_day28_Tob | 0.74 | 1.98 | 1.29 |
| 7_4M28_day1_Tob | 0.7 | 0.22 | 1.12 |
| 8_4M29_day1_Tob | 0.98 | 1.63 | 1.01 |
| 9_4M30_day1_Tob | 0.71 | 3.36 | 1.08 |
| 37_4M31_day5_Tob | 1.1 | 0.88 | 0.59 |
| 38_4M32_day5_Tob | 0.88 | 0.72 | 0.71 |
| 39_4M33_day5_Tob | 1.3 | 0.17 | 0.81 |
| 82_4M34_day28_Tob | 0.61 | 2.01 | 1.12 |

| Sample | H33998_DB71_seg19 | RATLRFI_DB71_seg9 | RATRAB13X_DB71_seg11-13 |
|---|---|---|---|
| 83_4M35_day28_Tob | 0.54 | 3.78 | 1.34 |
| 84_4M36_day28_Tob | 0.69 | 2.37 | 1.31 |
| 25_5F137_day1_CadCl | 2.29 | 0.27 | 0.95 |
| 26_5F138_day1_CadCl | 2.51 | 0.33 | 1.2 |
| 27_5F139_day1_CadCl | 0.01 | 0.32 | 0.95 |
| 54_5F141_day5_CadCl | 2 | 0.35 | 0.95 |
| 55_5F142_day5_CadCl | 0.82 | 0.31 | 0.9 |
| 100_5F143_day28_CadCl | 1.26 | 0.66 | 1.37 |
| 101_5F144_day28_CadCl | 0.59 | 0.85 | 0.92 |
| 102_5F145_day28_CadCl | 0.43 | 1.78 | 0.96 |
| 10_5M37_day1_CadCl | 0.76 | 0.27 | 1.05 |
| 11_5M38_day1_CadCl | 0.66 | 0.36 | 1.22 |
| 12_5M39_day1_CadCl | 0.69 | 0.23 | 1.14 |
| 40_5M40_day5_CadCl | 1.39 | 0.17 | 0.75 |
| 41_5M41_day5_CadCl | 1.36 | 0.18 | 0.75 |
| 42_5M42_day5_CadCl | 1.64 | 0.29 | 0.62 |
| 85_5M43_day28_CadCl | 0.45 | 0.28 | 1.11 |
| 86_5M44_day28_CadCl | 0.26 | 0.67 | 1.08 |
| 87_5M45_day28_CadCl | 0.41 | 0.56 | 0.88 |
| 28_6F146_day1_Dox | 1.25 | 0.25 | 1.1 |
| 29_6F147_day1_Dox | 1.34 | 0.21 | 0.9 |
| 30_6F148_day1_Dox | 2.14 | 0.31 | 1 |
| 56_6F149_day5_Dox | 2.39 | 0.46 | 1.17 |
| 57_6F150_day5_Dox | 1.29 | 0.29 | 1.16 |
| 58_6F151_day5_Dox | 3.68 | 0.18 | 0.94 |
| 103_6F171_day28_Dox | 0.48 | 0.72 | 0.74 |
| 104_6F172_day28_Dox | 0.58 | 0.4 | 0.95 |
| 105_6F173_day28_Dox | 0.96 | 1.47 | 1.05 |
| 13_6M46_day1_Dox | 0.97 | 0.46 | 0.88 |
| 14_6M47_day1_Dox | 1.07 | 0.48 | 1.09 |
| 15_6M48_day1_Dox | 2.75 | 0.24 | 0.86 |
| 43_6M49_day5_Dox | 1.19 | 0.47 | 0.7 |
| 44_6M50_day5_Dox | 1.37 | 0.25 | 0.83 |
| 88_6M71_day28_Dox | 0.64 | 8.34 | 0.91 |
| 89_6M72_day28_Dox | 0.44 | 0.53 | 0.77 |
| 90_6M73_day28_Dox | 0.54 | 1.08 | 0.84 |
| 115_7F155_day1_ValpA | 1.48 | 0.55 | 1.32 |
| 116_7F156_day1_ValpA | 0.82 | 0.32 | 0.96 |
| 117_7F157_day1_ValpA | 1.15 | 0.45 | 1.01 |
| 121_7F158_day5_ValpA | 0.76 | 0.39 | 0.79 |
| 122_7F159_day5_ValpA | 0.88 | 0.41 | 1.26 |
| 123_7F160_day5_ValpA | 1.19 | 0.39 | 1.41 |
| 127_7F161_day28_ValpA | 0.71 | 0.36 | 0.91 |

| Sample | H33998_DB71_seg19 | RATLRFI_DB71_seg9 | RATRAB13X_DB71_seg11-13 |
|---|---|---|---|
| 128_7F162_day28_ValpA | 0.56 | 0.54 | 0.91 |
| 129_7F163_day28_ValpA | 0.58 | 0.53 | 0.96 |
| 112_7M55_day1_ValpA | 0.37 | 0.39 | 0.79 |
| 113_7M56_day1_ValpA | 0.77 | 0.36 | 0.93 |
| 114_7M57_day1_ValpA | 0.53 | 0.44 | 0.83 |
| 118_7M58_day5_ValpA | 1.36 | 0.22 | 0.67 |
| 119_7M59_day5_ValpA | 1.11 | 0.21 | 0.8 |
| 120_7M60_day5_ValpA | 1.03 | 0.3 | 0.65 |
| 124_7M61_day28_ValpA | 0.69 | 0.23 | 0.88 |
| 125_7M62_day28_ValpA | 0.78 | 0.48 | 0.99 |
| 126_7M63_day28_ValpA | 0.52 | 0.33 | 0.69 |
| 62_8F181_day28_Naive | 0.69 | 0.39 | 1.07 |
| 63_8F182_day28_Naive | 1.23 | 0.45 | 1.07 |
| 64_8F183_day28_Naive | 0.85 | 0.34 | 0.79 |
| 59_8M81_day28_Naive | 1.25 | 0.42 | 1.08 |
| 60_8M82_day28_Naive | 0.99 | 0.25 | 0.66 |
| 61_8M83_day28_Naive | 0 | 0.26 | 0.77 |

| Sample | RNU24174_DB71_seg8 | W41270_DB81_seg11 |
|---|---|---|
| 68_1F101_day1_Saline | 0.57 | 0.6 |
| 69_1F102_day1_Saline | 0.41 | 0.86 |
| 70_1F103_day1_Saline | 0.32 | 1.31 |
| 74_1F104_day5_Saline | 0.26 | 0.59 |
| 75_1F105_day5_Saline | 0.28 | 0.83 |
| 109_1F107_day28_Saline | 0.33 | 0.36 |
| 110_1F108_day28_Saline | 0.24 | 0.41 |
| 111_1F109_day28_Saline | 0.33 | 0.39 |
| 65_1M1_day1_Saline | 0.24 | 0.57 |
| 66_1M2_day1_Saline | 0.15 | 0.99 |
| 67_1M3_day1_Saline | 0.29 | 0.54 |
| 71_1M4_day5_Saline | 0.15 | 0.68 |
| 72_1M5_day5_Saline | 0.33 | 1 |
| 73_1M6_day5_Saline | 0.12 | 0.46 |
| 106_1M7_day28_Saline | 0.24 | 0.33 |
| 107_1M8_day28_Saline | 0.15 | 0.26 |
| 108_1M9_day28_Saline | 0.27 | 0.33 |
| 16_2F110_day1_Gent | 0.47 | 1.24 |
| 17_2F111_day1_Gent | 0.5 | 0.67 |
| 18_2F112_day1_Gent | 0.47 | 1.19 |
| 45_2F113_day5_Gent | 0.31 | 2.04 |
| 46_2F114_day5_Gent | 0.22 | 1.34 |

| Sample | RNU24174_DB71_seg8 | W41270_DB81_seg11 |
|---|---|---|
| 47_2F115_day5_Gent | 0.81 | 2.42 |
| 91_2F116_day28_Gent | 1.53 | 0.94 |
| 92_2F117_day28_Gent | 1.2 | 0.81 |
| 93_2F118_day28_Gent | 0.59 | 0.81 |
| 1_2M10_day1_Gent | 0.74 | 0.46 |
| 2_2M11_day1_Gent | 0.37 | 0.68 |
| 3_2M12_day1_Gent | 0.26 | 0.57 |
| 31_2M13_day5_Gent | 0.65 | 2.03 |
| 32_2M14_day5_Gent | 0.36 | 1.08 |
| 33_2M15_day5_Gent | 0.4 | 1.2 |
| 76_2M16_day28_Gent | 1.91 | 1.42 |
| 77_2M17_day28_Gent | 0.69 | 0.75 |
| 78_2M18_day28_Gent | 0.65 | 0.84 |
| 19_3F119_day1_Cis | 0.57 | 1.11 |
| 20_3F120_day1_Cis | 0.32 | 0.86 |
| 21_3F121_day1_Cis | 0.36 | 0.76 |
| 48_3F122_day5_Cis | 0.97 | 1.97 |
| 49_3F123_day5_Cis | 0.7 | 1.9 |
| 50_3F124_day5_Cis | 0.6 | 1.79 |
| 94_3F125_day28_Cis | 8.38 | 1.45 |
| 95_3F126_day28_Cis | 12.63 | 1.8 |
| 96_3F127_day28_Cis | 12.14 | 1.48 |
| 4_3M19_day1_Cis | 0.2 | 0.33 |
| 5_3M20_day1_Cis | 0.27 | 0.63 |
| 6_3M21_day1_Cis | 0.69 | 0.71 |
| 34_3M22_day5_Cis | 1.3 | 1.51 |
| 35_3M23_day5_Cis | 0.31 | 1.07 |
| 36_3M24_day5_Cis | 3.6 | 3.12 |
| 79_3M25_day28_Cis | 10.21 | 1.94 |
| 80_3M26_day28_Cis | 7.3 | 1.11 |
| 81_3M27_day28_Cis | 8.08 | 1.45 |
| 22_4F128_day1_Tob | 0.29 | 1.01 |
| 23_4F129_day1_Tob | 0.32 | 0.44 |
| 24_4F130_day1_Tob | 0.53 | 0.83 |
| 51_4F131_day5_Tob | 0.46 | 2.6 |
| 52_4F132_day5_Tob | 0.76 | 3.25 |
| 53_4F133_day5_Tob | 0.47 | 2.67 |
| 97_4F134_day28_Tob | 0.55 | 1.01 |
| 98_4F135_day28_Tob | 0.65 | 0.73 |
| 99_4F136_day28_Tob | 0.69 | 0.9 |
| 7_4M28_day1_Tob | 0.29 | 0.57 |

| Sample | RNU24174_DB71_seg8 | W41270_DB81_seg11 |
|---|---|---|
| 8_4M29_day1_Tob | 0.49 | 0.79 |
| 9_4M30_day1_Tob | 0.51 | 0.76 |
| 37_4M31_day5_Tob | 0.09 | 0.62 |
| 38_4M32_day5_Tob | 0.2 | 0.81 |
| 39_4M33_day5_Tob | 0.14 | 1.21 |
| 82_4M34_day28_Tob | 0.54 | 0.92 |
| 83_4M35_day28_Tob | 0.93 | 1.03 |
| 84_4M36_day28_Tob | 0.62 | 0.79 |
| 25_5F137_day1_CadCl | 0.3 | 1.44 |
| 26_5F138_day1_CadCl | 0.29 | 1.78 |
| 27_5F139_day1_CadCl | 0.49 | 1.48 |
| 54_5F141_day5_CadCl | 0.33 | 2.49 |
| 55_5F142_day5_CadCl | 0.27 | 1.17 |
| 100_5F143_day28_CadCl | 0.21 | 0.86 |
| 101_5F144_day28_CadCl | 0.45 | 0.55 |
| 102_5F145_day28_CadCl | 1.21 | 0.83 |
| 10_5M37_day1_CadCl | 0.2 | 0.5 |
| 11_5M38_day1_CadCl | 0.4 | 0.62 |
| 12_5M39_day1_CadCl | 0.22 | 0.43 |
| 40_5M40_day5_CadCl | 0.12 | 1.02 |
| 41_5M41_day5_CadCl | 0.13 | 1.43 |
| 42_5M42_day5_CadCl | 0.12 | 1.52 |
| 85_5M43_day28_CadCl | 0.25 | 0.33 |
| 86_5M44_day28_CadCl | 0.39 | 0.27 |
| 87_5M45_day28_CadCl | 0.25 | 0.39 |
| 28_6F146_day1_Dox | 0.29 | 2.24 |
| 29_6F147_day1_Dox | 0.35 | 1.2 |
| 30_6F148_day1_Dox | 0.31 | 1.56 |
| 56_6F149_day5_Dox | 0.83 | 1.71 |
| 57_6F150_day5_Dox | 0.59 | 1.37 |
| 58_6F151_day5_Dox | 0.63 | 2.13 |
| 103_6F171_day28_Dox | 1.99 | 0.84 |
| 104_6F172_day28_Dox | 2.06 | 0.44 |
| 105_6F173_day28_Dox | 4.39 | 0.77 |
| 13_6M46_day1_Dox | 0.48 | 1.74 |
| 14_6M47_day1_Dox | 0.49 | 0.95 |
| 15_6M48_day1_Dox | 0.36 | 0.91 |
| 43_6M49_day5_Dox | 1.08 | 1.64 |
| 44_6M50_day5_Dox | 0.87 | 1.35 |
| 88_6M71_day28_Dox | 3.5 | 1.64 |
| 89_6M72_day28_Dox | 2.2 | 0.54 |

| Sample | RNU24174_DB71_seg8 | W41270_DB81_seg11 |
|---|---|---|
| 90_6M73_day28_Dox | 2.91 | 0.5 |
| 115_7F155_day1_ValpA | 0.45 | 0.87 |
| 116_7F156_day1_ValpA | 0.28 | 0.42 |
| 117_7F157_day1_ValpA | 0.28 | 0.64 |
| 121_7F158_day5_ValpA | 0.35 | 0.54 |
| 122_7F159_day5_ValpA | 0.51 | 0.55 |
| 123_7F160_day5_ValpA | 0.36 | 0.37 |
| 127_7F161_day28_ValpA | 0.23 | 0.65 |
| 128_7F162_day28_ValpA | 0.28 | 0.3 |
| 129_7F163_day28_ValpA | 0.29 | 0.4 |
| 112_7M55_day1_ValpA | 0.16 | 0.57 |
| 113_7M56_day1_ValpA | 0.19 | 0.49 |
| 114_7M57_day1_ValpA | 0.25 | 0.57 |
| 118_7M58_day5_ValpA | 0.2 | 0.7 |
| 119_7M59_day5_ValpA | 0.14 | 0.86 |
| 120_7M60_day5_ValpA | 0.17 | 0.62 |
| 124_7M61_day28_ValpA | 0.09 | 0.39 |
| 125_7M62_day28_ValpA | 0.19 | 0.42 |
| 126_7M63_day28_ValpA | 0.09 | 0.36 |
| 62_8F181_day28_Naive | 0.26 | 0.62 |
| 63_8F182_day28_Naive | 0.36 | 0.01 |
| 64_8F183_day28_Naive | 0.37 | 0.83 |
| 59_8M81_day28_Naive | 0.2 | 1.24 |
| 60_8M82_day28_Naive | 0.15 | 0.59 |
| 61_8M83_day28_Naive | 0.24 | 0.37 |

| Sample | W64472_DB81_seg2 | W83813_DB81_seg27 | MUSCYCG1R_DB81_seg15-17 |
|---|---|---|---|
| 68_1F101_day1_Saline | 0.45 | 0.94 | 0.67 |
| 69_1F102_day1_Saline | 0.35 | 0.77 | 0.94 |
| 70_1F103_day1_Saline | 0.43 | 1.03 | 0.6 |
| 74_1F104_day5_Saline | 0.35 | 1.03 | 0.76 |
| 75_1F105_day5_Saline | 0.56 | 0.99 | 0.75 |
| 109_1F107_day28_Saline | 0.24 | 0.48 | 0.57 |
| 110_1F108_day28_Saline | 0.38 | 0.66 | 0.76 |
| 111_1F109_day28_Saline | 0.61 | 0.67 | 0.7 |
| 65_1M1_day1_Saline | 0.65 | 0.68 | 0.47 |
| 66_1M2_day1_Saline | 0.56 | 0.87 | 0.41 |
| 67_1M3_day1_Saline | 1.02 | 0.94 | 0.4 |
| 71_1M4_day5_Saline | 1.02 | 1.29 | 0.86 |
| 72_1M5_day5_Saline | 0.75 | 1.2 | 0.92 |
| 73_1M6_day5_Saline | 0.62 | 1.07 | 0.54 |

| Sample | W64472_DB81_seg2 | W83813_DB81_seg27 | MUSCYCG1R_DB81_seg15-17 |
|---|---|---|---|
| 106_1M7_day28_Saline | 0.4 | 0.5 | 0.41 |
| 107_1M8_day28_Saline | 0.23 | 0.38 | 0.45 |
| 108_1M9_day28_Saline | 0.53 | 0.43 | 0.43 |
| 16_2F110_day1_Gent | 0.63 | 0.84 | 0.63 |
| 17_2F111_day1_Gent | 0.45 | 0.79 | 0.53 |
| 18_2F112_day1_Gent | 0.61 | 0.78 | 0.66 |
| 45_2F113_day5_Gent | 1.5 | 2.07 | 1.51 |
| 46_2F114_day5_Gent | 1.2 | 1.82 | 1.61 |
| 47_2F115_day5_Gent | 0.94 | 1.7 | 1.51 |
| 91_2F116_day28_Gent | 0.92 | 0.45 | 0.69 |
| 92_2F117_day28_Gent | 0.66 | 0.34 | 0.62 |
| 93_2F118_day28_Gent | 0.54 | 0.62 | 0.57 |
| 1_2M10_day1_Gent | 0.4 | 0.53 | 0.5 |
| 2_2M11_day1_Gent | 0.54 | 0.58 | 0.49 |
| 3_2M12_day1_Gent | 0.54 | 0.71 | 0.41 |
| 31_2M13_day5_Gent | 1.95 | 1.35 | 0.95 |
| 32_2M14_day5_Gent | 1.62 | 0.88 | 0.79 |
| 33_2M15_day5_Gent | 1.61 | 1.4 | 1.02 |
| 76_2M16_day28_Gent | 1.49 | 0.78 | 0.65 |
| 77_2M17_day28_Gent | 0.84 | 0.54 | 0.56 |
| 78_2M18_day28_Gent | 0.81 | 0.38 | 0.54 |
| 19_3F119_day1_Cis | 0.8 | 1.1 | 0.77 |
| 20_3F120_day1_Cis | 0.54 | 0.97 | 0.85 |
| 21_3F121_day1_Cis | 0.55 | 0.99 | 0.72 |
| 48_3F122_day5_Cis | 2.23 | 2.12 | 3.12 |
| 49_3F123_day5_Cis | 2.1 | 2.25 | 3.59 |
| 50_3F124_day5_Cis | 1.99 | 2.12 | 2.07 |
| 94_3F125_day28_Cis | 1.81 | 0.71 | 1.96 |
| 95_3F126_day28_Cis | 3.16 | 0.7 | 2.57 |
| 96_3F127_day28_Cis | 2.87 | 0.78 | 2.7 |
| 4_3M19_day1_Cis | 0.3 | 0.5 | 0.54 |
| 5_3M20_day1_Cis | 0.51 | 0.66 | 0.35 |
| 6_3M21_day1_Cis | 0.92 | 0.94 | 0.67 |
| 34_3M22_day5_Cis | 2.64 | 1.39 | 2.31 |
| 35_3M23_day5_Cis | 0.95 | 0.94 | 0.92 |
| 36_3M24_day5_Cis | 4.55 | 1.41 | 2.34 |
| 79_3M25_day28_Cis | 2.8 | 0.51 | 2.09 |
| 80_3M26_day28_Cis | 3.04 | 0.51 | 2.31 |
| 81_3M27_day28_Cis | 1.65 | 0.57 | 1.93 |
| 22_4F128_day1_Tob | 0.35 | 0.86 | 0.72 |
| 23_4F129_day1_Tob | 0.26 | 0.61 | 0.46 |
| 24_4F130_day1_Tob | 0.49 | 1 | 0.61 |
| 51_4F131_day5_Tob | 1.4 | 2.61 | 1.95 |

| Sample | W64472_DB81_seg2 | W83813_DB81_seg27 | MUSCYCG1R_DB81_seg15-17 |
|---|---|---|---|
| 52_4F132_day5_Tob | 1.55 | 1.93 | 1.44 |
| 53_4F133_day5_Tob | 1.27 | 1.76 | 1.15 |
| 97_4F134_day28_Tob | 0.48 | 0.75 | 0.77 |
| 98_4F135_day28_Tob | 0.43 | 0.49 | 0.59 |
| 99_4F136_day28_Tob | 0.62 | 0.63 | 0.73 |
| 7_4M28_day1_Tob | 0.45 | 0.65 | 0.43 |
| 8_4M29_day1_Tob | 0.66 | 0.73 | 0.49 |
| 9_4M30_day1_Tob | 0.44 | 0.48 | 0.47 |
| 37_4M31_day5_Tob | 0.93 | 0.7 | 0.4 |
| 38_4M32_day5_Tob | 0.96 | 1.13 | 0.69 |
| 39_4M33_day5_Tob | 1.3 | 1.4 | 1.07 |
| 82_4M34_day28_Tob | 0.44 | 0.48 | 0.72 |
| 83_4M35_day28_Tob | 0.85 | 0.56 | 0.59 |
| 84_4M36_day28_Tob | 0.79 | 0.55 | 0.71 |
| 25_5F137_day1_CadCl | 1.07 | 2.07 | 1.91 |
| 26_5F138_day1_CadCl | 1.07 | 2.19 | 1.36 |
| 27_5F139_day1_CadCl | 1.1 | 1.66 | 1.68 |
| 54_5F141_day5_CadCl | 1.65 | 3.79 | 1.72 |
| 55_5F142_day5_CadCl | 0.78 | 1.59 | 1.13 |
| 100_5F143_day28_CadCl | 0.89 | 0.95 | 0.88 |
| 101_5F144_day28_CadCl | 0.55 | 0.64 | 0.71 |
| 102_5F145_day28_CadCl | 0.43 | 0.46 | 0.65 |
| 10_5M37_day1_CadCl | 0.55 | 0.47 | 0.61 |
| 11_5M38_day1_CadCl | 1.45 | 0.51 | 0.73 |
| 12_5M39_day1_CadCl | 0.41 | 0.65 | 0.36 |
| 40_5M40_day5_CadCl | 2.23 | 1.48 | 1.2 |
| 41_5M41_day5_CadCl | 1.89 | 1.67 | 0.97 |
| 42_5M42_day5_CadCl | 1.63 | 1.76 | 1.15 |
| 85_5M43_day28_CadCl | 0.67 | 0.46 | 0.72 |
| 86_5M44_day28_CadCl | 0.47 | 0.34 | 0.44 |
| 87_5M45_day28_CadCl | 0.57 | 0.53 | 0.58 |
| 28_6F146_day1_Dox | 1.58 | 2.9 | 1.66 |
| 29_6F147_day1_Dox | 1.09 | 1.56 | 1.12 |
| 30_6F148_day1_Dox | 1.15 | 2 | 1.36 |
| 56_6F149_day5_Dox | 1.97 | 2.28 | 1.51 |
| 57_6F150_day5_Dox | 1.36 | 1.99 | 1.97 |
| 58_6F151_day5_Dox | 2.36 | 2.9 | 3.17 |
| 103_6F171_day28_Dox | 0.8 | 0.58 | 1.07 |
| 104_6F172_day28_Dox | 0.69 | 0.61 | 1.52 |
| 105_6F173_day28_Dox | 1.73 | 0.63 | 1.26 |
| 13_6M46_day1_Dox | 0.86 | 0.68 | 0.56 |
| 14_6M47_day1_Dox | 0.77 | 0.78 | 0.64 |
| 15_6M48_day1_Dox | 0.56 | 0.64 | 0.66 |

| Sample | W64472_DB81_seg2 | W83813_DB81_seg27 | MUSCYCG1R_DB81_seg15-17 |
|---|---|---|---|
| 43_6M49_day5_Dox | 3 | 1.3 | 1.65 |
| 44_6M50_day5_Dox | 2.54 | 1.41 | 2 |
| 88_6M71_day28_Dox | 1.71 | 0.51 | 0.99 |
| 89_6M72_day28_Dox | 0.83 | 0.55 | 0.98 |
| 90_6M73_day28_Dox | 1.15 | 0.51 | 1.28 |
| 115_7F155_day1_ValpA | 0.77 | 0.98 | 0.86 |
| 116_7F156_day1_ValpA | 0.36 | 0.52 | 0.61 |
| 117_7F157_day1_ValpA | 0.47 | 0.74 | 0.8 |
| 121_7F158_day5_ValpA | 0.49 | 0.59 | 0.94 |
| 122_7F159_day5_ValpA | 0.57 | 0.55 | 0.77 |
| 123_7F160_day5_ValpA | 0.33 | 0.74 | 1.07 |
| 127_7F161_day28_ValpA | 0.47 | 0.69 | 0.89 |
| 128_7F162_day28_ValpA | 0.2 | 0.41 | 0.61 |
| 129_7F163_day28_ValpA | 0.24 | 0.58 | 0.68 |
| 112_7M55_day1_ValpA | 0.85 | 0.82 | 0.67 |
| 113_7M56_day1_ValpA | 0.73 | 0.64 | 0.42 |
| 114_7M57_day1_ValpA | 0.44 | 0.53 | 0.51 |
| 118_7M58_day5_ValpA | 0.99 | 0.68 | 0.8 |
| 119_7M59_day5_ValpA | 0.83 | 1.11 | 1.1 |
| 120_7M60_day5_ValpA | 0.93 | 0.93 | 1.03 |
| 124_7M61_day28_ValpA | 0.66 | 0.52 | 0.77 |
| 125_7M62_day28_ValpA | 0.55 | 0.65 | 0.71 |
| 126_7M63_day28_ValpA | 0.24 | 0.39 | 0.39 |
| 62_8F181_day28_Naive | 0.47 | 1.03 | 0.68 |
| 63_8F182_day28_Naive | 0.57 | 1.24 | 0.65 |
| 64_8F183_day28_Naive | 0.46 | 1.03 | 0.58 |
| 59_8M81_day28_Naive | 1.26 | 2 | 1.31 |
| 60_8M82_day28_Naive | 0.69 | 0.94 | 0.83 |
| 61_8M83_day28_Naive | 0.39 | 0.58 | 0.45 |

| Sample | MUSCYCG1R_DB81_seg19-20 | W33294_DB81_seg23 | W33294_DB81_seg44 |
|---|---|---|---|
| 68_1F101_day1_Saline | 0.74 | 0.78 | 0.8 |
| 69_1F102_day1_Saline | 0.84 | 0.65 | 0.96 |
| 70_1F103_day1_Saline | 0.64 | 0.8 | 0.72 |
| 74_1F104_day5_Saline | 1.28 | 0.86 | 0.65 |
| 75_1F105_day5_Saline | 0.75 | 0.97 | 0.88 |
| 109_1F107_day28_Saline | 0.57 | 1 | 0.88 |
| 110_1F108_day28_Saline | 0.5 | 0.79 | 0.59 |
| 111_1F109_day28_Saline | 0.79 | 1.24 | 1 |
| 65_1M1_day1_Saline | 0.44 | 0.69 | 0.64 |
| 66_1M2_day1_Saline | 0.43 | 0.3 | 0.69 |
| 67_1M3_day1_Saline | 0.36 | 0.46 | 0.53 |
| 71_1M4_day5_Saline | 0.87 | 0.78 | 1.69 |

| Sample | MUSCYCG1R_DB81_seg19-20 | W33294_DB81_seg23 | W33294_DB81_seg44 |
|---|---|---|---|
| 72_1M5_day5_Saline | 0.85 | 1.18 | 1 |
| 73_1M6_day5_Saline | 0.78 | 0.9 | 0.86 |
| 106_1M7_day28_Saline | 0.44 | 0.61 | 0.63 |
| 107_1M8_day28_Saline | 0.34 | 0.62 | 0.48 |
| 108_1M9_day28_Saline | 0.51 | 0.84 | 0.55 |
| 16_2F110_day1_Gent | 0.67 | 1 | 1.02 |
| 17_2F111_day1_Gent | 0.55 | 0.51 | 0.8 |
| 18_2F112_day1_Gent | 0.44 | 0.94 | 0.77 |
| 45_2F113_day5_Gent | 1.23 | 1.64 | 1.64 |
| 46_2F114_day5_Gent | 1.41 | 1.96 | 1.72 |
| 47_2F115_day5_Gent | 1.42 | 1.51 | 2.13 |
| 91_2F116_day28_Gent | 0.56 | 0.64 | 0.66 |
| 92_2F117_day28_Gent | 0.74 | 1.3 | 0.71 |
| 93_2F118_day28_Gent | 0.42 | 0.85 | 1.04 |
| 1_2M10_day1_Gent | 1.7 | 0.57 | 0.77 |
| 2_2M11_day1_Gent | 0.48 | 0.74 | 0.5 |
| 3_2M12_day1_Gent | 0.92 | 0.61 | 0.64 |
| 31_2M13_day5_Gent | 1.43 | 1.25 | 1.12 |
| 32_2M14_day5_Gent | 1.04 | 0.82 | 0.93 |
| 33_2M15_day5_Gent | 1 | 0.95 | 0.94 |
| 76_2M16_day28_Gent | 0.79 | 0.57 | 0.42 |
| 77_2M17_day28_Gent | 0.4 | 0.8 | 0.85 |
| 78_2M18_day28_Gent | 0.55 | 0.67 | 0.69 |
| 19_3F119_day1_Cis | 0.86 | 0.36 | 1 |
| 20_3F120_day1_Cis | 0.81 | 0.28 | 0.95 |
| 21_3F121_day1_Cis | 0.81 | 1.11 | 1.04 |
| 48_3F122_day5_Cis | 2.77 | 2.03 | 2.31 |
| 49_3F123_day5_Cis | 3.44 | 1.99 | 2.19 |
| 50_3F124_day5_Cis | 2.45 | 1.58 | 2.15 |
| 94_3F125_day28_Cis | 1.64 | 0.95 | 0.35 |
| 95_3F126_day28_Cis | 2.47 | 0.97 | 0.67 |
| 96_3F127_day28_Cis | 1.67 | 0.82 | 0.73 |
| 4_3M19_day1_Cis | 0.85 | 0.59 | 0.6 |
| 5_3M20_day1_Cis | 0.71 | 0.49 | 0.84 |
| 6_3M21_day1_Cis | 1.18 | 0.64 | 0.96 |
| 34_3M22_day5_Cis | 1.83 | 0.84 | 1.47 |
| 35_3M23_day5_Cis | 1.47 | 0.59 | 1.16 |
| 36_3M24_day5_Cis | 2.33 | 1.34 | 1.51 |
| 79_3M25_day28_Cis | 2.13 | 0.95 | 0.83 |
| 80_3M26_day28_Cis | 1.97 | 0.82 | 0.89 |
| 81_3M27_day28_Cis | 2.21 | 0.88 | 0.83 |
| 22_4F128_day1_Tob | 0.73 | 0.85 | 0.98 |
| 23_4F129_day1_Tob | 0.63 | 0.56 | 0.57 |

| Sample | MUSCYCG1R_DB81_seg19-20 | W33294_DB81_seg23 | W33294_DB81_seg44 |
|---|---|---|---|
| 24_4F130_day1_Tob | 0.58 | 0.59 | 0.62 |
| 51_4F131_day5_Tob | 1.42 | 2.16 | 2.01 |
| 52_4F132_day5_Tob | 1.31 | 2.37 | 2.06 |
| 53_4F133_day5_Tob | 1.2 | 1.2 | 1.72 |
| 97_4F134_day28_Tob | 0.45 | 0.91 | 0.95 |
| 98_4F135_day28_Tob | 0.62 | 1.16 | 0.75 |
| 99_4F136_day28_Tob | 0.58 | 0.91 | 0.7 |
| 7_4M28_day1_Tob | 0.72 | 0.52 | 0.78 |
| 8_4M29_day1_Tob | 0.84 | 0.91 | 0.88 |
| 9_4M30_day1_Tob | 0.63 | 0.35 | 0.46 |
| 37_4M31_day5_Tob | 0.72 | 0.61 | 0.48 |
| 38_4M32_day5_Tob | 0.72 | 0.81 | 0.69 |
| 39_4M33_day5_Tob | 1.53 | 1.25 | 1.27 |
| 82_4M34_day28_Tob | 0.49 | 1.02 | 0.75 |
| 83_4M35_day28_Tob | 0.51 | 0.78 | 0.86 |
| 84_4M36_day28_Tob | 0.6 | 0.73 | 0.86 |
| 25_5F137_day1_CadCl | 2.22 | 1.9 | 1.79 |
| 26_5F138_day1_CadCl | 1 | 1.74 | 1.39 |
| 27_5F139_day1_CadCl | 1.68 | 1.36 | 1.57 |
| 54_5F141_day5_CadCl | 1.21 | 1.76 | 2.05 |
| 55_5F142_day5_CadCl | 1.01 | 1.7 | 1.37 |
| 100_5F143_day28_CadCl | 0.54 | 0.94 | 0.88 |
| 101_5F144_day28_CadCl | 0.62 | 0.97 | 0.69 |
| 102_5F145_day28_CadCl | 0.56 | 0.85 | 0.7 |
| 10_5M37_day1_CadCl | 0.58 | 0.62 | 0.63 |
| 11_5M38_day1_CadCl | 1.03 | 0.31 | 0.26 |
| 12_5M39_day1_CadCl | 0.78 | 0.37 | 0.63 |
| 40_5M40_day5_CadCl | 1.22 | 1.41 | 1.32 |
| 41_5M41_day5_CadCl | 1.12 | 1.45 | 1.43 |
| 42_5M42_day5_CadCl | 1.04 | 1.03 | 1.42 |
| 85_5M43_day28_CadCl | 0.31 | 0.7 | 0.73 |
| 86_5M44_day28_CadCl | 0.75 | 0.65 | 0.53 |
| 87_5M45_day28_CadCl | 0.44 | 0.65 | 0.53 |
| 28_6F146_day1_Dox | 1.17 | 2.47 | 2.1 |
| 29_6F147_day1_Dox | 1.12 | 1.04 | 1.3 |
| 30_6F148_day1_Dox | 1.16 | 1.36 | 2.02 |
| 56_6F149_day5_Dox | 1.94 | 1.06 | 1.19 |
| 57_6F150_day5_Dox | 1.79 | 2.06 | 1.76 |
| 58_6F151_day5_Dox | 3.43 | 2.03 | 2.76 |
| 103_6F171_day28_Dox | 1.24 | 0.82 | 0.87 |
| 104_6F172_day28_Dox | 1.29 | 0.97 | 1.09 |
| 105_6F173_day28_Dox | 0.93 | 0.62 | 0.65 |
| 13_6M46_day1_Dox | 0.98 | 0.74 | 0.74 |

| Sample | MUSCYCG1R_DB81_seg19-20 | W33294_DB81_seg23 | W33294_DB81_seg44 |
|---|---|---|---|
| 14_6M47_day1_Dox | 1.04 | 0.61 | 1.01 |
| 15_6M48_day1_Dox | 0.95 | 0.82 | 0.83 |
| 43_6M49_day5_Dox | 1.7 | 1.25 | 1 |
| 44_6M50_day5_Dox | 1.66 | 1.06 | 0.95 |
| 88_6M71_day28_Dox | 0.88 | 0.96 | 0.45 |
| 89_6M72_day28_Dox | 1.33 | 1 | 0.77 |
| 90_6M73_day28_Dox | 1.26 | 0.82 | 0.66 |
| 115_7F155_day1_ValpA | 0.68 | 1.67 | 0.86 |
| 116_7F156_day1_ValpA | 0.43 | 0.84 | 0.72 |
| 117_7F157_day1_ValpA | 0.77 | 1.21 | 1.19 |
| 121_7F158_day5_ValpA | 0.6 | 1.14 | 1.06 |
| 122_7F159_day5_ValpA | 0.45 | 1.13 | 0.85 |
| 123_7F160_day5_ValpA | 1.11 | 1.24 | 0.79 |
| 127_7F161_day28_ValpA | 0.55 | 0.98 | 0.61 |
| 128_7F162_day28_ValpA | 0.62 | 0.89 | 0.74 |
| 129_7F163_day28_ValpA | 0.5 | 1.16 | 0.95 |
| 112_7M55_day1_ValpA | 0.63 | 1.32 | 1.01 |
| 113_7M56_day1_ValpA | 0.53 | 0.94 | 0.7 |
| 114_7M57_day1_ValpA | 0.44 | 0.92 | 0.69 |
| 118_7M58_day5_ValpA | 0.88 | 1.09 | 1.66 |
| 119_7M59_day5_ValpA | 1.1 | 1.32 | 1.33 |
| 120_7M60_day5_ValpA | 1.08 | 1.01 | 1.31 |
| 124_7M61_day28_ValpA | 0.56 | 0.52 | 0.75 |
| 125_7M62_day28_ValpA | 0.44 | 0.85 | 0.85 |
| 126_7M63_day28_ValpA | 0.33 | 0.88 | 0.34 |
| 62_8F181_day28_Naive | 0.55 | 0.94 | 0.73 |
| 63_8F182_day28_Naive | 0.7 | 1.02 | 0.76 |
| 64_8F183_day28_Naive | 0.79 | 0.82 | 0.77 |
| 59_8M81_day28_Naive | 1.24 | 1.65 | 2.01 |
| 60_8M82_day28_Naive | 0.86 | 0.85 | 1.12 |
| 61_8M83_day28_Naive | 0.44 | 0.79 | 0.39 |

| Sample | RATRAB13X_DB81_seg 15-17 | RATRAB13X_DB81 _seg22 | MMU09507_DB81 _seg15 |
|---|---|---|---|
| 68_1F101_day1_Saline | 0.7 | 1.29 | 1.03 |
| 69_1F102_day1_Saline | 0.59 | 0.81 | 1.13 |
| 70_1F103_day1_Saline | 0.68 | 0.66 | 0.51 |
| 74_1F104_day5_Saline | 0.64 | 0.82 | 0.42 |
| 75_1F105_day5_Saline | 0.77 | 1.04 | 0.01 |
| 109_1F107_day28_Saline | 1.08 | 0.69 | 0.67 |
| 110_1F108_day28_Saline | 0.95 | 0.61 | 0.2 |
| 111_1F109_day28_Saline | 1.03 | 0.97 | 0.33 |
| 65_1M1_day1_Saline | 0.48 | 1 | 0.14 |
| 66_1M2_day1_Saline | 0.55 | 0.65 | 0.24 |

| Sample | RATRAB13X_DB81_seg 15-17 | RATRAB13X_DB81 _seg22 | MMU09507_DB81 _seg15 |
|---|---|---|---|
| 67_1M3_day1_Saline | 0.48 | 0.44 | 0.24 |
| 71_1M4_day5_Saline | 1.11 | 0.86 | 1 |
| 72_1M5_day5_Saline | 1.02 | 1.06 | 0.37 |
| 73_1M6_day5_Saline | 0.78 | 0.75 | 0.25 |
| 106_1M7_day28_Saline | 0.58 | 0.68 | 0.23 |
| 107_1M8_day28_Saline | 0.5 | 0.51 | 0.17 |
| 108_1M9_day28_Saline | 0.7 | 0.8 | 0.19 |
| 16_2F110_day1_Gent | 0.9 | 0.98 | 0.41 |
| 17_2F111_day1_Gent | 0.63 | 0.71 | 0.43 |
| 18_2F112_day1_Gent | 0.55 | 0.51 | 0.23 |
| 45_2F113_day5_Gent | 1.68 | 2.16 | 0.78 |
| 46_2F114_day5_Gent | 1.5 | 2.11 | 0.62 |
| 47_2F115_day5_Gent | 1.59 | 1.91 | 1.13 |
| 91_2F116_day28_Gent | 0.84 | 0.68 | 1.38 |
| 92_2F117_day28_Gent | 0.9 | 0.56 | 0.96 |
| 93_2F118_day28_Gent | 0.88 | 0.57 | 0.7 |
| 1_2M10_day1_Gent | 0 | 0.8 | 0.56 |
| 2_2M11_day1_Gent | 1.16 | 0.99 | 0.32 |
| 3_2M12_day1_Gent | 0.82 | 0.6 | 0.37 |
| 31_2M13_day5_Gent | 1.25 | 1.25 | 0.71 |
| 32_2M14_day5_Gent | 0.76 | 0.77 | 0.41 |
| 33_2M15_day5_Gent | 1.35 | 1.16 | 0.68 |
| 76_2M16_day28_Gent | 0.77 | 0.91 | 0.82 |
| 77_2M17_day28_Gent | 0.76 | 0.88 | 0.59 |
| 78_2M18_day28_Gent | 1.22 | 0.58 | 0.84 |
| 19_3F119_day1_Cis | 0.82 | 0.88 | 0 |
| 20_3F120_day1_Cis | 1.11 | 0.87 | 0.44 |
| 21_3F121_day1_Cis | 1.1 | 1.02 | 0.52 |
| 48_3F122_day5_Cis | 1.37 | 1.71 | 1.96 |
| 49_3F123_day5_Cis | 1.53 | 1.92 | 2.32 |
| 50_3F124_day5_Cis | 1.53 | 1.99 | 1.14 |
| 94_3F125_day28_Cis | 1.33 | 0.61 | 8.1 |
| 95_3F126_day28_Cis | 1.27 | 0.65 | 6.58 |
| 96_3F127_day28_Cis | 1.21 | 0.89 | 5.53 |
| 4_3M19_day1_Cis | 0.73 | 0.65 | 0.35 |
| 5_3M20_day1_Cis | 0.84 | 0.66 | 0.19 |
| 6_3M21_day1_Cis | 0.98 | 1.11 | 0.5 |
| 34_3M22_day5_Cis | 1.8 | 1.53 | 2.07 |
| 35_3M23_day5_Cis | 1.03 | 1.2 | 0.57 |
| 36_3M24_day5_Cis | 1.77 | 1.28 | 3.87 |
| 79_3M25_day28_Cis | 1.08 | 0.66 | 8.05 |
| 80_3M26_day28_Cis | 0.95 | 0.76 | 2.96 |
| 81_3M27_day28_Cis | 1.28 | 0.65 | 5.45 |

| Sample | RATRAB13X_DB81_seg 15-17 | RATRAB13X_DB81 _seg22 | MMU09507_DB81 _seg15 |
|---|---|---|---|
| 22_4F128_day1_Tob | 1.02 | 0.41 | 0.35 |
| 23_4F129_day1_Tob | 0.77 | 0.71 | 0.3 |
| 24_4F130_day1_Tob | 0.87 | 0.78 | 0.22 |
| 51_4F131_day5_Tob | 1.71 | 2.49 | 1.04 |
| 52_4F132_day5_Tob | 1.44 | 2.11 | 0.99 |
| 53_4F133_day5_Tob | 1.23 | 1.57 | 0.92 |
| 97_4F134_day28_Tob | 1.28 | 0.88 | 0.84 |
| 98_4F135_day28_Tob | 1.01 | 0.63 | 0.52 |
| 99_4F136_day28_Tob | 0.73 | 0.88 | 0.85 |
| 7_4M28_day1_Tob | 0.78 | 0.82 | 0.34 |
| 8_4M29_day1_Tob | 0.98 | 0.95 | 0.7 |
| 9_4M30_day1_Tob | 0.91 | 0.82 | 0.56 |
| 37_4M31_day5_Tob | 0.75 | 0.68 | 0.17 |
| 38_4M32_day5_Tob | 1.04 | 0.81 | 0.28 |
| 39_4M33_day5_Tob | 1.32 | 1.26 | 0.27 |
| 82_4M34_day28_Tob | 0.93 | 0.67 | 0.66 |
| 83_4M35_day28_Tob | 1.05 | 0.8 | 0.97 |
| 84_4M36_day28_Tob | 0.94 | 0.62 | 0.68 |
| 25_5F137_day1_CadCl | 1.63 | 1.73 | 0.81 |
| 26_5F138_day1_CadCl | 1.8 | 1.83 | 0.42 |
| 27_5F139_day1_CadCl | 1.55 | 1.16 | 1.1 |
| 54_5F141_day5_CadCl | 1.81 | 1.56 | 0.87 |
| 55_5F142_day5_CadCl | 0.97 | 1.1 | 0.56 |
| 100_5F143_day28_CadCl | 0.83 | 0.7 | 0.33 |
| 101_5F144_day28_CadCl | 0.89 | 0.9 | 0.66 |
| 102_5F145_day28_CadCl | 0.82 | 0.46 | 1.58 |
| 10_5M37_day1_CadCl | 0.73 | 0.92 | 0.15 |
| 11_5M38_day1_CadCl | 0.92 | 1.29 | 0.32 |
| 12_5M39_day1_CadCl | 1.01 | 0.67 | 0.33 |
| 40_5M40_day5_CadCl | 1.18 | 1.65 | 0.33 |
| 41_5M41_day5_CadCl | 0.88 | 1.58 | 0.49 |
| 42_5M42_day5_CadCl | 1.07 | 1.21 | 0.38 |
| 85_5M43_day28_CadCl | 1.43 | 0.63 | 0.42 |
| 86_5M44_day28_CadCl | 0.8 | 0.87 | 0.45 |
| 87_5M45_day28_CadCl | 0.64 | 0.49 | 0.42 |
| 28_6F146_day1_Dox | 0.99 | 2.07 | 0.48 |
| 29_6F147_day1_Dox | 1.04 | 0.89 | 0.52 |
| 30_6F148_day1_Dox | 1.32 | 1.66 | 0.93 |
| 56_6F149_day5_Dox | 1.85 | 1.95 | 2.07 |
| 57_6F150_day5_Dox | 1.51 | 1.89 | 1.46 |
| 58_6F151_day5_Dox | 1.9 | 3.3 | 1.82 |
| 103_6F171_day28_Dox | 0.66 | 0.54 | 4.47 |
| 104_6F172_day28_Dox | 0.69 | 1.05 | 2.99 |

| Sample | RATRAB13X_DB81_seg 15-17 | RATRAB13X_DB81 _seg22 | MMU09507_DB81 _seg15 |
|---|---|---|---|
| 105_6F173_day28_Dox | 0.91 | 0.63 | 2.89 |
| 13_6M46_day1_Dox | 0.88 | 0.69 | 0.74 |
| 14_6M47_day1_Dox | 1.15 | 0.58 | 0.71 |
| 15_6M48_day1_Dox | 0.75 | 0.77 | 0.59 |
| 43_6M49_day5_Dox | 0.96 | 1.06 | 1.74 |
| 44_6M50_day5_Dox | 1.2 | 1.32 | 1.67 |
| 88_6M71_day28_Dox | 0.54 | 0.37 | 3.04 |
| 89_6M72_day28_Dox | 0.78 | 0.64 | 2.93 |
| 90_6M73_day28_Dox | 1.02 | 0.45 | 2.13 |
| 115_7F155_day1_ValpA | 1.28 | 1.05 | 0.48 |
| 116_7F156_day1_ValpA | 0.7 | 0.56 | 0.34 |
| 117_7F157_day1_ValpA | 0.82 | 0.87 | 0.27 |
| 121_7F158_day5_ValpA | 0.7 | 1.07 | 0.62 |
| 122_7F159_day5_ValpA | 0.91 | 0.78 | 0.31 |
| 123_7F160_day5_ValpA | 1.47 | 1.13 | 0.42 |
| 127_7F161_day28_ValpA | 0.73 | 0.71 | 0.23 |
| 128_7F162_day28_ValpA | 0.82 | 0.85 | 0.45 |
| 129_7F163_day28_ValpA | 0.44 | 0.74 | 0.24 |
| 112_7M55_day1_ValpA | 0.79 | 1.45 | 0.2 |
| 113_7M56_day1_ValpA | 0.7 | 0.86 | 0.2 |
| 114_7M57_day1_ValpA | 0.75 | 0.63 | 0.29 |
| 118_7M58_day5_ValpA | 1.38 | 0.95 | 0.26 |
| 119_7M59_day5_ValpA | 1.14 | 1.14 | 0.3 |
| 120_7M60_day5_ValpA | 1.45 | 1.1 | 0.24 |
| 124_7M61_day28_ValpA | 0.59 | 0.96 | 0.12 |
| 125_7M62_day28_ValpA | 0.77 | 0.75 | 0.11 |
| 126_7M63_day28_ValpA | 0.49 | 0.62 | 0.21 |
| 62_8F181_day28_Naive | 0.68 | 0.9 | 0.5 |
| 63_8F182_day28_Naive | 0.77 | 0.84 | 0.6 |
| 64_8F183_day28_Naive | 0.68 | 0.88 | 0.73 |
| 59_8M81_day28_Naive | 1.77 | 1.46 | 0.47 |
| 60_8M82_day28_Naive | 0.83 | 0.8 | 0.8 |
| 61_8M83_day28_Naive | 0.54 | 0.53 | 0.43 |

***EXAMPLE 3: Validation analysis of the selected markers***

To validate the optimal signature for classification of renal toxicity of a compound after five days of application a test of the signatory polynucleotide/gene expression in rat kidney samples consisting of tissues from rats exposed to three drug compounds and a single control group was carried out (Teva Pharmaceutical Industries, IL). The purpose of this analysis was to test the ability of the optimized signature to correctly predict the level of toxicity of the three drug compounds prior to the ability to demonstrate renal damage using histopathological examination of the rat kidney samples.

In this Example, the samples with known renal toxic effect (detailed in plates 1-3 described in Table 25 and in Table 9) are referred to as the "labeled samples", and the samples of the blind test (detailed in plates 4-7 described in Table 25), where no information on the renal toxic effect was available, are referred to as the "un-labeled samples". The experimental details and the analysis performed initially on the labeled samples, as described in Example 2, are referred to as the "discovery stage" of the study, while the details and analysis performed at the second stage of the study are referred to as the "validation stage".

The signature disclosed in Table 13 herein was further revised and refined, and then validated by testing the un-labeled rat kidney samples, consisting of tissues from rats exposed to three drug compounds (T1 and T2 presented in Figures 1-2 and compound T3, Riluzole (6-(trifluoromethoxy)benzothiazol-2-amine) and from a single control group.

qRT-PCR was performed on the labeled and un-labeled samples as described in section "RT Preparation and Real-Time qRT-PCR Analysis" hereinabove, using primers for 8 amplicons of 4 genes (a wild-type and a splice-variant amplicon for each gene disclosed in Table 13), as detailed in Example 2 hereinabove. New primers were designed for part of the transcripts, and new conditions for several qRT-PCR reactions were used, as described below.

Two equivalent modified signatures were constructed based on new qRT-PCR results obtained ffrom the test conducted with the labeled samples, which are listed in Tables 26 and 27 below. The modified signatures were then applied to the un-labeled samples and successfully predicted the level of toxicity of the three test compounds as well as of the control.

Table 23 provides the normalized qRT-PCR results for the amplicons used for both classifiers (shown in Tables 26 and 27) on the labeled and un-labeled samples. The measurements were normalized in two steps. First - according to the house-keeping-genes normalization factor, and second - each plate was normalized according to ratio of the intensity measurements from samples appearing in it and in the shuffled (normalization) plate (Table 25 provides the plates details and the section "Inter-Plate Normalization" provides details of the normalization process). The normalized values were further scaled by a factor, for ease of viewing. This data, as well as the additional gender column was used by the classifiers to reach the toxicity calls. Table 23 also provides the true label (Normal/Toxic) for the labeled samples.

Table 23: Normalized qRT-PCR results for amplicons used with labeled and un-labeled samples

| Sample | W41270_DB81_seg11 | H31883_DB71_seg13 | MUSCYCG1R_DB81_seg19-20 |
|---|---|---|---|
| 56_6F149_day5_Dox | 0.851 | 1.377 | 1.438 |
| 48_3F122_day5_Cis | 0.705 | 2.695 | 1.643 |
| 47_2F115_day5_Gent | 2.64 | 1.515 | 1.644 |
| 110_1F108_day28_Saline | 0.775 | 0.741 | 0.431 |
| 34_3M22_day5_Cis | 1.262 | 3.816 | 2.034 |
| 44_6M50_day5_Dox | 1.017 | 2.129 | 1.321 |
| 106_1M7_day28_Saline | 0.489 | 0.728 | 0.804 |

| Sample | W41270_DB81_seg11 | H31883_DB71_seg13 | MUSCYCG1R_DB81_ seg19-20 |
|---|---|---|---|
| 116_7F156_day1_ValpA | 1.134 | 1.026 | 2.289 |
| 59_8M81_day28_Naive | 1.117 | 0.965 | 1.232 |
| 124_7M61_day28_ValpA | 0.543 | 0.732 | 0.588 |
| 65_1M1_day1_Saline | 1.029 | 0.933 | 1.176 |
| 74_1F104_day5_Saline | 0.801 | 0.911 | 1.516 |
| 57_6F150_day5_Dox | 1.053 | 0.905 | 1.723 |
| 43_6M49_day5_Dox | 1.551 | 2.586 | 1.536 |
| 128_7F162_day28_ValpA | 0.95 | 0.774 | 1.731 |
| 120_7M60_day5_ValpA | 0.631 | 0.846 | 1.019 |
| 114_7M57_day1_ValpA | 0.939 | 1.488 | 1.581 |
| 37_4M31_day5_Tob | 0.559 | 0.863 | 0.99 |
| 126_7M63_day28_ValpA | 0.459 | 0.713 | 0.589 |
| 112_7M55_day1_ValpA | 1.405 | 1.506 | 1.842 |
| 73_1M6_day5_Saline | 0.413 | 0.966 | 0.768 |
| 62_8F181_day28_Naive | 0.931 | 1.262 | 0.933 |
| 123_7F160_day5_ValpA | 1.428 | 0.85 | 1.509 |
| 109_1F107_day28_Saline | 0.743 | 0.775 | 0.942 |
| 118_7M58_day5_ValpA | 1.002 | 0.975 | 1.372 |
| 68_1F101_day1_Saline | 1.725 | 1.074 | 1.424 |
| 117_7F157_day1_ValpA | 1.225 | 0.999 | 1.929 |
| 36_3M24_day5_Cis | 3.597 | 5.526 | 4.156 |
| 33_2M15_day5_Gent | 0.983 | 0.948 | 1.085 |
| 127_7F161_day28_ValpA | 1.044 | 0.872 | 1.171 |
| 75_1F105_day5_Saline | 0.044 | 0.838 | 1.262 |
| 52_4F132_day5_Tob | 2.483 | 0.855 | 1.589 |
| 50_3F124_day5_Cis | 1.404 | 3.771 | 4.947 |
| 63_8F182_day28_Naive | 0.809 | 0.95 | 0.772 |
| 38_4M32_day5_Tob | 0.802 | 1.237 | 0.968 |
| 72_1M5_day5_Saline | 0.654 | 0.963 | 1.245 |
| 67_1M3_day1_Saline | 0.805 | 1.2 | 0.903 |
| 35_3M23_day5_Cis | 0.843 | 1.509 | 0.981 |
| 51_4F131_day5_Tob | 1.529 | 0.988 | 1.6 |
| 32_2M14_day5_Gent | 0.954 | 1.09 | 0.993 |
| 122_7F159_day5_ValpA | 0.617 | 0.791 | 0.94 |
| 121_7F158_day5_ValpA | 1.258 | 0.694 | 1.425 |
| 119_7M59_day5_ValpA | 0.997 | 1.082 | 1.585 |
| 71_1M4_day5_Saline | 0.605 | 0.731 | 0.975 |
| 111_1F109_day28_Saline | 0.996 | 0.904 | 1.014 |
| 60_8M82_day28_Naive | 0.631 | 1.255 | 1.006 |
| 129_7F163_day28_ValpA | 0.587 | 0.57 | 1.037 |
| 69_1F102_day1_Saline | 0.573 | 0.793 | 1.449 |
| 108_1M9_day28_Saline | 0.831 | 0.812 | 0.634 |
| 46_2F114_day5_Gent | 1.104 | 0.78 | 0.955 |

| Sample | W41270_DB81_seg11 | H31883_DB71_seg13 | MUSCYCG1R_DB81_ seg19-20 |
|---|---|---|---|
| 125_7M62_day28_ValpA | 1.011 | 0.979 | 0.52 |
| 113_7M56_day1_ValpA | 0.547 | 0.721 | 0.637 |
| 45_2F113_day5_Gent | 1.699 | 1.142 | 1.442 |
| 61_8M83_day28_Naive | 0.411 | 0.637 | 1.209 |
| 53_4F133_day5_Tob | 2.317 | 0.858 | 1.424 |
| 107_1M8_day28_Saline | 0.562 | 0.719 | 0.642 |
| 115_7F155_day1_ValpA | 1.544 | 1.041 | 0.9 |
| 58_6F151_day5_Dox | 1.791 | 1.227 | 2.913 |
| 31_2M13_day5_Gent | 0.82 | 1.402 | 1.842 |
| 49_3F123_day5_Cis | 2.113 | 1.642 | 3.172 |
| 39_4M33_day5_Tob | 0.946 | 1.646 | 2.062 |
| 70_1F103_day1_Saline | 1.021 | 1.173 | 1.629 |
| 66_1M2_day1_Saline | 0.753 | 0.883 | 0.705 |
| 64_8F183_day28_Naive | 0.843 | 0.943 | 1.282 |
| 54_UK5F126_T1_Ds2_Day1 | 1.265 | 1.153 | 0.609 |
| 123_UK4F119_T1_Ds1_Day5 | 0.657 | 1.01 | 0.816 |
| 60_UK7F139_T2_Ds1_Day1 | 3.38 | 0.7 | 0.126 |
| 17_UK5M29_T1_Ds2_Day1 | 0.653 | 1.19 | 0.835 |
| 117_UK2F107_control_Day5 | 1.229 | 2.274 | 0.68 |
| 109_UK12M69_T3_Ds1_Day5 | 0.482 | 1.3 | 0.62 |
| 18_UK5M30_T1_Ds2_Day1 | 0.383 | 0.623 | 0.696 |
| 57_UK5F129_T1_Ds2_Day1 | 0.472 | 0.798 | 0.804 |
| 151_UK14F180_T3_Ds2_Day5 | 0.363 | 0.705 | 1.004 |
| 7_UK3M13_T1_Ds1_Day1 | 0.734 | 0.923 | 0.299 |
| 113_UK14M80_T3_Ds2_Day5 | 0.605 | 0.846 | 0.658 |
| 72_UK11F164_T3_Ds1_Day1 | 0.282 | 0.582 | 0.114 |
| 104_UK10M56_T2_Ds2_Day5 | 0.668 | 1.256 | 0.655 |
| 91_UK6M31_T1_Ds2_Day5 | 1.164 | 1.663 | 1.018 |
| 124_UK4F120_T1_Ds1_Day5 | 0.703 | 1.013 | 1.499 |
| 114_UK14M81_T3_Ds2_Day5 | 0.43 | 0.949 | 1.003 |
| 69_UK9F154_T2_Ds2_Day1 | 0.92 | 0.762 | 0.111 |
| 46_UK1F105_control_Day1 | 0.349 | 0.692 | 0.164 |
| 34_UK11M64_T3_Ds1_Day1 | 0.649 | 1.074 | 0.726 |
| 130_UK6F132_T1_Ds2_Day5 | 1 | 1.554 | 0.919 |
| 3_UK1M3_control_Day1 | 0.398 | 0.756 | 0.171 |
| 75_UK13F173_T3_Ds2_Day1 | 0.897 | 0.662 | 0.798 |
| 101_UK8M47_T2_Ds1_Day5 | 0.397 | 1.149 | 0.172 |
| 102_UK8M48_T2_Ds1_Day5 | 0.337 | 2.147 | 0.179 |
| 96_UK6M36_T1_Ds2_Day5 | 0.71 | 0.996 | 0.756 |
| 39_UK13M76_T3_Ds2_Day1 | 0.359 | 0.643 | 0.158 |
| 19_UK7M37_T2_Ds1_Day1 | 0.623 | 0.7 | 0.51 |
| 144_UK12F167_T3_Ds1_Day5 | 1.957 | 1.301 | 0.638 |
| 2_UK1M2_control_Day1 | 0.342 | 0.812 | 0.222 |

| Sample | W41270_DB81_seg11 | H31883_DB71_seg13 | MUSCYCG1R_DB81_ seg19-20 |
|---|---|---|---|
| 52_UK3F118_T1_Ds1_Day1 | 0.566 | 0.706 | 2.31 |
| 31_UK11M61_T3_Ds1_Day1 | 0.64 | 0.919 | 0.96 |
| 129_UK6F131_T1_Ds2_Day5 | 0.009 | 1.36 | 0.359 |
| 23_UK7M41_T2_Ds1_Day1 | 0.481 | 0.88 | 0.938 |
| 43_UK1F102_control_Day1 | 0.406 | 2.051 | 0.328 |
| 81_UK2M8_control_Day5 | 0.686 | 1.318 | 0.252 |
| 80_UK2M7_control_Day5 | 0.788 | 1.981 | 0.273 |
| 70_UK11F161_T3_Ds1_Day1 | 0.457 | 0.764 | 0.232 |
| 16_UK5M28_T1_Ds2_Day1 | 0.78 | 0.517 | 0.385 |
| 79_UK13F178_T3_Ds2_Day1 | 0.49 | 0.802 | 0.92 |
| 50_UK3F116_T1_Ds1_Day1 | 0.66 | 0.913 | 1.49 |
| 112_UK12M72_T3_Ds1_Day5 | 0.722 | 1.65 | 0.649 |
| 146_UK12F169_T3_Ds1_Day5 | 1.807 | 1.003 | 0.716 |
| 140_UK10F156_T2_Ds2_Day5 | 1.186 | 1.386 | 0.345 |
| 143_UK10F160_T2_Ds2_Day5 | 0.308 | 0.676 | 0.169 |
| 10_UK3M16_T1_Ds1_Day1 | 0.181 | 0.757 | 0.291 |
| 94_UK6M34_T1_Ds2_Day5 | 0.391 | 1.037 | 0.871 |
| 147_UK12F170_T3_Ds1_Day5 | 0.483 | 1.338 | 1.035 |
| 71_UK11F163_T3_Ds1_Day1 | 0.575 | 0.611 | 0.183 |
| 122_UK2F112_control_Day5 | 0.592 | 1.127 | 0.159 |
| 47_UK1F106_control_Day1 | 0.405 | 0.823 | 0.196 |
| 15_UK5M27_T1_Ds2_Day1 | 0.755 | 0.906 | 0.76 |
| 97_UK8M43_T2_Ds1_Day5 | 0.233 | 1.884 | 0.107 |
| 106_UK10M59_T2_Ds2_Day5 | 0.497 | 1.01 | 0.513 |
| 118_UK2F108_control_Day5 | 0.911 | 2.171 | 0.279 |
| 148_UK12F171_T3_Ds1_Day5 | 0.899 | 0.576 | 1.226 |
| 35_UK11M65_T3_Ds1_Day1 | 0.374 | 0.663 | 1.044 |
| 21_UK7M39_T2_Ds1_Day1 | 0.662 | 0.67 | 0.555 |
| 38_UK13M75_T3_Ds2_Day1 | 0.347 | 0.969 | 0.203 |
| 55_UK5F127_T1_Ds2_Day1 | 0.885 | 0.849 | 0.739 |
| 100_UK8M46_T2_Ds1_Day5 | 0.493 | 1.539 | 0.113 |
| 67_UK9F152_T2_Ds2_Day1 | 1.747 | 0.832 | 0.211 |
| 131_UK6F133_T1_Ds2_Day5 | 0.964 | 1.84 | 0.852 |
| 142_UK10F159_T2_Ds2_Day5 | 0.711 | 1.128 | 0.148 |
| 128_UK4F124_T1_Ds1_Day5 | 1.527 | 1.373 | 1.598 |
| 95_UK6M35_T1_Ds2_Day5 | 0.713 | 1.348 | 0.761 |
| 8_UK3M14_T1_Ds1_Day1 | 0.396 | 0.615 | 0.271 |
| 138_UK8F147_T2_Ds1_Day5 | 0.626 | 1.163 | 1.399 |
| 41_UK13M78_T3_Ds2_Day1 | 0.174 | 0.632 | 0.275 |
| 76_UK13F175_T3_Ds2_Day1 | 0.912 | 1.119 | 2.153 |
| 152_UK14F181_T3_Ds2_Day5 | 0.762 | 0.942 | 1.567 |
| 86_UK4M20_T1_Ds1_Day5 | 0.473 | 1.353 | 0.183 |
| 59_UK7F137_T2_Ds1_Day1 | 0.473 | 0.613 | 0.115 |

| Sample | W41270_DB81_seg11 | H31883_DB71_seg13 | MUSCYCG1R_DB81_seg19-20 |
|---|---|---|---|
| 111_UK12M71_T3_Ds1_Day5 | 0.832 | 1.082 | 0.857 |
| 145_UK12F168_T3_Ds1_Day5 | 1.015 | 1.358 | 0.625 |
| 28_UK9M52_T2_Ds2_Day1 | 1.016 | 0.773 | 0.531 |
| 108_UK12M67_T3_Ds1_Day5 | 0.699 | 1.585 | 0.525 |
| 11_UK3M17_T1_Ds1_Day1 | 0.198 | 0.73 | 0.376 |
| 24_UK7M42_T2_Ds1_Day1 | 0.76 | 0.695 | 0.859 |
| 42_UK1F101_control_Day1 | 0.415 | 0.976 | 0.276 |
| 29_UK9M53_T2_Ds2_Day1 | 0.424 | 0.635 | 0.505 |
| 61_UK7F140_T2_Ds1_Day1 | 0.474 | 0.464 | 0.124 |
| 20_UK7M38_T2_Ds1_Day1 | 0.876 | 0.951 | 0.888 |
| 141_UK10F158_T2_Ds2_Day5 | 1.237 | 1.161 | 0.185 |
| 87_UK4M21_T1_Ds1_Day5 | 0.702 | 1.266 | 0.149 |
| 150_UK14F179_T3_Ds2_Day5 | 1.066 | 0.718 | 0.875 |
| 136_UK8F144_T2_Ds1_Day5 | 1.149 | 1.329 | 1.006 |
| 93_UK6M33_T1_Ds2_Day5 | 1.825 | 2.423 | 1.197 |
| 85_UK4M19_T1_Ds1_Day5 | 0.356 | 1.783 | 0.214 |
| 4_UK1M4_control_Day1 | 0.617 | 0.668 | 0.192 |
| 126_UK4F122_T1_Ds1_Day5 | 1.325 | 1.477 | 3.532 |
| 36_UK11M66_T3_Ds1_Day1 | 0.468 | 0.569 | 1.137 |
| 74_UK11F166_T3_Ds1_Day1 | 0.465 | 1.366 | 0.132 |
| 14_UK5M26_T1_Ds2_Day1 | 0.462 | 0.696 | 0.478 |
| 119_UK2F109_control_Day5 | 0.994 | 1.083 | 0.162 |
| 25_UK9M49_T2_Ds2_Day1 | 0.589 | 0.722 | 0.585 |
| 103_UK10M55_T2_Ds2_Day5 | 0.857 | 1.146 | 0.997 |
| 27_UK9M51_T2_Ds2_Day1 | 0.545 | 0.618 | 0.475 |
| 26_UK9M50_T2_Ds2_Day1 | 0.528 | 0.999 | 0.661 |
| 44_UK1F103_control_Day1 | 0.563 | 0.692 | 0.161 |
| 62_UK7F141_T2_Ds1_Day1 | 0.959 | 0.536 | 0.267 |
| 92_UK6M32_T1_Ds2_Day5 | 0.453 | 1.13 | 0.631 |
| 12_UK3M18_T1_Ds1_Day1 | 0.231 | 0.524 | 0.135 |
| 5_UK1M5_control_Day1 | 0.3 | 1.146 | 0.184 |
| 153_UK14F182_T3_Ds2_Day5 | 1.119 | 1.058 | 1.708 |
| 56_UK5F128_T1_Ds2_Day1 | 0.413 | 0.611 | 0.738 |
| 58_UK5F130_T1_Ds2_Day1 | 0.762 | 0.707 | 0.652 |
| 132_UK6F134_T1_Ds2_Day5 | 1.254 | 2.468 | 1.238 |
| 49_UK3F114_T1_Ds1_Day1 | 0.758 | 0.783 | 1.436 |
| 90_UK4M24_T1_Ds1_Day5 | 0.657 | 1.097 | 0.296 |
| 64_UK9F149_T2_Ds2_Day1 | 0.384 | 0.602 | 0.197 |
| 110_UK12M70_T3_Ds1_Day5 | 0.495 | 0.946 | 0.398 |
| 127_UK4F123_T1_Ds1_Day5 | 1.014 | 2.104 | 3.397 |
| 121_UK2F111_control_Day5 | 0.394 | 0.882 | 0.222 |
| 83_UK2M10_control_Day5 | 0.719 | 1.033 | 0.103 |
| 98_UK8M44_T2_Ds1_Day5 | 0.321 | 1.66 | 0.148 |

| Sample | W41270_DB81_seg11 | H31883_DB71_seg13 | MUSCYCG1R_DB81_ seg19-20 |
|---|---|---|---|
| 99_UK8M45_T2_Ds1_Day5 | 0.477 | 1.079 | 0.127 |
| 107_UK10M60_T2_Ds2_Day5 | 0.788 | 1.057 | 0.746 |
| 73_UK11F165_T3_Ds1_Day1 | 0.344 | 0.637 | 0.134 |
| 40_UK13M77_T3_Ds2_Day1 | 0.418 | 0.923 | 0.29 |
| 89_UK4M23_T1_Ds1_Day5 | 1.11 | 1.249 | 0.186 |
| 134_UK6F136_T1_Ds2_Day5 | 1.111 | 1.684 | 0.528 |
| 133_UK6F135_T1_Ds2_Day5 | 1.126 | 1.672 | 1.583 |
| 1_UK1M1_control_Day1 | 0.578 | 0.554 | 0.148 |
| 30_UK9M54_T2_Ds2_Day1 | 0.577 | 0.522 | 0.458 |
| 82_UK2M9_control_Day5 | 0.269 | 1.398 | 0.157 |
| 37_UK13M73_T3_Ds2_Day1 | 0.359 | 0.767 | 0.152 |
| 45_UK1F104_control_Day1 | 0.754 | 0.709 | 0.197 |
| 105_UK10M57_T2_Ds2_Day5 | 0.457 | 0.942 | 0.686 |
| 135_UK8F143_T2_Ds1_Day5 | 0.83 | 0.936 | 0.736 |
| 33_UK11M63_T3_Ds1_Day1 | 0.568 | 0.949 | 0.767 |
| 77_UK13F176_T3_Ds2_Day1 | 0.667 | 1.13 | 1.45 |
| 63_UK7F142_T2_Ds1_Day1 | 0.518 | 0.646 | 0.157 |
| 48_UK3F113_T1_Ds1_Day1 | 0.646 | 0.912 | 1.195 |
| 65_UK9F150_T2_Ds2_Day1 | 1.729 | 1.006 | 0.167 |
| 84_UK2M11_control_Day5 | 0.152 | 0.819 | 0.093 |
| 125_UK4F121_T1_Ds1_Day5 | 1.337 | 1.458 | 1.317 |
| 139_UK8F148_T2_Ds1_Day5 | 1.524 | 0.978 | 1.386 |
| 9_UK3M15_T1_Ds1_Day1 | 0.47 | 0.87 | 0.249 |
| 115_UK14M82_T3_Ds2_Day5 | 0.472 | 1.087 | 1.024 |
| 137_UK8F145_T2_Ds1_Day5 | 1.111 | 1.439 | 1.293 |
| 13_UK5M25_T1_Ds2_Day1 | 0.644 | 0.517 | 0.654 |
| 66_UK9F151_T2_Ds2_Day1 | 1.125 | 1.079 | 0.192 |
| 88_UK4M22_T1_Ds1_Day5 | 0.638 | 1.23 | 0.189 |
| 149_UK12F172_T3_Ds1_Day5 | 1.093 | 1.094 | 1.413 |
| 32_UK11M62_T3_Ds1_Day1 | 0.551 | 0.7 | 0.441 |
| 68_UK9F153_T2_Ds2_Day1 | 0.594 | 0.84 | 0.14 |
| 22_UK7M40_T2_Ds1_Day1 | 0.823 | 0.629 | 0.47 |
| 6_UK1M6_control_Day1 | 0.245 | 0.66 | 0.402 |
| 120_UK2F110_control_Day5 | 0.456 | 1.399 | 0.233 |
| 78_UK13F177_T3_Ds2_Day1 | 0.956 | 1.47 | 2.313 |
| 53_UK5F125_T1_Ds2_Day1 | 1.034 | 0.867 | 1.261 |
| 116_UK14M83_T3_Ds2_Day5 | 0.766 | 2.34 | 1.119 |
| 51_UK3F117_T1_Ds1_Day1 | 1.082 | 0.957 | 1.891 |
| 154_UK14F183_T3_Ds2_Day5 | 0.944 | 1.259 | 1.475 |
| 16_2F110_day1_Gent | 1.361 | 0.772 | 0.842 |
| 42_5M42_day5_CadCl | 1.067 | 1.057 | 1.036 |
| 102_5F145_day28_CadCl | 1.138 | 0.785 | 0.769 |
| 9_4M30_day1_Tob | 1.601 | 1.137 | 1.582 |

| Sample | W41270_DB81_seg11 | H31883_DB71_seg13 | MUSCYCG1R_DB81_ seg19-20 |
|---|---|---|---|
| 97_4F134_day28_Tob | 1.189 | 0.624 | 0.713 |
| 105_6F173_day28_Dox | 1.263 | 1.328 | 0.985 |
| 79_3M25_day28_Cis | 4.741 | 4.41 | 3.509 |
| 78_2M18_day28_Gent | 1.614 | 0.853 | 0.863 |
| 54_5F141_day5_CadCl | 2.101 | 1.154 | 2.018 |
| 5_3M20_day1_Cis | 0.679 | 0.668 | 0.806 |
| 77_2M17_day28_Gent | 1.342 | 0.861 | 1.072 |
| 82_4M34_day28_Tob | 1.217 | 0.324 | 0.76 |
| 12_5M39_day1_CadCl | 0.727 | 0.919 | 1.224 |
| 26_5F138_day1_CadCl | 1.183 | 1.005 | 1.097 |
| 94_3F125_day28_Cis | 4.104 | 2.839 | 3.443 |
| 93_2F118_day28_Gent | 1.315 | 0.553 | 0.617 |
| 17_2F111_day1_Gent | 1.037 | 1.118 | 1.155 |
| 92_2F117_day28_Gent | 1.194 | 0.817 | 0.865 |
| 89_6M72_day28_Dox | 0.73 | 1.54 | 2.342 |
| 55_5F142_day5_CadCl | 1.109 | 0.786 | 1.292 |
| 15_6M48_day1_Dox | 0.824 | 0.993 | 1.387 |
| 3_2M12_day1_Gent | 0.904 | 0.987 | 1.361 |
| 84_4M36_day28_Tob | 1.099 | 0.69 | 1.087 |
| 87_5M45_day28_CadCl | 0.529 | 0.652 | 1.356 |
| 25_5F137_day1_CadCl | 1.265 | 1.446 | 2.129 |
| 98_4F135_day28_Tob | 1.267 | 0.528 | 0.889 |
| 41_5M41_day5_CadCl | 0.769 | 1.132 | 3.613 |
| 83_4M35_day28_Tob | 1.373 | 0.551 | 1.268 |
| 2_2M11_day1_Gent | 1.08 | 1.115 | 2.011 |
| 13_6M46_day1_Dox | 1.286 | 1.283 | 1.761 |
| 24_4F130_day1_Tob | 1.084 | 1.104 | 0.967 |
| 101_5F144_day28_CadCl | 0.772 | 0.712 | 1.068 |
| 21_3F121_day1_Cis | 0.759 | 0.853 | 1.514 |
| 88_6M71_day28_Dox | 2.381 | 1.434 | 1.437 |
| 100_5F143_day28_CadCl | 0.578 | 0.681 | 0.68 |
| 10_5M37_day1_CadCl | 0.993 | 1.239 | 2.276 |
| 7_4M28_day1_Tob | 0.577 | 1.132 | 1.506 |
| 18_2F112_day1_Gent | 2.078 | 0.84 | 1.387 |
| 28_6F146_day1_Dox | 1.43 | 1.41 | 2.256 |
| 8_4M29_day1_Tob | 0.949 | 0.912 | 1.173 |
| 91_2F116_day28_Gent | 1.188 | 0.554 | 0.729 |
| 22_4F128_day1_Tob | 0.809 | 0.563 | 1.025 |
| 86_5M44_day28_CadCl | 0.654 | 0.849 | 2.128 |
| 14_6M47_day1_Dox | 1.148 | 1.042 | 1.516 |
| 40_5M40_day5_CadCl | 1.211 | 1.051 | 2.866 |
| 96_3F127_day28_Cis | 2.053 | 2.398 | 1.813 |
| 30_6F148_day1_Dox | 1.501 | 1.46 | 1.627 |

| Sample | W41270_DB81_seg11 | H31883_DB71_seg13 | MUSCYCG1R_DB81_seg19-20 |
|---|---|---|---|
| 103_6F171_day28_Dox | 1.44 | 1.642 | 1.991 |
| 19_3F119_day1_Cis | 1.331 | 1.416 | 1.492 |
| 85_5M43_day28_CadCl | 0.522 | 0.578 | 0.932 |
| 80_3M26_day28_Cis | 1.904 | 2.653 | 3.094 |
| 27_5F139_day1_CadCl | 0.983 | 1.632 | 1.856 |
| 76_2M16_day28_Gent | 1.941 | 0.842 | 1.661 |
| 90_6M73_day28_Dox | 0.652 | 1.611 | 2.105 |
| 29_6F147_day1_Dox | 0.935 | 1.116 | 1.551 |
| 23_4F129_day1_Tob | 0.983 | 0.661 | 0.987 |
| 1_2M10_day1_Gent | 0.85 | 1.053 | 2.861 |
| 20_3F120_day1_Cis | 1.029 | 2.092 | 3.259 |
| 104_6F172_day28_Dox | 0.787 | 1.408 | 1.779 |
| 6_3M21_day1_Cis | 1.224 | 2.559 | 2.003 |
| 95_3F126_day28_Cis | 4.516 | 5.592 | 7.02 |
| 81_3M27_day28_Cis | 3.866 | 3.563 | 3.473 |
| 99_4F136_day28_Tob | 1.798 | 0.807 | 0.903 |
| 4_3M19_day1_Cis | 0.496 | 1.134 | 1.277 |
| 11_5M38_day1_CadCl | 1 | 1.143 | 3.981 |

| Sample | W64472_DB81_seg2 | W83813_DB81_seg27 | AI045075_DB71_seg6 | Gender | Label |
|---|---|---|---|---|---|
| 56_6F149_day5_Dox | 2.03 | 1.442 | 1.248 | F | Toxic |
| 48_3F122_day5_Cis | 1.795 | 1.716 | 1.804 | F | Toxic |
| 47_2F115_day5_Gent | 1.733 | 1.688 | 1.337 | F | Toxic |
| 110_1F108_day28_Saline | 0.865 | 1.019 | 0.724 | F | Normal |
| 34_3M22_day5_Cis | 3.64 | 0.857 | 1.683 | M | Toxic |
| 44_6M50_day5_Dox | 2.924 | 0.986 | 1.348 | M | Toxic |
| 106_1M7_day28_Saline | 0.775 | 0.535 | 0.544 | M | Normal |
| 116_7F156_day1_ValpA | 1.082 | 1.179 | 1.006 | F | Normal |
| 59_8M81_day28_Naive | 2.184 | 1.679 | 1.27 | M | Normal |
| 124_7M61_day28_ValpA | 1.085 | 0.493 | 0.696 | M | Normal |
| 65_1M1_day1_Saline | 1.57 | 1.166 | 1.202 | M | Normal |
| 74_1F104_day5_Saline | 1.013 | 0.889 | 0.535 | F | Normal |
| 57_6F150_day5_Dox | 1.441 | 1.177 | 1.319 | F | Toxic |
| 43_6M49_day5_Dox | 3.4 | 1.095 | 1.844 | M | Toxic |
| 128_7F162_day28_ValpA | 0.788 | 1.254 | 0.906 | F | Normal |
| 120_7M60_day5_ValpA | 0.92 | 0.778 | 1.017 | M | Normal |
| 114_7M57_day1_ValpA | 1.123 | 0.912 | 1.436 | M | Normal |
| 37_4M31_day5_Tob | 1.193 | 0.589 | 1.327 | M | Toxic |
| 126_7M63_day28_ValpA | 0.965 | 0.46 | 0.669 | M | Normal |
| 112_7M55_day1_ValpA | 1.887 | 1.495 | 2.189 | M | Normal |

| Sample | W64472_ DB81_seg2 | W83813_ DB81_seg27 | AI045075_ DB71_seg6 | Gender | Label |
|---|---|---|---|---|---|
| 73_1M6_day5_Saline | 0.801 | 0.629 | 0.518 | M | Normal |
| 62_8F181_day28_Naive | 1.493 | 1.594 | 1.044 | F | Normal |
| 123_7F160_day5_ValpA | 1.226 | 1.17 | 1.145 | F | Normal |
| 109_1F107_day28_Saline | 0.718 | 0.642 | 0.982 | F | Normal |
| 118_7M58_day5_ValpA | 1.169 | 0.876 | 0.997 | M | Normal |
| 68_1F101_day1_Saline | 1.041 | 1.508 | 0.905 | F | Normal |
| 117_7F157_day1_ValpA | 0.995 | 1.13 | 0.983 | F | Normal |
| 36_3M24_day5_Cis | 6.414 | 1.461 | 4.298 | M | Toxic |
| 33_2M15_day5_Gent | 2.048 | 1.042 | 1.374 | M | Toxic |
| 127_7F161_day28_ValpA | 1.51 | 0.875 | 0.822 | F | Normal |
| 75_1F105_day5_Saline | 1.154 | 1.395 | 0.845 | F | Normal |
| 52_4F132_day5_Tob | 1.871 | 1.262 | 1.644 | F | Toxic |
| 50_3F124_day5_Cis | 1.976 | 1.366 | 1.503 | F | Toxic |
| 63_8F182_day28_Naive | 0.899 | 1.367 | 0.915 | F | Normal |
| 38_4M32_day5_Tob | 1.449 | 0.892 | 1.614 | M | Toxic |
| 72_1M5_day5_Saline | 0.943 | 1.059 | 0.763 | M | Normal |
| 67_1M3_day1_Saline | 3.263 | 1.107 | 1.751 | M | Normal |
| 35_3M23_day5_Cis | 1.448 | 0.758 | 0.93 | M | Toxic |
| 51_4F131_day5_Tob | 1.063 | 1.058 | 1.367 | F | Toxic |
| 32_2M14_day5_Gent | 1.962 | 0.702 | 1.402 | M | Toxic |
| 122_7F159_day5_ValpA | 0.702 | 0.926 | 0.883 | F | Normal |
| 121_7F158_day5_ValpA | 0.812 | 1.146 | 1.052 | F | Normal |
| 119_7M59_day5_ValpA | 0.894 | 0.992 | 0.877 | M | Normal |
| 71_1M4_day5_Saline | 1.141 | 0.796 | 0.887 | M | Normal |
| 111_1F109_day28_Saline | 0.864 | 1.01 | 1.061 | F | Normal |
| 60_8M82_day28_Naive | 2.082 | 1.127 | 1.021 | M | Normal |
| 129_7F163_day28_ValpA | 0.478 | 0.761 | 0.77 | F | Normal |
| 69_1F102_day1_Saline | 0.5 | 0.819 | 0.476 | F | Normal |
| 108_1M9_day28_Saline | 1.037 | 0.889 | 0.829 | M | Normal |
| 46_2F114_day5_Gent | 1.427 | 1.441 | 1.778 | F | Toxic |
| 125_7M62_day28_ValpA | 0.991 | 1.208 | 0.719 | M | Normal |
| 113_7M56_day1_ValpA | 1.092 | 0.818 | 1.098 | M | Normal |
| 45_2F113_day5_Gent | 1.403 | 1.395 | 1.636 | F | Toxic |
| 61_8M83_day28_Naive | 0.965 | 0.736 | 0.548 | M | Normal |
| 53_4F133_day5_Tob | 1.785 | 0.942 | 2.402 | F | Toxic |
| 107_1M8_day28_Saline | 0.596 | 0.284 | 0.393 | M | Normal |
| 115_7F155_day1_ValpA | 1.596 | 1.366 | 1.522 | F | Normal |
| 58_6F151_day5_Dox | 2.561 | 1.399 | 1.18 | F | Toxic |
| 31_2M13_day5_Gent | 1.53 | 0.76 | 1.256 | M | Toxic |
| 49_3F123_day5_Cis | 1.265 | 1.265 | 1.09 | F | Toxic |
| 39_4M33_day5_Tob | 1.447 | 1.117 | 1.224 | M | Toxic |
| 70_1F103_day1_Saline | 0.876 | 1.169 | 0.753 | F | Normal |
| 66_1M2_day1_Saline | 0.897 | 0.674 | 0.582 | M | Normal |

| Sample | W64472_ DB81_seg2 | W83813_ DB81_seg27 | AI045075_ DB71_seg6 | Gender | Label |
|---|---|---|---|---|---|
| 64_8F183_day28_Naive | 0.683 | 1.224 | 0.868 | F | Normal |
| 54_UK5F126_T1_Ds2_Day1 | 0.944 | 1.186 | 0.483 | F | |
| 123_UK4F119_T1_Ds1_Day5 | 0.571 | 0.795 | 0.692 | F | |
| 60_UK7F139_T2_Ds1_Day1 | 1.863 | 0.305 | 1.308 | F | |
| 17_UK5M29_T1_Ds2_Day1 | 1.213 | 0.984 | 0.759 | M | |
| 117_UK2F107_control_Day5 | 4.213 | 0.933 | 3.974 | F | |
| 109_UK12M69_T3_Ds1_Day5 | 0.852 | 0.754 | 0.615 | M | |
| 18_UK5M30_T1_Ds2_Day1 | 0.661 | 0.34 | 0.448 | M | |
| 57_UK5F129_T1_Ds2_Day1 | 0.559 | 0.689 | 0.547 | F | |
| 151_UK14F180_T3_Ds2_Day5 | 0.368 | 0.729 | 0.37 | F | |
| 7_UK3M13_T1_Ds1_Day1 | 1.118 | 0.216 | 0.843 | M | |
| 113_UK14M80_T3_Ds2_Day5 | 0.779 | 0.728 | 1.451 | M | |
| 72_UK11F164_T3_Ds1_Day1 | 0.483 | 0.322 | 0.354 | F | |
| 104_UK10M56_T2_Ds2_Day5 | 0.731 | 0.601 | 0.609 | M | |
| 91_UK6M31_T1_Ds2_Day5 | 0.803 | 1.092 | 0.797 | M | |
| 124_UK4F120_T1_Ds1_Day5 | 0.528 | 0.885 | 0.627 | F | |
| 114_UK14M81_T3_Ds2_Day5 | 0.656 | 0.933 | 0.53 | M | |
| 69_UK9F154_T2_Ds2_Day1 | 0.804 | 0.201 | 1.43 | F | |
| 46_UK1F105_control_Day1 | 0.734 | 0.542 | 0.825 | F | |
| 34_UK11M64_T3_Ds1_Day1 | 1.31 | 0.663 | 1.103 | M | |
| 130_UK6F132_T1_Ds2_Day5 | 0.863 | 1.125 | 0.766 | F | |
| 3_UK1M3_control_Day1 | 0.883 | 0.267 | 1.049 | M | |
| 75_UK13F173_T3_Ds2_Day1 | 0.659 | 0.67 | 0.444 | F | |
| 101_UK8M47_T2_Ds1_Day5 | 0.929 | 0.248 | 1.016 | M | |
| 102_UK8M48_T2_Ds1_Day5 | 0.996 | 0.203 | 1.11 | M | |
| 96_UK6M36_T1_Ds2_Day5 | 0.982 | 0.885 | 1.01 | M | |
| 39_UK13M76_T3_Ds2_Day1 | 1.566 | 0.137 | 1.146 | M | |
| 19_UK7M37_T2_Ds1_Day1 | 0.632 | 0.666 | 0.744 | M | |
| 144_UK12F167_T3_Ds1_Day5 | 1.176 | 1.146 | 1.082 | F | |
| 2_UK1M2_control_Day1 | 0.91 | 0.216 | 0.885 | M | |
| 52_UK3F118_T1_Ds1_Day1 | 0.683 | 0.843 | 0.636 | F | |
| 31_UK11M61_T3_Ds1_Day1 | 1.296 | 0.497 | 0.678 | M | |
| 129_UK6F131_T1_Ds2_Day5 | 0.554 | 0.55 | 0.629 | F | |
| 23_UK7M41_T2_Ds1_Day1 | 1.305 | 0.84 | 1.159 | M | |
| 43_UK1F102_control_Day1 | 1.43 | 0.612 | 0.851 | F | |
| 81_UK2M8_control_Day5 | 1.317 | 0.25 | 2.054 | M | |
| 80_UK2M7_control_Day5 | 2.363 | 0.112 | 1.078 | M | |
| 70_UK11F161_T3_Ds1_Day1 | 0.52 | 0.402 | 0.596 | F | |
| 16_UK5M28_T1_Ds2_Day1 | 0.462 | 0.56 | 0.396 | M | |
| 79_UK13F178_T3_Ds2_Day1 | 0.602 | 0.761 | 0.527 | F | |
| 50_UK3F116_T1_Ds1_Day1 | 0.49 | 0.926 | 0.572 | F | |
| 112_UK12M72_T3_Ds1_Day5 | 1.034 | 1.388 | 1.102 | M | |
| 146_UK12F169_T3_Ds1_Day5 | 0.822 | 0.659 | 1.032 | F | |

| Sample | W64472_ DB81_seg2 | W83813_ DB81_seg27 | AI045075_ DB71_seg6 | Gender | Label |
|---|---|---|---|---|---|
| 140_UK10F156_T2_Ds2_Day5 | 1.178 | 0.555 | 1.842 | F | |
| 143_UK10F160_T2_Ds2_Day5 | 0.485 | 0.354 | 0.637 | F | |
| 10_UK3M16_T1_Ds1_Day1 | 0.778 | 0.155 | 0.574 | M | |
| 94_UK6M34_T1_Ds2_Day5 | 0.527 | 0.654 | 0.539 | M | |
| 147_UK12F170_T3_Ds1_Day5 | 0.479 | 0.701 | 0.501 | F | |
| 71_UK11F163_T3_Ds1_Day1 | 0.859 | 0.394 | 0.85 | F | |
| 122_UK2F112_control_Day5 | 1.05 | 0.522 | 1.214 | F | |
| 47_UK1F106_control_Day1 | 0.495 | 0.276 | 0.605 | F | |
| 15_UK5M27_T1_Ds2_Day1 | 1.326 | 0.889 | 0.932 | M | |
| 97_UK8M43_T2_Ds1_Day5 | 1.445 | 0.11 | 1.566 | M | |
| 106_UK10M59_T2_Ds2_Day5 | 0.899 | 0.615 | 1.338 | M | |
| 118_UK2F108_control_Day5 | 1.325 | 0.362 | 1.597 | F | |
| 148_UK12F171_T3_Ds1_Day5 | 0.546 | 1.155 | 0.727 | F | |
| 35_UK11M65_T3_Ds1_Day1 | 0.567 | 0.898 | 0.918 | M | |
| 21_UK7M39_T2_Ds1_Day1 | 0.801 | 0.955 | 0.98 | M | |
| 38_UK13M75_T3_Ds2_Day1 | 2.291 | 0.276 | 1.479 | M | |
| 55_UK5F127_T1_Ds2_Day1 | 0.898 | 0.861 | 1.067 | F | |
| 100_UK8M46_T2_Ds1_Day5 | 1.42 | 0.209 | 1.449 | M | |
| 67_UK9F152_T2_Ds2_Day1 | 1.104 | 0.289 | 2.008 | F | |
| 131_UK6F133_T1_Ds2_Day5 | 0.989 | 1.025 | 0.935 | F | |
| 142_UK10F159_T2_Ds2_Day5 | 0.554 | 0.269 | 0.799 | F | |
| 128_UK4F124_T1_Ds1_Day5 | 0.745 | 1.511 | 1.162 | F | |
| 95_UK6M35_T1_Ds2_Day5 | 1.181 | 1.077 | 1.161 | M | |
| 8_UK3M14_T1_Ds1_Day1 | 1.501 | 0.178 | 1.13 | M | |
| 138_UK8F147_T2_Ds1_Day5 | 0.464 | 0.865 | 0.771 | F | |
| 41_UK13M78_T3_Ds2_Day1 | 1.042 | 0.164 | 0.566 | M | |
| 76_UK13F175_T3_Ds2_Day1 | 0.847 | 0.874 | 1.132 | F | |
| 152_UK14F181_T3_Ds2_Day5 | 0.587 | 0.81 | 0.666 | F | |
| 86_UK4M20_T1_Ds1_Day5 | 1.835 | 0.125 | 2.031 | M | |
| 59_UK7F137_T2_Ds1_Day1 | 0.492 | 0.183 | 0.54 | F | |
| 111_UK12M71_T3_Ds1_Day5 | 0.88 | 1.054 | 1.285 | M | |
| 145_UK12F168_T3_Ds1_Day5 | 0.601 | 0.791 | 0.928 | F | |
| 28_UK9M52_T2_Ds2_Day1 | 1.462 | 0.773 | 1.006 | M | |
| 108_UK12M67_T3_Ds1_Day5 | 0.8 | 0.818 | 1.133 | M | |
| 11_UK3M17_T1_Ds1_Day1 | 0.981 | 0.143 | 0.805 | M | |
| 24_UK7M42_T2_Ds1_Day1 | 0.791 | 0.798 | 0.709 | M | |
| 42_UK1F101_control_Day1 | 0.569 | 0.323 | 0.674 | F | |
| 29_UK9M53_T2_Ds2_Day1 | 0.692 | 0.595 | 0.729 | M | |
| 61_UK7F140_T2_Ds1_Day1 | 0.468 | 0.213 | 0.419 | F | |
| 20_UK7M38_T2_Ds1_Day1 | 1.076 | 0.931 | 1.059 | M | |
| 141_UK10F158_T2_Ds2_Day5 | 1.029 | 0.354 | 3.003 | F | |
| 87_UK4M21_T1_Ds1_Day5 | 0.991 | 0.208 | 0.904 | M | |
| 150_UK14F179_T3_Ds2_Day5 | 0.623 | 0.711 | 0.977 | F | |

| Sample | W64472_ DB81_seg2 | W83813_ DB81_seg27 | AI045075_ DB71_seg6 | Gender | Label |
|---|---|---|---|---|---|
| 136_UK8F144_T2_Ds1_Day5 | 0.532 | 0.922 | 1.02 | F | |
| 93_UK6M33_T1_Ds2_Day5 | 2.203 | 1.236 | 1.793 | M | |
| 85_UK4M19_T1_Ds1_Day5 | 0.687 | 0.165 | 0.937 | M | |
| 4_UK1M4_control_Day1 | 1.352 | 0.235 | 1.318 | M | |
| 126_UK4F122_T1_Ds1_Day5 | 0.945 | 1.328 | 1.367 | F | |
| 36_UK11M66_T3_Ds1_Day1 | 1.106 | 0.512 | 0.845 | M | |
| 74_UK11F166_T3_Ds1_Day1 | 1.064 | 0.356 | 1.252 | F | |
| 14_UK5M26_T1_Ds2_Day1 | 0.785 | 0.656 | 0.544 | M | |
| 119_UK2F109_control_Day5 | 0.907 | 0.515 | 1.067 | F | |
| 25_UK9M49_T2_Ds2_Day1 | 0.656 | 0.741 | 0.541 | M | |
| 103_UK10M55_T2_Ds2_Day5 | 1.446 | 1.022 | 0.831 | M | |
| 27_UK9M51_T2_Ds2_Day1 | 1.23 | 0.777 | 1.015 | M | |
| 26_UK9M50_T2_Ds2_Day1 | 0.866 | 0.725 | 0.588 | M | |
| 44_UK1F103_control_Day1 | 0.524 | 0.2 | 0.503 | F | |
| 62_UK7F141_T2_Ds1_Day1 | 1.072 | 0.29 | 0.836 | F | |
| 92_UK6M32_T1_Ds2_Day5 | 0.784 | 0.596 | 0.73 | M | |
| 12_UK3M18_T1_Ds1_Day1 | 0.439 | 0.169 | 0.4 | M | |
| 5_UK1M5_control_Day1 | 1.411 | 0.299 | 1.289 | M | |
| 153_UK14F182_T3_Ds2_Day5 | 1.24 | 1.599 | 1.436 | F | |
| 56_UK5F128_T1_Ds2_Day1 | 0.415 | 0.549 | 0.514 | F | |
| 58_UK5F130_T1_Ds2_Day1 | 0.502 | 0.725 | 0.639 | F | |
| 132_UK6F134_T1_Ds2_Day5 | 1.223 | 0.454 | 1.478 | F | |
| 49_UK3F114_T1_Ds1_Day1 | 0.752 | 0.825 | 0.49 | F | |
| 90_UK4M24_T1_Ds1_Day5 | 0.902 | 0.207 | 0.783 | M | |
| 64_UK9F149_T2_Ds2_Day1 | 0.741 | 0.365 | 0.857 | F | |
| 110_UK12M70_T3_Ds1_Day5 | 0.499 | 0.72 | 1.175 | M | |
| 127_UK4F123_T1_Ds1_Day5 | 0.938 | 1.593 | 1.089 | F | |
| 121_UK2F111_control_Day5 | 0.609 | 0.276 | 0.702 | F | |
| 83_UK2M10_control_Day5 | 1.034 | 0.412 | 1.069 | M | |
| 98_UK8M44_T2_Ds1_Day5 | 1.258 | 0.27 | 1.516 | M | |
| 99_UK8M45_T2_Ds1_Day5 | 1.447 | 0.299 | 1.237 | M | |
| 107_UK10M60_T2_Ds2_Day5 | 0.623 | 0.962 | 0.83 | M | |
| 73_UK11F165_T3_Ds1_Day1 | 0.507 | 0.205 | 0.37 | F | |
| 40_UK13M77_T3_Ds2_Day1 | 1.597 | 0.218 | 1.063 | M | |
| 89_UK4M23_T1_Ds1_Day5 | 1.35 | 0.2 | 1.364 | M | |
| 134_UK6F136_T1_Ds2_Day5 | 0.652 | 1.074 | 0.868 | F | |
| 133_UK6F135_T1_Ds2_Day5 | 1.056 | 1.674 | 1.487 | F | |
| 1_UK1M1_control_Day1 | 0.783 | 0.17 | 0.633 | M | |
| 30_UK9M54_T2_Ds2_Day1 | 0.837 | 0.614 | 0.471 | M | |
| 82_UK2M9_control_Day5 | 2.621 | 0.25 | 1.879 | M | |
| 37_UK13M73_T3_Ds2_Day1 | 1.35 | 0.211 | 1.117 | M | |
| 45_UK1F104_control_Day1 | 0.897 | 0.381 | 1.062 | F | |
| 105_UK10M57_T2_Ds2_Day5 | 1.049 | 0.626 | 0.878 | M | |

| Sample | W64472_ DB81_seg2 | W83813_ DB81_seg27 | AI045075_ DB71_seg6 | Gender | Label |
|---|---|---|---|---|---|
| 135_UK8F143_T2_Ds1_Day5 | 0.616 | 0.961 | 1.923 | F | |
| 33_UK11M63_T3_Ds1_Day1 | 1.844 | 0.989 | 1.025 | M | |
| 77_UK13F176_T3_Ds2_Day1 | 1.043 | 0.929 | 1.211 | F | |
| 63_UK7F142_T2_Ds1_Day1 | 0.969 | 0.387 | 0.49 | F | |
| 48_UK3F113_T1_Ds1_Day1 | 0.548 | 0.917 | 0.762 | F | |
| 65_UK9F150_T2_Ds2_Day1 | 1.73 | 0.32 | 3.044 | F | |
| 84_UK2M11_control_Day5 | 0.629 | 0.162 | 0.645 | M | |
| 125_UK4F121_T1_Ds1_Day5 | 0.606 | 0.893 | 0.831 | F | |
| 139_UK8F148_T2_Ds1_Day5 | 0.572 | 0.824 | 1.232 | F | |
| 9_UK3M15_T1_Ds1_Day1 | 1.502 | 0.245 | 1.416 | M | |
| 115_UK14M82_T3_Ds2_Day5 | 0.494 | 1.103 | 0.765 | M | |
| 137_UK8F145_T2_Ds1_Day5 | 0.773 | 1.303 | 0.94 | F | |
| 13_UK5M25_T1_Ds2_Day1 | 0.592 | 0.473 | 0.541 | M | |
| 66_UK9F151_T2_Ds2_Day1 | 1.211 | 0.444 | 2.152 | F | |
| 88_UK4M22_T1_Ds1_Day5 | 0.684 | 0.323 | 0.717 | M | |
| 149_UK12F172_T3_Ds1_Day5 | 0.677 | 1.096 | 1.021 | F | |
| 32_UK11M62_T3_Ds1_Day1 | 0.808 | 0.789 | 0.713 | M | |
| 68_UK9F153_T2_Ds2_Day1 | 0.666 | 0.254 | 0.71 | F | |
| 22_UK7M40_T2_Ds1_Day1 | 1.477 | 1.182 | 1.253 | M | |
| 6_UK1M6_control_Day1 | 0.749 | 0.127 | 0.476 | M | |
| 120_UK2F110_control_Day5 | 0.898 | 0.34 | 0.976 | F | |
| 78_UK13F177_T3_Ds2_Day1 | 1.227 | 1.454 | 0.575 | F | |
| 53_UK5F125_T1_Ds2_Day1 | 1.028 | 1.045 | 0.965 | F | |
| 116_UK14M83_T3_Ds2_Day5 | 3.181 | 0.911 | 1.037 | M | |
| 51_UK3F117_T1_Ds1_Day1 | 1.047 | 1.186 | 0.981 | F | |
| 154_UK14F183_T3_Ds2_Day5 | 0.704 | 1.018 | 0.58 | F | |
| 16_2F110_day1_Gent | 0.763 | 0.963 | 1.251 | F | |
| 42_5M42_day5_CadCl | 1.337 | 1.104 | 1.083 | M | |
| 102_5F145_day28_CadCl | 0.414 | 0.259 | 0.728 | F | |
| 9_4M30_day1_Tob | 1.43 | 1.126 | 2.04 | M | |
| 97_4F134_day28_Tob | 1.073 | 0.876 | 0.959 | F | |
| 105_6F173_day28_Dox | 2.157 | 0.734 | 0.964 | F | |
| 79_3M25_day28_Cis | 5.917 | 1.587 | 4.504 | M | |
| 78_2M18_day28_Gent | 1.55 | 0.852 | 1.734 | M | |
| 54_5F141_day5_CadCl | 2.282 | 1.782 | 1.866 | F | |
| 5_3M20_day1_Cis | 0.815 | 0.799 | 0.495 | M | |
| 77_2M17_day28_Gent | 1.353 | 0.857 | 3.06 | M | |
| 82_4M34_day28_Tob | 0.61 | 0.39 | 0.679 | M | |
| 12_5M39_day1_CadCl | 1.499 | 1.034 | 0.951 | M | |
| 26_5F138_day1_CadCl | 1.059 | 1.194 | 1.133 | F | |
| 94_3F125_day28_Cis | 2.504 | 1.749 | 2.301 | F | |
| 93_2F118_day28_Gent | 0.806 | 0.421 | 0.967 | F | |
| 17_2F111_day1_Gent | 0.963 | 0.98 | 0.889 | F | |

| Sample | W64472_ DB81_seg2 | W83813_ DB81_seg27 | AI045075_ DB71_seg6 | Gender | Label |
|---|---|---|---|---|---|
| 92_2F117_day28_Gent | 1.079 | 0.441 | 2.632 | F | |
| 89_6M72_day28_Dox | 1.613 | 0.686 | 0.669 | M | |
| 55_5F142_day5_CadCl | 0.854 | 0.906 | 0.805 | F | |
| 15_6M48_day1_Dox | 1.26 | 0.956 | 1.257 | M | |
| 3_2M12_day1_Gent | 1.567 | 1.02 | 1.072 | M | |
| 84_4M36_day28_Tob | 1.379 | 0.832 | 1.273 | M | |
| 87_5M45_day28_CadCl | 0.781 | 0.531 | 0.672 | M | |
| 25_5F137_day1_CadCl | 1.155 | 1.772 | 1.64 | F | |
| 98_4F135_day28_Tob | 1.144 | 0.621 | 1.021 | F | |
| 41_5M41_day5_CadCl | 1.662 | 1.157 | 1.409 | M | |
| 83_4M35_day28_Tob | 1.281 | 0.803 | 1.397 | M | |
| 2_2M11_day1_Gent | 1.869 | 1.409 | 1.848 | M | |
| 13_6M46_day1_Dox | 2.478 | 0.936 | 1.622 | M | |
| 24_4F130_day1_Tob | 1.5 | 1.153 | 5.307 | F | |
| 101_5F144_day28_CadCl | 0.827 | 0.783 | 0.975 | F | |
| 21_3F121_day1_Cis | 0.81 | 0.844 | 0.631 | F | |
| 88_6M71_day28_Dox | 3.226 | 0.442 | 1.434 | M | |
| 100_5F143_day28_CadCl | 1.098 | 1.033 | 1.58 | F | |
| 10_5M37_day1_CadCl | 1.622 | 1.027 | 1.108 | M | |
| 7_4M28_day1_Tob | 1.083 | 0.999 | 0.825 | M | |
| 18_2F112_day1_Gent | 1.408 | 0.898 | 2.053 | F | |
| 28_6F146_day1_Dox | 1.548 | 2.054 | 1.631 | F | |
| 8_4M29_day1_Tob | 1.619 | 1.049 | 1.223 | M | |
| 91_2F116_day28_Gent | 1.164 | 0.434 | 1.78 | F | |
| 22_4F128_day1_Tob | 0.73 | 0.799 | 1.195 | F | |
| 86_5M44_day28_CadCl | 1.107 | 0.817 | 0.836 | M | |
| 14_6M47_day1_Dox | 1.45 | 0.964 | 1.454 | M | |
| 40_5M40_day5_CadCl | 1.675 | 1.671 | 2.393 | M | |
| 96_3F127_day28_Cis | 2.643 | 0.786 | 1.555 | F | |
| 30_6F148_day1_Dox | 1.367 | 1.752 | 1.268 | F | |
| 103_6F171_day28_Dox | 1.462 | 0.719 | 0.653 | F | |
| 19_3F119_day1_Cis | 1.261 | 1.209 | 0.788 | F | |
| 85_5M43_day28_CadCl | 0.807 | 0.633 | 0.333 | M | |
| 80_3M26_day28_Cis | 3.371 | 0.786 | 1.428 | M | |
| 27_5F139_day1_CadCl | 1.372 | 1.894 | 1.263 | F | |
| 76_2M16_day28_Gent | 1.245 | 0.756 | 1.469 | M | |
| 90_6M73_day28_Dox | 1.604 | 0.586 | 0.785 | M | |
| 29_6F147_day1_Dox | 0.977 | 1.003 | 0.741 | F | |
| 23_4F129_day1_Tob | 1.166 | 0.982 | 1.623 | F | |
| 1_2M10_day1_Gent | 1.48 | 1.161 | 1.221 | M | |
| 20_3F120_day1_Cis | 1.096 | 1.345 | 0.899 | F | |
| 104_6F172_day28_Dox | 1.023 | 0.955 | 0.54 | F | |
| 6_3M21_day1_Cis | 2.455 | 1.09 | 1.64 | M | |

| Sample | W64472_ DB81_seg2 | W83813_ DB81_seg27 | AI045075_ DB71_seg6 | Gender | Label |
|---|---|---|---|---|---|
| 95_3F126_day28_Cis | 5.783 | 1.864 | 3.336 | F | |
| 81_3M27_day28_Cis | 3.008 | 1.371 | 1.753 | M | |
| 99_4F136_day28_Tob | 1.118 | 0.987 | 1.478 | F | |
| 4_3M19_day1_Cis | 1.176 | 0.902 | 0.811 | M | |
| 11_5M38_day1_CadCl | 1.512 | 0.957 | 1.069 | M | |

**Experimental Design**

Three compounds (T1, T2, T3) and a control were administered to male and female rats at various time points by oral gavage: 3 treatments (T1, T2, T3) were given in 2 doses (Ds1, Ds2) for two periods (one day and 5 days). Each group consisted of 6 male and 6 female rats. The additional groups consisted of 6 males and 6 female rats treated with control compound for a single day and for five days, as described in Table 24.

Table 24: Un-labeled samples groups and doses

| Groups | Test Items | Dose (mg/kg) | Route of administration | Days of treatment | Animals per group |
|---|---|---|---|---|---|
| 1M,F | Control - 1% methylcellulose | - | Oral gavage | 1 | 6 |
| 2M,F | | - | Oral gavage | 5 | 6 |
| 3M,F | T1 | 10 | Oral gavage | 1 | 6 |
| 4M,F | | 10 | Oral gavage | 5 | 6 |
| 5M,F | | 40 | Oral gavage | 1 | 6 |
| 6M,F | | 40 | Oral gavage | 5 | 6 |
| 7M,F | T2 | 25 | Oral gavage | 1 | 6 |
| 8M,F | | 25 | Oral gavage | 5 | 6 |
| 9M,F | | 50 | Oral gavage | 1 | 6 |
| 10M,F | | 50 | Oral gavage | 5 | 6 |
| 11M,F | T3 | 5 | Oral gavage | 1 | 6 |
| 12M,F | | 5 | Oral gavage | 5 | 6 |
| 13M,F | | 10 | Oral gavage | 1 | 6 |
| 14M,F | | 10 | Oral gavage | 5 | 6 |

T2 is a drug known to cause renal damage after 7 days of treatment at the 50 mg/kg dose. The chemical structure of T2 is shown in Figure 2. T2 "no observed effect level" (NOEL, i.e., the maximal dose at which no toxic effect is visible) is 15 mg/kg. Kidneys obtained from rats receiving 50 mg/kg of T2, demonstrated direct cortical tubular toxicity. Figures 3-5 demonstrate the results of histopathological examination of the treated kidneys after 7 days (Figures 3B, 4B and 5B), or of the control animals (Figures 3A, 4A and 5A). As demonstrated in Figures 3B, 4B and 5B the changes were multifocal, consisting of tubular basophilia, indicating regeneration following necrosis.

Figure 3 demonstrates histological section of the kidney, magnified x 200. Figure 3A represents a histological section of the kidney of a control animal. Normal aspects of cortical tubules (eosinophilic cytoplasm) are marked by arrows. Figure 3B represents a histological section of the kidney of a 50 mg/kg treated animal. Basophilic tubules in the Cortex, indicating post-necrotic regeneration are marked by arrows.

Figure 4 demonstrates histological section of the kidney, magnified x 400. Figure 4A represents a histological section of the kidney of a control animal. Normal aspects of cortical tubules (eosinophilic cytoplasm) are marked by arrows. Figure 4B represents a histological section of the kidney of a 50 mg/kg treated animal. Basophilic tubules in the Cortex, indicating post-necrotic regeneration are marked by arrows.

Figure 5 demonstrates histological section of the kidney, magnified x 600. Figure 5A represents a histological section of the kidney of a control animal. Normal aspects of cortical tubules (eosinophilic cytoplasm) are marked by arrows. Figure 5B represents a histological section of the kidney of a 50 mg/kg treated animal. Basophilic tubules in the Cortex, indicating post-necrotic regeneration are marked by arrows.

T1 is another drug which shows renal damage, mainly in females, after 28 days of treatment at the 30 mg/kg dose. The chemical structure of T1 is shown in Figure 1.

T3 is Riluzole (6-(trifluoromethoxy)benzothiazol-2-amine), which shows no renal damage after up to 35 days of treatment, and therefore was used as a negative control.

At autopsy, both kidneys of each animal were removed and each kidney was cross sectioned. Half of each kidney was sent to RNA extraction and analysis.

RNA Isolation from Kidney Tissues

RNA was isolated as described previously. RNA samples which did not meet the conventional RNA quality criteria including A260: A280 ratio, A260:A230 ratio; and RNA integrity as detected in agarose gel, were omitted from further processing.

RT Preparation and Real-Time qRT-PCR Analysis

RT was prepared and qRT-PCR performed and analyzed as described hereinabove. qRT-PCR was performed for the four genes comprising the signature of the discovery stage described in Table 13. Two amplicons were tested for each gene - one representing the wild-type transcript, and another representing an alternative splice variant. Each amplicon was measured using the labeled samples described in Table 9 herein, as well as the un-labeled samples. Samples from both groups (labeled and un-labeled) were tested in seven 96 well plates as described in Table 25. In order to correct possible run to run variations, the following controls were added: 1. several samples were repeated in all 96 wells plates; 2. control shuffled plate was designed which combined samples from all seven test plates.

Table 25 presents plates set up in a validation stage. The names of the un-labeled samples comprise of the rat's group, the rats ID number starts with UK (stand for unknown), the toxicant it was exposed to (T1, T2 or T3), the dose given Ds1 or Ds2 and day of treatment.

Table 25: Plates set up in the validation stage

**Plate 1**

| | | | | |
|---|---|---|---|---|
| 1 | Gentamycin | 1 | day 1 toxicants M | 1_2M10_day1_Gent |
| 2 | Gentamycin | 1 | | 2_2M11_day1_Gent |
| 3 | Gentamycin | 1 | | 3_2M12_day1_Gent |
| 4 | Cisplatin | 1 | | 4_3M19_day1_Cis |
| 5 | Cisplatin | 1 | | 5_3M20_day1_Cis |
| 6 | Cisplatin | 1 | | 6_3M21_day1_Cis |
| 7 | Tobramycin | 1 | | 7_4M28_day1_Tob |
| 8 | Tobramycin | 1 | | 8_4M29_day1_Tob |
| 9 | Tobramycin | 1 | | 9_4M30_day1_Tob |
| 10 | Cadmium chloride | 1 | | 10_5M37_day1_CadCl |
| 11 | Cadmium chloride | 1 | | 11_5M38_day1_CadCl |
| 12 | Cadmium chloride | 1 | | 12_5M39_day1_CadCl |
| 13 | Doxorubicin | 1 | | 13_6M46_day1_Dox |
| 14 | Doxorubicin | 1 | | 14_6M47_day1_Dox |
| 15 | Doxorubicin | 1 | | 15_6M48_day1_Dox |
| 16 | Valproic acid | 1 | | 112_7M55_day1_ValpA |
| 17 | Valproic acid | 1 | | 113_7M56_day1_ValpA |
| 18 | Valproic acid | 1 | | 114_7M57_day1_ValpA |
| 19 | Gentamycin | 1 | day 1 toxicants F | 16_2F110_day1_Gent |
| 20 | Gentamycin | 1 | | 17_2F111_day1_Gent |
| 21 | Gentamycin | 1 | | 18_2F112_day1_Gent |
| 22 | Cisplatin | 1 | | 19_3F119_day1_Cis |
| 23 | Cisplatin | 1 | | 20_3F120_day1_Cis |
| 24 | Cisplatin | 1 | | 21_3F121_day1_Cis |
| 25 | Tobramycin | 1 | | 22_4F128_day1_Tob |
| 26 | Tobramycin | 1 | | 23_4F129_day1_Tob |
| 27 | Tobramycin | 1 | | 24_4F130_day1_Tob |
| 28 | Cadmium chloride | 1 | | 25_5F137_day1_CadCl |
| 29 | Cadmium chloride | 1 | | 26_5F138_day1_CadCl |
| 30 | Cadmium chloride | 1 | | 27_5F139_day1_CadCl |
| 31 | Doxorubicin | 1 | | 28_6F146_day1_Dox |

| | | | | |
|---|---|---|---|---|
| 32 | Doxorubicin | 1 | | 29_6F147_day1_Dox |
| 33 | Doxorubicin | 1 | | 30_6F148_day1_Dox |
| 34 | Valproic acid | 1 | | 115_7F155_day1_ValpA |
| 35 | Valproic acid | 1 | | 116_7F156_day1_ValpA |
| 36 | Valproic acid | 1 | | 117_7F157_day1_ValpA |
| 37 | Naïve | 28 | Controls All days | 59_8M81_day28_Naïve |
| 38 | Naïve | 28 | | 60_8M82_day28_Naïve |
| 39 | Naïve | 28 | | 61_8M83_day28_Naïve |
| 40 | Naïve | 28 | | 62_8F181_day28_Naïve |
| 41 | Naïve | 28 | | 63_8F182_day28_Naïve |
| 42 | Naïve | 28 | | 64_8F183_day28_Naïve |
| 43 | Control/Saline | 1 | | 65_1M1_day1_Saline |
| 44 | Control/Saline | 1 | | 66_1M2_day1_Saline |
| 45 | Control/Saline | 1 | | 67_1M3_day1_Saline |
| 46 | Control/Saline | 1 | | 68_1F101_day1_Saline |
| 47 | Control/Saline | 1 | | 69_1F102_day1_Saline |
| 48 | Control/Saline | 1 | | 70_1F103_day1_Saline |

**Plate 2**

| | | | | |
|---|---|---|---|---|
| 1 | Gentamycin | 5 | day 5 toxicants M | 31_2M13_day5_Gent |
| 2 | Gentamycin | 5 | | 32_2M14_day5_Gent |
| 3 | Gentamycin | 5 | | 33_2M15_day5_Gent |
| 4 | Cisplatin | 5 | | 34_3M22_day5_Cis |
| 5 | Cisplatin | 5 | | 35_3M23_day5_Cis |
| 6 | Cisplatin | 5 | | 36_3M24_day5_Cis |
| 7 | Tobramycin | 5 | | 37_4M31_day5_Tob |
| 8 | Tobramycin | 5 | | 38_4M32_day5_Tob |
| 9 | Tobramycin | 5 | | 39_4M33_day5_Tob |
| 10 | Cadmium chloride | 5 | | 40_5M40_day5_CadCl |
| 11 | Cadmium chloride | 5 | | 41_5M41_day5_CadCl |
| 12 | Cadmium chloride | 5 | | 42_5M42_day5_CadCl |
| 13 | Doxorubicin | 5 | | 43_6M49_day5_Dox |
| 14 | Doxorubicin | 5 | | 44_6M50_day5_Dox |
| 15 | Valproic acid | 5 | | 118_7M58_day5_ValpA |
| 16 | Valproic acid | 5 | | 119_7M59_day5_ValpA |

| | | | | |
|---|---|---|---|---|
| 17 | Valproic acid | 5 | | 120_7M60_day5_ValpA |
| 18 | Gentamycin | 5 | day 5 toxicants F | 45_2F113_day5_Gent |
| 19 | Gentamycin | 5 | | 46_2F114_day5_Gent |
| 20 | Gentamycin | 5 | | 47_2F115_day5_Gent |
| 21 | Cisplatin | 5 | | 48_3F122_day5_Cis |
| 22 | Cisplatin | 5 | | 49_3F123_day5_Cis |
| 23 | Cisplatin | 5 | | 50_3F124_day5_Cis |
| 24 | Tobramycin | 5 | | 51_4F131_day5_Tob |
| 25 | Tobramycin | 5 | | 52_4F132_day5_Tob |
| 26 | Tobramycin | 5 | | 53_4F133_day5_Tob |
| 27 | Cadmium chloride | 5 | | 54_5F141_day5_CadCl |
| 28 | Cadmium chloride | 5 | | 55_5F142_day5_CadCl |
| 29 | Doxorubicin | 5 | | 56_6F149_day5_Dox |
| 30 | Doxorubicin | 5 | | 57_6F150_day5_Dox |
| 31 | Doxorubicin | 5 | | 58_6F151_day5_Dox |
| 32 | Valproic acid | 5 | | 121_7F158_day5_ValpA |
| 33 | Valproic acid | 5 | | 122_7F159_day5_ValpA |
| 34 | Valproic acid | 5 | | 123_7F160_day5_ValpA |
| 35 | Control/Saline | 5 | | 71_1M4_day5_Saline |
| 36 | Control/Saline | 5 | | 72_1M5_day5_Saline |
| 37 | Control/Saline | 5 | | 73_1M6_day5_Saline |
| 38 | Control/Saline | 5 | | 74_1F104_day5_Saline |
| 39 | Control/Saline | 5 | | 75_1F105_day5_Saline |
| 40 | Amph. Control | 1 | Control | 130_1M6a_day1_Ctrl |
| 41 | Amph. Control | 5 | | 131_1M13a_day5_Ctrl |
| 42 | Amph. Control | 28 | | 132_1M20a_day28_Ctrl |
| 43 | Amph. Control | 1 | | 133_1F106a_day1_Ctrl |
| 44 | Amph. Control | 5 | | 134_1F113a_day5_Ctrl |
| 45 | Amph. Control | 28 | | 135_1F120a_day28_Ctrl |
| 46 | Amphotericin | 1 | Amphotericin | 136_6M61a_day1_Amp |
| 47 | Amphotericin | 1 | | 137_6M62a_day1_Amp |
| 48 | Amphotericin | 1 | | 138_6M63a_day1_Amp |
| 49 | Amphotericin | 5 | | 139_6M67a_day5_Amp |
| 50 | Amphotericin | 5 | | 140_6M68a_day5_Amp |
| 51 | Amphotericin | 5 | | 141_6M69a_day5_Amp |
| 52 | Amphotericin | 28 | | 142_6M73a_day28_Amp |
| 53 | Amphotericin | 28 | | 143_6M74a_day28_Amp |

| | | | | |
|---|---|---|---|---|
| 54 | Amphotericin | 28 | | 144_6M75a_day28_Amp |
| 55 | Amphotericin | 1 | | 145_6F161a_day1_Amp |
| 56 | Amphotericin | 1 | | 146_6F162a_day1_Amp |
| 57 | Amphotericin | 1 | | 147_6F163a_day1_Amp |
| 58 | Amphotericin | 5 | | 148_6F167a_day5_Amp |
| 59 | Amphotericin | 5 | | 149_6F168a_day5_Amp |
| 60 | Amphotericin | 5 | | 150_6F169a_day5_Amp |
| 61 | Amphotericin | 28 | | 151_6F173a_day28_Amp |
| 62 | Amphotericin | 28 | | 152_6F174a_day28_Amp |
| 63 | Amphotericin | 28 | | 153_6F175a_day28_Amp |

**Plate 3**

| | | | | |
|---|---|---|---|---|
| 1 | Gentamycin | 28 | day 28 toxicants M | 76_2M16_day28_Gent |
| 2 | Gentamycin | 28 | | 77_2M17_day28_Gent |
| 3 | Gentamycin | 28 | | 78_2M18_day28_Gent |
| 4 | Cisplatin | 28 | | 79_3M25_day28_Cis |
| 5 | Cisplatin | 28 | | 80_3M26_day28_Cis |
| 6 | Cisplatin | 28 | | 81_3M27_day28_Cis |
| 7 | Tobramycin | 28 | | 82_4M34_day28_Tob |
| 8 | Tobramycin | 28 | | 83_4M35_day28_Tob |
| 9 | Tobramycin | 28 | | 84_4M36_day28_Tob |
| 10 | Cadmium chloride | 28 | | 85_5M43_day28_CadCl |
| 11 | Cadmium chloride | 28 | | 86_5M44_day28_CadCl |
| 12 | Cadmium chloride | 28 | | 87_5M45_day28_CadCl |
| 13 | Doxorubicin | 28 | | 88_6M71_day28_Dox |
| 14 | Doxorubicin | 28 | | 89_6M72_day28_Dox |
| 15 | Doxorubicin | 28 | | 90_6M73_day28_Dox |
| 16 | Valproic acid | 28 | | 124_7M61_day28_ValpA |
| 17 | Valproic acid | 28 | | 125_7M62_day28_ValpA |
| 18 | Valproic acid | 28 | | 126_7M63_day28_ValpA |
| 19 | Gentamycin | 28 | day 28 toxicants F | 91_2F116_day28_Gent |
| 20 | Gentamycin | 28 | | 92_2F117_day28_Gent |
| 21 | Gentamycin | 28 | | 93_2F118_day28_Gent |
| 22 | Cisplatin | 28 | | 94_3F125_day28_Cis |
| 23 | Cisplatin | 28 | | 95_3F126_day28_Cis |

| | | | | |
|---|---|---|---|---|
| 24 | Cisplatin | 28 | | 96_3F127_day28_Cis |
| 25 | Tobramycin | 28 | | 97_4F134_day28_Tob |
| 26 | Tobramycin | 28 | | 98_4F135_day28_Tob |
| 27 | Tobramycin | 28 | | 99_4F136_day28_Tob |
| 28 | Cadmium chloride | 28 | | 100_5F143_day28_CadCl |
| 29 | Cadmium chloride | 28 | | 101_5F144_day28_CadCl |
| 30 | Cadmium chloride | 28 | | 102_5F145_day28_CadCl |
| 31 | Doxorubicin | 28 | | 103_6F171_day28_Dox |
| 32 | Doxorubicin | 28 | | 104_6F172_day28_Dox |
| 33 | Doxorubicin | 28 | | 105_6F173_day28_Dox |
| 34 | Valproic acid | 28 | | 127_7F161_day28_ValpA |
| 35 | Valproic acid | 28 | | 128_7F162_day28_ValpA |
| 36 | Valproic acid | 28 | | 129_7F163_day28_ValpA |
| 37 | Control/Saline | 28 | Day 28 control | 106_1M7_day28_Saline |
| 38 | Control/Saline | 28 | | 107_1M8_day28_Saline |
| 39 | Control/Saline | 28 | | 108_1M9_day28_Saline |
| 40 | Control/Saline | 28 | | 109_1F107_day28_Saline |
| 41 | Control/Saline | 28 | | 110_1F108_day28_Saline |
| 42 | Control/Saline | 28 | | 111_1F109_day28_Saline |

**Plate 4**

| | | | | |
|---|---|---|---|---|
| 1 | control | 1 | 1_UK1M1_control_Day1 | TT4: Male Day 1 |
| 2 | control | 1 | 2_UK1M2_control_Day1 | |
| 3 | control | 1 | 3_UK1M3_control_Day1 | |
| 4 | control | 1 | 4_UK1M4_control_Day1 | |
| 5 | control | 1 | 5_UK1M5_control_Day1 | |
| 6 | control | 1 | 6_UK1M6_control_Day1 | |
| 7 | T1 Ds1 | 1 | 7_UK3M13_T1 Ds1_Day1 | |
| 8 | T1 Ds1 | 1 | 8_UK3M14_T1 Ds1_Day1 | |
| 9 | T1 Ds1 | 1 | 9_UK3M15_T1 Ds1_Day1 | |
| 10 | T1 Ds1 | 1 | 10_UK3M16_T1 Ds1_Day1 | |
| 11 | T1 Ds1 | 1 | 11_UK3M17_T1 Ds1_Day1 | |
| 12 | T1 Ds1 | 1 | 12_UK3M18_T1 Ds1_Day1 | |
| 13 | T1 Ds2 | 1 | 13_UK5M25_T1 Ds2_Day1 | |
| 14 | T1 Ds2 | 1 | 14_UK5M26_T1 Ds2_Day1 | |
| 15 | T1 Ds2 | 1 | 15_UK5M27_T1 Ds2_Day1 | |

| | | | | |
|---|---|---|---|---|
| 16 | T1 Ds2 | 1 | 16_UK5M28_T1 Ds2_Day1 | |
| 17 | T1 Ds2 | 1 | 17_UK5M29_T1 Ds2_Day1 | |
| 18 | T1 Ds2 | 1 | 18_UK5M30_T1 Ds2_Day1 | |
| 19 | T2 Ds1 | 1 | 19_UK7M37_T2 Ds1_Day1 | |
| 20 | T2 Ds1 | 1 | 20_UK7M38_T2 Ds1_Day1 | |
| 21 | T2 Ds1 | 1 | 21_UK7M39_T2 Ds1_Day1 | |
| 22 | T2 Ds1 | 1 | 22_UK7M40_T2 Ds1_Day1 | |
| 23 | T2 Ds1 | 1 | 23_UK7M41_T2 Ds1_Day1 | |
| 24 | T2 Ds1 | 1 | 24_UK7M42_T2 Ds1_Day1 | |
| 25 | T2 Ds2 | 1 | 25_UK9M49_T2 Ds2_Day1 | |
| 26 | T2 Ds2 | 1 | 26_UK9M50_T2 Ds2_Day1 | |
| 27 | T2 Ds2 | 1 | 27_UK9M51_T2 Ds2_Day1 | |
| 28 | T2 Ds2 | 1 | 28_UK9M52_T2 Ds2_Day1 | |
| 29 | T2 Ds2 | 1 | 29_UK9M53_T2 Ds2_Day1 | |
| 30 | T2 Ds2 | 1 | 30_UK9M54_T2 Ds2_Day1 | |
| 31 | T3 Ds1 | 1 | 31_UK11M61_T3 Ds1_Day1 | |
| 32 | T3 Ds1 | 1 | 32_UK11M62_T3 Ds1_Day1 | |
| 33 | T3 Ds1 | 1 | 33_UK11M63_T3 Ds1_Day1 | |
| 34 | T3 Ds1 | 1 | 34_UK11M64_T3 Ds1_Day1 | |
| 35 | T3 Ds1 | 1 | 35_UK11M65_T3 Ds1_Day1 | |
| 36 | T3 Ds1 | 1 | 36_UK11M66_T3 Ds1_Day1 | |
| 37 | T3 Ds2 | 1 | 37_UK13M73_T3 Ds2_Day1 | |
| 38 | T3 Ds2 | 1 | 38_UK13M75_T3 Ds2_Day1 | |
| 39 | T3 Ds2 | 1 | 39_UK13M76_T3 Ds2_Day1 | |
| 40 | T3 Ds2 | 1 | 40_UK13M77_T3 Ds2_Day1 | |
| 41 | T3 Ds2 | 1 | 41_UK13M78_T3 Ds2_Day1 | |

**Plate 5**

| | | | | |
|---|---|---|---|---|
| 42 | control | 1 | 42_UK1F101_control_Day1 | TT5: Female Day 1 |
| 43 | control | 1 | 43_UK1F102_control_Day1 | |
| 44 | control | 1 | 44_UK1F103_control_Day1 | |
| 45 | control | 1 | 45_UK1F104_control_Day1 | |
| 46 | control | 1 | 46_UK1F105_control_Day1 | |
| 47 | control | 1 | 47_UK1F106_control_Day1 | |
| 48 | T1 Ds1 | 1 | 48_UK3F113_T1 Ds1_Day1 | |
| 49 | T1 Ds1 | 1 | 49_UK3F114_T1 Ds1_Day1 | |
| 50 | T1 Ds1 | 1 | 50_UK3F116_T1 Ds1_Day1 | |
| 51 | T1 Ds1 | 1 | 51_UK3F117_T1 Ds1_Day1 | |
| 52 | T1 Ds1 | 1 | 52_UK3F118_T1 Ds1_Day1 | |

| | | | | |
|---|---|---|---|---|
| 53 | T1 Ds2 | 1 | 53_UK5F125_T1 Ds2_Day1 | |
| 54 | T1 Ds2 | 1 | 54_UK5F126_T1 Ds2_Day1 | |
| 55 | T1 Ds2 | 1 | 55_UK5F127_T1 Ds2_Day1 | |
| 56 | T1 Ds2 | 1 | 56_UK5F128_T1 Ds2_Day1 | |
| 57 | T1 Ds2 | 1 | 57_UK5F129_T1 Ds2_Day1 | |
| 58 | T1 Ds2 | 1 | 58_UK5F130_T1 Ds2_Day1 | |
| 59 | T2 Ds1 | 1 | 59_UK7F137_T2 Ds1_Day1 | |
| 60 | T2 Ds1 | 1 | 60_UK7F139_T2 Ds1_Day1 | |
| 61 | T2 Ds1 | 1 | 61_UK7F140_T2 Ds1_Day1 | |
| 62 | T2 Ds1 | 1 | 62_UK7F141_T2 Ds1_Day1 | |
| 63 | T2 Ds1 | 1 | 63_UK7F142_T2 Ds1_Day1 | |
| 64 | T2 Ds2 | 1 | 64_UK9F149_T2 Ds2_Day1 | |
| 65 | T2 Ds2 | 1 | 65_UK9F150_T2 Ds2_Day1 | |
| 66 | T2 Ds2 | 1 | 66_UK9F151_T2 Ds2_Day1 | |
| 67 | T2 Ds2 | 1 | 67_UK9F152_T2 Ds2_Day1 | |
| 68 | T2 Ds2 | 1 | 68_UK9F153_T2 Ds2_Day1 | |
| 69 | T2 Ds2 | 1 | 69_UK9F154_T2 Ds2_Day1 | |
| 70 | T3 Ds1 | 1 | 70_UK11F161_T3 Ds1_Day1 | |
| 71 | T3 Ds1 | 1 | 71_UK11F163_T3 Ds1_Day1 | |
| 72 | T3 Ds1 | 1 | 72_UK11F164_T3 Ds1_Day1 | |
| 73 | T3 Ds1 | 1 | 73_UK11F165_T3 Ds1_Day1 | |
| 74 | T3 Ds1 | 1 | 74_UK11F166_T3 Ds1_Day1 | |
| 75 | T3 Ds2 | 1 | 75_UK13F173_T3 Ds2_Day1 | |
| 76 | T3 Ds2 | 1 | 76_UK13F175_T3 Ds2_Day1 | |
| 77 | T3 Ds2 | 1 | 77_UK13F176_T3 Ds2_Day1 | |
| 78 | T3 Ds2 | 1 | 78_UK13F177_T3 Ds2_Day1 | |
| 79 | T3 Ds2 | 1 | 79_UK13F178_T3 Ds2_Day1 | |

**Plate 6**

| | | | | |
|---|---|---|---|---|
| 80 | control | 5 | 80_UK2M7_control_Day5 | TT6 Male Day 5 |
| 81 | control | 5 | 81_UK2M8_control_Day5 | |
| 82 | control | 5 | 82_UK2M9_control_Day5 | |
| 83 | control | 5 | 83_UK2M10_control_Day5 | |
| 84 | control | 5 | 84_UK2M11_control_Day5 | |
| 85 | T1 Ds1 | 5 | 85_UK4M19_T1 Ds1_Day5 | |
| 86 | T1 Ds1 | 5 | 86_UK4M20_T1 Ds1_Day5 | |
| 87 | T1 Ds1 | 5 | 87_UK4M21_T1 Ds1_Day5 | |
| 88 | T1 Ds1 | 5 | 88_UK4M22_T1 Ds1_Day5 | |
| 89 | T1 Ds1 | 5 | 89_UK4M23_T1 Ds1_Day5 | |

| | | | | |
|---|---|---|---|---|
| 90 | T1 Ds1 | 5 | 90_UK4M24_T1 Ds1_Day5 | |
| 91 | T1 Ds2 | 5 | 91_UK6M31_T1 Ds2_Day5 | |
| 92 | T1 Ds2 | 5 | 92_UK6M32_T1 Ds2_Day5 | |
| 93 | T1 Ds2 | 5 | 93_UK6M33_T1 Ds2_Day5 | |
| 94 | T1 Ds2 | 5 | 94_UK6M34_T1 Ds2_Day5 | |
| 95 | T1 Ds2 | 5 | 95_UK6M35_T1 Ds2_Day5 | |
| 96 | T1 Ds2 | 5 | 96_UK6M36_T1 Ds2_Day5 | |
| 97 | T2 Ds1 | 5 | 97_UK8M43_T2 Ds1_Day5 | |
| 98 | T2 Ds1 | 5 | 98_UK8M44_T2 Ds1_Day5 | |
| 99 | T2 Ds1 | 5 | 99_UK8M45_T2 Ds1_Day5 | |
| 100 | T2 Ds1 | 5 | 100_UK8M46_T2 Ds1_Day5 | |
| 101 | T2 Ds1 | 5 | 101_UK8M47_T2 Ds1_Day5 | |
| 102 | T2 Ds1 | 5 | 102_UK8M48_T2 Ds1_Day5 | |
| 103 | T2 Ds2 | 5 | 103_UK10M55_T2 Ds2_Day5 | |
| 104 | T2 Ds2 | 5 | 104_UK10M56_T2 Ds2_Day5 | |
| 105 | T2 Ds2 | 5 | 105_UK10M57_T2 Ds2_Day5 | |
| 106 | T2 Ds2 | 5 | 106_UK10M59_T2 Ds2_Day5 | |
| 107 | T2 Ds2 | 5 | 107_UK10M60_T2 Ds2_Day5 | |
| 108 | T3 Ds1 | 5 | 108_UK12M67_T3 Ds1_Day5 | |
| 109 | T3 Ds1 | 5 | 109_UK12M69_T3 Ds1_Day5 | |
| 110 | T3 Ds1 | 5 | 110_UK12M70_T3 Ds1_Day5 | |
| 111 | T3 Ds1 | 5 | 111_UK12M71_T3 Ds1_Day5 | |
| 112 | T3 Ds1 | 5 | 112_UK12M72_T3 Ds1_Day5 | |
| 113 | T3 Ds2 | 5 | 113_UK14M80_T3 Ds2_Day5 | |
| 114 | T3 Ds2 | 5 | 114_UK14M81_T3 Ds2_Day5 | |
| 115 | T3 Ds2 | 5 | 115_UK14M82_T3 Ds2_Day5 | |
| 116 | T3 Ds2 | 5 | 116_UK14M83_T3 Ds2_Day5 | |

**Plate 7**

| | | | | |
|---|---|---|---|---|
| 117 | control | 5 | 117_UK2F107_control_Day5 | TT7: Female Day 5 |
| 118 | control | 5 | 118_UK2F108_control_Day5 | |
| 119 | control | 5 | 119_UK2F109_control_Day5 | |
| 120 | control | 5 | 120_UK2F110_control_Day5 | |
| 121 | control | 5 | 121_UK2F111_control_Day5 | |
| 122 | control | 5 | 122_UK2F112_control_Day5 | |
| 123 | T1 Ds1 | 5 | 123_UK4F119_T1 Ds1_Day5 | |
| 124 | T1 Ds1 | 5 | 124_UK4F120_T1 Ds1_Day5 | |
| 125 | T1 Ds1 | 5 | 125_UK4F121_T1 Ds1_Day5 | |
| 126 | T1 Ds1 | 5 | 126_UK4F122_T1 Ds1_Day5 | |

| | | | | |
|---|---|---|---|---|
| 127 | T1 Ds1 | 5 | 127_UK4F123_T1 Ds1_Day5 | |
| 128 | T1 Ds1 | 5 | 128_UK4F124_T1 Ds1_Day5 | |
| 129 | T1 Ds2 | 5 | 129_UK6F131_T1 Ds2_Day5 | |
| 130 | T1 Ds2 | 5 | 130_UK6F132_T1 Ds2_Day5 | |
| 131 | T1 Ds2 | 5 | 131_UK6F133_T1 Ds2_Day5 | |
| 132 | T1 Ds2 | 5 | 132_UK6F134_T1 Ds2_Day5 | |
| 133 | T1 Ds2 | 5 | 133_UK6F135_T1 Ds2_Day5 | |
| 134 | T1 Ds2 | 5 | 134_UK6F136_T1 Ds2_Day5 | |
| 135 | T2 Ds1 | 5 | 135_UK8F143_T2 Ds1_Day5 | |
| 136 | T2 Ds1 | 5 | 136_UK8F144_T2 Ds1_Day5 | |
| 137 | T2 Ds1 | 5 | 137_UK8F145_T2 Ds1_Day5 | |
| 138 | T2 Ds1 | 5 | 138_UK8F147_T2 Ds1_Day5 | |
| 139 | T2 Ds1 | 5 | 139_UK8F148_T2 Ds1_Day5 | |
| 140 | T2 Ds2 | 5 | 140_UK10F156_T2 Ds2_Day5 | |
| 141 | T2 Ds2 | 5 | 141_UK10F158_T2 Ds2_Day5 | |
| 142 | T2 Ds2 | 5 | 142_UK10F159_T2 Ds2_Day5 | |
| 143 | T2 Ds2 | 5 | 143_UK10F160_T2 Ds2_Day5 | |
| 144 | T3 Ds1 | 5 | 144_UK12F167_T3 Ds1_Day5 | |
| 145 | T3 Ds1 | 5 | 145_UK12F168_T3 Ds1_Day5 | |
| 146 | T3 Ds1 | 5 | 146_UK12F169_T3 Ds1_Day5 | |
| 147 | T3 Ds1 | 5 | 147_UK12F170_T3 Ds1_Day5 | |
| 148 | T3 Ds1 | 5 | 148_UK12F171_T3 Ds1_Day5 | |
| 149 | T3 Ds1 | 5 | 149_UK12F172_T3 Ds1_Day5 | |
| 150 | T3 Ds2 | 5 | 150_UK14F179_T3 Ds2_Day5 | |
| 151 | T3 Ds2 | 5 | 151_UK14F180_T3 Ds2_Day5 | |
| 152 | T3 Ds2 | 5 | 152_UK14F181_T3 Ds2_Day5 | |
| 153 | T3 Ds2 | 5 | 153_UK14F182_T3 Ds2_Day5 | |
| 154 | T3 Ds2 | 5 | 154_UK14F183_T3 Ds2_Day5 | |

**Shuffled plate**

| Toxin used | Day | Final name |
|---|---|---|
| Cadmium chloride | 1 | 26_5F138_day1_CadCl |
| Cisplatin | 1 | 6_3M21_day1_Cis |
| Cisplatin | 1 | 5_3M20_day1_Cis |
| Control/Saline | 1 | 66_1M2_day1_Saline |
| Doxorubicin | 1 | 30_6F148_day1_Dox |
| Doxorubicin | 1 | 28_6F146_day1_Dox |
| Naïve | 28 | 61_8M83_day28_Naïve |
| Naïve | 28 | 62_8F181_day28_Naïve |
| Tobramycin | 1 | 8_4M29_day1_Tob |
| Valproic acid | 1 | 112_7M55_day1_ValpA |
| Valproic acid | 1 | 117_7F157_day1_ValpA |
| Cadmium chloride | 5 | 54_5F141_day5_CadCl |
| Cadmium chloride | 5 | 41_5M41_day5_CadCl |
| Cisplatin | 5 | 50_3F124_day5_Cis |
| Cisplatin | 5 | 36_3M24_day5_Cis |
| Control/Saline | 5 | 73_1M6_day5_Saline |
| Control/Saline | 5 | 71_1M4_day5_Saline |
| Doxorubicin | 5 | 43_6M49_day5_Dox |
| Tobramycin | 5 | 51_4F131_day5_Tob |
| Valproic acid | 5 | 123_7F160_day5_ValpA |
| Valproic acid | 5 | 122_7F159_day5_ValpA |
| Cadmium chloride | 28 | 85_5M43_day28_CadCl |
| Cadmium chloride | 28 | 86_5M44_day28_CadCl |
| Cisplatin | 28 | 79_3M25_day28_Cis |
| Cisplatin | 28 | 94_3F125_day28_Cis |
| Cisplatin | 28 | 81_3M27_day28_Cis |
| Control/Saline | 28 | 106_1M7_day28_Saline |
| Control/Saline | 28 | 107_1M8_day28_Saline |
| Control/Saline | 28 | 111_1F109_day28_Saline |
| Doxorubicin | 28 | 104_6F172_day28_Dox |
| Tobramycin | 28 | 84_4M36_day28_Tob |
| Valproic acid | 28 | 126_7M63_day28_ValpA |

| Toxin used | Day | Final name |
|---|---|---|
| control | 1 | 2_UK1M2_control_Day1 |
| T1 Ds1 | 1 | 7_UK3M13_T1 Ds1_Day1 |
| T1 Ds1 | 1 | 12_UK3M18_T1 Ds1_Day1 |
| T1 Ds2 | 1 | 16_UK5M28_T1 Ds2_Day1 |
| T2 Ds1 | 1 | 24_UK7M42_T2 Ds1_Day1 |
| T2 Ds1 | 1 | 20_UK7M38_T2 Ds1_Day1 |
| T3 Ds1 | 1 | 31_UK11M61_T3 Ds1_Day1 |
| T3 Ds2 | 1 | 38_UK13M75_T3 Ds2_Day1 |
| T3 Ds2 | 1 | 41_UK13M78_T3 Ds2_Day1 |
| control | 1 | 43_UK1F102_control_Day1 |
| control | 1 | 46_UK1F105_control_Day1 |
| T1 Ds1 | 1 | 51_UK3F117_T1 Ds1_Day1 |
| T1 Ds1 | 1 | 52_UK3F118_T1 Ds1_Day1 |
| T1 Ds2 | 1 | 56_UK5F128_T1 Ds2_Day1 |
| T2 Ds1 | 1 | 63_UK7F142_T2 Ds1_Day1 |
| T2 Ds1 | 1 | 61_UK7F140_T2 Ds1_Day1 |
| T2 Ds2 | 1 | 68_UK9F153_T2 Ds2_Day1 |
| T2 Ds2 | 1 | 66_UK9F151_T2 Ds2_Day1 |
| T3 Ds1 | 1 | 72_UK11F164_T3 Ds1_Day1 |
| T3 Ds2 | 1 | 78_UK13F177_T3 Ds2_Day1 |
| control | 5 | 80_UK2M7_control_Day5 |
| control | 5 | 83_UK2M10_control_Day5 |
| control | 5 | 84_UK2M11_control_Day5 |
| control | 5 | 82_UK2M9_control_Day5 |
| T1 Ds1 | 5 | 86_UK4M20_T1 Ds1_Day5 |
| T1 Ds1 | 5 | 88_UK4M22_T1 Ds1_Day5 |
| T1 Ds2 | 5 | 93_UK6M33_T1 Ds2_Day5 |
| T1 Ds2 | 5 | 94_UK6M34_T1 Ds2_Day5 |
| T2 Ds1 | 5 | 97_UK8M43_T2 Ds1_Day5 |
| T3 Ds1 | 5 | 108_UK12M67_T3 Ds1_Day5 |
| T3 Ds1 | 5 | 110_UK12M70_T3 Ds1_Day5 |
| control | 5 | 118_UK2F108_control_Day5 |
| T1 Ds1 | 5 | 125_UK4F121_T1 Ds1_Day5 |
| T1 Ds2 | 5 | 129_UK6F131_T1 Ds2_Day5 |
| T1 Ds2 | 5 | 131_UK6F133_T1 Ds2_Day5 |
| T2 Ds1 | 5 | 139_UK8F148_T2 Ds1_Day5 |
| T2 Ds2 | 5 | 140_UK10F156_T2 Ds2_Day5 |

| Toxin used | Day | Final name |
|---|---|---|
| T2 Ds2 | 5 | 143_UK10F160_T2 Ds2_Day5 |
| T3 Ds1 | 5 | 147_UK12F170_T3 Ds1_Day5 |
| T3 Ds1 | 5 | 146_UK12F169_T3 Ds1_Day5 |
| T3 Ds2 | 5 | 151_UK14F180_T3 Ds2_Day5 |
| T3 Ds2 | 5 | 153_UK14F182_T3 Ds2_Day5 |

Inter-Plate Normalization

To account for possible differences of qRT-PCR measurements between the different plates due to experimental artifacts, a normalization plate was planned. This plate, designated hereinabove 'shuffled plate', contained samples from all other plates. Real Time PCR was performed on this plate using the primers appropriate for all candidates in tables 26 and 27. For each other plate (plates 1-7 above) and each amplicon, the samples appearing also in the shuffled plate were examined - the ratio between the transcript abundance as measured in the plate to the abundance as measured in the shuffled plate was calculated, outliers were manually removed, and the geometric mean of the left ratios was taken as an additional multiplicative normalization factor for the plate and amplicon.

Real Time qPCR analysis of the selected markers

Example 3.1: Expression of tumor necrosis factor receptor superfamily, member 12a, AA686189, transcripts which are detectable by amplicon as depicted in sequence name W41270_DB81_seg11 in kidney tissues of treated or untreated rats

Expression of tumor necrosis factor receptor superfamily, member 12a transcripts detectable by or according to seg11 - W41270_DB81_seg11_F2R2 (SEQ ID NO: 254) amplicon and primers W41270_DB81_seg11_F2 (SEQ ID NO: 252) and W41270_DB81_seg11_R2 (SEQ ID NO: 253) was measured by real time PCR. The value of the expression was measured by Real-Time PCR and normalized relative to the expression of the house keeping genes, as described in section "RT Preparation and Real-Time RT-PCR Analysis" hereinabove and by the shuffled plate values, as described in section "Inter-Plate Normalization". Following optimization, described in Example 3 herein, the experiments were done using primers concentration of 50nM.

The column entitled W41270_DB81_seg11 in Table 23 contains the normalized expression values of the above-indicated tumor necrosis factor receptor superfamily, member 12a transcript in treated or untreated kidney samples.

As is evident from the column entitled W41270_DB81-seg11 in Table 23, the level of expression of the tumor necrosis factor receptor superfamily, member 12a transcript detectable by the above amplicon was higher in the samples treated with toxic compounds (samples 1-58 and 76-105 , in kidney tissue panel described in Table 9 hereinabove) than in the control samples (naive, saline and valproic acid treated samples; samples numbers 59-75 and 106-129, in kidney tissue panel described in Table 9 hereinabove). Statistical analysis was applied to verify the significance of these results, with a P-value for day 5 of $3.4E^{-05}$.

Primer pairs may also be used in the context of the present invention; for example, for the above experiment, the following primer pair was used as a non-limiting illustrative example only of a suitable primer pair: W41270_DB81_seg11_F2 (SEQ ID NO: 252) forward primer; and W41270_DB81_seg11_R2 (SEQ ID NO: 253) reverse primer.

Amplicons obtained through the use of any suitable primer pair; for example, for the above experiment, may also be used in the context of the present invention; the following amplicon was obtained as a non-limiting illustrative example only of a suitable amplicon:

W41270_DB81_seg11_F2R2 (SEQ ID NO: 254).

Forward primer (W41270_DB81_seg11_F2 (SEQ ID NO: 252)): GATCTGGGTAGGTGGTTGTTGG

Reverse primer (W41270_DB81_seg11_R2 (SEQ ID NO: 253)): CGCACACCCTTATAAAGTCCC

Amplicon (W41270_DB81_seg11_F2R2 (SEQ ID NO: 254)):

GATCTGGGTAGGTGGTTGTTGGGGCAGAAAGGAGGTCGTAGACTTAGGATATAGGAAACCA GGAAAAACTGACTGAGGAAGGGACTTTTATAAGGGTGTGCG

Example 3.2: Expression of Interferon stimulated exonuclease 20 (ISG20), AI045075, transcripts which are detectable by amplicon as depicted in sequence name AI045075_DB71_seg6 in kidney tissues of treated or untreated rats

Expression of Interferon stimulated exonuclease 20 (ISG20) transcripts detectable by or according to seg6 - AI045075_DB71_seg6_F2R2 (SEQ ID NO: 263) amplicon and primers AI045075_DB71_seg6_F2 (SEQ ID NO: 261) and AI045075_DB71_seg6_R2 (SEQ ID NO: 262) was measured by real time PCR. The value of the expression was measured by Real-Time PCR and normalized relative to the expression of the house keeping genes, as described in section "RT Preparation and Real-Time RT-PCR Analysis" hereinabove and by the shuffled plate values, as described in section "Inter-Plate Normalization".

The column entitled AI045075_DB71_seg6 in Table 23 contains the normalized expression values of the above-indicated Interferon stimulated exonuclease 20 (ISG20) transcript in treated or untreated kidney samples.

As is evident from the column entitled AI045075_DB71_seg6 in Table 23, the level of expression of the Interferon stimulated exonuclease 20 (ISG20) transcript detectable by the above amplicon was higher in the samples treated with toxic compounds (samples 1-58 and 76-105, in kidney tissue panel described in Table 9 hereinabove) than in the control samples (naive, saline and valproic acid treated samples; samples numbers- 59-75 and 106-129, in kidney tissue panel described in Table 9 hereinabove). Statistical analysis was applied to verify the significance of these results, P-value for day 5: $1.1E^{-06}$.

Primer pairs may also be used in the context of the present invention; for example, for the above experiment, the following primer pair was used as a non-limiting illustrative example only of a suitable primer pair: AI045075_DB71_seg6_F2 (SEQ ID NO: 261) forward primer; and AI045075_DB71_seg6_R2 (SEQ ID NO: 262) reverse primer.

Amplicons obtained through the use of any suitable primer pair; for example, for the above experiment, may also be used in the context of the present invention; the following amplicon was obtained as a non-limiting illustrative example only of a suitable amplicon:

AI045075_DB71_seg6_F2R2 (SEQ ID NO: 263).

Forward primer (AI045075_DB71_seg6_F2 (SEQ ID NO: 261)): GGGCCACAATGGAGCTCTAC

Reverse primer (AI045075_DB71_seg6_R2 (SEQ ID NO: 262)): ACAGGTCTCATTCATGGAAAACTATG

Amplicon (AI045075_DB71_seg6_F2R2 (SEQ ID NO: 263)):

GGGCCACAATGGAGCTCTACAAAATCTCTCAGCGACTCAGAGCCCAGCGAGGGCTGC CCTGCCTGGGAACATCAGCCTGAACTTCATCCTCATCCAGGATCAGAAGCAGCTACTCCTTG AAGGACCATAGTTTTCCATGAATGAGACCTGT

Example 3.3: Expression of Interferon stimulated exonuclease 20 (ISG20), AI045075, transcripts which are detectable by amplicon as depicted in sequence name W64472_DB81_seg2 in kidney tissues of treated or untreated rats

Expression of Interferon stimulated exonuclease 20 (ISG20), A045075, transcripts detectable by or according to seg2 - W64472_DB81_seg2_F6R1 (SEQ ID NO: 329) amplicon and primers W64472_DB81_seg2_F6 (SEQ ID NO: 327) and W64472_DB81_seg2_R1 (SEQ ID NO: 328) was measured by real time PCR. The value of the expression was measured by Real-Time PCR and normalized relative to the expression of the house keeping genes, as described in section "RT Preparation and Real-Time RT-PCR Analysis" hereinabove and by the shuffled plate values, as described in section "Inter-Plate Normalization".

The column entitled W64472_DB81_seg2 in Table 23 contains the normalized expression values of the above-indicated Interferon stimulated exonuclease 20 (ISG20), A045075, transcript in treated or untreated kidney samples.

As is evident from the column entitled W64472_DB81_seg2 in Table 23, the level of expression of the Interferon stimulated exonuclease 20 (ISG20), AI045075, transcript detectable by the above amplicon was higher in the samples treated with toxic compounds (samples 1-58 and 76-105, in kidney tissue panel described in Table 9 hereinabove) than in the control samples (naive, saline and valproic acid treated samples; samples numbers- 59-75 and 106-129, in kidney tissue panel described in Table 9 hereinabove). Statistical analysis was applied to verify the significance of these results, P-value for day 5: 1.0E$^{-05}$.

Primer pairs may also be used in the context of the present invention; for example, for the above experiment, the following primer pair was used as a non-limiting illustrative example only of a suitable primer pair: W64472_DB81_seg2_F6 (SEQ ID NO: 327) forward primer; and W64472_DB81_seg2_R1 (SEQ ID NO:328) reverse primer.

Amplicons obtained through the use of any suitable primer pair; for example, for the above experiment, may also be used in the context of the present invention; the following amplicon was obtained as a non-limiting illustrative example only of a suitable amplicon:

W64472_DB81_seg2_F6R1 (SEQ ID NO: 329).

Following optimization described in Example 3 herein, the following primers were used to amplify W64472_DB81_seg2_F6R1 (SEQ ID NO: 329) amplicon:

Forward primer (W64472_DB81_seg2_F6 (SEQ ID NO: 327)): ACAGCCTGATGCAGACAGC
Reverse primer (W64472_DB81_seg2_R1 (SEQ ID NO:328)): TCTGGTTCATTATCAAGGGAAGTTG
Amplicon (W64472_DB81_seg2_F6R1 (SEQ ID NO: 329)) :

ACAGCCTGATGCAGACAGCCCTGACTCAACCTGCCAGCCCCCTTACCTGGCCAGCCTTGA
GGAGATGGAACAGCCCAGGCTACAAGGCCTGCCCCCACTCCTCAACTTCCCTTGATAATG
AACCAGA

Example 3.4: Expression of Cyclin-G1 (CCNG1), H31883, transcripts which are detectable by amplicon as depicted in sequence name H31883_DB71_seg13 in kidney tissues of treated or untreated rats

Expression of Cyclin-G1 (CCNG1) transcripts detectable by or according to seg13 - H31883_DB71_seg13_F5R5 (SEQ ID NO: 332) amplicon and primers H31883_DB71_seg13_F5 (SEQ ID NO: 330) and H31883_DB71_seg13_R5 (SEQ ID NO: 331) was measured by real time PCR. The value of the expression was measured by Real-Time PCR and normalized relative to the expression of the house keeping genes, as described in section "RT Preparation and Real-Time RT-PCR Analysis" hereinabove and by the shuffled plate values, as described in section "Inter-Plate Normalization".

The column entitled H31883_DB71_seg13 in Table 23 contains the normalized expression values of the above-indicated Cyclin-G1 (CCNG1) transcript in treated or untreated kidney samples.

As is evident from the column entitled H31883_DB71_seg13 in Table 23, the level of expression of the Cyclin-G1 (CCNG1) transcript detectable by the above amplicon was higher in the samples treated with toxic compounds (samples 1-58 and 76-105, in kidney tissue panel described in hereinabove) than in the control samples (naive, saline and valproic acid treated samples; samples numbers- 59-75 and 106-129, in kidney tissue panel described in Table 9 hereinabove). Statistical analysis was applied to verify the significance of these results, P-value for day 5: 2.1E$^{-05}$.

Primer pairs may also be used in the context of the present invention; for example, for the above experiment, the following primer pair was used as a non-limiting illustrative example only of a suitable primer pair: H31883_DB71_seg13_F5 (SEQ ID NO: 330) forward primer; and H31883_DB71_seg13_R5 (SEQ ID NO: 331) reverse primer.

Amplicons obtained through the use of any suitable primer pair; for example, for the above experiment, may also be used in the context of the present invention; the following amplicon was obtained as a non-limiting illustrative example only of a suitable amplicon:

H31883_DB71_seg13_F5R5 (SEQ ID NO: 332).

Following optimization described in Example 3 herein, the following primers were used to amplify H31883_DB71_seg13_F5R5 (SEQ ID NO: 332) amplicon:

Forward primer (H31883_DB71_seg13_F5 (SEQ ID NO: 330)): CAGAATTGCTGCCTCAATCTAGTCC
Reverse primer (H31883_DB71_seg13_R5 (SEQ ID NO: 331)): GGTTTTGCAGATGTACTCGGTTCC Amplicon (H31883_DB71_seg13_F5R5 (SEQ ID NO: 332)):

CAGAATTGCTGCCTCAATCTAGTCCCATTTGAGAAAATTTGTTTCTACTGTCTCAATAA CTGGATGAAATATCACTCTGAAAACTTGCCTATTGCACTAAAGCTAGTTTAGGCTTGATAAA ACACTCCAGGAGGTTTTTACCACAGACTGTTTCTATTAAAACTGCTGCTTCTCATGTACAATT TTGTTTTAAAAGGAACCGAGTACATCTGCAAAACC

Example 3.5: Expression of Cyclin-G1 (CCNG1), H31883, transcripts which are detectable by amplicon as depicted in sequence name MUSCYCG1R_DB81_seg19-20 in kidney tissues of treated or untreated rats

Expression of Cyclin-G1 (CCNG1) transcripts detectable by or according to seg 19-20 - MUSCYCG1R_DB81_seg19-20_F1R1 (SEQ ID NO: 296) amplicon and primers MUSCYCG1R_DB81_seg19-20_F1 (SEQ ID NO: 294) and MUSCYCG1R_DB81_seg19-20_R1 (SEQ ID NO: 295) was measured by real time PCR. The value of the expression was measured by Real-Time PCR and normalized relative to the expression of the house keeping genes, as described in section "RT Preparation and Real-Time RT-PCR Analysis" hereinabove and by the shuffled plate values, as described in section "Inter-Plate Normalization". Following optimization described in Example 3 herein, the annealing temperature of the PCR was 62°C.

The column entitled MUSCYCG1R_DB81_seg19-20 in Table 23 contains the normalized expression values of the above-indicated Cyclin-G1 (CCNG1) transcript in treated or untreated kidney samples.

As is evident from the column entitled MUSCYCG1R_DB81_seg19-20 in Table 23, the level of expression of the Cyclin-G1 (CCNG1) transcript detectable by the above amplicon was higher in the samples treated with toxic compounds (samples 1-58 and 76-105, in kidney tissue panel described in Table 9 hereinabove) than in the control samples (naïve, saline and valproic acid treated samples; samples numbers- 59-75 and 106-129, in kidney tissue panel described in Table 9 hereinabove). Statistical analysis was applied to verify the significance of these results, P-value for day 5: $1.3E^{-04}$.

Primer pairs may also be used in the context of the present invention; for example, for the above experiment, the following primer pair was used as a non-limiting illustrative example only of a suitable primer pair: MUSCYCG1R_DB81_seg19-20_F1 (SEQ ID NO: 294) forward primer; and MUSCYCG1R_DB81_seg19-20_R1 (SEQ ID NO: 295) reverse primer.

Amplicons obtained through the use of any suitable primer pair; for example, for the above experiment, may also be used in the context of the present invention; the following amplicon was obtained as a non-limiting illustrative example only of a suitable amplicon: MUSCYCG1R_DB81_seg19-20_F1R1 (SEQ ID NO: 296).

Forward primer (MUSCYCG1R_DB81_seg19-20_F1 (SEQ ID NO: 294)): GAGATCCAAGCACTGAAGTATGTAGAGT

Reverse primer (MUSCYCG1R_DB81_seg19-20_R1 (SEQ ID NO: 295)): TCAGGAGTACAGTGGATACATTTCTCTT

Amplicon (MUSCYCG1R_DB81_seg19-20_F1R1 (SEQ ID NO: 296)):

GAGATCCAAGCACTGAAGTATGTAGAGTTAACAGAAGGAGTAGAATGTATTCAGAAA CATTCCAAGGTATGCCAAGGTGATAGCATTGATCCTATTAGCAAGCTACAAGAGAAATGTAT CCACTGTACTCCTGA

Example 3.6: Expression Etoposide induced 2.4 (EI24) mRNA, H31799, transcripts which are detectable by amplicon as depicted in sequence name W83813_DB81_seg27 in kidney tissues of treated or untreated rats

Expression of Etoposide induced 2.4 mRNA (EI24) transcripts detectable by or according to seg27 - W83813_DB81_seg27_F1R1 (SEQ ID NO: 287) amplicon and primers W83813_DB81_seg27_F1 (SEQ ID NO: 285) and W83813_DB81_seg27_R1 (SEQ ID NO: 286) was measured by real time PCR. The value of the expression was measured by Real-Time PCR and normalized relative to the expression of the house keeping genes, as described in section "RT Preparation and Real-Time RT-PCR Analysis" hereinabove and by the shuffled plate values, as described in section "Inter-Plate Normalization".

The column entitled W83813_DB81_seg27 in Table 23 contains the normalized expression values of the above-indicated Etoposide induced 2.4 mRNA (EI24) transcript in treated or untreated kidney samples.

As is evident from the column entitled W83813_DB81_seg27 in Table 23, the level of expression of the Etoposide induced 2.4 mRNA (EI24) transcript detectable by the above amplicon was higher in the samples treated with toxic compounds (samples 1-58 and 76-105, in kidney tissue panel described in Table 9 hereinabove) than in the control samples (naive, saline and valproic acid treated samples; samples numbers- 59-75 and 106-129, in kidney tissue panel described in Table 9 hereinabove). Statistical analysis was applied to verify the significance of these results, P-value for day 5: 0.04.

Primer pairs may also be used in the context of the present invention; for example, for the above experiment, the following primer pair was used as a non-limiting illustrative example only of a suitable primer pair: W83813_DB81_seg27_F1 (SEQ ID NO: 285) forward primer; and W83813_DB81_seg27_R1 (SEQ ID NO: 286) reverse primer.

Amplicons obtained through the use of any suitable primer pair; for example, for the above experiment, may also be used in the context of the present invention; the following amplicon was obtained as a non-limiting illustrative example only of a suitable amplicon W83813_DB81_seg27_F1R1 (SEQ ID NO: 287):

Forward primer (W83813_DB81_seg27_F1 (SEQ ID NO: 285)): AATCTAGGCTGCCTCCTGGAG

Reverse primer (W83813_DB81_seg27_R1 (SEQ ID NO: 286)): AGGTCAATTATACAAGGCATGACTTTAG

Amplicon (W83813_DB81_seg27_F1R1 (SEQ ID NO: 287)):

AATCTAGGCTGCCTCCTGGAGGAAGATACTTAGGAGTTCAGAAGTGAAGAGATGAGG
CTTATAATACTTTTTCCTAAAGTCATGCCTTGTATAATTGACCT

Classification Using qRT-PCR Data

Given the qRT-PCR panel for the selected genes detailed in Table 23, the optimal classifier was built based on the measured expression levels of the labeled samples, as described in Table 9. This was performed again to achieve a more accurate and controlled signature.

Two equivalent optimal signatures for prediction of renal damage at day-5, as derived in the validation stage analysis of the labeled samples, are given in Table 26 (four amplicons of three genes) and Table 27 (six amplicons of four genes). Table 28 summarizes the performance of the 4-genes signature on the various groups of the labeled samples.

Table 26: Optimal Signature Day 5 (Validation Stage Analysis)

| Contig | Gene name | Splice Variant / Wild Type | Internal name | Probeset |
|---|---|---|---|---|
| AA686189 | TNFRSF12A | SV | W41270_DB81_seg11 | 1371785_at |
| AI045075 | ISG20 | SV | W64472_DB81_seg2 | 1390507_at |
| AI045075 | ISG20 | WT | AI045075_DB71_seg6 | 1390507_at |
| H31883 | CCNG1 | WT | H31883_DB71_seg13 | 1367764_at |

Table 27: Optimal Signature Day 5 (Validation Stage Analysis)

| Contig | Gene name | Splice Variant / Wild Type | Internal name | Probeset |
|---|---|---|---|---|
| AA686189 | TNFRSF12A | SV | W41270_DB81_seg11 | 1371785_at |
| AI045075 | ISG20 | SV | W64472_DB81_seg2 | 1390507_at |
| AI045075 | ISG20 | WT | AI045075_DB71_seg6 | 1390507_at |
| H31883 | CCNG1 | WT | H31883_DB71_seg13 | 1367764_at |
| H31883 | CCNG1 | SV | MUSCYCG1R_DB81_seg19-20 | 1367764_at |
| H31799 | EI24 | SV | W83813_DB81_seg27 | 1388642_at |

Table 28: Performance of the 4-genes signature on the various groups of the labeled samples

| Group (Compound, Period, Gender) | No. of samples | Average Toxicity Score | Standard Deviation of Toxicity Score |
|---|---|---|---|
| Doxorubicin Day5 Male | 2 | 0.77 | 0.07 |
| Gentamycin Day5 Female | 3 | 0.73 | 0.12 |
| Cisplatin Day5 Female | 3 | 0.69 | 0.14 |
| Gentamycin Day5 Male | 3 | 0.63 | 0.03 |
| Cisplatin Day5 Male | 3 | 0.63 | 0.27 |
| Tobramycin Day5 Female | 3 | 0.61 | 0.28 |
| Doxorubicin Day5 Female | 3 | 0.61 | 0.19 |
| Tobramycin Day5 Male | 3 | 0.59 | 0.18 |
| Valproic-Acid Day1 Male | 3 | 0.57 | 0.42 |
| Saline Day1 Male | 3 | 0.51 | 0.45 |
| Naive Male | 3 | 0.41 | 0.42 |
| Valproic-Acid Day1 Female | 3 | 0.33 | 0.51 |
| Valproic-Acid Day5 Female | 3 | 0.18 | 0.3 |
| Naive Female | 3 | 0.14 | 0.24 |
| Saline Day1 Female | 3 | 0.11 | 0.14 |
| Valproic-Acid Day28 Female | 3 | 0.07 | 0.12 |
| Valproic-Acid Day5 Male | 3 | 0.02 | 0.02 |
| Valproic-Acid Day28 Male | 3 | 0.01 | 0 |
| Saline Day5 Female | 2 | 0.01 | 0.01 |
| Saline Day28 Female | 3 | 0.01 | 0.01 |
| Saline Day5 Male | 3 | 0 | 0 |
| Saline Day28 Male | 3 | 0 | 0 |

The two optimal classifiers (signatures described in Tables 26 and 27) were applied to the qRT-PCR reads of the un-labeled samples and their calls was used to assign a level of toxicity to each group and, eventually, each compound.

Results

qRT-PCR Data Analysis of Labeled Samples described in Table 9 and plate 1-3 in Table 25.

Transcripts abundance was measured using qRT-PCR for 8 amplicons from the 4 genes disclosed in Table 13 hereinabove. As in Example 1, an optimal signature for day-5 was identified, distinguishing the day-5 "Toxic" samples from all control (and naive rats) samples. Iterative feature selection and Random-Forest classifier were used as in the discovery stage, described in Example 1 hereinabove. The input data was the new qRT-PCR measurements and the gender of the rat as an extra two-values features. The same parameters as in the discovery stage were used again, performing cross validation by leaving-out randomly selected samples - 3 control samples and 3 toxic samples and repeating 500 times, choosing the bound on 'toxicity' as to maximize the accuracy. Two equivalent (but not identical in results) signatures were found, presented in Tables 26 and 27, consisting of 4 and 6 amplicons, coming from 3 and 4 genes, respectively. The cross-validation performance evaluations of these signatures show 80% sensitivity at about 85% specificity. The Out-Of-Bag OOB performance evaluation gave the confusion matrix presented in Table 29 (overall accuracy of 83%) and ROC curve presented in Figure 6.

Table 29: Confusion matrix

| | Normal | Toxic |
|---|---|---|
| Predicted as Normal | 34 | 4 |
| Predicted as Toxic | 7 | 19 |

It was possible to find a bound on the Random-Forest call - 30% of the trees in the forests giving a "Toxic" call - such that all "Toxic" samples pass that bound while only one group of control samples (Naive males) has more than 50% (2 out of 3) of its members pass that bound. See Tables 26 and 27 for details on the signatures and Table 28 for their performance

qRT-PCR Data Analysis of the Un-labeled Samples, described in plates 4-7 in Table 25

The classifiers described in Tables 26 and 27, were used for testing the un-labeled samples. Both classifiers were applied to the measurements of each sample, used the 30% bound from above, and the number of samples pass that bound in each group were summarized. The results are given in the following Table 30

Table 30

| Group (Compound, Dose, Period, Gender) | Animals (#) | ’Toxic’ by 4-genes Classifier (#) | ’Toxic’ by 6-genes Classifier (#) | ’Toxic’ by 4-genes Classifier (%) | ’Toxic’ by 6-genes Classifier (#) | Average ’Toxic’ (%) |
|---|---|---|---|---|---|---|
| T2 Ds1 Day5 Male | 6 | 5 | 5 | 83 | 83 | 0.83 |
| T1 Ds2 Day5 Female | 6 | 5 | 3 | 83 | 50 | 0.665 |
| control Day5 Male | 5 | 3 | 3 | 60 | 60 | 0.6 |
| T2 Ds2 Day1 Female | 6 | 4 | 3 | 67 | 50 | 0.585 |
| T3 Ds1 Day5 Male | 5 | 3 | 2 | 60 | 40 | 0.5 |
| T2 Ds2 Day5 Female | 4 | 2 | 2 | 50 | 50 | 0.5 |
| T1 Ds1 Day5 Male | 6 | 3 | 3 | 50 | 50 | 0.5 |
| T1 Ds1 Day5 Female | 6 | 3 | 3 | 50 | 50 | 0.5 |
| control Day5 Female | 6 | 3 | 2 | 50 | 33 | 0.415 |
| T3 Ds2 Day5 Male | 4 | 2 | 1 | 50 | 25 | 0.375 |
| control Day1 Male | 6 | 2 | 2 | 33 | 33 | 0.33 |
| T1 Ds2 Day5 Male | 6 | 2 | 2 | 33 | 33 | 0.33 |
| T3 Ds2 Day1 Male | 5 | 2 | 1 | 40 | 20 | 0.3 |
| T2 Ds2 Day5 Male | 5 | 1 | 2 | 20 | 40 | 0.3 |
| T2 Ds1 Day5 Female | 5 | 2 | 1 | 40 | 20 | 0.3 |
| T2 Ds1 Day1 Male | 6 | 2 | 1 | 33 | 17 | 0.25 |
| T3 Ds2 Day5 Female | 5 | 1 | 1 | 20 | 20 | 0.2 |
| T3 Ds2 Day1 Female | 5 | 1 | 1 | 20 | 20 | 0.2 |
| T3 Ds1 Day1 Female | 5 | 1 | 1 | 20 | 20 | 0.2 |
| T2 Ds1 Day1 Female | 5 | 1 | 1 | 20 | 20 | 0.2 |
| control Day1 Female | 6 | 1 | 1 | 17 | 17 | 0.17 |
| T1 Ds1 Day1 Male | 6 | 1 | 1 | 17 | 17 | 0.17 |
| T3 Ds1 Day5 Female | 6 | 1 | 0 | 17 | 0 | 0.085 |
| T3 Ds1 Day1 Male | 6 | 0 | 0 | 0 | 0 | 0 |

(continued)

| Group (Compound, Dose, Period, Gender) | Animals (#) | 'Toxic' by 4-genes Classifier (#) | 'Toxic' by 6-genes Classifier (#) | 'Toxic' by 4-genes Classifier (%) | 'Toxic' by 6-genes Classifier (#) | Average 'Toxic' (%) |
|---|---|---|---|---|---|---|
| T2 Ds2 Day1 Male | 6 | 0 | 0 | 0 | 0 | 0 |
| T1 Ds2 Day1 Male | 6 | 0 | 0 | 0 | 0 | 0 |
| | | | | | | |
| Group (Compound, Dose, Period, Gender) | Animals (#) | 'Toxic' by 4-genes Classifier (#) | 'Toxic' by 6-genes Classifier (#) | 'Toxic' by 4-genes Classifier (%) | 'Toxic' by 6-genes Classifier (#) | Average 'Toxic' (%) |
| T1 Ds2 Day1 Female | 6 | 0 | 0 | 0 | 0 | 0 |
| T1 Ds1 Day1 Female | 5 | 0 | 0 | 0 | 0 | 0 |

The first two groups - "T2 Ds1 Day5 Male" and "T1 Ds2 Day5 Female" showed a clear nephrotoxic effect (more than 66% of samples were marked as "Toxic" when averaging the two classifiers), while the following six groups - "control Day5 Male", "T2 Ds2 Day1 Female", "T3 Ds1 Day5 Male", "T2 Ds2 Day5 Female" , "T1 Ds1 Day5 Male", "T1 Ds1 Day5 Female" were also suspicious of showing the effect (at least 50% of samples predicted as 'Toxic' on average). Therefore, consistently with the information available for the compounds T1 and T2, the results demonstrated that T1 and T2 are nephrotoxic agents. The T3 and control groups showing 'possible' toxic effect are either real false-positive of the classifier, or a result of some technical problem.

Using the classifier the toxicity was established after the rats were treated for 5 days only, while the visible pathological damage was seen only after 28 days for T1 and after 7 days for T2, as shown in Figures 3-5.

Therefore, combinations of biomarkers according to the present invention can be used for early detection of drug-induced nephrotoxicity. These signatures predict the future occurrence of drug induced renal toxicity before it is detected using the traditional endpoint analysis such as histopathology or clinical chemistry.

According to the present invention, an optimized assay based on these signatures can be implemented in pre-clinical studies. The marker of the invention can be used alone or in combination with other known markers for renal damage identification.

**Claims**

1. A diagnostic isolated polynucleotide marker comprising the nucleic acid sequence set forth in SEQ. ID NO: 2.

2. The diagnostic marker of claim 1, wherein the isolated polynucleotide consists of the nucleic acid sequence set forth in SEQ. ID NO: 2.

3. A method for predicting onset of renal injury caused by treatment with a compound, comprising (a) measuring the expression level of a set of polynucleotide markers in a biological sample obtained from at least one test mammal at a selected time period after the compound had been administered to said mammal; and (b) determining whether the sample is in the positive class for onset of renal injury using a classifier comprising the set of polynucleotide markers for which the expression level is measured, wherein said expression of said set of markers is indicative of the onset of renal injury, **characterized in that**

(i) the set comprises four polynucleotide markers comprising a first polynucleotide having the nucleic acid sequence set forth in SEQ ID NO:2 or the nucleic acid sequence set forth in SEQ ID NO:254; a second polynucleotide having the nucleic acid sequence set forth in SEQ ID NO:4 or the nucleic acid sequence set forth in SEQ ID NO:5 or the nucleic acid sequence set forth in SEQ ID NO:266 or the nucleic acid sequence set forth in SEQ ID NO:329; a third polynucleotide having the nucleic acid sequence set forth in SEQ ID NO:7 or the nucleic acid sequence set forth in SEQ ID NO:8 or the nucleic acid sequence set forth in SEQ ID NO:287; and a fourth polynucleotide having the nucleic acid sequence set forth in SEQ ID NO:13 or the nucleic acid sequence set forth in SEQ ID NO:14 or the nucleic acid sequence set forth in SEQ ID NO:296; or
(ii) the set comprises four polynucleotide markers comprising a first polynucleotide having the nucleic acid sequence set forth in SEQ ID NO:2 or the nucleic acid sequence set forth in SEQ ID NO:254; a second polynucleotide having the nucleic acid sequence set forth in SEQ ID NO:4 or the nucleic acid sequence set forth in SEQ ID NO:5 or the nucleic acid sequence set forth in SEQ ID NO:266 or the nucleic acid sequence set forth in SEQ ID NO:329; a third polynucleotide having the nucleic acid sequence set forth in SEQ ID NO:49 or the nucleic acid sequence set forth in SEQ ID NO:263; and a fourth polynucleotide having the nucleic acid sequence set forth in SEQ ID NO:53 or the nucleic acid sequence set forth in SEQ ID NO:290 or the nucleic acid sequence set forth in SEQ ID NO:332; or
(iii) the set comprises six polynucleotide markers comprising a first polynucleotide having the nucleic acid sequence set forth in SEQ ID NO:2 or the nucleic acid sequence set forth in SEQ ID NO:254; a second polynucleotide having the nucleic acid sequence set forth in SEQ ID NO:4 or the nucleic acid sequence set forth in SEQ ID NO:5 or the nucleic acid sequence set forth in SEQ ID NO:266 or the nucleic acid sequence set forth in SEQ ID NO:329; a third polynucleotide having the nucleic acid sequence set forth in SEQ ID NO:49 or the nucleic acid sequence set forth in SEQ ID NO:263; a fourth polynucleotide having the nucleic acid sequence set forth in SEQ ID NO:7 or the nucleic acid sequence set forth in SEQ ID NO:8 or the nucleic acid sequence set forth in SEQ ID NO:287; a fifth polynucleotide having the nucleic acid sequence set forth in SEQ ID NO:13 or the nucleic acid sequence set forth in SEQ ID NO:14 or the nucleic acid sequence set forth in SEQ ID NO:296; and a sixth polynucleotide having the nucleic acid sequence set forth in SEQ ID NO:53 or the nucleic acid sequence set forth in SEQ ID NO:290 or the nucleic acid sequence set forth in SEQ ID NO:332.

4. The method of claim 3, wherein the biological sample is selected from the group consisting of kidney tissue, body fluid and body secretion.

5. The method of claim 3,
wherein the test compound is nephrotoxic agent selected from the group consisting of aminoglycosides; platinum based chemotherapy agents; heavy metals; DNA interacting drugs; antifungal agents; proximal tubule damaging agents; and vasoconstriction compounds; or
wherein the renal injury is associated with at least one kidney disease or pathology, selected from the group consisting of nephrotoxicity, renal toxicity, nephritis, kidney necrosis, kidney damage, glomerular and tubular injury, focal segmental glomerulosclerosis, kidney dysfunction, nephritic syndrome, acute renal failure, chronic renal failure, proximal tubal dysfunction, acute kidney transplant rejection and chronic kidney transplant refection.

6. The method of claim 3, wherein the selected time period after the sample had been obtained from the test subject is prior to or at the onset of the appearance of histopathological or clinical indications of renal injury, preferably wherein the selected time period is selected from the group consisting of about 1 day, about 5 days, about 7 days, about 14 days, about 21 or about 28 days after administration of the test compound.

7. The method of claim 3, wherein the level of expression is detected by an amplification or hybridization assay, preferably wherein the amplification assay is selected from the group consisting of quantitative or semi-quantitative PCR, Northern blot, dot or slot blot, nuclease protection and microarray assays.

8. A set of at least two isolated polynucleotide probes or primers comprising a nucleic acid sequence that specifically hybridizes to the nucleic acid sequence set forth in SEQ ID NO: 2 for use in predicting the onset of renal injury.

9. The set of isolated polynucleotide probes or primers of claim 8, wherein the probes or primers comprise a set of two polynucleotides consisting of a nucleic acid sequence set forth in SEQ ID NOs: 252-253.

10. A kit for detecting renal injury, comprising a set of probes or pairs of primers that hybridize to at least a plurality of markers and reagents for detecting the plurality of polynucleotide markers, **characterized in that**

(i) said plurality of polynucleotide markers comprises a first polynucleotide having a the nucleic acid sequence set forth in SEQ ID NO:2 or the nucleic acid sequence set forth in SEQ ID NO:254; a second polynucleotide having the nucleic acid sequence set forth in SEQ ID NO:4 or the nucleic acid sequence set forth in SEQ ID NO:5 or the nucleic acid sequence set forth in SEQ ID NO:266 or the nucleic acid sequence set forth in SEQ ID NO:329; a third polynucleotide having the nucleic acid sequence set forth in SEQ ID NO:7 or the nucleic acid sequence set forth in SEQ ID NO:8 or the nucleic acid sequence set forth in SEQ ID NO:287; and a fourth polynucleotide having the nucleic acid sequence set forth in SEQ ID NO:13 or the nucleic acid sequence set forth in SEQ ID NO:14 or the nucleic acid sequence set forth in SEQ ID NO:296; or
(ii) said plurality of polynucleotide markers comprises a first polynucleotide having the nucleic acid sequence set forth in SEQ ID NO:2 or the nucleic acid sequence set forth in SEQ ID NO:254; a second polynucleotide having the nucleic acid sequence set forth in SEQ ID NOs:4 or the nucleic acid sequence set forth in SEQ ID NO:5 or the nucleic acid sequence set forth in SEQ ID NO:266 or the nucleic acid sequence set forth in SEQ ID NO:329; a third polynucleotide having the nucleic acid sequence set forth in SEQ ID NO:49 or the nucleic acid sequence set forth in SEQ ID NO:263; and a fourth polynucleotide having the nucleic acid sequence set forth in SEQ ID NO:53 or the nucleic acid sequence set forth in SEQ ID NO:290 or the nucleic acid sequence set forth in SEQ ID NO:332; or
(iii) said plurality of polynucleotide markers comprises a first polynucleotide having the nucleic acid sequence set forth in SEQ ID NO:2 or the nucleic acid sequence set forth in SEQ ID NO:254; a second polynucleotide having the nucleic acid sequence set forth in SEQ ID NO:4 or the nucleic acid sequence set forth in SEQ ID NO:5 or the nucleic acid sequence set forth in SEQ ID NO:266 or the nucleic acid sequence set forth in SEQ ID NO:329; a third polynucleotide having the nucleic acid sequence set forth in SEQ ID NO:49 or the nucleic acid sequence set forth in SEQ ID NO:263; a fourth polynucleotide having the nucleic acid sequence set forth in SEQ ID NO:7 or the nucleic acid sequence set forth in SEQ ID NO:8 or the nucleic acid sequence set forth in SEQ ID NO:287; a fifth polynucleotide having the nucleic acid sequence set forth in SEQ ID NO:13 or the nucleic acid sequence set forth in SEQ ID NO:14 or the nucleic acid sequence set forth in SEQ ID NO:296; and a sixth polynucleotide having r the nucleic acid sequence set forth in SEQ ID NO:53 or the nucleic acid sequence set forth in SEQ ID NO:290 or the nucleic acid sequence set forth in SEQ ID NO:332.

11. The kit of claim 10, wherein the set comprises a probe or pair of primers having the nucleic acids sequence set forth in SEQ ID NO:252, SEQ ID NO:253 or a combination thereof; and

(i) a probe or pair of primer having the nucleic acid sequence set forth in SEQ ID NOs:264, 265 or a combination thereof, or SEQ ID NOs:327, 328 or a combination thereof; SEQ ID NOs:285, 286 or a combination thereof; and SEQ ID NOs:294, 295 or a combination thereof; or
(ii) a probe or pair of primer having the nucleic acid sequence set forth in SEQ ID NOs:264, 265 or a combination thereof, or SEQ ID NOs:327, 328 or a combination thereof, or SEQ ID NOs:261, 262 or a combination thereof; SEQ ID NOs:288, 289 or a combination thereof; and SEQ ID NOs:330, 331 or a combination thereof; or
(iii) a probe or pair of primer having the nucleic acid sequence set forth in SEQ ID NOs: 264, 265 or a combination thereof, or SEQ ID NOs:327, 328, or a combination thereof; SEQ ID NOs:261, 262 or a combination thereof; SEQ ID NOs: 285, 286 or a combination thereof; SEQ ID NOs:294, 295 or a combination thereof; and SEQ ID NOs: 288, 289 or a combination thereof or SEQ ID NOs:330, 331 or a combination thereof.

12. The kit of claim 10, wherein the reagents for detecting the at least two marker are reagents for employing a NAT-based technology, preferably wherein the NAT-based assay is selected from the group consisting of a PCR, Real-Time PCR, LCR, Self-Sustained Synthetic Reaction, Q-Beta Replicase, Cycling Probe Reaction, Branched DNA, RFLP analysis, DGGE/TGGE, Single-Strand Conformation Polymorphism, Dideoxy Fingerprinting, Microarrays, Fluorescence, *In Situ* Hybridization or Comparative Genomic Hybridization.

## Patentansprüche

1. Diagnostischer isolierter Polynukleotid-Marker welcher die Nukleinsäuresequenz gemäß SEQ. ID NO: 2 umfasst.

2. Diagnostischer Marker nach Anspruch 1, worin das isolierte Polynukleotid aus der Nukleinsäuresequenz gemäß SEQ. ID NO: 2 besteht.

3. Verfahren zur Vorhersage des Beginns von Nierenschädigungen, die durch Behandlung mit einer Verbindung hervorgerufen wurden, welches umfasst, (a) Erfassen des Expressionsniveaus eines Satzes an Polynukleotid-Marker in einer biologischen Probe, die von mindestens einem Test-Säuger erhalten wurde, zu einem bestimmten Zeitpunkt nach Verabreichung der Verbindung an den Säuger; und (b) Bestimmen, ob die Probe in einer positiven Klasse für den Beginn einer Nierenschädigung ist, wobei ein Klassifizierungsmittel verwendet wird, das den Satz an Polynukleotid-Marker umfasst, für den das Expressionsniveau bestimmt wird, wobei die Expression des Marker-Satzes den Beginn einer Nierenschädigung anzeigt, **dadurch gekennzeichnet, dass**

   (i) der Satz vier Polynukleotid-Marker umfasst, welche umfassen, ein erstes Polynukleotid, mit der Nukleinsäuresequenz gemäß SEQ ID NO:2 oder der Nukleinsäuresequenz gemäß SEQ ID NO:254; ein zweites Polynukleotid mit der Nukleinsäuresequenz gemäß SEQ ID NO:4 oder der Nukleinsäuresequenz gemäß SEQ ID NO:5 oder der Nukleinsäuresequenz gemäß SEQ ID NO:266 oder der Nukleinsäuresequenz gemäß SEQ ID NO:329; ein drittes Polynukleotid mit der Nukleinsäuresequenz gemäß SEQ ID NO:7 oder der Nukleinsäuresequenz gemäß SEQ ID NO:8 oder der Nukleinsäuresequenz gemäß SEQ ID NO:287; und ein viertes Polynukleotid mit der Nukleinsäuresequenz gemäß SEQ ID NO:13 oder der Nukleinsäuresequenz gemäß SEQ ID NO:14 oder der Nukleinsäuresequenz gemäß SEQ ID NO:296; oder
   (ii) der Satz vier Polynukleotid-Marker umfasst, die umfassen, ein erstes Polynukleotid mit der Nukleinsäuresequenz gemäß SEQ ID NO:2 oder der Nukleinsäuresequenz gemäß SEQ ID NO:254; ein zweites Polynukleotid mit der Nukleinsäuresequenz gemäß SEQ ID NO:4 oder der Nukleinsäuresequenz gemäß SEQ ID NO:5 oder der Nukleinsäuresequenz gemäß SEQ ID NO:266 oder der Nukleinsäuresequenz gemäß SEQ ID NO:329; ein drittes Polynukleotid mit der Nukleinsäuresequenz gemäß SEQ ID NO:49 oder der Nukleinsäuresequenz gemäß SEQ ID NO:263; und ein viertes Polynukleotid mit der Nukleinsäuresequenz gemäß SEQ ID NO:53 oder der Nukleinsäuresequenz gemäß SEQ ID NO:290 oder der Nukleinsäuresequenz gemäß SEQ ID NO:332; oder
   (iii) der Satz sechs Polynukleotid-Marker umfasst, welche umfassen, ein erstes Polynukleotid mit der Nukleinsäuresequenz gemäß SEQ ID NO:2 oder der Nukleinsäuresequenz gemäß SEQ ID NO:254; ein zweites Polynukleotid mit der Nukleinsäuresequenz gemäß SEQ ID NO:4 oder der Nukleinsäuresequenz gemäß SEQ ID NO:5 oder der Nukleinsäuresequenz gemäß SEQ ID NO:266 oder der Nukleinsäuresequenz gemäß SEQ ID NO:329; ein drittes Polynukleotid mit der Nukleinsäuresequenz gemäß SEQ ID NO:49 oder der Nukleinsäuresequenz gemäß SEQ ID NO:263; ein viertes Polynukleotid mit der Nukleinsäuresequenz gemäß SEQ ID NO:7 oder der Nukleinsäuresequenz gemäß SEQ ID NO:8 oder der Nukleinsäuresequenz gemäß SEQ ID NO:287; ein fünftes Polynukleotid mit der Nukleinsäuresequenz gemäß SEQ ID NO:13 oder der Nukleinsäuresequenz gemäß SEQ ID NO:14 oder der Nukleinsäuresequenz gemäß SEQ ID NO:296; und ein sechstes Polynukleotid mit der Nukleinsäuresequenz gemäß SEQ ID NO:53 oder der Nukleinsäuresequenz gemäß SEQ ID NO:290 oder der Nukleinsäuresequenz gemäß SEQ ID NO:332.

4. Verfahren nach Anspruch 3, wobei die biologische Probe ausgewählt ist aus der Gruppe bestehend aus Nierengewebe, Körperflüssigkeit und Körper-Sekreten.

5. Verfahren nach Anspruch 3,
   wobei die Test-Verbindung ein nephrotoxisches Mittel ist ausgewählt ist aus der Gruppe bestehend aus Aminoglykosiden; auf Platin basierenden chemotherapeutischen Mitteln; Schwermetallen; mit DNA interagierenden Arzneimitteln; anti-Pilz-Mitteln; proximale Tuben schädigenden Mitteln; und Vasokonstriktions-Verbindungen; oder
   wobei die Nieren-Schädigung mit mindestens einer Nieren-Erkrankung oder Pathologie assoziiert ist, ausgewählt ist aus der Gruppe bestehend aus Nephrotoxizität, renaler Toxizität, Nephritis, Nieren-Nekrose, Nieren-Schädigung, glomerulärer und tubulärer Schädigung, fokaler segmentaler Glomerulosklerose, Nieren-Dysfunktion, nephritischem Syndrom, akutem Nierenversagen, chronischem Nierenversagen, proximaler tubulärer Dysfunktion, akuter Nieren-Transplantat-Abstoßung und chronischer Nieren-Transplantat-Auffrischung.

6. Verfahren nach Anspruch 3, wobei die gewählte Zeitspanne nach Erhalt der Probe von dem Test-Individuum vor oder beim Auftreten histopathologischer oder klinischer Anzeichen einer Nieren-Verletzung liegt, vorzugsweise wobei die gewählte Zeitspanne ausgewählt ist aus der Gruppe bestehend aus etwa 1 Tag, etwa 5 Tagen, etwa 7 Tagen, etwa 14 Tagen, etwa 21 oder etwa 28 Tagen nach Verabreichung der Test-Verbindung.

7. Verfahren nach Anspruch 3, wobei das Expressionsniveau durch einen Amplifizierungs- oder Hybridiserungs-Assay erfasst wird, vorzugsweise wobei der Amplifizierungs-Assay ausgewählt ist aus der Gruppe bestehend aus quantitativer oder halb-quantitativer PCR, Northem Blot, Dot- oder Slot-Blot, Nuklease-Schutz und Mikroarray-Assays.

8. Satz von mindestens zwei isolierten Polynukleotid-Sonden oder -Primern, welcher eine Nukleinsäuresequenz umfasst, die spezifisch an einen Marker hybridisiert, der die Nukleinsäuresequenz gemäß SEQ ID NO: 2 umfasst, zur Verwendung bei der Vorhersage einer Nieren-Verletzung.

9. Satz isolierter Polynukleotid-Sonden oder -Primer nach Anspruch 8, worin die Sonden oder Primer einen Satz von zwei Polynukleotiden umfassen, die aus den Nukleinsäuresequenzen gemäß SEQ ID NOs: 252-253 bestehen.

10. Kit zum Nachweis einer Nieren-Schädigung, welcher einen Satz an Sonden oder Primer-Paaren umfasst, die an mindestens mehrere Marker hybridisieren, und Reagentien zum Nachweis der mehreren Polynukleotid-Marker, **dadurch gekennzeichnet, dass**

(i) die mehreren Polynukleotid-Marker umfassen, ein erstes Polynukleotid mit der Nukleinsäuresequenz gemäß SEQ ID NO:2 oder der Nukleinsäuresequenz gemäß SEQ ID NO:254; ein zweites Polynukleotid mit der Nukleinsäuresequenz gemäß SEQ ID NO:4 oder der Nukleinsäuresequenz gemäß SEQ ID NO:5 oder der Nukleinsäuresequenz gemäß SEQ ID NO:266 oder der Nukleinsäuresequenz gemäß SEQ ID NO:329; ein drittes Polynukleotid mit der Nukleinsäuresequenz gemäß SEQ ID NO:7 oder der Nukleinsäuresequenz gemäß SEQ ID NO:8 oder der Nukleinsäuresequenz gemäß SEQ ID NO:287; und ein viertes Polynukleotid mit der Nukleinsäuresequenz gemäß SEQ ID NO:13 oder der Nukleinsäuresequenz gemäß SEQ ID NO:14 oder der Nukleinsäuresequenz gemäß SEQ ID NO:296; oder
(ii) die mehreren Polynukleotid-Marker umfassen, ein erstes Polynukleotid mit der Nukleinsäuresequenz gemäß SEQ ID NO:2 oder der Nukleinsäuresequenz gemäß SEQ ID NO:254; ein zweites Polynukleotid mit der Nukleinsäuresequenz gemäß SEQ ID NOs:4 oder der Nukleinsäuresequenz gemäß SEQ ID NO:5 oder der Nukleinsäuresequenz gemäß SEQ ID NO:266 oder der Nukleinsäuresequenz gemäß SEQ ID NO:329; ein drittes Polynukleotid mit der Nukleinsäuresequenz gemäß SEQ ID NO:49 oder der Nukleinsäuresequenz gemäß SEQ ID NO:263; und ein viertes Polynukleotid mit der Nukleinsäuresequenz gemäß SEQ ID NO:53 oder der Nukleinsäuresequenz gemäß SEQ ID NO:290 oder der Nukleinsäuresequenz gemäß SEQ ID NO:332; oder
(iii) die mehreren Polynukleotid-Marker umfassen, ein erstes Polynukleotid mit der Nukleinsäuresequenz gemäß SEQ ID NO:2 oder der Nukleinsäuresequenz gemäß SEQ ID NO:254; ein zweites Polynukleotid mit der Nukleinsäuresequenz gemäß SEQ ID NO:4 oder der Nukleinsäuresequenz gemäß SEQ ID NO:5 oder der Nukleinsäuresequenz gemäß SEQ ID NO:266 oder der Nukleinsäuresequenz gemäß SEQ ID NO:329; ein drittes Polynukleotid mit der Nukleinsäuresequenz gemäß SEQ ID NO:49 oder der Nukleinsäuresequenz gemäß SEQ ID NO:263; ein viertes Polynukleotid mit der Nukleinsäuresequenz gemäß SEQ ID NO:7 oder der Nukleinsäuresequenz gemäß SEQ ID NO:8 oder der Nukleinsäuresequenz gemäß SEQ ID NO:287; ein fünftes Polynukleotid mit der Nukleinsäuresequenz gemäß SEQ ID NO:13 oder der Nukleinsäuresequenz gemäß SEQ ID NO:14 oder der Nukleinsäuresequenz gemäß SEQ ID NO:296; und ein sechstes Polynukleotid mit der Nukleinsäuresequenz gemäß SEQ ID NO:53 oder der Nukleinsäuresequenz gemäß SEQ ID NO:290 oder der Nukleinsäuresequenz gemäß SEQ ID NO:332.

11. Kit nach Anspruch 10, wobei der Satz eine Sonde oder ein Primer-Paar umfasst, mit der Nukleinsäuresequenz gemäß SEQ ID NO:252, SEQ ID NO:253 oder eine Kombination davon; und

(i) eine Sonde oder ein Primer-Paar mit der Nukleinsäuresequenz gemäß SEQ ID NOs:264, 265 oder eine Kombination davon, oder SEQ ID NOs:327, 328 oder eine Kombination davon; SEQ ID NOs:285, 286 oder eine Kombination davon; und SEQ ID NOs:294, 295 oder eine Kombination davon; oder
(ii) eine Sonde oder ein Primer-Paar mit der Nukleinsäuresequenz gemäß SEQ ID NOs:264, 265 oder eine Kombination davon, oder SEQ ID NOs:327, 328 oder eine Kombination davon, oder SEQ ID NOs:261, 262 oder eine Kombination davon; SEQ ID NOs:288, 289 oder eine Kombination davon; und SEQ ID NOs:330, 331 oder eine Kombination davon; oder
(iii) eine Sonde oder ein Primer-Paar mit der Nukleinsäuresequenz gemäß SEQ ID NOs: 264, 265 oder eine Kombination davon, oder SEQ ID NOs:327, 328, oder eine Kombination davon; SEQ ID NOs:261, 262 oder eine Kombination davon; SEQ ID NOs: 285, 286 oder eine Kombination davon; SEQ ID NOs:294, 295 oder eine Kombination davon; und SEQ ID NOs: 288, 289 oder eine Kombination davon oder SEQ ID NOs:330, 331 oder eine Kombination davon.

**12.** Kit nach Anspruch 10, worin die Reagentien zum Nachweis der mindestens zwei Marker Reagentien sind, um eine NAT-basierende Technology durchzuführen, vorzugsweise worin der NAT-basierende Assay ausgewählt ist aus der Gruppe bestehend aus PCR, Echtzeit-PCR, LCR, selbst erhaltender synthetischer Reaktion, Q-Beta Replikase, zyklisierender Sonden-Reaktion, verzweigter DNA, RFLP-Analyse, DGGE/TGGE, Einzelstrang-Konformations-Polymorphismus, Dideoxy-Fingerprinting, Mikroarrays, Fluoreszenz, *In Situ* Hybridisierung oder vergleichender Genom-Hybridisierung.

**Revendications**

**1.** Marqueur de diagnostic de type polynucléotide isolé comprenant la séquence d'acides aminés indiquée dans la SEQ ID NO : 2.

**2.** Marqueur de diagnostic selon la revendication 1, dans lequel le polynucléotide isolé se compose de la séquence d'acides aminés indiquée dans la SEQ ID NO : 2.

**3.** Méthode de prédiction de l'apparition d'une lésion rénale provoquée par un traitement avec un composé, consistant à (a) mesurer le taux d'expression d'une série de marqueurs polynucléotidiques dans un échantillon biologique obtenu à partir d'au moins un mammifère à tester à un moment déterminé après l'administration du composé audit mammifère ; et (b) déterminer si l'échantillon est dans la classe positive pour l'apparition d'une lésion rénale en utilisant un classificateur comprenant la série de marqueurs polynucléotidiques pour lesquels le taux d'expression est mesuré, ladite expression de ladite série de marqueurs étant indicatrice de l'apparition d'une lésion rénale, **caractérisée en ce que**

(i) la série comprend quatre marqueurs polynucléotidiques comprenant un premier polynucléotide ayant la séquence d'acides nucléiques indiquée dans la SEQ ID NO : 2 ou la séquence d'acides nucléiques indiquée dans la SEQ ID NO : 254 ; un deuxième polynucléotide ayant la séquence d'acides nucléiques indiquée dans la SEQ ID NO : 4 ou la séquence d'acides nucléiques indiquée dans la SEQ ID NO : 5 ou la séquence d'acides nucléiques indiquée dans la SEQ ID NO : 266 ou la séquence d'acides nucléiques indiquée dans la SEQ ID NO : 329 ; un troisième polynucléotide ayant la séquence d'acides nucléiques indiquée dans la SEQ ID NO : 7 ou la séquence d'acides nucléiques indiquée dans la SEQ ID NO : 8 ou la séquence d'acides nucléiques indiquée dans la SEQ ID NO : 287 ; et un quatrième polynucléotide ayant la séquence d'acides nucléiques indiquée dans la SEQ ID NO : 13 ou la séquence d'acides nucléiques indiquée dans la SEQ ID NO : 14 ou la séquence d'acides nucléiques indiquée dans la SEQ ID NO : 296 ; ou
(ii) la série comprend quatre marqueurs polynucléotidiques comprenant un premier polynucléotide ayant la séquence d'acides nucléiques indiquée dans la SEQ ID NO : 2 ou la séquence d'acides nucléiques indiquée dans la SEQ ID NO : 254 ; un deuxième polynucléotide ayant la séquence d'acides nucléiques indiquée dans la SEQ ID NO : 4 ou la séquence d'acides nucléiques indiquée dans la SEQ ID NO : 5 ou la séquence d'acides nucléiques indiquée dans la SEQ ID NO : 266 ou la séquence d'acides nucléiques indiquée dans la SEQ ID NO : 329 ; un troisième polynucléotide ayant la séquence d'acides nucléiques indiquée dans la SEQ ID NO : 49 ou la séquence d'acides nucléiques indiquée dans la SEQ ID NO : 263 ; et un quatrième polynucléotide ayant la séquence d'acides nucléiques indiquée dans la SEQ ID NO : 53 ou la séquence d'acides nucléiques indiquée dans la SEQ ID NO : 290 ou la séquence d'acides nucléiques indiquée dans la SEQ ID NO : 332 ; ou
(iii) la série comprend six marqueurs polynucléotidiques comprenant un premier polynucléotide ayant la séquence d'acides nucléiques indiquée dans la SEQ ID NO : 2 ou la séquence d'acides nucléiques indiquée dans la SEQ ID NO : 254 ; un deuxième polynucleotide ayant la séquence d'acides nucléiques indiquée dans la SEQ ID NO : 4 ou la séquence d'acides nucléiques indiquée dans la SEQ ID NO : 5 ou la séquence d'acides nucléiques indiquée dans la SEQ ID NO : 266 ou la séquence d'acides nucléiques indiquée dans la SEQ ID NO : 329 ; un troisième polynucléotide ayant la séquence d'acides nucléiques indiquée dans la SEQ ID NO : 49 ou la séquence d'acides nucléiques indiquée dans la SEQ ID NO : 263 ; un quatrième polynucléotide ayant la séquence d'acides nucléiques indiquée dans la SEQ ID NO : 7 ou la séquence d'acides nucléiques indiquée dans la SEQ ID NO : 8 ou la séquence d'acides nucléiques indiquée dans la SEQ ID NO : 287 ; un cinquième polynucléotide ayant la séquence d'acides nucléiques indiquée dans la SEQ ID NO : 13 ou la séquence d'acides nucléiques indiquée dans la SEQ ID NO : 14 ou la séquence d'acides nucléiques indiquée dans la SEQ ID NO : 296 ; et un sixième polynucléotide ayant la séquence d'acides nucléiques indiquée dans la SEQ ID NO : 53 ou la séquence d'acides nucléiques indiquée dans la SEQ ID NO : 290 ou la séquence d'acides nucléiques indiquée dans la SEQ ID NO : 332.

**4.** Méthode selon la revendication 3, dans laquelle l'échantillon biologique est sélectionné dans le groupe constitué par un tissu rénal, un fluide corporel et une sécrétion corporelle.

**5.** Méthode selon la revendication 3,
dans laquelle le composé à tester est un agent néphrotoxique sélectionné dans le groupe constitué par les aminoglycosides ; les agents chimiothérapeutiques à base de platine ; les métaux lourds ; les médicaments interagissant avec l'ADN ; les agents antifongiques ; les agents endommageant le tubule proximal ; et les composés vasoconstricteurs ; ou
dans laquelle la lésion rénale est associée à au moine une maladie ou pathologie rénale, sélectionnée dans le groupe constitué par une néphrotoxicité, une toxicité rénale, une néphrite, une nécrose rénale, une lésion rénale, une lésion glomérulaire et tubulaire, une glomérulosclérose segmentaire focale, un dysfonctionnement rénal, un syndrome néphritique, une insuffisance rénale aiguë, une insuffisance rénale chronique, un dysfonctionnement tubaire proximal, un rejet de greffe de rein aigu et un rejet de greffe de rein chronique.

**6.** Méthode selon la revendication 3, dans laquelle le moment déterminé après l'obtention de l'échantillon obtenu à partir du sujet à tester se situe avant ou au moment de l'apparition d'indications histopathologiques ou cliniques de lésion rénale, de préférence le moment déterminé est sélectionné dans le groupe constitué par environ 1 jour, environ 5 jours, environ 7 jours, environ 14 jours, environ 21 jours ou environ 28 jours après l'administration du composé à tester.

**7.** Méthode selon la revendication 3, dans laquelle le taux d'expression est détecté par un essai d'amplification ou d'hybridation, de préférence dans laquelle l'essai d'amplification est sélectionné dans le groupe constitué par une PCR quantitative ou semi-quantitative, un transfert de Northern, une hybridation sur tâche, des essais de protection de nucléase et de microréseaux.

**8.** Série d'au moins deux sondes ou amorces de type polynucléotide isolé comprenant une séquence d'acides nucléiques qui s'hybride spécifiquement à la séquence d'acides aminés indiquée dans la SEQ ID NO : 2 pour une utilisation dans la prédiction de l'apparition d'une lésion rénale.

**9.** Série de sondes ou d'amorces de type polynucléotide isolée selon la revendication 8, dans laquelle les sondes ou les amorces comprennent une série de deux polynucléotides constitués d'une séquence d'acides aminés indiquée dans les SEQ ID NO : 252-253.

**10.** Kit de détection d'une lésion rénale, comprenant une série de sondes ou d'amorces qui s'hybrident à au moins une pluralité de marqueurs et de réactifs pour la détection de la pluralité de marqueurs polynucléotidiques, **caractérisé en ce que**

(i) ladite pluralité de marqueurs polynucléotidiques comprend un premier polynucléotide ayant la séquence d'acides nucléiques indiquée dans la SEQ ID NO : 2 ou la séquence d'acides nucléiques indiquée dans la SEQ ID NO : 254 ; un deuxième polynucléotide ayant la séquence d'acides nucléiques indiquée dans la SEQ ID NO : 4 ou la séquence d'acides nucléiques indiquée dans la SEQ ID NO : 5 ou la séquence d'acides nucléiques indiquée dans la SEQ ID NO : 266 ou la séquence d'acides nucléiques indiquée dans la SEQ ID NO : 329 ; un troisième polynucleotide ayant la séquence d'acides nucléiques indiquée dans la SEQ ID NO : 7 ou la séquence d'acides nucléiques indiquée dans la SEQ ID NO : 8 ou la séquence d'acides nucléiques indiquée dans la SEQ ID NO : 287 ; et un quatrième polynucléotide ayant la séquence d'acides nucléiques indiquée dans la SEQ ID NO : 13 ou la séquence d'acides nucléiques indiquée dans la SEQ ID NO : 14 ou la séquence d'acides nucléiques indiquée dans la SEQ ID NO : 296 ; ou
(ii) ladite pluralité de marqueurs polynucléotidiques comprend un premier polynucléotide ayant la séquence d'acides nucléiques indiquée dans la SEQ ID NO : 2 ou la séquence d'acides nucléiques indiquée dans la SEQ ID NO : 254 ; un deuxième polynucléotide ayant la séquence d'acides nucléiques indiquée dans la SEQ ID NO : 4 ou la séquence d'acides nucléiques indiquée dans la SEQ ID NO : 5 ou la séquence d'acides nucléiques indiquée dans la SEQ ID NO : 266 ou la séquence d'acides nucléiques indiquée dans la SEQ ID NO : 329 ; un troisième polynucléotide ayant la séquence d'acides nucléiques indiquée dans la SEQ ID NO : 49 ou la séquence d'acides nucléiques indiquée dans la SEQ ID NO : 263 ; et un quatrième polynucléotide ayant la séquence d'acides nucléiques indiquée dans la SEQ ID NO : 53 ou la séquence d'acides nucléiques indiquée dans la SEQ ID NO : 290 ou la séquence d'acides nucléiques indiquée dans la SEQ ID NO : 332 ; ou

(iii) ladite pluralité de marqueurs polynucléotidiques comprend un premier polynucléotide ayant la séquence d'acides nucléiques indiquée dans la SEQ ID NO : 2 ou la séquence d'acides nucléiques indiquée dans la SEQ ID NO : 254 ; un deuxième polynucléotide ayant la séquence d'acides nucléiques indiquée dans la SEQ ID NO : 4 ou la séquence d'acides nucléiques indiquée dans la SEQ ID NO : 5 ou la séquence d'acides nucléiques indiquée dans la SEQ ID NO : 266 ou la séquence d'acides nucléiques indiquée dans la SEQ ID NO : 329 ; un troisième polynucléotide ayant la séquence d'acides nucléiques indiquée dans la SEQ ID NO : 49 ou la séquence d'acides nucléiques indiquée dans la SEQ ID NO : 263 ; un quatrième polynucléotide ayant la séquence d'acides nucléiques indiquée dans la SEQ ID NO : 7 ou la séquence d'acides nucléiques indiquée dans la SEQ ID NO : 8 ou la séquence d'acides nucléiques indiquée dans la SEQ ID NO : 287 ; un cinquième polynucléotide ayant la séquence d'acides nucléiques indiquée dans la SEQ ID NO : 13 ou la séquence d'acides nucléiques indiquée dans la SEQ ID NO : 14 ou la séquence d'acides nucléiques indiquée dans la SEQ ID NO : 296 ; et un sixième polynucléotide ayant la séquence d'acides nucléiques indiquée dans la SEQ ID NO : 53 ou la séquence d'acides nucléiques indiquée dans la SEQ ID NO : 290 ou la séquence d'acides nucléiques indiquée dans la SEQ ID NO : 332.

**11.** Kit selon la revendication 10, dans lequel la série comprend une sonde ou une paire d'amorces ayant la séquence d'acides nucléiques indiquée dans la SEQ ID NO : 252, la SEQ ID NO : 253 ou une combinaison de celles-ci ; et

(i) une sonde ou une paire d'amorces ayant la séquence d'acides nucléiques indiquée dans la SEQ ID NO : 264, la SEQ ID NO : 265 ou une combinaison de celles-ci, ou la SEQ ID NO : 327, la SEQ ID NO : 328 ou une combinaison de celles-ci ; la SEQ ID NO : 285, la SEQ ID NO : 286 ou une combinaison de celles-ci ; et la SEQ ID NO : 294, la SEQ ID NO : 295 ou une combinaison de celles-ci ; ou

(ii) une sonde ou une paire d'amorces ayant la séquence d'acides nucléiques indiquée dans la SEQ ID NO : 264, la SEQ ID NO : 265 ou une combinaison de celles-ci, ou la SEQ ID NO : 327, la SEQ ID NO : 328 ou une combinaison de celles-ci, ou la SEQ ID NO : 261, la SEQ ID NO : 262 ou une combinaison de celles-ci ; la SEQ ID NO : 288, la SEQ ID NO : 289 ou une combinaison de celles-ci ; et la SEQ ID NO : 330, la SEQ ID NO : 331 ou une combinaison de celles-ci ; ou

(iii) une sonde ou une paire d'amorces ayant la séquence d'acides nucléiques indiquée dans la SEQ ID NO : 264, la SEQ ID NO : 265 ou une combinaison de celles-ci, ou la SEQ ID NO : 327, la SEQ ID NO : 328 ou une combinaison de celles-ci ; la SEQ ID NO : 261, la SEQ ID NO : 262 ou une combinaison de celles-ci ; la SEQ ID NO : 285, la SEQ ID NO : 286 ou une combinaison de celles-ci ; la SEQ ID NO : 294, la SEQ ID NO : 295 ou une combinaison de celles-ci ; et la SEQ ID NO : 288, la SEQ ID NO : 289 ou une combinaison de celles-ci ou la SEQ ID NO : 330, la SEQ ID NO : 331 ou une combinaison de celles-ci.

**12.** Kit selon la revendication 10, dans lequel les réactifs pour la détection d'au moins deux marqueurs sont des réactifs destinés à être utilisés dans une technologie NAT, de préférence dans lequel l'essai basé sur la technologie NAT est sélectionné dans le groupe constitué par une PCR, une PCR en temps réel, une LCR, une réaction synthétique autogène, une amplification par Q-bêta réplicase, une amplification par dégradation cyclique de sonde, un ADN ramifié, une analyse RFLP, une DFFE/TGGE, un polymorphisme de conformation des ADN simples brins, une cartographie peptidique à base de didésoxy, des microréseaux, une fluorescence, une hybridation *in situ* ou une hybridation génomique comparative.

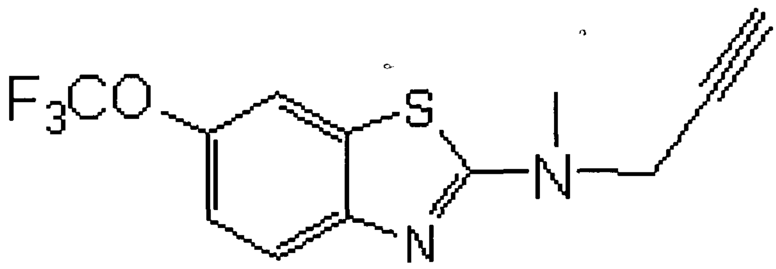

FIGURE 1

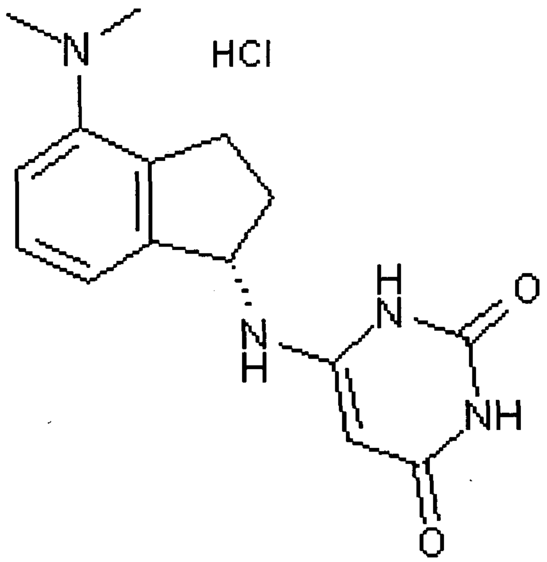

FIGURE 2

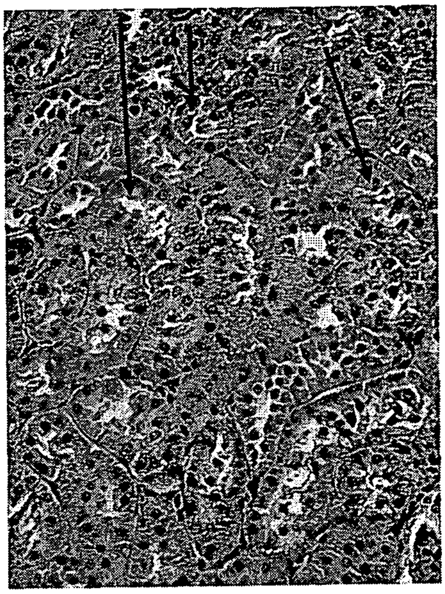

FIGURE 3A

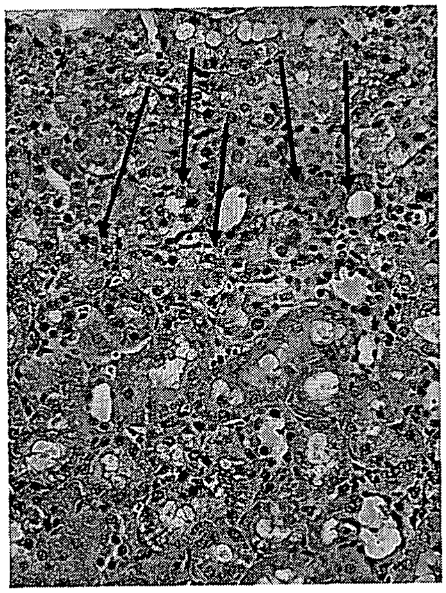

FIGURE 3B

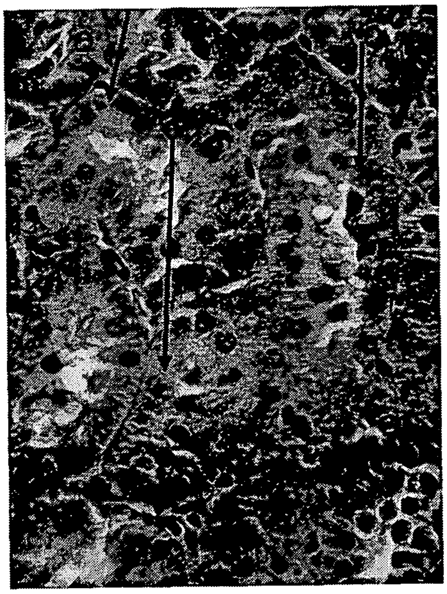

FIGURE 4A

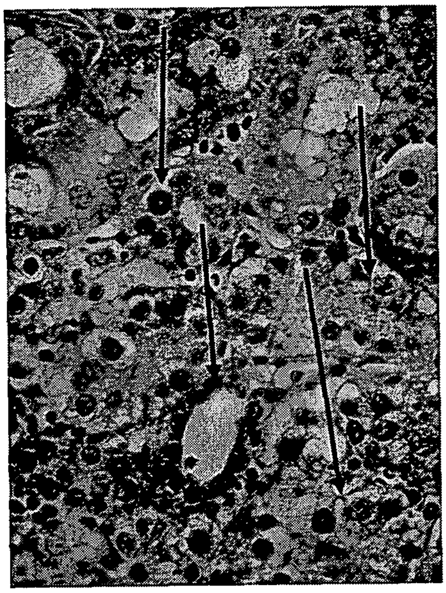

FIGURE 4B

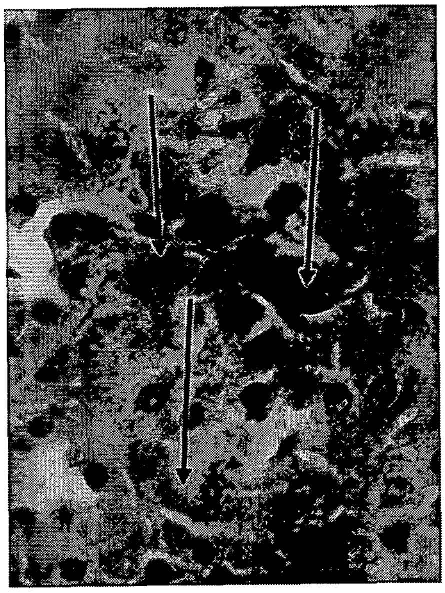

FIGURE 5A

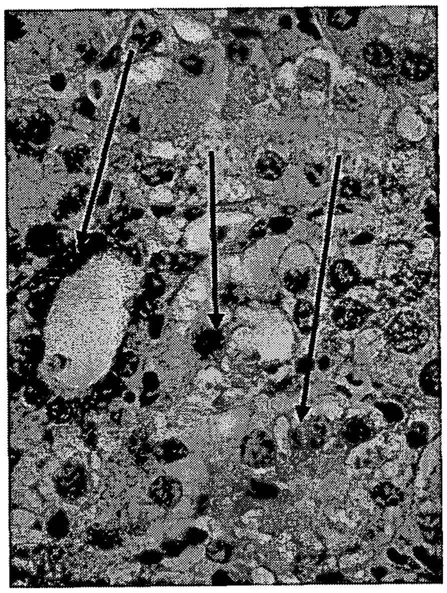

FIGURE 5B

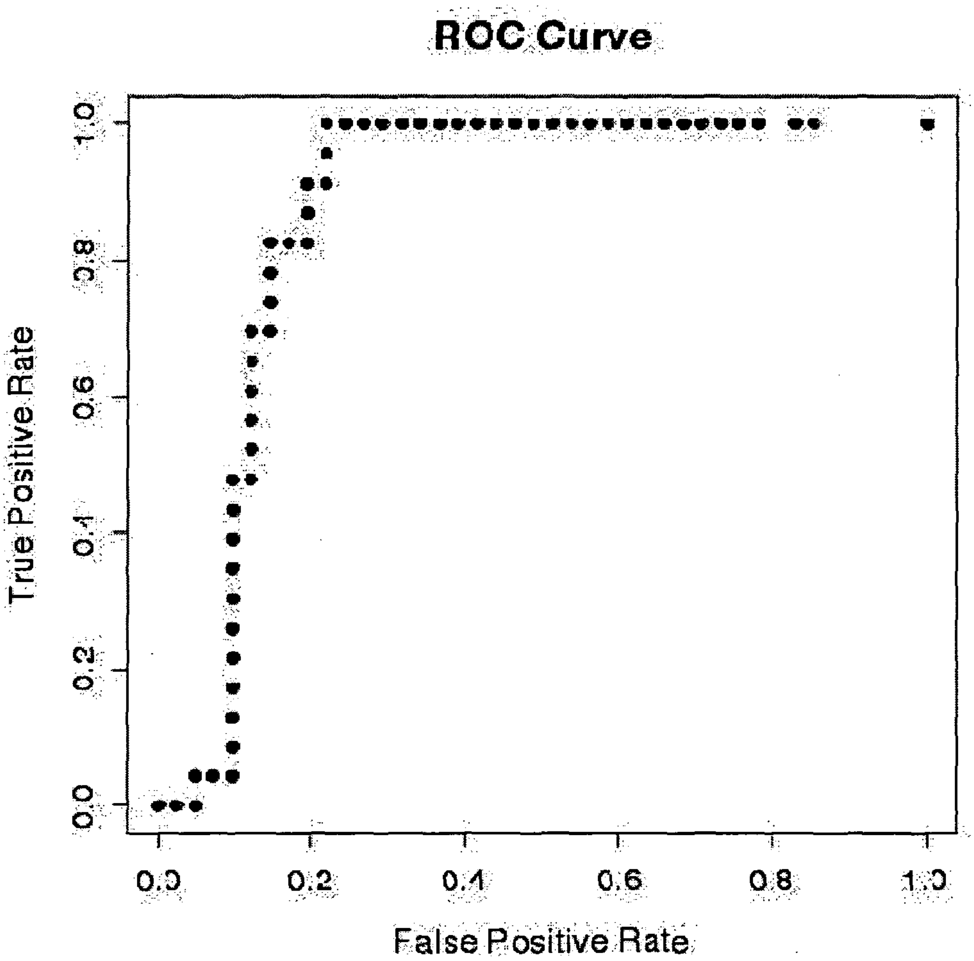
ROC Curve
True Positive Rate
0.0
0.2
0.4
0.6
0.8
1.0
False Positive Rate
0.0
0.2
0.4
0.6
0.8
1.0

FIGURE 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006033701 A
- WO 02095000 A
- US 4562157 A
- US 5143854 A
- US 5556961 A
- US 5968740 A
- US 6153744 A
- WO 9942813 A
- US 5474796 A
- US 5807522 A
- WO 9511755 A
- US 4683195 A
- US 4683202 A, Mullis and Mullis
- WO 9001069 A1
- US 6625545 B
- US 426002 A
- US 20040101876 A
- WO 2004048598 A
- WO 200295000 A
- WO 0295000 A


### Non-patent literature cited in the description


- **SCHRIER et al.** *J Clin Invest,* 2004, vol. 114 (1), 5-14
- **MATEJKA GL. et al.** *Exp Nephrol,* 1998, vol. 6, 253-264
- **NORMAN JT. et al.** *Proc Natl Acad Sci USA,* 1988, vol. 85, 6768-6772
- **SAFIRSTEIN R. et al.** *Kidney Int,* 1990, vol. 37, 1515-1521
- **HAMMERMAN et al.** *Curr Oppin Nephrol Hypertens,* 1998, vol. 7, 419-424
- **YOSHIDA et al.** *Kidney International,* 2002, vol. 61, 1646-1654
- **AMIN R.P et al.** *Environ Health Perspect.,* 2004, vol. 112 (4), 465-479
- **THOMAS R.S.** *Mol Pharmacol.,* 2001, vol. 60 (6), 1189-1194
- **YUEN P S T et al.** *Kidney Intern.,* 2006, vol. 70 (10), 1750-1758
- **KHARASCH E D et al.** *Toxicological Sciences,* 2006, vol. 90 (2), 419-31
- **BREIMAN ; LEO.** Random Forests. *Machine Learning,* 2001, vol. 45 (1), 5-32
- **LOCKHART et al.** *NatBiotechnol,* 1996, vol. 14, 1675-1680
- **MCGALL et al.** *Proc Nat Acad Sci USA,* 1996, vol. 93, 13555-13460
- **KWOH et al.** *Proc. Natl. Acad. Sci. USA,* 1989, vol. 86, 1173-1177
- **LIZARDI et al.** *BioTechnology,* 1988, vol. 6, 1197-1202
- **MALEK et al.** *Methods Mol. Biol.,* 1994, vol. 28, 253-260
- **SAMBROOK et al.** Molecular Cloning -A Laboratory Manual. CSH Laboratories, 1989
- **AUSUBEL et al.** Current Protocols in Molecular Biology. John Wiley & Sons Inc, 1989
- **HARLOW ; LANE.** *Antibodies, A Laboratory Manual,* 1988
- **SOREK, R. ; AST, G. ; GRAUR, D.** Alu-containing exons are alternatively spliced. *Genome Res,* 2002, vol. 12, 1060-7
- **PETER et al.** *Physiol. Genomics,* 2006, vol. 25, 375-386
- **THOMPSON et al.** *Environ Health Perspect.,* March 2004, vol. 112 (4), 488-94